(19) Europäisches Patentamt
European Patent Office
Office européen des brevets

(11) EP 2 408 927 B1

(12) **EUROPEAN PATENT SPECIFICATION**

(45) Date of publication and mention of the grant of the patent:
**27.11.2013 Bulletin 2013/48**

(21) Application number: **10715096.3**

(22) Date of filing: **19.03.2010**

(51) Int Cl.:
***C12Q 1/00*** *(2006.01)* ***G01N 33/542*** *(2006.01)*
***C12Q 1/34*** *(2006.01)*

(86) International application number:
**PCT/EP2010/001770**

(87) International publication number:
**WO 2010/105851 (23.09.2010 Gazette 2010/38)**

(54) **PROBE COMPOUND FOR DETECTING AND ISOLATING ENZYMES AND MEANS AND METHODS USING THE SAME**

SONDENVERBINDUNG ZUM NACHWEISEN UND ISOLIEREN VON ENZYMEN SOWIE MITTEL UND VERFAHREN ZU IHRER VERWENDUNG

COMPOSÉ DE SONDE POUR DÉTECTER ET ISOLER LES ENZYMES ET SUPPORTS ET PROCÉDÉS D'UTILISATION ASSOCIÉS

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **19.03.2009 EP 09003977**
**19.03.2009 US 210482 P**

(43) Date of publication of application:
**25.01.2012 Bulletin 2012/04**

(73) Proprietors:
- **Helmholtz-Zentrum für Infektionsforschung GmbH**
  **38124 Braunschweig (DE)**
- **Institute of Catalysis (CSIC)**
  **28049 Madrid (ES)**

(72) Inventors:
- **GOLYSHIN, Peter, N.**
  **Bangor LL57 2UW (GB)**
- **GOLYSHINA, Olga, V.**
  **Bangor LL57 2UW (GB)**
- **TIMMIS, Kenneth, N.**
  **38300 Wolfenbüttel (DE)**
- **CHERNIKOVA, Tatyana**
  **700007 Taschkent (UZ)**
- **WALICZEK, Agnes**
  **38100 Braunschweig (DE)**
- **FERRER, Manuel**
  **E-28007 Madrid (SE)**
- **BELOQUI, Ana**
  **E-28049 Madrid (ES)**
- **GUAZZARONI, Maria, E.**
  **E-28049 Madrid (ES)**
- **VIEITES, Jose, M.**
  **E-28049 Madrid (ES)**
- **PAZOS, Florencio**
  **E-28049 Madrid (ES)**
- **DE LACEY, Antonio, Lopez**
  **E-28049 Madrid (ES)**
- **FERNANDEZ, Victor, M.**
  **E-28049 Madrid (ES)**

(74) Representative: **Forstmeyer, Dietmar et al**
**Boeters & Lieck**
**Oberanger 32**
**80331 München (DE)**

(56) References cited:
**EP-A- 1 669 760** **WO-A1-97/28275**
**JP-A- 2004 093 478** **US-A1- 2002 160 526**
**US-A1- 2005 123 932** **US-A1- 2008 081 329**

- **KOSTIC N M ED - INGLESE JAMES: "TRANSITION-METAL COMPLEXES AS SPECTROSCOPIC PROBES FOR SELECTIVE COVALENT LABELING AND CROSS-LINKING OF PROTEINS" METHODS IN ENZYMOLOGY, ACADEMIC PRESS INC, SAN DIEGO, CA, US, vol. 226, 1 January 1993 (1993-01-01), pages 565-576, XP008063637 ISSN: 0076-6879**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 408 927 B1

## Description

### Background of the Invention

The present invention relates to a probe compound for detecting and isolating enzymes, to a method for producing the probe compound, to means for detecting enzymes and means for isolating enzymes, to a method for producing the means for detecting and isolating enzymes, and to a method for detecting and isolating enzymes using the same.

In more detail, the present invention relates to a probe compound that can comprise any substrate or metabolite of an enzymatic reaction in addition to an indicator component, such as, for example, a fluorescence dye, or the like. Moreover, the present invention relates to means for detecting enzymes in form of an array. The array according to the present invention comprises any number of probe compounds of the invention which each comprise a different metabolite of interconnected metabolites representing the central pathways in all forms of life. The probe compound and the array of the invention can be used for detecting specific enzyme-substrate interactions associated with the corresponding substrate(s) or metabolite(s), which allows to identify substrate-specific enzymatic activity in a sample. Moreover, the present invention relates to a method for detecting enzymes involving the application of cell extracts or the like to the array of the invention which leads to reproducible enzymatic reactions with the substrates. These specific enzymatic reactions trigger the indicator (e.g. a fluorescence signal) and bind the enzymes to the respective cognate substrates. Moreover, the invention relates to means for isolating enzymes in form of nanoparticles coated with the probe compound of the invention. The immobilisation of the cognate substrates or metabolites on the surface of nanoparticles by means of the probe compounds allows capturing and isolating the respective enzyme, e.g. for subsequent sequencing. In short, the probe compound of the invention provides two new aspects: first, that only an active protein (enzyme) triggers the indicator signal, and second, that the active protein subsequently binds to the probe compound.

In recent years, the new discipline of "functional genomics" has greatly accelerated the research on the genomic basis of life processes in health and disease, and has significantly improved our understanding of such processes, their regulation and underlying mechanisms. However, until relatively recently, functional genomics has been sequence-centric, that is, functional assignments and metabolic network reconstructions have mostly depended on the genome sequence of the organism in question, combined with bioinformatic analyses based on homologies to known gene/protein-relationships. In addition to the fact that a significant fraction of genes in databases available today has a questionable annotation, many are not annotated at all, which adds further uncertainties to analyses and predictions based on them. With the recent advent of "metabolomics", functional insights into the metabolic state of a cell became possible independently of sequence information. However, problems of metabolite identification and quantification still exist, and the link with the cognate metabolic pathways is still heavily dependent upon sequence-based metabolic reconstructions. Furthermore, it is currently very difficult to derive global metabolic overviews of non-sequenced organisms or communities of organisms existing in an individual habitat or biotop (biocoenosis).

Therefore, there exists an urgent need to solve the twin problems of the identification of metabolites and the enzymes involved in their transformation and, simultaneously, to begin the critical process of rigorous annotation of yet un-annotated and incorrectly annotated open reading frames (genes), and thereby improving the utility of the exponentially growing body of genome sequence information. Thus, an activity-based, annotation-independent procedure for the global assessment of cellular responses is urgently needed.

"Microarrays" or "biochips" have proven to be an important and indispensable tool for the fast gain and processing of information required in the field. Here, the term 'array' refers to a collection of a large number of different test compounds preferably arranged in a planar plane, e.g. by attachment on a flat surface, such as a glass slide surface, or by occupying special compartments or wells provided on a plate, such as a micro-titre plate. The test compounds, which are also referred to as probes, probe compounds or probe molecules, are usually bound or immobilised on the flat surface or to the walls of a compartment, respectively. The use of arrays allows for the rapid, simultaneous testing of all probe molecules with respect to their interaction with an analyte or a mixture of analytes in a sample. The analytes of the sample are often referred to as target molecules. The advantage of a planar array over a test (assay) having immobilised probe molecules on mobile elements, such as, for example, beads, is that in an array the chemical structure and/or the identity of the immobilised probe molecules is precisely defined by their location in the array surface. A specific local test signal, which is produced, for example, by an interaction between the probe molecule and the analyte molecule, can accordingly be immediately assigned to a type of molecule or to a probe molecule. As evidence of an interaction between a probe molecule and an analyte molecule, it is also possible to use the enzymatic conversion of the probe by the biomolecule, with the result that a local test signal can also disappear and accordingly serves as direct evidence. Particularly in miniaturised form, arrays having biological probe molecules are also known as "biochips".

Usually, the surface of the microarray having the bound probe molecules is brought into contact, over its entire area, with the solution of the analyte molecules from a sample. Then, the solution is usually removed after a predetermined incubation time. Alternatively, appropriate amounts of the sample solution are filled into the respective compartments

(wells) of the array. When the specific and selective interaction between the probe molecule and an analyte molecule is complete, a signal is generated at the location of the probe molecule. That signal can either be produced directly, for example by binding of a fluoresence-labelled biomolecule, or can be generated in further treatments with detection reagents, for example in the form of an optical or radioactive signal. Many different technical details relating to procedure and detection are well known and completely described in the art. There are numerous array protocols and processes which are adapted for automatic handling by corresponding (robotic) apparatuses, thus allowing for high reliability and reproducibility of information gain and processing.

Examples of known arrays in the prior art are nucleic acid arrays of DNA fragments, cDNAs, RNAs, PCR products, plasmids, bacteriophages and synthetic PNA oligomers, which are selected by means of hybridisation, with formation of a double-strand molecule, to give complementary nucleic acid analytes. In addition, protein arrays of antibodies, proteins expressed in cells or phage fusion proteins (phage display) play an important part. Furthermore, compound arrays of synthetic peptides, analogues thereof, such as peptoids, oligocarbamates or generally organic chemical compounds, are known, which are selected, for example, by means of binding to affinitive protein analytes or other analytes by means of enzymatic reaction. Moreover, arrays of chimaeras and conjugates of the said probe molecules have been described.

DNA microarray technology has a vast potential for improving the understanding of microbial systems. Microarray-based genomic technology is a powerful tool for viewing the expression of thousands of genes simultaneously in a single experiment. While this technology was initially designed for transcriptional profiling of a single species, its applications have been dramatically extended to environmental applications in recent years. The use of microarrays to profile metagenomic libraries may also offer an effective approach for characterizing many clones rapidly. As an example, a fosmid library was obtained and further arrayed on a glass slide. This format is referred to as a metagenome microarray (MGA). In the MGA format, the '*probe*' and '*target*' concept is a reversal of those of general cDNA and oligonucleotide microarrays: targets (fosmid clones) are spotted on a slide and a specific gene probe is labelled and used for hybridization. This format of microarray may offer an effective metagenome-screening approach for identifying clones from metagenome libraries rapidly without the need of laborious procedures for screening various target genes.

However, one of the greatest challenges in using microarrays for analyzing environmental samples is the low detection sensitivity of microarray-based hybridization in combination with the low biomass often present in samples from environmental settings. Microarrays for expression profiling can be divided into two broad categories, microarrays based on the deposition of preassembled DNA probes (cDNA microarrays) and those based on *in situ* synthesis of oligonucleotide probes (e.g. Affymetrix arrays, oligonucleotide microarrays). Applications employing DNA microarrays include, for example, the characterization of microbial communities from environmental samples such as soil and water. Various types of DNA microarrays have been applied to study the microbial diversity of various environments. Those include, for example, oligonucleotides, cDNA (PCR amplified DNA fragments), and whole genome DNA.

One of the major problems associated with nucleic acid-based micro-arrays is derived from the short half-life of mRNA, and that mRNA in bacteria and archaea usually comprise only a small fraction of total RNA. Moreover, the study of the gene expression from an environmental sample using DNA microarrays is a challenging task. First, the sensitivity may often be a part of the problem in PCR-based cDNA microarrays, since only genes from populations contributing to more than 5% of the community DNA can be detected. Second, samples often contain a variety of environmental contaminants that affects the quality of RNA and DNA hybridization and makes it difficult to extract undegraded mRNA. The specificity of the extraction method plays a central role and should vary depending on the site of sampling, as there must be sufficient discrimination between probes. However, there is a promising perspective for microarrays in determining the relative abundance of a microorganism bearing a specific functional gene in a complex environment.

However, specificity is a key issue, since one needs to distinguish the differences in hybridization signals due to population abundance from those due to sequence divergence. Furthermore, annotation and the comprehensive functional characterization of proteins or RNA molecules remain difficult, error-prone processes, but systems microbiology relies heavily on a thorough understanding of the functions of gene products.

At the moment, after DNA micro-arrays, the peptide arrays are the most popular. In this kind of arrays, peptides with different chemical composition are synthesised and immobilized on glass slides. The peptides may also contain a marker, such as a fluorescence dye marker (e.g. a fluorescent cyanine dye known under the name 'Cy3'), but here the detection method is only based on the lowered fluorescence obtained with a protein bound to the molecule. There is no enzymatic reaction necessary for the signal, so un-specific bindings may occur and trigger a signal, which may lead to incorrect assignments. Further, there is no possibility to reconstruct metabolic networks.

Another array alternative is to bind proteins to a slide. Such system is usually not based on the detection of a fluorescence signal, but rather on the utilization of surface Plasmon resonance. This system has been exploited for the analysis of molecular interactions, i.e. protein-protein or molecule-protein interactions.

In view of the problems encountered in the prior art, the present invention is therefore based on the object of providing a novel probe compound, which allows for the testing of a reactive interaction of an enzyme with a small

molecule or enzymatic substrate. The probe compound should allow for the easy linkage of all small molecules or substrates necessary for the life functions of an organism or communities living in a habitat (biocoenosis). Thus, a plurality of probe compounds should allow for the construction of a 'reactome array' or microarray which allows for the testing of all life supporting enzymatic reactions of an organism or community simultaneosly. Particularly, the probe compound should provide a highly sensitive, accurate, reproducible, and robust high-throughput tool for a genome-wide analysis of the metabolic status of an organism or community. In this context, the term "genome-wide analysis" means an analysis that is independent of genome sequence. Moreover, the probe compound should also allow for use in the isolation of an enzyme so that said enzyme may be further analysed or identified in a subsequent step. Moreover, the probe compound should also allow for the identification of small molecules, substrates and/or metabolites which are metabolised by an organism or community, thus allowing the identification of biologic pathways or the direct comparison of the reactomes of different organisms, which might be applied in the search for new targets for drug-screening.

JP2004093478 discloses a transition metal complex (see fig. 1, claim 1) that is based on a nickel or cobalt atom and nitrilotriacetic acid (see claim 2) that is linked via two histidine residues to a receptor protein (see fig. 1, A, here: human estrogen receptor, see fig. 4). A signal is produced with the help of an electrode.

EP1669760 discloses labelled transition metal complexes to be attached to biological entities such as DNA.

**Summary of the Invention**

The object of the invention is solved by a probe compound comprising a transition metal complex and a reactive component comprising a test component and an indicator component, wherein the test component and the indicator component are linked to form the reactive component, and wherein the reactive component is linked to the transition metal complex according to claim 1:

A probe compound tor detecting specific enzyme-substrate interactions comprising a transition metal complex and a reactive component of general formula (X):

$$\text{His-}L_{\text{His-Tc}}\text{-TC-}L_{\text{TCI-IC}}\text{-IC-}L_{\text{IC-His}}\text{-His} \qquad \text{formula (X)}$$

wherein His represents a histidine residue, TC represents a test component, IC represents an indicator component, and each of $L_{\text{HiS-TC}}$, $L_{\text{TC-IC}}$ and $L_{\text{IC-His}}$ independently represents optional linker components, wherein the reactive component is linked to the transition metal complex by the two histidine residues, wherein the test component comprises a substrate, a metabolite, a pseudo-substrate or an inhibitor of an enzyme, and wherein the indicator component comprises a dye.

The probe compound of the invention provides a means for testing of a reactive interaction of an enzyme with a small molecule or enzymatic substrate. The probe compound may be readily used in combination with all small molecules or substrates necessary for the life functions of an organism or communities living in a habitat (biocoenosis). Further, the probe compound provides a highly sensitive, accurate, reproducible, and robust high-throughput tool for a genome-wide analysis of the metabolic status of an organism or community. It allows the fast and reliable detection of a substrate specific enzyme interaction. Moreover, the probe compound may be readily used to detect the involvement of one enzymatic substrate in different metabolic pathways. Moreover, the probe compound provides a means to immobilise a substrate-specific enzyme, which can be advantageously used to isolate this enzyme from a sample.

A preferred embodiment of the probe compound of the invention can be illustrated by the following general formula (1) :

```
                    L_MC-TC  -  TC
                   /              \
AC - L_AC-MC - MC                  L_TC-IC        Formula (1)
                   \              /
                    L_MC-IC  -  IC
```

wherein AC represents an optional anchoring component, MC represents the transition metal complex, TC represents the test component, IC represents the indicator component, and $L_{\text{AC-MC}}$, $L_{\text{MC-TC}}$, $L_{\text{TC-IC}}$ and $L_{\text{MC-IC}}$ each independently represents an optional linker component between the respective components indicated by the subscripts, wherein $L_{\text{MC-TC}}$ and $L_{\text{MC-IC}}$ each independently comprise a histidine residue.

In more detail, the present invention relates to a probe compound that can comprise any substrate or metabolite of an enzymatic reaction in addition to an indicator component, such as, for example, a fluorescence dye, or the like. In short, the probe compound of the invention provides two new aspects: first, that only an active protein (enzyme) triggers the indicator signal, and second, that the active protein subsequently binds to the probe compound.

The object of the invention is also solved by a method for preparing the probe compound of the invention. The inventive method provides a versatile method for preparing all embodiments of the probe compound. Moreover, the method of the invention can be used for the identical and reproducible production of probe compounds comprising different enzymatic substrates, which allows for the ready use in automatic processes, such as parallel synthesis or the like.

The object is also solved by an array which comprises a plurality of different probe compounds of the invention. The array (which is sometimes referred to as "reactome array" in the following) can be used for the simultaneous detection of all reactive interactions between the probe compounds and analyte molecules (enzymes) from a sample. The array also provides a fast and reliable way to detect all metabolic pathways active in an organism or community, and may be used advantageously for an activity-based, annotation-independent procedure for the global assessment of cellular responses. The array can include a number of interconnected metabolites representing central pathways in all forms of life. The application of cell extracts to the array leads to reproducible enzymatic reactions with substrates that trigger the indicator signal and bind enzymes to cognate substrates.

The invention also provides a method for producing an array according to the invention, which allows for a versatile, fast and reproducible production of arrays according to the invention.

Moreover, the object is also solved by an isolation means comprising a probe compound according to the invention and a nanoparticle, preferably a magnetic nanoparticle. The isolation means according to the invention allows for the substrate specific interaction and binding of an enzyme which can then be isolated by means, such as, for example, filtration, gravitation force (centrifugation), an external magnetic force, or the like. The invention also provides a method for producing an isolation means according to the invention. The immobilisation of the cognate substrates or metabolites on the surface of nanoparticles by means of the probe compounds allows capturing and isolating the respective enzyme, e.g. for subsequent sequencing.

Moreover, the object is solved by a method for detecting enzymes using the probe compound according to the invention, or the array according to the invention, as well as by a method for isolating enzymes, using the isolation means according to the invention.

The particular subject-matter of the invention and its preferred embodiments will be described in more detail in the following description as well as in the examples and figures attached thereto.

**Brief Description of the Figures**

**Figure 1** shows a schematic overview of the array strategy, including a summary of the four major steps in the construction and analysis of arrays. *Steps* 1 and 2: extensive data and synthetic mining effort to produce a library of metabolites than can be arrayed on glass slides in a spatially-addressable manner; *steps* 3 nad 4: detection and analysis of enzymatic reactions following application of cell lysates to the array, and metabolic reconstructions.

**Figure 2** shows an overview of the array strategy, including a summary of the four major steps in the construction of metabolite complexes. (1) 2-step synthesis of bi-functional, non-fluorescent Cy3 dye component; (2) preparation of the His-tagged substrate (1-indanone is shown as a model substrate), (3) preparation of $Co^{2+}$ linker molecules; and (4) synthesis of Cy3-metabolite complexes containing an amine with a nitrogen-to-metabolite 'labile' bond proximal to the catalysis reaction site.

**Fig. 3** illustrates the reactome strategy. The generic structure of reactome metabolites involves three linked components, the enzyme substrate-metabolite, the quenched dye, and the linker used to immobilize the complex on the array or on nanoparticles. The substrate-metabolite is linked to the quenched dye though a labile nitrogen bond, and both the dye and the substrate are anchored to the Co(II)-containing poly (A) linker by histidine 'tags'. Details of the synthetic strategy are provided in Figures 2 and 8.
An enzyme-catalysed chemical change in the substrate at a position adjacent to the weakly amine region causes rupture of the labile nitrogen:metabolite bond, and release of the quenched Cy3 dye. This in turn provokes release of the reaction product and the histidine 'tags' anchored to the Co(II), thereby exposing an active cobalt cation which ligates and immobilizes the enzyme on the array spot. The released dye is no longer quenched and gives a fluorescent signal. The nature of the reaction and the catalysis product is defined by the position to which the quenched dye and the substrate are linked (see table 2).

**Figure 4** shows Dose-response curves determined with pure *E. coli* β-Gal and Cy3-linked X-Gal. **(A)** Substrate dose response with fixed amount of β-Gal (5 ng/ml); **(B)** β-Gal dose response with fixed amount of Cy3-modified X-Gal (2.52 pmol/ml). For each experiment, normalized intensity values and fit curves were scaled relative to the maximum asymptotic values of the fit.

**Figure 5** shows the Receiver Operating Characteristic (ROC) curve of the array. The ROC shows the capacity of the array to discriminate compounds potentially metabolised by *P. putida* from those which are not metabolised. The "true positive rate" (TRP) is plotted on the Y-axis against the "false positive rate" (FRP) on the X-axis. The diagonal line represents the discriminative power of a random method.

**Figure 6** shows an overall comparison of metabolites transformed by lysates of the three communities KOL, VUL and L'A. **(A)** Pairwise comparisons of the compounds metabolized by lysates of the KOL, VUL and L'A metagenome libraries. **(B)** Overall comparison of compounds metabolized by the three libraries. (C) Pairwise comparisons of the compounds metabolized by lysates of the individual metagenome libraries and that of *P. putida.*

**Figure 7** shows dose-response curves determined with purified *P. putida* KT2440 proteins (A) and metagenomic proteins (B). Left and right figures represent protein and molecule dose responses. Results shown are the average of three independent assays, and were corrected for background signal. Results are not fitted to any model. The spotting process was carried out using a MicroGrid II micro-arrayer (Biorobotics) by spotting 0.25 nL droplets of SMs-Cy3 solutions (spot size 400 μm diameter with concentrations ranging from 0 to 0.25 pmol/ml) and further arrayed with 60 μl of a solution of pure enzyme (from 16 to 90 ng/ml in PBS buffer, depending on the enzyme used) (left column) or by spotting 0.25 nL droplets of SMs-Cy3 solution (spot size 400 μm diameter with concentration of 0.4 pmol/ml) and further arrayed with 60 μl of solution of pure enzyme at different concentrations (from 0 to 6000 μg/ml in PBS buffer). Signals were analyzed and quantified using GenePix pro 4.1 software (Axon). As shown, Cy3 fluorescence emission increased with increasing the amount of both protein and substrate, whereas inactive proteins did not (see below). (C) FTIR spectrum of L'A62 hydrogenase. Inset shows the $H_2$-uptake activity using methyl viologen as acceptor.

**Figure 8** summarizes the major steps used for the construction of metabolite complexes corresponding to the 26 different synthetic methods. Abbreviations used are as follows: 1,8-BDN (1,8-bis-(dimethylamino)-naphthalene); REBr (hybrid halogenase/dehalogenase; MeOH (methanol), (E) compound (Cy3 intermediate containing histidine and linkers).

**Figure 9** shows representative molecules of the different synthetic methods used to link metabolites with histidine and the dye molecule.

**Detailed Description of the Invention**

The present invention provides a probe compound comprising a transition metal complex and a reactive component comprising a test component and an indicator component, wherein the test component and the indicator component are linked to form the reactive component, and wherein the reactive component is linked to the transition metal complex according to claim 1:

A probe compound tor detecting specific enzyme-substrate interactions comprising a transition metal complex and a reactive component of general formula (X):

$$\text{His-}L_{\text{His-Tc}}\text{-TC-}L_{\text{TCI-IC}}\text{-IC-}L_{\text{IC-His}}\text{-His} \qquad \text{formula (X)}$$

wherein His represents a histidine residue, TC represents a test component, IC represents an indicator component, and each of $L_{HiS-TC}$, $L_{TC-IC}$ and $L_{IC-His}$ independently represents optional linker components, wherein the reactive component is linked to the transition metal complex by the two histidine residues, wherein the test component comprises a substrate, a metabolite, a pseudo-substrate or an inhibitor of an enzyme, and wherein the indicator component comprises a dye.

The probe compound of the invention thus comprises the following components: a transition metal complex and a reactive component. The reactive component in turn comprises the following components: a test component and an indicator

component, which are linked to form the reactive component. Further, the reactive component is linked to the transition metal complex by histidine residues.

A preferred embodiment of the probe compound of the invention can be illustrated by the following general formula (1) :

```
                      LMC-TC  -  TC
                     /             \
AC  -  LAC-MC  -  MC                LTC-IC          Formula (1)
                     \             /
                      LMC-IC  -  IC
```

wherein AC represents an optional anchoring component, MC represents the transition metal complex, TC represents the test component, IC represents the indicator component, and $L_{AC\text{-}MC}$, $L_{MC\text{-}TC}$, $L_{TC\text{-}IC}$ and $L_{MC\text{-}IC}$ each independently represents an optional linker moiety between the respective components indicated by the subscripts.

The probe compound for detecting specific enzyme-substrate interactions is defined according to claim 1:

> A probe compound tor detecting specific enzyme-substrate interactions comprising a transition metal complex and a reactive component of general formula (X):
>
> $$\text{His-}L_{\text{His-Tc}}\text{-TC-}L_{\text{TCl-IC}}\text{-IC-}L_{\text{IC-His}}\text{-His} \qquad \text{formula (X)}$$
>
> wherein His represents a histidine residue, TC represents a test component, IC represents an indicator component, and each of $L_{\text{HiS-TC}}$, $L_{\text{TC-IC}}$ and $L_{\text{IC-His}}$ independently represents optional linker components, wherein the reactive component is linked to the transition metal complex by the two histidine residues, wherein the test component comprises a substrate, a metabolite, a pseudo-substrate or an inhibitor of an enzyme, and wherein the indicator component comprises a dye.

Herein, the term "linked" means that two components are connected with each other by means of at least one chemical bond, preferably by one, two, or three chemical bonds, and most preferred by exactly one chemical bond. A chemical bond indicates any chemical bond known in the art, such as, for example, an ionic bond, a covalent bond, including a single bond, a double bond, a triple bond, or another suitable multiple bond, and a hydrogen bond, and the like. Preferably, the chemical bond between the individual components is a covalent single bond. Preferably, the "link" or chemical bond between two components is a direct bond between the two components; but it may also comprise a suitable linker atom or molecule, if necessary. In some cases, one or both of the components to be linked will have to be activated in order to allow for the formation of a chemical bond or link. The procedures and means to be applied for such activation and formation of chemical bonds are standard knowledge of the field, and any skilled person will immediately know how to proceed.

Since the individual components are required to be linked (or chemically bonded) to each other as specified, the term "component" is used to indicate the respective parts of the probe compound which are characterised by their respective function as determined in the following. Herein, the term "component" is meant to refer to a specified part or moiety of a molecule, which is identified by its function within this molecule. For example, the term "indicator component" refers to a part or moiety of the molecule, which comprises a signal-generating function, e.g. a fluorescence dye or the like, that allows for indicating the location of the probe compound. Similarly, the term "test component" refers to a part or moiety of the molecule, which comprises a substrate or metabolite, thus being the site of enzymatic interaction with the probe compound. The respective components are linked by chemical bonds to form the molecule, i.e. the probe compound. It should be noted that, in the context of this invention, the term "component" is used with the meaning of "part" or "moiety" of one compound or molecule, and not to refer to individual members of a multi-molecule system. The terms "component", "part" and "moiety" may be used alternatively with this meaning. However, for reasons of consistency, the term "component" will also be used to refer to the respective molecules before they are reacted to form the respective part (component) of the probe compound, or after they are released from the probe compound,' respectively. A person skilled in the art will readily understand the exact nature of the respective molecules, as well as their contribution to the probe compound.

The term "linked" indicates the presence of at least one chemical bond between the corresponding components. A chemical bond can be a hydrogen bond, an ionic bond or a covalent bond, including a bond between a transition metal atom and its surrounding ligand atoms in a coordination compound (also referred to as "coordination bond" in the

following). Preferably, the chemical bond(s) between the components of the probe molecule are covalent bonds, further preferred covalent single bonds, wherein the bond(s) between the reactive component and the transition metal complex preferably are coordination bonds. The probe compound of the invention is required to have bonds between the transition metal complex and the reactive component, as well as between the test component and the indicator component forming the reactive component. However, the respective components may also be linked by additional bonds, as long as such bonds do not hinder the function of the probe compound. For example, the reactive component may be linked to the transition metal complex by at least one coordination bond formed between the test component moiety of the reactive component and the central metal atom of the transition metal complex and by at least one coordination bond formed between the indicator component moiety of the reactive component and the central metal atom of the transition metal complex. In a preferred embodiment, the reactive component is linked to the transition metal complex by exactly one coordination bond formed between the test component moiety of the reactive component and the central metal atom of the transition metal complex and by exactly one coordination bond formed between the indicator component moiety of the reactive component and the central metal atom of the transition metal complex. The respective coordination bonds can be direct bonds between the metal atom and a suitable atom of the test or indicator components, or a bond formed via a suitable linker moiety, which is linked to the test and/or indicator component, respectively. A preferred linker moiety is a histidine residue. By forming links via a linker moiety, it is possible to obtain a reproducible binding property for all test and indicator components.

The term "test component" is used for the part or component of the probe compound which comprises a substrate or metabolite. Herein, the terms "substrate" and "metobolite" refer to any molecule capable of specific interaction with the active site of an enzyme. The substrate or metabolite is comprised in the test component in such a manner that its characteristic structure and/or functional groups necessary for interaction with the active site of an enzyme are maintained within the test component. Therefore, the substrate or metabolite is preferably linked to the other components of the probe compound at positions of the substrate or metabolite molecule, which are not involved in enzyme-substrate interaction. Optionally, a spacer or linker moiety can be used to provide a suitable binding position on the the test component, which allows for an unimpeded enzyme interaction. In this case, the term "test component" refers to the component comprising both the substrate and the spacer moiety. The test component may be linked to the probe compound by chemical bonds involving positions, i.e. atoms or functional groups, of the substrate or metabolite and/or an optional spacer or linker moiety.

The term "test component" is preferably used to indicate a component comprising a so-called "small organic molecule" that can interact with an enzyme. The term "small organic molecule" refers to a molecule comprising of carbon and hydrogen atoms, optionally including nitrogen, oxygen, phosphorous, sulfur, and/or halogen (F, Cl, Br, I) atoms, and having a molecular weight of 5000 Da or less, preferably of 2000 Da or less. Preferably, the test component comprises a known substrate of at least one enzyme. Moreover, the test component can also comprise a pseudo-substrate or inhibitor of a known enzyme. However, the test component may also comprise a molecule suspected to interact with the active site of an enzyme. The test component may also comprise a small organic molecules for the search for pharmaceutical active ingredients. According to one embodiment, the "test component" does not comprise a polymeric compound based on nucleic acids, such as DNA, cDNA, RNA, or the like, or a polymeric compound based on amino acids, such as peptides, proteins, or the like. According to another embodiment, the test component may comprise at least one nucleic acid and/or amino acid, if necessary. Preferably, the test component comprises one or two nucleic acids, or one or two amino acids. According to another embodiment, the test component comprises a polymeric compound, such as a polymeric compound based on natural occurring sugar units or the like, e.g. cellulose or the like. Preferable, a polymeric compound has a molecular weight of 5000 Da or less, i.e. 5 kDa or less. According to another embodiment, the test component comprises a polymeric compound based on nucleic acids, such as DNA, cDNA, RNA, or the like, and/or a polymeric compound based on amino acids, such as peptides, proteins, or the like. Moreover, the test component can be preferably functionalised with a linker component or moiety suitable for binding to the transition metal complex. Such functionalisation is especially advantageous for test components comprising substrates, which do not readily form coordination bonds. In a preferred embodiment, the test component is functionalised with a histidine molecule or residue (sometimes referred to as a "His-tag" in the following). The amino acid histidine was found to be especially versatile, because it may be linked to a test component or indicator component by either its amine function or its carboxylic acid function, while the imidazole ring provides for a good coordination bond to the transition metal atom. A histidine residue may be linked to the substrate or metabolite comprised in the test component or to another part of the test component, either directly or using a suitable linker moiety. Similarly, a histidine residue may be linked to the dye comprised in the indicator component or to another part of the indicator component, either directly or using a suitable linker moiety. A skilled person will know how to link a histidine residue to the desired site or position of a test or indicator component, whether a special activation and/or linker moiety will be required, as well as the starting materials and conditions necessary therefor, etc. A His-tag has the additional advantage to ensure an identical binding property of all possible substrates to the transition metal complex. Moreover, it was found that a link including a His-tag may be advantageously broken upon enzymatic reaction of a test component comprising a His-tag. Moreover, it was found that

by selecting the site of histidine binding to the test component, it is possible to prepare probe compounds which allow for the identification of different metabolic pathways and the enzymes involved therein.

The term "indicator component" is used to indicate a molecule which can generate a signal, thus indicating the location of the probe molecule, e.g. on an array. That signal can either be produced directly, for example by adsorbance or fluorescence, or can be generated in further treatments with detection reagents, for example in the form of an optical or radioactive signal. Preferably, the indicator component comprises a dye, further preferred a fluorescence dye. The term "fluorescence dye" indicates a molecule showing fluorescence upon irradiation with a suitable light source. Preferably, an indicator component comprises a fluorescent azo compound or a cyanine compound, or the like. Especially preferred is a cyanine compound, which is known in the art under the name of "Cy3". If necessary, the moiety having the fluorescence property is further modified, e.g. by addition of a suitable linker moiety, in order to allow the binding to the test component, and/or to the transition metal complex. For example, a Cy3 dye available as its Cy3-NHS-ester may be reacted with both histidine and a 4-amino-3-butyric acid linker to allow for linking with both the transition metal complex and the test component, respectively. An additional linker moiety has the advantage to ensure an identical and reproducible binding to the indicator component and/or the transition metal complex, which allows for the ready use in parallel synthesis or the like.

The test component is linked to the indicator component to form the reactive component. Preferably, the test component is linked to the indicator component by at least one covalent chemical bond, further preferred by one, two, or three covalent bonds, and still further preferred by exactly one covalent bond. A preferred example of such a covalent bond is a carbon-oxygen single bond, which can be formed between the test component and the indicator component by various chemical reactions, such as, for example, a condensation reaction, an addition reaction, an oxidation reaction, and the like. A preferred example is a condensation reaction between a carboxylic acid and an alcohol, or between two alcohols, or an addition reaction wherein the oxygen atom of an alcohol function is added to an aliphatic or aromatic carbon atom, or the like. It should be noted that such bond forming reaction is not restricted to the examples given above, and that there is no prejudice regarding which individual function should be present in the respective molecules to become the respective components, etc. A person skilled in the art will immediately know which combinations of functional groups will be required to form a corresponding chemical bond between the individual molecules to become the respective components of the probe compound, and also which starting materials and conditions etc. are required to form the desired chemical bond between the components. Another preferred covalent bond is a carbon-nitrogen single bond. Preferably, the carbon-nitrogen single bond is part of a quaternary amine function (quaternary ammonium function) comprised in the link between test component and indicator component. The link or bond between the test component and the indicator component may also be formed between the test component and a linker moiety previously attached to the indicator component, or *vice versa.* An additional linker moiety has the advantage to ensure an identical and reproducible binding to the test component, which allows for the ready use in parallel synthesis or the like. In a preferred embodiment, the indicator component comprises an amino butyric acid linker moiety, and a bond or link to the test component is formed using the carboxylic acid function of this linker moiety. Preferably, the link between indicator component and test component comprises a linker moiety comprising an amino butyric acid linker residue attached to the indicator component, the carboxylic acid function of which is linked to another linker moiety comprising a quaternary amine function, which in turn is linked to the test component. Such linker moiety can be formed, for example, by reacting an indicator component, e.g. a fluorescence dye, first with 4-amino-3-butanoate and then with N,N-dimethylethanolamine. Linking with the test component under formation of a quaternary amine can then be obtained by reacting the so-prepared indicator component having a linker moiety comprising an amino butyric acid residue, the carboxylic acid function of which is esterised by N,N-dimethylethanolamine, with a iodine-containing test component in the presence of 1,8-bis-(dimethylamino)-naphthalene, or a similar method.

The reactive component comprising the test component and the indicator component, which are linked to each other, optionally by a linker moiety or molecule, is in turn linked to the transition metal complex. Preferably, the reactive component is linked to the transition metal complex by two, three or four coordination bonds, and further preferred by exactly two coordination bonds. That means that at least one atom of the reactive component is a direct ligand atom of the transition metal atom of the transition metal complex, thus being part of the immediate coordination sphere of the central transition metal atom of the transition metal complex. The term "at least one atom of the reactive component" also includes an atom of a linker moiety, which can optionally be attached to either the test component or the indicator component. For example, in a preferred embodiment, a histidine molecule is attached as a linker moiety (His-tag) to the reactive component. In this case,’ the "at least one atom of the reactive component" can also indicate an atom of the His-tag, preferably a nitrogen atom of the imidazole ring system. Preferably, the reactive component is linked to the transition metal complex by two coordination bonds, wherein one ligand atom is an atom of the test component and the other ligand atom is an atom of the indicator component. In a preferred embodiment, the reactive component comprises a His-tag linked to the test component and a His-tag linked to the indicator component. In this case, the reactive component is linked to the transition metal complex by two coordination bonds, wherein each bond includes one atom of one of the respective Histags.

A preferred coordination bond between the reactive component and the transition metal complex is a M-O-R-bond, wherein M indicates the transition metal atom and O-R indicates an oxygen atom or function of a molecule R constituting the reactive component. Preferred examples of suitable oxygen functions are an alcoholate function ($R-O^-$), a carboxylate function ($RCOO^-$), a peroxide function ($R-O-O^-$), or the like. Another preferred coordination bond between the reactive component and the transition metal complex is a M-N-R-bond, wherein M indicates the transition metal atom and N-R indicates a nitrogen atom or function of a molecule R constituting the reactive component. An example for such nitrogen function is an aliphaptic amine function, including a primary, secundary and tertiary amine function. The nitrogen atom can also be part of an aromatic or (partially) saturated ring system, such as, for example, a pyrrol, imidazole, diazole or triazole ring, or the like. A specially preferred coordination bond is a coordination bond comprising a nitrogen atom of an imidazole ring, which may be part of a histidine moiety or His-tag. Another preferred coordination bond is a M-S-R or a M-C-R single bond, wherein M indicates the transition metal atom and S and C, respectively, indicate a sulphur or carbon function of a molecule R constituting the reactive component. It should be noted that the coordination bond between reactive component and transition metal complex is not restricted to the examples given above, and that there is no prejudice regarding which individual function should be present in the respective molecules to form the desired coordination bond, etc. A person skilled in the art will immediately know which functional groups will be required to form a corresponding coordination bond, and also which starting materials and conditions etc. are required.

The transition metal complex preferably comprises at least one transition metal atom and at least one ligand molecule, wherein at least one coordination bond is formed between the ligand molecule and the transition metal atom. The at least one ligand molecule preferably comprises one or more atoms or funtions which can form a coordination bond with a transition metal atom. Preferred examples for atoms or function are an oxygen atom, a nitrogen atom, a phosphorous atom, a sulphur atom, or the like. These atom or functions all can form a coordination bond with a transition metal atom, and are the same as exemplified above. Preferably, a ligand molecule comprises more than one atom or function that can form a coordination bond with a transition metal atom, further preferred two to eight of such atoms or functions, still further preferred three to five of such atoms or functions, and most preferred four or five of such atoms of functions. In a preferred embodiment of the present invention, the transition metal complex comprises exactly one transition metal atom and exactly one ligand molecule comprising more than one atom or function that can form a coordination bond with a transition metal atom. Herein, the term "transition metal atom" is used to indicate the central transition metal atom of the transition metal complex, which is linked to both the ligand molecule(s) and the reactive component by coordination bonds as defined above. Examples of suitable transition metal atoms are Ti, V, Mn, Fe, Co, Ni, Cu, Zn, Mo, W, Pt, Au, or the like. Preferred examples are Co, Ni, and Cu. The transition metal atom means any transition metal atom irrespective of its oxidation state. The term transition metal ion is also used for a transition metal atom that carries a net charge, which is sometimes referred to as a "transition metal ion" in the art, wherein a formal charge or oxidation state is assigned to the transition metal atom or ion. Preferred oxidation states of transition metal atoms of the present invention are from 0 to +4, preferably +2 to +3, and especially preferred +2. Preferred examples of transition metal atoms are Co(II), Ni(II), and Cu(II). Accordingly, the coordinating atoms or functions of the ligand molecule may be assigned with a formal charge, wherein preferred charges range from 0 to -2, wherein a charge of 0 or -1 is especially preferred. As used herein, the term "ligand molecule" refers to a molecule that comprises at least one atom or function which forms a direct coordination bond to the central atom of a transition metal complex, preferably one, two, three, four, five, six, seven, or eight such atoms or functions. It should be noted that the coordination bond between ligand molecule and transition metal atom is not restricted to the examples given above, and that there is no prejudice regarding which individual function should be present in the ligand molecule to form the desired coordination bond, etc. A person skilled in the art will immediately know which functional groups will be required to form a corresponding coordination bond, and also which starting materials and conditions etc. are required.

The term "probe compound" indicates a compound or molecule that can interact with an enzyme (or analyte molecule) in a reactive manner, as outlined in the following. The term "interaction with an enzyme in a reactive manner" indicates an interaction wherein the reactive component is not only bound to the enzyme, but also transformed in a reaction catalysed by the enzyme (metabolised). The reaction catalysed by the enzyme can be any reaction catalysed by an enzyme, such as, for example, an oxidation or reduction reaction, an addition reaction, a hydrolytic bond cleaving reaction, an elimination reaction, an isomerisation reaction, or a condensation reaction.

Preferably, the reaction catalysed by the enzyme is a hydrolytic cleaving reaction or an elimination reaction, wherein the enzyme cleaves the link between the test component, or its reaction product, respectively, and the indicator component and/or the transition metal complex.

Preferably, the interaction with the enzyme results in a cleavage of the link between the test component and the transition metal complex by the enzyme, whereupon a reaction product comprising the metabolised test component and the indicator component together is formed. Further, the interaction with the enzyme may also result in a cleavage of both the link between the test component and the transition metal complex and the link between the test component and the indicator component by the enzyme, whereupon more than one reaction products comprising the metabolised test component and/or the indicator component in separate molecules, may be released from the probe compound. For

example, in a preferred embodiment wherein the test component is linked to the transition metal complex by a histidine moiety (His-tag), the link between the histidine is cleaved by the enzymatic reaction. As a result, the reaction product comprising both the test component and the indicator component remains linked to the transition metal complex, and thus to the remainder of the probe compound. The test component or the reaction product thereof may remain bound to the active site of the enzyme, thus immobilising the enzyme to the probe compound, or its reaction product, respectively. Moreover, the signal characteristic of the indicator component, which is triggered by the enzymatic reaction, also remains at the probe compound.

Alternatively, the interaction with the enzyme results in a cleavage of the link between the indicator component and the test component by the enzyme, resulting in a reaction product comprising the indicator component alone, or a reaction product comprising the metabolised test component and the indicator component together, or more than one reaction products comprising the metabolised test component and/or the indicator component in separate molecules. Here, the term "reaction product comprising the indicator component (or test component)" indicates a compound or molecule based on the indicator component or test component, respectively, which is metabolised and/or released by the corresponding reaction catalysed by the enzyme.

Alternatively, the interaction with the enzyme results in a cleavage of the both the link between the test component and the transition metal complex and the link between the test component and the indicator component by the enzyme, whereupon a reaction product comprising the metabolised test component, or more than one reaction products comprising the metabolised test component and/or the indicator component in separate molecules, may be released from the probe compound. For example, in a preferred embodiment wherein the test component is linked to the transition metal complex by a histidine moiety (His-tag), the link between the histidine is cleaved by the enzymatic reaction, and the link to the indicator component is cleaved as well.

Preferably, the reaction product comprising the indicator component is not released from the probe compound, i.e. the reaction product comprising the indicator component remains part of the probe compound, or its reaction product, respectively. For example, in a preferred embodiment wherein the indicator component is linked to the transition metal complex by a histidine moiety (His-tag), the histidine remains linked to both the transition metal complex and the indicator component upon enzymatic cleavage of the link between the test component and the indicator component. As a result, the reaction product comprising the indicator component remains linked to the transition metal complex, and thus to the remainder of the probe compound. Thereby, the signal characteristic of the indicator component, which is triggered by the enzymatic reaction, also remains at the probe compound.

Preferably, the reaction product comprising the test component is not released from the probe compound. For example, in a preferred embodiment wherein the test component is linked to the transition metal complex by a histidine moiety (His-tag), only the link between the histidine and the test component is cleaved by the enzyme. As a result, the reaction product comprising both the test component and the indicator component remains linked to the transition metal complex, and thus to the remainder of the probe compound. Thereby, the test component or the reaction product thereof may remain bound to the active site of the enzyme, thus immobilising the enzyme to the probe compound, or its reaction product, respectively. Moreover, the signal characteristic of the indicator component, which is triggered by the enzymatic reaction, also remains at the probe compound.

Preferably, the interaction of the probe compound with an enzyme results in the cleavage of both the links between transition metal complex and the reactive component as well as the link between the test component and the indicator component. This in turn exposes the transition metal atom which then ligates the enzyme, which can be used to immobilize the enzyme on an array spot or the like. The released indicator component has a changed binding situation which may preferably result in the generation of a signal.

The exact nature of all reaction products has not been revealed yet, but it is observed that an enzyme-specific reaction of the probe compound of the invention results in binding of the enzyme to the transition metal complex and the generation of a signal by the indicator component, e.g. the generation of a fluorescence signal.

It was found that the probe compound of the present invention allows for the detection of enzyme concentrations which are as low as 1.5 ng/ml protein or 2.5 pmol/ml substrate, respectively.

It was found that the probe compound of the invention advantageously allows for the testing of a reactive interaction of an enzyme with a small molecule or enzymatic substrate. The presence of the central transition metal complex further allows to provide a probe compound wherein all small molecules or substrates necessary for the life functions of an organism or communities living in a habitat can be readily included. Information about substrates that are involved in one or more metabolic reactions and about the enzymes involved in the corresponding metabolic reactions may be found, for example, in the Kyoto Encyclopedia of Genes and Genomes (KEGG Database), the University of Minnesota Biocatalysis and Biodegration Database (UM-BBD), PubMed, or the like. The probe compound of the invention was shown to be highly versatile for the identification of the reactive interaction with any small molecule or substrate tested so far. The key characteristic of the probe compound is that a productive reaction with a cognate enzyme releases the indicator component, producing a detectable signal, e.g. a fluorescent signal, and simultaneously results in the capture of the reacting enzyme through coordination with the transition metal complex. Non-productive interactions of

proteins with the probe compound, such as, for example, binding without chemical reaction, do not lead to the release of the indicator component and the associated production of a detectable signal. An example of this reactional behaviour is shown in Figure 3. The probe compound provides a highly sensitive, accurate, reproducible, and robust high-throughput tool for a genome-wide analysis of the metabolic status of an organism or community. Advantageously, the complete reactome (i.e. the complement of metabolic reactions of an organism) can be provided without prior knowledge of its sequence in as little time as 30 minutes or less.

Preferably, the transition metal complex comprises a cobalt or copper atom, and most preferred a cobalt atom. It was found that a cobalt or copper complex shows an advantageous binding property to all reactive components of interest, thus allowing for a most versatile use of the probe component of the invention. Especially preferred is a cobalt complex wherein the central cobalt atom is assigned a formal oxidation state of +2.

Preferably, the transition metal complex comprises a multidentate ligand molecule, i.e. a ligand molecule comprising two or more ligand atoms bound to the central transition metal atom. Further preferred, the transition metal complex comprises a multidentate ligand molecule comprising two, three, four, five, or six ligand atoms bound to the central transition metal atom. Especially preferred, the transition metal complex comprises a multidentate ligand molecule whose coordinating ligand atoms do not occupy all possible coordination positions of the central transition metal atom. For example, in the case of a central cobalt atom ($Co^{2+}$), which usually exhibits coordination numbers of five or six, the multidentate ligand may occupy five, four or three of the potential coordination (binding) positions.

Thus, a preferred ligand molecule for a central cobalt atom should provide three, four, or five coordinating ligand atoms, especially preferred four coordinating ligand atoms. A preferred example for a ligand molecule is a molecule comprising at least one coordinating nitrogen atom, such as, for example, a nitrogen atom of an aliphatic amine function, and at least one coordinating oxygen atom, such as, for example, an oxygen of a carboxilic acid function. A preferred example for a ligand molecule comprises nitrotriacetic acid. For example, in the case of a central cobalt atom, a ligand molecule based on nitrotriacetic acid will occupy four of the five or six potential coordination or binding positions, thus leaving one or two free binding positions for binding of the reactive component. However, a person skilled in the art will readily know which other ligand molecules exhibit similar properties and thus can also be used in the present invention.

Preferably, the transition metal complex comprises an anchoring component. The term "anchoring component" indicates a molecule or function that can be used to attach the probe compound to a solid surface, or to another component or molecule, or the like. Thus, the transition metal complex, and thereby the whole probe compound, can be attached to any suitable solid surface, or component or molecule, known in the art. Suitable solid surfaces may be porous surfaces, such as, for example, paper or cellulose substrates or the like, or non-porous surfaces, such as, for example, glass surfaces, or surfaces of polymeric materials, such as a surface of polycarbonate, or the like. For example, the anchoring component can be used to attach the probe compound to the surface of a glass slide in order to form an array thereon. Alternatively, the anchoring component can be used to attach the reactive compound to another component, such as, for example, a nanoparticle. The anchoring component may be any molecule or function known in the art which has the desired function to allow for the attachment to a solid surface, another component or molecule, or the like. A person skilled in the art will readily know which molecule or function should be used for a desired attachment. Preferably, the anchoring component comprises a polymeric compound, further preferred a polymeric compound of a biomolecule. A specially preferred anchoring component comprises a poly A chain, i.e. a polymer of adenosin. Any poly A chain known in the art may be used. Preferably, the poly A chain has a molecular weight of from 10 kDa to 150 kDa, further preferred of from 50 kDa to 120 kDa, and most preferred of about 100 kDa. A poly A chain is known in the art for binding to glass surfaces, activated silica, or the like. A poly A chain can be easily attached to a glass surface or the like, which might be optionally activated, and polymerised thereon using UV radiation according to standard protocols.

In another preferred embodiment of the anchoring component comprises at least one thiol function, which allows for the attachment to metal clusters, such as, for example, gold nanoparticles. However, another function which is known to bind to a desired metal cluster or nanoparticle can also be used. A compound which has a carboxylate group or a phosphate group in one moiety and a thiol group in the other moiety is preferably used. A preferred example for an anchoring component comprising thiol functions is an $\alpha$-dihydrolipoic acid residue (or a 6,8-dithioctic acid residue, TA). Depending on the nature of the surface of the metal cluster or nanoparticle to be used, i.e. either depending on the nature of the metal surface itself, or depending on the nature of a first activation or coordination layer, a skilled person will know which anchoring compound should to use to form an anchoring link in the sense of the invention.

Optionally, the anchoring component is linked to the transition metal complex by a suitable spacer component or moiety, such as, for example, an aliphatic hydrocarbon chain having at least one suitable functional group(s) for linking, such as, for example, a pentyl moiety having an amino group, or the like.

The anchoring component is directly linked to the transition metal complex. Preferably, the anchoring component is linked to the transition metal complex by a covalent bond, which is formed between an atom of the anchoring component and the ligand molecule of the transition metal complex. The anchoring component can also be linked to the transition metal complex by a coordination bond, which is formed between an atom of the anchoring component and the central transition metal atom of the transition metal complex. Especially preferred, the multidentate ligand of the transition metal

complex comprises the anchoring component.

In a preferred embodiment of the probe compound of the present invention, the transition metal complex comprises a nitrotriacetic acid Co(II) complex. It was found that the presence of this transition metal complex allows for the most versatile adaptation of the probe compound for the identification of the reactive interaction with any small molecule or substrate tested so far. A preferred transition metal complex comprising an anchoring component is provided by using the compound $N_\alpha$,$N_\alpha$-bis-(carboxymethyl)-L-lysine, or (S)-N-(5-amino-1-carboxypentyl)-imino-diacetic acid, respectively, as a ligand molecule for forming a cobalt(II) complex. In the so-formed cobalt complex, the aminopentyl residue, which is attached to one of the acetic acid groups of the ligand molecule, constitutes an anchoring component or a spacer moiety for attaching another anchoring component. The amine function of this anchoring component itself can be readily used to attach the transition metal complex, and thus the whole probe compound, to another component or molecule, such as, for example, a nanoparticle. Moreover, the amine function of this anchoring component can also be used to further attach a polymeric chain, such as, for example, a poly A chain, which can be used to attach the transition metal complex, and thus the whole probe compound, to a solid surface, such as, for example, a glass slide, in order to advantageously manufacture an array comprising corresponding probe compounds.

Thus, the term "probe compound" indicates a compound or molecule that can be immobilised on a supporting base by the anchoring component.

Preferably, the indicator component comprises a fluorescence dye, further preferred a fluorescent azo compound or a cyanine compound, examples of which are known in the art under the names of "Cy3" or "Cy5" or the like. By using this well-established dyes, it is possible to use the corresponding hardware available commercially, such as, for example, wave-length optimised reader apparatuses, corresponding software solutions, and the like. However, a person skilled in the art will understand that the present invention can easily be adopted to other indicator components and/or detection systems, if required. A person skilled in the art will also know how to carry out such adaptation.

Preferably, the test component comprises a known substrate, a metabolite, a pseudo-substrate, or an inhibitor of an enzyme. Further preferred, the test component comprises a molecule identified as a substrate of at least one metabolic reaction in the Kyoto Encyclopedia of Genes and Genomes (KEGG Database), the University of Minnesota Biocatalysis and Biodegration Database (UM-BBD), PubMed, or the like. Further preferred, the test component comprises a compound selected from the group listed in Table 1, 2 and 3, respectively.

By using such test components, it is possible to prepare probe compounds according to the invention which collectively form most of the central metabolic networks of cellular systems. However, the test component may also comprise additional metabolites characteristic of microbial metabolic activities not yet assigned or included in these databases, as well as

In a specially preferred embodiment of the probe compound of the present invention, the transition metal complex is represented by the $N_\alpha$,$N_\alpha$-bis-(carboxymethyl)-L-lysine cobalt(II) complex, which may further comprise an anchoring component comprising a poly A chain or a 6,8-dithioctic acid (TA) linked to the free amine function of the ligand molecule. In this embodiment, an indicator component comprises a Cy3 fluorescent dye, which comprises a histidine moiety (His-tag) for linking to the transition metal complex and a aminobutyric acid moiety as an optional linker moiety for linking to the test component. The test component may comprise any enzymatic substrate or other suitable small organic molecule, for example, one of the substrates listed in Table 1. The test component is linked to the indicator component via the optional aminobutyric acid linker moiety and further comprises a histidine moiety (His-tag) for linking to the transition metal complex. Preferably, the link between the test component and the indicator component further comprises a quaternary amine function. By choosing the appropriate position for introducing the histidine moiety, the involvement of one substrate in different metabolic pathways can be detected by the probe compound. Preferred positions for binding the histidine moiety are shown in Table 2. The so-formed reactive component is linked to the transition metal complex by two coordination bonds, one of which comprises the His-tag linked to the test component, while the other comprises the His-tag of the indicator component. It was surprisingly found that, if the reactive component is linked to the transition metal complex, the characteristic fluorescence activity of the indicator component is no longer observed. In other words, the probe compound remains silent with respect to the characteristic fluorescence signal. This would result in a dark spot in an assay or array position. When the probe compound is brought into contact with an enzyme having a function specific to the test component or substrate comprised in the probe compound, the test component or substrate is metabolised. It was surprisingly found that this enzymatic reaction has two effects. First, the characteristic fluorescence signal of the indicator component is observed again, and second, the enzyme remains bound to the transition metal compound. Thus, the probe compound allows for the unambiguous detection of the specific enzymatic reaction by flurescence detection. Moreover, the probe compound allows for an immobilisation of the substrate-specific enzyme, which can be advantageously used to isolate this enzyme from a sample.

In case of a probe compound of the invention comprising a Cy3 dye, it is observed that the the fluorescence dye is quenched, i.e. does no longer emit its characteristic fluorescence signal, if the reactive component is linked to the transition metal complex to form the probe compound of the invention. In any other form, the dye emits its characteristic fluorescence signal and cannot be used to detect productive enzymatic reactions. Only when a productive enzymatic

reaction occurs between an enzyme and the probe compound of the invention, the dye is released and the fluorescence is observed. This behaviour of the probe compound of the invention is the major difference to standard systems such as the labelling of proteins with a dye or DNA with a dye to produce protein arrays or DNA arrays, respectively, because the dye is permanently fluorescent in those cases.

The probe compound according to the above-discussed preferred embodiment of the invention can be illustrated by the following general formula (2):

$$\begin{array}{ccccc} & & \text{His-tag} - \text{TC} & & \\ & / & & \backslash & \\ \text{AC} - L_{AC\text{-}MC} - \text{MC} & & & & L_{TC\text{-}IC} \\ & \backslash & & / & \\ & & \text{His-tag} - \text{IC} & & \end{array} \qquad \text{Formula (2)}$$

wherein AC represents an optional anchoring component, preferably poly A or TA, MC represents the $N_\alpha$,$N_\alpha$-bis-(carboxymethyl)-L-lysine cobalt(II) complex, TC represents the test component, IC represents the fluorescence dye Cy3, His-tag represents a histidine moiety as described above, and $L_{AC\text{-}MC}$ and $L_{TC\text{-}IC}$ each represent optional linker moieties between the respective components indicated by the subscripts. Preferably, the $L_{TC\text{-}IC}$ linker moiety comprises a quartenary amine function.

Although the exact mechanisms involved in these reactions are not completely understood yet, it is believed that the linking of the reactive component to the transition metal complex influences the indicator component in such a manner that its electronic structure necessary for its characteristic fluourescence signal is disturbed. The reaction of the enzyme with the test component or substrate is believed to alter or break down the reactive component, which influences the binding properties of the reactive component or its respective metabolised products, respectively, to the transition metal complex. Thereby, the adverse effect on the indicator component is no longer present so that the indicator component can again exhibit its characteristic fluorescence signal. Moreover, this enzymatic alteration or break down of the reactive component may also create new binding sites at the reactive component and/or the transition metal complex, which result in the binding of the enzyme.

The present invention also provides a method for preparing the probe compound. The method for preparing the probe compound of the invention comprises the following steps:

a) Preparing a transition metal complex by reacting a suitable salt of a transition metal with a desired ligand molecule, optionally comprising an anchoring component. Herein, the appropriate conditions of solvent, pH, temperature and the like can be chosen according to known procedures. If necessary, the thus-obtained transition metal complex can be further purified by standard procedures, such as, for example, filtration, re-crystallisation, or the like. In a preferred embodiment, $N_\alpha$,$N_\alpha$-bis-(carboxymethyl)-L-lysine is reacted with cobalt(II) chloride to form the corresponding complex.

b) If an anchoring component is desired, it may also be incorporated at this stage. For example, in a preferred embodiment, the above cobalt(II) complex of the $N_\alpha$,$N_\alpha$-bis-(carboxymethyl)-L-lysine ligand may be reacted with a suitably activated poly A chain or with 6,8-dithioctic acid, or the like.

c) In a separate reaction, a substrate molecule to be incorporated in the test component is coupled to an indicator component, optionally including a linker moiety. If necessary, either one or both of the test component and indicator component is previously transferred into an activated form suitable for coupling, according to standard techniques. Herein, the appropriate conditions of solvent, pH, temperature and the like can be chosen according to known procedures. For example, a Cy3 dye available as its Cy3-NHS-ester may be reacted with a 4-amino-3-butyric acid linker to allow for linking with the test component. Preferably, a linker moiety between the test component and the indicator component comprises a quaternary amine function (a quartenary ammonium nitrogen atom). If necessary, the thus-obtained reactive component can be further purified by standard procedures, such as, for example, filtration, re-crystallisation, or the like.

d) Optionally, the test component and/or the indicator component is functionalised with a moiety suitable for binding to the transition metal complex, either before or after formation of the reactive component. Such functionalisation is especially advantageous for indicator components or test components, which do not readily form coordination bonds. In a preferred embodiment, the test component and/or the indicator component is functionalised with a histidine molecule (sometimes referred to as a "His-tag" in the following). A His-tag has the additional advantage to ensure an identical binding property of all possible test components (substrates) and/or indicator components (dyes)

to the transition metal complex.

e) In a subsequent step, the so-prepared reactive component comprising the test component and the indicator component is linked to the transition metal complex to form the probe compound according to the present invention. Herein, the appropriate conditions of solvent, pH, temperature and the like can be chosen according to known procedures. If necessary, the thus-obtained probe compound can be further purified by standard procedures, such as, for example, filtration, re-crystallisation, or the like.

The method comprises the steps a), c) and e), and the optional steps b) and/or d), if desired. The optional steps b) and d) can be carried out at any stage of the method. For example, step d) may be carried out before step c), or subsequent to step b), and step b) may be carried out before step a), subsequent to step a), or subsequent to step e), respectively. In a preferred embodiment, the individual steps are carried out in the order of steps a), b), d), c, and e). Alternatively, the steps may be carried out in the order of steps b), a), c), d), and e), or in the order of steps a), d), c), e), and b). A person skilled in the art will know which order and conditions are most suitable for the respective task.

Preferably, the step for linking the indicator component to the test component and/or the step for linking the histidine residue to the test component involves an activation of the test component by specific halogenation with iodine (I). For example, a selected position (atom or group) of the substrate or metabolite component comprised in the test component is selectively halogenated with iodine. This can be done by standard chemical reactions or enzymatically. The halogenated position allows for substitution reactions replacing the iodine atom by the corresponding component or residue.

The present invention also provides an array for detecting enzymes comprising a plurality of different probe compounds according to the invention. The array comprises a plurality of different probe compounds according to the invention (sometimes referred to as library of probe compounds in the following), i.e. probe compounds according to the invention which differ at least with respect to the test component comprised therein. Preferably, the probe compounds only differ with respect to the test component comprised therein. A plurality of different probe compounds can be prepared using automatic procedures, for example, parallel synthesis protocols and apparatuses, or the like. The term "comprising a plurality of different probe compounds" means at least two different probe compounds, and may comprise up to several thousands different probe compounds. For example, apparatuses and protocols, which are known and commercially available at the moment, allow the standard production of arrays based on glass slides wherein from 5000 to 15000 different probe compounds may be deposited on a single glass slide by micro-spotting techniques, or the like. In a preferred embodiment, about 2500 different probe compounds are included in the array (cf. example). Alternatively, the array may be constructed using a plate providing separate compartments or wells, such as, for example, a micro-titre plate, which is commercially available with, e.g., 384 wells, or different numbers of wells. A complete array may comprise one or more slides or plates depending on the actual number of probe compounds included in the array, as well as on the density available on the respective medium. Preferably, an array comprises more than one copy of a library of probe compounds on one or more supports. For example, a library or sub-library of probe compounds may be provided in duplicate or triplicate on one support or slide.

In one embodiment of the array, the plurality of probe compounds is attached to a planar surface of a suitable support or carrier. A support may be any support used in the art, preferred examples of which are glass surfaces, preferably of glass slides, surfaces of polymeric materials, such as polyacetate surfaces, or the like, or surfaces of cellulose or paper materials, or the like. In order to be attached to a solid surface, such as, for example, the glass surface of a glass slide, the probe compounds are provided with a suitable anchoring component. A preferred anchoring component known from the art is a poly A chain or tail, which can be readily attached onto a glass surface or the like following standard protocols using irradiation by UV light. Other suitable solid surfaces and corresponding anchoring components as well as protocols for their use are known in the art, and a person skilled in the art will be able to select an appropriate combination. For example, when using polymeric supports, it is advantageous for the bonds between the probe molecules and the polymeric support to be chemical bonds, especially covalent chemical bonds, which allow a long-lasting, stable bond between the probe molecules and the polymeric support.

In the case of using a well plate, the probe compounds do not necessarily require an anchoring component because they can be held in the respective wells without attachment to the wall. Preferably, probe compounds are attached to the walls of a well of a well plate. Therefor, all methods known in the art may be used.

The array of the invention (which is sometimes referred to as "reactome array") can be used for the simultaneous detection of reactive interactions between the probe compounds and all analyte molecules (enzymes) from a sample. In particular, the array provides a solution to the problem of the identification of metabolites and the enzymes involved in their transformation. The array provides a fast and reliable way to detect all metabolic pathways active in an organism or community, and may be used advantageously for an activity-based, annotation-independent procedure for the global assessment of cellular responses.

The invention also provides a method for producing an array according to the invention. An array may be

produced using well plates, such as commercially micro-titre plates, wherein each probe compound of the plurality of different probe compounds according to the invention is filled into an individual well, together with appropriate amounts of solvent, cofactors, cations, supplements, or the like, which are known or predicted to be required for the expected enzyme reaction. The filling of the wells may be carried out automatically, e.g. by a suitable robotic apparatus, or the like. Alternatively, the array is preferably produced using different probe compounds, which all comprise the same anchoring component. The different probe compounds having an anchoring component are then arranged onto an appropriate support surface, e.g. the surface of a glass slide, and subsequently bound thereto with the anchoring component. The arranging and binding of the different probe components may be carried out automatically, e.g. using a suitable robotic apparatus, or the like, and following established procedures and protocols. In a preferred embodiment, different probe compounds comprising a poly A chain as an anchoring component are spotted onto a glass slide by using a robotic apparatus, and subsequent fixation is achieved by crosslinking the poly A tails according to an established protocol using UV radiation. However, several other methods for arranging and fixing the different probe molecules onto a suitable support surface are known in the art. Using the method of the invention allows for a versatile, fast and reproducible production of arrays according to the invention.

Moreover, the invention also provides an isolation means comprising a nanoparticle and a probe compound according to the invention. A probe compound is preferably provided with a suitable anchoring component and attached to a nanoparticle. The term "nanoparticle" means a particle having a maximum dimension of less than 500 nm, preferably of less than 300 nm, and most preferred of about 100 nm. A minimum dimension is about 10 nm, preferably about 50 nm. A maximum or minimum dimension of a nanoparticle refers to the diameter in the case of a spherical nanoparticle. Examples for suitable nanoparticles are known in the art, as well as methods for producing the same, or anchoring components for linking the probe compound to the same. The probe compound may be linked to any nanoparticle known in the art, preferably a magnetic nanoparticle. Preferably, a nanoparticle comprises one or more metallic elements, including the transition metal elements. Preferred examples of nanoparticles comprise metallic elements, either pure metallic elements, such as, for example gold nanoparticles, or alloys comprising different metallic elements, or oxides of metallic elements, such as, for example, iron oxides, or the like. Preferred oxidic nanoparticles may comprise silicon, e.g. in form of silicon oxide. Moreover, preferred nanoparticles may also have a layered structure, e.g. an oxidic core coated by a metallic layer, such as a gold-coated silicon oxide nanoparticle, or a metallic core coated by an oxidic layer, such as a cobalt core coated with an iron oxide layer. For example, the synthesis and application of suitable magnetic gold nanoparticles is described by Abad et al. in J. Am. Chem. Soc. 127, 5689 (2005). Preferably, a nanoparticle has a magnetic property. A preferred anchoring component for linking a probe molecule to a magnetic gold nanoparticle is 6,8-dithioctic acid or $\alpha$-dihydrolipoic acid, respectively, which advantageously may be linked to a transition metal complex comprising the cobalt(II) complex of $N_\alpha$,$N_\alpha$-bis-(carboxymethyl)-L-lysine (ANTA-Co(II)) by forming an amide bond between the carboxylic acid function of the 6,8-dithioctic acid and the amine function of the ligand molecule. The isolation means according to the invention allows for the substrate specific interaction and binding of an enzyme which can then be isolated by means of filtration, gravitation force (centrifugation), an external magnetic force in case of a magnetic nanoparticle, or the like. It was found that, owing to the substrate specific binding of an enzyme by the probe compound, the isolation means of the invention allows for the directed isolation of an enzyme because of its substrate specificity.

The invention also provides a method for producing a isolation means according to the invention. In this method, a probe compound comprising a suitable anchoring component is prepared according to the method of producing a probe compound according to the invention, and subsequently attached to a suitable nanoparticle prepared according to known methods. Preferably, the probe compound is attached to a magnetic nanoparticle. The method according to the invention provides an easy access to an isolation means having specific binding sites for an enzyme.

Moreover, the invention also provides a method for detecting enzymes in a substrate specific manner, using the probe compound according to the invention or the array according to the invention. An array comprising a plurality of different probe compounds is prepared according to the method of the invention. The array can preferably comprise more than one copy of the respective library of probe compounds, either by providing the library on one support or slide in duplicate or triplicate or in more copies, or by providing more than one support or slide comprising identical sets or libraries of probe compounds. The array is then brought in contact with a sample comprising the analyte molecules, i.e. the enzymes whose substrate specific activity is to be tested. This step is also referred to as incubation with a sample. A sample in the context of the method includes any kind of solution of analyte molecules (enzymes) that can enter into an enzymatic reaction with the probe compounds on the array. These include especially biological samples obtained from the lysis of cells of bacteria, archeae, or higher organisms, but also from biological fluids such as blood, serum, secretions, lymph, dialysate, liquor, sap, body fluid from insects, worms, maggots, etc. Also included is extraction from natural sources such as biopsies, animal and plant organs or parts, cell, insect, worm, bacteria, microbe and parasitic matter as well as supernatants of cell cultures and of bacterial, microbial or parasitic cultures. A sample may also be a chemical-synthetic sample containing, for example, synthetic proteins, or the like. After incubation is complete, the sample is removed from the array. In order to obtain a complete removal of the sample, the array may be washed one or more times. Then, the array is analysed for the presence of the signal characteristic for the indicator component.

Preferably, a fluorescence signal is read out and processed by a suitable apparatus, which is available in the art. All protocols and procedures known in the art may be used, including automatic processing by robotic apparatuses and the like. In a preferred embodiment, using an array comprising probe compounds comprising a Cy3 fluorescence dye, the fluorescence signal is preferably measured using a laser scanning system.

The method of the invention using the array of the invention allows to assess the complete reactome of an organism or community in one step. That is, all substrate specific enzymatic reactions which are performed within the metabolic pathways necessary for the individual life form(s) is readily accessible. This advantageously allows for the accurate reconstruction of metabolic pathways.

Moreover, the invention also provides a method for isolating enzymes using the isolation means according to the invention. According to the method, the nanoparticles are used to isolate enzymes which are bound to the isolation means by the probe molecules after the substrate specific enzymatic reaction. The reaction can be carried out under the same conditions as described above for the array of the invention, including all processes and protocols that are known in the art. Preferably, the isolation means is brought into contact with a sample comprising analyte molecules (enzymes) that can enter into an enzymatic reaction with the probe compounds on the isolation means. The isolation means carrying the enzymes are then isolated by magnetic means or by filtration. The isolated enzymes can then be analysed using standard techniques, such as, for example, sequence analysis, mass spectrometry, or the like. Identification of enzymes whose function is verified by the specific reaction with the probe compound of the invention allows for the detection of metabolic pathways as well as for the correct annotation of unsequenced genes and/or unknown proteins or enzymes. The method can be used to complete the metabolic pathway map of all life forms, as well as specialised parts thereof. The method can also be used to reconstruct the global metabolism of complete communities living in distinct natural (microbial) communities.

Moreover, the method of the invention can be advantageously used in the search for new drugs, in particular in screening for potential new targets: here the guiding principle is selective toxicity, so the search is for molecular targets in the target organism that do not exist in a human. The method of the invention is also useful for the identification of drugs that are specific for certain pathogens, so that treatment of an infected patient would not result in the elimination of a major part of the body flora, as is currently the case with common broad spectrum antibiotics, and all the negative consequences of this that ensue. Using the array to obtain reactome profiles of the representatives of the major phyla of life allows their comparison and identification of individual reactions/enzymes that are specific for each branch or clusters of branches. Some of these phylum-specific functions will be known already, but others may be new: such metabolic reactions/enzymes may then serve as newly-discovered targets for drug screening. For example, the comparison of the human reactome with the composite reactome of bacteria and archaea may identify new targets for broad spectrum antibiotics, or the comparison of the reactomes of the various bacterial phyla may reveal potential targets specific for individual phyla, such as those to which, e.g., Neissseria, Pseudomonas, Mycobacterium, Vibrio, Staph, Strep, Pneumo, coliforms belong, providing a route to more specific, narrow spectrum antibiotics. Moreover, the comparison of reactomes of photosynthetic organisms may identify new targets for herbicides specific for, e.g., moss, or algae, or monocots, or dicots etc. that would allow the development of new generation anti-moss treatments that would not affect other plants, anti-algal treatments not active against other plants, etc., etc. Moreover, the comparison of the reactomes of insect vectors of disease (mosquito, black fly, etc) with those of other insects, humans etc. could identify vector-specific targets.

In other words, the present invention provides a versatile tool for obtaining information on the reactomes of all major branches of the tree of life which can be advantageously used in future drug development. Through the selective inhibition of pathogens, there become available a lot of applications involving the selective inhibition of specific microbial populations, either because they are known to be problematic, or simply to test experimentally their contribution to a particular process (e.g. the role of a particular GI tract organism in a particular GI physiological role, like folate production, the role of a particular rhizosphere organism in protection from fungal infections, etc.). Thus the array provides the means of identifying phylum-specific metabolic targets for inhibitors that, in turn, can be used to inhibit specifically those phyla in microbial communities to assess their role in a particular process.

The use of the reactome array of the invention for identifying new targets can be applied from human medicine to agriculture to dental medicine to shipping (anti-algal compounds to prevent algal fouling; anti-sulphate reducer compounds to prevent corrosion of steel) to construction (e.g. anti-sulphate reducer compounds to prevent corrosion of steel), etc., and to research tools (agonists/antagonists for all manner of selected life forms).

The present invention provides a procedure to measure on an array, enzymatic activities of cells against many of the standard substrates and metabolites that characterize life, plus other substrates of interest. Because of the chemical design of the substrates of the array, the array provides the identity of the reaction, the reaction products and, in a subsequent step, the enzyme itself. It therefore links a substrate/metabolite with its cognate enzyme. The reactome array thus forges a thus far missing link between metabolome and genome. Since many of the metabolites on the array are connected in pathways, it is also possible to reconstruct the metabolic network operating in any organism without any prior genomic information.

The use of the array to investigate the reactome of a microbial community is particularly interesting. The array represents a new possibility to have a metabolic overview of an entire community, without perturbing it in any way prior to preparation of the community lysate for analysis. And, in cases where it is difficult to obtain sufficient biomass for direct analysis, it is even possible to obtain a small molecule microarray analysis of a metagenomic library of a DNA sample of a community or enrichment and thereby obtain a detailed overview of the global metabolism of the sampled community. The reactome analysis described here uncovered new metabolic activities in organisms and communities (46 new functions in P. putida KT2440 and five in metagenomic communities, including a novel hydrogenase; cf. Table 4 and Figures 6 and 7, SEQ ID NOS. 13 to 16, 21 to 24, 29 to 32, 38 to 43, and 50 to 54), which not only provide interesting opportunities for new lines of investigation, but also revealed new metabolic components of niche specificity and predominant microbial pathways shaping the overall metabolism of the individual habitats. Especially, the hydrogenase (cf. Table 4 and Fig. 7, SEQ ID NOS. 50 to 54), which is a rather small enzyme, may be of interest in the field of energy production. Moreover, a novel reBr halogenase/dehalogenase, i.e. a multifunctional $\alpha/\beta$-hydrolase, was mined from a metagenome library of a microbial community in seawater contamined with petroleum hydrocarbons, with a novel hydrolytic phenotype, namely the cleavage of both 'common' p-nitrophenyl (pNP) esters and haloalkanoates, and weak activity towards haloalkanes (paper in preparation, SEQ. ID. NOS. 57 to 59). This halogenase/dehalogenase enzyme was found to be useful for the introduction of iodine into the test component in the syntheses of the probe compounds of the present invention.

Since a physico-chemical analysis of habitats is rather selective in terms of the parameters measured, detection limits defined by the instruments used, and usually does not discriminate between bioavailable and non-bioavailable levels, whereas the array scores most of the metabolic potential of the cell or community in relation to the prevailing conditions and bioavailable fraction of compounds, another application of the array is the habitat characterization by metabolic profiling. This type of application can also be used in diagnosis of diseases/intake of drugs/toxic substances that influence metabolic activities of the microbial flora, through reactome analysis of faecal/skin biota, forensic analyses of diverse types (e.g. groundwater pollution), prospecting (e.g. for natural gas seeps that indicate underlying reservoirs), detection of manufacturing sites of illegal substances, etc. Indeed, the design of custom arrays for use with particular organisms or communities will entrain a diverse spectrum of applications relating to enzyme activity profiles, including the phenotyping of organisms (microbes, plants, higher animals and humans), populations, mutant libraries and transgene libraries, the direct diagnosis of diseases and quality control in food industries, to cite some.

The present invention will be illustrated in more detail in the following examples, but it is not restricted to the special embodiments exemplified in these examples.

Commonly used chemical and molecular-biological working methods are not described in detail here, but they can be referred to in, for example, Houben-Weyl, Methods of Organic Synthesis.

**EXAMPLES**

**EXAMPLE 1:**

**General techniques.**

Unless specified otherwise, reactions were carried out with dry solvents freshly purified by passage through a column of activated alumina (A-2) and supported copper redox catalyst (Q-5 reactant). All other reagents were purified according to standard literature methods or used as obtained from commercial sources.

NMR spectra of all compounds were recorded at 600, 500, 400, or 300 MHz, using Varian 1-600, Varian 1-500, Varian M-400, Varian M-300, and Bruker Biospin 300 instruments. $^1H$ NMR chemical shifts were reported relative to residual $CHCl_3$ (7.26 ppm). $^{13}C$ NMR data were recorded at 125, 100, or 75 MHz, using Varian 1-500, Varian M-400, or Bruker Biospin 300 MHz instruments, respectively. $^{13}C$ NMR chemical shifts are reported relative to the central line of $CDCl_3$ (77.0 ppm). $^{59}Co$ NMR measurements were carried out at room temperature with Bruker ASX-200 ($B_0$=4.7 T, Larmor frequency $v_0$=48.1 and 52.9 MHz in $^{59}Co$ resonance, Bruker MSL-300 ($B_0$=7.1 T, $v_0$=71.2 and 79.4 MHz), and Bruker Avance DSX-500 ($B_0$=11.7 T, $v_0$=120.4 and 132.3 MHz) spectrometers. Single-pulse MAS spectra were obtained by using a Bruker MAS probe with a cylindrical 4-mm o.d. rotor. When necessary, continuous-wave proton decoupling with a radiofrequency (RF) field of 50 kHz was applied during acquisition. Spinning frequencies $v_r$ up to 17 kHz were utilized. A short pulse length of 1 $\mu$s corresponding to a nonselective n/12 pulse determined using an aqueous or DMSO solution of small molecules (SMs) was employed. Recycle times were 1 and 90 s in $^{59}Co$. The baseline distortions resulting from the spectrometer dead time (5-10 $\mu$s) were removed computationally using a polynomial baseline correction routine. The dead-time problem was then,overcome by Fourier transformation of the NMR signal, starting at the top of the first rotational echo.

Molecular masses were analyzed at the SIDI Core Facility of the Autonomous University of Madrid. For each experiment, a magnetic high resolution mass spectrometer (8000 v acceleration) was used, with ionization source FAB

(LSIMS - liquid secondary ion mass spectrometry with Cs ions) using m-nitrobenzoic alcohol (m-NBA) as matrix. The samples (0.5-1.2 g) were dissolved in acetone, methanol or DMSO, depending of the solubility of the SMs-Cy3. Microanalyses were performed by the SIDI Core Facility of the Autonomous University of Madrid, and are quoted to the nearest 0.1% for all elements, except for hydrogen, which is quoted to the nearest 0.05%. Reported atomic percentages are within the error limits of ± 0.3%.

For N-terminal amino acid sequencing, polyacrylamide gel electrophoresis under denaturing conditions (SDS-PAGE: 10%, v/v) was performed according to Laemmli (U.K. Laemmli, Nature 227, 680 (1970)), using a Mini-PROTEAN cell apparatus (Bio-Rad), and protein bands were blotted to a polyvinylidene difluoride (PVDF) membrane. The PVDF membrane was stained with Coomassie Brilliant Blue R-250, after which the bands of the proteins were cut out and processed for N-terminal amino acid sequencing. The peptide sequences were initially scored against blastp nr to identify the best hits among full-length proteins, and then converted into coding sequences and screened for potential protein encoding genes (PEGs) via tblastn and tblastx search (F. Stephen et al., Nucleic Acids Res 25, 3389 (1997)) against the comprehensive non-redundant database sourced from the nucleotide (nr/nt) collection, reference genomic sequences (refseq_genomic), whole genome shotgun reads (wgs) and environmental samples (env_nt) databases. This was chosen empirically to increase the number of matching potentially coding elements. Based on the best BLAST hits, degenerate oligos were designed and used to amplify full length ORFs from the metagenome libraries.

**EXAMPLE 2:**

**Bacterial strains, culture, and growth conditions.**

*E. coli* DH5F' was used as a recipient for pGEMT plasmid (Promega) constructs containing cloned PCR fragments of P. putida KT2440 encoding hypothetical proteins and metagenomic proteins. E. coli TOP10 (Invitrogen) was used as a recipient for pCCFOS vector (EPICENTRE) constructs. *E. coli* cultures were grown in Luria-Bertani (LB) medium and incubated at 37°C on an orbital platform operating at 200 rpm. When required, cultures were supplemented with the following antibiotics: ampicillin (100μg/ml), nalidixic acid (10 μg/ml) and chloramphenicol (12.5 μg/ml). P. putida KT2440 was grown in M9 minimal medium with 15 mM succinate as carbon source in 100-ml flasks shaken at 30 °C and 150 rpm from an initial turbidity at 600 nm of 0.02 to a final value of 0.7±0.05. Samples (3 ml) were removed, the cells harvested by centrifugation at 4°C, and the cell pellets were washed with 20 mM Hepes pH 7.0 before storing at -20°C until use.

**EXAMPLE 3:**

**General reactome strategy.**

The general reactome strategy comprises five stages for array construction and protein-SMs transformation detection as follows (cf. Figures 1 to 3).

[1] ***Data searching and compound identification***

Initially, an extensive data mining effort, focused mainly on the Kyoto Encyclopedia of Genes and Genomes (KEGG Database: http://www.genome.ad.jp/kegg/), the University of Minnesota Biocatalysis and Biodegradation Database (UM-BBD: http://umbbd.msi.umn.edu/) and PubMed (http://www.ncbi.nlm.nih.gov/sites/entrez), was undertaken to produce a list of compounds to be synthesized that are substrates of one or more metabolic reactions and that collectively form most of the central metabolic networks of cellular systems. Additional metabolites characteristic of microbial metabolic activities were also identified for synthesis.

[2] ***Compound synthesis, modification and arraying***

A library of 2483 identified SMs was synthesized using the strategies specified in Table 1. The purity of each SM was confirmed by NMR and molecular mass. Individual SMs were coupled to the Cy3 fluorescent dye and subsequently combined on specific positions to a nitrotriacetic-Co(II) complex containing a terminal poly A-tail (Table 2). Importantly, during synthesis, the Cy3 dye is attached to the molecule at specific positions that allow control of the reaction product formed, through creating for each molecule different Cy3-variants that collectively serve as substrates for all possible reactions described in KEGG; the array thus assays multiple distinct reactions of the same metabolite. The resulting derivatives were dissolved at different concentrations from 0.5 to 100 nM (six concentrations) in dimethyl sulfoxide (DMSO) and stored at -70°C until use in 384-well microtiter plates. Each well also contained cofactors, cations and supplements known or predicted to be required for efficient transformations. The 2483 SMs included in this study,

together with the position of modification with Cy3 are given in Tables 1 and 2. These procedures were also used to synthesize Cy3-modified X-Gal (obtained from Boehringer Mannheim), which was used as substrate for the β-galactosidase of *E. coli* (provided also by Boehringer Mannheim). Individual SMs were subsequently spotted onto Corning UltraGAPS glass slides in a spatially addressable manner, by means of a MicroGridII spotting device from Biorobotics operating at 20°C and 50% relative humidity, and subsequently immobilized by standard UV crosslinking.

[3] ***Array analysis and cell extract***

60 μl quantities of cell lysates of microbial cultures, or libraries of metagenomic clones of microbial communities, diluted in PBS buffer to a final protein concentration of 0.1 mg/ml, were layered on the array, which was subsequently incubated at room temperature for 30 min.

[4] ***Data analysis and metabolic reconstruction***

Arrays were scanned for fluorescence with a GenePix 4000B scanner (Axon Instruments) operating at 532 nm at 10 μm resolution with 100% laser power, and images (spot and background intensities were quantified in triplicate or more) were analyzed using the GenePix v5.1 software (Axon Instruments). Reconstruction of metabolic maps were carried out with GraphViz and SOI Linux software.

[5] ***Protein identification with gold beads***

Proteins reacting with specific metabolites were identified by incubating a protein extract with metabolite-coated gold nano-particles, followed by protein sequencing of the captured proteins, reverse translation of the sequences into gene sequences, cloning of the corresponding genes, hyper-expression of the genes, and purification and characterization of the corresponding proteins.

**EXAMPLE 4:**

**General procedure for the synthesis of probe compounds.**

The general reaction strategy showing the successive steps for the construction of Cy3 modified metabolites and their integration into probe compounds is described below.

***1. Preparation* of *nitrilotriacetic-Co(II) complexes.***

The amino-nitrilotriacetic-Co(II) complex was formed by reaction of *N*R,*N*R-bis(carboxymethyl)-L-lysine hydrate (ANTA, Fluka) with an excess of cobalt(II) chloride (Sigma) in 20 mM HEPES in aqueous solution (36). Excess cobalt was precipitated by increasing the pH to 10, and the precipitate was removed by filtration through a 0.2 μm membrane (PTFE, Amicon).

***2. Incorporation of poly(A) tails in nitrilotriacetic-Co(II) complexes (optional).***

Activation of the phosphate groups of the poly(A) tails (Sigma Genosys, average molecular weight: 100 kDa) and subsequent amidation with the Co(II) complex was performed by overnight incubation with 3 mM N-hydroxysuccinimide (NHS, Fluka) and 3 mM 1-ethyl-3-[3c-(dimethylamino)propyl]-carbodiimide (EDC, Sigma) in 20 mM HEPES buffer (pH 7.5).

***3. Generation of double* activated *Cy3 dye.***

Cyanine dye was linked via a histidine tag and a flexible linker through which the dye is linked to the Co(II) complex and the metabolite, respectively. Briefly, a histidine molecule was firstly incorporated to the Cy3 dye by enzymatic acidolysis of Cy3 NHS (a succinimidyl ester, GE Healthcare) with histidine and immobilized Lewatit lipase EL1 (37) from a cow rumen metagenome (37.39 mol% incorporation of histidine in 24 h, at a ratio 1 histidine:4 NHS ester). 3-Methyl-2-butanol was used as solvent with a water content of up to 3.2%, and the reaction was carried out at 50-55 °C. High-performance preparative liquid chromatography was used to analyze and purify the products of the acidolysis reaction. Purified Cy3-His was dissolved in DMSO at a concentration up to 4 M and stored at -70°C until use

In a second step, the Cy3-His was joined to a 4-amino-3-butyric acid linker. Briefly, the linker was dissolved in 0.1 M sodium borate buffer pH 8.5 and Cy3-His dissolved in a small amount of neutral water was added in aliquots until

equimolar concentrations were reached. After incubation for 2 hours at room temperature, the labeled product was purified by reverse phase HPLC on a Chemcobond 5C18 ODS column (4.6 x 150 mm). Elution was carried out with a linear gradient of 6% to 50% acetonitrile in 50 mM ammonium formate, pH 7.0, over a period of 30 min at a flow rate 1.0 mL/min, with monitoring of the eluate at 550 nm. The yield was 46%. HRMS (MALDI) calculated for $C_{44}H_{55}N_6O_{11}S_2$ [$M^+$] was 908.0906 and found was 908.0955. The Cy3-His-4-amino-3-butyric acid (*N*,*N*-dimethylamino) ethylester was synthesized by Lewatit lipase EL1-mediated esterification as described above. The final product was purified by reverse phase HPLC, as described above, except that the gradient was 6% to 80% acetonitrile. The yield was 0.1%. HRMS (MALDI) calculated for $C_{53}H_{64}N_9O_{13}S_2$ [$M^+$] was 1099.2815 and found was 1099.2841.

***4. His-tagged* metabolite *library synthesis.***

The primary synthetic challenge involved finding a reaction path from a functionalized core with the highest yield (>26-90% overall). The purified metabolites were characterized by NMR and HPLC and were found to be 90% pure; yields were >20%. A number of standard strategies were employed, and new ones developed for the synthesis of compound libraries synthesized on solid supports or by parallel synthesis using separate reaction vessels. The full spectrum of synthesis methods used in the present study (as well as NMR and high resolution mass spectra) will be available to the community on our web server (http://biology.bangor.ac.uk/people/staff/025123) and are briefly described in Table 1, together with metabolite synthesis strategies. In some cases, the metabolites were directly purified from pure cultures using preparative HPLC (Waters 2795 XE). The purity of each SM was confirmed by NMR and mass determination (see Table 1). SMs were reconstituted and diluted in PBS buffer, DMSO or a mixture of both, accordingly to their solubility properties, and stored in 384-well microtiter plates at -70°C until used. Metabolites were further functionalized via incorporation of a histidine tag at specific positions (SM-His; see Tables 1 to 3) using solid-solid and solid-liquid phase synthesis.

General synthetic methods to incorporate His-tags to different metabolites (SM) are listed in the following.

| *Metabolite characteristics* | *Synthetic method to incorporate His tags* |
|---|---|
| OH-containing metabolites | Lipase (*Thermomyces lanuginosus*) esterification with histidine in 3-Methyl-2-butanol (Ferrer et al., Tetrahedron (2000) 56: 4053-4061) |
| $NH_2$-containing metabolites | Lipase (*Candida antarctica*) amidation with histidine in 3-methyl-2-butanol (Plou et al., Journal of Biotechnology (2002) 96: 55-66) |
| Aliphatic metabolite (linear) | Enzymatic incorporation of OH- groups via a wide spectrum dioxygenase followed by esterification with lipase (*Thermomyces lanuginosus*) and histidine in 3-methyl-2-butanol. |
| Aliphatic metabolite (circular) | The metabolite is dissolved in trifluoroacetic acid (20 ml) and refluxed for two hours under nitrogen. The solvent is evaporated and the residue is extracted with ethyl acetate. The organic layer is washed with water, brine and dried over MgSO4. The hydroxylated compound is then esterified with histidine using *Thermomyces lanuginosus* lipase in 3-methyl-2-butanol. |
| Aromatic | The -OH group is incorporated as described by Callahan et al., Bioorganic and Medical Chemistry Letters 16, 3802 (2006) and the hydroxylated compound is then esterified with histidine using *Thermomyces lanuginosus* lipase in 3-methyl-2-butanol. In this case a double bond is lost during the synthesis. |

***5. Generation of His-tagged metabolite-Cy3 library.***

Incorporation of the fluorescence dye in the metabolite was achieved by reaction of the SM-His molecules with activated Cy3-His-4-amino-3-butyric acid (*N*,*N*-dimethylamino) ethylester, using solid-solid and solid-liquid phase synthesis. The resulting library is composed of individual metabolites coupled at different positions to Cy3 dye molecules, both of which carry histidine tags. The coupling of Cy3 at different positions was designed to create the full spectrum of potential substrates needed to detect all possible catalytic reactions described in KEGG for a given core metabolite. Thus, each type of Cy3 substituent configuration determines the identity of the reaction and the reaction products. And, only when a reaction occurs at a specific position is the Cy3 dye released to give a fluorescent signal.

General synthetic methods to incorporate different His-tagged metabolites (SM) to the activated Cy3 are listed in the following.

| *Metabolite characteristics* | *Synthetic method to incorporate His tags* |
|---|---|
| Halogenated metabolites | The activated Cy3 is reacted with SM-His halide in the presence of a quaternary ammonium salt such as benzyltriethylammonium chloride using 50% aqueous sodium hydroxide as an acid-removing agent (Bull. Chem. Soc. Jpn., 54, 1879 (1981)). |
| Non halogenated aliphatic metabolite (linear) | An halogenated moiety is incorporated via treatment of the His-SM with CHC13 + Fe(CO)5. Afterwards, the halogenated derivative is incorporated to the Cy3 as above |
| Non halogenated aliphatic metabolite (circular) | Enzymatic C-C bond formation via a wide spectrum aldolase. |
| Aromatic | Aromatic nucleophilic substitution reaction between His-SM and activated Cy3 in anhydrous dimethylsulfoxide as described by Médebielle (Tetrahedron Letters, 37, 5119-5122 (1996)) |

***6. Incorporation of His-tagged metabolite-Cy3 derivatives* to *the poly(A)-nitrilotriacetic-Co(II) complex.***

The final step in the array development is the incubation of the histidine functionalized Cy3-SM with poly (A)-nitrilotriacetic-Co(II) complexes in 50 mM phosphate buffer, 50 mM NaCl, pH 7.5 for 1 hour at 25°C. when required, DMSO was added to increase substrate solubility. The resulting complexes were separated from unbound enzyme molecules by HPLC as described above. To ensure that each SM-Cy3 binds through both of its His residues, $^{59}$NMR was performed, and only derivatives incorporating single SM-Cy3 molecules were purified, and stored in 384 microtiter plates at -70°C until used. These molecules were used directly for the construction of the array.

***7. Binding of His-tagged metabolite-Cy3 derivatives to gold nanoparticles (optional).***

Au-6,8-dithioctic acid (TA) clusters were synthesized as described by Abad *et al*. (Abad et al. in J. Am. Chem. Soc. 127, 5689 (2005)) and used to create Au-TA-ANTA-Co(II)-SMs-Cy3 clusters. Briefly, the Au-TA clusters were linked to the ANTA-Co(II)-SMs-Cy3 (prepared as described above) by overnight amidation in a single step in the presence of 3 mM N-hydroxysuccinimide (NHS, Fluka) and 3 mM 1-ethyl-3-[3 ¢ -(dimethylamino)propyl]carbodiimide (EDC, Sigma) in 20 mM HEPES buffer (pH 7.5). Further purification was carried out by ultrafiltration through low-adsorption hydrophilic 30000 NMWL cutoff membranes (regenerated cellulose, Amicon).

**SYNTHESIS EXAMPLE 1:**

The following Synthesis Example 1 provides a complete synthesis of a probe compound comprising 5-bromo-4-chloro-3-indoyl-beta-galactopyranoside (X-Gal) as test component (SM) and Cy3 as indicator component. Both the test component and the indicator component are linked to a transition metal complex comprising cobalt by one histidine linker moiety each. Test component and indicator component are linked by 4-amino-3-butyric acid as a linker moiety. The so-prepared probe compound is used for the analysis of the sensitivity of the reactome array.

*1. Preparation of nitrilotriacetic-Co(II) complexes.*

The amino-nitrilotriacetic-Co(II) complex was formed by reaction of *N*R,*N*R-bis(carboxymethyl)-L-lysine hydrate (ANTA, Fluka) with an excess of cobalt(II) chloride (Sigma) in 20 mM HEPES in aqueous solution (J. M. Abad et al., J Am Chem Soc 127, 5689 (2005)). Excess cobalt was precipitated by increasing the pH to 10, and the precipitate was removed by filtration through a 0.2 μm membrane (PTFE, Amicon).

*2. Incorporation of poly(A) tails in nitrilotriacetic-Co (II) complexes.*

Activation of the phosphate groups of the poly(A) tails (Sigma Genosys, average molecular weight: 100 kDa) and subsequent amidation with the Co(II) complex was performed by overnight incubation with 3 mM N-hydroxysuccinimide (NHS, Fluka) and 3 mM 1-ethyl-3-[3 ¢ -(dimethylamino)propyl]-carbodiimide (EDC, Sigma) in 20 mM HEPES buffer (pH 7.5).

*3. Generation of double activated Cy3 dye.*

Cyanine dye was linked via a histidine tag and a flexible linker through which the dye is linked to the Co(II) complex and the metabolite, respectively. Briefly, a histidine molecule was firstly incorporated to the Cy3 dye by enzymatic acidolysis of Cy3 NHS (a succinimidyl ester, GE Healthcare) with histidine and immobilized Lewatit lipase EL1 (37) from a cow rumen metagenome (37.39 mol% incorporation of histidine in 24 h, at a ratio 1 histidine:4 NHS ester). 3-Methyl-2-butanol was used as solvent with a water content of up to 3.2%, and the reaction was carried out at 50-55 °C. High-performance preparative liquid chromatography was used to analyze and purify the products of the acidolysis reaction. Purified Cy3-His was dissolved in DMSO at a concentration up to 4 M and stored at -70°C until use.

In a second step, the Cy3-His was joined to a 4-amino-3-butyric acid linker. Briefly, the linker was dissolved in 0.1 M sodium borate buffer pH 8.5 and Cy3-His dissolved in a small amount of neutral water was added in aliquots until equimolar concentrations were reached. After incubation for 2 hours at room temperature, the labeled product was purified by reverse phase HPLC on a Chemcobond 5C18 ODS column (4.6 x 150 mm). Elution was carried out with a linear gradient of 6% to 50% acetonitrile in 50 mM ammonium formate, pH 7.0, over a period of 30 min at a flow rate 1.0 mL/min, with monitoring of the eluate at 550 nm. The yield was 46%. HRMS (MALDI) calculated for $C_{44}H_{55}N_6O_{11}S_2$ [$M^+$] was 908.0906 and found was 908.0955. The Cy3-His-4-amino-3-butyric acid (N,N-dimethylamino) ethylester was synthesized by Lewatit lipase EL1-mediated esterification as described above. The final product was purified by reverse phase HPLC, as described above, except that the gradient was 6% to 80% acetonitrile. The yield was 0.1%. HRMS (MALDI) calculated for $C_{53}H_{64}N_9O_{13}S_2$ [$M^+$] was 1099.2815 and found was 1099.2841.

*4. Preparation of His-tagged 5-bromo-4-chloro-3-indolyl-beta-galactopyranoside (X-Gal).*

A histidine molecule was firstly incorporated to the X-Gal by enzymatic esterification of histidine with X-Gal and immobilized *Thermomyces lanuginosus* lipase EL1 (11.2 mol% incorporation of histidine in 24 h, at a ratio 2 histidine : 1 X-Gal). 3-Methyl-2-butanol was used as solvent with a water content of up to 3.0%, and the reaction was carried out at 45°C. High-performance preparative liquid chromatography using nucleosil C18 column using methanol:water as mobile phase was used to analyze and purify the products. Purified X-Gal-His was stored at -20°C until use. This method was used to link His molecules to carboxylate containing molecules.

*5. Inorporation of X-Gal-His to Cy3-His-4-amino-3-butyric acid (N,N-dimethylamino) ethylester.*

The activated *Cy3 is reacted with X-Gal-His* halide in the presence of a quaternary ammonium salt such as benzyltriethylammonium chloride using 50% aqueous sodium hydroxide as an acid-removing agent (Bull. Chem. Soc. Jpn., 54, 1879 (1981)). By using this method, the Cy3 molecule is attached via the linker moiety to the halogenated moiety in the X-Gal molecule.

*6. Incorporation of His-tagged X-Gal-Cy3 derivatives to the poly (A) -nitrilotriacetic-Co (II) complex.*

The final step in the array development is the incubation of the histidine functionalized Cy3-X-Gal with poly (A)-nitrilotriacetic-Co(II) complexes in 50 mM phosphate buffer, 50 mM NaCl, pH 7.5 for 1 hour at 25°C. When required, DMSO was added to increase substrate solubility. The resulting complexes were separated from unbound enzyme molecules by HPLC as described above. To ensure that each SM-Cy3 binds through both of its His residues, $^{59}$NMR was performed, and only derivatives incorporating single SM-Cy3 molecules were purified, and stored in 384 microtiter plates at -70°C until used. These molecules were used directly for the construction of the array.

**EXAMPLE 5:**

**Improved procedure for the synthesis of probe compounds.**

***1. Preparation* of *nitrilotriacetic-Co(II) complexes***

The procedure was adapted from J. M. Abad et al., J Am Chem Soc 127, 5689 (2005), where the full synthesis is described. The amino-nitrilotriacetic-Co(II) complex **(A)** was formed by reaction of *N*R,*N*R-bis(carboxymethyl)-L-lysine hydrate (ANTA, Fluka) (5 g; 19 mmol) with an excess of cobalt(II) chloride (Sigma) (24.7 g; 190 mmol) in 20 mM HEPES in aqueous solution (10 ml total volume) overnight at 25°C. Excess cobalt was precipitated by increasing the pH to 10 by adding 4 N NaOH, and the precipitate was removed by filtration through a 0.2 μm membrane (PTFE, Amicon) (2.3 g, 53% yield; white solid crystalline powder) **(A)**.

*Reaction scheme:*

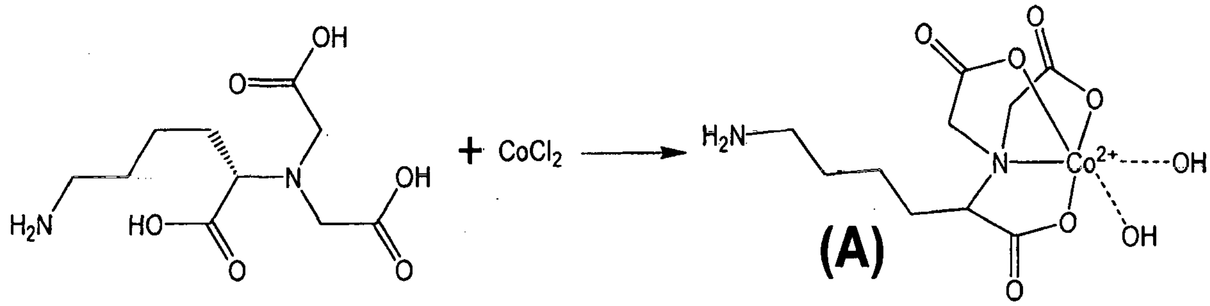

*2. Incorporation of poly(A) tails to nitrilotriacetic-Co(II) complexes.*

The procedure was adapted from J. M. Abad et al., J Am Chem Soc 127, 5689 (2005). Briefly, activation of the phosphate groups of the poly(A) tails (Sigma Genosys) (2.091 mg; 258 nmol) and subsequent amidation with the Co(II) complex was performed by overnight incubation at 25°C with 3 mM N-hydroxysuccinimide (NHS, Fluka) and 3 mM 1-ethyl-3- [3 ¢ -(dimethylamino)propyl]-carbodiimide (EDC, Sigma) in 20 mM HEPES buffer (pH 7.5). Concentration of the pure fractions (freeze drier) afforded the poly(A)-ANTA-Co(II) complexes **(B)** as a white solid crystalline powder (1.82 mg; ~86%).

*Reaction scheme:*

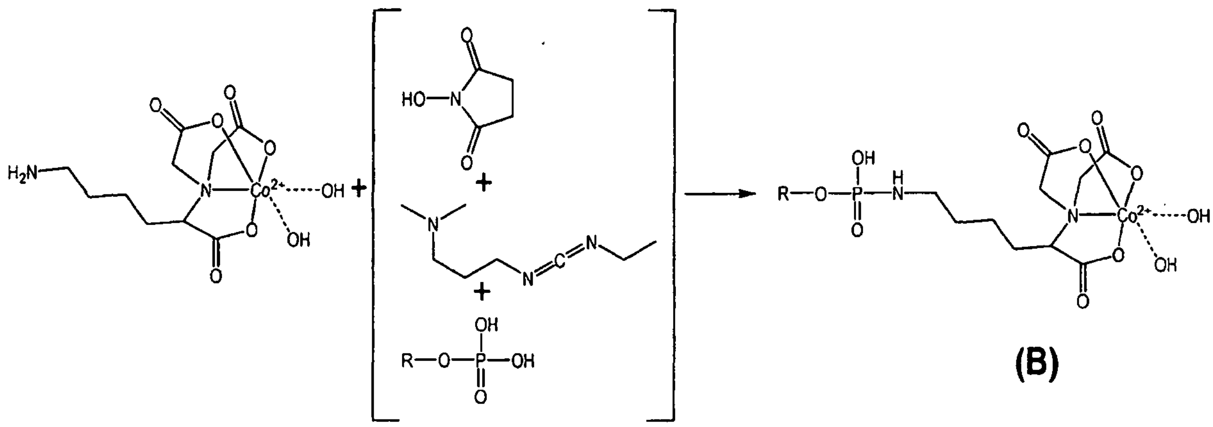

***3. Modification of cyanine dye with histidine***

A histidine molecule was firstly incorporated to the Cy3 dye by enzymatic transamidolysis of Cy3 NHS (a succinimidyl ester, GE Healthcare) with histidine and immobilized Lewatit lipase EL1 (D. Reyes-Duarte et al., Angew Chem Int Ed Engl 44, 7553 (2005)) from a cow rumen metagenome. All reactions were performed in the dark. Solvents were dried over 3 Å molecular sieves for 24 h prior to use. Briefly, Cy3 NHS (250 mg, 0.344 mmol) was dissolved in 5 mL of dimethylsulfoxide (DMSO) and 2-methyl-2-butanol was slowly added to a final volume of 25 mL. The biocatalyst (Lewatit lipase EL1, 2.5 g) and 3 Å molecular sieves (2.5 g) were then added and the suspension maintained 30 min at 40°C with magnetic stirring. Then, histidine (Sigma) (213 mg; 1.38 mmol) was added. When the conversion of Cy3 NHS to the corresponding amide reached the maximum value (determined by HPLC: 37.39 mol% incorporation of histidine in 24 h, at a ratio 4 histidine:1 Cy3 NHS ester), the mixture was cooled, filtered and washed with 2-methyl-2-butanol. The crude product was purified by semipreparative reverse-phase HPLC (Prontosil-AQ, 5 lm, 120 A, 250 · 8 mm column), and compounds were eluted with acetonitrile (20% for 5 min, followed by linear gradients to 45% in 5 min, to 50% in 7 min and to 100% in 2 min) in triethylammonium hydrogen carbonate buffer (0.01 M, pH 8.6) at a flow of 3 mL/min.

Fractions containing the product **(C)** (retention time 10.43 min, UV detection at 280 nm and 545 nm) were combined and dried by lyophilisation. The product (C) (101 mg; ~37%) was further dissolved in dimethylsulfoxide (DMSO) at a concentration up to 4 M and stored at -70°C until use. The water solubility of the compound was found to be >0.38 M at room temperature as determined by absorbance. The compound **(C)** had an extinction coefficient (ε) of 130810 l/mol·cm an its $\lambda_{max}$ 545 nm as determined in 10 mM Tris-HCl at pH 7.4 according to the method of C.R. Cantor and I. Tinoco, J. Mol. Biol. 13, 65 (1965). Mass spectrometry was used to confirm the structure. HRMS: calculated for $C_{37}O_9S_2N_5H_{44}$ [$M^+H^+$] was 766.2580, found 766.2597. As shown, the result was within of the calculated molecular mass.

*Reaction scheme:*

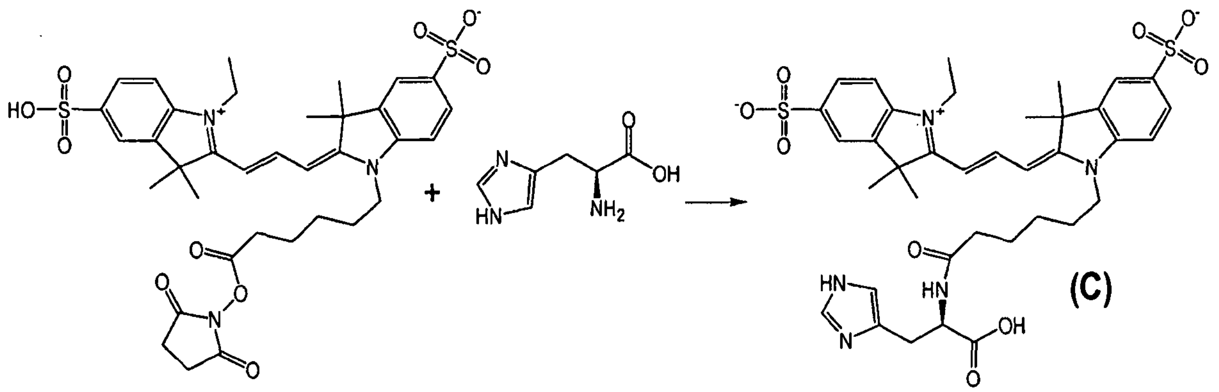

**(C)**

***4. Modification of his tagged cyanine with 4-amino-3-butanoate***

The 4-amino-3-butanoate was incorporated to **(C)** through the sulfonate. Two methods were used. First, sulfonyl chloride his tagged cyanine was firstly prepared from **(C)** according to J. Sokolowska-Gajda and H.S. Freeman, Dyes and Pigm 14, 35 (1990). This compound was used for a sulfonamide reaction (R-NH-$SO_2$-Cy3) with 4-amino-3-butanoate as described by Z. Wai et al., The proceeding of the 3rd International Conference on Functional Molecules 167 (2005). Briefly, to a solution of 4-amino-3-butanoate (60 mg; 0.58 mmol) in dry acetonitrile (10 ml), $K_2CO_3$ (1.0 g) was added and then a suspension of sulfonyl chloride Cy3-His (10 mg; 0.013 mmol) in dry DMSO (15 ml) was added dropwise. Then, the reaction mixture was stirred at 40°C for 4 hrs and maintained for further 2 hrs. Then, the reaction mixture was cooled to room temperature, poured into water and extracted with ethyl acetate EtOAc. The solid obtained was dried under vacuum to give the product **(D)** as a light red powder. After recrystallized from acetonitrile, solid sulfonamide was obtained with the yield 32.0% (3.6 mg). The product was further purified by HPLC as described below. Additionally, the sulphonamide reaction was also performed as described by C. Tsopelas et al., J Nucl Med 43, 1377 (2002) with small modifications. Briefly, 4-amino-3-butanoate (60 mg; 0.58 mmol) was added to a 10 ml 0.1 M $NaHCO_3$ (pH 8.0) solution containing sulfonate Cy3-His (10 mg; 0.013 mmol) and further incubated at 40°C for 600 min with swirling (100 rpm) in the dark. Under these conditions, 27 mol% incorporation of the dye was achieved. The product was recovered by semipreparative reverse phase HPLC analysis performed on a VPODS C-18 column (150 x 4.6 mm) at a flow rate of 1.0 mL/min for analysis, and PRC-ODS C-18 column (250 x 20 mm) at a flow rate of 10.0 mL/min for preparative scale. Detection was performed at 552 nm. HPLC solvents consist of water containing 0.1% TFA (solvent A) and acetonitrile containing 0.1% TFA (solvent B). Concentration of the pure fractions *in vacuo* afforded purified product **(D)** (2.7 mg; ~24%). In both cases, purified product was further dissolved in dimethylsulfoxide (DMSO) at a concentration up to 4 M and stored at -70°C until use. The mass spectrometry data was in agreement with the formation of one sulfonamide bond. HRMS: calculated for $C_{41}O_{10}S_2N_6H_{52}$ [$M^+H^+$] was 852.3186, found 852.3152.

*Reaction scheme:*

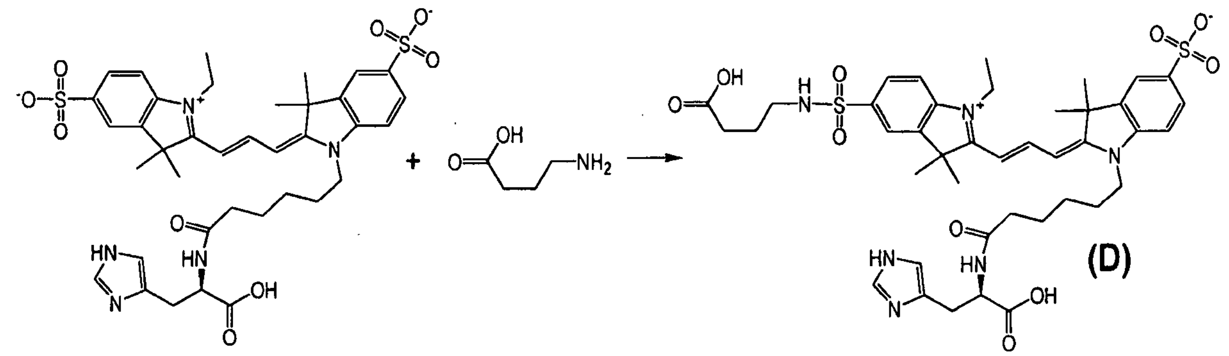


### *5. Modification of intermediate (D) with N,N-dimethylethanolamine*

Compound **(D)** was subjected to direct esterification with N,N-dimethylethanolamine using immobilized Lewatit lipase EL1 (D. Reyes-Duarte et al., Angew Chem Int Ed Engl 44, 7553 (2005)) from a cow rumen metagenome. Briefly, compound (D) (25 mg, 0.029 mmol) was dissolved in 5 mL of dimethylsulfoxide (DMSO) and 2-methyl-2-butanol was slowly added to a final volume of 25 mL. The biocatalyst (Lewatit lipase EL1, 2.5 g) and 3 Å molecular sieves (2.5 g) were then added and the suspension maintained 30 min at 40°C with magnetic stirring. Then, N,N-dimethylethanolamine (25 mg; 0.29 mmol) was added. When the conversion of the corresponding ester reached the maximum value (determined by HPLC: 53 mol% in 48 h), the mixture was cooled, filtered and washed with 2-methyl-2-butanol. The product was recovered by evaporating the tert-amyl alcohol. The crude product was redisolved in DMSO and further purified by semipreparative reverse-phase HPLC (Prontosil-AQ, 5 1m, 120 A, 250 · 8 mm column equipped with a Prontosil-AQ, 5 1m, 120 A, 33 · 8 mm pre-column, and compounds were eluted with acetonitrile (20% for 5 min, followed by linear gradients to 45% in 5 min, to 50% in 7 min and to 100% in 2 min) in triethylammonium hydrogen carbonate buffer (0.01 M, pH 8.6) at a flow of 3 mL/min. Fractions containing the product **(E)** (retention time 14.79 min, UV detection at 280 nm and 542 nm) were combined and dried by lyophilisation. The product **(E)** (13.5 mg; ~50%) was further dissolved in dimethylsulfoxide (DMSO) at a concentration up to 4 M and stored at -70°C until use. The water solubility of the compound was found to be approx. 0.24 M at room temperature as determined by absorbance. The compound **(E)** had an extinction coefficient ($\varepsilon$) of 112728 l/mol·cm an its $\lambda_{max}$ 545 nm as determined in 10 mM Tris-HCl at pH 7.4 according to the method of C.R. Cantor and I. Tinoco (*loc. cit.*). Mass spectrometry was used to confirm the structure. H HRMS: calculated for $C_{45}O_{10}S_2N_7H_{61}$ [$M^+H^+$] was 923.3921, found 923.3950. As shown, the result was within of the calculated molecular mass.

*Reaction scheme:*

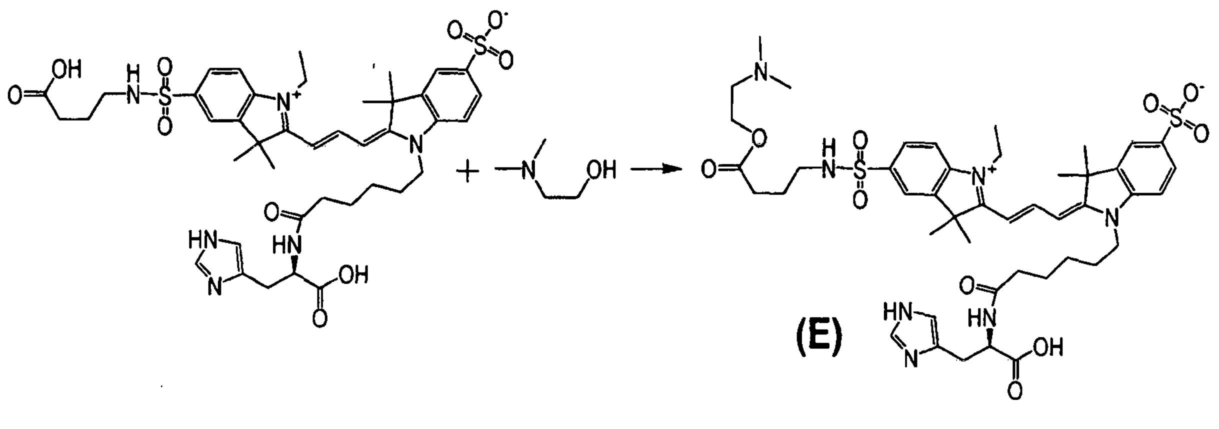


### *6. Metabolite library*

The primary synthetic challenge involved finding a reaction path from a functionalized core with the highest yield (>26-90% overall). The purified metabolites were characterized by HPLC and HRMS and were found to be 90% pure; yields were >20%. First, the comprehensive collection of metabolites was obtained. Table 1 shows a complete description of synthetic and purification methods and commercial suppliers. In case the metabolite was purified from cell extract of an organism, the chromatographic description of the method used for separation and the amount of metabolite per gram of extract are specifically shown. When a synthetic method was used, the exact and experimental masses (HRMS (MALDI)) are shown, together with the precise information of mass and molar quantities of reagents

used, reaction conditions, work-up procedure and isolated yield in mass as well as percentage. As can be seen in Table S1 enzymatic and chemical methods were used. Metabolites were reconstituted and diluted in PBS buffer, DMSO or a mixture of both, accordingly to their solubility properties, and stored in 384-well microtiter plates at -70°C until used.

*Reaction/purification/source scheme:*

Commercial sources

Substrates → M (metabolite)

Purification from cells

***7. Histidine and dye labelled metabolite library synthesis***

The metabolite array is constituted by thousands of molecules that are modified to link them to a fluorofore (i.e. Cy3, or possibly any other with similar characteristics) and a histidine molecule. Overall, there are a number of strategies to perform those addition reactions based on the linking and nature positions which are described below. Briefly, the methods include specific halogenation reactions by the action of a promiscuous halogenase, esterification or transesterification or amidation or transamidation reactions by the action of a promiscuous lipase and carbon-carbon bond formation in the presence of the proton sponge 1,8-bis-(dimethylamino)-napthalene. Figure 8 shows a schematic representation of each of the different general methods used to create the histidine and dye labelled metabolite library synthesis. In this figure a representative metabolite is shown.

Even though approx. 2500 molecules were modified to anchor them to a dye, few dozen of general procedures can be considered to perform the synthesis, named "synthetic method 1 to 30" described below. These general methods are based on the nature of position of the molecules to which the histidine moiety and the dye moiety are attached. Table 8 shows the linking position to which both components are attached to the metabolites. Below the general synthetic methods are described, each of them describing the individual and succesive steps. Further, general HPLC purification methods to separate the final dye labelled metabolites are described.

A number of abbreviations are used below: DMSO (dimethylsulfoxide); 2M2B (2-methyl-2-butanol); 1,8-BDN (1,8-bis-(dimethylamino)-naphthalene); $CH_3CN$ (acetonitrile), α-KG (α-ketoglutarate), HPLC (high pressure liquid chromatography), EL1-Lewatit (immobilized Lewatit lipase EL1 from a cow rumen metagenome), I-metabolite (Iodine containing metabolite; E (Cy3 intermediate containing histidine and linkers).

The source of enzymes used for synthetic purposes are as follow. EL1, Protein-engineered lipase isolated from cow rumen metagenome (Angewandte Chemie International Edition (2005) 44: 7553-7557); Dehalogenase: multifunctional α/β-hydrolase mined from a metagenome library of a microbial community in seawater contaminated with petroleum hydrocarbons, with a novel hydrolytic phenotype, namely the cleavage of both 'common' p-nitrophenyl (pNP) esters and haloalkanoates, and weak activity towards haloalkanes (Table 5; SEQ ID Nos. 57 to 59; *paper in preparation*) ; Halogenase: this enzyme corresponds to the same dehalogenase described above but it is able to perform halogenation reactions in organic media. The promiscuity of the enzyme can be also seen in the capacity to perform two different reactions. In the presence of the cofactor α-ketoglutarate the enzyme is able to activate non-activated alkyl groups and in the presence of NADH is able to halogenate activate molecules containing alkenyl groups.

Below we described the general methods which consist in three steps: first, the incorporation of iodide to the metabolite; second, the incoprotation oh histidine to the halogenated metabolite; third, the incorporation of the dye to the previous intermediate. While the first and second methods may differs among the different methods, the third remains equal:

*Step 3. Formation of Cy3-labeled metabolite.* The corresponding labeled quaternary ammonium metabolite were obtained in the presence of 1,8-BDN as described by R.A. Kaufman *et al.* (*loc. cit.*) and F. Mazzetti, R. M. Lemmon, J Org Chem 22, 228 (1957) with small modifications. Briefly, the,general method for the synthesis of quaternary amines is as follows. Reaction mixture (2 ml) contains histidine tagged I-metabolite (0.078 mmol), 0.78 mmol of **(E)** and 1,8-BDN at a final concentration of 100 mM in DMSO. The temperature was controlled at 32°C. After incubation (range from 11.52 to 424 min) the product was recovered by evaporating the $CH_3CN$. The labeled product was purified by semi-preparative reverse-phase HPLC. The purified metabolite was found to be 98% pure.

Therefore, a full description of steps 1 and 2 is only given below. Reference is made to Figure 8, which shows exemplary reaction schemes for each of the following 26 synthetic methods, and Figure 9, which shows the exemplary metabolite used in the respective synthetic methods, wherein the position, to which histidine is linked, is marked by a black arrow, and that, to which the dye is linked, is marked by a grey arrow.

**Synthetic method 1:** this method is designed to perform direct halogenation to two sp3 carbon atoms (e.g. a carbon atom of a saturated hydrocarbon bond, e.g. a terminal methyl carbon atom or a methylene carbon atom, e.g.

within a chain or ring) and further incorporation of His (black arrow in Figure 9) and Cy3 (grey arrow in Figure 9) component to those sp3 carbon atoms - i.e. both components to two different sp3 carbon atoms.

Example of metabolite subjected to this synthetic protocol is the metabolite nr. 1 shown in Table 1 and Figures 8 and 9, wherein Hys and Cy3 are linked to two different sp3 carbon atoms of an alkyl group.

*1. Formation of I-metabolite.* The iodide halogenation of metabolite was performed via α-KG. The halogenation reaction contained the metabolite (2.1 mM), KI (5.0 mM), and halogenase (32 μM) in phosphate buffer (20 mM, pH 7.8) plus 2 mM α-KG and up to 5% DMSO, in a final volume of 0.5 ml. After 10 h of incubation at 37°C, the reaction mixture was transferred to a 1.5 ml filtration unit (3000 NMWL membrane cut-off) to remove the enzyme, and the product was purified by semi preparative HPLC. I-metabolite was reconstituted and diluted in DMSO and stored at -70°C until used at a concentration of 10 μg/ml. HRMS data clearly show that the enzyme incorporated two "I" per molecule.

*2. Incorporation of histidine.* I-metabolite was further functionalized via incorporation of a histidine tag. The incorporation of histidine was performed in the presence of the non-nucleophilic base and proton sponge 1,8-BDN as described by R.A. Kaufman et al. (*loc. cit.*) and F. Mazzetti and R.M. Lemmon (*loc. cit.*) with small modifications.

Briefly, to a solution of I-metabolite (concentration range from 0.1 to 2.3 mmol), histidine (concentration range from 0.44 to 1 mmol), and 1,8-BDN at a final concentration of 10 mM were added, in $CH_3CN$ (10 ml final volume). The temperature was controlled at 32°C. After incubation (range from 11.52 to 424 min) the product was recovered by evaporating the $CH_3CN$. The corresponding product was purified by semi-preparative HPLC. The purified metabolite was found to be 98% pure.

**Synthetic method 2:** this method is designed to perform direct halogenation to two sp2 carbon atoms (e.g. a carbon atom of an aromatic carbon bond or a carbon atom of an unsaturated hydrocarbon bond, e.g. terminal, or within a chain or ring) and further incorporation of His (black arrow in Figure 9) and Cy3 (grey arrow in Figure 9) component to those groups - i.e. both components to two different sp2 carbon atoms.

Example of metabolite subjected to this synthetic protocol is the metabolite nr. 7 shown in Table 1 and Figures 8 and 9, wherein His and Cy3 are linked to two differnt sp2 carbon atoms of an aryl group.

*1. Formation* of *I-metabolite*. The iodide halogenation of metabolite was performed via $I^-$ as follows. The general halogenation procedure via cofactor is as follows. The reaction mixtures were incubated at 37°C with metabolite (0.080 mmol), KI (75 mM), 2 mM NADH, and halogenase (4 mg/ml, 100 μL) in phosphate buffer (20 mM, pH = 7.8), containing up to 20% DMSO (to increase metabolite solubility), in a final volume of 0.5 ml. After 24 hour of incubation, reaction product(s) were separated by semi-preparative HPLC. I-metabolite was reconstituted and diluted in DMSO and stored at -70°C until used at a concentration of 10 μg/ml. HRMS data clearly show that the enzyme incorporated two "I" per molecule.

*2. Incorporation of histidine.* I-metabolite was further functionalized via incorporation of a histidine tag. The incorporation of histidine was performed in the presence of the non-nucleophilic base and proton sponge 1,8-BDN as described by R.A. Kaufman *et al*. (*loc. cit.*) and F. Mazzetti and R.M. Lemmon (*loc. cit.*) with small modifications. Briefly, to a solution of I-metabolite (concentration range from 0.1 to 2.3 mmol), histidine (concentration range from 0.44 to 1 mmol), and 1,8-BDN at a final concentration of 10 mM were added, in $CH_3CN$ (10 ml final volume). The temperature was controlled at 32°C. After incubation (range from 11.52 to 424 min) the product was recovered by evaporating the $CH_3CN$. The corresponding product was purified by semi-preparative HPLC. The purified metabolite was found to be 98% pure.

**Synthetic method 3**: this method is designed to perform direct halogenation to both a sp3 carbon atom (e.g. a carbon atom of a saturated hydrocarbon bond, e.g. a terminal methyl carbon atom or a methylene carbon atom, e.g. within a chain or ring) and a sp2 carbon atom (e.g. a carbon atom of an aromatic carbon bond or a carbon atom of an unsaturated hydrocarbon bond, e.g. terminal, or within a chain or ring) in the same metablite and further incorporation of His (black arrow in Figure 9) and Cy3 (grey arrow in Figure 9) component to those groups - i.e. one component to a sp3 carbon atom and one component to a sp2 carbon atom.

Example of metabolite subjected to this synthetic protocol is the metabolite nr. 8 shown in Table 1 and Figures 8 and 9, wherein one of His and Cy3 is linked to a sp3 carbon atom of an methyl group, while the other is linked to an aryl carbon atom.

*1. Formation* of *I-metabolite*. The iodide halogenation of metabolite was performed via $I^-$ as follows. The general halogenation procedure via cofactor is as follows. The reaction mixtures were incubated at 37°C with metabolite (0.080 mmol), KI (75 mM), 2 mM NADH, and halogenase (4 mg/ml, 100 μL) in phosphate buffer (20 mM, pH = 7.8), containing up to 20% DMSO (to increase metabolite solubility), in a final volume of 0.5 ml. After 24 hour of incubation,

reaction product(s) were separated by semi-preparative HPLC. I-metabolite was reconstituted and diluted in DMSO and stored at -70°C until used at a concentration of 10 μg/ml. HRMS data clearly show that the enzyme incorporated one "I" per molecule. Further, a second iodide was incorporated via α-KG. The halogenation reaction contained the metabolite (2.1 mM), KI (5.0 mM), and halogenase (32 μM) in phosphate buffer (20 mM, pH 7.8) plus 2 mM α-KG and up to 5% DMSO, in a final volume of 0.5 ml. After 10 h of incubation at 37°C, the reaction mixture was transferred to a 1.5 ml filtration unit (3000 NMWL membrane cut-off) to remove the enzyme, and the product was purified by semi preparative HPLC. I-metabolite was reconstituted and diluted in DMSO and stored at -70°C until used at a concentration of 10 μg/ml. HRMS data clearly show that the enzyme incorporated one "I" per molecule in this step, and two "I" per molecule at the end of the two-steps halogenation process.

*2. Incorporation of histidine.* I-metabolite was further functionalized via incorporation of a histidine tag. The incorporation of histidine was performed in the presence of the non-nucleophilic base and proton sponge 1,8-BDN as described by R.A. Kaufman *et. al. (loc. cit.*) and F. Mazzetti and R.M. Lemmon (*loc. cit.*) with small modifications. Briefly, to a solution of I-metabolite (concentration range from 0.1 to 2.3 mmol), histidine (concentration range from 0.44 to 1 mmol), and 1,8-BDN at a final concentration of 10 mM were added were added, in $CH_3CN$ (10 ml final volume). The temperature was controlled at 32°C. After incubation (range from 11.52 to 424 min) the product was recovered by evaporating the $CH_3CN$. The corresponding product was purified by semi-preparative HPLC. The purified metabolite was found to be 98% pure.

**Synthetic method 4**: this method is designed to perform direct halogenation to a sp3 carbon atom (e.g. a carbon atom of a saturated hydrocarbon bond, e.g. a terminal methyl carbon atom or a methylene carbon atom, e.g. within a chain or ring) to link it to Cy3 (grey arrow in Figure 9) component and incorporation of His (black arrow in Figure 9) through esterification with an OH group of the metabolite - i.e. one component to a sp3 carbon atom and one component to an OH group.

Example of metabolite subjected to this synthetic protocol is the metabolite nr. 36 shown in Table 1 and Figures 8 and 9.

*1. Formation of I-metabolite.* The iodide halogenation of metabolite was performed via α-KG. The halogenation reaction contained the metabolite (2.1 mM), KI (5.0 mM), and halogenase (32 μM) in phosphate buffer (20 mM, pH 7.8) plus 2 mM α-KG and up to 5% DMSO, in a final volume of 0.5 ml. After 10 h of incubation at 37°C, the reaction mixture was transferred to a 1.5 ml filtration unit (3000 NMWL membrane cut-off) to remove the enzyme, and the product was purified by semi-preparative HPLC. I-metabolite was reconstituted and diluted in DMSO and stored at -70°C until used at a concentration of 10 μg/ml. HRMS data clearly show that the enzyme incorporated one "I" per molecule.

*2. Incorporation of histidine.* I-metabolite was further functionalized via incorporation of a histidine tag. A histidine molecule was incorporated by enzymatic esterification of the previous intermediate and EL1-Lewatit. Solvents were dried over 3 Å molecular sieves for 24 h prior to use. Briefly, metabolite (0.344 mmol) was dissolved in 5 mL of DMSO and 2M2B was slowly added to a final volume of 25 mL. The biocatalyst (Lewatit lipase EL1, 2.5 g) and 3 Å molecular sieves (2.5 g) were then added and the suspension maintained 30 min at 40°C with magnetic stirring. Then, histidine (1.38 mmol) was added. When the conversion to the corresponding ester reached the maximum value, the mixture was cooled, filtered and washed with 2M2B. The product was recovered by evaporating the tert-amyl alcohol and the crude product was purified by semi-preparative HPLC. Fractions containing the product were combined and dried by lyophilisation. I-metabolite was reconstituted and diluted in DMSO and stored at -70°C until used at a concentration of 10 μg/ml.

**Synthetic method 5**: this method is designed to perform direct esterification through an -OH group and further halogenation to a sp3 carbon atom (e.g. a carbon atom of a saturated hydrocarbon bond, e.g. a terminal methyl carbon atom or a methylene carbon atom, e.g. within a chain or ring) to link it to Cy3 (grey arrow in Figure 9) component and His (black arrow in Figure 9) - i.e. both components to two different OH groups.

Example of metabolite subjected to this synthetic protocol is the metabolite nr. 132 shown in Table 1 and Figures 8 and 9.

*1. Formation of I-metabolite.* The metabolite was subjected to transesterification with vinyl acetate using ELI-Lewatit. Briefly, metabolite (0.029 mmol) was dissolved in 5 mL of DMSO and 2M2B was slowly added to a final volume of 25 mL. The biocatalyst (Lewatit lipase EL1, 2.5 g) and 3 Å molecular sieves (2.5 g) were then added and the suspension maintained 30 min at 40°C with magnetic stirring. Then, vinyl acetate (0.29 mmol) was added. When the conversion of the corresponding ester reached the maximum value, the mixture was cooled, filtered and washed with 2M2B. The product was recovered by evaporating the tert-amyl alcohol. The crude product was further purified by semi-preparative HPLC. The product so obtained was further subjected to iodide halogenation via α-KG. The

halogenation reaction contained the metabolite (2.1 mM), KI (5.0 mM), and halogenase (32 μM) in phosphate buffer (20 mM, pH 7.8) plus 2 mM α-KG and up to 5% DMSO, in a final volume of 0.5 ml. After 10 h of incubation at 37°C, the reaction mixture was transferred to a 1.5 ml filtration unit (3000 NMWL membrane cut-off) to remove the enzyme, and the product was purified by semi-preparative HPLC. I-metabolite was reconstituted and diluted in DMSO and stored at -70°C until used at a concentration of 10 μg/ml.

*2. Incorporation of histidine*. I-metabolite was further functionalized via incorporation of a histidine tag. A histidine molecule was incorporated by enzymatic esterification of the previous intermediate and EL1-Lewatit. Solvents were dried over 3 Å molecular sieves for 24 h prior to use. Briefly, metabolite (0.344 mmol) was dissolved in 5 mL of DMSO and 2M2B was slowly added to a final volume of 25 mL. The biocatalyst (Lewatit lipase EL1, 2.5 g) and 3 Å molecular sieves (2.5 g) were then added and the suspension maintained 30 min at 40°C with magnetic stirring. Then, histidine (1.38 mmol) was added. When the conversion to the corresponding ester reached the maximum value, the mixture was cooled, filtered and washed with 2M2B. The product was recovered by evaporating the tert-amyl alcohol and the crude product was purified by semi-preparative HPLC. Fractions containing the product were combined and dried by lyophilisation. I-metabolite was reconstituted and diluted in DMSO and stored at -70°C until used at a concentration of 10 μg/ml.

**Synthetic method 6**: this method is designed to perform direct halogenation to a sp3 carbon atom (e.g. a carbon atom of a saturated hydrocarbon bond, e.g. a terminal methyl carbon atom or a methylene carbon atom, e.g. within a chain or ring) to link it to Cy3 (grey arrow in Figure 9) component and incorporation of His (black arrow in Figure 9) through an $NH_2$ group of the metabolite - i.e. one component to a sp3 carbon atom and one component to an $NH_2$ group.

Example of metabolite subjected to this synthetic protocol is the metabolite nr. 154 shown in Table 1 and Figures 8 and 9.

*1. Formation of I-metabolite.* The iodide halogenation of metabolite was performed via α-KG. The halogenation reaction contained the metabolite (2.1 mM), KI (5.0 mM), and halogenase (32 μM) in phosphate buffer (20 mM, pH 7.8) plus 2 mM α-KG and up to 5% DMSO, in a final volume of 0.5 ml. After 10 h of incubation at 37°C, the reaction mixture was transferred to a 1.5 ml filtration unit (3000 NMWL membrane cut-off) to remove the enzyme, and the product was purified by semi-preparative HPLC. I-metabolite was reconstituted and diluted in DMSO and stored at -70°C until used at a concentration of 10 μg/ml. HRMS data clearly show that the enzyme incorporated one "I" per molecule.

*2. Incorporation of histidine.* I-metabolite was further functionalized via incorporation of a histidine tag. A histidine molecule was incorporated by enzymatic amidation of the previous intermediate and EL1-Lewatit. Solvents were dried over 3 Å molecular sieves for 24 h prior to use. Briefly, metabolite (0.344 mmol) was dissolved in 5 mL of DMSO and 2M2B was slowly added to a final volume of 25 mL. The biocatalyst (Lewatit lipase EL1, 2.5 g) and 3 Å molecular sieves (2.5 g) were then added and the suspension maintained 30 min at 40°C with magnetic stirring. Then, histidine (1.38 mmol) was added. When the conversion to the corresponding amide reached the maximum value, the mixture was cooled, filtered and washed with 2M2B. The crude product was further purified by semi-preparative HPLC. I-metabolite was reconstituted and diluted in DMSO and stored at -70°C until used at a concentration of 10 μg/ml.

**Synthetic method 7**: this method is designed to perform direct esterification through an -OH group and further halogenation to a sp3 carbon atom to link it to Cy3 (grey arrow in Figure 9) component and incorporation of His (black arrow in Figure 9) through an $NH_2$ group of the metabolite - i.e. one component to an $NH_2$ group and one component to an OH group.

Example of metabolite subjected to this synthetic protocol is the metabolite nr. 196 shown in Table 1 and Figures 8 and 9.

*1. Formation of I-metabolite.* The metabolite was subjected to transesterification with vinyl acetate using EL1-Lewatit. Briefly, metabolite (0.029 mmol) was dissolved in 5 mL of DMSO and 2M2B was slowly added to a final volume of 25 mL. The biocatalyst (Lewatit lipase EL1, 2.5 g) and 3 Å molecular sieves (2.5 g) were then added and the suspension maintained 30 min at 40°C with magnetic stirring. Then, vinyl acetate (0.29 mmol) was added. When the conversion of the corresponding ester reached the maximum value, the mixture was cooled, filtered and washed with 2M2B. The product was recovered by evaporating the tert-amyl alcohol. The crude product was further purified by semi-preparative HPLC. The product so obtained was further subjected to iodide halogenation via α-KG. The halogenation reaction contained the metabolite (2.1 mM), KI (5.0 mM), and halogenase (32 μM) in phosphate buffer (20 mM, pH 7.8) plus 2 mM α-KG and up to 5% DMSO, in a final volume of 0.5 ml. After 10 h of incubation at 37°C, the reaction mixture was transferred to a 1.5 ml filtration unit (3000 NMWL membrane cut-off) to remove the enzyme, and the product was purified by semi-preparative HPLC. I-metabolite was reconstituted and diluted in DMSO and

stored at -70°C until used at a concentration of 10 μg/ml.

*2. Incorporation of histidine.* I-metabolite was further functionalized via incorporation of a histidine tag. A histidine molecule was incorporated by enzymatic amidation of the previous intermediate and EL1-Lewatit. Solvents were dried over 3 Å molecular sieves for 24 h prior to use. Briefly, metabolite (0.344 mmol) was dissolved in 5 mL of DMSO and 2M2B was slowly added to a final volume of 25 mL. The biocatalyst (Lewatit lipase EL1, 2.5 g) and 3 Å molecular sieves (2.5 g) were then added and the suspension maintained 30 min at 40°C with magnetic stirring. Then, histidine (1.38 mmol) was added. When the conversion to the corresponding amide reached the maximum value, the mixture was cooled, filtered and washed with 2M2B. The crude product was further purified by semi-preparative HPLC. I-metabolite was reconstituted and diluted in DMSO and stored at -70°C until used at a concentration of 10 μg/ml.

**Synthetic method 8**: this method is designed to perform partial dehalogenation in (fully) halogenated aliphatic compounds, followed by halogenation (iodization) to two different sp3 carbon atoms (e.g. a carbon atom of a saturated hydrocarbon bond, e.g. a terminal methyl carbon atom or a methylene carbon atom, e.g. within a chain or ring) and further incorporation of His (black arrow in Figure 9) and Cy3 (grey arrow in Figure 9) component to those sp3 carbon atoms - i.e. both components to two different sp3 carbon atoms after a three-step dehalogenation, esterification and halogenation.

Example of metabolite subjected to this synthetic protocol is the metabolite nr. 1692 shown in Table 1 and Figures 8 and 9.

*1. Formation of I-metabolite.* The iodide halogenation of metabolite was performed via α-KG prior selective dehalogenation. First, the metabolite (100 μl of a 100 mM solution in MeOH) was added to 900 μl in phosphate buffer (20 mM, pH 7.8) and dehalogenase (32 μM). Reaction was allowed to proceed at 40°C for 5 hours. Then the reaction mixture was transferred to a 1.5 ml filtration unit (3000 NMWL membrane cut-off) to remove the enzyme, and the product (mixture of partial dehalogenated products) was concentrated by liophilization. The purified intermediate (95% pure) was used for a subsequent iodization reaction as follows. The metabolite was subjected to transesterification with vinyl acetate using EL1-Lewatit. Briefly, metabolite (0.029 mmol) was dissolved in 5 mL of DMSO and 2M2B was slowly added to a final volume of 25 mL. The biocatalyst (Lewatit lipase EL1, 2.5 g) and 3 Å molecular sieves (2.5 g) were then added and the suspension maintained 30 min at 40°C with magnetic stirring. Then, vinyl acetate (0.29 mmol) was added. When the conversion of the corresponding ester reached the maximum value, the mixture was cooled, filtered and washed with 2M2B. The product was recovered by evaporating the tert-amyl alcohol. The crude product was further purified by semi-preparative HPLC. The product so obtained was further subjected to iodide halogenation via α-KG. The halogenation reaction contained the metabolite (2.1 mM), KI (5.0 mM), and halogenase (32 μM) in phosphate buffer (20 mM, pH 7.8) plus 2 mM α-KG and up to 5% DMSO, in a final volume of 0.5 ml. After 10 h of incubation at 37°C, the reaction mixture was transferred to a 1.5 ml filtration unit (3000 NMWL membrane cut-off) to remove the enzyme, and the product was purified by semi-preparative HPLC. I-metabolite was reconstituted and diluted in DMSO and stored at -70°C until used at a concentration of 10 μg/ml. HRMS data clearly show that the enzyme incorporated two "I" per molecule.

*2. Incorporation of histidine.* I-metabolite was further functionalized via incorporation of a histidine tag. The incorporation of histidine was performed in the presence of the non-nucleophilic base and proton sponge 1,8-BDN as described by R.A. Kaufman *et al. (loc. cit.)* and F. Mazzetti and R.M. Lemmon *(loc. cit.)* with small modifications. Briefly, to a solution of I-metabolite (concentration range from 0.1 to 2.3 mmol), histidine (concentration range from 0.44 to 1 mmol), and 1,8-BDN at a final concentration of 10 mM were added were added, in $CH_3CN$ (10 ml final volume). The temperature was controlled at 32°C. After incubation (range from 11.52 to 424 min) the product was recovered by evaporating the $CH_3CN$. The corresponding product was purified by semi-preparative HPLC. The purified metabolite was found to be 98% pure.

**Synthetic method 9**: this method is designed to perform direct halogenation to a sp2 carbon atom (e.g. a carbon atom of an aromatic carbon bond or a carbon atom of an unsaturated hydrocarbon bond, e.g. terminal, or within a chain or ring) and further incorporation of Cy3 (grey arrow in Figure 9) component to this group and incorporation of His (black arrow in Figure 9) through an $NH_2$ group of the metabolite.

Example of metabolite subjected to this synthetic protocol is the metabolite nr. 306 shown in Table 1 and Figures 8 and 9.

*1. Formation of I-metabolite*. The iodide halogenation of metabolite was performed via $I^-$ as follows. The general halogenation procedure via cofactor is as follows. The reaction mixtures were incubated at 37°C with metabolite (0.080 mmol), KI (75 mM), 2 mM NADH, and halogenase (4 mg/ml, 100 μL) in phosphate buffer (20 mM, pH = 7.8), containing up to 20% DMSO (to increase metabolite solubility), in a final volume of 0.5 ml. After 24 hour of incubation,

reaction product(s) were separated by semi-preparative HPLC. I-metabolite was reconstituted and diluted in DMSO and stored at -70°C until used at a concentration of 10 μg/ml. HRMS data clearly show that the enzyme incorporated one "I" per molecule after purification.

2. *Incorporation of histidine*. I-metabolite was further functionalized via incorporation of a histidine tag. A histidine molecule was incorporated by enzymatic amidation of the previous intermediate and EL1-Lewatit. Solvents were dried over 3 Å molecular sieves for 24 h prior to use. Briefly, metabolite (0.344 mmol) was dissolved in 5 mL of DMSO and 2M2B was slowly added to a final volume of 25 mL. The biocatalyst (Lewatit lipase EL1, 2.5 g) and 3 Å molecular sieves (2.5 g) were then added and the suspension maintained 30 min at 40°C with magnetic stirring. Then, histidine (1.38 mmol) was added. when the conversion to the corresponding amide reached the maximum value, the mixture was cooled, filtered and washed with 2M2B. The product was recovered by evaporating the tert-amyl alcohol and the crude product was purified by semi-preparative HPLC. Fractions containing the product were combined and dried by lyophilisation. I-metabolite was reconstituted and diluted in DMSO and stored at -70°C until used at a concentration of 10 μg/ml.

**Synthetic method 10**: this method is designed to perform direct esterification through a -COOH group and further halogenation to a sp3 carbon atom to link it to Cy3 (grey arrow in Figure 9) component and incorporation of His (black arrow in Figure 9) through halogenation to a sp3 carbon atom (e.g. a carbon atom of a saturated hydrocarbon bond, e.g. a terminal methyl carbon atom or a methylene carbon atom, e.g. within a chain or ring).

Example of metabolite subjected to this synthetic protocol is the metabolite nr. 327 shown in Table 1 and Figures 8 and 9.

1. *Formation* of *I-metabolite.* The metabolite was subjected to direct esterification with ethanol using EL1-Lewatit. Briefly, metabolite (0.029 mmol) was dissolved in 5 mL of DMSO and 2M2B was slowly added to a final volume of 25 mL. The biocatalyst (Lewatit lipase EL1, 2.5 g) and 3 Å molecular sieves (2.5 g) were then added and the suspension maintained 30 min at 40°C with magnetic stirring. Then, ethanol (0.29 mmol) was added. When the conversion of the corresponding ester reached the maximum value, the mixture was cooled, filtered and washed with 2M2B. The product was recovered by evaporating the tert-amyl alcohol. The crude product was further purified by semi-preparative HPLC. The product so obtained was further subjected to iodide halogenation via α-KG. The halogenation reaction contained the metabolite (2.1 mM), KI (5.0 mM), and halogenase (32 μM) in phosphate buffer (20 mM, pH 7.8) plus 2 mM α-KG and up to 5% DMSO, in a final volume of 0.5 ml. After 10 h of incubation at 37°C, the reaction mixture was transferred to a 1.5 ml filtration unit (3000 NMWL membrane cut-off) to remove the enzyme, and the product was purified by semi-preparative HPLC. I-metabolite was reconstituted and diluted in DMSO and stored at -70°C until used at a concentration of 10 μg/ml. Following the two-step reaction process, HRMS data clearly show that the enzyme incorporated two "I" per molecule after purification.

2. *Incorporation of histidine.* I-metabolite was further functionalized via incorporation of a histidine tag. The incorporation of histidine was performed in the presence of the non-nucleophilic base and proton sponge 1,8-BDN as described by R.A. Kaufman *et al*. *(loc. cit.*) and F. Mazzetti and R.M. Lemmon (*loc*. *cit.*) with small modifications. Briefly, to a solution of I-metabolite (concentration range from 0.1 to 2.3 mmol), histidine (concentration range from 0.44 to 1 mmol), and 1,8-BDN at a final concentration of 10 mM were added, in $CH_3CN$ (10 ml final volume). The temperature was controlled at 32°C. After incubation (range from 11.52 to 424 min) the product was recovered by evaporating the $CH_3CN$. The corresponding product was purified by semi-preparative HPLC. The purified metabolite was found to be 98% pure.

**Synthetic method 11**: this method is designed to perform direct esterification through a -COOH group and further halogenation to a sp3 carbon atom to link it to Cy3 (grey arrow in Figure 9) component and incorporation of His (black arrow in Figure 9) through an··OH group of the metabolite.

Example of metabolite subjected to this synthetic protocol is the metabolite nr. shown in Table 1 and Figures 8 and 9.

1. *Formation of I-metabolite.* The metabolite was subjected to direct esterification with ethanol using EL1-Lewatit. Briefly, metabolite (0.029 mmol) was dissolved in 5 mL of DMSO and 2M2B was slowly added to a final volume of 25 mL. The biocatalyst (Lewatit lipase EL1, 2.5 g) and 3 Å molecular sieves (2.5 g) were then added and the suspension maintained 30 min at 40°C with magnetic stirring. Then, ethanol (0.29 mmol) was added. When the conversion of the corresponding ester reached the maximum value, the mixture was cooled, filtered and washed with 2M2B. The product was recovered by evaporating the tert-amyl alcohol. The crude product was further purified by semi-preparative HPLC. The product so obtained was further subjected to iodide halogenation via α-KG. The halogenation reaction contained the metabolite (2.1 mM), KI (5.0 mM), and halogenase (32 μM) in phosphate buffer (20 mM, pH 7.8) plus 2 mM α-KG and up to 5% DMSO, in a final volume of 0.5 ml. After 10 h of incubation at 37°C,

the reaction mixture was transferred to a 1.5 ml filtration unit (3000 NMWL membrane cut-off) to remove the enzyme, and the product was purified by semi-preparative HPLC. I-metabolite was reconstituted and diluted in DMSO and stored at -70°C until used at a concentration of 10 μg/ml. HRMS data clearly show that the enzyme incorporated one "I" per molecule

*2. Incorporation of histidine.* I-metabolite was further functionalized via incorporation of a histidine tag. A histidine molecule was incorporated by enzymatic esterification of the previous intermediate and EL1-Lewatit. Solvents were dried over 3 Å molecular sieves for 24 h prior to use. Briefly, metabolite (0.344 mmol) was dissolved in 5 mL of DMSO and 2M2B was slowly added to a final volume of 25 mL. The biocatalyst (Lewatit lipase EL1, 2.5 g) and 3 Å molecular sieves (2.5 g) were then added and the suspension maintained 30 min at 40°C with magnetic stirring. Then, histidine (1.38 mmol) was added. When the conversion to the corresponding ester reached the maximum value, the mixture was cooled, filtered and washed with 2M2B. The crude product was further purified by semi-preparative HPLC. I-metabolite was reconstituted and diluted in DMSO and stored at -70°C until used at a concentration of 10 μg/ml.

**Synthetic method 12:** this method is designed to perform direct esterification through two -COOH groups and further halogenation to a sp3 carbon atom to link them to Cy3 (grey arrow in Figure 9) and His (black arrow in Figure 9) components.

Example of metabolite subjected to this synthetic protocol is the metabolite nr. 397 shown in Table 1 and Figures 8 and 9.

*1. Formation* of I-metabolite. The metabolite was subjected to direct esterification with ethanol using EL1-Lewatit. Briefly, metabolite (0.029 mmol) was dissolved in 5 mL of DMSO and 2M2B was slowly added to a final volume of 25 mL. The biocatalyst (Lewatit lipase EL1, 2.5 g) and 3 Å molecular sieves (2.5 g) were then added and the suspension maintained 30 min at 40°C with magnetic stirring. Then, ethanol (0.29 mmol) was added. When the conversion of the corresponding ester reached the maximum value, the mixture was cooled, filtered and washed with 2M2B. The product was recovered by evaporating the tert-amyl alcohol. The crude product was further purified by semi-preparative HPLC. The product so obtained was further subjected to iodide halogenation via α-KG. The halogenation reaction contained the metabolite (2.1 mM), KI (5.0 mM), and halogenase (32 μM) in phosphate buffer (20 mM, pH 7.8) plus 2 mM α-KG and up to 5% DMSO, in a final volume of 0.5 ml. After 10 h of incubation at 37°C, the reaction mixture was transferred to a 1.5 ml filtration unit (3000 NMWL membrane cut-off) to remove the enzyme, and the product was purified by semi-preparative HPLC. I-metabolite was reconstituted and diluted in DMSO and stored at -70°C until used at a concentration of 10 μg/ml. HRMS data clearly show that the enzyme incorporated two "I" per molecule.

*2. Incorporation of histidine*. I-metabolite was further functionalized via incorporation of a histidine tag. The incorporation of histidine was performed in the presence of the non-nucleophilic base and proton sponge 1,8-BDN as described by R.A. Kaufman et al. (*loc. cit.*) and F. Mazzetti and R.M. Lemmon (*loc. cit.*) with small modifications. Briefly, to a solution of I-metabolite (concentration range from 0.1 to 2.3 mmol), histidine (concentration range from 0.44 to 1 mmol), and 1,8-BDN at a final concentration of 10 mM were added, in $CH_3CN$ (10 ml final volume). The temperature was controlled at 32°C. After incubation (range from 11.52 to 424 min) the product was recovered by evaporating the $CH_3CN$. The corresponding product was purified by semi-preparative HPLC. The purified metabolite was found to be 98% pure.

**Synthetic method 13:** this method is designed to perform direct halogenation to a sp2 carbon atom (e.g. a carbon atom of an aromatic carbon bond or a carbon atom of an unsaturated hydrocarbon bond, e.g. terminal, or within a chain or ring) and further incorporation of Cy3 (grey arrow in Figure 9) component and direct amidation through a -COOH group to incorporate the His (black arrow in Figure 9) component.

Example of metabolite subjected to this synthetic protocol is the metabolite nr. 398 shown in Table 1 and Figures 8 and 9.

*1. Formation of I-metabolite.* The iodide halogenation of metabolite was performed via $I^-$ as follows. The general halogenation procedure via cofactor is as follows. The reaction mixtures were incubated at 37°C with metabolite (0.080 mmol), KI (75 mM), 2 mM NADH, and halogenase (4 mg/ml, 100 μL) in phosphate buffer (20 mM, pH = 7.8), containing up to 20% DMSO (to increase metabolite solubility), in a final volume of 0.5 ml. After 24 hour of incubation, reaction product(s) were separated by semi-preparative HPLC. I-metabolite was reconstituted and diluted in DMSO and stored at -70°C until used at a concentration of 10 μg/ml. HRMS data clearly show that the enzyme incorporated one "I" per molecule.

*2. Incorporation of histidine*. I-metabolite was further functionalized via incorporation of a histidine tag. A histidine molecule was incorporated by enzymatic amidation of the previous intermediate and EL1-Lewatit. Solvents were

dried over 3 Å molecular sieves for 24 h prior to use. Briefly, metabolite (0.344 mmol) was dissolved in 5 mL of DMSO and 2M2B was slowly added to a final volume of 25 mL. The biocatalyst (Lewatit lipase EL1, 2.5 g) and 3 Å molecular sieves (2.5 g) were then added and the suspension maintained 30 min at 40°C with magnetic stirring. Then, histidine (1.38 mmol) was added. When the conversion to the corresponding amide reached the maximum value, the mixture was cooled, filtered and washed with 2M2B. The product was recovered by evaporating the tert-amyl alcohol and the crude product was purified by semi-preparative HPLC. Fractions containing the product were combined and dried by lyophilisation. I-metabolite was reconstituted and diluted in DMSO and stored at -70°C until used at a concentration of 10 μg/ml.

**Synthetic method 14:** this method is designed to perform direct esterification through a -COOH group and further halogenation to a sp3 carbon atom to link it to Cy3 (grey arrow in Figure 9) and direct amidation through a $NH_2$ group to incorporate the His (black arrow in Figure 9) component.

Example of metabolite subjected to this synthetic protocol is the metabolite nr. 860 shown in Table 1 and Figures 8 and 9.

1. *Formation of I-metabolite*. The metabolite was subjected to direct esterification with ethanol using EL1-Lewatit. Briefly, metabolite (0.029 mmol) was dissolved in 5 mL of DMSO and 2M2B was slowly added to a final volume of 25 mL. The biocatalyst (Lewatit lipase EL1, 2.5 g) and 3 Å molecular sieves (2.5 g) were then added and the suspension maintained 30 min at 40°C with magnetic stirring. Then, ethanol (0.29 mmol) was added. When the conversion of the corresponding ester reached the maximum value, the mixture was cooled, filtered and washed with 2M2B. The product was recovered by evaporating the tert-amyl alcohol. The crude product was further purified by semi-preparative HPLC. The product so obtained was further subjected to iodide halogenation via α-KG. The halogenation reaction contained the metabolite (2.1 mM), KI (5.0 mM), and halogenase (32 μM) in phosphate buffer (20 mM, pH 7.8) plus 2 mM α-KG and up to 5% DMSO, in a final volume of 0.5 ml. After 10 h of incubation at 37°C, the reaction mixture was transferred to a 1.5 ml filtration unit (3000 NMWL membrane cut-off) to remove the enzyme, and the product was purified by semi-preparative HPLC. I-metabolite was reconstituted and diluted in DMSO and stored at -70°C until used at a concentration of 10 μg/ml. HRMS data clearly show that the enzyme incorporated one "I" per molecule.

2. *Incorporation of histidine.* I-metabolite was further functionalized via incorporation of a histidine tag. A histidine molecule was incorporated by enzymatic amidation of the previous intermediate and EL1-Lewatit. Solvents were dried over 3 Å molecular sieves for 24 h prior to use. Briefly, metabolite (0.344 mmol) was dissolved in 5 mL of DMSO and 2M2B was slowly added to a final volume of 25 mL. The biocatalyst (Lewatit lipase EL1, 2.5 g) and 3 Å molecular sieves (2.5 g) were then added and the suspension maintained 30 min at 40°C with magnetic stirring. Then, histidine (1.38 mmol) was added. When the conversion to the corresponding amide reached the maximum value, the mixture was cooled, filtered and washed with 2M2B. The product was recovered by evaporating the tert-amyl alcohol and the crude product was purified by semi-preparative HPLC. Fractions containing the product were combined and dried by lyophilisation. I-metabolite was reconstituted and diluted in DMSO and stored at -70°C until used at a concentration of 10 μg/ml.

**Synthetic method 15:** this method is designed to perform direct transesterification through a -OH group and further halogenation to a sp3 carbon atom to link it to Cy3 (grey arrow in Figure 9) and direct amidation through a $NH_2$ group to incorporate the His (black arrow in Figure 9) component.

Example of metabolite subjected to this synthetic protocol is the metabolite nr. 543 shown in Table 1 and Figures 8 and 9.

1. *Formation of I-metabolite.* The metabolite was subjected to transesterification with vinyl acetate using EL1-Lewatit. Briefly, metabolite (0.029 mmol) was dissolved in 5 mL of DMSO and 2M2B was slowly added to a final volume of 25 mL. The biocatalyst (Lewatit lipase EL1, 2.5 g) and 3 Å molecular sieves (2.5 g) were then added and the suspension maintained 30 min at 40°C with magnetic stirring. Then, vinyl acetate (0.29 mmol) was added. When the conversion of the corresponding ester reached the maximum value, the mixture was cooled, filtered and washed with 2M2B. The product was recovered by evaporating the tert-amyl alcohol. The crude product was further purified by semi-preparative HPLC. The product so obtained was further subjected to iodide halogenation via α-KG. The halogenation reaction contained the metabolite (2.1 mM), KI (5.0 mM), and halogenase (32 μM) in phosphate buffer (20 mM, pH 7.8) plus 2 mM α-KG and up to 5% DMSO, in a final volume of 0.5 ml. After 10 h of incubation at 37°C, the reaction mixture was transferred to a 1.5 ml filtration unit (3000 NMWL membrane cut-off) to remove the enzyme, and the product was purified by semi-preparative HPLC. I-metabolite was reconstituted and diluted in DMSO and stored at -70°C until used at a concentration of 10 μg/ml. HRMS data clearly show that the enzyme incorporated one "I" per molecule.

*2. Incorporation of histidine*. I-metabolite was further functionalized via incorporation of a histidine tag. A histidine molecule was incorporated by enzymatic amidation of the previous intermediate and EL1-Lewatit. Solvents were dried over 3 Å molecular sieves for 24 h prior to use. Briefly, metabolite (0.344 mmol) was dissolved in 5 mL of DMSO and 2M2B was slowly added to a final volume of 25 mL. The biocatalyst (Lewatit lipase EL1, 2.5 g) and 3 Å molecular sieves (2.5 g) were then added and the suspension maintained 30 min at 40°C with magnetic stirring. Then, histidine (1.38 mmol) was added. When the conversion to the corresponding ester reached the maximum value, the mixture was cooled, filtered and washed with 2M2B. The product was recovered by evaporating the tert-amyl alcohol and the crude product was purified by semi-preparative HPLC. Fractions containing the product were combined and dried by lyophilisation. I-metabolite was reconstituted and diluted in DMSO and stored at -70°C until used at a concentration of 10 μg/ml.

**Synthetic method 16:** this method is designed to perform direct transesterification through a -OH group and further halogenation to a sp3 carbon atom to link it to Cy3 (grey arrow in Figure 9) component and incorporation of His (black arrow in Figure 9) through a -COOH group of the metabolite.

Example of metabolite subjected to this synthetic protocol is the metabolite nr. 1179 shown in Table 1 and Figures 8 and 9.

*1. Formation of I-metabolite.* The metabolite was subjected to direct transesterification with vinyl acetate using EL1-Lewatit. Briefly, metabolite (0.029 mmol) was dissolved in 5 mL of DMSO and 2M2B was slowly added to a final volume of 25 mL. The biocatalyst (Lewatit lipase EL1, 2.5 g) and 3 Å molecular sieves (2.5 g) were then added and the suspension maintained 30 min at 40°C with magnetic stirring. Then, vinyl acetate (0.29 mmol) was added. When the conversion of the corresponding ester reached the maximum value, the mixture was cooled, filtered and washed with 2M2B. The product was recovered by evaporating the tert-amyl alcohol. The crude product was further purified by semi-preparative HPLC. The product so obtained was further subjected to iodide halogenation via α-KG. The halogenation reaction contained the metabolite (2.1 mM), KI (5.0 mM), and halogenase (32 μM) in phosphate buffer (20 mM, pH 7.8) plus 2 mM α-KG and up to 5% DMSO, in a final volume of 0.5 ml. After 10 h of incubation at 37°C, the reaction mixture was transferred to a 1.5 ml filtration unit (3000 NMWL membrane cut-off) to remove the enzyme, and the product was purified by semi-preparative HPLC. I-metabolite was reconstituted and diluted in DMSO and stored at -70°C until used at a concentration of 10 μg/ml. HRMS data clearly show that the enzyme incorporated one "I" per molecule.

*2. Incorporation of histidine*. I-metabolite was further functionalized via incorporation of a histidine tag. A histidine molecule was incorporated by enzymatic amidation of the previous intermediate and EL1-Lewatit. Solvents were dried over 3 Å molecular sieves for 24 h prior to use. Briefly, metabolite (0.344 mmol) was dissolved in 5 mL of DMSO and 2M2B was slowly added to a final volume of 25 mL. The biocatalyst (Lewatit lipase EL1, 2.5 g) and 3 Å molecular sieves (2.5 g) were then added and the suspension maintained 30 min at 40°C with magnetic stirring. Then, histidine (1.38 mmol) was added. When the conversion to the corresponding amide reached the maximum value, the mixture was cooled, filtered and washed with 2M2B. The product was recovered by evaporating the tert-amyl alcohol and the crude product was purified by semi-preparative HPLC. Fractions containing the product were combined and dried by lyophilisation. I-metabolite was reconstituted and diluted in DMSO and stored at -70°C until used at a concentration of 10 μg/ml.

**Synthetic method 17:** this method is designed to perform direct esterification through one -OH group and further halogenation to a sp3 carbon atom to link it to Cy3 (grey arrow in Figure 9) and and His (black arrow in Figure 9) components.

Example of metabolite subjected to this synthetic protocol is the metabolite nr. 1212 shown in Table 1 and Figures 8 and 9.

*1. Formation of I-metabolite.* The metabolite was subjected to direct transesterification with vinyl propionate using EL1-Lewatit. Briefly, metabolite (0.029 mmol) was dissolved in 5 mL of DMSO and 2M2B was slowly added to a final volume of 25 mL. The biocatalyst (Lewatit lipase EL1, 2.5 g) and 3 Å molecular sieves (2.5 g) were then added and the suspension maintained 30 min at 40°C with magnetic stirring. Then, vinyl propionate (0.29 mmol) was added. When the conversion of the corresponding ester reached the maximum value, the mixture was cooled, filtered and washed with 2M2B. The product was recovered by evaporating the tert-amyl alcohol. The crude product was further purified by semi-preparative HPLC. The product so obtained was further subjected to iodide halogenation via α-KG. The halogenation reaction contained the metabolite (2.1 mM), KI (5.0 mM), and halogenase (32 μM) in phosphate buffer (20 mM, pH 7.8) plus 2 mM α-KG and up to 5% DMSO, in a final volume of 0.5 ml. After 10 h of incubation at 37°C, the reaction mixture was transferred to a 1.5 ml filtration unit (3000 NMWL membrane cut-off) to remove the enzyme, and the product was purified by semi-preparative HPLC. I-metabolite was reconstituted and diluted in

DMSO and stored at -70°C until used at a concentration of 10 μg/ml. HRMS data clearly show that the enzyme incorporated two "I" per molecule.

*2. Incorporation of histidine.* I-metabolite was further functionalized via incorporation of a histidine tag. The incorporation of histidine was performed in the presence of the non-nucleophilic base and proton sponge 1,8-BDN as described by R.A. Kaufman *et al*. (*loc. cit.*) and F. Mazzetti and R.M. Lemmon (*loc. cit.*) with small modifications. Briefly, to a solution of I-metabolite (concentration range from 0.1 to 2.3 mmol), histidine (concentration range from 0.44 to 1 mmol), and 1,8-BDN at a final concentration of 10 mM were added, in $CH_3CN$ (10 ml final volume). The temperature was controlled at 32°C. After incubation (range from 11.52 to 424 min) the product was recovered by evaporating the $CH_3CN$. The corresponding product was purified by semi-preparative HPLC. The purified metabolite was found to be 98% pure.

**Synthetic method 18:** this method is designed to perform direct halogenation to one sp3 carbon atom (e.g. a carbon atom of a saturated hydrocarbon bond, e.g. a terminal methyl carbon atom or a methylene carbon atom, e.g. within a chain or ring) and further incorporation of His (black arrow in Figure 9) and Cy3 (grey arrow in Figure 9) components to this moiety

Example of metabolite subjected to this synthetic protocol is the metabolite nr. 417 shown in Table 1 and Figures 8 and 9.

*1-1. Formation of I-metabolite I.* The iodide halogenation of metabolite was performed via α-KG. The halogenation reaction contained the metabolite (2.1 mM), KI (5.0 mM), and halogenase (32 μM) in phosphate buffer (20 mM, pH 7.8) plus 2 mM α-KG and up to 5% DMSO, in a final volume of 0.5 ml. After 10 h of incubation at 37°C, the reaction mixture was transferred to a 1.5 ml filtration unit (3000 NMWL membrane cut-off) to remove the enzyme, and the product was purified by semi-preparative HPLC. I-metabolite was reconstituted and diluted in DMSO and stored at -70°C until used at a concentration of 10 μg/ml. HRMS data clearly show that the enzyme incorporated one "I" per molecule.

*1-2. Incorporation of ethyl amine I-metabolite I.* The corresponding metabolite was used to incorporate an ethyl group throught the reaction with ethyl amine in the presence of 1,8-BDN as described by R.A. Kaufman *et al.* (*loc. cit.*) and F. Mazzetti and R.M. Lemmon (*loc. cit.*) with small modifications. Briefly, reaction mixture (2 ml) contains I-metabolite I (0.078 mmol), 0.78 mmol of ethyl amine and 1,8-BDN at a final concentration of 100 mM in DMSO (10 ml final volume). The temperature was controlled at 32°C. After incubation (range from 11.52 to 424 min) the product was recovered by evaporating the $CH_3CN$. The labeled product was purified by semi-preparative HPLC. The purified metabolite was found to be 98% pure.

*1-3. Formation of I-metabolite II.* The iodide halogenation of metabolite was performed via α-KG. The halogenation reaction contained the metabolite (2.1 mM), KI (5.0 mM), and halogenase (32 μM) in phosphate buffer (20 mM, pH 7.8) plus 2 mM α-KG and up to 5% DMSO, in a final volume of 0.5 ml. After 10 h of incubation at 37°C, the reaction mixture was transferred to a 1.5 ml filtration unit (3000 NMWL membrane cut-off) to remove the enzyme, and the product was purified by semi-preparative HPLC. I-metabolite was reconstituted and diluted in DMSO and stored at -70°C until used at a concentration of 10 μg/ml. HRMS data clearly show that the enzyme incorporated two "I" *per* molecule. *2. Incorporation of histidine.* I-metabolite was further functionalized via incorporation of a histidine tag. The incorporation of histidine was performed in the presence of the non-nucleophilic base and proton sponge 1,8-BDN as described by R.A. Kaufman and *et al.* (*loc. cit.*) and F. Mazzetti and R.M. Lemmon (*loc. cit.*) with small modifications. Briefly, to a solution of I-metabolite (concentration range from 0.1 to 2.3 mmol), histidine (concentration range from 0.44 to 1 mmol), and 1,8-BDN at a final concentration of 10 mM were added, in $CH_3CN$ (10 ml final volume). The temperature was controlled at 32°C. After incubation (range from 11.52 to 424 min) the product was recovered by evaporating the $CH_3CN$. The corresponding product was purified by semi-preparative HPLC. The purified metabolite was found to be 98% pure.

**Synthetic method 19:** this method is designed to perform direct esterification through a -COOH group and further halogenation to a sp3 carbon atom to link it to Cy3 (grey arrow in Figure 9) and direct halogenation to a sp2 carbon atom (e.g. a carbon atom of an aromatic carbon bond or a carbon atom of an unsaturated hydrocarbon bond, e.g. terminal, or within a chain or ring) and further incorporation of His (grey arrow in Figure 9) component

Example of metabolite subjected to this synthetic protocol is the metabolite nr. 1332 shown in Table 1 and Figures 8 and 9.

*1. Formation of I-metabolite.* The metabolite was subjected to direct esterification with ethanol using EL1-Lewatit. Briefly, metabolite (0.029 mmol) was dissolved in 5 mL of DMSO and 2M2B was slowly added to a final volume of 25 mL. The biocatalyst (Lewatit lipase EL1, 2.5 g) and 3 Å molecular sieves (2.5 g) were then added and the suspension maintained 30 min at 40°C with magnetic stirring. Then, ethanol (0.29 mmol) was added. When the

conversion of the corresponding ester reached the maximum value, the mixture was cooled, filtered and washed with 2M2B. The product was recovered by evaporating the tert-amyl alcohol. The crude product was further purified by semi-preparative HPLC. The product so obtained was further subjected to iodide halogenation via α-KG. The halogenation reaction contained the metabolite (2.1 mM), KI (5.0 mM), and halogenase (32 μM) in phosphate buffer (20 mM, pH 7.8) plus 2 mM α-KG and up to 5% DMSO, in a final volume of 0.5 ml. After 10 h of incubation at 37°C, the reaction mixture was transferred to a 1.5 ml filtration unit (3000 NMWL membrane cut-off) to remove the enzyme, and the product was purified by semi-preparative HPLC. I-metabolite was reconstituted and diluted in DMSO and stored at -70°C until used at a concentration of 10 μg/ml. Using this intermediate a second iodization reaction was performed via $I^-$. The general halogenation procedure via cofactor is as follows. The reaction mixtures were incubated at 37°C with metabolite (0.080 mmol), KI (75 mM), 2 mM NADH, and halogenase (4 mg/ml, 100 μL) in phosphate buffer (20 mM, pH = 7.8), containing up to 20% DMSO (to increase metabolite solubility), in a final volume of 0.5 ml. After 24 hour of incubation, reaction product(s) were separated by semi-preparative HPLC. I-metabolite was reconstituted and diluted in DMSO and stored at -70°C until used at a concentration of 10 μg/ml. HRMS data clearly show that the enzyme incorporated two "I" per molecule after the two-step process.

*2. Incorporation of histidine.* I-metabolite was further functionalized via incorporation of a histidine tag. The incorporation of histidine was performed in the presence of the non-nucleophilic base and proton sponge 1,8-BDN as described by R.A. Kaufman *et al.* (*loc. cit.*) and F. Mazzetti and R.M. Lemmon (*loc. cit.*) with small modifications. Briefly, to a solution of I-metabolite (concentration range from 0.1 to 2.3 mmol), histidine (concentration range from 0.44 to 1 mmol), and 1,8-BDN at a final concentration of 10 mM were added, in $CH_3CN$ (10 ml final volume). The temperature was controlled at 32°C. After incubation (range from 11.52 to 424 min) the product was recovered by evaporating the $CH_3CN$. The corresponding product was purified by semi-preparative HPLC. The purified metabolite was found to be 98% pure.

**Synthetic method 20:** this method is designed to perform direct halogenation to a sp3 carbon atom (e.g. a carbon atom of a saturated hydrocarbon bond, e.g. a terminal methyl carbon atom or a methylene carbon atom, e.g. within a chain or ring) and further incorporation of Cy3 (grey arrow in Figure 9) component to this alkyl group and direct amidation through a -COOH group to link it to His (grey arrow in Figure 9) component.

Example of metabolite subjected to this synthetic protocol is the metabolite nr. 1790 shown in Table 1 and Figures 8 and 9.

*1. Formation of I-metabolite.* The iodide halogenation of metabolite was performed via α-KG. The halogenation reaction contained the metabolite (2.1 mM), KI (5.0 mM), and halogenase (32 μM) in phosphate buffer (20 mM, pH 7.8) plus 2 mM α-KG and up to 5% DMSO, in a final volume of 0.5 ml. After 10 h of incubation at 37°C, the reaction mixture was transferred to a 1.5 ml filtration unit (3000 NMWL membrane cut-off) to remove the enzyme, and the product was purified by semi-preparative HPLC. I-metabolite was reconstituted and diluted in DMSO and stored at -70°C until used at a concentration of 10 μg/ml. HRMS data clearly show that the enzyme incorporated one "I" per molecule.

*2. Incorporation of histidine.* I-metabolite was further functionalized via incorporation of a histidine tag. A histidine molecule was incorporated by enzymatic amidation of the previous intermediate and EL1-Lewatit. Solvents were dried over 3 Å molecular sieves for 24 h prior to use. Briefly, metabolite (0.344 mmol) was dissolved in 5 mL of DMSO and 2M2B was slowly added to a final volume of 25 mL. The biocatalyst (Lewatit lipase EL1, 2.5 g) and 3 Å molecular sieves (2.5 g) were then added and the suspension maintained 30 min at 40°C with magnetic stirring. Then, histidine (1.38 mmol) was added. When the conversion to the corresponding amide reached the maximum value, the mixture was cooled, filtered and washed with 2M2B. The product was recovered by evaporating the tert-amyl alcohol and the crude product was purified by semi-preparative HPLC. Fractions containing the product were combined and dried by lyophilisation. I-metabolite was reconstituted and diluted in DMSO and stored at -70°C until used at a concentration of 10 μg/ml.

**Synthetic method 21:** this method is designed to perform direct esterification through a -COOH group and further halogenation to a sp3 carbon atom to link it to Cy3 (grey arrow in Figure 9) and His (grey arrow in Figure 9) components.

Example of metabolite subjected to this synthetic protocol is the metabolite nr. 1409 shown in Table 1 and Figures 8 and 9.

*1. Formation of I-metabolite.* The metabolite was subjected to direct esterification with ethanol using EL1-Lewatit. Briefly, metabolite (0.029 mmol) was dissolved in 5 mL of DMSO and 2M2B was slowly added to a final volume of 25 mL. The biocatalyst (Lewatit lipase EL1, 2.5 g) and 3 Å molecular sieves (2.5 g) were then added and the suspension maintained 30 min at 40°C with magnetic stirring. Then, ethanol (0.29 mmol) was added. When the

conversion of the corresponding ester reached the maximum value, the mixture was cooled, filtered and washed with 2M2B. The product was recovered by evaporating the tert-amyl alcohol. The crude product was further purified by semi-preparative HPLC. The product so obtained was further subjected to iodide halogenation via α-KG. The halogenation reaction contained the metabolite (2.1 mM), KI (5.0 mM), and halogenase (32 μM) in phosphate buffer (20 mM, pH 7.8) plus 2 mM α-KG and up to 5% DMSO, in a final volume of 0.5 ml. After 10 h of incubation at 37°C, the reaction mixture was transferred to a 1.5 ml filtration unit (3000 NMWL membrane cut-off) to remove the enzyme, and the product was purified by semi-preparative HPLC. I-metabolite was reconstituted and diluted in DMSO and stored at -70°C until used at a concentration of 10 μg/ml. HRMS data clearly show that the enzyme incorporated two "I" per molecule.

*2. Incorporation of histidine.* I-metabolite was further functionalized via incorporation of a histidine tag. The incorporation of histidine was performed in the presence of the non-nucleophilic base and proton sponge 1,8-BDN as described by R.A. Kaufman *et al.* (*loc. cit.*) and F. Mazzetti and R.M. Lemmon (*loc. cit.*) with small modifications. Briefly, to a solution of I-metabolite (concentration range from 0.1 to 2.3 mmol), histidine (concentration range from 0.44 to 1 mmol), and 1,8-BDN at a final concentration of 10 mM were added, in $CH_3CN$ (10 ml final volume). The temperature was controlled at 32°C. After incubation (range from 11.52 to 424 min) the product was recovered by evaporating the $CH_3CN$. The corresponding product was purified by semi-preparative HPLC. The purified metabolite was found to be 98% pure.

**Synthetic method 22:** this method is designed to perform direct halogenation to one sp2 carbon atom (e.g. a carbon atom of an aromatic carbon bond or a carbon atom of an unsaturated hydrocarbon bond, e.g. terminal, or within a chain or ring) and further incorporation of His (black arrow in Figure 9) and Cy3 (grey arrow in Figure 9) components to this moiety.

Example of metabolite subjected to this synthetic protocol is the metabolite nr. 1614 shown in Table 1 and Figures 8 and 9.

*1-1. Formation of I-metabolite I.* The iodide halogenation of metabolite was performed via $I^-$ as follows. The general halogenation procedure via cofactor is as follows. The reaction mixtures were incubated at 37°C with metabolite (0.080 mmol), KI (75 mM), 2 mM NADH, and halogenase (4 mg/ml, 100 μL) in phosphate buffer (20 mM, pH = 7.8), containing up to 20% DMSO (to increase metabolite solubility), in a final volume of 0.5 ml. After 24 hour of incubation, reaction product(s) were separated by semi-preparative HPLC. I-metabolite was reconstituted and diluted in DMSO and stored at -70°C until used at a concentration of 10 μg/ml. HRMS data clearly show that the enzyme incorporated one "I" per molecule.

*1-2. Incorporation of ethyl amine.* The corresponding metabolite was used to incorporate an ethyl group throught the reaction with ethyl amine in the presence of 1,8-BDN as described by R.A. Kaufman *et al*. (*loc. cit.*) and F. Mazzetti and R.M. Lemmon (*loc. cit*.) with small modifications. Briefly, reaction mixture (2 ml) contains I-metabolite (0.078 mmol), 0.78 mmol of ethyl amine and 1,8-BDN at a final concentration of 100 mM in DMSO (10 ml final volume). The temperature was controlled at 32°C. After incubation (range from 11.52 to 424 min) the product was recovered by evaporating the $CH_3CN$. The labeled product was purified by semi-preparative HPLC. The purified metabolite was found to be 98% pure.

*1-3. Formation of I-metabolite II.* The iodide halogenation of metabolite was performed via α-KG. The halogenation reaction contained the metabolite (2.1 mM), KI (5.0 mM), and halogenase (32 μM) in phosphate buffer (20 mM, pH 7.8) plus 2 mM α-KG and up to 5% DMSO, in a final volume of 0.5 ml. After 10 h of incubation at 37°C, the reaction mixture was transferred to a 1.5 ml filtration unit (3000 NMWL membrane cut-off) to remove the enzyme, and the product was purified by semi-preparative HPLC. I-metabolite was reconstituted and diluted in DMSO and stored at -70°C until used at a concentration of 10 μg/ml. HRMS data clearly show that the enzyme incorporated two "I" per molecule. *2. Incorporation of histidine.* I-metabolite was further functionalized via incorporation of a histidine tag. The incorporation of histidine was performed in the presence of the non-nucleophilic base and proton sponge 1,8-BDN as described by R.A. Kaufman *et al*. (*loc*. *cit*.) and F. Mazzetti and R.M. Lemmon (*loc. cit*.) with small modifications. Briefly, to a solution of I-metabolite (concentration range from 0.1 to 2.3 mmol), histidine (concentration range from 0.44 to 1 mmol), and 1,8-BDN at a final concentration of 10 mM were added, in $CH_3CN$ (10 ml final volume). The temperature was controlled at 32°C. After incubation (range from 11.52 to 424 min) the product was recovered by evaporating the $CH_3CN$. The corresponding product was purified by semi-preparative HPLC. The purified metabolite was found to be 98% pure.

**Synthetic method 23:** this method is designed to perform direct halogenation to a sp3 carbon atom (e.g. a carbon atom of a saturated hydrocarbon bond, e.g. a terminal methyl carbon atom or a methylene carbon atom, e.g. within a chain or ring) and further incorporation of Cy3 (grey arrow in Figure 9) component and direct amidation through a -COOH group to link it to His (grey arrow in Figure 9) component.

Example of metabolite subjected to this synthetic protocol is the metabolite nr. 394 shown in Table 1 and Figures 8 and 9.

1. *Formation of I-metabolite.* The iodide halogenation of metabolite was performed via α-KG. The halogenation reaction contained the metabolite (2.1 mM), KI (5.0 mM), and halogenase (32 μM) in phosphate buffer (20 mM, pH 7.8) plus 2 mM α-KG and up to 5% DMSO, in a final volume of 0.5 ml. After 10 h of incubation at 37°C, the reaction mixture was transferred to a 1.5 ml filtration unit (3000 NMWL membrane cut-off) to remove the enzyme, and the product was purified by semi-preparative HPLC. I-metabolite was reconstituted and diluted in DMSO and stored at -70°C until used at a concentration of 10 μg/ml. HRMS data clearly show that the enzyme incorporated one "I" per molecule.
2. *Incorporation of histidine.* I-metabolite was further functionalized via incorporation of a histidine tag. A histidine molecule was incorporated by enzymatic amidation of the previous intermediate and EL1-Lewatit. Solvents were dried over 3 Å molecular sieves for 24 h prior to use. Briefly, metabolite (0.344 mmol) was dissolved in 5 mL of DMSO and 2M2B was slowly added to a final volume of 25 mL. The biocatalyst (Lewatit lipase EL1, 2.5 g) and 3 Å molecular sieves (2.5 g) were then added and the suspension maintained 30 min at 40°C with magnetic stirring. Then, histidine (1.38 mmol) was added. When the conversion to the corresponding amide reached the maximum value, the mixture was cooled, filtered and washed with 2M2B. The product was recovered by evaporating the tert-amyl alcohol and the crude product was purified by semi-preparative HPLC. Fractions containing the product were combined and dried by lyophilisation. I-metabolite was reconstituted and diluted in DMSO and stored at -70°C until used at a concentration of 10 μg/ml.

**Synthetic method 24:** this method is designed to perform partial dehalogenation in an halogenated compound, followed by halogenation (iodization) to an alkyl group and further incorporation of Cy3 (grey arrow) component to this alkyl group and direct esterification through one -OH group to link it to His (black arrow) component.

Example of metabolite subjected to this synthetic protocol is the metabolite nr. 1997 shown in Table 1 and Figure 9.

1. *Formation of I-metabolite.* The iodide halogenation of metabolite was performed via α-KG prior selective dehalogenation. First, the metabolite (100 μl of a 10 mM solution in MeOH final concentration of 1 mM) was added to 900 μl in phosphate buffer (20 mM, pH 7.8) and dehalogenase (32 μM). Reaction was allowed to proceed at 40°C for 5 hours. Then the reaction mixture was transferred to a 1.5 ml filtration unit (3000 NMWL membrane cut-off) to remove the enzyme, and the product (mixture of partial dehalogenated products) was concentrated by liophilization. The purified intermediate ( 95% pure) was used for a subsequent iodization reaction as follows. The metabolite was subjected to transesterification with vinyl acetate using EL1-Lewatit. Briefly, metabolite (0.029 mmol) was dissolved in 5 mL of DMSO and 2M2B was slowly added to a final volume of 25 mL. The biocatalyst (Lewatit lipase EL1, 2.5 g) and 3 Å molecular sieves (2.5 g) were then added and the suspension maintained 30 min at 40°C with magnetic stirring. Then, vinyl acetate (0.29 mmol) was added. When the conversion of the corresponding ester reached the maximum value, the mixture was cooled, filtered and washed with 2M2B. The product was recovered by evaporating the tert-amyl alcohol. The crude product was further purified by semi-preparative HPLC. The product so obtained was further subjected to iodide halogenation via α-KG. The halogenation reaction contained the metabolite (2.1 mM), KI (5.0 mM), and halogenase (32 μM) in phosphate buffer (20 mM, pH 7.8) plus 2 mM α-KG and up to 5% DMSO, in a final volume of 0.5 ml. After 10 h of incubation at 37°C, the reaction mixture was transferred to a 1.5 ml filtration unit (3000 NMWL membrane cut-off) to remove the enzyme, and the product was purified by semi-preparative HPLC. I-metabolite was reconstituted and diluted in DMSO and stored at -70°C until used at a concentration of 10 μg/ml. HRMS data clearly show that the enzyme incorporated two "I" per molecule.
2. *Incorporation of histidine.* I-metabolite was further functionalized via incorporation of a histidine tag. The incorporation of histidine was performed in the presence of the non-nucleophilic base and proton sponge 1,8-BDN as described by R.A. Kaufman *et al*. (*loc. cit.*) and F. Mazzetti and R.M. Lemmon (*loc. cit.*) with small modifications. Briefly, to a solution of I-metabolite (concentration range from 0.1 to 2.3 mmol), histidine (concentration range from 0.44 to 1 mmol), and 1,8-BDN at a final concentration of 10 mM were added were added, in $CH_3CN$ (10 ml final volume). The temperature was controlled at 32°C. After incubation (range from 11.52 to 424 min) the product was recovered by evaporating the $CH_3CN$. The corresponding product was purified by semi-preparative HPLC. The purified metabolite was found to be 98% pure.

**Synthetic method 25:** this method is designed to perform direct transamidation through one $-NH_2$ group and further halogenation to a sp3 carbon atom to link it to Cy3 (grey arrow in Figure 9) and and His (black arrow in Figure 9) components.

Example of metabolite subjected to this synthetic protocol is the metabolite nr. 1481 shown in Table 1 and

Figures 8 and 9.

*1. Formation of I-metabolite.* The metabolite was subjected to direct transamidation with vinyl propionate using EL1-Lewatit. Briefly, metabolite (0.029 mmol) was dissolved in 5 mL of DMSO and 2M2B was slowly added to a final volume of 25 mL. The biocatalyst (Lewatit lipase EL1, 2.5 g) and 3 Å molecular sieves (2.5 g) were then added and the suspension maintained 30 min at 40°C with magnetic stirring. Then, acetate (0.29 mmol) was added. When the conversion of the corresponding amide reached the maximum value, the mixture was cooled, filtered and washed with 2M2B. The product was recovered by evaporating the tert-amyl alcohol. The crude product was further purified by semi-preparative HPLC. The product so obtained was further subjected to iodide halogenation via α-KG. The halogenation reaction contained the metabolite ($2.1^-$ mM), KI (5.0 mM), and halogenase (32 μM) in phosphate buffer (20 mM, pH 7.8) plus 2 mM α-KG and up to 5% DMSO, in a final volume of 0.5 ml. After 10 h of incubation at 37°C, the reaction mixture was transferred to a 1.5 ml filtration unit (3000 NMWL membrane cut-off) to remove the enzyme, and the product was purified by semi-preparative HPLC. I-metabolite was reconstituted and diluted in DMSO and stored at -70°C until used at a concentration of 10 μg/ml. HRMS data clearly show that the enzyme incorporated two "I" per molecule at the end of the two-step process.

*2. Incorporation of histidine.* I-metabolite was further functionalized via incorporation of a histidine tag. The incorporation of histidine was performed in the presence of the non-nucleophilic base and proton sponge 1,8-BDN as described by R.A. Kaufman *et al.* (*loc. cit.*) and F. Mazzetti and R.M. Lemmon (*loc. cit.*) with small modifications. Briefly, to a solution of I-metabolite (concentration range from 0.1 to 2.3 mmol), histidine (concentration range from 0.44 to 1 mmol), and 1,8-BDN at a final concentration of 10 mM were added, in $CH_3CN$ (10 ml final volume). The temperature was controlled at 32°C. After incubation (range from 11.52 to 424 min) the product was recovered by evaporating the $CH_3CN$. The corresponding product was purified by semi-preparative HPLC. The purified metabolite was found to be 98% pure.

**Synthetic method 26:** this method is designed to perform partial dehalogenation in an halogenated unsaturated compound, followed by halogenation (iodization) to a sp3 carbon atom and further incorporation of Cy3 (grey arrow in Figure 9) component to this sp3 carbon atom and direct halogenation to a sp3 carbon atom and further incorporation of His (black arrow in Figure 9) component to this sp3 carbon atom.

Example of metabolite subjected to this synthetic protocol is the metabolite nr. 242 shown in Table 1 and Figures 8 and 9.

*1. Formation of I-metabolite.* The iodide halogenation of metabolite was performed via α-KG prior selective dehalogenation. First, the metabolite (100 μl of a 10 mM solution in MeOH final concentration of 1 mM) was added to 900 μl in phosphate buffer (20 mM, pH 7.8) and dehalogenase (32 μM). Reaction was allowed to proceed at 40°C for 5 hours. Then the reaction mixture was transferred to a 1.5 ml filtration unit (3000 NMWL membrane cut-off) to remove the enzyme, and the product (mixture of partial dehalogenated products) was concentrated by liophilization. The purified intermediate ( 95% pure) was used for a subsequent iodization reaction as follows. The metabolite was subjected to transesterification with vinyl acetate using EL1-Lewatit. Briefly, metabolite (0.029 mmol) was dissolved in 5 mL of DMSO and 2M2B was slowly added to a final volume of 25 mL. The biocatalyst (Lewatit lipase EL1, 2.5 g) and 3 Å molecular sieves (2.5 g) were then added and the suspension maintained 30 min at 40°C with magnetic stirring. Then, vinyl acetate (0.29 mmol) was added. When the conversion of the corresponding ester reached the maximum value, the mixture was cooled, filtered and washed with 2M2B. The product was recovered by evaporating the tert-amyl alcohol. The crude product was further purified by semi-preparative HPLC. The product so obtained was further subjected to iodide halogenation via α-KG. The halogenation reaction contained the metabolite (2.1 mM), KI (5.0 mM), and halogenase (32 μM) in phosphate buffer (20 mM, pH 7.8) plus 2 mM α-KG and up to 5% DMSO, in a final volume of 0.5 ml. After 10 h of incubation at 37°C, the reaction mixture was transferred to a 1.5 ml filtration unit (3000 NMWL membrane cut-off) to remove the enzyme, and the product was purified by semi-preparative HPLC. I-metabolite was reconstituted and diluted in DMSO and stored at -70°C until used at a concentration of 10 μg/ml. HRMS data clearly show that the enzyme incorporated two "I" per molecule at the end of the two-step proccess.

*2. Incorporation of histidine.* I-metabolite was further functionalized via incorporation of a histidine tag. The incorporation of histidine was performed in the presence of the non-nucleophilic base and proton sponge 1,8-BDN as described by R.A. Kaufman *et al*. (*loc*. *cit*.) and F. Mazzetti and R.M. Lemmon (*loc*. *cit*.) with small modifications. Briefly, to a solution of I-metabolite (concentration range from 0.1 to 2.3 mmol), histidine (concentration range from 0.44 to 1 mmol), and 1,8-BDN at a final concentration of 10 mM were added, in $CH_3CN$ (10 ml final volume). The temperature was controlled at 32°C. After incubation (range from 11.52 to 424 min) the product was recovered by evaporating the $CH_3CN$. The corresponding product was purified by semi-preparative HPLC. The purified metabolite was found to be 98% pure.

**Synthetic method 27 for labelling DNA 1 (5-GAC GCT GCC GAA TTC TGG CTT GCT AGG ACA TCT TTG CCC ACG TTG ACC C-3):** The substrate contain a mixture of labelled metabolites at different position of the DNA substrate (see attached figure). Only when an endonuclease cut close to the base where the Cy3 and His are close, the Cy3 is released. When attached to G, C or A the synthetic method includes a direct halogenation to a sp3 carbon atom to link it to Cy3 (grey arrow in Figure 9) component and incorporation of His (black arrow in Figure 9) through an $NH_2$ group of the metabolite.

Example of metabolite subjected to this synthetic protocol is the DNA nucleotide shown in Figure 9.

1. *Formation of I-metabolite.* The iodide halogenation of metabolite was performed via α-KG. The halogenation reaction contained the oligonucleotide (6.6 nmol), KI (5.0 mM), and halogenase (131 μM) in phosphate buffer (20 mM, pH 7.8) plus 2 mM α-KG, in a final volume of 0.8 ml. After 150 h of incubation at 37°C, the reaction mixture was transferred to 1.5 ml filtration unit (3-kDa membrane cut-off) to remove the enzyme, and the products were purified by semi-preparative HPLC. 1-metabolite was reconstituted and diluted in DMSO and stored at -70°C until used at a concentration of 1.5 nmol/ml. HRMS data clearly show that the enzyme incorporated one "I" per molecule per base. I-metabolite obtained is a complex mixture of halogenated molecules in which G, C, A and T bases are halogenated.

2. *Incorporation of histidine.* I-metabolite was further functionalized via incorporation of a histidine tag. A histidine molecule was incorporated by enzymatic amidation of the previous intermediate and EL1-Lewatit. Solvents were dried over 3 Å molecular sieves for 24 h prior to use. Briefly, metabolite (21.8 nmol) was dissolved in 5 mL of DMSO and 2M2B was slowly added to a final volume of 25 mL. The biocatalyst (Lewatit lipase EL1, 2.5 g) and 3 Å molecular sieves (2.5 g) were then added and the suspension maintained 30 min at 40°C with magnetic stirring. Then, histidine (12.9 mmol) was added. An excess of histidine was used to facilitate the reaction yield. When the conversion to the corresponding ester reached the maximum value, the mixture was cooled, filtered and washed with 2M2B. The crude product was further purified by semi-preparative HPLC to obtain a mixture of his tagged oligonucleotide. Using this method histidine linking is mainly performed at the G, C and A bases due to the presence of an amine group through which the histidine is incorporated. I-metabolite was reconstituted and diluted in DMSO and stored at -70°C until used at a concentration of 1.1nmol/ml.

When attached to thyamine (T) the synthetic method includes a direct halogenation to sp3 carbon atoms and further incorporation of His (black arrow in Figure 9) and Cy3 (grey arrow in Figure 9) component to those sp3 carbon atoms.

Example of metabolite subjected to this synthetic protocol is the DNA nucleotide shown in Figure 9.

1. *Formation of I-metabolite.* The iodide halogenation of metabolite was performed via α-KG. The halogenation reaction contained the oligonucleotide (6.6 nmol), KI (5.0 mM), and halogenase (131 μM) in phosphate buffer (20 mM, pH 7.8) plus 2 mM α-KG, in a final volume of 0.8 ml. After 150 h of incubation at 37°C, the reaction mixture was transferred to 1.5 ml filtration unit (3-kDa membrane cut-off) to remove the enzyme, and the products were purified by semi-preparative HPLC. I-metabolite was reconstituted and diluted in DMSO and stored at -70°C until used at a concentration of 1.5 nmol/ml. HRMS data clearly show that the enzyme incorporated one "I" per molecule per G, T and A while incorporated two "I" per thymine (T). I-metabolite obtained is a complex mixture of halogenated molecules in which G, C, A and T bases are halogenated.

2. *Incorporation of histidine.* I-metabolite was further functionalized via incorporation of a histidine tag. The incorporation of histidine was performed in the presence of the non-nucleophilic base and proton sponge 1,8-BDN as described by R.A. Kaufman *et al*. (*loc. cit.*) and F. Mazzetti and R.M. Lemmon (*loc. cit.*) with small modifications. Briefly, to a solution of I-metabolite (1.3 nmol), histidine (6.8 nmol), and 1,8-BDN at a final concentration of 1 μM were added, in $CH_3CN$ (1 ml final volume). The temperature was controlled at 32°C. After 34.1 min incubation the product was recovered by evaporating the $CH_3CN$. The corresponding products were purified by semi-preparative HPLC. Using this method a heterogeneous mixture of products is formed: histidine tags can be incorporated to G, A and C through one position of the the ribose ring or to the two positions of T (one to the ribose and one to the base). Therefore, the product should be extensively purified by semi-preparative HPLC, to obtain the desired product, namely, that containing the His tag in the pyrimidine ring of thymine (T).

**Synthetic method 28 for labelling DNA 1 3 (5- TGG TCA TCA GGG CTT TAC CTC C<u>C</u>G GAC AAT C<u>C</u>G GAG CTT ACG GAG TAC CTG TAG A<u>G</u>C TTC CTG TGC AAG C-3):** direct halogenation to a sp3 carbon atom to link it to Cy3 (grey arrow in Figure 9) component and incorporation of His (black arrow in Figure 9) through an $NH_2$ group of the metabolite. Only when an endonuclease cut close to the base where the Cy3 and His are close, the Cy3 is released. When attached to G, C or A the synthetic method includes a direct halogenation to a sp3 carbon atom to link it to Cy3 (grey arrow in Figure 9) component and incorporation of His (black arrow in Figure 9) through an $NH_2$ group

of the metabolite. When attached to thyamine (T) the synthetic method includes a direct halogenation to sp3 carbon atoms and further incorporation of His (black arrow in Figure 9) and Cy3 (grey arrow in Figure 9) component to those sp3 carbon atoms.

The conditions for synthesis are those described in the method 27.

**Synthetic method 29 for labelling labelling lambda DNA digested with *Sau3AI*:** direct halogenation to a sp3 carbon atom to link it to Cy3 (grey arrow in Figure 9) component and incorporation of His (black arrow in Figure 9) through an $NH_2$ group of the metabolite (in the G base at the 5'-moiety).

DNA substrate is prepared as follows:

20 μl concentrated lambda DNA (12 μg) (from NEB)
7 μl Buffer NEB1 10X
7 μl BSA 10X
2 μl MilliQ water
36 μl Sau3AI 0.4U/μl
Total reaction volume: 70 μl

Incubate 40-60 min at 37°C. Stop reactions by adding 65 mM EDTA 0.5 M pH 8 (1.5 μl for each 10 μl reaction volume) and heat the samples to 65 °C 15 min. Sample is loaded on a 20 cm long preparative gel 2% agarose, run it at 30-35 V overnight at 4°C and cut and stain the slots with the DNA marker. Under UV light cut out the part of the gel blocks with the DNA markers in the range of ca. 100-200 bp. Cut out the desired gel region (25-40 Kb gel region) and trim excess agarose. Then proceed to the agarose gel digestion following the GELase (EPICENTRE) protocol and concentrate DNA. Once isolated the DNA then proceed as described below.

*1. Formation of I-metabolite*. The iodide halogenation of metabolite was performed via α-KG. The halogenation reaction contained the oligonucleotide (6.6 nmol), KI (5.0 mM), and halogenase (131 μM) in phosphate buffer (20 mM, pH 7.8) plus 2 mM α-KG, in a final volume of 0.8 ml. After 150 h of incubation at 37°C, the reaction mixture was transferred to 1.5 ml filtration unit (3-kDa membrane cut-off) to remove the enzyme, and the products were purified by semi-preparative HPLC. I-metabolite was reconstituted and diluted in DMSO and stored at -70°C until used at a concentration of 1.5 nmol/ml. HRMS data clearly show that the enzyme incorporated one "I" per molecule per G, T and A while incorporated two "I" per thymine (T). I-metabolite obtained is a complex mixture of halogenated molecules in which G, C, A and T bases are halogenated.

Steps 2 is similar to those described in synthesis 27.

**Synthetic method 30 for labelling labelling a protein substrate (rhodonase):** the protein is partially unfolded and then a direct halogenation is performed to the valine, leucine and isoleucine amino acids to link them to Cy3 (grey arrow in Figure 9) and His (black arrow in Figure 9) components.

Partially unfolded rhodonase was prepared and purified as described elsewhere (Ferrer et al., Mol. Microbiol. 53, 167-182 (2005).

*1. Formation of I-metabolite.* The iodide halogenation of protein substrate was performed via α-KG at the N-terminal position. The halogenase used in this study showed a catalytic core which was accessible to the linearized protein, namely at its N-terminal position. The halogenation reaction contained the protein (1.0 nmol), KI (5.0 mM), and halogenase (108 μM) in phosphate buffer (20 mM, pH 7.8) plus 2 mM α-KG, in a final volume of 0.5 ml. After 96 h of incubation at 37°C, the reaction mixture was transferred to a 1.5 ml filtration unit (3000 NMWL membrane cut-off) to remove the non-enzymatic reaction components, and the product was purified by semi-preparative HPLC using a Bio-Sil SEC 400 column (Bio-Rad) pre-equilibrated with phosphate buffer. Separation was performed at room temperature at a flow rate of 1 ml min $^{1}$. The following standards were used to calibrate the gel filtration column and to ensure than the protein substrate and the halogenase proteins are perfectly separated from the reaction mixture: E.GroEL (840 kDa), tyroglobulin (669 kDa), ferritin (440 kDa), gamma-globulin (158 kDa) (Ferrer et al., Mol. Microbiol. 53, 167-182 (2005). Iodide protein was reconstituted and diluted in PBS buffer and stored at -70°C until used at a concentration of 2 pmol/ml.

*2. Incorporation of histidine*. Iodide protein was further functionalized via incorporation of a histidine tag. The incorporation of histidine was performed in the presence of the non-nucleophilic base and proton sponge 1,8-BDN as described by R.A. Kaufman *et al.* (*loc. cit.*) and F. Mazzetti and R.M. Lemmon (*loc. cit.*) with small modifications. Briefly, to a solution of halogenated protein (1.2 pmol), histidine (4.2 μmol), and 1,8-BDN at a final concentration of 10 mM were added, in $CH_3CN$ (10 ml final volume). The temperature was controlled at 32°C. After 148 min incubation the product was recovered by evaporating the $CH_3CN$. The corresponding product was purified by fast preparative liquid chromatography (FPLC) as described elsewhere (Ferrer et al., Mol. Microbiol. 53, 167-182 (2005). Under

these conditions the protein suffers an extensive denaturalization process.

To purify any of the above intermediates of final products, any of the following HPLC purification methods can be used.

**Method 1 (standard for the purification of the final labelled metabolite)**

Prontosil-AQ, 5 lm, 120 A, 250 · 8 mm column equipped with a Prontosil-AQ, 5 lm, 120 A, 33 · 8 mm pre-column, and compounds were eluted with acetonitrile (20% for 5 min, followed by linear gradients to 45% in 5 min, to 50% in 7 min and to 100% in 2 min) in triethylammonium hydrogen carbonate buffer (0.01 M, pH 8.6) at a flow of 0.4 to 3 mL/min.

**Method 2 (standard for medium polar compounds)**

*Analytic:* on a Mediterránea 5 μM C-18 column (250x4.6 mm, Macherey-Nagel), equilibrated with 85% MeOH: 15% $H_2O$ plus 0.1% acetic acid. Column was kept at 35°C and elution was performed at 55 atm and 0.7 ml/ml. Detection was performed both by light scattering (temp 68.2 °C, 1.8 l/min) and PDA (250, 300 and 400 nm).

*Semi-preparative:* on a Mediterránea 5 μM C-18 column (250x4.6 mm, Macherey-Nagel), equilibrated with 85% MeOH:15% $H_2O$ plus 0.1% acetic acid. Column was kept at 35°C and elution was performed at 55 atm and 3.0 ml/ml. Detection was performed both by light scattering (temp 68.2 °C, 1.8 l/min) and PDA (250, 300 and 400 nm).

**Method 3 (standard for medium polar esters)**

*Analytic:* using a ternary pump (model 9012, Varian) coupled to a thermostatized (25 °C) autosampler (model L-2200, VWR International). The temperature of the column was kept constant at 45 °C. Detection was performed using a photodiode array detector (ProStar, Varian) in series with an evaporative light scattering detector (ELSD, model 2000ES, Alltech), and integration was carried out using the Varian Star LC workstation 6.41. For the analysis the column was a Lichrospher 100 RP8 (4.6 × 125 mm, 5 μm, Analisis Vinicos), and the mobile phase was 70:30 (v/v) $H_2O$/methano ($H_2O$ contained 0.1% of acetic acid) at 1 mL/min for 5 min. Then, a gradient from this mobile phase to 50:50 (v/v) $H_2O$/ methanol was performed in 5 min, and this eluent was maintained during 15 min. For preparative scale the column was Mediterranea-C18 (4.6 × 150 mm, 5 μm, Teknokroma, Spain). The mobile phase was 90:10 (v/v) methanol/$H_2O$ ($H_2O$ contained 0.1% of formic acid) at 1.5 mL/min.

*Semi-preparative:* using a ternary pump (model 9012, Varian) coupled to a thermostatized (25 °C) autosampler (model L-2200, VWR International). The temperature of the column was kept constant at 45 °C. Detection was performed using a photodiode array detector (ProStar, Varian) in series with an evaporative light scattering detector (ELSD, model 2000ES, Alltech), and integration was carried out using the Varian Star LC workstation 6.41. For preparative scale the column was Mediterranea-C18 (4.6 × 150 mm, 5 μm, Teknokroma, Spain). The mobile phase was 90:10 (v/v) methanol/$H_2O$ ($H_2O$ contained.0.1% of formic acid) at 1.5 mL/min.

**Method 4 (standard for short sugars)**

*Analytic:* Using a pump (Spectra-Physics Inc., Model SP 8810) coupled to a Nucleosil 100-C18 column (250mm×4.6 mm) (Sugelabor, Spain). The mobile phase was water at 0.5 ml $min^{1}$. The column was kept constant at 40 °C. A differential refractometer (Waters, model 2410) was used and set to a constant temperature of 45 °C.

*Semi-preparative 1:* using a system equipped with a Waters Delta 600 pump, a Nucleosil 100-C18 column (250mm×10 mm) (Sugelabor, Spain) coupled to a precolumn (50mm×10 mm) packed with the same stationary phase. A differential refractometer (Varian, model 9040) set to 35 °C and a fraction collector (Waters) were used. Water was the mobile phase (2.4 ml $min^1$), and the column temperature was kept constant at 40 °C.

*Semi-preparative 2*: with a quaternary pump (Delta 600, Waters) coupled to a 5 μM Lichrosorb-$NH_2$ column (4.6mmx250 mm) (Merck). Detection was performed using an evaporative light scattering detector DDL-31 (Eurosep) equilibrated at 85 C. Acetonitrile:water 85:15 (v/v), degassed with helium, was used as mobile phase at 0.9 ml $min^{1}$ for 8.1 min. Then, a gradient from this eluent to acetonitrile:water 75:25 (v/v) was performed in 2 min, and held for 6 min. A new gradient to acetonitrile:water 70:30 (v/v) was performed in 5 min and held for 14 min. Total analysis time was 35 min. The column temperature was kept constant at 25 °C.

**Method 5 (standard for short length esters)**

*Analytic*: The pump (Spectra-Physics Inc., Model SP 8810) was coupled to a Nucleosil 100-C18 column (250mm×4.6 mm) (Sugelabor, Spain). The mobile phase was 80% MeOH:20% $H_2O$ at 0.5 ml $min^1$. The column was

kept constant at 40 °C. A differential refractometer (Waters, model 2410) was used and set to a constant temperature of 45 °C.

*Semi-preparative:* using a system equipped with a Waters Delta 600 pump, a Nucleosil 100-C18 column (250mm×10 mm) (Sugelabor, Spain) coupled to a precolumn (50mm×10 mm) packed with the same stationary phase. A differential refractometer (Varian, model 9040) set to 35 °C and a fraction collector (Waters) were used. 80% MeOH: 20% $H_2O$ was the mobile phase (2.4 ml min $^1$), and the column temperature was kept constant at 40 °C.

**Method 6 (standard for long length fatty and their sugar derivatives)**

*Analytic:* using a system equipped with a Spectra-Physics pump, a Sugelabor Nucleosil 100-C18 column (250 × 4.6 mm) and a refraction index detector (Spectra-Physics). For the analysis methanol/water 95:5 (v/v) was used as mobile phase (flow rate 1.5 mL/min) and the temperature of the column was kept constant at 40°C.

*Semi-preparative:* using a system equipped with a Waters Delta 600 pump, a Nucleosil 100-C18 column (250mm×10 mm) (Sugelabor, Spain) coupled to a precolumn (50mm×10 mm) packed with the same stationary phase. A differential refractometer (Varian, model 9040) set to 35 °C and a fraction collector (Waters) were used. 90% MeOH: 10% $H_2O$ or 95% Meon:5% $H_2O$ was the mobile phase (2.8 ml min $^1$), and the column temperature was kept constant at 40 °C.

**Method 7 (standard for folate derivatives)**

*Analytic:* on a Nucleosil 100-C18 column (250 × 4.6 mm) using isocratic program of 88% A and 12% B in 30 min at a flow rate of 0.3 ml/min. Mobile phase A consisted of 0.1% formic acid while mobile phase B consisted of 0.1% formic acid in a 95:5 acetonitrile/water solution. Diode array detector (DAD) was set at 290 nm.

*Semi-preparative:* using a system equipped with a Waters Delta 600 pump, a Nucleosil 100-C18 column (250mm×10 mm) (Sugelabor, Spain) coupled to a precolumn (50mm×10 mm) packed with the same stationary phase. A differential refractometer (Varian, model 9040) set to 35 °C and a fraction collector (Waters) were used. 80% MeOH: 20% $H_2O$ was the mobile phase (2.4 ml min $^1$), and the column temperature was kept constant at 40 °C.

**Method 8 (standard for short length alkanes)**

*Analytic:* on a 5 μM LiChroCart 125-4 RP 18 column (Merck). The initial solvent composition was 20% methanol-80% phosphate buffer (20 mM), pH 4.8, reaching 100% methanol within 14 min at a flow rate of 1 ml $min^{-1}$. Detection was performed both by light scattering (temp 68.2 °C, 1.8 l/min).

*Semi-preparative:* on a 5 μM LiChroCart 125-4 RP 18 column (Merck) at a flow rate of 1 ml $min^{-1}$. For sufficient separation, the solvent system, consisting of methanol (eluent A) and ammonium acetate buffer (20 mM, pH 4.8) (eluent B), was started in a ratio of 20% A and 80% B and reached 70% A and 30% B within 12.5 min, and then it was changed to 100% A within 30 s and held constant for another 5 min.

**Method 9 (standard for medium and long length alkanes)**

*Analytic:* on a 2.7 μM Halo C8 (150x4.6 mm) column with MeOH:$H_2O$:acetic acid (750:250:4) as buffer A and acetonitrile:methanol:THF:acetic acid (500:375:125:4) as buffer B at a flow rate of 0.8 ml $min^{-1}$. Gradient was performed from 100 buffer A to 100% buffer B in 60 min. Column was kept at 35°C and elution was performed at 55 atm and 0.8 ml/ml. Detection was performed both by light scattering (temp 68.2 °C, 1.8 1/min).

*Semi-preparative:* on a 2.7 μM Halo C8 (150×4.6 mm) column with MeOH:$H_2O$:acetic acid (750:250:4) as buffer A and acetonitrile:methanol:THF:acetic acid (500:375:125:4) as buffer B and at a flow rate of 2.2 ml $min^{-1}$. Gradient was performed from 100 buffer A to 100% buffer B in 60 min. Column was kept at 35°C and elution was performed at 55 atm and 0.8 ml/ml. Detection was performed both by light scattering (temp 68.2 °C, 1.8 l/min).

**Method 10**

*Analytic 1*: on a Nucleosil 100-C18 column (250 × 4.6 mm) using isocratic program of 88% A and 12% B in 30 min at a flow rate of 0.3 ml/min. Mobile phase A consisted of 0.1% formic acid while mobile phase B consisted of 0.1% formic acid in a 95:5 acetonitrile/water solution. Diode array detector (DAD) was set at 290 nm.

*Analytic 1*: on a SC125/Lichrospher column (250 × 4.6 mm). The mobile phase was 0.01% (vol/vol) $H_3PO_4$ (87%) and 50% (vol/vol) methanol at 1.0 ml min $^1$. The column was kept constant at 40 °C. A differential refractometer (Waters, model 2410) was used and set to a constant temperature of 35 °C.

*Semi-preparative:* using a system equipped with a Waters Delta 600 pump, a Nucleosil 100-C18 column

(250mm×10 mm) (Sugelabor, Spain) coupled to a precolumn (50mm×10 mm) packed with the same stationary phase. A differential refractometer (Varian, model 9040) set to 35 °C and a fraction collector (Waters) were used. 0.01% (vol/vol) $H_3PO_4$ (87%) and 50% (vol/vol) methanol was the mobile phase (2.0 ml min [1]), and the column temperature was kept constant at 40 °C.

### *8. Incorporation of (H) derivatives to the poly(A)-nitrilotriacetic-Co(II) complex*

The final step in the labeled metabolite development is the incubation of the histidine functionalized Cy3-metabolites with poly(A)-nitrilotriacetic-Co(II) complexes in 50 mM phosphate buffer, 50 mM NaCl, pH 7.5 for 1 hour at 25°C. When required, DMSO was added to increase substrate solubility up to 50%. Briefly, 0.015 mmol (13 mg) **(H)** was dissolved in 5 ml 50 mM phosphate buffer, 150 mM NaCl, pH 7.5 (PBS), containing up to 50% DMSO depending on the solubility of the molecule, with 0.025 mmol **(B)** in a 15 ml falcon tube which was placed on a rotatory shaker for 1 h at 25°C. To ensure that each **(H)** molecule binds to **(B)** through both of its His residues, analytical HPLC and $^{59}$Co-NMR analyses were performed (see data in Table S1) and only derivatives incorporating single Cy3-labelled molecules **(I)** were purified by semipreparative reverse-phase HPLC (Prontosil-AQ, 5 lm, 120 A, 250 · 8 mm column equipped with a Prontosil-AQ, 5 lm, 120 A, 33 · 8 mm pre-column, and compounds were eluted with acetonitrile (20% for 5 min, followed by linear gradients to 45% in 5 min, to 50% in 7 min and to 100% in 2 min) in triethylammonium hydrogen carbonate buffer (0.01 M, pH 8.6) at a flow of 0.4 to 3 mL/min. Overall, yields higher than 93% were achieved. Fractions containing labelled metabolites were pooled and solvent evaporated. Molecules were dissolved in PBS buffer containing 50% DMSO and stored in 384 microtiter plates at-70°C until used at concentration of 40 μM.

*Reaction scheme (a general schematic metabolite "M" is represented)*:*

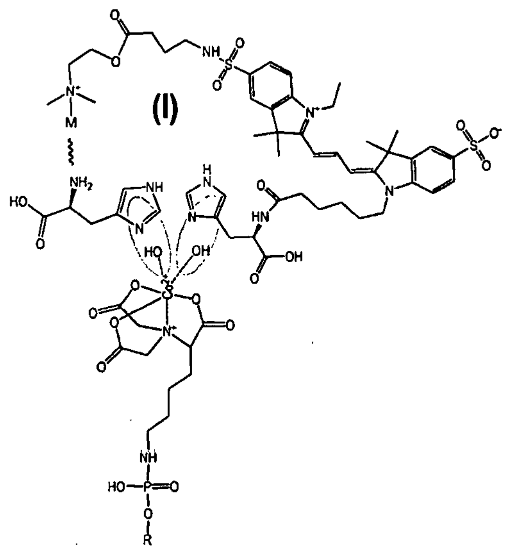

* R, indicated the Poly(A) tail used to bind to the glass slides.

*9. Binding of (H) to gold nanoparticles*

Au-6,8-dithioctic acid (TA) clusters were synthesized as described by Abad et al., J Am Chem Soc 127, 5689 (2005) and used to create Au-TA-ANTA-Co(II)-metabolite-Cy3 clusters. The Au-TA clusters were linked to **(H)** by overnight amidation in a single step in the presence of 3 mM *N*-hydroxysuccinimide (NHS, Fluka) and 3 mM 1-ethyl-3-[3¢-(dimethylamino)propyl]carbodiimide (EDC, Sigma) in 20 mM HEPES buffer (pH 7.5). Further purification of nanoparticles **(J)** containing Cy3 labeled metabolites was carried out by ultrafiltration through low-adsorption hydrophilic 30000 NMWL cutoff membranes (regenerated cellulose, Amicon). As an average value, a concentration of $9 \times 10^{10}$ particles/ml of diameter ~2.9 ± 0.8 nm that corresponds to a surface area of ~ 141 ± 3 $cm^2$/ml, binds 62.5 pmol of **(H)**.

*Reaction scheme (a general schematic metabolite "M" is represented)*:

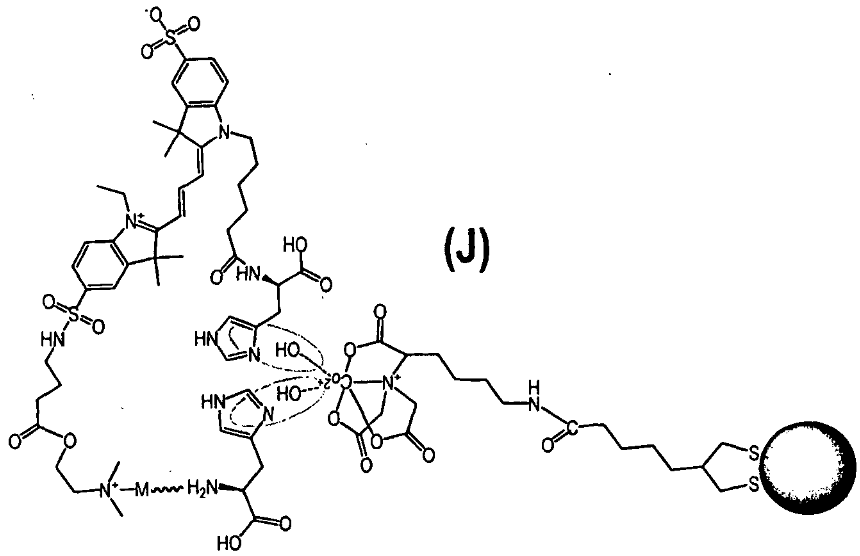

(J)

SYNTHESIS EXAMPLE 1:

***Step (1) to (5) are described above.***

***Step 6. X-Gal source***

5-Bromo-4-chloro-3-indolyl β-D-galactopyranoside (X-Gal) was provided by Roche Diagnostics (IN, USA) (ref. 10651745001), further reconstituted and diluted in DMSO, and stored at-70°C until used at a concentration of 100 mg/ml.

***Step 7. Formation of iodide X-Gal, further incorporation of histidine to iodide-X-Gal and formation of Cy3-labeled X-Gal***

The iodide halogenation of X-Gal was performed via $I^-$ as follows. The reaction mixture was incubated at 37°C with X-Gal (0.080 mmol; 32.69 mg dissolved in 0.1 ml DMSO), KI (75 mM), 2 mM NADH, and REBr dehalogenase (4 mg/ml, 100 μL) in phosphate buffer (20 mM, pH 7.8) at a final volume of 0.5 ml. The final volume of DMSO was keep at 20% v/v in order to maintain the solubility of the X-Gal. After 24 hour of incubation reaction product was separated by HPLC on a Hypersil 5 μM C-18 column (250x4.6 mm, Macherey-Nagel) equilibrated with $KH_2PO_4$ (50 mM) and acetonitrile (85:15 v/v). Runs were performed by gradient elution from a starting mobile phase of $KH_2PO_4$ (50 mM) and acetonitrile (85:15 v/v) to a final mobile phase consisting of $KH_2PO_4$ (50 mM) and acetonitrile (60:40 v/v). The purified iodide X-Gal was found to be 95% pure (13.8 g, 26% yield; white solid crystalline powder). Iodide X-Gal was reconstituted and diluted in DMSO and stored at -70°C until used at a concentration of 10 mg/ml. HRMS data clearly show that the enzyme incorporated two "I" per molecule. HRMS: calculated for $C_{14}H_{13}BrClI_2NO_6$, 658.7704, $[M^+H^+]$ found was 659.7770.

*Reaction scheme:*

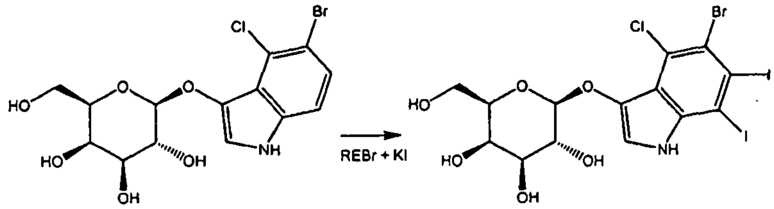

Iodide X-Gal was further functionalized via incorporation of a histidine tag. The incorporation of histidine was performed in the presence of the non-nucleophilic base and proton sponge 1,8-bis-(dimethylamino)-napthalene (Sigma) as described by R.A. Kaufman *et al.* (*loc. cit.*) and F. Mazzetti and R.M. Lemmon (*loc. cit.*) with small modifications. Briefly, to a solution of iodide X-Gal (0.1 mmol; 65.8 mg), histidine (1 mmol, 156 mg), and 1,8-bis-(dimethylamino)-napthalene at a final concentration of 10 mM, in $CH_3CN$. The temperature was controlled at 32°C. After 100 min incubation the product was recovered by evaporating the $CH_3CN$. The corresponding product was purified by semipreparative reverse-phase HPLC (Prontosil-AQ, 5 lm, 120 A, 250 · 8 mm column equipped with a Prontosil-AQ, 5 lm, 120 A, 33 · 8 mm pre-column, and compounds were eluted with acetonitrile (20% for 5 min, followed by linear gradients to 45% in 5 min, to 50% in 7 min and to 100% in 2 min) in triethylammonium hydrogen carbonate buffer (0.01 M, pH 8.6) at a flow of 0.4 to 3 mL/min. The purified metabolite was found to be 98% pure (21 mg, 32% yield; white solid crystalline powder). Mass spectrometry was used to confirm the structure. HRMS: calculated for $C_{20}H_{21}BrClIN_4O_8$ was 685.9276, [$M^+H^+$] found 686.9266. As shown, the result was within of the calculated molecular mass.

*Reaction scheme:*

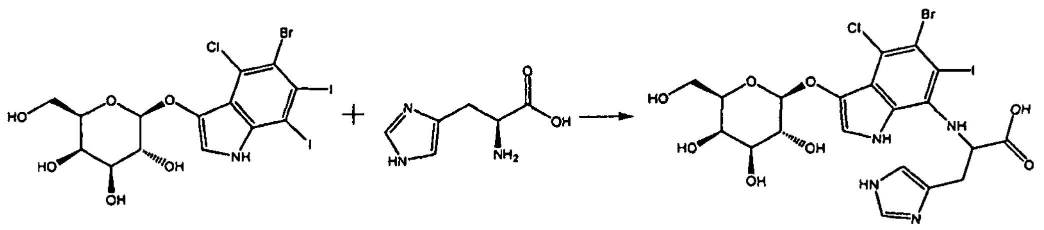

The corresponding labeled quaternary ammonium X-Gal were obtained in the presence of 1,8-bis-(dimethylamino)-napthalene (Sigma) as described by R.A. Kaufman *et al.* (*loc.* cit.) and F. Mazzetti and R.M. Lemmon (*loc. cit.*) with small modifications. Briefly, the general method for the synthesis of quaternary amines is as follows. Reaction mixture (2 ml) contains histidine tagged iodide-X-Gal (0.078 mmol, 53.4 mg), 0.78 mmol of **(E)** and 1,8-bis-(dimethylamino)-napthalene at a final concentration of 100 mM in DMSO. The temperature was controlled at 32°C. After 100 min incubation the product was recovered by evaporating the $CH_3CN$. The labeled product was purified by semipreparative reverse-phase HPLC (Prontosil-AQ, 5 lm, 120 A, 250 · 8 mm column equipped with a Prontosil-AQ, 5 lm, 120 A, 33 · 8 mm pre-column, and compounds were eluted with acetonitrile (20% for 5 min, followed by linear gradients to 45% in 5 min, to 50% in 7 min and to 100% in 2 min) in triethylammonium hydrogen carbonate buffer (0.01 M, pH 8.6) at a flow of 0.4 to 3 mL/min. The purified metabolite was found to be 98% pure (47.3 mg, 41% yield; white solid crystalline powder). Mass spectrometry was used to confirm the structure. HRMS: calculated for $C_{65}H_{82}BrClN_{11}O_{15}S_2$ was 1482.4153, [$M^+H^+$] found 1483.4106.

*Reaction scheme*

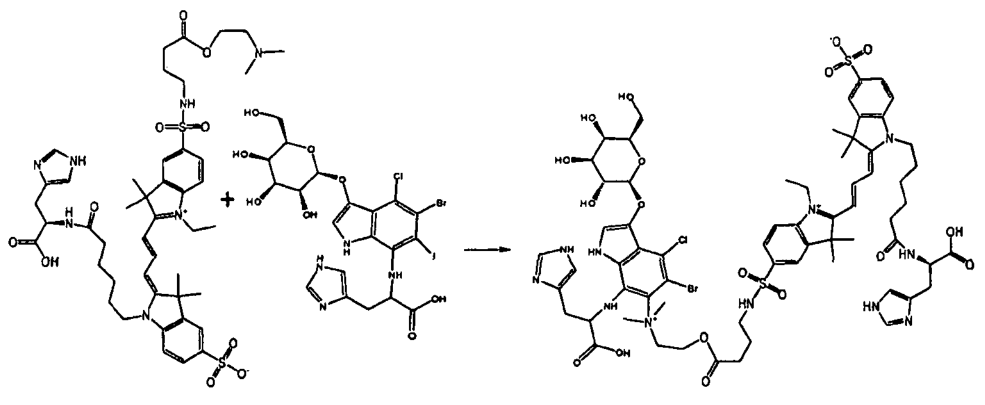

*Steps (8) and (9) are as described above.*

**EXAMPLE 6:**

**SM spotting and detection of protein-SM transformations.**

SM-Cy3s (0.25 nL droplets of 3.5 pmol/l in DMSO/PBS = 1:1; spot size 400 μm diameter) were spotted by means of a MicroGrid II micro-arrayer (Biorobotics) onto a glass slide, followed by fixation by cross-linking through the poly A tail. Each SM was spotted in triplicate on each slide. Buffer controls were applied for comparison. Sixty μl of cell lysate at a protein concentration of 0.1 mg/ml in PBS buffer was deposited on the slide and incubated at room temperature for 30 to 180 min. PBS buffer was used as a control. The slide was then washed with PBS and deionized water, dried by standard array slide centrifugation protocol and fluorescence intensities of the spots were measured with a microarray laser scanning system (Axon) set to a pmt of 500 and 100% laser power. Signals were analyzed and quantified using GenePix pro 4.1 software (Axon). Through the analysis of three micro-arrays of three biological replicates, average values and standard deviations were calculated using the Microsoft Excel programme. The average deviation is given in the caption of Table 3. Each data point was normalized to the signal intensity obtained with X-Gal-Cy3 and pure β-galactosidase (150 pg/ml), and normalized signal intensities were compared to those produced by the control array incubated with buffer. Normalization with the Cy3 β-Gal-signal intensity eliminated errors of signal intensity variation between arrays. All values given in Table 3 are therefore Cy3-signal corrected signal intensities after lysate incubation compared to buffer-only incubation. On the average of four Cy3-signal intensity measurements, the signal intensity of SM-Cy3 on the buffer-incubated array was 0.2% lower than the signal intensity of lysate-incubated array, showing high reproducibility of the Cy3-signal intensity and thus the legitimacy of using it as standardization factor.

**EXAMPLE 7:**

**Data Analysis.** After background subtraction, signal intensities for each replica were normalized and manually analyzed using Excel program (Microsoft) and GenePix Pro 6 Demo Program (http://www.moleculardevices.com/product literature/family links.php?familyid=14). MultiExperiment Viewer software (Sun Microsystems Inc) was used to visualize and compare differences in signal intensity.

**EXAMPLE 8:**

**Construction of (meta)genomic libraries.** DNA was extracted from three environments which differdiffering by in regard to the species composition and richness and main environmental constraints and the corresponding insert-libraries were constructed.

*Kolguev Island coastal seawater* (KOL): A 200 ml sample of the coastal seawater of Kolguev Island (Barents Sea, Russia) was placed into a 1 L Erlenmeyer flask containing sterile crude oil (Arabian light, 0.5 % (vol/vol)) and nutrients ($[NH4]_2PO_4$, 0.05% (w/vol)). After four weeks of incubation at 4 °C on a rotary shaker, total DNA was extracted the culture with G'NOME DNA Extraction Kit (Qbiogene; Carlsbad, California), and a metagenome expression library in the bacteriophage lambda-based ZAP phagemid vector (ZAP Express Kit, Stratagene), was constructed as described in the manufacturers' protocols. A library of 8 x $10^6$ phage particles, average insert size about 6 kbp, was thereby generated. Phage particles were used to infect *E. coli* XL1 Blue MRF' and subsequent mass excision was performed by using *E. coli* XLOLR cells, as recommended by the supplier. Vulcano *Island (VUL)*: The fosmid library was established from the DNA isolated from the enrichment of microbial community from acidic pool of Porto Levante on Vulcano Island, Italy. Sulphfur and iron-containing sandy volcanic acidic (pH 1.5-4) hydrothermal pool sample was enriched with the medium 874 (pH 1.7) (DSMZ, http://www.dsmz.de) containing 0.1% (w/vol) yeast extract and was incubated for 4 weeks at 45°C with shaking. The total amount of fosmid clones was 11520. The DNA was extracted using G'NOME DNA Extraction Kit (BIO101, Qbiogene) and was cloned using Fosmid Library Production Kit (Epicentre) as recommended by the suppliers.

L'Atalante *seawater-brine interface sample (L'A):* The brineseawater interface above hypersaline anoxic basin L'Atalante (Eastern Mediterranean Sea) was sampled during the MedBio2 oceanographic cruise in December 2006 from the depth of 3.431 meters. The 50 mL-samples were placed into sterile 100 ml Hungate bottles containing resazurine (anoxia indicator), 1 g/L yeast extract and 2 mM $^{13}$C-glucose. The salinity measured immediately after the cast was 180 g/L (NaCl). After six months of incubation at 14°C in the dark, the total DNA was extracted with G'NOME DNA Extraction Kit (BIO101, Qbiogene) and was further cloned using Fosmid Library Production Kit (Epicentre) as recommended by the suppliers.

*P. putida* KT2440: the total DNA was extracted with G'NOME DNA Extraction Kit (BIO101, Qbiogene) from 100 ml culture of this bacterium grown as described above and was further cloned using Fosmid Library Production Kit (Epicentre) as recommended by the suppliers.

**EXAMPLE 9:**

**Culture conditions and general procedure for the preparation of protein lysates.** Cells of *P. putida* KT2440 were grown at 30°C in 100 mL-flasks filled with 10 mL minimal medium (MM) prepared as follows. A solution with "Epure" water containing $(NH_4)_2SO_4$ (2 g/L), $Na_2HPO_4 12H_2O$ (6 g/L), $KH_2PO_4$ (3 g/L) and NaCl (3 g/L) was adjusted to pH 7.0 ± 0.2 and then autoclaved. The medium was supplemented with 20 mM $MgSO_4$, 10 mM $FeSO_4$ and 15 mM Na-succinate (from a stock solution sterilized by filtration through a 0.22 μm filter (Millipore)). Like in case of L'A and Volcano libraries, the individual clones were incubated overnight without shaking at 37°C in LB medium, 12.5 g/ml chloramphenicol, in 384 microtiter plates. An aliquot of 10 μl each culture were pooled together in appropriate flasks filled with 1L medium and subsequently incubated 6 additional hours ($OD_{600nm}$ 1.5) at 37°C. For lambda phage Kolguev library, mass excision in *E. coli* XLOLR was done following the protocol recommended by the supplier. The pool of clones (from phagemids of fosmids) was grown with shaking at 37°C in LB medium, with 50 g/ml kanamycin until $OD_{600nm}$ 1.5. After cultivation the cells were harvested by centrifugation (5000 g) for 15 min to yield 2-3 g/L of pellet. The cell pellet was frozen at -80°C overnight and then thawed. Cold PBS buffer was added directly to the frozen pellets (1.2 ml per 0.3 grs cell pellet). The mixture was vortexed to homogeneity and subsequently sonicated for 2 min (total time). The extract was centrifuged for 15 min at 15,000 g to separate cell debris and intact cells. The supernatant was carefully collected avoiding disturbing the pellet, and transferred to new tubes. Then, the extracts were immersed in liquid nitrogen and subjected to lyophilisation in order to avoid volatile contaminants. After that, extracts were resuspended in 1.2 mL PBS buffer and protein concentration was determined by a standard procedure (38) and further fixed to 0.1 mg/ml.

**EXAMPLE 10:**

**Representative procedure for the synthesis of SMs-Cy3 gold nanoparticles for identification and N-terminal sequencing of SM-acting proteins.** Au-6,8-dithioctic acid (TA) clusters were synthesized as described by Abad *et al*. (36) and used to create Au-TA-ANTA-Co(II)-SMs-Cy3 clusters. Briefly, the Au-TA clusters were incorporated to the ANTA-Co(II)-SMs-Cy3 by overnight amidation in a single step in the presence of 3 mM *N*-hydroxysuccinimide (NHS, Fluka) and 3 mM 1-ethyl-3-[3 ¢ -(dimethylamino)propyl]carbodiimide (EDC, Sigma) in 20 mM HEPES buffer (pH 7.5).13 Further purification was carried out by ultrafiltration through low-adsorption hydrophilic 30 000 NMWL cutoff membranes (regenerated cellulose, Amicon). Enzymatic binding was carried out by incubation of the functionalized nanoparticles (40 g $cm^{-3}$) in PBS buffer, pH 7.5 overnight at room temperature with protein solution (0.1 mg/ml in PBS). Au-TA-ANTA-Co2+-protein nanoparticles were separated from unbound enzyme molecules by ultrafiltration through low-adsorption hydrophilic 100 000 NMWL cutoff membranes (Amicon). Identification of bound proteins was performed by *in situ* trypsin digestion and ESI-Q-TOF MS/MS mapping (M. Ferrer et al., Nature 445, 91 (2007)) that provides improved mass measurement accuracy for peptide sequencing and enables unambiguous protein identification. ESI-Q-TOF MS/MS analyses were performed at the Sequencing Core Facility of the Autonomous University of Madrid. For each experiment, up to three binding experiments were prepared and analyzed.

**EXAMPLE 11:**

**PCR amplification of genes encoding hypothetical proteins from *P. putida* KT2440 and metagenomic libraries.**

*P. putida* KT2440 and metagenomic hypothetical proteins were amplified by PCR from genomic and metagenomic DNA using the set of primers detailed in Table 4. Cycling parameters were 2 min at 95°C followed by 25 cycles at 95°C for 30 s, 66°C for 30 s, and 72°C for 20-140s (see specific elongation temperature for each protein inTable 4), and ending with 10 min at 72°C. KOL-1, -2 and -7 and VUL-9 proteins were amplified by PCR from the corresponding metagenomic DNA library using the set of primers detailed in Table 4. Cycling parameters were 5 min at 95°C followed by 25 cycles at 95°C for 1 min, 55°C for 1 min, and 72°C for 1 min, and ending with 7 min at 72°C. PCR products were ligated in pGEMT plasmid (Promega).

**EXAMPLE 12:**

**Gene cloning, expression and purification.** All proteins characterized in this study were cloned into pET-41 Ek/LIC vector (Novagen) and expressed with an N-terminal fusion to 6xHis tag, according to manufacturer's instructions and plasmids were subsequently isolated and introduced into *E. coli* ORIGAMI(DE3)pLysS expression host. Full-length of gene coding for proteins were amplified from the *P. putida* genome by polymerase chain reaction (PCR), whereas full-length of proteins from libraries were amplified with degenerated primers based on the Q-TOF peptide obtained (Table 4). Proteins were expressed *in Escherichia coli* strain ORIGAMI(DE3)pLysS (Novagen). Cultures were grown

overnight in Luria-Bertani medium containing 100 mg/ml ampicillin and 50 mg/ml kanamycin, then diluted 1:100 in fresh medium. Cells were grown at 37°C to a final $OD_{600}$ of 0.5, induced with 1 mM isopropyl--D-thiogalactopyranoside (IPTG) and incubated at 37°C for an additional 4 h. Cell pellets were collected by centrifugation at 4 1C for 20 min at 8,000g. Pellets were then suspended in 25 ml prechilled lysis buffer (Complete EDTA-free protease inhibitor tablet (Roche), 150 mM NaCl, 1 mM dithiothreitol, 50 mM Hepes, pH 7.0, 5 mM imidazole) and lysed by sonication on ice for 3 min with 30 s intervals. Cell lysates were centrifuged once more at 4°C for 20 min at 30,000 g, and the soluble fractions were retained. Proteins were purified using a 1-ml HisTag (Novagen) according to the manufacturer's protocols and eluted in 250 mM imidazole and 50 mM HEPES, pH 7.0. Elutions of 500 1 were collected, pooled and concentrated 100-fold using Microcon YM-3 spin columns (Millipore). The molarities of all purified proteins were determined by using the corresponding extinction coefficient. Purified proteins were stored at -80 °C until further use.

**EXAMPLE 13:**

**Receiver Operating Characteristic (ROC) analysis.**

The "receiver operating characteristic" (ROC) analysis is used to evaluate the performance of a binary classifier separating two populations (positive and negative cases) and it is becoming a standard in evaluating and comparing prediction methods (T. Fawcett, Pattern Recogn Lett 27, 861 (2006)). This analysis can be applied to classifiers (prediction methods) which associate a numerical score to each case. Ideally the classifier would tend to associate high scores to the positive cases and low scores to the negative ones (or the other way around). The analysis is performed by sorting all the cases (positives and negatives) by their associated scores. This sorted list is then cut at different score thresholds and for each cut the true and false positive rates (TPR and FPR) are calculated as

$$TPR=TP/(TP+FN)$$

$$FPR= 1-(TN/(TN+FP))$$

Where, TP: "true positives" - cases predicted as positives (above the threshold for that particular cut) which are in fact positives (correct predictions); FN: "false negatives" - cases predicted as negatives (below the threshold) which are actually positives (wrong predictions); TN: "true negatives"; FP: "false positives". "TP/(TP+FN) is also known as "sensitivity" and gives an idea of the fraction of positives recovered at this particular cut of the list (note that TP+FN is the total number of positive cases). "TN/(TN+FP)" is also known as "specificity".

The ROC plot is constructed by plotting TPR against FPR for the different cuts of the list. In such a representation, a random method (a classifier without discriminative power which spreads positives and negatives uniformly across the sorted list of scores) will produce a diagonal line from (0,0) to (1,1). Methods with some discriminative power will generate curves above this line. The higher the curve (closer to the (0,1) corner) the better the method. A method with a perfect discriminative power (which puts all the positives together at the top of the list, associated to the highest scores) would be represented by a single point at (0,1). A parameter based in this representation which quantifies the global performance of a method in the AUC ("area under curve"). The random method commented above will produce an AUC value of 0.50, while discriminative methods will produce AUC values between 0.50 and 1.00 (perfect discrimination).

In this study case, we used this analysis for evaluating the ability of our array to discriminate compounds which are actually metabolized by *P. putida* from those which are not. The score is the fluorescence intensity, under the idea that there will be a positive relationship between the intensity in the array and the compound being metabolized in *P putida.* We assume that compounds metabolized by *P. putida* are those which act as substrates in one or more chemical reactions in the metabolism of *P. putida* as reconstructed from KEGG (cf. above) for that organism. We took as *P. putida* reactions those for which the EC code or the KEGG orthologs code (ko) are associated to a *P. putida* K2440 gene ("PPU" in KEGG nomenclature).

**EXAMPLE 14:**

**Calculation of Z-scores for comparing samples in terms of functional classes.** The z-score ($Z_i$) for a given intensity value ($I_i$) is calculated as:

$$Z_i = \frac{I_i - \overline{I}}{\sigma}$$

Where $\overline{I}$ and $\sigma$ are the average and standard deviation of the all the intensity values in the array respectively. A Zi value of 0 means that the intensity is right in the average. A positive value means that the intensity is higher than the average, and the other way around for negative values.

For each KEGG functional class for which more than 1676 compounds are in the array, we calculate the average $Z_i$ value of all the compounds belonging to that class ($\overline{Z}_i$). A high $\overline{Z}_i$ value indicates that this class of compounds is highly metabolized in that particular sample (array). Comparing the $\overline{Z}_i$ values for a given class in two arrays (samples) it is possible to known which metabolic activities are "emphasized" or "repressed" from one condition to the other. To quantify that, for each class we calculate the difference of its $\overline{Z}_i$ values in the two samples

$$( \Delta\overline{Z_i} = \overline{Z_{i2}} - \overline{Z_{i1}} ) \ .$$

It is important to note that the results in terms of which classes are over/under expressed are exactly the same using $Z_i$ or $I_i$ values since, by definition, they are correlative (see equation above). We used Zi values simply because they are easier to interpret in terms of relative intensities.

**EXAMPLE 15:**

**Hydrogenase activity and IR measurements.** The oxidation of $H_2$ (*$H_2$ uptake*) was followed spectrophotometrically due to the reduction of methyl viologen (MV) as described by De Lacey et al., J Biol Anorg Chem 9, 636 (2004):

$H_2$ $2e^-$ + $2H^+$ +$2BV^{2+}$ $2BV^{\cdot+}$

*Buffers:* The buffers used for the activity assay were: 1 mM MV in Tris-HCl 20 mM buffer pH 8.1 and sodium dithionite 10 or 100 mM in Tris-HCl 20 mM buffer pH 8.1.

Sample preparation: 0.2 mg/mL L'A62 protein in buffer Tris-HCl 20 mM pH 8.1. To this solution 1 μL of dithionite 10 mM solution is added.

The IR spectra was measured as described by De Lacey *et al.* using a protein solution of 12 mM in Tris-HCl 50 mM pH 8.1.

**EXAMPLE 16:**

**Construction rRNA gene clone libraries and clone sequencing.**

PCR amplification was performed with 0.1 ng DNA template. 16S rRNA genes were amplified using the Eubacteria-specific forward primer F27 (5'- AGAGTTTGATCMTGGCTCAG-3') in case of KOL and L'A and a universal F530 primer (5'-TCCGTGCCAGCAGCCGCCG-3') for VUL library, in all cases in the combination with the universal reverse primer R1492 (5'-CGGYTACCTTGTTACGACTT-3'). Amplification was done in 20 μl reaction volume with recombinant Taq DNA Polymerase (Invitrogen, Gemany) and original reagents, according to the basic PCR protocol, with an annealing temperature of 45°C (VUL) and 50°C C (L'A and KOL), for 30 cycles. PCR amplicons were purified by electrophoresis on 0.8% agarose gels, followed by isolation from excised bands using a QIAEX II Gel Extraction Kit (Qiagen, Germany). The purified PCR products were ligated into plasmid vector pCRII-TOPO (TOPO TA Cloning kit, Invitrogen, Germany) with subsequent transformation into electrocompetent cells of E. coli (TOP 10) (Invitrogen, Germany). After blue/white screening, randomly picked clones were resuspended in PCR-lysis solution A without proteinase K (67 mM Tris-Cl (pH 8.8); 16 mM $NH_4SO_4$; 5 M - mercaptoethanol; 6.7 mM MgCl 2; 6.7 M EDTA (pH 8.0) (Sambrook and Russel, 2002) and heated at 95C C for 5 min. The lysate (1μl) was used as DNA template for PCR amplification using primers M13F (5'-GACGTTGTAAAACGACGGCCAG-3') and M13R (5'-GAGGAAACAGCTATGACCATG-3'). After verification on the agarose gel, PCR products were purified with MinElute 96 UF PCR purification kit (Qiagen, Germany) and sequenced with M13 and M13R primers according to the protocol for BigDye Terminator v1.1 Cycle Sequencing Kit from Applied Biosystems (USA).

**EXAMPLE 17:**

**Dose-response curves determined with *E. coli* β-galactosidase (β-Gal).**

To prove that the present array can discern active and non-active proteins and in parallel to determine the sensitivity of the system, we determined the molecule dose-response behaviour of active and inactive -Gal and *vice versa.* 5-Bromo-4-chloro-3-indolyl-D-galactopyranoside (X-Gal) modified with Cy3 as described in SYNTHESIS EXAMPLE 1, was used as substrate. Figure 4 shows the X-Gal-associated pixel intensity from the scanned slides plotted against the amount of X-Gal. As shown, 30 min incubation with a solution of only 5 ng pure β-Gal $ml^{-1}$ at 20°C was sufficient to ensure 50% of maximum fluorescence ($F_{50}$) when 0.25 nl of a solution containing 0.12 pmol $ml^{-1}$ substrate was spotted (signal to noise (S/N) ratio above 71), while inactive protein was unable to release the Cy3 dye. Further, using 2.52 pmol X-Gal-Cy3 $ml^{-1}$ 50% of maximum fluorescence was reached above 1.86 ng β-Gal $ml^{-1}$ (signal saturation above 95 ng $ml^{-1}$). According to this data, micro-array analysis were further performed by spotting 0.25 nl of substrate solutions at concentration of 2.52 pmol $ml^{-1}$ and by arraying the slides with at least 0.10 mg protein lysate $ml^{-1}$ to ensure detection of all proteins in the extract (it should be noted that enzyme concentrations as low as 1.5 ng $ml^{-1}$ were sufficient to ensure appropriate detection with a S/N 10).

**EXAMPLE 18:**

**Characteristics of microbial communities examined.**

In order to assess the utility of the array for the analysis of complex microbial communities as represented in metagenomic libraries, we obtained samples from three habitats with distinct physico-chemical characteristics and therefore distinct microbial communities and metabolic characteristics. The general characteristics of the three habitats are as follows: (*i*) acidic (pH 1.0-3) sulfur- and iron-rich (from 3 to more than 500 mg/l) sediments of a hydrothermal pool (25-75°C) of Porto Di Levante, Vulcano Island (Italy) (*ii*) oil-polluted, cold (1°C) coastal seawater sampled near Kolguev Island, Barents Sea, Russia, and (*iii*) the seawater-brine interface of the deep hypersaline anoxic brine lake of the L'Atalante Basin, Eastern Mediterranean Sea (14°C). All samples were used to produce enrichments cultures to obtain higher biomass levels for the subsequent analyses. The Vulcano Island sediment was introduced into an acidic (pH 1.7) ferrous iron- and sulfate-rich liquid medium and incubated for 4 weeks at 45°C to produce enrichment VOL; the Koluev Island coastal water was enriched with crude oil and incubated for 4 weeks at 4°C to produce enrichment KOL; and the seawater:brine interface sample from the L'Atalante Basin was supplemented with glucose and yeast extract and incubated anaerobically for 6 months at 14°C, to produce enrichment L'A. Thus: the microbial communities obtained for analysis represent communities from very distinct, rather extreme habitats: a low energy, low nutrient, heavy metal-rich habitat (VOL), a nutrient and energy-rich, organic pollutant-contaminated habitat (KOL), and a hypersaline, anaerobic environment, inoculated with a sample taken also from a high pressure habitat but which was subsequently maintained at atmospheric pressure, so should contain facultative barophiles (L'A).

**EXAMPLE 19:**

**General *pan-reactome* considerations.**

As shown in Figure 6, the VUL reactome consisted of 807 compounds, the KOL reactome consisted of 1493 compounds, and the L'A reactome 2386. The restricted metabolic activity of the VUL sample was not unexpected, since the diversity of the community is low, the biomass concentration is low, and the prevailing physico-chemical conditions highly selective of a restricted metabolism. Similarly, the reactome of KOL was also not unexpected, since the excess carbon in the hydrocarbon-based enrichment leads to high cell densities and much recycling of cellular carbon in all its diverse forms. At first sight, it might seem that the extremely diverse metabolic profile of the L'A metagenome library is surprising, given the highly restricted diversity of the original community. However, it has been shown that a wide range of physico-chemical conditions prevail in the extremely steep chemoclines of the seawater:hypersaline brine interfaces of the brine lakes and this might select for organisms expressing a broader range of metabolic activities. In addition, it should be kept in mind that, on one hand, the oligotrophic environment selects for organisms expressing a wide range of nutrient scavenging systems constitutively at low levels, and in addition, anaerobic metabolism and salt and pressure tolerance systems not specified or expressed by the other two communities, and, on the other, the *E. coli* cellular environment may result in expression of a range of aerobic metabolism systems encoded, but not necessarily expressed under natural conditions, by the community organisms. Nevertheless, the exceptional richness of the metabolic profile of the L'A library is impressive.

**EXAMPLE 20:**

**The micro-array served as experimental platform to identify gene functions.**

The methodology presented here provides a new window to study the functional composition of single cells and microbial communities without apparent need of sequence information as well as to identify many uncharacterized gene-coding enzymes. This has been shown by combining the array concept with high-throughput mass spectrometry peptide sequencing using metabolite-containing nanoparticles. To further prove this hypothesis we extent this analysis not only for *P. putida* extracts but also for the metagenome-derived extracts to analyse the possibility of mining protein diversity.

To test this, we randomly select nine SMs exhibiting positive signals (fluorescence values up to 35512) in the micro-array: four for *P. putida lysates* (*cis*-2-hydroxypenta-2,4-dienoate, γ-carboxymuconolactone, 3-hydroxyanthranilate and dimethylallyl diphosphate) and five for KOL, VUL and L'A lysates (undecane, (S)-4,5-dihydroxypentan-2,3-dione, 2-bromo-1-chloropropane, phosphatidylinositol-4,5-bisphosphate and methyl viologen). To identify the proteins responsible for their transformation the corresponding Cy3 derivatives were synthesized, immobilized in a gold-magnetic nanoparticle, and further incubated with *P. putida* or library lysates (Figure 2, *right*). After 30 min at 20°C incubation, the gold particles were separated by either magnetic attachment or centrifugation (5000 g x 15 min) and the attached proteins were identified by trypsin digestion followed by Q-TOF sequencing. Using peptide sequence fragments, degenerated oligonucleotides were designed, the corresponding genes were amplified using (meta)genomic DNA, cloned into the pET30 Ek/LIC expression vector and the proteins purified with a 6 x His tag. Analysis of fluorescence emission when different amounts of pure protein were incubated with different concentration of substrates and *vice versa* revealed that the obtained results matched the reactome data (Table 3): Cy3 fluorescence emission increased when increasing the amount of both protein and substrate while inactive proteins do not (Figure 7).

*P. putida* proteins: Sequence analysis revealed the identity of proteins acting against *cis*-2-hydroxypenta-2,4-dienoate, γ-carboxymuconolactone, 3-hydroxyanthranilate and dimethylallyl diphosphate - all of them correspond to the hypothetical proteins with no assigned function annotated PP_1394, PP_1752, PP_2949 and PP_1642 (Table 4). However, the experimental analyses provided here suggest that these enzymes are accordingly new 2-keto-4-pentenoate hydratase, 3-carboxy-*cis*,*cis*-muconate cycloisomerase, 3-hydroxyanthranilate 3,4-dioxygenase and isopentenyl-diphosphate delta-isomerase (see Table 4). This has also been shown by examining the activity of the pure proteins against standard substrates (data not shown). The correlation of this identity with genomic context is as follows: here we observed that surrounding compounds are also metabolized by *P. putida* lysates, thus suggesting the presence of new metabolic pathways. A case of interest is the ability of protein PP_1394 to transform the conversion of *cis*-2-hydroxypenta-2,4-dienoate to *cis*-2-hydroxypenta-2,4-dienoate as a part of the biphenyl degradation pathway. This pathway is not annotated in KEGG for *P. putida* KT2440 strain (cf. above), in spite of the fact that strains F1, GB1 and W619 clearly possess some genes responsible for *p*-cymene and 4-chlorobiphenyl degradation (see http://www.genome.jp/kegg/). In contrast, array hits were found for the entire set of metabolites ranging from *p*-cymene to 2-hydroxy-6-oxo-7-methylocta-2,4-dienoate as well as from 4-chlorobiphenyl to *cis*-2,3-dihydro-2,3-dihydroxy-4'-chlorobiphenyl (fluorescence values up to 6931) (Figure 7) and further analysis of nanoparticle proteome analysis have also revealed the identity of proteins doing those transformations. These data suggest that the lysate of KT2440 contains enzymes that are able to metabolize those intermediates, even though genome information *per se* does not provide any evidence for that. This suggests in turn that many proteins may potentially enable, yet unknown, important catabolic activities expanding our knowledge on microbial metabolism of this bacterium.

*Metagenomic* proteins: Sequence analysis revealed that all of metagenomic proteins acting against undecane, (S)-4,5-dihydroxypentan-2,3-dione, 2-bromo-1-chloropropane, phosphatidylinositol-4,5-bisphosphate and methyl viologen exhibited a high degree of similarity to predicted hypothetical proteins with no function assigned (Table 4), except L'A62, a 162-amino acid polypeptide with a predicted molecular mass of 18,068 Da along with estimated pI of 4.55, that exhibited high similarity to a predicted [NiFe] hydrogenase from *Carboxythermus hydrogenoformans* ($7e^{-54}$). Data suggest these enzymes are new alkane hydroxylase, S-ribosylhomocysteine lyase, haloalkane dehalogenase, phosphatidylinositol-bisphosphatase and hydrogenase. To prove that assigned function are correct we select and preliminary characterized one of the most promising enzyme candidates, the L'A62 protein and reaffirmed this was indeed the case. A FTIR (Fourier Transform Infrared) spectrum of protein 62 was recorded at a final concentration of 8 M (Figure 7). The bands that appear in the 2150-1900 $cm^{-1}$ region are typical of the 1 carbonyl and 2 cyanide ligands of the active site of standard NiFe-hydrogenases (J.C. Fontecilla-Camps et al., Chem Rev 107, 4273 (2007)). The band of frequency value of 1947 $cm^{-1}$ and the group of bands between 2080 and 2095 $cm^{-1}$ correspond to the carbonyl ligand and the cyanide ligands respectively of this type of hydrogenases in the "unready" and "ready" oxidized states (A. De Lacey et al., Biochem Biophys Acta 832, 69 (1985)). The band at 1936 $cm^{-1}$ can be assigned to the carbonyl ligand of irreversibly inactivated enzyme, whereas the bands at 2060 and 2073 $cm^{-1}$ can be assigned to the cyanide ligands of the same state. Further, the $H_2$-uptake activity of protein 62 was measured in a spectrophotometer using methyl viologen as electron acceptor

(Figure 7, inset). The protein isolated under aerobic conditions had the typical lag-phase of several minutes in the activity profile of standard NiFe-hydrogenases in the "unready" oxidized state (V. M. Fernandez et al., Biochim Biophys Acta 832, 69 (1985)). Incubation under $H_2$ of the protein during several hours led to disappearing of the lag-phase and an increase of the maximum activity. This activation process is also a functional characteristic signature of standard NiFe-hydrogenases. Remarkably, the structural and functional data measured suggest an active site very similar to that of standard NiFe-hydrogenases, although the overall protein structure is unique up to date for this type enzymes (just 1 subunit of very small size, an amino acid sequence with no motifs for iron-sulfur clusters nor with the typical L1 or L2 signatures) (P.M. Vignais, B. Billoud, Chem Rev 107, 4206 (2007)).

**Table 1. Full description of synthetic methods used in the present study to synthesized all small molecules used for array imprinting**

| Compound | Molecular Formula | No. | FAB-HR [M+H]+ found | 59Co-NRM (300 MHz, CD3CN) | Source / Catalysts |
|---|---|---|---|---|---|
| gamma-Nonalactone | C9H16O2 | 1 | 1235,522 | 7610 | SIGMA-ALDRICH-FLUKA |
| epsilon-Caprolactone | C6H10O2 | 2 | 1193,441 | 7290 | SIGMA-ALDRICH-FLUKA |
| 10-Oxodecanoate | C10H18O3 | 3 | 1265,548 | 7090 | Linoleic acid + LIPOOXYGENASE from rice leaves (Oryza sativa cv. Aichiasahi) → (8E,12Z)-10-hydroperoxyoctadeca-8,12-dienoic acid + HYDROPEROXIDE LYASE from Penicillium sp. → 10-oxodecanoic acid |
| 1-Bromodecane | C10H21Br | 4 | 1300,478 | 6930 | Spectrum Chemicals |
| 2-Bromodecane | C10H21Br | 5 | 1300,478 | 8641 | Chemos GmbH |
| 10-Formyltetrahydrofolate | C20H23N7O7 | 6 | 1552,742 | 7230 | Serine + tetrahydrofolate + rabbit liver serine transhydroxymethylase → 5,10-methylenetetrahydrofolate |
| 4-Hydroxy-5-phenyltetrahydro-1,3-oxazin-2-one | C10H11NO3 | 7 | 1272,499 | 6120 | Felbamate → 3-Carbamoyl-2-phenylpropionaldehyde (reversible cyclization) → 4-Hydroxy-5-phenyltetrahydro-1,3-oxazin-2-one |
| 5-Chloro-3-methylcatechol | C7H7ClO2 | 8 | 1237,881 | 7080 | 3,5-Trichlorotoluene + TETRACHLOROBENZENE DIOXYGENASE of Ralstonia sp. PS12 → 5-Chloro-3-methylcatechol |
| 3-Methylcrotonyl-CoA | C26H42N7O17P3S | 9 | 1928,934 | 6980 | Isovaleryl-CoA + 3-METHYLCROTONYL-COENZYME A CARBOXYLASE Arabidopsis thaliana → 3-Methylcrotonyl-CoA |
| 2-Chloromaleylacetate | C6H5ClO5 | 10 | 1271,853 | 14470 | 2,6-dichloro-p-hydroquinone + 2,6-DICHLORO-P-HYDROQUINONE 1,2-DIOXYGENASE of Sphingomonas chlorophenolica → 2-Chloromaleylacetate |
| 1,3-Dichloropentane | C5H10Cl2 | 11 | 1220,337 | 14350 | SIGMA-ALDRICH-FLUKA |
| 11-Hydroxyundecanoate | C11H22O3 | 12 | 1281,590 | 14190 | SIGMA-ALDRICH-FLUKA |
| 11-Oxoundecanoate | C11H21O3 | 13 | 1280,583 | 7000 | Eicosaenoic acid + LIPOOXYGENASE from rice leaves (Oryza sativa cv. Aichiasahi) → (8E,12Z)-10-hydroperoxyundeca-8,12-dienoic acid + HYDROPEROXIDE LYASE from Penicillium sp. → 10-oxodecanoic acid |

| | | | | | |
|---|---|---|---|---|---|
| Oxatriazole | CHN3O | 14 | 1150,335 | 15924 | Cyclopentane + TRANSITION METAL ALKYLIDENE COMPLEXES → Oxatriazole |
| 1,2,3,5-Oxatriazole | CHN3O | 15 | 1150,335 | 9083 | Cyclopentane + TRANSITION METAL ALKYLIDENE COMPLEXES → 1,2,3,5-Oxatriazole |
| 1,2,3-Oxadiazole | C2H2N2 O | 16 | 1149,347 | 7056 | Cyclopentane + TRANSITION METAL ALKYLIDENE COMPLEXES → 1,2,3-Oxatriazole |
| 1,2,3-Triazine | C3H3N3 | 17 | 1160,374 | 6630 | SIGMA-ALDRICH-FLUKA |
| v-Triazole | C2H3N3 | 18 | 1148,363 | 14520 | SIGMA-ALDRICH-FLUKA |
| (R)-1,2,4-Butanetriol | C4H10O 3 | 19 | 1185,418 | 14434 | SIGMA-ALDRICH-FLUKA |
| 1,4,2-Dioxazole | C2H3NO 2 | 20 | 1152,349 | 13630 | Cyclopentane + TRANSITION METAL ALKYLIDENE COMPLEXES → 1,4,2-Dioxazole |
| 1,3,4-Oxadiazine | C3H4N2 O | 21 | 1163,374 | 6800 | Cyanoacetylhydrazine + ω-bromoacetophenone → p-Bromoacetophenone-a-cyanoacetylhydrazone → 1,3,4-oxadiazine |
| 2-Methyl-1,3,4-oxadiazole | C3H4N2 O | 22 | 1163,374 | 13082 | Cyanoacetylhydrazine + ω-bromoacetophenone → p-Bromoacetophenone-a-cyanoacetylhydrazone → 2-methyl-1,3,4-oxadiazine |
| 1,3,5-Triazine | C3H3N3 | 23 | 1160,374 | 12710 | SIGMA-ALDRICH-FLUKA |
| (1R,2S)-1,2-Dihydronaphthalene-1,2-diol | C10H10 O2 | 24 | 1241,485 | 12350 | SIGMA-ALDRICH-FLUKA |
| 1,2,4-Trimethylbenzene | C9H12 | 25 | 1199,491 | 7380 | SIGMA-ALDRICH-FLUKA |
| Oxathiazine | C3H3NO S | 26 | 1180,420 | 7210 | THERMAL CYCLOREVERSION: Benzene + t-C4H9 (reflux) → Oxathiazine |
| 1,2,5-Oxadiazole | C2H2N2 O | 27 | 1149,347 | 7100 | Cyclopentane + TRANSITION METAL ALKYLIDENE COMPLEXES → 1,2,5-Oxatriazole |
| 2,4,6-Tribromophenol | C6H3Br3 O | 28 | 1410,098 | 8350 | SIGMA-ALDRICH-FLUKA |
| 1,2-Benzoquinone | C6H4O2 | 29 | 1187,394 | 12990 | SIGMA-ALDRICH-FLUKA |
| 1,4-Dichloro-2-butene | C4H6Cl2 | 30 | 1418,194 | 11810 | SIGMA-ALDRICH-FLUKA |
| 1,2-Didecanoyl-3-beta-D-galactosyl-sn-glycerol | C11H16 O10(C20 H40O4) | 31 | 1732,073 | 8210 | Galactosyldiacylglycerol + decanoic acid + 1,2-DIDECANOYL-3-BETA-D-GALACTOSYL-SN-GLYCEROL ACYLHYDROLASE → 1,2-Didecanoyl-3-beta-D-galactosyl-sn-glycerol |
| Decane-1,2-diol | C10H22 O2 | 32 | 1253,580 | 7350 | SIGMA-ALDRICH-FLUKA |
| Dodecane-1,2-diol | C12H26 | 33 | 1281,634 | 6280 | SIGMA-ALDRICH-FLUKA |

| | | | | | |
|---|---|---|---|---|---|
| | O2 | | | | |
| 1,2,4-Triazole | C2H3N3 | 34 | 1148,363 | 9590 | SIGMA-ALDRICH-FLUKA |
| 1,2-Dipropionyl-3-beta-D-galactosyl-sn-glycerol | C11H16 O10(C6H 12O4) | 35 | 1519,698 | 8820 | Galactosyldiacylglycerol + propionic acid + 1,2-DIPROPANOYL-3-BETA-D-GALACTOSYL-SN-GLYCEROL ACYLHYDROLASE → 1,2-Didecanoyl-3-beta-D-galactosyl-sn-glycerol |
| 1,2-Dibutyryl-3-beta-D-galactosyl-sn-glycerol | C17H30 O10 | 36 | 1547,752 | 7840 | Galactosyldiacylglycerol + butyric acid + 1,2-DIBUTYRYL-3-BETA-D-GALACTOSYL-SN-GLYCEROL ACYLHYDROLASE → 1,2-Didecanoyl-3-beta-D-galactosyl-sn-glycerol |
| cis-1,2-Dihydroxy-1,2-dihydro-8-methylnaphthale | C11H12 O2 | 37 | 1255,512 | 6940 | 8-Methylnaphthale + 1,2-DIHYDROXY-8-METHYLNAPHTHALENE DIOXYGENASE Pseudomonas sp. → cis-1,2-Dihydroxy-1,2-dihydro-8-methylnaphthale |
| 1,2-Butanediol | C4H10O 2 | 38 | 1169,419 | 6590 | SIGMA-ALDRICH-FLUKA |
| 1,2-Dihexanoyl-3-beta-D-galactosyl-sn-glycerol | C11H16 O10(C12 H24O4) | 39 | 1603,859 | 7360 | Galactosyldiacylglycerol + hexanoic acid + 1,2-DIHEXANOYL-3-BETA-D-GALACTOSYL-SN-GLYCEROL ACYLHYDROLASE → 1,2-Didecanoyl-3-beta-D-galactosyl-sn-glycerol |
| 1,2-Dihydroxynaphthalene | C10H8O 2 | 40 | 1239,469 | 7270 | Naphthale + 1,2-DIHYDROXY-8-METHYLNAPHTHALENE DIOXYGENASE Pseudomonas sp. → 1,2-Dihydroxynaphthalene |
| 1,2-Diazole | C3H4N2 | 41 | 1147,375 | 6450 | SIGMA-ALDRICH-FLUKA |
| 1,1-Dibromopentane | C5H10Br 2 | 42 | 1309,239 | 10250 | SIGMA-ALDRICH-FLUKA |
| 1,2-Dichlorobenzene | C6H4Cl2 | 43 | 1226,301 | 8450 | SIGMA-ALDRICH-FLUKA |
| 1,2-Dichloropentane | C5H10Cl 2 | 44 | 1220,337 | 7690 | SIGMA-ALDRICH-FLUKA |
| 1,1-Dichloropentane | C5H10Cl 2 | 45 | 1220,337 | 7350 | SIGMA-ALDRICH-FLUKA |
| 3-Hydroxy-1-indanone | C9H8O2 | 46 | 1227,458 | 6572 | 1-Indanone + METQGENOMIC P450 MONOOXYGENASE → 3-Hydroxy-1-indanone |
| 1,5-Diaminopentane | C5H14N2 | 47 | 1181,476 | 8308 | SIGMA-ALDRICH-FLUKA |
| (1S,2S)-1,2-Dihydronaphthalene-1,2-diol | C10H10 O2 | 48 | 1241,485 | 7944 | Naphthale + 1,2-DIHYDROXY-8-METHYLNAPHTHALENE DIOXYGENASE Pseudomonas sp. → 1,2-Dihydroxynaphthalene |
| 1,2-Didodecanoyl-3-beta-D-galactosyl-sn-glycerol | C11H16 O10(C24 H48O4) | 49 | 1788,181 | 6168 | Galactosyldiacylglycerol + dodecanoic acid + 1,2-DIDODECANOYL-3-BETA-D-GALACTOSYL-SN-GLYCEROL ACYLHYDROLASE → 1,2-Didecanoyl-3-beta-D-galactosyl-sn-glycerol |

| | | | | | |
|---|---|---|---|---|---|
| 1,2-Dihydroxydibenzothiophene | C12H8O2S | 50 | 1295,551 | 7470 | 1-Hydroxy-2-oxolimonene + 1-HYDROXY-2-OXOLIMONENE 1,2-MONOOXYGENASE → 1,2-Dihydroxydibenzothiophene |
| cis-1,2-Dihydroxy-1,2-dihydrodibenzothiophene | C12H10O2S | 51 | 1297,567 | 6470 | 1-Hydroxy-2-oxolimonene + 1-HYDROXY-2-OXOLIMONENE 1,2-MONOOXYGENASE → cis-1,2-Dihydroxy-1,2-dihydrodibenzothiophene |
| 1,2-Didodecanoyl-3-O-(alpha-D-glucopyranosyl(1->2)-O-alpha-D-glucopyranosyl)sn-glycerol | C17H26O15(C24H48O4) | 52 | 1934,324 | 7309 | Didodecanoylglycerol + alpha-D-glucopyranosyl(1->2)-O-alpha-D-glucopyranosyl + 1,2-DIDECANOYL-alpha-D-glucopyranosyl(1->2)-O-alpha-D-glucopyranosyl-SN-GLYCEROL ACYLHYDROLASE → 1,2-Didodecanoyl-3-O-(alpha-D-glucopyranosyl(1->2)-O-alpha-D-glucopyranosyl)sn-glycerol |
| 1,2-Didodecanoyl-3-O-(alpha-D-glucopyranosyl)-sn-glycerol | C11H16O10(C24H48O4) | 53 | 1788,181 | 6190 | Didodecanoylglycerol + alpha-D-glucopyranosyl + 1,2-DIDECANOYL-alpha-D-glucopyranosyl-SN-GLYCEROL ACYLHYDROLASE → 1,2-Didodecanoyl-3-O-(alpha-D-glucopyranosyl)-sn-glycerolglycerol |
| 1,2-Didodecanoyl-sn-glycerol | C5H6O5(C24H48O4) | 54 | 1626,039 | 7367 | SIGMA-ALDRICH-FLUKA |
| 1,2-Oxazine | C4H9NO | 55 | 1166,418 | 6451 | SIGMA-ALDRICH-FLUKA |
| 1,2-Dioctanoyl-3-beta-D-galactosyl-sn-glycerol | C11H16O10(C16H32O4) | 56 | 1675,966 | 8457 | Galactosyldiacylglycerol + octanoic acid + 1,2-DIOCTANOYL-3-BETA-D-GALACTOSYL-SN-GLYCEROL ACYLHYDROLASE → 1,2-Dioctanoyl-3-beta-D-galactosyl-sn-glycerol |
| 6-Deoxyerythronolide | C21H38O6 | 57 | 1465,826 | 7656 | Prepared as described by Crimmins, and Slade, *Org. Lett.* 10, 2191 (2006) |
| 1,2-Dipalmitoyl-beta-D-galactosyl-sn-glycerol | C11H16O10(C32H64O4) | 58 | 1884,397 | 6572 | Galactosyldiacylglycerol + octanoic acid + 1,2-DIOCTANOYL-3-BETA-D-GALACTOSYL-SN-GLYCEROL ACYLHYDROLASE → 1,2-Dioctanoyl-3-beta-D-galactosyl-sn-glycerol |
| 1,2-Dipalmitoyl-3-O-[alpha-D-glucopyranosyl(1->2)-O-alpha-D-glucopyranosyl]-sn-glycerol | C17H26O15(C32H64O4) | 59 | 2046,539 | 7134 | Dipalmitoylglycerol + alpha-D-glucopyranosyl(1->2)-O-alpha-D-glucopyranosyl + 1,2-DIPALMITOYL-alpha-D-glucopyranosyl(1->2)-O-alpha-D-glucopyranosyl-SN-GLYCEROL ACYLHYDROLASE → 1,2-Dipalmitoyl-3-O-(alpha-D-glucopyranosyl(1->2)-O-alpha-D-glucopyranosyl)sn-glycerol |
| 1,2-Dipalmitoyl-3-O-alpha-D-glucopyranosyl-sn-glycerol | C11H16O10(C32H64O4) | 60 | 1884,397 | 6308 | Dipalmitoylglycerol + alpha-D-glucopyranosyl + 1,2-DIPALMITOYL-alpha-D-glucopyranosyl-SN-GLYCEROL ACYLHYDROLASE → 1,2-Dipalmitoyl-3-O-(alpha-D-glucopyranosyl)-sn-glycerolglycerol |
| 1,2-Epoxycyclohexane | C6H10O | 61 | 1177,441 | 6944 | SIGMA-ALDRICH-FLUKA |
| 1,2-Heptadecanediol | C17H36O2 | 62 | 1351,769 | 6168 | 1,2-Heptadecanediol was synthesized from palmitic acid as described by H.K. Mangold, J. Org. Chem. 24: 405 (1959) |
| 1,7-Heptanediol | C7H16O | 63 | 1211,500 | 6470 | SIGMA-ALDRICH-FLUKA |

| | | | | | |
|---|---|---|---|---|---|
| | 2 | | | | |
| Hexacosane-1,2-diol | C26H54 O2 | 64 | 1478,011 | 7309 | Hexacosane-1,2-diol was synthesized from hexacosanoic acid as described by H.K. Mangold, *J. Org. Chem.* 24: 405 (1959) |
| 1,2-Hexadecanediol | C16H34 O2 | 65 | 1337,742 | 7190 | SIGMA-ALDRICH-FLUKA |
| 1,2-Hexanediol | C6H14O 2 | 66 | 1197,473 | 7001 | SIGMA-ALDRICH-FLUKA |
| 1,2-Nonanediol | C9H20O 2 | 67 | 1239,554 | 6959 | Nonane-1,2-diol was synthesized from nonanoic acid as described by H.K. Mangold, J. Org. Chem. 24: 405 (1959) |
| 1,2-Nonadecanediol | C19H40 O2 | 68 | 1379,822 | 8330 | 1,2-Nonadecanediol was synthesized from nonadecanoic acid as described by H.K. Mangold, *J. Org. Chem.* 24: 405 (1959) |
| 3-Isopropylbut-3-enoyl-CoA | C28H46N 7O17P3S | 69 | 1956,988 | 7360 | 2,6-Dimethyl-5-methylene-3-oxo-heptanoyl-CoA + CoA + ACETYLTRANSFERASA Arabidopsis thaliana → 3-Isopropylbut-3-enoyl-CoA |
| 1,2-Octadecanediol | C18H38 O2 | 70 | 1365,796 | 6940 | 1,2-Octadecanediol was synthesized from octadecanoic acid as described by H.K. Mangold, *J. Org. Chem.* 24: 405 (1959) |
| 1,2-Octanediol | C8H18O 2 | 71 | 1225,527 | 6100 | SIGMA-ALDRICH-FLUKA |
| D-()-Solketal propionate | C8H14O 4 | 72 | 1253,493 | 6757 | Solketal + METAGENOMIC URANIA ESTERASE→ D-(-)-Solketal propionate |
| 1,3-Oxazole | C3H3NO | 73 | 1148,360 | 6256 | SIGMA-ALDRICH-FLUKA |
| 1,2-Pentanediol | C5H12O 2 | 74 | 1183,446 | 6621 | SIGMA-ALDRICH-FLUKA |
| 1,2-Dibutyryl-sn-glycerol | C5H6O5( C8H16O 4) | 75 | 1385,609 | 6644 | Glycerol + ethyl butanoate + CalA lipase + EL1 METAGENOMIC LIPASE → 1,2-Dibutyryl-sn-glycerol |
| Propane-1,2-diol 1-phosphate | C3H9O5 P | 76 | 1235,372 | 8900 | Isopropanol + NAD+ + RHODIUM PHOSPHINE COMPLEX + PROPANEDIOL-PHOSPHATE DEHYDROGENASE from Thermanaerobium brockii → Propane-1,2-diol 1-phosphate |
| Propane-1,2-diol | C3H8O2 | 77 | 1155,392 | 8958 | SIGMA-ALDRICH-FLUKA |
| (R)-Propane-1,2-diol | C3H8O2 | 78 | 1155,392 | 7924 | SIGMA-ALDRICH-FLUKA |
| (S)-Propane-1,2-diol | C3H8O2 | 79 | 1155,392 | 8860 | SIGMA-ALDRICH-FLUKA |
| 1,2-bis-O-Sinapoyl-beta-D-glucoside | C28H32 O14 | 80 | 1671,850 | 7721 | HPLC purification from *Pseudomonas putida* KT2440 |
| 1,2-Tetracosanediol | C24H50 | 81 | 1594,321 | 6609 | 1,2-Tetracosanediol was synthesized from tetracosanoic acid as described by |

| | | | | | |
|---|---|---|---|---|---|
| | O2 | | | | H.K. Mangold, *J. Org. Chem.* 24: 405 (1959) |
| 1,2-Thiazole | C3H3NS | 82 | 1164,421 | 7448 | SIGMA-ALDRICH-FLUKA |
| 1,2-Undecanediol | C11H24 O2 | 83 | 1267,607 | 7379 | 1,2-Undecanediol was synthesized from undecanoic acid as described by H.K. Mangold, *J. Org. Chem.* 24: 405 (1959) |
| 2-Chloro-4-hydroxy-6-amino-1,3,5-triazine | C3H3ClN 4O | 84 | 1223,861 | 8085 | Polycondensation with BISPHENOL |
| 1,3,4-Oxadiazole | C2H2N2 O | 85 | 1149,347 | 9050 | Cyclopentane + TRANSITION METAL ALKYLIDENE COMPLEXES → 1,3,4-Oxadiazole |
| 1,3,4-Thiadiazole | C2H2N2 S | 86 | 1165,408 | 8820 | THERMAL CYCLOREVERSION: Benzene + t-C4H9 (reflux) → 1,3,4-Thiadiazole |
| Dithiazine | C3H3NS 2 | 87 | 1196,481 | 6870 | Vinylsulphonamide + methyl isothiocyanate + CO3K2 |
| 1,2,5-Oxadiazine | C3H4N2 O | 88 | 1163,374 | 6579 | Cyanoacetylhydrazine + ω-bromoacetophenone → p-Bromoacetophenone-a-cyanoacetylhydrazone → 1,2,5-Oxadiazine |
| 1,3,5-Oxadithiane | C3H6OS 2 | 89 | 1201,496 | 6453 | Cyanoacetylhydrazine + ω-bromoacetophenone → p-Bromoacetophenone-a-cyanoacetylhydrazone → 1,3,5-Oxadithiane |
| Thiadiazine | C16H7Cl 4FN4S | 90 | 1527,425 | 6253 | Cyanoacetylhydrazine + ω-bromoacetophenone → p-Bromoacetophenone-a-cyanoacetylhydrazone → 1,3,4-oxadiazine |
| (2S)-2-Isopropylmalate | C7H12O 5 | 91 | 1255,466 | 6524 | SIGMA-ALDRICH-FLUKA |
| 3-Phospho-D-glyceroyl phosphate | C3H8O1 0P2 | 92 | 1329,335 | 9094 | Syntesized as described by I.D. Spiers et al., Carbohydr Res. 282, 81 (1996) |
| 1,3,8-Trihydroxynaphthalene | C10H8O 3 | 93 | 1255,468 | 8968 | Scytalone (500 mg) which was isolated from Phialaphora lagerbergii was dissolved in trifluoroacetic acid (20 ml) and refluxed for two hours under nitrogen. The solvent was evaporated and the residue was extracted with ethyl acetate. The organic layer was washed with water, brine and dried over MgSO4. Evaporation of the solvent yielded a pale green–brown solid (348 mg, 71%) which was purified twice by preparative TLC eluting with chloroform–acetone–formic acid (89101). |
| 1,3-Bisphospho-D-glycerate | C3H8O1 0P2 | 94 | 1329,335 | 8406 | 3-phospho-D-glycerate (Paul A. Sims and George H. Reed, 2004) + METAGENOMIC PHOSPHOGLYCERATE KINASE → 1,3-Bisphospho-D-glycerate |
| 1,3-Butanediol | C4H10O 2 | 95 | 1169,419 | 6391 | SIGMA-ALDRICH-FLUKA |

| | | | | | |
|---|---|---|---|---|---|
| 2,4-Dichloroaniline | C6H5Cl2N | 96 | 1225,316 | 9314 | SIGMA-ALDRICH-FLUKA |
| 1,3-Diazole | C3H4N2 | 97 | 1147,375 | 6284 | SIGMA-ALDRICH-FLUKA |
| 1,3-Dibromobenzene | C18H12Br6 | 98 | 1787,014 | 6204 | SIGMA-ALDRICH-FLUKA |
| 1,2-Dihexanoyl-sn-glycerol | C5H6O5(C12H24O4) | 99 | 1441,717 | 7330 | Glycerol + ethyl hexanoate + CalA lipase + EL1 METAGENOMIC LIPASE → 1,2-Dihexanoyl-sn-glycerol |
| Deoxyuridine | C9H12N2O5 | 100 | 1307,501 | 7932 | SIGMA-ALDRICH-FLUKA |
| 1,3-Dichloropentane | C5H10Cl2 | 101 | 1220,337 | 6757 | Pentane + Cl2 → 1,3-Dichloropentane |
| 1,3-Dichlorobenzene | C6H4Cl2 | 102 | 1787,014 | 6318 | SIGMA-ALDRICH-FLUKA |
| 1,3-Didecanoyl-sn-glycerol | C5H6O5(C20H40O4) | 103 | 1569,931 | 8924 | Glycerol + ethyl decanoate + CalA lipase + EL1 METAGENOMIC LIPASE → 1,2-Didecanoyl-sn-glycerol |
| 1,3-Dimethylbenzene | C8H10 | 104 | 1185,464 | 7917 | SIGMA-ALDRICH-FLUKA |
| 1,7-Dimethyluric acid | C7H8N4O3 | 105 | 1275,462 | 7414 | SIGMA-ALDRICH-FLUKA |
| 1,3-Diolein | C39H72O5 | 106 | 1700,294 | 9731 | SIGMA-ALDRICH-FLUKA |
| 1,3,2-Dioxazole | C2H3NO2 | 107 | 1152,349 | 6711 | Cyclopentane + TRANSITION METAL ALKYLIDENE COMPLEXES → 1,3,2-Dioxazole |
| (R)-2-Methylmalate | C5H8O5 | 108 | 1211,412 | 7501 | SIGMA-ALDRICH-FLUKA |
| 1,3,5-Trithiane | C3H6S3 | 109 | 1217,557 | 8750 | SIGMA-ALDRICH-FLUKA |
| 1,2-Dipalmitoyl-3-myristoylglycerol | C49H94O6 | 110 | 1858,578 | 6800 | SIGMA-ALDRICH-FLUKA |
| 1,3-Dioctadecanoyl-sn-glycerol | C5H6O5(C36H72O4) | 111 | 1794,361 | 7071 | 1,3-Trioctadecanoate (SIGMA-ALDRICH-FLUKA) + EL1 METAGENOMIC LIPASE ® 1,3-Dioctadecanoyl-sn-glycerol |
| 1,3-Dithiane | C4H8S2 | 112 | 1199,524 | 8682 | SIGMA-ALDRICH-FLUKA |
| 1,2-Dioleyl-sn-glycerol | C5H6O5(C36H68O4) | 113 | 1790,329 | 8592 | Glycerol + ethyl oleate + CalA lipase + EL1 METAGENOMIC LIPASE → 1,2-Dioleyl-sn-glycerol |

| | | | | | |
|---|---|---|---|---|---|
| 3-Hydroxy-3-methyl-2-oxobutanoate | C5H8O4 | 114 | 1211,412 | 6220 | 2-Acetolactate + METAGENOMIC ACETOLACTATE SYNTHASE → 3-Hydroxy-3-methyl-2-oxobutanoate |
| Pentadecane-1,2-diol | C49H94 O6 | 115 | 1858,578 | 6750 | Pentadecane-1,2-diol was synthesized from pentadecanoic acid as described by H.K. Mangold, *J. Org. Chem.* 24, 405 (1959) |
| Propane-1,3-diol | C3H8O2 | 116 | 1155,392 | 6251 | SIGMA-ALDRICH-FLUKA |
| 2,6-Dihydroxycyclohexane-1-carboxyl-CoA | C28H46N 7O19P3S | 117 | 1988,986 | 9940 | HPLC purification from *Pseudomona putida* KT2440 |
| 1,3-Oxathiole | C3H4OS | 118 | 1167,421 | 6940 | 3-(3-Methylbenzofuran-2-yl)-3-oxopropanenitrile + phenyl isothiocyanate + α-halodiketones → 1,3-oxathiole |
| 1,3-Oxazole | C3H3NO | 119 | 1148,360 | 9120 | SIGMA-ALDRICH-FLUKA |
| 1,3-Pentanediol | C5H12O 2 | 120 | 1183,446 | 7810 | Chem Industries |
| dTTP | C10H17N 2O14P3 | 121 | 1561,467 | 6830 | SIGMA-ALDRICH-FLUKA |
| 6-Hydroxycyclohex-1-ene-1-carboxyl-CoA | C28H44N 7O18P3S | 122 | 1970,971 | 8916 | HPLC purification from *Pseudomona putida* KT2440 |
| 1,4-Butanediol | C4H10O 2 | 123 | 1169,419 | 8541 | SIGMA-ALDRICH-FLUKA |
| 1-Butanoyl-sn-glycerol 3-phosphate | C4H8O7 P(C4H8O 2) | 124 | 1366,480 | 6231 | 1,2-Dioctanoyl-sn-glycerol 3-phosphate + ethyl butanoate + CalA lipase + EL1 METAGENOMIC LIPASE → 1-Butanoyl-sn-glycerol 3-phosphate |
| 1,4-Dichloro-2-butene | C4H6Cl2 | 125 | 1418,194 | 9321 | SIGMA-ALDRICH-FLUKA |
| D-Erythrose 4-phosphate | C4H9O7 P | 126 | 1263,382 | 9120 | SIGMA-ALDRICH-FLUKA |
| 1,4-Dichlorobutane | C4H8Cl2 | 127 | 1924,191 | 7810 | SIGMA-ALDRICH-FLUKA |
| 1,3-Dichloropropane | C3H6Cl2 | 128 | 1418,257 | 6830 | SIGMA-ALDRICH-FLUKA |
| 1,4-Dimethylbenzene | C8H10 | 129 | 1185,464 | 8602 | SIGMA-ALDRICH-FLUKA |
| 1,4-Dioxane | C4H8O2 | 130 | 1167,403 | 7410 | SIGMA-ALDRICH-FLUKA |
| 1,4-Dithiane | C4H8S2 | 131 | 1199,524 | 9731 | SIGMA-ALDRICH-FLUKA |
| Maltose | C12H22 O11 | 132 | 1419,624 | 9590 | SIGMA-ALDRICH-FLUKA |
| Maltotriose | C18H32 O16 | 133 | 1581,767 | 8820 | SIGMA-ALDRICH-FLUKA |
| Maltotetraose | C24H42 | 134 | 1743,909 | 7220 | SIGMA-ALDRICH-FLUKA |

| | | | | | |
|---|---|---|---|---|---|
| | O21 | | | | |
| Maltopentaose | C30H52 O26 | 135 | 1906,052 | 9267 | SIGMA-ALDRICH-FLUKA |
| Maltohexaose | C36H62 O31 | 136 | 2068,194 | 9237 | SIGMA-ALDRICH-FLUKA |
| Maltoheptaose | C42H72 O36 | 137 | 2230,336 | 7550 | SIGMA-ALDRICH-FLUKA |
| 1,5-Anhydro-D-fructose | C6H10O 5 | 138 | 1225,439 | 9030 | Prepared as describe by G. Dekanyb *et al.*, *Carbohydr Res* 342, 1249 (2007) |
| 1,5-Anhydro-D-mannitol | C6H12O 5 | 139 | 1227,455 | 6430 | 1,5-Anhydro-D-fructose + METAGENOMIC 1,5-ANHYDRO-D-FRUCTOSE REDUCTASE →1,5-Anhydro-D-mannitol |
| 1,5-Anhydro-D-glucitol | C6H12O 5 | 140 | 1227,455 | 9130 | Carbosynth (http://www.carbosynth.com) |
| 1,2-Dihydroxycyclohexa-3,5-diene-1-carboxylate | C7H8O4 | 141 | 1235,434 | 9331 | HPLC purification from *Pseudomona putida* KT2440 |
| 1,5-Dichloropentane | C5H10Cl 2 | 142 | 1220,337 | 9202 | SIGMA-ALDRICH-FLUKA |
| 1-Heptadecanoyl-sn-glycero-phosphocholine | C9H20N O7P(C17 H34O2) | 143 | 1634,987 | 8520 | SIGMA-ALDRICH-FLUKA |
| (15S)-15-Hydroxy-5,8,11-cis-13-trans-icosatetraenoate | C20H32 O3 | 144 | 1399,769 | 7215 | HPLC purification from tune fish |
| 1,2-Pentanediol | C5H12O 2 | 145 | 1183,446 | 9910 | SIGMA-ALDRICH-FLUKA |
| 2-Pentadecanol | C15H32 O | 146 | 1307,715 | 6910 | ChemIndustry (http://www.chemindustry.com) |
| 1,1-Dichlorohexane | C9H14Cl 2N2O2 | 147 | 1332,425 | 6410 | 1-Pentene + CHCl3 + Fe(CO)5 → 1,1-Dichlorohexane |
| 1,6-Naphthyridine | C8H6N2 | 148 | 1209,445 | 8061 | ChemIndustry (http://www.chemindustry.com) |
| 5-Methyluracil | C5H6N2 O2 | 149 | 1205,411 | 8300 | ChemIndustry (http://www.chemindustry.com) |
| Methyl viologen | C12H14N 2 | 150 | 1265,553 | 6410 | SIGMA-ALDRICH-FLUKA |
| 1,7-Naphthyridine | C8H6N2 | 151 | 1209,445 | 8310 | ChemIndustry (http://www.chemindustry.com) |
| 1,8-Dichlorooctane | C8H16Cl | 152 | 1262,418 | 8210 | SIGMA-ALDRICH-FLUKA |

| | | | | | |
|---|---|---|---|---|---|
| | 2 | | | | |
| 1,8-Naphthyridine | C8H6N2 | 153 | 1209,445 | 7210 | ChemIndustry (http://www.chemindustry.com) |
| 1-Aminocyclopropane-1-carboxylate | C4H7NO2 | 154 | 1164,402 | 7110 | SIGMA-ALDRICH-FLUKA |
| 1-Acetyl-sn-glycerol 3-phosphate | C4H8O7P(CH2O2) | 155 | 1308,400 | 6340 | 1,2-Dioctanoyl-sn-glycerol 3-phosphate + ethyl acetate + CalA lipase + EL1 METAGENOMIC LIPASE → 1-acetyl-sn-glycerol 3-phosphate |
| 1-Acetyl-5-Hydroxy-1,5-dihydro-pyrrol-2-one | C9H17NO3 | 156 | 1250,537 | 8600 | Prepared as described by M. I.Toşa *et al.*, *Tetrahedron: Asymmetry* 19, 2068 (2008) |
| 1-Butyrylglycerol | C4H7O4C4H8O2 | 157 | 1270,501 | 6830 | Tributyrin + CalA lipase + EL1 METAGENOMIC LIPASE → 1-Butyrylglycerol |
| 1-Octanoyl-sn-glycerol-3-phosphate | C11H25O11P | 158 | 1422,587 | 8020 | SIGMA-ALDRICH-FLUKA |
| 3,5-Dibromo-4-hydroxybenzoate | C7H4Br2O3 | 159 | 1359,213 | 7981 | To MeOH (1 L) stirring at -30 OC was added acetyl chloride (50 mL, 0.70 mol) in a dropwise fashion. After the addition was complete, 3,5-dibromo-4-hydroxybenzoic acid (25 g, 84.5 mmol) was added all at once as a solid and the resulting solution allowed to warm to room temperature. After 18 h, the reaction was concentrated to give 25 g (95 %) of 2 as a white solid which was used without further purification. |
| 4-Bromophenyl acetate | C8H7BrO2 | 160 | 1294,343 | 7180 | SIGMA-ALDRICH-FLUKA |
| 1-Bromobutane | C4H9Br | 161 | 1216,316 | 6320 | SIGMA-ALDRICH-FLUKA |
| 1-Bromodecane | C10H21Br | 162 | 1300,478 | 6040 | SIGMA-ALDRICH-FLUKA |
| 1,4-Dichlorobenzene | C6H4Cl2 | 163 | 1226,301 | 6530 | SIGMA-ALDRICH-FLUKA |
| beta-D-Fructose 6-phosphate | C6H13O9P | 164 | 1337,462 | 6139 | SIGMA-ALDRICH-FLUKA |
| Dicarbomethoxynaphthalene | C14H12O4 | 165 | 1323,543 | 8820 | Prepared as described by R. D. Deanin et al. (1968) |
| 1-Butanoyl-sn-glycerol-3-phosphocholine | C9H20NO7P(C4H8O2) | 166 | 1436,638 | 8320 | SIGMA-ALDRICH-FLUKA |
| 2-Chloro-4-methylphenol | C7H7ClO | 167 | 1221,881 | 8520 | SIGMA-ALDRICH-FLUKA |
| 1-Chlorobutane | C4H9Cl | 168 | 1171,865 | 7120 | SIGMA-ALDRICH-FLUKA |

| | | | | | |
|---|---|---|---|---|---|
| 1-Chlorodecane | C10H21Cl | 169 | 1256,027 | 6220 | SIGMA-ALDRICH-FLUKA |
| 3-Chloropropanoic acid | C3H5ClO2 | 170 | 1187,822 | 6504 | SIGMA-ALDRICH-FLUKA |
| Chlorotoluene | C7H7Cl | 171 | 1205,882 | 7958 | SIGMA-ALDRICH-FLUKA |
| 1-Deoxy-D-altro-heptulose 7-phosphate | C7H15O9P | 172 | 1337,462 | 7930 | HPLC purification from *Pseudomona putida* KT2440 |
| trans-1-Decalone | C10H16O | 173 | 1231,533 | 6854 | SIGMA-ALDRICH-FLUKA |
| 1-Decanol | C10H22O | 174 | 1237,580 | 7770 | SIGMA-ALDRICH-FLUKA |
| 1-Decanoyl-sn-glycerol 3-phosphate | C4H8O7P(C10H20O2) | 175 | 1450,641 | 6679 | 1,2-Dioctanoyl-sn-glycerol 3-phosphate + ethyl decanoate + CalA lipase + EL1 METAGENOMIC LIPASE → 1-Decanoyl-sn-glycerol 3-phosphate |
| 1-Decanoyl-sn-glycero-phosphocholine | C9H20NO7P(C10H20O2) | 176 | 1536,798 | 7448 | SIGMA-ALDRICH-FLUKA |
| 1-Dodecanoyl-sn-glycero-phosphocholine | C9H20NO7P(C12H24O2) | 177 | 1564,852 | 7386 | SIGMA-ALDRICH-FLUKA |
| 1-Dodecanol | C12H26O | 178 | 1265,634 | 7337 | SIGMA-ALDRICH-FLUKA |
| 1,5-Naphthyridine | C8H6N2 | 179 | 1209,445 | 9281 | ChemIndustry (http://www.chemindustry.com) |
| 2,6-Dichlorophenol | C6H4Cl2O | 180 | 1242,300 | 9141 | SIGMA-ALDRICH-FLUKA |
| 3,4-Dichlorophenol | C6H4Cl2O | 181 | 1226,301 | 9106 | SIGMA-ALDRICH-FLUKA |
| 1H-2-Benzopyran-1-one | C9H6O2 | 182 | 1225,442 | 8057 | SIGMA-ALDRICH-FLUKA |
| 1-Hydroxybutane-1,2,4-tricarboxylate | C7H10O7 | 183 | 1269,449 | 10080 | Prepared as described by C. Schmitz *et al.*, *J Org Chem* 61, 1817 (1996) |
| 1-Heptadecanol | C17H36O | 184 | 1335,769 | 7320 | SIGMA-ALDRICH-FLUKA |
| 1-Dodecanoyl-sn-glycerol 3-phosphate | C4H8O7P(C12H24O2) | 185 | 1462,696 | 9357 | 1,2-Dioctanoyl-sn-glycerol 3-phosphate + ethyl dodecanoate + CalA lipase + EL1 METAGENOMIC LIPASE → 1-Dodecanoyl-sn-glycerol 3-phosphate |

| | | | | | |
|---|---|---|---|---|---|
| 2-Heptanol | C7H16O | 186 | 1195,500 | 6346 | SIGMA-ALDRICH-FLUKA |
| 1-Heptanol | C7H16O | 187 | 1195,500 | 9220 | SIGMA-ALDRICH-FLUKA |
| 1-Hexacosanol | C26H54O | 188 | 1451,008 | 9262 | 1-Hexacosanol was synthesized from hexacosane as described by H.K. Mangold, *J Org Chem* 24, 405 (1959) |
| 1-Palmitoylglycerophosphocholine | C24H51NO7P | 189 | 1559,943 | 12224 | SIGMA-ALDRICH-FLUKA |
| beta-D-Fructose 1,6-bisphosphate | C6H14O12P2 | 190 | 1417,442 | 6215 | SIGMA-ALDRICH-FLUKA |
| 1-Hexanoyl-sn-glycero-phosphocholine | C9H20NO7P(C6H12O2) | 191 | 1464,692 | 7551 | SIGMA-ALDRICH-FLUKA |
| 1-Methylnicotinamide | C7H9N2O | 192 | 1216,457 | 7136 | SIGMA-ALDRICH-FLUKA |
| 4-Imidazolone-5-propanoate | C6H8N2O3 | 193 | 1235,437 | 7115 | 3-( I H-imidazol-4-yl)-2-propenoic acid + UROCANASE METAGENOMIC → 4-Imidazolone-5-propanoate (Elizabeth Percy et al., 1998) |
| 1-Methylnaphthalene | C11H10 | 194 | 1221,497 | 9087 | SIGMA-ALDRICH-FLUKA |
| 5-Hydroxyindoleacetaldehyde | C10H9NO2 | 195 | 1254,484 | 6830 | SIGMA-ALDRICH-FLUKA |
| 2,5-Diaminopyrimidine nucleoside triphosphate | C9H18N5O14P3 | 196 | 1590,513 | 6582 | SIGMA-ALDRICH-FLUKA |
| Indan-1-ol | C9H10O | 197 | 1213,474 | 9226 | SIGMA-ALDRICH-FLUKA |
| 1-Indanone | C9H8O | 198 | 1211,458 | 6172 | SIGMA-ALDRICH-FLUKA |
| 1R-Indenol | C9H8O | 199 | 1211,458 | 8443 | Prepared as described by Ming-Chang P et al. (2007) |
| 1-Methoxynaphthalene | C11H10O | 200 | 1237,496 | 6192 | SIGMA-ALDRICH-FLUKA |
| 1-Monododecanoylglycerol | C4H7O4(C10H20O2) | 201 | 1370,661 | 8542 | Glycerol + ethyl laurate + EL1 METAGENOMIC LIPASE → 1-Monododecanoylglycerol |
| 1-Monotetradecanoylglycerol | C4H7O4(C14H28O2) | 202 | 1426,769 | 9761 | Glycerol + ethyl tetradecanoate + EL1 METAGENOMIC LIPASE → 1-Monotetradecanoylglycerol |
| 2-Carboxy-2,3-dihydro-5,6-dihydroxyindole | C9H9NO4 | 203 | 1274,471 | 8241 | HPLC purification from *Streptomyces antibioticus* |
| Methyl pyridine-3-carboxylate | C7H7O2N | 204 | 1237,496 | 6831 | Ethyl 2-methylpyridine-3-carboxylate + EL1 METAGENOMIC LIPASE → Methyl |

| | | | | | |
|---|---|---|---|---|---|
| | | | | | pyridine-3-carboxylate |
| 2-Hydroxymethylnaphthalene | C11H10O | 205 | 1237,496 | 8821 | SIGMA-ALDRICH-FLUKA |
| 1-Hexanol | C6H14O | 206 | 1181,473 | 11200 | SIGMA-ALDRICH-FLUKA |
| 1-Monohexanoylglycerol | C4H7O4(C6H12O2) | 207 | 1298,555 | 10570 | Glycerol + ethyl hexanoate + EL1 METAGENOMIC LIPASE → 1-Monohexanoylglycerol |
| 1-Monooctanoylglycerol | C4H7O4(C8H16O2) | 208 | 1326,608 | 10010 | Glycerol + ethyl octanoate + EL1 METAGENOMIC LIPASE → 1-Monooctanoylglycerol |
| 1-Monodecanoylglycerol | C4H7O4(C10H20O2) | 209 | 1354,662 | 9550 | Glycerol + ethyl decanoate + EL1 METAGENOMIC LIPASE → 1-Monodecanoylglycerol |
| 1-Nitrosonaphthalene | C10H7NO | 210 | 1236,468 | 10413 | 1-Nitronaphthalene + alkali metal nitrite in dilute mineral acid → 1-Nitrosonaphthalene |
| 1-Nitronaphthalene | C10H7NO2 | 211 | 1252,468 | 6020 | SIGMA-ALDRICH-FLUKA |
| 1-Nitronaphthalene-5,6-oxide | C10H7NO3 | 212 | 1268,467 | 9610 | 1-nitronaphtalene+ TRANSITION METAL ALKYLIDENE COMPLEXES → 1-Nitronaphthalene-5,6-oxide |
| 1-Nitronaphthalene-7,8-oxide | C10H7NO3 | 213 | 1268,467 | 6210 | 1-nitronaphtalene+ TRANSITION METAL ALKYLIDENE COMPLEXES → 1-Nitronaphthalene-7,8-oxide |
| 1-Nonadecanol | C19H40O | 214 | 1363,822 | 9610 | SIGMA-ALDRICH-FLUKA |
| 1-Nonanoyl-sn-glycero-3-phosphocholine | C9H20NO7P(C9H18O2) | 215 | 1522,772 | 7030 | SIGMA-ALDRICH-FLUKA |
| 1-Nonanol | C9H20O | 216 | 1223,554 | 7190 | SIGMA-ALDRICH-FLUKA |
| 1-Oleyl-sn-glycerol 3-phosphate | C4H8O7P(C18H36O2) | 217 | 1562,856 | 9041 | SIGMA-ALDRICH-FLUKA |
| 6-Oxo-2-hydroxycyclohexane-1-carboxyl-CoA | C28H44N7O19P3S | 218 | 1986,970 | 9137 | HPLC purification from *Pseudomona putida* KT2440 |
| 2-Octanol | C8H18O | 219 | 1209,527 | 8135 | SIGMA-ALDRICH-FLUKA |
| 1-Octadecanoyl-sn-glycero-phosphocholine | C9H20NO7P(C18 | 220 | 1649,013 | 6158 | SIGMA-ALDRICH-FLUKA |

| | | | | | |
|---|---|---|---|---|---|
| | H36O2) | | | | |
| 1-Octadecanol | C18H38O | 221 | 1349,796 | 6071 | SIGMA-ALDRICH-FLUKA |
| 1-Octanoyl-sn-glycero-3-phosphocholine | C9H20NO7P(C8H16O2) | 222 | 1508,745 | 9072 | SIGMA-ALDRICH-FLUKA |
| 1-Octanol | C8H18O | 223 | 1209,527 | 9482 | SIGMA-ALDRICH-FLUKA |
| 1-Palmitoyl-3-stearoyl-rac-glycerol | C37H72O5 | 224 | 1676,272 | 9352 | SIGMA-ALDRICH-FLUKA |
| 1-Pentanol | C5H12O | 225 | 1167,446 | 9303 | SIGMA-ALDRICH-FLUKA |
| 1-O-Hexadecanoyl-glycerol | C4H7O4(C16H32O2) | 226 | 1454,822 | 8531 | Glycerol + ethyl hexadecane + CalA LIPASE + EL1 METAGENOMIC LIPASE → 1-Monohexadecanoylglycerol |
| 1-Propionyl-sn-glycero-3-phosphocholine | C9H20NO7P(C3H6O2) | 227 | 1438,610 | 9605 | SIGMA-ALDRICH-FLUKA |
| 1-Hexadecanoyl-sn-glycerol 3-phosphate | C4H8O7P(C16H32O2) | 228 | 1534,802 | 7225 | 1,2-Dioctanoyl-sn-glycerol 3-phosphate + ethyl hexadodecanoate + CalA lipase + EL1 METAGENOMIC LIPASE → 1-Hexadecanoyl-sn-glycerol 3-phosphate |
| Phenylacetate | C8H8O2 | 229 | 1215,447 | 6512 | SIGMA-ALDRICH-FLUKA |
| 12-Pyren-1-yldodecanoic acid | C28H32O2 | 230 | 1479,858 | 8537 | FLUKA |
| 2,3,4-Trichlorobiphenyl | C12H7Cl3 | 231 | 1336,843 | 7223 | Sinojie (HK) Limited (China) |
| 1-Pyrroline | C4H7N | 232 | 1148,403 | 7603 | SIGMA-ALDRICH-FLUKA |
| 1-O-Sinapoyl beta-D-glucoside | C17H22O10 | 233 | 1465,652 | 8592 | HPLC purification from *Pseudomona putida* KT2440 |
| 1-Tetracosanol | C24H50O | 234 | 1433,957 | 9142 | SIGMA-ALDRICH-FLUKA |
| 1-Tetradecanoyl-sn-glycero-phosphocholine | C9H20NO7P(C14H28O2) | 235 | 1592,906 | 8732 | SIGMA-ALDRICH-FLUKA |
| 2,2',5-Trichlorobiphenyl | C12H7Cl3 | 236 | 1336,843 | 9471 | SIGMA-ALDRICH-FLUKA |
| 1-Undecanol | C11H24 | 237 | 1251,607 | 9561 | SIGMA-ALDRICH-FLUKA |

| | | | | | |
|---|---|---|---|---|---|
| | O | | | | |
| 2,1-Benzisoxazole | C7H5NO | 238 | 1198,420 | 6310 | Connect Marketing GMBH (www.connect-market.com) |
| 1-Tetradecanol | C14H30 O | 239 | 1293,688 | 7451 | SIGMA-ALDRICH-FLUKA |
| 1-Bromo-2-(2-bromoethyl)benzene | C8H8Br2 | 240 | 1343,256 | 9021 | SIGMA-ALDRICH-FLUKA |
| 1-Chloro-2-(chlorophenylmethyl)benzene | C13H10C l2 | 241 | 1316,425 | 9850 | SIGMA-ALDRICH-FLUKA |
| 2,2-Dichloropropanoic acid | C3H4Cl2 O2 | 242 | 1170,814 | 7123 | SIGMA-ALDRICH-FLUKA |
| 2,3,4-Trichlorophenol | C6H3Cl3 O | 243 | 1260,746 | 7840 | SIGMA-ALDRICH-FLUKA |
| 1-Pyrroline-5-carboxylate | C5H7NO 2 | 244 | 1192,413 | 8765 | Synthesized as described by D.J. Hayzer et al., Anal. Biochem. 96, 94 (1979) |
| 2,3,5,6-Tetrachlorobiphenyl | C12H6Cl 4 | 245 | 1371,288 | 9559 | SIGMA-ALDRICH-FLUKA |
| 2-(2,6-Dibromophenyl)-1H-pyrrole | C10H7Br 2N | 246 | 1380,277 | 9173 | SIGMA-ALDRICH-FLUKA |
| 2,4,5-Trichlorophenol | C16H16C l6N2O2 | 247 | 1544,330 | 7030 | SIGMA-ALDRICH-FLUKA |
| 2,3,6-Trichlorophenol | C6H3Cl3 O | 248 | 1260,746 | 6158 | SIGMA-ALDRICH-FLUKA |
| 2,3-Butanediol | C4H10O 2 | 249 | 1169,419 | 9072 | SIGMA-ALDRICH-FLUKA |
| 1-Phenyl-1,3-butanedion | C10H10 O2 | 250 | 1241,485 | 9352 | SIGMA-ALDRICH-FLUKA |
| 2,4,4'-Trichlorobiphenyl | C12H7Cl 3 | 251 | 1336,843 | 9303 | SIGMA-ALDRICH-FLUKA |
| cis-2,3-Dihydroxy-2,3-dihydro-p-cumate | C10H14 O4 | 252 | 1277,515 | 8135 | HPLC purification from *Pseudomona putida* KT2440 |
| 2,3-Dihydro-2,3-dihydroxybiphenyl | C12H12 O2 | 253 | 1267,523 | 9561 | SIGMA-ALDRICH-FLUKA |
| 3-(4-Chlorophenyl)benzene-1,2-diol | C12H9Cl O2 | 254 | 1299,952 | 7451 | Synthesized as described by M. Sylvestre *et al., Appl Environ Microbiol* 62, 2710 (1996) |
| 1-Chloro-3-(2-chlorophenyl)benzene | C12H8Cl 2 | 255 | 1302,398 | 9021 | Naphtalene-catalysed lithiation of 2-(chlorophenyl)-1,3-dioxolane |

| | | | | | |
|---|---|---|---|---|---|
| 2,3-Dihydro-2,3-dihydroxybenzoate | C7H8O4 | 256 | 1235,434 | 9850 | SIGMA-ALDRICH-FLUKA |
| 1,3-Thiazole | C3H3NS | 257 | 1164,421 | 7603 | SIGMA-ALDRICH-FLUKA |
| 2,3-Dihydroxybenzoate | C7H6O4 | 258 | 1233,418 | 8592 | SIGMA-ALDRICH-FLUKA |
| (2,3-Dihydroxybenzoyl)adenylate | C17H18N5O10P | 259 | 1562,629 | 9142 | Synthesized as described by Callahan *et al.*, *Biiorganic and Medical Chemistry Letters* 16, 3802 (2006) |
| 2,3-Dihydroxybiphenyl | C12H10O2 | 260 | 1265,507 | 9471 | SIGMA-ALDRICH-FLUKA |
| trans-2,3-Dihydroxycinnamate | C9H8O4 | 261 | 1259,456 | 9561 | SIGMA-ALDRICH-FLUKA |
| 2,4,6-Trichlorobiphenyl | C12H7Cl3 | 262 | 1336,843 | 7451 | SIGMA-ALDRICH-FLUKA |
| 2,2'-Dimethoxybiphenyl | C14H14O2 | 263 | 1293,561 | 9021 | SIGMA-ALDRICH-FLUKA |
| 2,3-Dihydroxy-3-methylpentanoate | C6H12O4 | 264 | 1227,455 | 8537 | SIGMA-ALDRICH-FLUKA |
| 2-(Formamido)-N1-(5-phospho-D-ribosyl)acetamidine | C8H16N3O8P | 265 | 1407,536 | 7603 | Prepared as described by Mitsuhiro Kinoshita et al. (1978) |
| 2,3-Dihydroxyphenylpropanoate | C9H10O4 | 266 | 1261,472 | 7232 | SIGMA-ALDRICH-FLUKA |
| 2,3-Dihydrofuran | C4H6O | 267 | 1149,387 | 9512 | SIGMA-ALDRICH-FLUKA |
| (R)-2,3-Dihydroxy-3-methylbutanoate | C5H10O4 | 268 | 1213,428 | 9912 | Synthesized as described by Moen and Anthonsen, *J Mol Cat B* 50, 74 (2008) |
| 1,2-Dimethylnaphthalene | C12H12 | 269 | 1235,524 | 9181 | SIGMA-ALDRICH-FLUKA |
| 2,3-Diketo-L-gulonate | C6H8O7 | 270 | 1269,449 | 7146 | SIGMA-ALDRICH-FLUKA |
| 2,3-Dihydroxy-isovalerate | C5H10O4 | 271 | 1213,428 | 6251 | SIGMA-ALDRICH-FLUKA |
| 2,3-Dihydroxy-p-cumate | C10H12O4 | 272 | 1275,499 | 6310 | SIGMA-ALDRICH-FLUKA |
| cis-2,3-Dihydro-2,3-dihydroxy-4'-chlorobiphenyl | C12H11ClO2 | 273 | 1301,968 | 8821 | Synthesized as described by Moen and Anthonsen, *J Mol Cat B* 50, 74 (2008) |
| 2,4,5-Trichlorobiphenyl | C12H7Cl3 | 274 | 1336,843 | 7811 | SIGMA-ALDRICH-FLUKA |
| 2,4',5-Trichlorobiphenyl | C12H7Cl3 | 275 | 1336,843 | 7881 | SIGMA-ALDRICH-FLUKA |
| 4-Hydroxyphenylpyruvate | C9H8O4 | 276 | 1259,456 | 8821 | SIGMA-ALDRICH-FLUKA |

| | | | | | |
|---|---|---|---|---|---|
| 2,3-Dihydrodipicolinate | C7H7NO4 | 277 | 1248,433 | 6205 | Pyruvate + L-aspartate-semialdehyde + DIHYDRODIPICOLINATE SYNTHASE from Physcomitrella patens → 2,3-dihydrodipicolinate |
| 2',2,4-Trichlorobiphenyl | C12H7Cl3 | 278 | 1336,843 | 9632 | SIGMA-ALDRICH-FLUKA |
| 2,4,6-Trichlorophenol | C6H3Cl3O | 279 | 1260,746 | 9304 | SIGMA-ALDRICH-FLUKA |
| 2,4-Dichlorobenzoate | C7H4Cl2O2 | 280 | 1270,311 | 6527 | SIGMA-ALDRICH-FLUKA |
| 1-Chloro-4-(2,2-dichloro-1-phenylethyl)benzene | C14H11Cl3 | 281 | 1364,897 | 7284 | Privided by Betapharma(Shanghai)Co.,Ltd |
| 2-Bromo-1,3-dichlorobenzene | C6H3BrCl2 | 282 | 1305,197 | 9937 | SIGMA-ALDRICH-FLUKA |
| 2,4-Dichlorophenoxyacetate | C8H6Cl2O3 | 283 | 1300,336 | 6815 | Synthesized as described by F.W. Harris and L.K. Post, *Polymer Letters Edition* 13, 225 (1975) |
| gamma-Glutamyl-gamma-aminobutyraldehyde | C9H16N2O4 | 284 | 1279,534 | 6070 | Isolated as described by V.V. Dasu *et al., Microbiology* 152, 2265 (2006) |
| 2,4-Dichlorophenol | C6H4Cl2O | 285 | 1242,300 | 7564 | SIGMA-ALDRICH-FLUKA |
| 2,4-Dinitroaniline | C6H5N3O4 | 286 | 1262,420 | 7183 | SIGMA-ALDRICH-FLUKA |
| 2,4,5-Trichlorophenol | C6H3Cl3O | 287 | 1276,745 | 7986 | SIGMA-ALDRICH-FLUKA |
| S)-2-[5-Amino-1-(5-phospho-D-ribosyl)imidazole-4-carboxamido]succinate | C13H19N4O12P | 288 | 1531,612 | 8271 | Synthesized as described by I. Horak *et al., Die Angewandte Makromolekulare Chemie* 165, 79 (2003) |
| 2,5-Dichloro-2,5-cyclohexadiene-1,4-diol | C6H6Cl2O2 | 289 | 1260,315 | 6509 | SIGMA-ALDRICH-FLUKA |
| 1,4-Dichloro-2-phenylbenzene | C12H8Cl2 | 290 | 1302,398 | 7212 | Synthesized as described by Shim *et al., Bull Korean Chem Soc* 15, 845 (1994) |
| 2,5-Dichlorohydroquinone | C6H4Cl2O2 | 291 | 1258,300 | 7304 | Synthesized as described by Martin *et al., Eur J Biochem* 261, 533 (1999) |
| 2,5-Dichlorophenol | C6H4Cl2O | 292 | 1242,300 | 7968 | SIGMA-ALDRICH-FLUKA |
| 2,5-Didehydro-D-gluconate | C6H8O7 | 293 | 1269,449 | 7610 | HPLC purification from *Pseudomona putida* KT2440 |
| 2,5-Diamino-6-(5'- | C9H16N5 | 294 | 1430,554 | 8743 | HPLC purification from *Pseudomona putida* KT2440 |

| | | | | | |
|---|---|---|---|---|---|
| phosphoribosylamino)-4-pyrimidineone | O8P | | | | |
| 2,5-Diamino-6-hydroxy-4-(5'-phosphoribosylamino)-pyrimidine | C9H16N O7P | 295 | 1358,527 | 8654 | HPLC purification from *Pseudomona putida* KT2440 |
| 2,5-Didehydro-D-gluconate | C6H8O7 | 296 | 1269,449 | 8619 | Synthesized as described by R.R. Schmidt and W. Frick, *Tetrahedron* 44, 7163 (1988) |
| 2,5-Dihydroxyphenyl acetate | C8H8O4 | 297 | 1247,445 | 8413 | SIGMA-ALDRICH-FLUKA |
| 2-cis,6-trans-Farnesol | C15H26 O | 298 | 1301,667 | 8234 | SIGMA-ALDRICH-FLUKA |
| (2Z,6E)-3,7,11-Trimethyldodeca-2,6,10-trienal | C15H24 O | 299 | 1299,651 | 8219 | SIGMA-ALDRICH-FLUKA |
| LL-2,6-Diaminoheptanedioate | C7H14N2 O4 | 300 | 1253,496 | 6330 | Provided by Alfa Aesar (http://www.alfa.com/alf/laboratory_chemical_suppliers.htm) |
| meso-2,6-Diaminoheptanedioate | C7H14N2 O4 | 301 | 1253,496 | 8432 | Syntesized by direct esterification of LL-2,6-Diaminoheptanedioate with MeOH using metagenom,ic lipase EL1 |
| 2,6-Dimethylnaphthalene | C12H12 | 302 | 1235,524 | 8954 | SIGMA-ALDRICH-FLUKA |
| 2-trans,6-trans-Farnesol | C15H26 O | 303 | 1301,667 | 8074 | SIGMA-ALDRICH-FLUKA |
| 2-trans,6-trans-Farnesal | C15H26 O | 304 | 1299,651 | 6610 | SIGMA-ALDRICH-FLUKA |
| 2-Amino-3-oxobutanoate | C4H7NO 3 | 305 | 1180,402 | 7831 | SIGMA-ALDRICH-FLUKA |
| 2-Amino benzene sulfonate | C6H7NO 3S | 306 | 1236,484 | 9451 | GOKUL EXIMP (http://gokuleximp.tradeindia.com) |
| cis-2,3-Dihydroxy-2,3-dihydroethylbenzene | C8H12O 2 | 307 | 1219,479 | 9442 | SIGMA-ALDRICH-FLUKA |
| Methyl 3-hydroxydecanoate | C11H22 O3 | 308 | 1281,590 | 8711 | SIGMA-ALDRICH-FLUKA |
| 2-Amino-4-hydroxy-6-hydroxymethyl-7,8-dihydropteridine diphosphate | C7H11N5 O8P2 | 309 | 1432,466 | 9870 | HPLC purification from *Pseudomona putida* KT2440 |
| 2-Amino-4-hydroxy-6-hydroxymethyl-7,8-dihydropteridine | C7H9N5 O2 | 310 | 1272,505 | 6660 | HPLC purification from *Pseudomona putida* KT2440 |
| 2-Amino-7,8-dihydro-4-hydroxy-6-(diphosphooxymethyl)pteridine | C7H11N5 O8P2 | 311 | 1432,466 | 8371 | HPLC purification from *Pseudomona putida* KT2440 |

| | | | | | |
|---|---|---|---|---|---|
| 2-Aceto-2-hydroxybutanoate | C6H10O4 | 312 | 1239,466 | 9451 | Provided by CNAMP Chemical (msbamb@hotmail.com) |
| (S)-2-Aceto-2-hydroxybutanoate | C6H10O4 | 313 | 1239,466 | 8442 | Isolated by hydrolysis of methyl-2-Aceto-2-hydroxybutanoate with metagenomic lipase EL1 |
| 2-Aminobenzenesulfonate | C6H7NO3S | 314 | 1236,484 | 8711 | Synthesized as described by Smith *et al., Acta Crystallographica Section E* 60, 836 (2004) |
| 2-Aminobenzoate | C7H7NO2 | 315 | 1200,435 | 8401 | SIGMA-ALDRICH-FLUKA |
| 2-Amino-3-oxobutanoate | C4H5NO3 | 316 | 1178,387 | 7890 | 2-Amino-3-oxobutenoate + desaturase from *Bacillus* sp. |
| 2-Arachidonoylglycerol | C23H38O4 | 317 | 1457,849 | 7511 | Arachidonic acid (SIGMA-ALDRICH-FLUKA) + + EL1 METAGENOMIC LIPASE ® 2-Arachidonoylglycerol |
| 2,3-Dihydroxyethylbenzene | C8H10O2 | 318 | 1217,463 | 8621 | Ethylbenzene (SIGMA-ALDRICH-FLUKA) + P450 BM-3 ® 2,3-Dihydroxyethylbenzene |
| 4-Fluorobenzoate | C7H5FO2 | 319 | 1219,411 | 10210 | SIGMA-ALDRICH-FLUKA |
| 2-Hydroxymuconate | C6H6O5 | 320 | 1237,407 | 9810 | 3-chlorocatechol + 2,3-DIHYDROXYBIPHENYL 1,2-DIOXYGENASE from Sphingomonas sp. strain BN6 → 2-Hydroxymuconate |
| 2,3-Bisphospho-D-glycerate | C3H8O10P2 | 321 | 1359,362 | 8810 | 3-phospho-D-glycerate (Paul A. Sims and George H. Reed, 2004) + METAGENOMIC PHOSPHOGLYCERATE KINASE → 1,3-Bisphospho-D-glycerate |
| Bromobenzene-3,4-dihydrodiol | C6H7BrO2 | 322 | 1270,321 | 8600 | Extracted from leaves as described by J.M. Hur *et al, Bioscience, Biotechnology, and Biochemistry* 67, 945 (2003) |
| 2-Butyne-1,4-diol | C4H6O2 | 323 | 1149,387 | 8417 | Prepared as described by S. S. Kale *et al,Industrial & Engineering Chemistry Product Research and Development* 20, 309 (1981). |
| Bromobenzene | C6H5Br | 324 | 1236,307 | 7215 | SIGMA-ALDRICH-FLUKA |
| 2-Bromobutane | C4H9Br | 325 | 1216,316 | 9155 | SIGMA-ALDRICH-FLUKA |
| 2-Bromoethylbenzene | C8H9Br | 326 | 1264,360 | 9370 | SIGMA-ALDRICH-FLUKA |
| 2-Bromopropanoic acid | C3H5BrO2 | 327 | 1246,299 | 6870 | SIGMA-ALDRICH-FLUKA |
| 1-Bromo-2-ethenylbenzene | C8H7Br | 328 | 1262,344 | 7268 | SIGMA-ALDRICH-FLUKA |
| Butan-2-ol | C4H10O | 329 | 1153,419 | 7275 | SIGMA-ALDRICH-FLUKA |
| (E)-2-Chlorobut-2-ene | C4H7Cl | 330 | 1169,849 | 6801 | SIGMA-ALDRICH-FLUKA |
| 2-Chlorobiphenyl | C12H9Cl | 331 | 1267,953 | 6336 | SIGMA-ALDRICH-FLUKA |

| | | | | | |
|---|---|---|---|---|---|
| 2-Chlorobutane | C4H9Cl | 332 | 1171,865 | 6304 | SIGMA-ALDRICH-FLUKA |
| 2-Chlorodecane | C10H21Cl | 333 | 1256,027 | 8003 | SIGMA-ALDRICH-FLUKA |
| 1-(2-Carboxyphenylamino)-1'-deoxy-D-ribulose 5'-phosphate | C12H16NO9P | 334 | 1428,531 | 9403 | HPLC purification from *Pseudomona putida* KT2440 |
| 2-Dehydro-3-deoxy-D-arabino-heptonate 7-phosphate | C7H13O10P | 335 | 1381,472 | 9992 | HPLC purification from *Pseudomona putida* KT2440 |
| 2-Chloropropanoic acid | C3H5ClO2 | 336 | 1201,848 | 9542 | SIGMA-ALDRICH-FLUKA |
| cis-2-Hydroxypenta-2,4-dienoate | C5H6O3 | 337 | 1207,424 | 9352 | 2,3-Dihydroxytoluene is catalyzed by catechol 2,3-dioxygenase to produce cis,cis-2-hydroxy-6-oxohepta-2,4-dienoate. This compound is hydrolyzed to 2-oxopent-4-enoate, or its tautomeric dienol form cis-2-hydroxypenta-2,4-dienoate |
| 1-Chloro-2-methylbenzene | C7H7Cl | 338 | 1205,882 | 8852 | SIGMA-ALDRICH-FLUKA |
| 3-(p-Chlorophenyl)coumarin | C15H9ClO2 | 339 | 1335,985 | 8522 | SIGMA-ALDRICH-FLUKA |
| 2-Phenylpropanenitrile | C9H9N | 340 | 1210,474 | 6301 | Prepared from 1-bromo-1-phenylethane using Kolbe Nitrile Synthesis (Organikum, 22. Edition (German), Wiley-VCH, Weinheim, 2004) |
| 2-Dehydro-3-deoxy-D-gluconate 6-phosphate | C6H11O9P | 341 | 1351,445 | 9351 | Lipase catalyze transesterification (CalB) of vinyl propionate and phenol |
| 2-Dehydro-3-deoxy-L-arabinonate | C5H8O5 | 342 | 1211,412 | 6541 | HPLC purification from *Pseudomona putida* KT2440 |
| 2-Dehydro-3-deoxy-D-gluconate | C6H10O6 | 343 | 1241,439 | 8331 | HPLC purification from *Pseudomona putida* KT2440 |
| 2-Chlorophenol | C6H5ClO | 344 | 1207,855 | 9721 | SIGMA-ALDRICH-FLUKA |
| 2-Deoxy-D-gluconate | C6H12O6 | 345 | 1243,455 | 7221 | HPLC purification from *Pseudomona putida* KT2440 |
| 2-Dehydro-D-glucose | C6H10O6 | 346 | 1255,466 | 8911 | Isolated as described by Leitner *et al.*, *Biocatl. Biotransf.* 16, 365 (1998) |
| 2-Dehydro-3-deoxy-D-xylonate | C5H8O5 | 347 | 1211,412 | 9111 | Isolated as described by C. Sygmund *et al.*, *J Biotechnol* 133, 334 (2008) |
| 5-Dehydro-2-deoxy-D-gluconate | C6H10O6 | 348 | 1241,439 | 7501 | Isolated as described by C. Sygmund *et al.*, *J Biotechnol* 133, 334 (2008) |
| 2-Dehydro-D-gluconate | C6H10O7 | 349 | 1257,438 | 9910 | Isolated as described by C. Sygmund *et al.*, *J Biotechnol* 133, 334 (2008) |
| 2-Deoxy-D-glucose | C6H12O5 | 350 | 1227,455 | 6880 | SIGMA-ALDRICH-FLUKA |
| 2-Dodecanoylglycerol | C4H7O4( | 351 | 1398,715 | 6670 | Glycerol + ethyl dodecane + EL1 METAGENOMIC LIPASE → 2- |

| | | | | | |
|---|---|---|---|---|---|
| | C12H24 O2) | | | | Monododecanoylglycerol |
| 2-Decanoylglycerol | C4H7O4( C10H20 O2) | 352 | 1354,662 | 6871 | Glycerol + ethyl decane + EL1 METAGENOMIC LIPASE → 2-Monodecanoylglycerol |
| 2-Dehydro-3-deoxy-D-galactonate | C6H10O 6 | 353 | 1241,439 | 7147 | Isolated as described by C. Sygmund *et al., J Biotechnol* 133, 334 (2008) |
| 2-Dehydro-3-deoxy-D-galactonate 6-phosphate | C6H11O 9P | 354 | 1351,445 | 8821 | Isolated as described by C. Sygmund *et al., J Biotechnol* 133, 334 (2008) |
| Decan-2-ol | C10H22 O | 355 | 1237,580 | 7592 | SIGMA-ALDRICH-FLUKA |
| 2-Keto-L-gulonate | C6H10O 7 | 356 | 1257,438 | 7603 | Methyl-2-keto-gulonate (SIGMA-ALDRICH-FLUKA) + EL1 METAGENOMIC LIPASE ® 2-KETO-L-GULONATE |
| 2-Dehydropantoate | C6H10O 4 | 357 | 1225,439 | 7174 | Pantoate + 2-DEHYDROPANTOATE 2-REDUCTASE from *Escherichia coli* = 2-dehydropantoate |
| 2-Dehydropantolactone | C6H8O3 | 358 | 1207,424 | 7311 | Pantolactone (SIGMA-ALDRICH-FLUKA) + PANTOLACTONE DEHYDROGENASE METAGENOMIC ® 2-dehydropantolactone |
| 2H-1,2,4-Benzoxadiazine | C7H6N2 O | 359 | 1213,433 | 7303 | Azo-coupling of diazomethane with benzenediazonium-2-oxide → 2H-1,3,4-benzoxadiazine (P.W. Chow and N. Ishikawa, 1974) |
| Demethylmenaquinone | C50H70 O2 | 360 | 1782,402 | 8792 | Menaquinone (SIGMA-ALDRICH-FLUKA) + METHYL TRANSFERASE METAGENOMIC ® Demethylmenaquinone |
| Dodecan-2-ol | C12H26 O | 361 | 1265,634 | 6310 | SIGMA-ALDRICH-FLUKA |
| 2-Deoxy-D-ribose 5-phosphate | C5H11O 7P | 362 | 1277,409 | 7731 | SIGMA-ALDRICH-FLUKA |
| 2-Deoxy-D-ribose 1-phosphate | C5H11O 7P | 363 | 1277,409 | 9111 | SIGMA-ALDRICH-FLUKA |
| 2-Ethylhexan-1-ol | C8H18O | 364 | 1209,527 | 9351 | SIGMA-ALDRICH-FLUKA |
| 2-Demethylmenaquinone | C15H14 O2(C25H 40) | 365 | 1646,165 | 13466 | Menaquinone (SIGMA-ALDRICH-FLUKA) + METHYL TRANSFERASE METAGENOMIC ® Demethylmenaquinone |
| 2H-1,2-Oxazine | C4H9NO | 366 | 1166,418 | 8989 | SIGMA-ALDRICH-FLUKA |
| 2-Hydroxy-2-hydropyrone-4,6-dicarboxylate | C7H6O6 | 367 | 1249,418 | 9019 | SIGMA-ALDRICH-FLUKA |
| 2H-1,2-Benzoxazine | C12H17N | 368 | 1298,583 | 10950 | SIGMA-ALDRICH-FLUKA |

| | | | | | |
|---|---|---|---|---|---|
| | 3O | | | | |
| 4-Methyl-1,3-oxazinane-2-thione | C5H9NOS | 369 | 1210,489 | 9269 | SIGMA-ALDRICH-FLUKA |
| 3-Methyl-2H-1,4-benzoxazine | C9H9NO | 370 | 1226,473 | 9429 | Synthesized as described in S Atarashi *et al.*, *Journal of Heterocyclic Chemistry* 28, 329 (1991) |
| 2-Hydroxy-3-carboxy-6-oxo-7-methylocta-2,4-dienoate | C10H12O6 | 371 | 1307,498 | 9439 | As described in J. Eaton, *J Bacteriol* 178, 1351 (1996) |
| 2H-1-Benzopyran-2-one | C9H6O2 | 372 | 1225,442 | 9770 | SIGMA-ALDRICH-FLUKA |
| 2H-1-Benzopyran | C9H8O | 373 | 1211,458 | 9279 | SIGMA-ALDRICH-FLUKA |
| 3H-1,2,4-dioxazole | C2H3NO2 | 374 | 1152,349 | 8688 | Cyclopentane + TRANSITION METAL ALKYLIDENE COMPLEXES → 3H-1,2,4-dioxazole |
| (S)-2-Hydroxy-2-methyl-3-oxobutanoate | C5H8O4 | 375 | 1211,412 | 9419 | 2-Acetolactate + METAGENOMIC ACETOLACTATE SYNTHASE → 3-Hydroxy-3-methyl-2-oxobutanoate. This product is them methylated by direct esterification with MeOH with EL1 lipase |
| 2-Phenyl-4-propyl-tetrahydro-1,4-oxazine | C13H19NO | 376 | 1284,597 | 10010 | As described in S.D. Lee *et al.*, *Tetrahedron Letters* 25, 3399 (1984) |
| 2-Hydroxy-3-oxopropanoate | C3H4O4 | 377 | 1197,385 | 8998 | As described in S.D. Lee *et al.*, *Tetrahedron Letters* 25, 3399 (1984) |
| 2-Hydroxy-3-oxosuccinate | C4H4O6 | 378 | 1241,395 | 9438 | HPLC purification from *Pseudomona putida* KT2440 |
| 2-Hydroxy-5-methyl-cis,cis-muconic semialdehyde | C7H8O4 | 379 | 1219,435 | 9519 | As described in A. Fishman *et al.*, *Pseudomonas(Springer US)* 237-286 (2006) |
| 2-Hexadecanoyl-glycerol | C4H7O4(C16H32O2) | 380 | 1454,822 | 11713 | Glycerol + ethyl hexadecanoate + EL1 METAGENOMIC LIPASE → 2-Monohexadecanoylglycerol |
| 2-Hydroxy-6-keto-2,4-heptadienoate | C7H8O4 | 381 | 1235,434 | 9519 | Provided by CNAMP Chemical (msbamb@hotmail.com) |
| 2-Hydroxy-6-oxo-7-methylocta-2,4-dienoate | C9H12O4 | 382 | 1263,488 | 9799 | Provided by CNAMP Chemical (msbamb@hotmail.com) |
| 2-Hydroxy-6-oxo-6-phenylhexa-2,4-dienoate | C12H10O4 | 383 | 1297,505 | 10140 | SIGMA-ALDRICH-FLUKA |
| 2-Hydroxyadipate | C6H10O5 | 384 | 1225,439 | 9579 | Synthesized as described by P. Borgeat *et al.*, *J Biol Chem* 251, 7816 (1976) |
| 2-Hydroxybutane-1,2,4-tricarboxylate | C7H10O7 | 385 | 1299,476 | 10019 | Synthesized by homoacotinase from *Thermus thermophilus* |
| 2-Hydroxycyclohexan-1-one | C6H10O2 | 386 | 1193,441 | 9099 | SIGMA-ALDRICH-FLUKA |

| | | | | | |
|---|---|---|---|---|---|
| Heptadec-2-nol | C17H36O | 387 | 1335,769 | 10522 | SIGMA-ALDRICH-FLUKA |
| 2-Heptadecanoylglycerol | C20H40O4 | 388 | 1198,393 | 9148 | Glycerol + ethyl heptadecanoate + EL1 METAGENOMIC LIPASE → 2-Monoheptadecanoylglycerol |
| Hexacosan-1-ol | C46H96O2 | 389 | 1760,564 | 14770 | SIGMA-ALDRICH-FLUKA |
| L-Argininosuccinate | C10H18N4O6 | 390 | 1353,574 | 10860 | SIGMA-ALDRICH-FLUKA |
| Hexadecan-2-ol | C16H34O | 391 | 1321,742 | 10382 | SIGMA-ALDRICH-FLUKA |
| 2-Hexanoylglycerol | C4H7O4(C6H12O2) | 392 | 1314,554 | 10310 | Glycerol + ethyl hexanoate + EL1 METAGENOMIC LIPASE → 2-Monohexanoylgycerol |
| Hexan-2-ol | C6H14O | 393 | 1181,473 | 8979 | SIGMA-ALDRICH-FLUKA |
| (R)-2-Hydroxyglutarate | C5H8O5 | 394 | 1211,412 | 9439 | SIGMA-ALDRICH-FLUKA |
| 2,4-Dichlorobenzoyl-CoA | C28H38Cl2N7O17P3S | 395 | 2019,831 | 16654 | SIGMA-ALDRICH-FLUKA |
| 2H-Imidazole | C3H4N2 | 396 | 1147,375 | 8638 | SIGMA-ALDRICH-FLUKA |
| 2-Hydroxymalonate | C3H4O5 | 397 | 1213,385 | 9158 | SIGMA-ALDRICH-FLUKA |
| 2-Hydroxymuconate semialdehyde | C6H6O4 | 398 | 1205,408 | 9379 | SIGMA-ALDRICH-FLUKA |
| 2-Hydroxyhexadecanal | C16H32O2 | 399 | 1335,726 | 10522 | SIGMA-ALDRICH-FLUKA |
| 2-Bromo-1-chloropropane | C3H6BrCl | 400 | 1236,734 | 9532 | SIGMA-ALDRICH-FLUKA |
| 1-chloro-2-ethenylbenzene | C8H7Cl | 401 | 1217,893 | 9343 | SIGMA-ALDRICH-FLUKA |
| Pyran-2-one | C5H4O2 | 402 | 1175,383 | 8918 | SIGMA-ALDRICH-FLUKA |
| 2H-Pyran | C5H6O | 403 | 1161,398 | 8778 | SIGMA-ALDRICH-FLUKA |
| p-Tolualdehyde | C8H8O | 404 | 1199,447 | 9159 | SIGMA-ALDRICH-FLUKA |
| 2-Hydroxybiphenyl | C12H10O | 405 | 1249,507 | 9660 | SIGMA-ALDRICH-FLUKA |
| 2'-Hydroxybiphenyl-2-sulfinate | C12H10O3S | 406 | 1313,566 | 10300 | Synthesized as described by J.D. Van Hamme *et al., Appl Environ Microbiol* 70, 1487 (2004) |
| 2-Isothiocyanatoethylbenzene | C9H9NS | 407 | 1242,534 | 9590 | Provided by Dayang Chemicals Co., Ltd. |

| | | | | | |
|---|---|---|---|---|---|
| | | | | | (http://www.chemindustry.com.cn/dayang/products.html) |
| N-Methylimidazolidine-2,4-dione | C4H6N2 O2 | 408 | 1193,400 | 9098 | HPLC purification from *Pseudomona putida* KT2440 |
| 2-Indanone | C9H8O | 409 | 1211,458 | 9279 | SIGMA-ALDRICH-FLUKA |
| 2-Isopropylmaleate | C7H10O 4 | 410 | 1237,450 | 9539 | Direct esterification of Maleic acid and isopropanol with lipase EL1 |
| Benzoyl acetyl-CoA | C30H42N 7O18P3S | 411 | 1992,977 | 17094 | Synthesized as described by K. Rossen *et al., Tetrahedron Letters* 36, 6419 (1995) |
| 2H-Pyrrole | C4H5N | 412 | 1146,388 | 8628 | SIGMA-ALDRICH-FLUKA |
| 2-Dehydro-3-deoxy-D-gluconate | C6H10O 6 | 413 | 1241,439 | 9739 | Isolated as described by C. Sygmund *et al., J Biotechnol* 133, 334 (2008) |
| 2-Keto-D-gluconic acid | C6H10O 7 | 414 | 1288,473 | 9899 | SIGMA-ALDRICH-FLUKA |
| 2-Keto-3-methylbutyric acid | C5H8O3 | 415 | 1210,428 | 9269 | SIGMA-ALDRICH-FLUKA |
| 2-Oxoisovalerate | C5H8O3 | 416 | 1210,428 | 9269 | SIGMA-ALDRICH-FLUKA |
| 4-Methylthio-2-oxobutanoate | C5H8O3 S | 417 | 1227,473 | 9439 | SIGMA-ALDRICH-FLUKA |
| 2-Oxopent-4-enoic acid | C5H6O3 | 418 | 1207,424 | 9098 | Synthesized as described by W.L. Collinsworth *et al., J. Bacteriol.* 113, 922 (1973) |
| 2-Methyl-1,3-cyclopentaion | C6H10 | 419 | 1161,442 | 8779 | Provided by MPBio |
| 2-Methyl-4-amino-5-hydroxymethylpyrimidine diphosphate | C6H11N3 O7P2 | 420 | 1362,414 | 10949 | HPLC purification from Saccharomyces cerevisiae |
| 2-Methyl-1-propanol | C4H10O | 421 | 1153,419 | 8699 | SIGMA-ALDRICH-FLUKA |
| 2-Maleylacetate | C6H6O5 | 422 | 1237,407 | 9539 | Transesterification of maleic acid plus vinyl acetate with EL1 lipase |
| 2-Methyl-1,3-dioxolan | C7H12O 4 | 423 | 1239,466 | 1602 | SIGMA-ALDRICH-FLUKA |
| 2-Methylbenzyl alcohol | C8H10O | 424 | 1201,463 | 9179 | SIGMA-ALDRICH-FLUKA |
| 2-Methylbenzo-1,3-dithiole | C8H8S2 | 425 | 1247,568 | 1683 | SIGMA-ALDRICH-FLUKA |
| 2-Methylbutanoyl-CoA | C26H44N 7O17P3S | 426 | 1930,950 | 16474 | 4-methylbutanoic aicd + CoA + ACETYLTRANSFERASA Arabidopsis thaliana → 4-Methylbutanoyl-CoA |
| S-Glutaryldihydrolipoamide | C13H23N O4S2 | 427 | 1384,747 | 11172 | HPLC purification from Arabidopsis thaliana. |
| cis-2-Methylaconitate | C7H8O6 | 428 | 1267,433 | 9839 | cis-methylcitrate + METHYLCITRATE DEHYDRATASE METAGENOMIC → cis- |

| | | | | | |
|---|---|---|---|---|---|
| | | | | | 2-Methylaconitate |
| 2-Methylcitrate | C7H10O7 | 429 | 1269,449 | 10019 | Oxaloacetate + 2-METHYLCITRATE SYNTHASE METAGENOMIC (Horswill et al., 2001) |
| 2-C-Methyl-D-erythritol 4-phosphate | C5H13O7P | 430 | 1279,425 | 10119 | Synthesized as described by S. Raghavan and T. Sreekanth, *Tetrahedron Letters* 48, 9090 (2007) |
| 2-C-Methyl-D-erythritol 2,4-cyclodiphosphate | C5H12O9P2 | 431 | 1341,390 | 10738 | Synthesized as described by P. Narayanasamy *et al., Tetrahedron Letters* 49, 29 (2008) |
| 1-Methoxy-2-phenylbenzene | C13H12O | 432 | 1263,534 | 9800 | As described by D.A. Widdowson and Y.Z. Zhang., *Tetrahedron*, 42, 2111 (1986) |
| 1-Hydroxymethylnaphthalene | C11H10O | 433 | 1237,496 | 9539 | SIGMA-ALDRICH-FLUKA |
| o-Methylphenol | C7H8O | 434 | 1187,436 | 9179 | SIGMA-ALDRICH-FLUKA |
| 2-Methylpyridine | C6H7N | 435 | 1172,425 | 8889 | SIGMA-ALDRICH-FLUKA |
| [(2S)-Oxiran-2-yl]methyl acetate | C5H8O3 | 436 | 1195,413 | 9119 | Prepared as described by L-L Shen *et al., Bioorg Med Chem* 16, 3321 (2008) |
| 2'-O-Methyllicodione | C16H14O5 | 437 | 1365,581 | 10820 | Licodione + S-adenosyl-L-methionine + METHYLTRANSFERASE METAGENOMIC → 2'-O-Methyllicodione |
| 2-Methylnaphthalene | C11H10 | 438 | 1221,497 | 9379 | SIGMA-ALDRICH-FLUKA |
| 2-Hexaprenyl-3-methyl-6-methoxy-1,4-benzoquinone | C38H56O3 | 439 | 1640,157 | 13566 | HPLC purification from *Saccharomyces cerevisiae* |
| 2-Naphthoic acid | C11H8O2 | 440 | 1251,480 | 9679 | SIGMA-ALDRICH-FLUKA |
| 2-Nitroaniline | C6H6N2O2 | 441 | 1217,422 | 9339 | SIGMA-ALDRICH-FLUKA |
| 2-Nitronaphthalene | C10H7NO2 | 442 | 1252,468 | 9689 | SIGMA-ALDRICH-FLUKA |
| 1-Methyl-2-nitrobenzene | C7H7NO2 | 443 | 1216,435 | 9329 | SIGMA-ALDRICH-FLUKA |
| 2-Methylserine | C4H9NO3 | 444 | 1182,418 | 9149 | SIGMA-ALDRICH-FLUKA |
| 2-Octaprenyl-3-methyl-5-hydroxy-6-methoxy-1,4-benzoquinone | C48H72O4 | 445 | 1792,393 | 15119 | HPLC purification from Saccharomyces cerevisiae |
| 2-Octaprenyl-6-methoxyphenol | C47H72O2 | 446 | 1748,384 | 14679 | HPLC purification from Saccharomyces cerevisiae |
| Nonadecan-2-ol | C19H40O | 447 | 1363,822 | 10803 | Synthesized from hexacosanoic acid as described by Mangold, H. K. 1959. Syntheses of unsaturated aldehydes. J. Org. Chem. 24: 405-407. |

| | | | | | |
|---|---|---|---|---|---|
| 2-Nonadecanoylglycerol | C4H7O4(C19H38O2) | 448 | 1496,903 | 12134 | Glycerol + ethylnonadecanoate + EL1 LIPASE METAGENOMIC → 2-Nonadecanoylglycerol |
| 1-Nonadecanoyl-sn-glycero-phosphocholine | C9H20NO7P(C19H38O2) | 449 | 1663,040 | 13795 | SIGMA-ALDRICH-FLUKA |
| 2-Nonanoylglycerol | C4H7O4(C9H18O2) | 450 | 1356,634 | 10731 | Glycerol + ethylnonanoate + EL1 LIPASE METAGENOMIC → 2-Nonanoylglycerol |
| Nonan-2-ol | C9H20O | 451 | 1223,554 | 9400 | SIGMA-ALDRICH-FLUKA |
| 2-Nonaprenyl-6-hydroxyphenol | C52H80O2 | 452 | 1816,503 | 7688 | HPLC purification from *Saccharomyces cerivisiae* |
| 2-Nonaprenylphenol | C52H80O | 453 | 1800,503 | 7570 | HPLC purification from *Saccharomyces cerivisiae* |
| 2-Oxobutanoate | C4H6O3 | 454 | 1181,386 | 8978 | As described in Synthesis, p. 118, 1975 |
| 2-Octadecanoylglycerol | C4H7O4(C18O36O2) | 455 | 2022,569 | 11993 | Glycerol + ethyloctadecanoate + EL1 LIPASE METAGENOMIC → 2-Octadecanoylglycerol |
| Octadecan-2-ol | C18H38O | 456 | 1349,796 | 10662 | SIGMA-ALDRICH-FLUKA |
| 2-Octanoylglycerol | C4H7O4(C8H16O2) | 457 | 1342,607 | 10111 | Glycerol + ethyloctanoate + EL1 LIPASE METAGENOMIC → 2-Octanoylglycerol |
| 2-Octanol | C8H18O | 458 | 1209,527 | 9260 | SIGMA-ALDRICH-FLUKA |
| 2-Phenylphenol | C12H10O | 459 | 1249,507 | 9329 | SIGMA-ALDRICH-FLUKA |
| 2-Thiocyanatoethylbenzene | C9H9NS | 460 | 1242,534 | 9269 | As described by H.L. Wheeler and G.S. Jamieson *J. Amer. Chem. Soc.*, 24, 743 (1902) |
| 1-Monobutanoyl-glycerol | C4H7O4(C4H8O2) | 461 | 1286,500 | 10029 | Glycerol + ethyl butyrate + + CalA LIPASE + EL1 METAGENOMIC LIPASE → 1-Monobutanoylglycerol |
| 2-Octaprenyl-6-methoxy-1,4-benzoquinol | C47H70O3 | 462 | 1762,368 | 9521 | HPLC purification from Saccharomyces cerevisiae |
| 2-Octaprenyl-3-methyl-5-hydroxy-6-methoxy-1,4-benzoquinol | C47H71NO2 | 463 | 1761,384 | 6412 | HPLC purification from Saccharomyces cerevisiae |
| 2-Octaprenyl-3-methyl-6-methoxy- | C46H70 | 464 | 1734,358 | 7870 | HPLC purification from Saccharomyces cerevisiae |

| | | | | | |
|---|---|---|---|---|---|
| 1,4-benzoquinol | O2 | | | | |
| 2-Octaprenyl-6-methoxyphenol | C47H72 O2 | 465 | 1748,384 | 9770 | Reacting dibenzyl ether with propylcarbonyloxybenzoic and optionally anhydrides thereof in the presence of one or more acids as catalyst. |
| 2-Oxooctadecanoic acid | C18H34 O3 | 466 | 1377,763 | 10942 | Synthesized as described by S. Ahmad *et al.*, *J Am Oil Chem Soc* 63, 1343 (1986) |
| 2-Octaprenylphenol | C46H70 O | 467 | 1718,358 | 14348 | HPLC purification from Saccharomyces cerevisiae |
| 3-Phenylbutan-1-ol | C10H14 O | 468 | 1229,517 | 9460 | SIGMA-ALDRICH-FLUKA |
| 2-Phospho-4-(cytidine 5'-diphospho)-2-C-methyl-D-erythritol | C14H26N 3O17P3 | 469 | 1664,588 | 13970 | HPLC purification from Arabidopsis thaliana |
| Pentan-2-ol | C5H12O | 470 | 1167,446 | 8839 | SIGMA-ALDRICH-FLUKA |
| D-Glycerate 2-phosphate | C3H7O7 P | 471 | 1249,355 | 9818 | Synthesised as described by Lilley *et al.*, *Anal Biochem* 148, 282 (1985) |
| 2-Propylglycerol | C4H7O4( C3H6O2) | 472 | 1272,473 | 9889 | Glycerol + ethyl propionate + EL1 LIPASE METAGENOMIC → 2-propylglycerol |
| 2-Phosphoglycolate | C2H5O6 P | 473 | 1249,355 | 9518 | As described in Kursula and Werenga, *J Biol Chem* 278, 9544 (2003) |
| 2-Pyrone-4,6-dicarboxylate | C7H4O6 | 474 | 1247,403 | 9798 | As described in Maruyama, *J Biochem* 93, 557 (1983) |
| 2-Octaprenyl-6-methoxy-1,4-benzoquinone | C47H70 O3 | 475 | 1762,368 | 14153 | HPLC purification from Saccharomyces cerevisiae |
| Propionaldehyde | C3H6O | 476 | 1137,376 | 8538 | SIGMA-ALDRICH-FLUKA |
| 2-Propen-1-ol | C3H6O | 477 | 1137,376 | 8538 | SIGMA-ALDRICH-FLUKA |
| 4,5-Dihydro-1H-pyrazole | C3H6N2 | 478 | 1149,390 | 8658 | Synthesized as described by Barsoum and Girgis, *Eur J Med Chem* (doi:10.1016/j.ejmech.2008.10.020) (2008) |
| Pyran-2-one | C5H4O2 | 479 | 1175,383 | 8918 | SIGMA-ALDRICH-FLUKA |
| 2,3-Dihydro-1H-pyrrole | C4H7N | 480 | 1148,403 | 8649 | Synthesized as described by Barsoum and Girgis, *Eur J Med Chem* (doi:10.1016/j.ejmech.2008.10.020) (2008) |
| (2S)-Flavanone | C15H12 O2 | 481 | 1303,556 | 10200 | SIGMA-ALDRICH-FLUKA |
| (2S)-Flavan-4-ol | C15H14 O2 | 482 | 1305,572 | 10220 | SIGMA-ALDRICH-FLUKA |
| (1R,6R)-2-Succinyl-6-hydroxy-2,4-cyclohexadiene-1-carboxylate | C11H12 O6 | 483 | 1319,509 | 10360 | Synthesized as described in Asymmetric Synthesis with Chemical and Biological Methods (Eds. D. Enders and K-E. Jaeger) Wiley-VCH Verlag GmbH & Co. KGaA |
| 2-S-isocapryloyl-3R-hydroxymethyl- | C13H22 | 484 | 1321,611 | 8510 | HPLC purification from Saccharomyces cerevisiae |

| | | | | | |
|---|---|---|---|---|---|
| gamma-butyrolactone | O4 | | | | |
| 2-Octaprenyl-6-hydroxyphenol | C46H70 O2 | 485 | 1734,358 | 13873 | HPLC purification from Saccharomyces cerevisiae |
| 2-Tetradecanoylglycerol | C4H7O4( C14H28 O2) | 486 | 1426,769 | 11432 | Glycerol + ethyl tetradecanoate + EL1 LIPASE METAGENOMIC → 2-tetradecanoylglycerol |
| Tetracosan-2-ol | C24H50 O | 487 | 1433,957 | 11051 | SIGMA-ALDRICH-FLUKA |
| Tetradecan-2-ol | C14H30 O | 488 | 1293,688 | 9829 | SIGMA-ALDRICH-FLUKA |
| 2-Methyl-4,5-dihydro-1,3-thiazole | C4H7NS | 489 | 1180,463 | 8969 | Synthesized as described by M Tiecco *et al.*, *Tetrahedron: Asymmetry* 13, 429 (2002) |
| Undecan-2-ol | C11H24 O | 490 | 1251,607 | 9681 | SIGMA-ALDRICH-FLUKA |
| 1-Chloro-2-{1-(4-chlorophenyl)ethenyl]benzene | C14H10C l2 | 491 | 1328,436 | 10449 | A described in Synthesis, p. 66, 1990 |
| L-Dicysteine | C6H12N2 O4S2 | 492 | 1303,589 | 9450 | SIGMA-ALDRICH-FLUKA |
| 3,4,5-Trichlorophenol | C6H3Cl3 O | 493 | 1260,746 | 9932 | SIGMA-ALDRICH-FLUKA |
| 3',4',5-Trihydroxy-3,7-dimethoxyflavone | C17H14 O7 | 494 | 1409,591 | 11260 | Provided by Xiamen Bioshining Biotechnology Co.,ltd (http://www.bioshining.net) |
| 3,4-Dichloroaniline | C6H5Cl2 N | 495 | 1225,316 | 9578 | SIGMA-ALDRICH-FLUKA |
| 1,3-Dichloro-2-phenylbenzene | C18H13C l2 | 496 | 1379,504 | 10960 | 2-Phenylbenzene (SIGMA-ALDRICH-FLUKA) + halogenase (metagenomic) |
| 3,4-Dihydroxymandelate | C8H8O5 | 497 | 1263,445 | 9799 | See Sugumaran, *Biochemistry* 25, 4489 (1986) and Cabanes *et al.*, *Biochem J* 256, 681 (1988) |
| 3,4-Dihydroxyphenylacetaldehyde | C8H8O3 | 498 | 1215,447 | 9479 | Synthesized as described by W. Li, *Bioorganic Chemistry* 26, 45 (1998) |
| 3,4-Dihydroxy-trans-cinnamate | C9H8O4 | 499 | 1259,456 | 9759 | Synthesized as described by W. Li, *Bioorganic Chemistry* 26, 45 (1998) |
| 3,4-Dihydroxybenzoate | C7H6O4 | 500 | 1217,419 | 9499 | HPLC purification from Saccharomyces cerevisiae |
| Cystathionine | C7H14N2 O4S | 501 | 1315,582 | 10180 | SIGMA-ALDRICH-FLUKA |
| 3,4-Dihydroxyphenylacetate | C8H8O4 | 502 | 1247,445 | 9639 | HPLC purification from Aspergillus nidulans |
| 3,4-Dichlorobut-1-ene | C4H6Cl2 | 503 | 1204,294 | 9207 | Synthesized as described by T.N. Rostovshchikova *et al.*, *Russiand Chemical* |

| | | | | | |
|---|---|---|---|---|---|
| | | | | | *Bulletin* 55, 1768 (2006) |
| 3,4-Dihydro-1 (2H) naphthalene | C14H16 O2 | 504 | 1295,577 | 7401 | Synthesized as described by Habbad, *Crystal Structure Communications* C42, 581 (1986) |
| 3-(3,4-Dihydroxyphenyl)lactate | C9H10O 5 | 505 | 1277,472 | 9939 | Synthesized as described by Rao *et al. Archiv der Pharmazie* 321, 179 (2006) |
| 3-(4-Hydroxyphenyl)pyruvate | C9H8O4 | 506 | 1259,456 | 9899 | See Paine et al., *Chemistry - A European Journal* 13, 6073 (2007) |
| 3,5-Dihydroxybenzoic acid | C7H6O4 | 507 | 1217,419 | 10208 | SIGMA-ALDRICH-FLUKA |
| 1,3-Dichloro-5-phenylbenzene | C12H8Cl 2 | 508 | 1302,398 | 9479 | 5-Phenylbenzene (SIGMA-ALDRICH-FLUKA) + halogenase (metagenomic) |
| 2-Fluorobenzoate | C7H5FO 2 | 509 | 1219,411 | 9359 | SIGMA-ALDRICH-FLUKA |
| 3-Amino-1,2,4-triazole | C2H4N4 | 510 | 1147,378 | 8798 | SIGMA-ALDRICH-FLUKA |
| 3-Amino-5-deoxyfructose 6-phosphate | C6H14O 5NP | 511 | 1274,452 | 7550 | HPLC purification from Pseudomonas putida KT2440 |
| 3-Fluorobenzoate | C7H5FO 2 | 512 | 1219,411 | 9169 | SIGMA-ALDRICH-FLUKA |
| Monochlorobenzene | C6H5Cl | 513 | 1191,856 | 9083 | SIGMA-ALDRICH-FLUKA |
| 3-Carboxy-2-hydroxy-4-methylpentanoate | C7H12O 5 | 514 | 1255,466 | 9719 | HPLC purification from Pseudomonas putida KT2440 |
| 3-Carboxy-3-hydroxy-4-methylpentanoate | C7H12O 5 | 515 | 1255,466 | 9719 | HPLC purification from Arabidopsis thaliana |
| 3-Chloro-4-hydroxy-phenylacetate | C9H10O 3 | 516 | 1245,473 | 8858 | HPLC purification from Pseudomonas putida KT2440 |
| 3-Carboxy-4-methyl-2-oxopentanoate | C8H12O 5 | 517 | 1267,477 | 9839 | HPLC purification from Pseudomonas putida KT2440 |
| 4-Chlorobenzoate | C7H5ClO 2 | 518 | 1235,866 | 9523 | SIGMA-ALDRICH-FLUKA |
| 1-Chloro-3-phenylbenzene | C12H9Cl | 519 | 1267,953 | 9844 | Supplied by Roth (Germany) |
| 1-Chloro-3-ethenylbenzene | C8H7Cl | 520 | 1217,893 | 9343 | Supplied by Roth (Germany) |
| 3-Chlorotoluene | C7H7Cl | 521 | 1205,882 | 9223 | Supplied by Capot Fine Chemical Professional (http://www.capotchem.com/) |
| 3-Carbamoyloxymethylcephem | C10H9N3 O6S(CH3 )2 | 522 | 1408,624 | 11251 | Synthesized as described in Patent US4448775 |
| 3-Deoxy-arabino-heptulonate 7-phosphate | C7H13O 10P | 523 | 1351,445 | 9279 | Lactic acid plus vinyl acetate with metagenomic EL1 lipase |

| | | | | | |
|---|---|---|---|---|---|
| 3-Dehydrocarnitine | C7H14NO3 | 524 | 1239,490 | 9549 | SIGMA-ALDRICH-FLUKA |
| 3-Dehydro-L-gulonate 6-phosphate | C6H11O10P | 525 | 1367,445 | 10729 | HPLC purification from Pseudomonas putida KT2440 |
| 3-Dehydroquinate | C7H10O6 | 526 | 1283,476 | 9869 | See Bartlett *et al., J Org Chem* 59, 2082 (1994) |
| 3-Dehydro-L-gulonate | C6H10O7 | 527 | 1287,465 | 9899 | HPLC purification from Pseudomonas putida KT2440 |
| 1,4,2-Oxathiazole | C2H3NOS | 528 | 1168,409 | 8919 | Cyclopentane + TRANSITION METAL ALKYLIDENE COMPLEXES → 1,4,2-Oxathiazole |
| 2,3-Dichloropentane | C5H10Cl2 | 529 | 1220,337 | 9297 | SIGMA-ALDRICH-FLUKA |
| 3-Dehydro-L-threonate | C4H6O5 | 530 | 1197,385 | 9298 | L-threonate + NAD+ + metagenomic L-threonate 3-dehydrogenase= 3-dehydro-L-threonate + |
| 3-Demethylubiquinone-9 | C53H80O4 | 531 | 1860,512 | 15044 | HPLC purification from Streptomyces sp. |
| 3-Ethylcatechol | C8H10O2 | 532 | 1217,463 | 9390 | SIGMA-ALDRICH-FLUKA |
| 3H-Dioxazole | C2H3NO2 | 533 | 1152,349 | 8688 | Cyclopentane + TRANSITION METAL ALKYLIDENE COMPLEXES → 3H-Dioxazole |
| 3H-1,2-Dithiole | C5H8 | 534 | 1147,415 | 681 | Synthesized by the method of Meinetsberger et al. (Meinetsberger,E., Schoffer,A. and Behringer,H. (1977) Eine einfache und ergiebige Herstellung von 3-Thioxo-1,2-dithiol (1,2-Trithion). Synthesis, 802–803) |
| 3-Dehydroshikimate | C7H8O5 | 535 | 1235,434 | 9679 | Synthesized as described by Li *et al., J Am Chem Soc* 127, 2874 (2005) |
| 2H-1,3-Dithiole | C5H8 | 536 | 1147,415 | 6810 | Synthesized by the method of Meinetsberger et al. (Meinetsberger,E., Schoffer,A. and Behringer,H. (1977) Eine einfache und ergiebige Herstellung von 3-Thioxo-1,2-dithiol (1,2-Trithion). Synthesis, 802–803) |
| 3-Hydroxy-2-benzopyrone | C9H6O3 | 537 | 1241,441 | 9579 | Provided by Apin Chemicals Limited (UK) |
| (2S,3S)-3-Hydroxy-2-methylbutanoyl-CoA | C26H44N7O18P3S | 538 | 1946,949 | 8989 | Provided by Alfa Chem (NY, USA) |
| 3-Hydroxy-2-methylpropanoate | C4H8O3 | 539 | 1167,403 | 8998 | SIGMA-ALDRICH-FLUKA |
| 3-Hydroxyanthranilate | C7H7NO3 | 540 | 1216,435 | 9489 | Synthesized as described by W.P. Tood and B.K. Carpenter, *Preparative Biochemistry and Biotechnology* 19, 155 (1989) |
| 3-Hydroxy-4-methylanthranilate | C8H9NO3 | 541 | 1246,461 | 9629 | Provided by Alfa Aesar (http://www.alfa.com/alf/laboratory_chemical_suppliers.htm) |

| | | | | | |
|---|---|---|---|---|---|
| 3-Hydroxyoxolan-2-one | C8H12O6 | 542 | 1283,476 | 9999 | Synthesis as described in E. Vedejs, *Journal of the American Chemical Society* 96, 5944 (1974) |
| (2S)-2,4-Diamino-3-hydroxy-4-oxobutanoic acid | C4H8N2O4 | 543 | 1225,442 | 9439 | As in D.L. Boger *et al. J Org Chem* 65, 6770 (2000) |
| (3S)-3-Hydroxydecanoyl-CoA | C24H39N7O18P3S(C10H20O2) | 544 | 2090,155 | 18066 | (3S)-3-hydroxydecanoate + CoA + ACETYLTRANSFERASA Arabidopsis thaliana → (3S)-3-Hydroxydecanoyl-CoA |
| 3-Hydroxybenzaldehyde | C7H6O2 | 545 | 1201,420 | 9179 | As in Kalb, and Gross. 1926. Chem. Ber. 59:733. |
| 2-Hydroxybenzyl alcohol | C7H8O2 | 546 | 1203,436 | 9199 | As in Saniger et al. (2004) Tetrahedron 60, 11453-11464 |
| 3-Hydroxybenzyl amine | C13H7NO3 | 547 | 1304,500 | 10089 | As in Zidar and Kikelj (2008) Tetrahedron 64, 5756-5761 |
| 3-Hydroxybenzoate | C7H6O3 | 548 | 1217,419 | 9339 | As in Synthetic Communications, 16, p. 331, 1986 |
| 3-Hydroxycinnamate | C9H8O3 | 549 | 1243,457 | 9599 | Provided by Advanced Synthesis |
| (3S)-3-Hydroxyhexacosyl-CoA | C24H39N7O18P3S(C6H12O2) | 550 | 2034,048 | 17505 | (3S)-3-Hydroxyhexacoate + CoA + ACETYLTRANSFERASA Arabidopsis thaliana → (3S)-3-Hydroxyhexacosyl-CoA |
| 3-Hydroxybutan-2-one | C4H8O2 | 551 | 1167,403 | 8839 | As in J. Chem. Soc., Perkin Trans. 1, 1983, 2435 - 2440. |
| (3S)-3-Hydroxydodecanoyl-CoA | C24H39N7O18P3S(C12O24O2) | 552 | 2478,003 | 18347 | (3S)-3-Hydroxydodecanoate + CoA + ACETYLTRANSFERASA Arabidopsis thaliana → (3S)-3-Hydroxydodecanoyl-CoA |
| (3S)-3-Hydroxyhexadecanoyl-CoA | C24H39N7O18P3S(C16H32O2) | 553 | 2174,317 | 18908 | (3S)-3-Hydroxyhexadecanoate + CoA + ACETYLTRANSFERASA Arabidopsis thaliana → (3S)-3-Hydroxyhexadecanoyl-CoA |
| 3H-Indole | C8H7N | 554 | 1196,447 | 9129 | SIGMA-ALDRICH-FLUKA |
| 3-Hydroxymethyl ceph-3-em-4-carboxylate | C9H8N2O5S(CH3)2 | 555 | 1379,626 | 10500 | As in Harbridge et al. (1995) Bioorganic & Medicinal Chemistry Letters 5, 657-662. |
| (3R)-3-Hydroxytetradecanoic acid | C14H28O3 | 556 | 1323,671 | 10401 | Synthesized as described by Liu *et al.*, *Bulletin of the Chemical Society of Japan* 70, 1441 (1997) |
| 3-Hydroxy-cis,cis-muconate | C6H6O5 | 557 | 1237,407 | 9539 | HPLC purification from *Sphingomonas* sp. |
| (3S)-3-Hydroxyoctadecanoyl-CoA | C24H39N | 558 | 2202,370 | 19188 | (3S)-3-Hydroxyoctadecanoate + CoA + ACETYLTRANSFERASA Arabidopsis |

| | | | | | |
|---|---|---|---|---|---|
| | 7O18P3S (C18H36 O2) | | | | thaliana → (3S)-3-Hydroxyoctadecanoyl-CoA |
| (3R)-3-Hydroxypalmitoyl-CoA | C24H39N 7O18P3S (C16H32 O2) | 559 | 2174,317 | 18908 | (3R)-3-Hydroxypalmitate + CoA + ACETYLTRANSFERASA Arabidopsis thaliana → (3R)-3-Hydroxypalmitoyl-CoA |
| 3-Hydroxyhexadecanoic acid | C16H32 O3 | 560 | 1351,725 | 10682 | Synthesized as described by Liu *et al.*, *Bulletin of the Chemical Society of Japan* 70, 1441 (1997) |
| 3-Hexaprenyl-4,5-dihydroxybenzoate | C37H54 O4 | 561 | 1642,130 | 13586 | HPLC purification from *Saccharomyces cerivisiae* |
| 3-Hydroxypimeloyl-CoA | C28H46N 7O20P3S | 562 | 2004,986 | 17214 | 3-Hydroxypimelate + CoA + ACETYLTRANSFERASA Arabidopsis thaliana → 3-Hydroxypimeloyl-CoA |
| 3-Hexaprenyl-4-hydroxy-5-methoxybenzoate | C38H56 O4 | 563 | 1656,156 | 13726 | HPLC purification from *Saccharomyces cerivisiae* |
| 3-Hydroxypropanal | C3H6O2 | 564 | 1153,376 | 8698 | As in D.J. Phillips *et. al.*, *Tetrahedron* 63, 10528 (2007) |
| 3-(3-Hydroxy-phenyl)-propanoic acid | C9H10O 3 | 565 | 1245,473 | 9619 | Provided by R&D Chemicals |
| 3H-Pyrrole | C4H5N | 566 | 1146,388 | 8628 | SIGMA-ALDRICH-FLUKA |
| (3S)-3-Hydroxytetradecanoyl-CoA | C24H39N 7O18P3S (C14H28 O2) | 567 | 2146,263 | 18627 | (3S)-3-Hydroxytetradecanoate + CoA + ACETYLTRANSFERASA Arabidopsis thaliana → (3S)-3-Hydroxytetradecanoyl-CoA |
| 3-beta-Hydroxysterol dodecanoate | C18H27 O2(C10H 20O2) | 568 | 1526,975 | 12995 | Synthesized as described by Liu *et al.*, *Bulletin of the Chemical Society of Japan* 70, 1441 (1997) |
| 3-beta-Hydroxysterol hexanoate | C18H27 O2(C6H1 2O2) | 569 | 1470,867 | 11873 | Synthesized as described by Liu *et al.*, *Bulletin of the Chemical Society of Japan* 70, 1441 (1997) |
| 3-beta-Hydroxysterol hexadecanoate | C18H27 O2(C16H 32O2) | 570 | 1611,136 | 13276 | Synthesized as described by Liu *et al.*, *Bulletin of the Chemical Society of Japan* 70, 1441 (1997) |
| 3-Hydroxypropanoate | C3H6O3 | 571 | 1169,375 | 8858 | Synthesized as described by Liu *et al.*, *Bulletin of the Chemical Society of Japan* 70, 1441 (1997) |
| 3-beta-Hydroxysterol oleate | C18H27 | 572 | 1637,174 | 13556 | Synthesized as described by Liu *et al.*, *Bulletin of the Chemical Society of Japan* |

| | | | | | |
|---|---|---|---|---|---|
| | O2(C18H34O2) | | | | 70, 1441 (1997) |
| 3-beta-Hydroxysterol tetradecanoate | C18H27O2(C14H28O2) | 573 | 1583,083 | 12995 | Synthesized as described by Liu *et al.*, *Bulletin of the Chemical Society of Japan* 70, 1441 (1997) |
| 3-beta-Hydroxysterol decanoate | C18H27O2(C10H20O2) | 574 | 1526,975 | 12995 | Synthesized as described by Liu *et al.*, *Bulletin of the Chemical Society of Japan* 70, 1441 (1997) |
| 3-beta-Hydroxysterol octanoate | C18H27O2(C8H16O2) | 575 | 1498,921 | 12154 | Synthesized as described by Liu *et al.*, *Bulletin of the Chemical Society of Japan* 70, 1441 (1997) |
| (S)-3-(Imidazol-5-yl)lactate | C6H8N2O3 | 576 | 1235,437 | 9519 | Provided by J. Haohua Industry Co., Ltd (http://www.jnhaohua.com) |
| 3-(Imidazol-5-yl)pyruvate | C6H6N2O3 | 577 | 1233,421 | 9499 | Provided by J. Haohua Industry Co., Ltd (http://www.jnhaohua.com) |
| 3-Methylbutanol | C5H12O | 578 | 1167,446 | 8839 | SIGMA-ALDRICH-FLUKA |
| 3-Indoleacetonitrile | C10H8N2 | 579 | 1235,483 | 9519 | SIGMA-ALDRICH-FLUKA |
| 3-Methylbutan-2-ol | C5H12O | 580 | 1167,446 | 8839 | SIGMA-ALDRICH-FLUKA |
| 3-Methoxy-4-hydroxy-trans-cinnamate | C10H10O4 | 581 | 1273,483 | 10190 | Ferulic acid + vinyl acetate + metagenomic lipase EL1 |
| Glutaconyl-CoA | C26H39N7O19P3S | 582 | 1957,908 | 16744 | HPLC purification from *Saccharomyces cerivisiae* |
| 3-Methylbenzyl alcohol | C8H10O | 583 | 1201,463 | 9179 | SIGMA-ALDRICH-FLUKA |
| 3-Methyl-1-benzothiophene | C9H8S | 584 | 1227,519 | 9119 | As described by N. Arnau *et. al., Tetrahedron*, 49, 11019 (1993) |
| C1-(3-Indolyl)-glycerol 3-phosphate | C11H14NO6P | 585 | 1366,506 | 10830 | HPLC purification from *Arabidopsis thaliana* |
| 3-Methylbutanoyl-CoA | C26H44N7O17P3S | 586 | 1930,950 | 16474 | 4-Methylbutanoate + CoA + ACETYLTRANSFERASA Arabidopsis thaliana → 4-Methylbutanoyl-CoA |
| 3-Hydroxytoluene | C7H8O | 587 | 1187,436 | 9039 | SIGMA-ALDRICH-FLUKA |
| 3-(Methoxycarbonyl)pent-2-enedioate | C7H8O6 | 588 | 1267,433 | 9839 | As in Odilk and Koomen, *Tetrahedron 41*, 1893 (1985) |
| 3-Methylcatechol | C7H8O2 | 589 | 1203,436 | 9199 | SIGMA-ALDRICH-FLUKA |
| trans-2-(4-Nitrophenyl)-3-phenyloxirane | C14H11NO3 | 590 | 1320,543 | 10370 | As in Narender *et al., Helvetica Chimica Acta* 90, 1107 (2007) |
| 3-Methyl-2-oxobutanoate | C5H8O3 | 591 | 1195,413 | 9119 | As described by Jung *et al., Synthetic Communications*, 29, 3659 (1999) |

| | | | | | |
|---|---|---|---|---|---|
| 3-Methylquinoline | C10H9N | 592 | 1222,485 | 9389 | Provided by Alfa Aesar (http://www.alfa.com/alf/laboratory_chemical_suppliers.htm) |
| (S)-3-Methyl-2-oxopentanoate | C6H10O3 | 593 | 1209,440 | 9259 | As described by Jung *et al.*, *Synthetic Communications*, 29, 3659 (1999) |
| 3-Methoxy-5,7,3',4'-tetrahydroxyflavone | C16H12O7 | 594 | 1395,564 | 8839 | SIGMA-ALDRICH-FLUKA |
| 3-Oxodecanoyl-CoA | C31H52N7O18P3S | 595 | 2015,068 | 17315 | 3-Oxodecanoate (Ackman et al. (1960) Tetrahedron 8, 221-238) + CoA + ACETYLTRANSFERASA Arabidopsis thaliana 3-Oxodecanoyl-CoA |
| 3-Nitrobenzoic acid | C7H5NO4 | 596 | 1246,418 | 9629 | SIGMA-ALDRICH-FLUKA |
| (3-Nitrophenyl)methanol | C7H7NO3 | 597 | 1232,434 | 9489 | As described by G. Gallagher *et al.*, *Journal of Medicinal Chemistry* 28, 1533 (1985) |
| 2,4,6-Trinitrotoluene | C7H5N3O6 | 598 | 1306,430 | 9459 | SIGMA-ALDRICH-FLUKA |
| 3-Oxobutyryl-CoA | C24H37N7O18P3S (C4H8O2) | 599 | 2003,978 | 16862 | 3-Oxobutyrate (Ackman et al. (1960) Tetrahedron 8, 221-238) + CoA + ACETYLTRANSFERASA Arabidopsis thaliana 3-Oxobutyryl-CoA |
| 4-Nitroanilide | C6H7N2O2 | 600 | 1244,424 | 9329 | SIGMA-ALDRICH-FLUKA |
| 3-Oxododecanoyl-CoA | C33H56N7O18P3S | 601 | 2043,121 | 17596 | 3-Oxododecanoate (Ackman et al. (1960) Tetrahedron 8, 221-238) + CoA + ACETYLTRANSFERASA Arabidopsis thaliana 3-Oxododecanoyl-CoA |
| 3-Oxohexanoyl-CoA | C27H44N7O18P3S | 602 | 1958,960 | 16754 | 3-Oxohexanoate (Ackman et al. (1960) Tetrahedron 8, 221-238) + CoA + ACETYLTRANSFERASA Arabidopsis thaliana 3-Oxohexanoyl-CoA |
| 3-Oxohexadecanoyl-CoA | C37H64N7O18P3S | 603 | 2099,229 | 18157 | 3-Oxohexadecanoate (Ackman et al. (1960) Tetrahedron 8, 221-238) + CoA + ACETYLTRANSFERASA Arabidopsis thaliana 3-Oxohexadecanoyl-CoA |
| 3-Oxooctadecanoyl-CoA | C39H68N7O18P3S | 604 | 2127,283 | 18437 | 3-Oxooctadecanoate (Ackman et al. (1960) Tetrahedron 8, 221-238) + CoA + ACETYLTRANSFERASA Arabidopsis thaliana 3-Oxooctadecanoyl-CoA |
| 3-Oxohexacosyl-CoA | C24H37N7O18P3S (C6H12O2) | 605 | 2032,032 | 17485 | 3-Oxohexacosanoate (Ackman et al. (1960) Tetrahedron 8, 221-238) + CoA + ACETYLTRANSFERASA Arabidopsis thaliana 3-Oxohexacosyl-CoA |
| 3-Oxooctanoyl-CoA | C29H48N7O18P3S | 606 | 1987,014 | 17035 | 3-Oxooctanoate (Ackman et al. (1960) Tetrahedron 8, 221-238) + CoA + ACETYLTRANSFERASA Arabidopsis thaliana 3-Oxooctanoyl-CoA |
| 3-Oxopropanoate | C3H4O3 | 607 | 1181,386 | 8838 | 3-Oxobutyrate (Ackman et al. (1960) Tetrahedron 8, 221-238) + Ethanol + |

| | | | | | |
|---|---|---|---|---|---|
| | | | | | metagenomic lipase EL1 |
| 2,3-Didehydro-pimeloyl-CoA | C28H44N7O19P3S | 608 | 1831,815 | 9430 | SIGMA-ALDRICH-FLUKA |
| 3-Octaprenyl-4-hydroxybenzoate | C47H70O3 | 609 | 1762,368 | 14788 | HPLC purification from *Saccharomyces cerivisiae* |
| 3-Oxotetradecanoyl-CoA | C35H60N7O18P3S | 610 | 2071,175 | 17876 | 3-Oxotetradecanoate (Ackman et al. (1960) Tetrahedron 8, 221-238) + CoA + ACETYLTRANSFERASA Arabidopsis thaliana 3-Oxotetradecanoyl-CoA |
| 3-Oxoadipyl-CoA | C27H42N7O20P3S | 611 | 1988,943 | 17054 | 3-Oxoadipate (Ackman et al. (1960) Tetrahedron 8, 221-238) + CoA + ACETYLTRANSFERASA Arabidopsis thaliana 3-Oxoadipyl-CoA |
| 3-Oxoadipate | C6H8O5 | 612 | 1239,423 | 9559 | SIGMA-ALDRICH-FLUKA |
| 3-Oxoadipate enol-lactone | C6H6O4 | 613 | 1221,407 | 9379 | HPLC purification from *Streptomyces coelicor* |
| 3-Phospho-D-glycerate | C3H7O7P | 614 | 1249,355 | 9818 | Synthesized as described by Sims and Reed, *J Mol Cat B* 32, 77 (2005) |
| 3-Phenylpropan-1-ol | C9H12O | 615 | 1215,490 | 11079 | Provided by Oakwood (Oakwood Products, Inc.) |
| 3-Phosphohydroxypyruvate | C3H5O7P | 616 | 1277,366 | 9798 | Provided by ChemPep Inc. |
| 3-Phenyl-propionic acid | C9H10O2 | 617 | 1229,474 | 9023 | Provided by Alfa Aesar (http://www.alfa.com/alf/laboratory_chemical_suppliers.htm) |
| 5-O-(1-Carboxyvinyl)-3-phosphoshikimate | C10H13O10P | 618 | 1387,478 | 9813 | Provided by Alfa Aesar (http://www.alfa.com/alf/laboratory_chemical_suppliers.htm) |
| 3-Phosphonooxypyruvate | C3H5O7P | 619 | 1277,366 | 7963 | HPLC purification from *Arabidopsis thaliana* |
| 2,5-dihydro-1H-pyrrole | C4H7N | 620 | 1148,403 | 8207 | As in Synthetic Communications, 20, p. 227, 1990 |
| (3R)-3-Hydroxy-butanoyl-CoA | C24H39N7O18P3S (C4H8O2) | 621 | 1989,994 | 17294 | (R)-3-Hydroxy-butanoate + CoA + ACETYLTRANSFERASA Arabidopsis thaliana → (R)-3-Hydroxy-butanoyl-CoA |
| 3-Sulfocatechol | C6H6O5S | 622 | 1269,467 | 8283 | As in Junker et al. (1994) Biochem J 300, 429-436. |
| 4,4'-Dichlorobiphenyl | C12H8Cl2 | 623 | 1302,398 | 8560 | SIGMA-ALDRICH-FLUKA |
| 3-Thiaoctanoyl-CoA | C29H51N7O18P3S | 624 | 1990,038 | 9540 | 3-Thiaoctanoate + CoA + ACETYLTRANSFERASA Arabidopsis thaliana → 3-Thiaoctanoyl-CoA |
| (3S)-3-Hydroxy-butanoyl-CoA | C24H39N7O18P3S | 625 | 1989,994 | 14471 | (S)-3-Hydroxy-butanoate + CoA + ACETYLTRANSFERASA Arabidopsis thaliana → (3S)-3-Hydroxy-butanoyl-CoA |

| | | | | | |
|---|---|---|---|---|---|
| | (C4H8O2 ) | | | | |
| 4-Acetamidobutanoate | C6H11N O3 | 626 | 1224,455 | 7905 | Provided by Alfa Aesar (http://www.alfa.com/alf/laboratory_chemical_suppliers.htm) |
| (4S,5S)-4,5-Dihydroxy-2,6-dioxohexanoate | C6H8O6 | 627 | 1239,423 | 8165 | HPLC purification from *Saccharomyces cerivisiae* |
| (4S)-4,6-Dihydroxy-2,5-dioxohexanoate | C6H8O6 | 628 | 1239,423 | 8165 | HPLC purification from *Saccharomyces cerivisiae* |
| Adenine | C5H5N5 | 629 | 1198,426 | 7821 | SIGMA-ALDRICH-FLUKA |
| 4-Aminobutanoate | C4H9NO 2 | 630 | 1166,418 | 7552 | As in Davies et al. (2007) Org Biomol Chem 5, 1961-1969 |
| 4-Aminobutanal | C4H9NO | 631 | 1150,419 | 7418 | As in Kaname et al. (2001) Chemical 6 Pharmaceutical Bulletin 49, 531-536. |
| 4-Aminobenzoate | C7H7NO 2 | 632 | 1216,435 | 7838 | Provided by Alfa Aesar (http://www.alfa.com/alf/laboratory_chemical_suppliers.htm) |
| 4-Amino-4-deoxychorismate | C10H11N O5 | 633 | 1304,498 | 8578 | HPLC purification from *Escherichia coli* |
| 4-Amino-5-hydroxymethyl-2-methylpyrimidine | C6H9N3 O | 634 | 1202,454 | 7855 | HPLC purification from *Saccharomyces cerivisiae* |
| 4-Amino-2-methyl-5-phosphomethylpyrimidine | C6H10N3 O4P | 635 | 1282,434 | 8527 | HPLC purification from *Saccharomyces cerivisiae* |
| 4-Bromobutan-1-ol | C4H9BrO | 636 | 1232,315 | 7971 | SIGMA-ALDRICH-FLUKA |
| 4-Bromobenzoic acid | C7H5BrO 2 | 637 | 1280,317 | 8375 | SIGMA-ALDRICH-FLUKA |
| Bromobenzene-2,3-dihydrodiol | C6H7BrO 2 | 638 | 1270,321 | 8291 | HPLC purification from *Arabidopsis thaliana* |
| 4-Bromocatechol | C6H5BrO 2 | 639 | 1252,306 | 8274 | SIGMA-ALDRICH-FLUKA |
| 4-Bromophenol | C6H5BrO | 640 | 1236,307 | 8139 | SIGMA-ALDRICH-FLUKA |
| 1-Bromo-4-methylbenzene | C7H7Br | 641 | 1250,333 | 8123 | SIGMA-ALDRICH-FLUKA |
| 4-Dimethylaminophenol | C8H11N O | 642 | 1216,478 | 7838 | Provided by GIDEON |
| 4-Carboxy-2-hydroxyhexa-2,4-dienedioate | C7H6O7 | 643 | 1281,417 | 8384 | HPLC purification from *Saccharomyces cerivisiae* |
| 4-Carboxy-2-hydroxy-cis,cis-muconate | C7H6O7 | 644 | 1281,417 | 8384 | HPLC purification from *Pseudomonas putida* KT2440 |

| | | | | | |
|---|---|---|---|---|---|
| 4-Carboxy-2-hydroxymuconate semialdehyde | C7H6O7 | 645 | 1265,417 | 8249 | HPLC purification from *Pseudomonas putida* KT2440 |
| 4-(Cytidine 5'-diphospho)-2-C-methyl-D-erythritol | C14H25N3O14P2 | 646 | 1600,608 | 11066 | HPLC purification from *Saccharomyces cerivisiae* |
| 4-Carboxy-2-oxo-4-pentenoate | C6H6O5 | 647 | 1251,434 | 8014 | HPLC purification from *Saccharomyces cerivisiae* |
| 4-Carboxy-4-hydroxy-2-oxoadipate | C7H8O8 | 648 | 1313,459 | 8535 | HPLC purification from *Saccharomyces cerivisiae* |
| 5,7,4'-Trihydroxy-3'-methoxyflavone | C16H12O6 | 649 | 1379,564 | 9208 | Provided by Hainan Zhongxin Chemical Co., Ltd. |
| 4-Chlorobenzoyl-CoA | C28H39ClN7O17P3S | 650 | 1985,385 | 14001 | 4-Chlorobenzoate + CoA + ACETYLTRANSFERASA Arabidopsis thaliana → 4-Chlorobenzoyl-CoA |
| 1-Chloro-4-ethenylbenzene | C8H7Cl | 651 | 1217,893 | 7850 | Provided by Alfa Aesar (http://www.alfa.com/alf/laboratory_chemical_suppliers.htm) |
| 4-Dehydropantoate | C6H10O4 | 652 | 1225,439 | 7914 | HPLC purification from *Saccharomyces cerivisiae* |
| 4-Chlorotoluene | C7H7Cl | 653 | 1205,882 | 7749 | SIGMA-ALDRICH-FLUKA |
| 5-Amino-6-(ribosylamino)-2,4-(1H,3H)-pyrimidinedione 5'-phosphate | C9H15N4O9P | 654 | 1417,511 | 9662 | HPLC purification from *Saccharomyces cerivisiae* |
| 4-Ethyl phenol | C8H10O | 655 | 1201,463 | 7712 | SIGMA-ALDRICH-FLUKA |
| 1-Fluoro-4-methylbenzene | C7H7F | 656 | 1189,427 | 7611 | As in Journal of Medicinal Chemistry, 21, p. 38, 1978 |
| Diazepine | C5H6N2 | 657 | 1173,412 | 7476 | As in Dieltiens and Claeys (2005) Chemical communications, ISSN 1359-7345, Nº. 35, 2005 , pags. 4477-4478 |
| 4-Hydroxy-2-oxopentanoate | C5H8O4 | 658 | 1195,413 | 7796 | As in Boger DL et al. (2000) J Org Chem 65, 6770-6772 |
| 4-Hydroxy-2-oxoglutarate | C5H6O6 | 659 | 1255,422 | 8048 | As in Boger DL et al. (2000) J Org Chem 65, 6770-6772 |
| 4-Hydroxymandelate | C8H8O4 | 660 | 1247,445 | 8098 | HPLC purification from *Streptomyces coelicor* |
| Biphenyl | C12H10 | 661 | 1233,508 | 7981 | SIGMA-ALDRICH-FLUKA |
| 4-Hydroxy-2-butynal | C4H4O2 | 662 | 1147,372 | 7392 | Synthesized as described by Ichikizaki *et al.*, *Bulletin of the Chemical Society of Japan* 28, 80 (1955) |
| 3-Methyl-1,3-oxazinane | C5H11NO | 663 | 1180,445 | 7535 | As in Tetrahedron Letters, 26, p. 293, 1985 |
| 6-Methyl-3,4-dihydro-2H-1,4-benzoxazine | C9H11NO | 664 | 1228,489 | 7939 | As in Torisu et al. (2004) Biorganic & Medicinal Chemistry 12, 5361-5378 |
| 4-Phenyl-1,3-oxazinane-2-thione | C10H11NOS | 665 | 1272,560 | 8309 | Provided by Bepharm Ltd |

| | | | | | |
|---|---|---|---|---|---|
| 1,3-Benzoxazine-2,4-dione | C8H5NO3 | 666 | 1242,430 | 8056 | SIGMA-ALDRICH-FLUKA |
| 4-Hydroxy-3-methoxybenzoate | C8H8O4 | 667 | 1247,445 | 8098 | Provided by Unibest Biopharma (Shanghai) Co., Ltd. |
| 4-Hydroxy-3-methoxycinnamic acid | C10H10O4 | 668 | 1273,483 | 8317 | Provided by Apin Chemicals Limited (UK) |
| (1S,4S)-4-Hydroxy-3-oxocyclohexane-1-carboxylate | C7H10O4 | 669 | 1237,450 | 8014 | HPLC purification from *Pseudomonas putida* KT2440 |
| 4-Hydroxy-4-methyl-2-oxoglutarate | C6H8O6 | 670 | 1239,423 | 8165 | HPLC purification from *Pseudomonas putida* KT2440 |
| 4-(Hydroxymethyl)phenol | C7H8O2 | 671 | 1203,436 | 7729 | Synthesized as described by Payne *et al., Journal of Chemical Research* 6, 402 (2006) |
| 4-Hydroxybenzoylformate | C8H6O4 | 672 | 1245,429 | 8081 | Synthesized as described by Wiyakrutta and Meevootisom, *J Biotehcnol* 55, 193 (1997) |
| 4-(Aminomethyl)phenol | C7H9NO | 673 | 1202,451 | 7720 | As described by Yasuda *et al., Chemistry Letters* 23, 453 (1994) |
| 4-Hydroxybenzoyl-CoA | C28H40N7O18P3S | 674 | 1966,939 | 14143 | 4-Hydroxy-benzoate + CoA + ACETYLTRANSFERASA Arabidopsis thaliana → 4-Hydroxybenzoyl-CoA |
| 4-Hydroxybenzaldehyde | C7H6O2 | 675 | 1201,420 | 7712 | SIGMA-ALDRICH-FLUKA |
| 3-Methoxy-4-hydroxymandelate | C9H10O5 | 676 | 1277,472 | 8351 | HPLC purification from *Pseudomonas putida* KT2440 |
| 4-Hydroxybutanoate | C4H8O3 | 677 | 1167,403 | 7560 | SIGMA-ALDRICH-FLUKA |
| 4-Hydroxycinnamate | C9H8O3 | 678 | 1243,457 | 8065 | SIGMA-ALDRICH-FLUKA |
| 4-Hydroxy-L-threonine | C4H9NO4 | 679 | 1212,444 | 7821 | HPLC purification from *Saccharomyces cerivisiae* |
| D-4-Hydroxyphenylglycine | C8H9NO3 | 680 | 1246,461 | 8090 | Provided by Apin Chemicals Limited (UK) |
| 3-Hydroxybenzyl alcohol | C7H8O2 | 681 | 1203,436 | 7729 | SIGMA-ALDRICH-FLUKA |
| 3-Hydroxy-2-formylbenzothiophene | C9H6O2S | 682 | 1257,502 | 8183 | As described by Bressler et al., *Appl Environ Microbiol* 67, 821 (2001) |
| L-4-Hydroxyglutamate semialdehyde | C5H9NO4 | 683 | 1210,428 | 7922 | HPLC purification from *Saccharomyces cerivisiae* |
| 4-Methyl-2-oxopentanoate | C6H10O3 | 684 | 1209,440 | 7779 | As described by Pearce *et at., Chem Commun* 2431 (2000) |
| 4-Hydroxybenzoate | C7H6O3 | 685 | 1217,419 | 7846 | SIGMA-ALDRICH-FLUKA |
| 3-Hydroxy-3-methyl-2-oxopentanoate | C6H10O4 | 686 | 1225,439 | 7914 | As described by Pearce *et at., Chem Commun* 2431 (2000) |

| | | | | | |
|---|---|---|---|---|---|
| 4'-Methoxy-5,7-dihydroxyflavone | C16H12O5 | 687 | 1363,565 | 9074 | Provided by Alfa Aesar (http://www.alfa.com/alf/laboratory_chemical_suppliers.htm) |
| p-Methylbenzaldehyde | C8H8O | 688 | 1199,447 | 7695 | SIGMA-ALDRICH-FLUKA |
| 4-Methyl benzyl alcohol | C8H10O | 689 | 1201,463 | 7712 | SIGMA-ALDRICH-FLUKA |
| 3-Methylbutanal | C5H10O | 690 | 1165,430 | 7409 | SIGMA-ALDRICH-FLUKA |
| 4-Methylcatechol | C7H8O2 | 691 | 1203,436 | 7729 | SIGMA-ALDRICH-FLUKA |
| Methyl-cyclohexane | C7H14 | 692 | 1177,485 | 7511 | Synthesized as described by Besson *et al.*, *Chemistry - A European Journal* 6, 949 (2000) |
| 4-Methylcyclohexan-1-ol | C7H14O | 693 | 1193,484 | 7645 | SIGMA-ALDRICH-FLUKA |
| 4-Methylcyclohexan-1-one | C7H12O | 694 | 1191,468 | 7628 | As in Tetrahedron Letters, 25, p. 233, 1984 |
| 4-Methylcycloheptan-1-ol | C8H16O | 695 | 1207,511 | 7763 | As in Tetrahedron Letters, 25, p. 233, 1984 |
| 4-Methylphenol | C7H8O | 696 | 1187,436 | 7594 | SIGMA-ALDRICH-FLUKA |
| 5-(2-Hydroxyethyl)-4-methylthiazole | C6H9NOS | 697 | 1222,500 | 7889 | As by Caira and Nassimbeni, *Acta Crystallographica* B 30, 2332 (1974) |
| 4'-O-Methylisoflavone | C16H12O3 | 698 | 1331,566 | 8805 | HPLC purification from *Saccharomyces cerivisiae* |
| 2-Hydroxy-6-oxo-octa-2,4-dienoate | C8H10O4 | 699 | 1249,461 | 8115 | HPLC purification from *Rhodococcus rhodochrous* NCIMB 13259 |
| 4-Methyl-5-(2-phosphoethyl)-thiazole | C6H10NO4PS | 700 | 1302,480 | 8561 | HPLC purification from *Saccharomyces cerivisiae* |
| 4-Methylumbelliferyl glucuronide | C16H16O9 | 701 | 1431,594 | 9646 | SIGMA-ALDRICH-FLUKA |
| 4-Nitrophenol | C6H5NO3 | 702 | 1218,408 | 7854 | SIGMA-ALDRICH-FLUKA |
| 4-Nitroaniline | C6H6N2O2 | 703 | 1217,422 | 7846 | SIGMA-ALDRICH-FLUKA |
| 4-Nitrotoluene | C7H7NO2 | 704 | 1216,435 | 7838 | SIGMA-ALDRICH-FLUKA |
| 4-Oxalocrotonate | C6H6O5 | 705 | 1237,407 | 8014 | As described by M.C.Fitzgerald *et al. J Am Chem Soc* 117, 11075 (1995) |
| 4-Oxalomesaconate | C7H6O7 | 706 | 1281,417 | 8384 | HPLC purification from *Pseudomonas putida* KT2440 |
| 4-Oxoproline | C5H7NO3 | 707 | 1208,412 | 7770 | SIGMA-ALDRICH-FLUKA |
| 4-Phospho-L-aspartate | C4H8NO7P | 708 | 1276,381 | 8476 | HPLC purification from *Pseudomonas putida* KT2440 |

| | | | | | |
|---|---|---|---|---|---|
| 4-Phospho-D-erythronate | C4H9O8P | 709 | 1309,408 | 8501 | As described by Burgos and Salmon, *Tetrahedron Letters* 45, 3365 (2004) |
| D-4'-Phosphopantothenate | C9H18NO8P | 710 | 1378,515 | 9200 | HPLC purification from *Saccharomyces cerivisiae* |
| (R)-4'-Phosphopantothenoyl-L-cysteine | C12H23N2O9PS | 711 | 1481,652 | 10066 | HPLC purification from *Saccharomyces cerivisiae* |
| Pyran-4-one | C5H4O2 | 712 | 1175,383 | 7493 | As described by Linneaus and Dorman, *J. Org. Chem* 32, 4105 (1967) |
| 5-O-Methyl-myo-inositol | C7H14O6 | 713 | 1257,482 | 8317 | SIGMA-ALDRICH-FLUKA |
| 4-(1-D-Ribitylamino)-5-aminouracil | C9H16N4O6 | 714 | 1339,547 | 9007 | HPLC purification from *Saccharomyces cerivisiae* |
| 4-Chlorobiphenyl | C12H9Cl | 715 | 1267,953 | 8271 | SIGMA-ALDRICH-FLUKA |
| 5,7,4'-Trihydroxyflavone | C15H10O5 | 716 | 1333,538 | 8956 | HPLC purification from *Saccharomyces cerivisiae* |
| 2-Aminodecanoic acid | C10H21NO2 | 717 | 1266,580 | 8259 | SIGMA-ALDRICH-FLUKA |
| 1-(5-Phospho-D-ribosyl)-5-amino-4-imidazolecarboxylate | C9H14N3O9P | 718 | 1432,522 | 9536 | Provided by Advanced Synthesis Technologies (CA, USA) |
| 2,2'-Dihydroxybiphenyl | C12H10O2 | 719 | 1249,507 | 8250 | SIGMA-ALDRICH-FLUKA |
| 5-Amino-4-oxopentanoate | C5H9NO3 | 720 | 1193,421 | 7787 | As in Tetrahedron Letters, 25, p. 2977, 1984 |
| 5-Amino-6-(5'-phosphoribitylamino)uracil | C9H17N4O9P | 721 | 1419,527 | 9679 | HPLC purification from *Saccharomyces cerivisiae* |
| 5-Amino-6-(5'-phosphoribosylamino)uracil | C9H15N4O9P | 722 | 1417,511 | 9662 | HPLC purification from *Saccharomyces cerivisiae* |
| 5-Amino-4-oxooctanoate | C8H12O7N2 | 723 | 1311,489 | 7551 | 5-aminooctanoic acid + LIPOOXYGENASE from rice leaves (Oryza sativa cv. Aichiasahi) → 5-amino-4-oxooctanoate |
| 2-Aminohexadecanoic acid | C16H33NO2 | 724 | 1350,741 | 8966 | SIGMA-ALDRICH-FLUKA |
| Benzoyl acetyl-CoA | C30H42N7O18P3S | 725 | 1992,977 | 12211 | Benzoyl acetate + CoA + ACETYLTRANSFERASA Arabidopsis thaliana → Benzoyl acetyl-CoA |
| 5-Benzamido-2-benzyl-4-oxopentanoate | C12H16ClNO3 | 726 | 1337,014 | 7830 | HPLC purification from *Pseudomonas putida* KT2440 |
| 5-Bromo-4-chloro-3-indoyl | C8H4BrC | 727 | 1403,752 | 7401 | SIGMA-ALDRICH-FLUKA |

| | | | | | |
|---|---|---|---|---|---|
| phosphate | INO4P | | | | |
| 5-carboxyamino-1-(5-phospho-D-ribosyl)imidazole | C9H14N3O9P | 728 | 1402,496 | 9536 | HPLC purification from *Saccharomyces cerivisiae* |
| 5-Dehydro-D-fructose | C6H10O6 | 729 | 1241,439 | 8182 | HPLC purification from *Saccharomyces cerivisiae* |
| 2-Dehydro-D-glucono-1,5-lactone | C6H8O6 | 730 | 1253,450 | 8165 | Provided by Carbosynth |
| 5-Dehydro-D-gluconate | C6H10O7 | 731 | 1287,465 | 8317 | Provided by Hangzhou Capot Chemical Co.,Ltd. |
| 5'-Fluoro-5'-deoxyadenosine | C10H12FN5O3 | 732 | 1417,594 | 8948 | SIGMA-ALDRICH-FLUKA |
| 5-Dehydro-4-deoxy-D-glucarate | C6H8O7 | 733 | 1285,449 | 8300 | HPLC purification from *Saccharomyces cerivisiae* |
| 1-(5'-Phosphoribosyl)-5-formamido-4-imidazolecarboxamide | C10H15N4O9P | 734 | 1429,522 | 8324 | As described by Yamada *et al.*, *Bulletin of the Chemical Society of Japan* 41, 241 (1968) |
| 5H-1,2,5-Oxathiazole | C2H3NOS | 735 | 1168,409 | 7770 | Cyclopentane + TRANSITION METAL ALKYLIDENE COMPLEXES → 5H-1,2,5-Oxathiazole |
| 5-Hydroxyfuranocoumarin | C11H6O4 | 736 | 1281,462 | 7996 | HPLC purification from *Pseudomonas putida* KT2440 |
| S-Methyl-5-thio-5-deoxy-D-ribose 1-phosphate | C6H13O7S2 | 737 | 1324,582 | 7112 | HPLC purification from *Saccharomyces cerivisiae* |
| 2,5-Diamino-6-(5'-triphosphoryl-3',4'-trihydroxy-2'-oxopentyl)-amino-4-oxopyrimidine | C9H18N5O14P3 | 738 | 1590,513 | 8168 | HPLC purification from *Saccharomyces cerivisiae* |
| S-Methyl-5-thio-D-ribose 1-phosphate | C6H13O7PS | 739 | 1323,496 | 8112 | HPLC purification from *Saccharomyces cerivisiae* |
| S-Methyl-5-thio-D-ribulose 1-phosphate | C6H13O7PS | 740 | 1323,496 | 8112 | HPLC purification from *Saccharomyces cerivisiae* |
| 8-Methoxyfuranocoumarin | C12H8O4 | 741 | 1295,489 | 8024 | As by Kutney *et al. Tetrahedron* 28, 5091 (1972) |
| 5-Methoxyfuranocoumarin | C12H8O4 | 742 | 1295,489 | 8124 | As by Kutney *et al. Tetrahedron* 28, 5091 (1972) |
| 5-Methylthioadenosine | C11H15N5O3S | 743 | 1360,630 | 7187 | SIGMA-ALDRICH-FLUKA |
| 5-Methyltetrahydrofolate | C20H25N7O6 | 744 | 1522,759 | 8511 | SIGMA-ALDRICH-FLUKA |
| 6-Hydroxyhexanoate | C6H12O | 745 | 1211,456 | 8856 | SIGMA-ALDRICH-FLUKA |

| | | | | | |
|---|---|---|---|---|---|
| | 3 | | | | |
| 5-Nitro-1,2,4-triazol-3-one | C2H2N4 O3 | 746 | 1237,413 | 6651 | SIGMA-ALDRICH-FLUKA |
| 2(3H)-oxazolone | C3H3NO 2 | 747 | 1164,360 | 8762 | As described in The Chemistry of Heterocyclic Compounds: A Series of Monographs (Eds. Edward C. Taylor, Peter Wipf, Arnold Weissberger) (2004) |
| N1-(5-Phospho-alpha-D-ribosyl)-5,6-dimethylbenzimidazole | C14H19N 2O7P | 748 | 1437,585 | 8309 | HPLC purification from *Saccharomyces cerivisiae* |
| 4-Pyridoxolactone | C8H7NO 3 | 749 | 1244,445 | 9922 | As described by Tamura *et al., Journal of Biosciences and Bioengineering* 106, 460 (2008) |
| 6-Acetyl-beta-D-galactoside | C8H14O 7 | 750 | 1285,492 | 9036 | SIGMA-ALDRICH-FLUKA |
| 1,2-Ditetradecanoyl-sn-glycerol | C5H6O5( C28H56 O4) | 751 | 1682,146 | 9798 | Glycerol + ethyl tetradecanoate + CalA LIPASE + EL1 LIPASE METAGENOMIC → 1,2-Ditetradecanoyl-sn-glycerol |
| 6-Chloro-3-indolyl-beta-D-galactoside | C14H15C lNO6 | 752 | 1408,026 | 7401 | SIGMA-ALDRICH-FLUKA |
| 6-Chloro-3-indolyl-beta-D-glucoside | C14H15C lNO6 | 753 | 1408,026 | 8530 | SIGMA-ALDRICH-FLUKA |
| 6-S-Acetyldihydrolipoamide | C10H19N O2S2 | 754 | 1328,684 | 9091 | HPLC purification from Arabidopsis thaliana. |
| Melibiose | C12H22 O11 | 755 | 1419,624 | 9277 | SIGMA-ALDRICH-FLUKA |
| 1,2-Didodecanoyl-sn-glycerol | C5H6O5( C24H48 O4) | 756 | 1626,039 | 8658 | Glycerol + ethyl dodecanoate + CalA LIPASE + EL1 LIPASE METAGENOMIC → 1,2-Didodecanoyl-sn-glycerol |
| 2,3,6-trimethyl-1,3-oxazinane | C7H15N O | 757 | 1208,499 | 8850 | As in Journal of the American Chemical Society, 91, p. 763, 1969 |
| 5-Methylthio-D-ribose | C6H12O 4S | 758 | 1257,543 | 7952 | As in Baddiley et al. (1951) Nature 167, 359-360 |
| 6-Hydroxymethyl 7,8-dihydropterin | C7H9N5 O2 | 759 | 1343,540 | 6282 | HPLC purification from *Arabidopsis thaliana* |
| 6-Hydroxymethyl 7,8-dihydropterin pyrophosphate | C7H9N5 O9P2 | 760 | 1432,422 | 7551 | HPLC purification from *Arabidopsis thaliana* |
| 6-Oxohexanoate | C6H10O 3 | 761 | 1209,440 | 7852 | Hexanoic acid + LIPOOXYGENASE from rice leaves (Oryza sativa cv. Aichiasahi) → 6-Oxohexanoate |

| | | | | | |
|---|---|---|---|---|---|
| 6-Phospho-2-dehydro-D-gluconate | C6H11O10P | 762 | 1337,418 | 7140 | HPLC purification from *Saccharomyces cerivisiae* |
| 2'-Aminobiphenyl-2,3-diol | C12H11NO2 | 763 | 1264,522 | 7994 | HPLC purification from *Pseudomonas putida* KT2440 |
| 6-Phospho-D-glucono-1,5-lactone | C6H11O9P | 764 | 1335,446 | 8108 | HPLC purification from *Arabidopsis thaliana* |
| 6-Pyruvoyltetrahydropterin | C9H11N5O3 | 765 | 1300,516 | 7066 | HPLC purification from *Arabidopsis thaliana* |
| 5-Hydroxy-L-tryptophan | C11H12N2O3 | 766 | 1299,524 | 7032 | As described by Boroda *et al., Journal of Labelled Compounds & Radiopharmaceuticals* 46, 691 (2003) |
| 1-Deaza-5-azapurine | C5H6N4 | 767 | 1201,426 | 8836 | Modified from US Patent 4617304 |
| 1-Deazapurine | C6H5N3 | 768 | 1198,423 | 8830 | As by Betbeder et al. (1989) Nucleic Acids Res 17, 4217-4222 |
| 6-Phospho-D-gluconate | C6H13O10P | 769 | 1369,461 | 8144 | Synthesized as described by Smith *et al., Acta Crystallographica* B 30, 1760 (1974) |
| 7H-pyrrolo[2,3-d]pyrimidine | C7H6N2 | 770 | 1197,434 | 8321 | Synthesized as described by Choi *et al. Bioorg Med Chem Lett* 16, 2689 (2006) |
| Xanthen-9-one | C13H8O2 | 771 | 1275,502 | 7937 | SIGMA-ALDRICH-FLUKA |
| 8-Hydroxyfuranocoumarin | C11H6O4 | 772 | 1281,462 | 7985 | As by Kutney *et al., Tetrahedron* 28, 5091 (1972) |
| Uridine 5'-triphosphate | C9H15N2O15P3 | 773 | 1563,440 | 10241 | SIGMA-ALDRICH-FLUKA |
| 1,2-Anthracenediol | C14H12O2 | 774 | 1291,545 | 8066 | SIGMA-ALDRICH-FLUKA |
| 9,10-Dihydrophenanthrene | C14H12 | 775 | 1259,546 | 7810 | SIGMA-ALDRICH-FLUKA |
| Acyclic-7-deazapurine | C11H13N5O4 | 776 | 1356,580 | 8602 | As by Betbeder *et al., Nucleic Acids Res* 17, 4217 (1989) |
| 9-Oxononanoic acid | C9H16O3 | 777 | 1251,521 | 7745 | Nonanoic acid + LIPOOXYGENASE from rice leaves (Oryza sativa cv. Aichiasahi) → 9-Oxononanoic acid |
| 8-Amino-7-oxononanoate | C9H17NO3 | 778 | 1266,536 | 7866 | 8-Amino-nonanoate + LIPOOXYGENASE from rice leaves (Oryza sativa cv. Aichiasahi) → 8-Amino-7-oxononanoate |
| Adenosine 5'-phosphosulfate | C10H14N5O10PS | 779 | 1490,580 | 9786 | SIGMA-ALDRICH-FLUKA |
| 3-Acetoacetyl-CoA | C25H40N7O18P3S | 780 | 1930,906 | 13182 | 3-acetoacetate + CoA + ACETYLTRANSFERASA Arabidopsis thaliana → 3-Acetoacetyl-CoA |

| | | | | | |
|---|---|---|---|---|---|
| Tri-L-Alanine | C9H17N3O4 | 781 | 1294,549 | 8218 | SIGMA-ALDRICH-FLUKA |
| Aminoacetaldehyde | C2H5NO | 782 | 1122,366 | 6840 | SIGMA-ALDRICH-FLUKA |
| (S)-5-Oxo-2,5-dihydrofuran-2-acetate | C6H6O4 | 783 | 1221,407 | 7504 | HPLC purification from *Pseudomonas putida* KT2440 |
| 2,2-Azino-bis-3-ethylbenzthiazoline-6-sulfonic acid | C18H24N6O6S6 | 784 | 1692,081 | 7121 | SIGMA-ALDRICH-FLUKA |
| Acetyl-CoA | C23H38N7O17P3S | 785 | 1888,870 | 6421 | SIGMA-ALDRICH-FLUKA |
| N-Acetyl-D-glucosamine | C8H15NO6 | 786 | 1284,508 | 6904 | SIGMA-ALDRICH-FLUKA |
| Acetoacetate | C4H6O3 | 787 | 1181,386 | 9253 | SIGMA-ALDRICH-FLUKA |
| Acetaldehyde | C2H4O | 788 | 1123,350 | 6330 | SIGMA-ALDRICH-FLUKA |
| O-Acetylcarnitine | C9H18NO4 | 789 | 1283,543 | 8001 | SIGMA-ALDRICH-FLUKA |
| Acetate | C2H4O2 | 790 | 1123,350 | 7824 | SIGMA-ALDRICH-FLUKA |
| Acenaphthalen-1-ol | C12H10O | 791 | 1249,507 | 6282 | Provided by Alfa Aesar (http://www.alfa.com/alf/laboratory_chemical_suppliers.htm) |
| N-Acetyl-D-mannosamine 6-phosphate | C8H16NO9P | 792 | 1364,488 | 6583 | As described by Ghost and Roseman, *Proc Natl Acad Sci USA* 47, 955 (1961) |
| Naphthyl-2-methyl-succinyl-CoA | C36H48N7O19P3S | 793 | 2087,090 | 10824 | Naphthyl-2-methyl-succinate (HPLC purification from *Pseudomonas* sp.) + CoA + ACETYLTRANSFERASA Arabidopsis thaliana - Naphthyl-2-methyl-succinyl-CoA |
| 1-Phenylethanone | C8H8O | 794 | 1199,447 | 6497 | As in Journal of the American Chemical Society, 85, p. 3410, 1963 |
| Acetoin | C4H8O2 | 795 | 1167,403 | 6304 | SIGMA-ALDRICH-FLUKA |
| N-Acetyl-L-glutamyl 5-phosphate | C7H12NO8P | 796 | 1348,445 | 6391 | HPLC purification from *Pseudomonas putida* KT2440 |
| N-Acetyl-L-glutamate 5-semialdehyde | C7H11NO4 | 797 | 1252,465 | 6815 | HPLC purification from *Pseudomonas putida* KT2440 |
| Adenosylcobalamin 5'-phosphate | C72H101CoN18O20P2 | 798 | # | 12281 | HPLC purification from Arabidopsis thaliana |
| 2-Hydroxy-3-oxoadipate | C6H8O6 | 799 | 1269,449 | 6832 | Synthesized as described by Borgeat *et al.*, *J Biol Chem* 251, 7816 (1976) |
| N-Acetyl-D-glucosamine 6-phosphate | C8H16NO9P | 800 | 1395,522 | 6583 | Synthesized as described by Maley and Lardy, *J Am Chem Soc* 78, 1393 (1956) |
| N-Acetyl-L-glutamate | C7H11N | 801 | 1282,492 | 6911 | SIGMA-ALDRICH-FLUKA |

| | | | | | |
|---|---|---|---|---|---|
| | O5 | | | | |
| 2-Amino-3-oxoadipate | C6H9NO5 | 802 | 1268,465 | 7827 | Synthesized as described by Borgeat *et al.*, *J Biol Chem* 251, 7816 (1976) |
| Acenaphthalene | C12H8 | 803 | 1231,492 | 7689 | SIGMA-ALDRICH-FLUKA |
| N-Acetylneuraminate | C11H19NO9 | 804 | 1372,571 | 6632 | HPLC purification from Arabidopsis thaliana |
| 3-Carboxy-2-hydroxy-4-methylpentanoate | C7H12O5 | 805 | 1255,466 | 7833 | 4-methylpentanoate + LIPOOXYGENASE from rice leaves (Oryza sativa cv. Aichiasahi) → 3-oxo-4-methylpentanoate |
| cis-Aconitate | C6H6O6 | 806 | 1237,407 | 9820 | SIGMA-ALDRICH-FLUKA |
| N2-Acetyl-L-ornithine | C7H14N2O3 | 807 | 1237,497 | 6821 | SIGMA-ALDRICH-FLUKA |
| N5-Propyl-L-ornithine ester | C6H10N2O3(C3H6O2) | 808 | 1295,533 | 6169 | SIGMA-ALDRICH-FLUKA |
| Acryloyl-CoA | C24H38N7O17P3S | 809 | 1900,881 | 9706 | 2-butenoic acid + CoA + ACETYLTRANSFERASA Arabidopsis thaliana → 2-butenoyl-CoA |
| Acridine | C13H9N | 810 | 1258,518 | 6851 | As by Elderfield, Hetetocyclic Compound et at. (1955) J. Chem. Soc. 1082 |
| Acrolein | C3H4O | 811 | 1135,361 | 6112 | SIGMA-ALDRICH-FLUKA |
| O-Acetyl-L-serine | C5H9NO4 | 812 | 1210,428 | 8658 | SIGMA-ALDRICH-FLUKA |
| Adenosine | C10H13N5O4 | 813 | 1330,542 | 6379 | SIGMA-ALDRICH-FLUKA |
| N-Acetyl-D-glucosamine 1-phosphate | C8H16NO9P | 814 | 1364,488 | 6583 | SIGMA-ALDRICH-FLUKA |
| N-Acetyl-D-mannosamine | C8H15NO6 | 815 | 1284,508 | 6103 | SIGMA-ALDRICH-FLUKA |
| Acetyl phosphate | C2H5O5P | 816 | 1219,330 | 5616 | SIGMA-ALDRICH-FLUKA |
| Agmatine | C5H14N4 | 817 | 1193,490 | 5557 | SIGMA-ALDRICH-FLUKA |
| 6-Aminopurine | C5H5N5 | 818 | 1198,426 | 5586 | SIGMA-ALDRICH-FLUKA |
| 4-Aminobiphenyl | C12H11N | 819 | 1232,523 | 5791 | SIGMA-ALDRICH-FLUKA |
| Adenosyl cobinamide | C58H84CoN16O11 | 820 | 2303,636 | 12219 | HPLC purification from *Saccharomyces cerivisiae* |
| Adenosyl cobinamide phosphate | C58H85C | 821 | 2383,616 | 12699 | HPLC purification from *Saccharomyces cerivisiae* |

| | | | | | |
|---|---|---|---|---|---|
| | oN16O14 P | | | | |
| Adenosylcobalamin | C72H100 CoN18O 17P | 822 | 2642,901 | 13901 | HPLC purification from *Saccharomyces cerivisiae* |
| ADP | C10H15N 5O10P2 | 823 | 1490,502 | 7339 | SIGMA-ALDRICH-FLUKA |
| ADP-L-glycero-D-manno-heptose | C17H27N 5O16P2 | 824 | 1682,671 | 8493 | HPLC purification from *Saccharomyces cerivisiae* |
| ADP-D-glycero-D-manno-heptose | C17H27N 5O16P2 | 825 | 1682,671 | 8493 | HPLC purification from *Saccharomyces cerivisiae* |
| Alginate | (C120H1 60O120) | 826 | 4599,842 | 8637 | SIGMA-ALDRICH-FLUKA |
| 2-amino-4,6-dimethoxypyrimidine | C6H8N3 O2 | 827 | 1233,445 | 8510 | Provided by DSM |
| ADP-ribose | C15H23N 5O14P2 | 828 | 1622,618 | 8060 | SIGMA-ALDRICH-FLUKA |
| Hexanedinitrile | C6H8N2 | 829 | 1187,439 | 5424 | SIGMA-ALDRICH-FLUKA |
| Adenosine-GDP-cobinamide | C68H97C oN21O21 P2 | 830 | 2728,825 | 14771 | HPLC purification from *Saccharomyces cerivisiae* |
| Undecane | C11H24 | 831 | 1235,608 | 5714 | SIGMA-ALDRICH-FLUKA |
| 3,4-Dihydroxymandelaldehyde | C8H8O4 | 832 | 1247,445 | 5785 | HPLC purification from *Saccharomyces cerivisiae* |
| 1-Acetyl-sn-glycero-3-phosphoethanolamine | C6H13N O7P(C2H 4O2) | 833 | 1365,496 | 6589 | HPLC purification from *Saccharomyces cerivisiae* |
| S-Adenosyl-L-homocysteine | C14H20N 6O5S | 834 | 1532,769 | 7082 | SIGMA-ALDRICH-FLUKA |
| 2-Amino-4-hydroxy-6-(erythro-1,2,3-trihydroxypropyl)dihydropteridine triphosphate | C9H16N5 O13P3 | 835 | 1558,471 | 7747 | HPLC purification from *Saccharomyces cerivisiae* |
| 1-Acetyl-sn-glycero-3-phosphocholine | C9H20N O7P(C2H 4O2) | 836 | 1408,584 | 6848 | SIGMA-ALDRICH-FLUKA |
| 1-Acetyl-sn-glycerol 3-phosphate | C4H8O7 | 837 | 1322,427 | 6331 | SIGMA-ALDRICH-FLUKA |

| | | | | | |
|---|---|---|---|---|---|
| | P(C2H4O 2) | | | | |
| Acetylhistone | C7H16N O2 | 838 | # | 8121 | SIGMA-ALDRICH-FLUKA |
| D-Alanine | C3H7NO 2 | 839 | 1152,391 | 6195 | SIGMA-ALDRICH-FLUKA |
| 1-(5'-Phosphoribosyl)-5-aminoimidazole | C8H14N3 O7P | 840 | 1358,486 | 7638 | SIGMA-ALDRICH-FLUKA |
| 5-Amino-1-(5-phospho-D-ribosyl)imidazole-4-carboxamide | C9H15N4 O8P | 841 | 1401,512 | 7939 | HPLC purification from *Saccharomyces cerivisiae* |
| D-Alanyl-D-alanine | C6H12N2 O3 | 842 | 1223,470 | 6693 | SIGMA-ALDRICH-FLUKA |
| (S)-2-Acetolactate | C5H8O4 | 843 | 1211,412 | 6496 | SIGMA-ALDRICH-FLUKA |
| 2-Ketoglutaric acid | C5H6O5 | 844 | 1225,396 | 6594 | SIGMA-ALDRICH-FLUKA |
| N-Acetyl-beta-alanine | C5H9NO 3 | 845 | 1225,463 | 6489 | SIGMA-ALDRICH-FLUKA |
| L-Alanine | C3H7NO 2 | 846 | 1152,391 | 6195 | SIGMA-ALDRICH-FLUKA |
| Alanyllactate | C6H11N O4 | 847 | 1240,454 | 6700 | SIGMA-ALDRICH-FLUKA |
| N-Methyl-L-alanine | C4H9NO 2 | 848 | 1182,418 | 6293 | SIGMA-ALDRICH-FLUKA |
| 5-Aminolevulinate | C5H9NO 3 | 849 | 1194,429 | 6489 | SIGMA-ALDRICH-FLUKA |
| Alditol | C2H5O2( C4H8O2) | 850 | 1212,464 | 6616 | SIGMA-ALDRICH-FLUKA |
| D-Gluconic acid | C7H12O 7 | 851 | 1285,492 | 6945 | SIGMA-ALDRICH-FLUKA |
| D-Aldose | C6H12O 6 | 852 | 1257,482 | 6833 | SIGMA-ALDRICH-FLUKA |
| ADP-glucose | C16H25N 5O15P2 | 853 | 1652,644 | 9698 | SIGMA-ALDRICH-FLUKA |
| Allyl alcohol | C3H6O | 854 | 1120,370 | 5978 | SIGMA-ALDRICH-FLUKA |
| D-Allose | C6H12O 6 | 855 | 1243,455 | 6833 | SIGMA-ALDRICH-FLUKA |

| | | | | | |
|---|---|---|---|---|---|
| Allophanate | C2H4N2 O3 | 856 | 1197,388 | 6300 | Etyl allophanate (provided by gs-chem) + metagenomic lipase EL1 |
| S-Adenosyl-L-methionine | C15H22N 6O5S | 857 | 1461,733 | 8361 | SIGMA-ALDRICH-FLUKA |
| 5-Amino-4-imidazole carboxylate | C4H5N3 O2 | 858 | 1220,426 | 6461 | HPLC purification from *Saccharomyces cerivisiae* |
| (S)-Allantoin | C4H6N4 O3 | 859 | 1306,476 | 6678 | SIGMA-ALDRICH-FLUKA |
| L-2-Aminoadipate 6-semialdehyde | C6H11N O3 | 860 | 1238,482 | 6588 | Synthesized as described by Borgeat *et al., J Biol Chem* 251, 7816 (1976) |
| Altrose | C6H12O 6 | 861 | 1257,482 | 6833 | SIGMA-ALDRICH-FLUKA |
| (R)-Allantoin | C4H6N4 O3 | 862 | 1306,476 | 6776 | SIGMA-ALDRICH-FLUKA |
| Aminofructose 6-phosphate | C6H14N O8P | 863 | 1322,451 | 7386 | HPLC purification from *Saccharomyces cerivisiae* |
| 2-Aminomuconate 6-semialdehyde | C6H7NO 3 | 864 | 1204,424 | 6559 | HPLC purification from *Saccharomyces cerivisiae* |
| L-2-Aminoadipate | C6H11N O4 | 865 | 1224,455 | 6700 | HPLC purification from *Saccharomyces cerivisiae* |
| D-Altronate | C6H12O 7 | 866 | 1273,481 | 6945 | SIGMA-ALDRICH-FLUKA |
| Acetyl-maltose | C14H24 O12 | 867 | 1461,661 | 8262 | SIGMA-ALDRICH-FLUKA |
| S-Adenosyl-L-threonine | C14H20N 7O6 | 868 | 1445,654 | 7741 | SIGMA-ALDRICH-FLUKA |
| S-Adenosylmethioninamine | C14H23N 6O3S | 869 | 1418,732 | 6303 | SIGMA-ALDRICH-FLUKA |
| Allantoate | C4H8N4 O4 | 870 | 1269,455 | 9298 | SIGMA-ALDRICH-FLUKA |
| Methyl D-glucoside | C7H14O 6 | 871 | 1257,482 | 7080 | SIGMA-ALDRICH-FLUKA |
| 5-Aminovaleric acid | C5H11N O2 | 872 | 1180,445 | 7985 | SIGMA-ALDRICH-FLUKA |
| Aminoacetone | C3H7NO | 873 | 1136,392 | 8449 | SIGMA-ALDRICH-FLUKA |

| | | | | | |
|---|---|---|---|---|---|
| Aminoimidazole ribotide | C8H14N3O7P | 874 | 1358,486 | 10474 | Provided by Hangzhou Capot Chemical Co.,Ltd. |
| 2-Aminophenol | C6H7NO | 875 | 1172,425 | 8449 | SIGMA-ALDRICH-FLUKA |
| S-Adenosyl-4-methylthio-2-oxobutanoate | C15H20N5O6S | 876 | 1461,712 | 8449 | HPLC purification from *Saccharomyces cerivisiae* |
| Naphthyl-2-methylene-succinyl-CoA | C36H46N7O19P3S | 877 | 2085,074 | 8097 | Naphthyl-2-methylene-succinate (HPLC purification from *Pseudomonas* sp.) + CoA + ACETYLTRANSFERASA Arabidopsis thaliana - Naphthyl-2-methylene-succinyl-CoA |
| Naphthyl-2-hydroxymethyl-succinyl CoA | C36H48N7O20P3S | 878 | 2103,090 | 8097 | Naphthyl-2-hydroxymethyl-succinate (HPLC purification from *Pseudomonas* sp.) + CoA + ACETYLTRANSFERASA Arabidopsis thaliana - Naphthyl-2-hydroxymethyl-succinyl CoA |
| 2-Aminomuconate | C6H7NO4 | 879 | 1250,449 | 8746 | HPLC purification from *Saccharomyces cerivisiae* |
| Amylose | (C120H2000O100) | 880 | 4306,145 | 10044 | SIGMA-ALDRICH-FLUKA |
| Amylopectin | C30H52O26 | 881 | 1892,025 | 7425 | SIGMA-ALDRICH-FLUKA |
| 5-Carboxymethyl-2-oxohex-3-ene-1,6-dioate | C8H5O7 | 882 | 1292,421 | 6367 | HPLC purification from *Saccharomyces cerivisiae* |
| AMP | C10H14N5O7P | 883 | 1410,522 | 7048 | SIGMA-ALDRICH-FLUKA |
| 1,3-Cyclohexanedione | C6H8O2 | 884 | 1191,425 | 9234 | SIGMA-ALDRICH-FLUKA |
| 3-Hydroxycyclohexanone | C6H10O2 | 885 | 1193,441 | 7689 | As in Tetrahedron, 50, p. 3843, 1994 |
| Naphthyl-2-oxomethyl-succinyl-CoA | C36H46N7O20P3S | 886 | 2101,074 | 8145 | Naphthyl-2-oxomethyl-succinate (HPLC purification from *Pseudomonas* sp.) + CoA + ACETYLTRANSFERASA Arabidopsis thaliana - Naphthyl-2-oxomethyl-succinyl-CoA |
| Naphthalen-1-yl hydrogen phosphate | C10H10NaO5P | 887 | 1343,447 | 11190 | As in Journal of the American Chemical Society, 72, p. 624, 1950 |
| Anisole | C7H8O | 888 | 1187,436 | 6367 | SIGMA-ALDRICH-FLUKA |
| Naphthalen-1-yl acetate | C12H10O2 | 889 | 1265,507 | 6367 | As described by Kumar *et al., Bioorganic 6 Medicinal Chemistry Letters* 16, 2719 (2006) |
| Aniline | C6H7N | 890 | 1172,425 | 8363 | SIGMA-ALDRICH-FLUKA |
| Anthranilate | C7H7NO2 | 891 | 1216,435 | 9106 | SIGMA-ALDRICH-FLUKA |

| | | | | | |
|---|---|---|---|---|---|
| Anserine | C10H16N4O3 | 892 | 1319,559 | 10742 | HPLC purification from *Saccharomyces cerivisiae* |
| Anthracene | C14H10 | 893 | 1257,530 | 7401 | SIGMA-ALDRICH-FLUKA |
| D-Arabinono-1,4-lactone | C5H8O5 | 894 | 1225,439 | 7809 | As by Punzo et al. (2005) Acta Crys. E61, o326-o327 |
| P1,P5-Bis(5'-adenosyl) pentaphosphate | C20H29N10O22P5 | 895 | 1979,670 | 6367 | As described by Gillespie and Hudspeth, *Proc Natl Acad Sci USA* 90, 2170 (1993) |
| 3-O-L-Alanyl-1-O-phosphatidylglycerol | C10H19NO10P(CH3) | 896 | 1422,567 | 6367 | HPLC purification from *Saccharomyces cerivisiae* |
| N-Acetylputrescine | C6H14N2O | 897 | 1193,487 | 7409 | SIGMA-ALDRICH-FLUKA |
| 3-Aminopropanal | C3H7NO | 898 | 1136,392 | 8561 | As described by Castillo *et al.*, *Organic Lett* 8, 6067 (2006) |
| alpha-Aminopropiononitrile | C3H6N2 | 899 | 1133,390 | 7977 | As described by Kawashiro *et al.*, *Bulletin of the Chemical Society of Japan* 50, 2956 (1977) |
| alpha-Pinene | C10H16 | 900 | 1215,534 | 8433 | SIGMA-ALDRICH-FLUKA |
| 5'-Adenylyl sulfate | C10H14N5O10PS | 901 | 1490,580 | 8265 | SIGMA-ALDRICH-FLUKA |
| 3'-Phosphoadenylyl sulfate | C10H15N5O13P2S | 902 | 1570,561 | 8129 | HPLC purification from *Saccharomyces cerivisiae* |
| L-Arabinono-1,5-lactone | C5H8O5 | 903 | 1211,412 | 7345 | As described by Alistair *et al.*, *Tetrahedron: assymetry* 13, 2667 (2002) |
| D-Arabinose 5-phosphate | C5H11O8P | 904 | 1293,409 | 7320 | SIGMA-ALDRICH-FLUKA |
| D-Arabinose | C5H10O5 | 905 | 1227,455 | 8577 | SIGMA-ALDRICH-FLUKA |
| L-Arabinose | C5H10O5 | 906 | 1227,455 | 6367 | SIGMA-ALDRICH-FLUKA |
| P1,P4-Bis(5'-adenosyl) tetraphosphate | C20H28N10O19P4 | 907 | 1899,690 | 7937 | As described by Watanabe *et al.*, *Acta Cryst.* C52, 338 (1996) |
| L-Arginine | C6H14N4O2 | 908 | 1237,500 | 7985 | SIGMA-ALDRICH-FLUKA |
| Biphenyl-2,3-diol | C12H10O2 | 909 | 1265,507 | 10241 | As described in Ishigooka *et al.*, *Agricultural and Biological Chemistry* 50, 1045 (1996) |
| Arbutin 6-phosphate | C12H17O10P | 910 | 1431,532 | 8066 | HPLC purification from *Saccharomyces cerivisiae* |
| Arene oxide | C6H6O | 911 | 1173,409 | 7810 | As described by Agarwal *et al. J. Chem. Soc., Perkin Trans.* 1, 1969 (1990) |

| | | | | | |
|---|---|---|---|---|---|
| Arachidic acid | C20H40O2 | 912 | 1391,833 | 8602 | SIGMA-ALDRICH-FLUKA |
| L-Arginine methyl ester | C6H13N4O2(CH3) | 913 | 1251,527 | 7745 | SIGMA-ALDRICH-FLUKA |
| Arg-Pro-Lys-Pro-Gln-Gln-Phe-Phe-Gly-Leu-Met-NH2 | C75H108N20O13 | 914 | # | 9402 | SIGMA-ALDRICH-FLUKA |
| Arginine p-nitroaniline | C12H18N6O3 | 915 | 1357,611 | 6392 | SIGMA-ALDRICH-FLUKA |
| L-Arginosuccinic acid | C10H18N4O6 | 916 | 1353,574 | 13182 | SIGMA-ALDRICH-FLUKA |
| L-Aspartate 4-semialdehyde | C4H7NO3 | 917 | 1180,402 | 8218 | SIGMA-ALDRICH-FLUKA |
| Ascorbyl butyrate | C10H16O6 | 918 | 1295,531 | 9592 | SIGMA-ALDRICH-FLUKA |
| Ascorbyl propionate | C9H14O2 | 919 | 1217,506 | 9910 | SIGMA-ALDRICH-FLUKA |
| Ascorbyl acetate | C8H12O2 | 920 | 1203,479 | 8401 | SIGMA-ALDRICH-FLUKA |
| Ascorbyl hexanoate | C12H20O2 | 921 | 1259,587 | 7910 | SIGMA-ALDRICH-FLUKA |
| L-Asparagine | C4H8N2O3 | 922 | 1195,416 | 8137 | SIGMA-ALDRICH-FLUKA |
| Ascorbyl octanoate | C14H24O2 | 923 | 1287,640 | 7830 | SIGMA-ALDRICH-FLUKA |
| Arylamine | C6H7N | 924 | 1172,425 | 6720 | Provided by DSL |
| L-Aspartate | C4H7NO4 | 925 | 1196,401 | 8002 | SIGMA-ALDRICH-FLUKA |
| Atropine | C17H23NO3 | 926 | 1352,672 | 6848 | As described by I.L.Finar, *Organic Chemistry* Vol. 2, 636 (1965) |
| 2-Hydroxylminostilbene | C14H11NO | 927 | 1288,544 | 6367 | As described by Chang, *J Heterocyclic Chem* 20, 237 (1983) |
| Atrazine | C8H14ClN5 | 928 | 1294,983 | 8777 | SIGMA-ALDRICH-FLUKA |
| 4-Hydroxymethylsalicylate | C8H7O4 | 929 | 1230,438 | 14431 | Provided by Dayang Chemicals Co., Ltd. |
| ATP | C10H16N | 930 | 1570,482 | 7329 | SIGMA-ALDRICH-FLUKA |

| | | | | | |
|---|---|---|---|---|---|
| | 5O13P3 | | | | |
| Azetidine | C3H7N | 931 | 1136,392 | 7072 | As described by Y. Ju and R. S. Varma, *J. Org. Chem.* 71, 135 (2006) |
| Aziridine | C2H5N | 932 | 1122,366 | 8521 | As described by A. J. Catino *et al.*, *Org. Lett.* 7, 2787 (2005) |
| 1-(5'-Phosphoribosyl)-5-amino-4-imidazolecarboxamide | C9H15N4 O8P | 933 | 1401,512 | 7753 | SIGMA-ALDRICH-FLUKA |
| gamma-Butyrobetainyl-CoA | C28H50N 8O17P3S | 934 | # | 16375 | HPLC purification from *Saccharomyces cerivisiae* |
| Cycloheptaamylose | C42H70 O35 | 935 | 2198,294 | 7777 | SIGMA-ALDRICH-FLUKA |
| Butanoyldolichol | C21H35 O2(C50H 80) | 936 | 2079,990 | 8777 | HPLC purification from soybean |
| Benzyl 2-methyl-3-oxobutanoate | C12H14 O3 | 937 | 1285,538 | 7881 | As described by Cristaue et *al.*, *Current Organis Synthesis* 2, 113 (2005) |
| Benzyl (2R,3S)-2-methyl-3-hydroxybutanoate | C12H16 O3 | 938 | 1287,554 | 7769 | As described by Kuramoto *et al.*, *Bioscience, Biotechnology, and Biochemistry* 63, 598 (1999) |
| 4-Phenylbutan-2-one | C10H12 O | 939 | 1227,501 | 7585 | As described in Chemistry Letters, 12, 1537, (1983) |
| Phenylmethyl benzoate | C14H12 O2 | 940 | 1291,545 | 8842 | As describd in Tetrahedron Letters, 27, p. 2383, 1986 |
| Benzoyl-CoA | C28H40N 7O17P3S | 941 | 1950,940 | 7777 | Benzoic acid + CoA + ACETYLTRANSFERASA Arabidopsis thaliana - benzoyl-CoA |
| Phenylmethyl hexanoate | C13H18 O2 | 942 | 1285,582 | 7760 | Phenylmethyl acetate + vinyl hexanoate + metagenomic lipase EL1 |
| Phenylmethyl acetate | C9H10O 2 | 943 | 1229,474 | 7761 | Provided by Alfa Aesar (http://www.alfa.com/alf/laboratory_chemical_suppliers.htm) |
| Phenylmethyl propanoate | C10H12 O2 | 944 | 1243,501 | 8225 | Phenylmethyl acetate + vinyl propanoate + metagenomic lipase EL1 |
| (R,S)-Tetrahydrobenzylisoquinoline | C16H17N | 945 | 1302,615 | 12941 | Method modified from Baxendale (2003) Heterocycles 60, 2707-2715 |
| Benzimidazole | C7H6N2 | 946 | 1197,434 | 7801 | SIGMA-ALDRICH-FLUKA |
| Benzyl alcohol | C7H8O | 947 | 1187,436 | 6816 | SIGMA-ALDRICH-FLUKA |
| 2,1-Benzoxazole | C7H5NO | 948 | 1198,420 | 7545 | SIGMA-ALDRICH-FLUKA |
| Benzene | C6H6 | 949 | 1157,410 | 8506 | SIGMA-ALDRICH-FLUKA |
| Poly-beta-Hydroxybutyrate | (C40H60 O20) | 950 | 1940,201 | 8777 | SIGMA-ALDRICH-FLUKA |

| | | | | | |
|---|---|---|---|---|---|
| Benzamide | C7H7NO | 951 | 1200,435 | 8137 | SIGMA-ALDRICH-FLUKA |
| 1,2-Benzophenanthrene | C18H12 | 952 | 1307,590 | 7488 | SIGMA-ALDRICH-FLUKA |
| 1-Benzothiophene | C8H6S | 953 | 1213,492 | 7409 | SIGMA-ALDRICH-FLUKA |
| 2-Benzothiophene | C8H6S | 954 | 1213,492 | 7449 | SIGMA-ALDRICH-FLUKA |
| Betaine aldehyde | C5H12NO | 955 | 1181,453 | 7721 | SIGMA-ALDRICH-FLUKA |
| Benzocyclobutene | C8H6 | 956 | 1181,432 | 16292 | As described by Jun-Xiao *et al., Journal of Chemical Research* 6, 430 (2004) |
| Benzofuran | C8H6O | 957 | 1197,431 | 16932 | SIGMA-ALDRICH-FLUKA |
| 2,6-Dichlorobenzonitrile | C7H3Cl2N | 958 | 1251,311 | 18535 | Synthesized as described by Kalevaru *et al., Catalysis Today* (doi:10.1016/j.cattod.2008.09.009) (2008) |
| Benzoate | C7H6O2 | 959 | 1201,420 | 9786 | SIGMA-ALDRICH-FLUKA |
| 2-Methylthiobenzothiazole | C8H7NS2 | 960 | 1260,567 | 11323 | As in Chemistry of Heterocyclic Compounds (1973) 9, 1138-1141 |
| 2H-Benzotriazole | C6H5N3 | 961 | 1198,423 | 11323 | SIGMA-ALDRICH-FLUKA |
| Benzoxazole | C7H5NO | 962 | 1198,420 | 34554 | SIGMA-ALDRICH-FLUKA |
| Benzocycloheptene | C11H14 | 963 | 1225,529 | 6367 | Synthesized as described by Liu *et al., Chinese Chemical Letters* 19, 428 (2008) |
| Betaine | C5H11NO2 | 964 | 1196,445 | 10747 | SIGMA-ALDRICH-FLUKA |
| Benzonitrile | C8H6O | 965 | 1197,431 | 7233 | SIGMA-ALDRICH-FLUKA |
| N-Benzoylanthranilate | C14H11NO3 | 966 | 1320,543 | 19694 | SIGMA-ALDRICH-FLUKA |
| CDP-1,2-Dihexadecanoylglycerol | C14H19N3O15P2(C32H64O4) | 967 | 2123,418 | 7618 | Synthesized as described by Kolicheski *et al., Journal of Analytical and Applied Products* 80, 92 (2007) |
| Myrcene | C10H16 | 968 | 1215,534 | 7713 | SIGMA-ALDRICH-FLUKA |
| Bromobenzene-2,3-oxide | C6H5BrO | 969 | 1252,306 | 8785 | As described by Born *et al., Drug Metabolism and Disposition* 25, 1318 (1997) |
| Nicofuranose | C30H24N4O10 | 970 | 1679,838 | 9443 | Provided by Chemsynlab Pharmaceutical Science & Technology Co. Ltd. |
| Bromobenzene-3,4-oxide | C6H5BrO | 971 | 1252,306 | 10330 | As described by Born *et al., Drug Metabolism and Disposition* 25, 1318 (1997) |
| 2-Bromomaleylacetate | C6H5BrO5 | 972 | 1330,330 | 9130 | Maleylacetate (SIGMA-ALDRICH-FLUKA) + dehalogenase (metagenomic) |
| m-Bromobenzotrifluoride | C7H4BrF3 | 973 | 1304,305 | 8441 | Provided by Advanced Synthesis |

| | | | | | |
|---|---|---|---|---|---|
| Butanoyl-CoA | C25H42N7O17P3S | 974 | 1916,923 | 6367 | Butenoic acid+ CoA + ACETYLTRANSFERASA Arabidopsis thaliana - butenoyl-CoA |
| 4-Aminophenol | C6H7NO | 975 | 1172,425 | 7080 | SIGMA-ALDRICH-FLUKA |
| epsilon-N-Biotinyl-L-lysine | C16H28N4O4S | 976 | 1435,780 | 8729 | SIGMA-ALDRICH-FLUKA |
| Butylamine | C4H11N | 977 | 1152,435 | 9074 | SIGMA-ALDRICH-FLUKA |
| Butyrate | C4H8O2 | 978 | 1167,403 | 7649 | SIGMA-ALDRICH-FLUKA |
| Butyl butyrate | C8H16O2 | 979 | 1223,511 | 7424 | SIGMA-ALDRICH-FLUKA |
| Butyryl-CoA | C25H42N7O17P3S | 980 | 1916,923 | 7536 | Butyric acid+ CoA + ACETYLTRANSFERASA Arabidopsis thaliana - butyryl-CoA |
| Uridine | C9H12N2O6 | 981 | 1323,500 | 7417 | SIGMA-ALDRICH-FLUKA |
| 1-(5-Phospho-D-ribosyl)-5-amino-4-imidazolecarboxylate | C9H14N3O9P | 982 | 1402,496 | 7080 | Synthesized as described by Takahashi *et al.*, *Bulletin of the Chemical Society of Japan* 53, 557 (1980) |
| Cyclohexa-1,5-diene-1-carbonyl-CoA | C28H42N7O17P3S | 983 | 1952,956 | 7657 | HPLC purification from *Saccharomyces cerivisiae* |
| Cellobiose-1,5-lactone | C12H20O11 | 984 | 1417,608 | 7192 | SIGMA-ALDRICH-FLUKA |
| Hexadec-1-ene | C16H32 | 985 | 1303,727 | 7417 | SIGMA-ALDRICH-FLUKA |
| Cadaverine | C5H14N2 | 986 | 1165,476 | 7561 | SIGMA-ALDRICH-FLUKA |
| Caffeate | C9H8O4 | 987 | 1259,456 | 7937 | Alpin Chemical |
| Caffeoyl-CoA | C30H42N7O19P3S | 988 | 2008,976 | 7809 | Caffeic acid + CoA + ACETYLTRANSFERASA Arabidopsis thaliana → Caffeoyl-CoA |
| Butyl paraben | C11H14O3 | 989 | 1273,527 | 11083 | SIGMA-ALDRICH-FLUKA |
| N-Carbamyl-L-glutamate | C6H10N2O5 | 990 | 1283,479 | 6832 | SIGMA-ALDRICH-FLUKA |
| Methyl caffeate | C10H10O4 | 991 | 1273,483 | 7809 | Provided by Apin Chemicals Limited (UK) |
| Catechol | C6H6O2 | 992 | 1189,409 | 7200 | SIGMA-ALDRICH-FLUKA |
| (-)-trans-Carveol | C10H16O | 993 | 1231,533 | 9556 | SIGMA-ALDRICH-FLUKA |
| 3',5'-Cyclic AMP | C10H12N | 994 | 1392,507 | 7384 | SIGMA-ALDRICH-FLUKA |

| | | | | | |
|---|---|---|---|---|---|
| | 5O6P | | | | |
| epsilon-Caprolactam | C6H11N O | 995 | 1192,456 | 7632 | SIGMA-ALDRICH-FLUKA |
| Hexanoyl-CoA | C27H46N 7O17P3S | 996 | 1944,977 | 7529 | SIGMA-ALDRICH-FLUKA |
| 5-Carboxy-2-pentenoyl-CoA | C27H42N 7O19P3S | 997 | 1972,943 | 7809 | HPLC purification from *Saccharomyces cerivisiae* |
| Carbazole | C12H9N | 998 | 1246,507 | 7745 | SIGMA-ALDRICH-FLUKA |
| 8-Hydroxy-2-methyl-alpha-carboline | C12H10N 2O | 999 | 1277,520 | 8621 | HPLC purification from *Saccharomyces cerivisiae* |
| N-Butyl-beta-carboline-3-carboxylate | C16H16N 2O2 | 1000 | 1347,612 | 7497 | As described by Medina *et al., Proc Natl Acad Sci USA* 83, 4952 (1986) |
| 8-Hydroxy-2-methyl-beta-carboline | C12H10N 2O | 1001 | 1277,520 | 8531 | HPLC purification from *Saccharomyces cerivisiae* |
| 8-Hydroxy-2-methyl-gamma-carboline | C12H10N 2O | 1002 | 1277,520 | 8041 | HPLC purification from *Saccharomyces cerivisiae* |
| Carnosine | C9H14N4 O3 | 1003 | 1289,533 | 9443 | SIGMA-ALDRICH-FLUKA |
| Carvone | C10H14 O | 1004 | 1229,517 | 6367 | SIGMA-ALDRICH-FLUKA |
| 2-(3-Carboxy-3-aminopropyl)-L-histidine | C10H16N 4O4 | 1005 | 1319,559 | 9211 | HPLC purification from *Saccharomyces cerivisiae* |
| Catechin | C15H14 O6 | 1006 | 1353,570 | 10862 | SIGMA-ALDRICH-FLUKA |
| N-Carbamoyl-L-aspartate | C5H8N2 O5 | 1007 | 1269,452 | 9721 | As described by Cao *et al., Australian Journal of Chemistry* 47, 903 (1994) |
| 3-Isopropylmalate | C7H12O 5 | 1008 | 1255,466 | 9721 | Isopropanol + methylmalate + metagenomic lipase EL1 |
| Cobinamide | C48H72C oN11O8 | 1009 | 2069,398 | 17862 | Synthesized as described by Renz, *Biochem Biophys Res Commun* 30, 373 (1968) |
| Carbamoyl phosphate | CH4NO5 P | 1010 | 1289,379 | 9370 | Synthesized as described by Renz, *Biochem Biophys Res Commun* 30, 373 (1968) |
| m-Aminophenol | C6H7NO | 1011 | 1188,424 | 9051 | SIGMA-ALDRICH-FLUKA |
| 4-Hydroxyphenylglyoxylate | C8H6O4 | 1012 | 1245,429 | 9621 | Provided by Astatech |
| 3',5'-Cyclic CMP | C9H12N3 | 1013 | 1368,481 | 11012 | SIGMA-ALDRICH-FLUKA |

| | | | | | |
|---|---|---|---|---|---|
| | O7P | | | | |
| 3',5'-Cyclic dAMP | C10H12N5O5P | 1014 | 1376,507 | 11092 | SIGMA-ALDRICH-FLUKA |
| trans-Hex-2-enoyl-CoA | C27H44N7O17P3S | 1015 | 1942,961 | 15348 | trans-Hex-2-enoate (provided by Honest Joy Chemicals) + CoA + ACETYLTRANSFERASA Arabidopsis thaliana → trans-Hex-2-enoyl-CoA |
| cis-1,2-Dihydrobenzene-1,2-diol | C6H8O2 | 1016 | 1191,425 | 9081 | Benzene + 1,2-benzene dioxygenase (metagenomic) |
| Capryldihydroxyacetonephosphate | C4H6O7P(C8H16O2) | 1017 | 1420,571 | 11372 | HPLC purification from *Saccharomyces cerivisiae* |
| cis-1,2-Dihydro-3-ethylcatechol | C8H12O2 | 1018 | 1219,479 | 9361 | Provided by Chiral Quest (Jiashan) Co., Ltd. |
| CDP-1,2-Dinonadecanoylglycerol | C14H19N3O15P2(C9H18O2) | 1019 | 1768,802 | 14855 | HPLC purification from *Saccharomyces cerivisiae* |
| CDP-butyrylglycerol | C13H20N3O14P2(C4H8O2) | 1020 | 1671,664 | 13884 | HPLC purification from *Saccharomyces cerivisiae* |
| Benzyl-L-cysteinamide | C33H32N2O5S | 1021 | 1647,984 | 6282 | As by Murakami *et al.*, *Bulletin of the Chemical Society of Japan* 52, 2996 (1979) |
| CDP-Hexadecanoylglycerol | C13H20N3O14P2(C16H32O2) | 1022 | 1839,987 | 9730 | HPLC purification from *Saccharomyces cerivisiae* |
| CDP-1,2-Dibutyrylglycerol | C14H19N3O15P2(C4H8O2)2 | 1023 | 1786,773 | 8846 | HPLC purification from *Saccharomyces cerivisiae* |
| CDP-choline | C14H26N4O11P2 | 1024 | 1567,625 | 8027 | HPLC purification from *Saccharomyces cerivisiae* |
| CDP-Dodecanoylglycerol | C13H20N3O14P2(C12H24O2) | 1025 | 1783,879 | 9379 | HPLC purification from *Saccharomyces cerivisiae* |
| CDP-1,2-Dinonanoylglycerol | C14H19N | 1026 | 1927,042 | 10274 | HPLC purification from *Saccharomyces cerivisiae* |

| | | | | | |
|---|---|---|---|---|---|
| | 3O15P2(C9H18O2)2 | | | | |
| CDP-Tetradecanoylglycerol | C13H20N3O14P2(C14H28O2) | 1027 | 1811,933 | 9554 | HPLC purification from *Saccharomyces cerivisiae* |
| CDP-1,2-Ditetradecanoylglycerol | C14H19N3O15P2(C14H28O2)2 | 1028 | 2067,310 | 11150 | HPLC purification from *Saccharomyces cerivisiae* |
| CDP-1,2-Dihexanoylglycerol | C14H19N3O15P2(C16H32O2)2 | 1029 | 2123,418 | 11501 | HPLC purification from *Saccharomyces cerivisiae* |
| CDP-1,2-Dioleylglycerol | C14H19N3O15P2(C18H36O2)2 | 1030 | 2179,525 | 12252 | HPLC purification from *Saccharomyces cerivisiae* |
| CDP-1,2-Dioctanoylglycerol | C14H19N3O15P2(C8H16O2)2 | 1031 | 1898,988 | 10098 | HPLC purification from *Saccharomyces cerivisiae* |
| CDP-Octanoylglycerol | C13H20N3O14P2(C8H16O2) | 1032 | 1727,772 | 9028 | HPLC purification from *Saccharomyces cerivisiae* |
| Cytidine diphosphate | C9H15N3O11P2 | 1033 | 1482,476 | 7495 | SIGMA-ALDRICH-FLUKA |
| CDP-1,2-Didodecanoylglycerol | C14H19N3O15P2(C12H24O2)2 | 1034 | 2011,203 | 10800 | HPLC purification from *Saccharomyces cerivisiae* |
| CDP-1,2-Dioctadecanoylglycerol | C14H19N3O15P2( | 1035 | 2175,494 | 10274 | HPLC purification from *Saccharomyces cerivisiae* |

| | | | | | |
|---|---|---|---|---|---|
| | C18H34O2)2 | | | | |
| CDP-1,2-Dipropionylglycerol | C14H19N3O15P2(C3H6O2)2 | 1036 | 1758,719 | 9222 | HPLC purification from *Saccharomyces cerivisiae* |
| CDP-Decanoylglycerol | C13H20N3O14P2(C10H20O2) | 1037 | 1755,825 | 9203 | HPLC purification from *Saccharomyces cerivisiae* |
| CDP-ethanolamine | C11H20N4O11P2 | 1038 | 1594,608 | 8127 | HPLC purification from *Saccharomyces cerivisiae* |
| 3-Hydroxyaminophenol | C6H7NO2 | 1039 | 1204,424 | 9211 | SIGMA-ALDRICH-FLUKA |
| CDP-glycerol | C12H21N3O13P2 | 1040 | 1540,557 | 12733 | HPLC purification from *Saccharomyces cerivisiae* |
| CDP-hexanoylglycerol | C13H20N3O14P2(C6H12O2) | 1041 | 1699,718 | 14165 | HPLC purification from *Saccharomyces cerivisiae* |
| CDP-Nonanoylglycerol | C13H20N3O14P2(C9H18O2) | 1042 | 1741,799 | 14585 | HPLC purification from *Saccharomyces cerivisiae* |
| CDP-Nonadecanoylglycerol | C13H20N3O14P2(C19H38O2) | 1043 | 1882,067 | 15988 | HPLC purification from *Saccharomyces cerivisiae* |
| CDP-propionylglycerol | C13H20N3O14P2(C3H6O2) | 1044 | 1657,637 | 13744 | HPLC purification from *Saccharomyces cerivisiae* |
| 4-Hydroxybiphenyl | C12H10O | 1045 | 1249,507 | 9662 | SIGMA-ALDRICH-FLUKA |
| Cellobiose | C12H22O11 | 1046 | 1405,597 | 11383 | SIGMA-ALDRICH-FLUKA |

| | | | | | |
|---|---|---|---|---|---|
| Cellotriose | C18H32O16 | 1047 | 1567,740 | 13005 | SIGMA-ALDRICH-FLUKA |
| Cellotetraose | C24H42O21 | 1048 | 1729,882 | 14626 | SIGMA-ALDRICH-FLUKA |
| Cellulose | (C6H10O5)20 | 1049 | 4306,145 | 40395 | SIGMA-ALDRICH-FLUKA |
| cis-3-(3-Carboxyethenyl)-3,5-cyclohexadiene-1,2-diol | C9H10O4 | 1050 | 1245,473 | 9781 | HPLC purification from *Saccharomyces cerivisiae* |
| CDP-1,2-Didecanoylglycerol | C14H19N3O15P2(C12H24O2)2 | 1051 | 2011,203 | 17279 | HPLC purification from *Saccharomyces cerivisiae* |
| L-Cysteinylglycine | C5H10N2O3S | 1052 | 1241,503 | 9742 | SIGMA-ALDRICH-FLUKA |
| Cetyl alcohol | C16H34O | 1053 | 1321,742 | 10384 | SIGMA-ALDRICH-FLUKA |
| 4-Chlorocatechol | C6H5ClO2 | 1054 | 1207,855 | 9405 | SIGMA-ALDRICH-FLUKA |
| 3',5'-Cyclic GMP | C10H12N5O7P | 1055 | 1408,506 | 11412 | SIGMA-ALDRICH-FLUKA |
| Cyclohex-2-enone | C6H8O | 1056 | 1175,425 | 8921 | Provided by Nanjing Sunglow Imp & Exp Co., Ltd. |
| 6-Carboxyhexanoyl-CoA | C28H46N7O19P3S | 1057 | 2003,013 | 17058 | 6-Carboxyhexenoate + CoA + ACETYLTRANSFERASA Arabidopsis thaliana → 6-Carboxyhexenoyl-CoA |
| Cyclohexan-1,2-dione | C6H8O2 | 1058 | 1191,425 | 9081 | Provided by PSN Chemical Technology Co., Ltd |
| Cyclohexanone | C6H10O | 1059 | 1177,441 | 8941 | SIGMA-ALDRICH-FLUKA |
| Chitin | (C8H13NO5)20 | 1060 | 5127,195 | 48607 | SIGMA-ALDRICH-FLUKA |
| Chitosan | C12H24N2O9(C6H11NO4)20 | 1061 | 4626,780 | 43602 | SIGMA-ALDRICH-FLUKA |
| Chitobiose | C16H28N2O11 | 1062 | 1487,702 | 12204 | SIGMA-ALDRICH-FLUKA |
| Choline | C5H14NO | 1063 | 1183,469 | 9001 | SIGMA-ALDRICH-FLUKA |

| | | | | | |
|---|---|---|---|---|---|
| Phenyl acetate | C8H8O2 | 1064 | 1215,447 | 9321 | SIGMA-ALDRICH-FLUKA |
| Ethyl acetate | C4H8O2 | 1065 | 1167,403 | 8840 | SIGMA-ALDRICH-FLUKA |
| Tolylacetate | C9H10O 2 | 1066 | 1229,474 | 9461 | SIGMA-ALDRICH-FLUKA |
| Ethyl butyryl acetate | C16H30 O4 | 1067 | 1365,708 | 9541 | SIGMA-ALDRICH-FLUKA |
| N-Acetyl-D-glucosamine 1,6-bisphosphate | C8H17N O12P2 | 1068 | 1458,495 | 11772 | SIGMA-ALDRICH-FLUKA |
| 4-Chlorophenol | C6H5ClO | 1069 | 1207,855 | 9245 | SIGMA-ALDRICH-FLUKA |
| Chorismate | C10H10 O6 | 1070 | 1305,482 | 10221 | SIGMA-ALDRICH-FLUKA |
| 5,7-Dihydroxychromone | C9H6O4 | 1071 | 1257,440 | 9741 | As described by Garazd *et al., Chemistry of Natural Compounds* 34, 435 (2006) |
| Citrate | C6H8O7 | 1072 | 1285,449 | 9881 | SIGMA-ALDRICH-FLUKA |
| CMP | C9H14N3 O8P | 1073 | 1386,497 | 11192 | SIGMA-ALDRICH-FLUKA |
| Cinnamoyl-CoA | C30H42N 7O17P3S | 1074 | 1976,978 | 16938 | Cinnamic acid + CoA + ACETYLTRANSFERASA Arabidopsis thaliana → Cinnamoyl-CoA |
| L-Citrulline | C6H13N3 O3 | 1075 | 1268,511 | 9711 | SIGMA-ALDRICH-FLUKA |
| Cinnamaldehyde | C9H8O | 1076 | 1211,458 | 9281 | SIGMA-ALDRICH-FLUKA |
| Cinnamyl acetate | C11H12 O2 | 1077 | 1255,512 | 9722 | SIGMA-ALDRICH-FLUKA |
| Cinnoline | C8H6N2 | 1078 | 1209,445 | 9261 | SIGMA-ALDRICH-FLUKA |
| 4-Chloro-m-cresol | C7H7ClO | 1079 | 1221,881 | 9385 | SIGMA-ALDRICH-FLUKA |
| 1-Bromopentane | C5H11Br | 1080 | 1230,343 | 9470 | SIGMA-ALDRICH-FLUKA |
| 1-Chloropentane | C5H11Cl | 1081 | 1185,892 | 9025 | SIGMA-ALDRICH-FLUKA |
| 2-Hydroxy-2,4-pentadienoate | C5H6O3 | 1082 | 1177,398 | 9100 | SIGMA-ALDRICH-FLUKA |
| Styrene oxide | C8H8O | 1083 | 1199,447 | 9161 | SIGMA-ALDRICH-FLUKA |
| 2,3-Dibromopentane | C5H10Br 2 | 1084 | 1309,239 | 10259 | SIGMA-ALDRICH-FLUKA |
| 1,2,3-Trimethylbenzene | C9H12 | 1085 | 1199,491 | 9161 | SIGMA-ALDRICH-FLUKA |
| trans-Aconitate | C6H6O6 | 1086 | 1267,433 | 9701 | SIGMA-ALDRICH-FLUKA |
| (R)-(+)-Citronellal | C10H18 O | 1087 | 1233,549 | 9502 | SIGMA-ALDRICH-FLUKA |

| | | | | | |
|---|---|---|---|---|---|
| (-)-Citronellol | C10H20O | 1088 | 1235,565 | 9522 | SIGMA-ALDRICH-FLUKA |
| Cinnamyl alcohol | C9H10O | 1089 | 1196,467 | 9301 | SIGMA-ALDRICH-FLUKA |
| CMP-3-deoxy-D-manno-octulosonate | C17H26N3O15P | 1090 | 1606,675 | 13394 | HPLC purification from *Saccharomyces cerivisiae* |
| Chlorobenzene | C6H5Cl | 1091 | 1191,856 | 9115 | SIGMA-ALDRICH-FLUKA |
| 2-Chlorobenzoate | C7H5ClO2 | 1092 | 1235,866 | 9555 | SIGMA-ALDRICH-FLUKA |
| Cardiolipin | C13H18O17P2(C3H6O2)4 | 1093 | 1883,838 | 14524 | HPLC purification from Arabidopsis thaliana |
| 3-Carboxy-cis,cis-muconate | C7H6O6 | 1094 | 1265,417 | 9821 | HPLC purification from *Pseudomonas putida KT2440* |
| 4-Chlorobutan-1-ol | C4H9ClO | 1095 | 1171,865 | 9045 | Provided by Alfa Aesar (http://www.alfa.com/alf/laboratory_chemical_suppliers.htm) |
| Chloroxylenol | C8H9ClO | 1096 | 1235,908 | 9171 | As described by Cox, *Acta Cryst.* C51, 1361 (1995) |
| Styrene | C8H8 | 1097 | 1183,448 | 9001 | SIGMA-ALDRICH-FLUKA |
| Chlorogenate | C16H18O9 | 1098 | 1433,610 | 11503 | SIGMA-ALDRICH-FLUKA |
| 2-Amino-3-carboxymuconate semialdehyde | C7H7NO5 | 1099 | 1264,433 | 9811 | HPLC purification from *Pseudomonas putida* KT2440 |
| gamma-Carboxymuconolactone | C7H6O6 | 1100 | 1249,418 | 9821 | HPLC purification from *Pseudomonas putida* KT2440 |
| Coenzyme A | C21H36N7O16P3S | 1101 | 1846,832 | 15636 | SIGMA-ALDRICH-FLUKA |
| Coniferyl alcohol | C10H12O3 | 1102 | 1259,500 | 9762 | SIGMA-ALDRICH-FLUKA |
| Coniferaldehyde | C10H10O3 | 1103 | 1257,484 | 9261 | SIGMA-ALDRICH-FLUKA |
| 3-Chloro-cis,cis-muconate | C6H5ClO4 | 1104 | 1255,853 | 9725 | SIGMA-ALDRICH-FLUKA |
| Coronamic acid | C6H11NO2 | 1105 | 1208,456 | 11534 | SIGMA-ALDRICH-FLUKA |
| Crotonoyl-CoA | C25H40N7O17P3S | 1106 | 1914,907 | 14395 | Crotonate (Abaco Inc) + CoA + ACETYLTRANSFERASA Arabidopsis thaliana → Crotonoyl-CoA |
| 3-Ethyltoluene | C9H12 | 1107 | 1199,491 | 9161 | SIGMA-ALDRICH-FLUKA |

| | | | | | |
|---|---|---|---|---|---|
| 4-Coumarate | C9H8O3 | 1108 | 1243,457 | 9601 | SIGMA-ALDRICH-FLUKA |
| p-Coumaroyl-CoA | C30H42N7O18P3S | 1109 | 1992,977 | 17098 | p-Coumarate (Apin Chemicals) + CoA + ACETYLTRANSFERASA Arabidopsis thaliana → p-Coumaroyl-CoA |
| Coumaran | C8H8O | 1110 | 1199,447 | 9161 | As described by Shriner and Witte, *J Am Chem Soc* 61, 2328 (1939) |
| Coumarin | C9H6O2 | 1111 | 1225,442 | 9421 | SIGMA-ALDRICH-FLUKA |
| Methyl p-coumarate | C10H10O3 | 1112 | 1257,484 | 9741 | Alpin Chemical |
| CDP-Octadecanoylglycerol | C13H20N3O14P2(C8H16O2) | 1113 | 1727,772 | 14445 | HPLC purification from *Saccharomyces cerivisiae* |
| 2-Coumarinate | C9H8O3 | 1114 | 1243,457 | 9601 | Provided by Shanghai Qiude Biochemical Engineering Co., Ltd. |
| Creatine | C4H9N3O2 | 1115 | 1194,431 | 9271 | SIGMA-ALDRICH-FLUKA |
| Ethylglycocyamine | C4H9N3O2 | 1116 | 1210,431 | 9271 | As described by Armstrong, *J. Org. Chem* 21, 503 (1956) |
| 3-Chlorocatechol | C6H5ClO2 | 1117 | 1223,855 | 9050 | SIGMA-ALDRICH-FLUKA |
| CTP | C9H16N3O14P3 | 1118 | 1546,456 | 12792 | SIGMA-ALDRICH-FLUKA |
| 2-Chloro-cis,cis-muconate | C6H5ClO4 | 1119 | 1255,853 | 9725 | As described by Schmidt and Knackmuss, *Appl Microbiol Biotechnol* 20, 351 (1984) |
| Cytosine | C4H5N3O | 1120 | 1174,401 | 9070 | SIGMA-ALDRICH-FLUKA |
| Coronafacic acid | C12H16O3 | 1121 | 1287,554 | 10042 | As described by Nara *et al.*, *Tetrahedron*, 53, 9509 (1997) |
| Crotono-betaine | C8H16NO | 1122 | 1221,518 | 9382 | As described by Löster and Seim, *Journal of Labelled Compounds and Radiopharmaceuticals* 38, 179 (1998) |
| 3,5-Dichlorocatechol | C6H4Cl2O2 | 1123 | 1242,300 | 9750 | SIGMA-ALDRICH-FLUKA |
| Coronatine | C18H25NO4 | 1124 | 1398,697 | 11154 | SIGMA-ALDRICH-FLUKA |
| p-Cumic aldehyde | C10H12O | 1125 | 1227,501 | 9441 | Provided by Nanjing Rich Native & Animal Products Co., Ltd. |
| Cumene | C9H12 | 1126 | 1199,491 | 9161 | SIGMA-ALDRICH-FLUKA |

| | | | | | |
|---|---|---|---|---|---|
| p-Cumic alcohol | C10H14O | 1127 | 1229,517 | 9462 | SIGMA-ALDRICH-FLUKA |
| N6-(1,2-Dicarboxyethyl)-AMP | C14H18N5O11P | 1128 | 1526,596 | 12833 | HPLC purification from *Pseudomonas putida* KT2440 |
| Cyanobenzene | C7H5N | 1129 | 1309,326 | 8991 | HPLC purification from *Pseudomonas putida* KT2440 |
| Cyclobutane | C4H8 | 1130 | 1135,404 | 8520 | SIGMA-ALDRICH-FLUKA |
| Cycloheptane | C7H14 | 1131 | 1177,485 | 8941 | SIGMA-ALDRICH-FLUKA |
| Cycloheptadecane | C17H34 | 1132 | 1317,754 | 10344 | SIGMA-ALDRICH-FLUKA |
| Cyclohexanol | C6H12O | 1133 | 1179,457 | 8961 | SIGMA-ALDRICH-FLUKA |
| Cyclohexylamine | C6H13N | 1134 | 1178,473 | 8951 | SIGMA-ALDRICH-FLUKA |
| Cyclohexane | C6H12 | 1135 | 1163,458 | 8801 | SIGMA-ALDRICH-FLUKA |
| Cyclohexadecane | C16H32 | 1136 | 1303,727 | 10204 | SIGMA-ALDRICH-FLUKA |
| Cyclohexene oxide | C6H10O | 1137 | 1177,441 | 8941 | SIGMA-ALDRICH-FLUKA |
| Cyclononane | C9H18 | 1138 | 1205,539 | 9222 | SIGMA-ALDRICH-FLUKA |
| Cyclopentane | C5H10 | 1139 | 1149,431 | 8661 | SIGMA-ALDRICH-FLUKA |
| Urea | CON2H4 | 1140 | 1208,415 | 7510 | SIGMA-ALDRICH-FLUKA |
| Cyclopropane | C3H6 | 1141 | 1121,377 | 8380 | SIGMA-ALDRICH-FLUKA |
| dADP | C10H15N5O9P2 | 1142 | 1474,503 | 12072 | SIGMA-ALDRICH-FLUKA |
| D-Cycloserine | C3H6N2O2 | 1143 | 1165,389 | 8980 | SIGMA-ALDRICH-FLUKA |
| Cycloundecane | C11H22 | 1144 | 1233,592 | 9502 | SIGMA-ALDRICH-FLUKA |
| L-Cysteine | C3H7NO2S | 1145 | 1214,478 | 9171 | SIGMA-ALDRICH-FLUKA |
| trans-Cyclohexane-1,2-diol | C6H12O2 | 1146 | 1195,457 | 9121 | SIGMA-ALDRICH-FLUKA |
| Decanoic acid | C10H20O2 | 1147 | 1251,565 | 9682 | SIGMA-ALDRICH-FLUKA |
| Cyclopentanone | C5H8O | 1148 | 1163,414 | 8800 | SIGMA-ALDRICH-FLUKA |
| Caproyldihydroxyacetoneposphate | C4H6O7P(C8H16O2) | 1149 | 1420,571 | 11062 | HPLC purification from *Pseudomonas putida* KT2440 |
| L-Cystine | C6H12N2O4S2 | 1150 | 1333,615 | 10363 | SIGMA-ALDRICH-FLUKA |

| | | | | | |
|---|---|---|---|---|---|
| Acetylcysteine | C5H9NO3S | 1151 | 1256,515 | 9591 | SIGMA-ALDRICH-FLUKA |
| Cysteine p-nitroaniline | C9H14O4SN | 1152 | 1295,572 | 6730 | SIGMA-ALDRICH-FLUKA |
| Cystinyl p-nitroalinine | C12H18O6N3S2 | 1153 | 1427,709 | 7850 | SIGMA-ALDRICH-FLUKA |
| Methyl 2-phenylacetate | C9H10O2 | 1154 | 1229,474 | 9461 | SIGMA-ALDRICH-FLUKA |
| Cytidine | C9H13N3O5 | 1155 | 1322,516 | 10392 | SIGMA-ALDRICH-FLUKA |
| 2,4-Dihydrofuran | C4H6O | 1156 | 1149,387 | 8660 | SIGMA-ALDRICH-FLUKA |
| Diethyl 2-methyl-3-oxosuccinate | C9H14O5 | 1157 | 1281,504 | 9982 | As described in Organic Syntheses, Coll. Vol. 2, p. 272, 1943 |
| D-4-Hydroxyphenyl-glycine methyl ester | C9H11NO3 | 1158 | 1260,488 | 9771 | As described by Crosby, Tetrahedron 47, 4789 (1991) |
| L-Cystathionine | C7H14N2O4S | 1159 | 1315,582 | 10182 | SIGMA-ALDRICH-FLUKA |
| Deoxyadenosine | C10H13N5O3 | 1160 | 1314,543 | 10472 | SIGMA-ALDRICH-FLUKA |
| D-Galactono-1,5-lactone | C6H10O6 | 1161 | 1241,439 | 9741 | As described by Namme *et al., Tetrahedron* 62, 9183 (2006) |
| 6,7-Dimethyl-8-(1-D-ribityl)lumazine | C13H18N4O6 | 1162 | 1405,606 | 11223 | SIGMA-ALDRICH-FLUKA |
| Deoxy-5-methylcytidylate | C10H16N3O7P | 1163 | 1400,524 | 11172 | HPLC purification from *Pseudomonas putida* KT2440 |
| Cyclopenta[b]pyridine | C8H9N | 1164 | 1198,463 | 9151 | As described by Ryabov *et al., J. Chem. Soc Dalton Trans* 2995 (2002) |
| D-Aldonolactone | C6H10O6 | 1165 | 1241,439 | 9741 | Provided by Made-in-Cina.com |
| Octadecanoylglycerone phosphate | C4H6O7P(C18H36O2) | 1166 | 1560,840 | 12775 | HPLC purification from *Saccharomyces cerivisiae* |
| D-Arabinitol | C5H12O5 | 1167 | 1215,444 | 9481 | SIGMA-ALDRICH-FLUKA |
| 1,3-Diaminopropane | C3H10N2 | 1168 | 1153,422 | 8701 | SIGMA-ALDRICH-FLUKA |
| 7,8-Diaminononanoate | C9H20N2 | 1169 | 1251,567 | 9842 | HPLC purification from *Saccharomyces cerivisiae* |

| | | | | | |
|---|---|---|---|---|---|
| | O2 | | | | |
| 3,4-Dihydroxy-2-butanone 4-phosphate | C4H9O6P | 1170 | 1247,383 | 9800 | HPLC purification from *Saccharomyces cerivisiae* |
| D-Arabonate | C5H10O6 | 1171 | 1259,454 | 9621 | SIGMA-ALDRICH-FLUKA |
| 2-Ethyltoluene | C9H12 | 1172 | 1199,491 | 9161 | SIGMA-ALDRICH-FLUKA |
| 2'-Deoxy-5-hydroxymethylcytidine-5'-diphosphate | C10H17N3O11P2 | 1173 | 1480,504 | 12132 | HPLC purification from *Saccharomyces cerivisiae* |
| D-Arabitol | C5H12O5 | 1174 | 1229,471 | 9481 | SIGMA-ALDRICH-FLUKA |
| Methyl decanoate | C11H22O2 | 1175 | 1265,591 | 9822 | SIGMA-ALDRICH-FLUKA |
| trans-Dec-2-Enoyl-CoA | C31H52N7O17P3S | 1176 | 1999,069 | 17159 | HPLC purification from *Saccharomyces cerivisiae* |
| beta-Alanyl-L-lysine | C9H19N3O3 | 1177 | 1310,592 | 10132 | SIGMA-ALDRICH-FLUKA |
| Decanoyl-CoA | C37H66N7O17P3S | 1178 | 2085,246 | 18021 | SIGMA-ALDRICH-FLUKA |
| D-Galacturonate 1-phosphate | C6H11O10P | 1179 | 1351,445 | 10391 | HPLC purification from *Arabidopsis thaliana* |
| N6-(L-1,3-Dicarboxypropyl)-L-lysine | C11H20N2O6 | 1180 | 1369,612 | 10723 | HPLC purification from *Saccharomyces cerivisiae* |
| 2'-Deoxy-5-hydroxymethylcytidine-5'-triphosphate | C10H18N3O14P3 | 1181 | 1560,483 | 12932 | HPLC purification from *Saccharomyces cerivisiae* |
| dCMP | C9H14N3O7P | 1182 | 1370,497 | 10722 | SIGMA-ALDRICH-FLUKA |
| dCDP | C9H15N3O10P2 | 1183 | 1450,477 | 11212 | SIGMA-ALDRICH-FLUKA |
| 3-Hydroxy-4-trimethylammoniobutanoate | C7H16NO3 | 1184 | 1241,506 | 9582 | HPLC purification from *Saccharomyces cerivisiae* |
| Dihydrothymine | C5H8N2O2 | 1185 | 1207,427 | 9241 | As described by Scholhof *et al.*, *Nucleic Acids Res.* 16, 319 (1988) |
| dCTP | C9H16N3O13P3 | 1186 | 1530,457 | 12632 | SIGMA-ALDRICH-FLUKA |
| O-Butanoylcarnitine | C11H21N | 1187 | 1310,589 | 10273 | SIGMA-ALDRICH-FLUKA |

| | | | | | |
|---|---|---|---|---|---|
| | O4 | | | | |
| 3',4',5,7-Tetrahydroxy-3-methoxyflavone | C16H12O7 | 1188 | 1395,564 | 11122 | Provided by Xiamen Bioshining Biotechnology Co.,ltd (http://www.bioshining.net) |
| trans-Dodec-2-Enoyl-CoA | C33H56N7O17P3S | 1189 | 2027,122 | 17440 | HPLC purification from *Saccharomyces cerivisiae* |
| Methyl dodecanoate | C13H26O2 | 1190 | 1293,645 | 10103 | SIGMA-ALDRICH-FLUKA |
| Dodecanoate | C12H24O2 | 1191 | 1279,618 | 9963 | SIGMA-ALDRICH-FLUKA |
| Dodecanoyl-CoA | C33H58N7O17P3S | 1192 | 2029,138 | 17460 | SIGMA-ALDRICH-FLUKA |
| 1-Tetradecanoyl-sn-glycerol 3-phosphate | C4H8O7P(C14H28O2) | 1193 | 1506,749 | 11924 | HPLC purification from *Saccharomyces cerivisiae* |
| Dodecanoyldolichol | C21H35O2(C5H8)5C12H24O2 | 1194 | 1939,718 | 16162 | HPLC purification from *Saccharomyces cerivisiae* |
| Decanoyldihydroxyacetonephosphate | C4H6O7P(C12H24O2) | 1195 | 1476,679 | 11933 | HPLC purification from *Saccharomyces cerivisiae* |
| Decanoyldolichol | C21H35O2(C5H8)5C10H20O2 | 1196 | 1911,664 | 20449 | HPLC purification from *Saccharomyces cerivisiae* |
| Decane | C10H22 | 1197 | 1221,581 | 9382 | SIGMA-ALDRICH-FLUKA |
| Decanal | C10H20O | 1198 | 1235,565 | 9522 | SIGMA-ALDRICH-FLUKA |
| Octadecanal | C18H36O | 1199 | 1347,780 | 10645 | SIGMA-ALDRICH-FLUKA |
| 2'-Deoxy-5-hydroxymethylcytidine 5'-phosphate | C10H16N3O8P | 1200 | 1400,524 | 11332 | HPLC purification from *Saccharomyces cerivisiae* |
| 2-Deoxy-D-ribose | C5H12O10P2 | 1201 | 1357,389 | 10901 | SIGMA-ALDRICH-FLUKA |
| Dihydrouracil | C4H6N2 | 1202 | 1193,400 | 9100 | SIGMA-ALDRICH-FLUKA |

| | | | | | |
|---|---|---|---|---|---|
| | O2 | | | | |
| (S)-2-Hydroxyglutarate | C5H8O5 | 1203 | 1241,439 | 9441 | SIGMA-ALDRICH-FLUKA |
| D-Fucose | C6H12O5 | 1204 | 1227,455 | 9601 | SIGMA-ALDRICH-FLUKA |
| D-Glucono-1,5-lactone | C6H10O6 | 1205 | 1241,439 | 9741 | SIGMA-ALDRICH-FLUKA |
| dGDP | C10H15N5O10P2 | 1206 | 1490,502 | 12232 | SIGMA-ALDRICH-FLUKA |
| D-Glucarate | C6H10O8 | 1207 | 1303,464 | 10061 | SIGMA-ALDRICH-FLUKA |
| dGMP | C10H14N5O7P | 1208 | 1410,522 | 11432 | SIGMA-ALDRICH-FLUKA |
| 4-Bromophenol-2,3-epoxide | C6H5BrO2 | 1209 | 1268,306 | 9050 | Using monooxygenase from *Pseudomonas mendocina* (Fishman et al. Biotechnol Bioeng 87, 779-790 (2004) |
| dGTP | C10H16N5O13P3 | 1210 | 1570,482 | 13032 | SIGMA-ALDRICH-FLUKA |
| 1-Hydroxy-2-naphthoate | C11H8O3 | 1211 | 1267,479 | 9841 | Provided by Lucky (Shenyang) Technological Industries Co., Ltd. |
| Dihydroxyacetone | C3H6O2 | 1212 | 1151,403 | 8860 | SIGMA-ALDRICH-FLUKA |
| Dihydroxyacetone phosphate | C3H7O6P | 1213 | 1247,383 | 9660 | SIGMA-ALDRICH-FLUKA |
| (1S,3R,4S)-3,4-Dihydroxycyclohexane-1-carboxylate | C7H12O4 | 1214 | 1223,467 | 9561 | HPLC purification from *Arabidopsis thaliana* |
| 7,8-Dihydrofolate | C19H21N7O6 | 1215 | 1506,717 | 12394 | SIGMA-ALDRICH-FLUKA |
| trans-2,3-Dihydroxycinnamate | C9H8O4 | 1216 | 1259,456 | 9761 | SIGMA-ALDRICH-FLUKA |
| 1,4-Dihydroxy-2-naphthoate | C11H8O4 | 1217 | 1283,478 | 10001 | HPLC purification from *Arabidopsis thaliana* |
| 2-Amino-4-hydroxy-6-(D-erythro-1,2,3-trihydroxypropyl)-7,8-dihydropteridine | C9H13N5O4 | 1218 | 1332,558 | 10512 | HPLC purification from *Pseudomona putida* KT2440 |
| (S)-Dihydroorotate | C5H6N2O4 | 1219 | 1251,436 | 9541 | SIGMA-ALDRICH-FLUKA |
| Dihydroneopterin phosphate | C9H14N5 | 1220 | 1412,538 | 11312 | SIGMA-ALDRICH-FLUKA |

| | | | | | |
|---|---|---|---|---|---|
| | O7P | | | | |
| 3-(2,3-Dihydroxyphenyl)propanoate; 2,3-Dihydroxyphenylpropanoate | C9H10O4 | 1221 | 1261,472 | 9781 | Provided by Hengda Inc. |
| Dihydropteroate | C14H14N6O3 | 1222 | 1377,601 | 11103 | HPLC purification from *Arabidopsis thaliana* |
| (S)-4,5-dihydroxypentan-2,3-dione | C5H8O4 | 1223 | 1209,440 | 9281 | As described by Ho *et al., Journal of Pharmaceutical Sciences* 63, 1474 (2006) |
| 3-Dehydrosphinganine | C18H37NO2 | 1224 | 1378,795 | 10955 | HPLC purification from Arabidopsis thaliana |
| 3',5-Dihydroxy-3,4',7-trimethoxyflavone | C18H16O7 | 1225 | 1423,618 | 11403 | As described by Smith *et al., Acta Cryst.*E57, o973 (2001) |
| (5R)-3,4-Dihydroxy-5-(hydroxymethyl)furan-2(5H)-one | C5H6O5 | 1226 | 1209,396 | 9420 | Provided by Zerenex Molecular Limited |
| 2,4-Dibromophenol | C6H4Br2O | 1227 | 1315,203 | 9680 | SIGMA-ALDRICH-FLUKA |
| 2,6-Dibromophenol | C6H4Br2O | 1228 | 1315,203 | 8880 | SIGMA-ALDRICH-FLUKA |
| Deoxyguanosine | C10H13N5O4 | 1229 | 1330,542 | 10632 | SIGMA-ALDRICH-FLUKA |
| Dibenzofuran | C12H8O | 1230 | 1247,491 | 9641 | SIGMA-ALDRICH-FLUKA |
| Dibenzo-p-dioxin | C12H8O2 | 1231 | 1263,491 | 9801 | SIGMA-ALDRICH-FLUKA |
| dTMP | C10H15N2O8P | 1232 | 1401,508 | 11182 | SIGMA-ALDRICH-FLUKA |
| 4-Chlorophenylacetate | C8H7ClO2 | 1233 | 1249,892 | 9665 | SIGMA-ALDRICH-FLUKA |
| D-Iditol | C6H14O6 | 1234 | 1259,498 | 9781 | SIGMA-ALDRICH-FLUKA |
| Digallate | C14H10O9 | 1235 | 1385,525 | 11182 | SIGMA-ALDRICH-FLUKA |
| Dihydrocoumarin | C9H8O2 | 1236 | 1227,458 | 9441 | SIGMA-ALDRICH-FLUKA |
| 9-Hydroxynonanoate | C9H18O3 | 1237 | 1253,537 | 9692 | By hydrolysis of 9-hydroxy-nonanoic acid methyl ester (Synthetic Communications, 20, p. 907, 1990) |
| 10-Hydroxydecanoate | C10H20O3 | 1238 | 1267,564 | 9661 | SIGMA-ALDRICH-FLUKA |
| 2,4-Dinitrotoluene | C7H6N2 | 1239 | 1261,431 | 9501 | SIGMA-ALDRICH-FLUKA |

| | | | | | |
|---|---|---|---|---|---|
| | O4 | | | | |
| Deoxyinosine | C10H12N4O4 | 1240 | 1315,527 | 10482 | SIGMA-ALDRICH-FLUKA |
| Dioxybenzone | C14H12O4 | 1241 | 1323,543 | 10402 | SIGMA-ALDRICH-FLUKA |
| 2,3-Bis(3-Hydroxytetradecanoyl)-D-glucosaminyl-1,6-beta-D-2,3-bis(3-hydroxytetradecanoyl)-beta-D-glucosaminyl 1-phosphate | C68H129N2O20P | 1242 | 2405,044 | 21220 | HPLC purification from *Saccharomyces cerivisiae* |
| 2,3-Diketo-5-methylthiopentyl-1-phosphate | C6H11O6PS | 1243 | 1321,480 | 10382 | HPLC purification from *Saccharomyces cerivisiae* |
| 4-Carboxymethylenebut-2-en-4-olide | C6H4O4 | 1244 | 1219,392 | 9360 | HPLC purification from *Saccharomyces cerivisiae* |
| cis-2-Methyl-5-isopropylhexa-2,5-dienoic acid | C10H16O2 | 1245 | 1247,533 | 9642 | HPLC purification from *Saccharomyces cerivisiae* |
| cis-2-Methyl-5-isopropylhexa-2,5-dienoyl-CoA | C31H50N7O17P3S | 1246 | 1997,053 | 17139 | HPLC purification from *Saccharomyces cerivisiae* |
| D-Mannuronate | C6H10O7 | 1247 | 1271,465 | 9901 | As described by Roussel et al. (2005) European Journal of Organic Chemistry 2005, 3085-3094 |
| D-Lyxose | C5H10O5 | 1248 | 1213,428 | 9461 | SIGMA-ALDRICH-FLUKA |
| Dihydrolipoamide | C8H17NOS2 | 1249 | 1286,646 | 10033 | HPLC purification from *Arabidopsis thaliana* |
| D-Mannonate | C6H12O7 | 1250 | 1259,454 | 9921 | SIGMA-ALDRICH-FLUKA |
| Dimethoxybenzidine o-dianisidine | C14H16N2O2 | 1251 | 1307,590 | 6951 | Provided by Acros Organics N.V. |
| 5,6-Dimethylbenzimidazole | C9H10N2 | 1252 | 1225,488 | 9421 | As described by Renz *et al., Z Naturforsch* 52, 287 (1997) |
| Dimethylglycine | C4H9NO2 | 1253 | 1182,418 | 8991 | SIGMA-ALDRICH-FLUKA |
| Nalpha,Nalpha-Dimethyl-L-histidine | C8H13N3O2 | 1254 | 1262,507 | 9792 | SIGMA-ALDRICH-FLUKA |
| DNA substrate lambda DNA + Sau3A | # | 1255 | # | # | Fermentas |
| DNA resolvase substrate 5- | # | 1256 | # | # | Sigma Genosys |

| | | | | | |
|---|---|---|---|---|---|
| GACGCTGCCGAATTCTGGCTTGC TAGGACATCTTTGCCCACGTTGA CCC-3 | | | | | |
| 4alpha-Methylzymosterol | C29H46 O | 1257 | 1489,980 | 11947 | Prepared as described by A. V. Baranovskii *et al.*, *Bioorg Khim* 28, 277 (2002) |
| 6,7-Dimethyl-8-(1-D-ribityl)lumazine | C13H18N 4O6 | 1258 | 1389,607 | 11223 | HPLC purification from *Saccharomyces cerivisiae* |
| Dimethylallyl diphosphate | C5H12O 7P2 | 1259 | 1325,391 | 9801 | Purified as described by Silver and Fall, *Plant Physiology* 97, 1588 (1991) |
| DNA substrate lambda DNA + HindIII | # | 1260 | # | # | In house digestion |
| DNA substrate TAAGCTCCGGATTGTCCGGGAG GTAAAGCCCTGAT | # | 1261 | # | # | Sigma Genosys |
| DNA substrate CACAGGAAGCTCTACAGGTACTC CG | # | 1262 | # | # | Sigma Genosys |
| DNA substrate TGGTCATCAGGGCTTTACCTCCC GGACAATCCGGAGCTTACGGAG TACCTGTAGAGCTTCCTGTGCAA GC | # | 1263 | # | # | Sigma Genosys |
| DNA substrate SspI X174 DNA | # | 1264 | # | # | Sigma Genosys |
| DNA Helicase substrate dsDNA: 5-GCACTGGCCGTCGTTTTACC-3 | # | 1265 | # | # | Sigma Genosys |
| DNA Gyrase substrate relaxed pBR322 | # | 1266 | # | # | Sigma Genosys |
| DNA Topoisomerase substrate 40-base single-stranded oligodeoxyribonucleotides | # | 1267 | # | # | Sigma Genosys |
| Deamino-NAD+ | C21H27N 6O15P2 | 1268 | 1728,721 | 10061 | SIGMA-ALDRICH-FLUKA |
| n-Dodecyl -D-maltoside | C24H46 O11 | 1269 | 1589,919 | 10581 | SIGMA-ALDRICH-FLUKA |
| Docosan-1-ol | C22H46 | 1270 | 1405,903 | 7410 | SIGMA-ALDRICH-FLUKA |

| | | | | | |
|---|---|---|---|---|---|
| | O | | | | |
| Dodecane | C12H26 | 1271 | 1249,635 | 6755 | SIGMA-ALDRICH-FLUKA |
| Dodecanoyldihydoxyacetonephosphate | C3H6O6PR | 1272 | 451,392 | 9891 | HPLC purification from *Saccharomyces cerivisiae* |
| Dolichol | C20H36O(C5H8)5 | 1273 | 1712,394 | 6735 | As described by Bizzarri *et al.*, *Biogerontology* 4, 353 (2003) |
| Dolichyl D-mannosyl phosphate | C26H47O9P(C5H8)5 | 1274 | 1954,517 | 7115 | HPLC purification from *Arabidopsis thaliana* |
| Dolichyl phosphate | C20H37O4P(C5H8)5 | 1275 | 1792,374 | 6668 | HPLC purification from *Arabidopsis thaliana* |
| Dehydrodolichol diphosphate | C20H36O7P2(C5H8)5 | 1276 | 1870,339 | 7988 | HPLC purification from *Arabidopsis thaliana* |
| O-Decanoyl-L-carnitine | C17H33NO4 | 1277 | 1394,750 | 6854 | SIGMA-ALDRICH-FLUKA |
| O-Propanoylcarnitine | C10H19NO4 | 1278 | 1296,562 | 6407 | SIGMA-ALDRICH-FLUKA |
| Dephospho-CoA | C21H35N7O13P2S | 1279 | 1750,854 | 6307 | SIGMA-ALDRICH-FLUKA |
| Dethiobiotin | C10H18N2O3 | 1280 | 1293,561 | 6668 | HPLC purification from a biotin auxotroph of Escherichia coli K-12 (Eisenberg and Krell (1969) J Biol Chem 244, 5503-5509 |
| 2-[3-Carboxy-3-(methylammonio)propyl]-L-histidine | C11H19N4O4 | 1281 | 1350,593 | 7068 | HPLC purification from *Saccharomyces cerivisiae* |
| Diethyl (2R,3R)-2-methyl-3-hydroxysuccinate | C9H16O5 | 1282 | 1283,520 | 6200 | As described by Lee and Kim, *Bioorganic & Medicinal Chemistry* 10, 913 (2002) |
| dTDP | C10H16N2O11P2 | 1283 | 1481,488 | 6674 | SIGMA-ALDRICH-FLUKA |
| D-Ribitol 5-phosphate | C5H13O8P | 1284 | 1295,425 | 8956 | SIGMA-ALDRICH-FLUKA |
| D-Ribonate | C5H10O6 | 1285 | 1228,420 | 8949 | SIGMA-ALDRICH-FLUKA |
| D-Ribulose | C5H10O | 1286 | 1213,428 | 8949 | SIGMA-ALDRICH-FLUKA |

| | | | | | |
|---|---|---|---|---|---|
| | 5 | | | | |
| Diethyl (2S,3R)-2-methyl-3-hydroxysuccinate | C9H16O5 | 1287 | 1283,520 | 8949 | As described by Lee and Kim, *Bioorganic & Medicinal Chemistry* 10, 913 (2002) |
| Phenanthrene-4,5-dicarboxylate | C16H8O4 | 1288 | 1343,533 | 8962 | As described by Longridge *et al., Acta Cryst* C54, IUC9800028 (1998) |
| Deoxyribose | C5H10O4 | 1289 | 1213,428 | 8962 | SIGMA-ALDRICH-FLUKA |
| 2-Acetamido-2,6-dideoxy-D-glucose | C8H15NO5 | 1290 | 1268,508 | 9036 | HPLC purification from *Pseudomonas aeruginosa* |
| dTDP-4-Amino-4,6-dideoxy-D-glucose | C16H27N3O14P2 | 1291 | 1610,647 | 9069 | HPLC purification from *Saccharomyces cerivisiae* |
| dTDP-4-Dehydro-6-deoxy-D-glucose | C16H24N2O15P2 | 1292 | 1625,615 | 9069 | HPLC purification from *Saccharomyces cerivisiae* |
| dTDP-4-Dehydro-6-deoxy-L-mannose | C16H24N2O15P2 | 1293 | 1609,615 | 8962 | HPLC purification from *Saccharomyces cerivisiae* |
| dTDP-4-oxo-6-deoxy-alpha-D-glucose | C16H24N2O15P2 | 1294 | 1609,615 | 8962 | HPLC purification from *Saccharomyces cerivisiae* |
| dTDP-6-deoxy-L-mannose | C16H26N2O15P2 | 1295 | 1611,631 | 6321 | As described by Bird and Jones, *Canadian Journal of Chemistry* 41, 1877 (1963) |
| dTDP-6-deoxy-L-talose | C16H26N2O15P2 | 1296 | 1611,631 | 6882 | HPLC purification from *Saccharomyces cerivisiae* |
| dTDP-alpha-D-desosamine | C18H31N3O13P2 | 1297 | 1638,701 | 6307 | HPLC purification from *Saccharomyces cerivisiae* |
| dTDP-glucose | C16H26N2O16P2 | 1298 | 1643,630 | 9162 | SIGMA-ALDRICH-FLUKA |
| dTDP-D-galactose | C16H26N2O16P2 | 1299 | 1643,630 | 6200 | SIGMA-ALDRICH-FLUKA |
| dTDP-D-fucose | C16H26N2O15P2 | 1300 | 1611,631 | 8675 | SIGMA-ALDRICH-FLUKA |
| dTDP-L-rhamnose | C16H26N2O15P2 | 1301 | 1611,631 | 6234 | SIGMA-ALDRICH-FLUKA |
| Dibenzothiophene | C12H8S | 1302 | 1263,552 | 8203 | SIGMA-ALDRICH-FLUKA |
| 4,5-Dihydroxy-4,5-dihydropyrene | C16H12O2 | 1303 | 1315,567 | 6174 | Purified from *Mycobacterium flavescens* |
| D-Xylulose | C5H10O | 1304 | 1213,428 | 9699 | SIGMA-ALDRICH-FLUKA |

| | | | | | |
|---|---|---|---|---|---|
| | 5 | | | | |
| dTDP-D-galacturonate | C16H24N2O17P2 | 1305 | 1641,614 | 6520 | HPLC purification from *Saccharomyces cerivisiae* |
| 1-Deoxy-D-xylulose | C5H10O4 | 1306 | 1197,429 | 6521 | Provided by Echelon (www.echelon-inc.com) |
| Thiamin triphosphate | C12H20N4O10P3S | 1307 | 1584,590 | 6868 | SIGMA-ALDRICH-FLUKA |
| Tropinone | C8H13NO | 1308 | 1218,494 | 6361 | SIGMA-ALDRICH-FLUKA |
| 1,2-Dioctanoyl-sn-glycerol | C5H6O5(C8H16O2)2 | 1309 | 1513,824 | 6708 | Glycerol + ethyloctanoate+ CalA LIPASE + EL1 LIPASE METAGENOMIC → 1,2-Dioctanoyl-sn-glycerol |
| Quinazoline | C8H6N2 | 1310 | 1209,445 | 6513 | As described by Chilin et al., *Tetrahedron Letters* 48, 3229 (2007) |
| 1,2-Dihexadecanoyl-sn-glycerol | C5H6O5(C16H32O2)2 | 1311 | 1738,254 | 6394 | Glycerol + ethylhexadecanoate+ CalA LIPASE + EL1 LIPASE METAGENOMIC → 1,2-Dihexadecanoyl-sn-glycerol |
| 1-Deoxy-D-xylulose 5-phosphate | C5H11O7P | 1312 | 1277,409 | 5693 | As described by Thiel and Adam, *Tetrahedron Letters*, 40, 5307 (1999) |
| D-Xylonate | C5H10O6 | 1313 | 1259,454 | 5306 | SIGMA-ALDRICH-FLUKA |
| 4,5-Dihydroxypyrene | C16H10O2 | 1314 | 1313,551 | 7510 | Purified from *Mycobacterium flavescens* |
| Ethyl 3-oxohexanoate | C8H14O3 | 1315 | 1237,494 | 6894 | Provided by Taicang Jinhai Medicine Chemical Plant |
| 3,4-Dihydroxyphenanthrene | C14H10O2 | 1316 | 1289,529 | 6287 | As described by Horaguchi et al., *Bulletin of the Chemical Society of Japan* 47, 485 (1974) |
| 3,6-Dichloro-cis-1,2-dihydroxycyclohexa-3,5-diene | C6H6Cl2O2 | 1317 | 1244,315 | 7242 | HPLC purification from *Pseudomona putida* KT2440 |
| L-Erythro-4-Hydroxyglutamate | C5H9NO5 | 1318 | 1256,454 | 6777 | HPLC purification from *Saccharomyces cerivisiae* |
| 1,2-Epoxypropane | C3H6O | 1319 | 1137,376 | 6522 | SIGMA-ALDRICH-FLUKA |
| Docosapentaenoic acid methyl ester | C23H36O2 | 1320 | 1423,835 | 8551 | SIGMA-ALDRICH-FLUKA |
| Docosapentaenoic acid | C22H34O2 | 1321 | 1409,808 | 8951 | SIGMA-ALDRICH-FLUKA |

| | | | | | |
|---|---|---|---|---|---|
| D-erythro-1-(Imidazol-4-yl)glycerol 3-phosphate | C6H11N2O6P | 1322 | 1301,434 | 7964 | HPLC purification from *Saccharomyces cerivisiae* |
| 2-Indanone oxime | C9H9NO | 1323 | 1226,473 | 6374 | Provided by VAJRA MOLECULES LTD |
| (+)-Epicatechin | C15H14O6 | 1324 | 1353,570 | 6428 | SIGMA-ALDRICH-FLUKA |
| 2,3-Dihydroxy-4'-chlorobiphenyl | C12H9ClO2 | 1325 | 1299,952 | 6470 | Purified as described by Massé *et al.*, *Biomedical and Environmental Mass Spectrometry* 18, 27 (2005) |
| Epimelibiose | C12H22O11 | 1326 | 1405,597 | 6374 | SIGMA-ALDRICH-FLUKA |
| Epichlorohydrin | C3H5ClO | 1327 | 1171,822 | 6107 | SIGMA-ALDRICH-FLUKA |
| 1,2-Didecanoyl-sn-glycerol | C5H6O5(C12H24O2)2 | 1328 | 1626,039 | 7310 | Glycerol + ethyldecanoate+ CalA LIPASE + EL1 LIPASE METAGENOMIC → 1,2-Didecanoyl-sn-glycerol |
| Episterol | C28H46O | 1329 | 1477,969 | 6107 | As described by Takatsuto *et al.*, *Nihon yukagaku kaishi* 48, 37 (1999) |
| Ethyl (R)-3-Hydroxyhexanoate | C8H16O3 | 1330 | 1239,510 | 6120 | Provided by ISCA Technologies |
| trans-2-Methyl-5-isopropylhexa-2,5-dienoic acid | C10H16O2 | 1331 | 1247,533 | 6374 | As described by Linares *et al.*, *Bioresource Technology* 99, 4590 (2008) |
| trans-2-Chlorodienelactone | C6H3ClO4 | 1332 | 1267,864 | 6441 | HPLC purification from *Pseudomonas* sp. |
| 1-Formyl-2-indanone | C10H8O2 | 1333 | 1239,469 | 6307 | As described by Park *et al.*, *Bull Korean Chem Soc* 25, 927 (2004) |
| Erythrose | C4H8O4 | 1334 | 1183,402 | 6081 | SIGMA-ALDRICH-FLUKA |
| (R)-2-Hydroxystearate | C18H36O3 | 1335 | 1379,779 | 6481 | SIGMA-ALDRICH-FLUKA |
| D-Erythrulose | C4H8O4 | 1336 | 1183,402 | 6321 | SIGMA-ALDRICH-FLUKA |
| Erythritol | C4H10O4 | 1337 | 1185,418 | 5713 | SIGMA-ALDRICH-FLUKA |
| Ethyl (S)-3-Hydroxyhexanoate | C8H16O3 | 1338 | 1239,510 | 6002 | Provided by ISCA Technologies |
| N-Isopropylammelide | C6H10N4O2 | 1339 | 1249,468 | 6361 | HPLC purification from *Pseudomonas* sp. |
| Ethyl benzoate | C9H10O2 | 1340 | 1229,474 | 5306 | SIGMA-ALDRICH-FLUKA |

| | | | | | |
|---|---|---|---|---|---|
| Ethyl butyrate | C6H12O2 | 1341 | 1195,457 | 6014 | SIGMA-ALDRICH-FLUKA |
| Ethyl cinnamate | C11H12O2 | 1342 | 1255,512 | 6094 | SIGMA-ALDRICH-FLUKA |
| Ethylguaiacol | C9H12O2 | 1343 | 1231,490 | 5987 | SIGMA-ALDRICH-FLUKA |
| Ethanolamine | C2H7NO | 1344 | 1124,381 | 6227 | SIGMA-ALDRICH-FLUKA |
| Ethylhexyl palmitate | C24H48O2 | 1345 | 1447,941 | 6414 | SIGMA-ALDRICH-FLUKA |
| Ethyl heptanoate | C9H18O2 | 1346 | 1237,538 | 6414 | SIGMA-ALDRICH-FLUKA |
| Ethylphenyl sulfide | C8H12OS | 1347 | 1235,539 | 9760 | SIGMA-ALDRICH-FLUKA |
| Ethylbenzene | C8H10 | 1348 | 1185,464 | 5306 | SIGMA-ALDRICH-FLUKA |
| Ethyl lactate | C5H10O3 | 1349 | 1197,429 | 7574 | SIGMA-ALDRICH-FLUKA |
| Ethynylbenzene | C8H6 | 1350 | 1181,432 | 10544 | SIGMA-ALDRICH-FLUKA |
| o-Hydroxybenzoic acid | C7H6O3 | 1351 | 1217,419 | 10558 | SIGMA-ALDRICH-FLUKA |
| Ethyl paraben | C9H10O3 | 1352 | 1245,473 | 7408 | SIGMA-ALDRICH-FLUKA |
| Ethyl salicylate | C9H10O3 | 1353 | 1245,473 | 6407 | SIGMA-ALDRICH-FLUKA |
| D-Fructose 1-phosphate | C6H13O9P | 1354 | 1323,435 | 6789 | SIGMA-ALDRICH-FLUKA |
| Formaldehyde | CH2O2 | 1355 | | 6951 | SIGMA-ALDRICH-FLUKA |
| D-Fructose 2,6-bisphosphate | C6H14O12P2 | 1356 | 1403,415 | 6934 | SIGMA-ALDRICH-FLUKA |
| FAD | C27H33N9O15P2 | 1357 | 1848,855 | 6401 | SIGMA-ALDRICH-FLUKA |
| FADH2 | C27H35N9O15P2 | 1358 | 1850,871 | 6923 | SIGMA-ALDRICH-FLUKA |
| 1-Hexanoyl-sn-glycerol 3-phosphate | C4H8O7P(C6H12O2) | 1359 | 1394,534 | 11599 | HPLC purification from *Saccharomyces cerivisiae* |
| N-Formylanthranilate | C8H7NO | 1360 | 1228,446 | 6601 | SIGMA-ALDRICH-FLUKA |

| | | | | | |
|---|---|---|---|---|---|
| | 3 | | | | |
| Farnesol | C15H26O | 1361 | 1301,667 | 6694 | SIGMA-ALDRICH-FLUKA |
| FAXX | # | 1362 | # | # | In house-purification (López-Cortés et al., 2007) |
| L-Fuculose 1-phosphate | C6H13O8P | 1363 | 1307,436 | 6414 | SIGMA-ALDRICH-FLUKA |
| L-Fuculose | C6H12O5 | 1364 | 1227,455 | 6788 | SIGMA-ALDRICH-FLUKA |
| 1-Hexadecanol | C16H34O | 1365 | 1323,758 | 5947 | SIGMA-ALDRICH-FLUKA |
| Feruloyl-CoA | C31H44N7O19P3S | 1366 | 2023,003 | 5987 | Crotonate (Abaco Inc) + CoA + ACETYLTRANSFERASA Arabidopsis thaliana → Feruloyl-CoA |
| Ferulate | C10H10O4 | 1367 | 1273,483 | 7401 | Provided by Apin Chemicals Limited (UK) |
| Methyl ferulate | C11H12O4 | 1368 | 1287,510 | 8349 | Provided by Apin Chemicals Limited (UK) |
| 5-Hydroxyferulate | C10H10O5 | 1369 | 1289,483 | 6895 | Provided by Apin Chemicals Limited (UK) |
| Diphenylenemethane | C13H10 | 1370 | 1245,519 | 5613 | Provided by Beijing Shlht Chemical Technology |
| Flavone | C15H10O2 | 1371 | 1301,540 | 6991 | SIGMA-ALDRICH-FLUKA |
| Fluorobenzene | C6H5F | 1372 | 1175,401 | 6387 | SIGMA-ALDRICH-FLUKA |
| Formiminoglycine | C3H6N2O2 | 1373 | 1165,389 | 6467 | As described by Rabinowitz and Pricer, *J Am Chem Soc*, 78, 4176 (1956) |
| N2-Formyl-N1-(5-phospho-D-ribosyl)glycinamide | C8H15N2O9P | 1374 | 1377,486 | 6294 | HPLC purification from *Saccharomyces cerivisiae* |
| FMN | C17H21N4O9P | 1375 | 1535,646 | 7748 | SIGMA-ALDRICH-FLUKA |
| Flavonol | C15H10O3 | 1376 | 1317,539 | 7482 | HPLC purification from *Arabidopsis thaliana* |
| Formate | CH2O2 | 1377 | 1266,255 | 7855 | SIGMA-ALDRICH-FLUKA |
| 2-Hydroxy-6-oxo-6-(4'-chlorophenyl)-hexa-2,4-dienoate | C12H9ClO4 | 1378 | 1331,950 | 6507 | Provided by Alfa Aesar (http://www.alfa.com/alf/laboratory_chemical_suppliers.htm) |
| 2-Dehydro-3-deoxy-D-fuconate | C6H10O5 | 1379 | 1225,439 | 5306 | HPLC purification from *Saccharomyces cerivisiae* |

| | | | | | |
|---|---|---|---|---|---|
| N-Formimidoyl-L-glutamate | C6H10N2O4 | 1380 | 1267,479 | 6601 | HPLC purification from *Saccharomyces cerivisiae* |
| (R)-2,3-Dihydroxy-3-methylpentanoate | C6H12O4 | 1381 | 1227,455 | 6775 | HPLC purification from *Saccharomyces cerivisiae* |
| 5-Formamido-1-(5-phospho-D-ribosyl)imidazole-4-carboxamide | C10H15N4O9P | 1382 | 1429,522 | 6927 | HPLC purification from *Saccharomyces cerivisiae* |
| 4'-O-beta-D-Glucosyl-cis-p-coumarate | C15H18O8 | 1383 | 1389,601 | 6641 | HPLC purification from *Saccharomyces cerivisiae* |
| Farnesyl diphosphate | C15H28O7P2 | 1384 | 1461,628 | 6507 | SIGMA-ALDRICH-FLUKA |
| D-Fructose | C6H12O6 | 1385 | 1243,455 | 6080 | SIGMA-ALDRICH-FLUKA |
| Fructosamine | C6H13NO5 | 1386 | 1242,470 | 8531 | SIGMA-ALDRICH-FLUKA |
| D-Fructuronate | C6H10O7 | 1387 | 1257,438 | 6407 | SIGMA-ALDRICH-FLUKA |
| Farnesal | C15H24O | 1388 | 1298,643 | 7041 | SIGMA-ALDRICH-FLUKA |
| L-Fucose 1-phosphate | C6H13O8P | 1389 | 1307,436 | 5306 | SIGMA-ALDRICH-FLUKA |
| Fumarylacetoacetate | C8H8O6 | 1390 | 1279,444 | 5306 | HPLC purification from *Saccharomyces cerivisiae* |
| D-Fuconate | C6H12O6 | 1391 | 1243,455 | 5306 | SIGMA-ALDRICH-FLUKA |
| Fumarate | C4H4O4 | 1392 | 1195,370 | 5306 | SIGMA-ALDRICH-FLUKA |
| L-Fucose | C6H12O5 | 1393 | 1227,455 | 6453 | SIGMA-ALDRICH-FLUKA |
| 4-Formylsalicylic acid | C8H6O4 | 1394 | 1259,456 | 6440 | HPLC purification from *Saccharomyces cerivisiae* |
| D-Glucose 1-phosphate | C6H13O9P | 1395 | 1323,435 | 7028 | SIGMA-ALDRICH-FLUKA |
| Glutathionyl-OH-1,2-dihydronaphthalene | C20H24N2O7S | 1396 | 1515,776 | 8041 | HPLC purification from *Arabidopsis thaliana* |
| Glutathionyl-1,2-dihydronaphthalene | C20H24NO5S | 1397 | 1469,771 | 6841 | HPLC purification from *Arabidopsis thaliana* |
| D-Glucono-1,5-lactate | C6H10O6 | 1398 | 1257,438 | 9542 | SIGMA-ALDRICH-FLUKA |

| | | | | | |
|---|---|---|---|---|---|
| Furazan-3,4-diol | C2H2N2O3 | 1399 | 1179,374 | 9531 | Provided by Chemical Block Ltd. |
| Glycerol-3-phosphate | C3H9O6P | 1400 | 1235,372 | 7016 | SIGMA-ALDRICH-FLUKA |
| Glyceraldehyde 3-phosphate | C3H7O6P | 1401 | 1233,356 | 5887 | SIGMA-ALDRICH-FLUKA |
| sn-Glycero-3-phosphocholine | C8H21NO6P | 1402 | 1321,530 | 6160 | SIGMA-ALDRICH-FLUKA |
| Glycerophosphoglycerol | C6H15O8P | 1403 | 1309,452 | 6507 | SIGMA-ALDRICH-FLUKA |
| sn-Glycero-3-phosphoethanolamine | C5H14NO6P | 1404 | 1278,441 | 7041 | SIGMA-ALDRICH-FLUKA |
| sn-Glycero-3-phospho-1-inositol | C9H19O11P | 1405 | 1397,515 | 6501 | SIGMA-ALDRICH-FLUKA |
| beta-D-Glucose 6-phosphate | C6H13O9P | 1406 | 1337,462 | 8670 | SIGMA-ALDRICH-FLUKA |
| Hexadecanoate | C16H32O2 | 1407 | 1335,726 | 5306 | SIGMA-ALDRICH-FLUKA |
| 4-Aminobutyraldehyde | C4H9NO | 1408 | 1150,419 | 7589 | As described by Grigorenko *et al., Tetrahedron* 63, 2257 (2007) |
| gamma-Amino-gamma-cyanobutanoate | C5H8N2O2 | 1409 | 1221,454 | 6707 | HPLC purification from *Pseudomonas* sp. |
| D-Galactose | C6H12O6 | 1410 | 1243,455 | 6614 | SIGMA-ALDRICH-FLUKA |
| alpha-D-Galactose 1-phosphate | C6H13O9P | 1411 | 1323,435 | 6534 | SIGMA-ALDRICH-FLUKA |
| D-Galactosamine | C6H13NO5 | 1412 | 1242,470 | 6440 | SIGMA-ALDRICH-FLUKA |
| Galactomannan | C18H32O16 | 1413 | 1567,740 | 6721 | SIGMA-ALDRICH-FLUKA |
| D-Galactosaminoglycan | # | 1414 | # | # | SIGMA-ALDRICH-FLUKA |
| Galactinol | C12H22O11 | 1415 | 1405,597 | 7054 | SIGMA-ALDRICH-FLUKA |
| D-Galactarate | C6H10O8 | 1416 | 1303,464 | 7034 | SIGMA-ALDRICH-FLUKA |
| D-Galactonate | C6H12O | 1417 | 1289,481 | 7034 | SIGMA-ALDRICH-FLUKA |

| | | | | | |
|---|---|---|---|---|---|
| | 7 | | | | |
| 3-O-Methylgallate | C8H8O5 | 1418 | 1263,445 | 6601 | SIGMA-ALDRICH-FLUKA |
| Gallate | C7H6O5 | 1419 | 1233,418 | 6501 | SIGMA-ALDRICH-FLUKA |
| n-Propyl gallate | C10H12 O5 | 1420 | 1291,499 | 11266 | SIGMA-ALDRICH-FLUKA |
| Galactitol | C6H14O 6 | 1421 | 1245,471 | 5880 | SIGMA-ALDRICH-FLUKA |
| Galactitol 1-phosphate | C6H15O 9P | 1422 | 1325,451 | 6274 | SIGMA-ALDRICH-FLUKA |
| D-Glucosamine 6-phosphate | C6H14N O8P | 1423 | 1336,478 | 6268 | SIGMA-ALDRICH-FLUKA |
| D-Glucosamine 1-phosphate | C6H14N O8P | 1424 | 1322,451 | 6267 | SIGMA-ALDRICH-FLUKA |
| D-Galacturonate | C6H10O 7 | 1425 | 1271,465 | 5707 | SIGMA-ALDRICH-FLUKA |
| D-Glucosamine | C6H13N O5 | 1426 | 1242,470 | 6427 | SIGMA-ALDRICH-FLUKA |
| Octanoyl-CoA | C29H50N 7O17P3S | 1427 | 1973,031 | 6888 | SIGMA-ALDRICH-FLUKA |
| gamma-Butyrolactone | C4H6O2 | 1428 | 1165,387 | 6874 | SIGMA-ALDRICH-FLUKA |
| 4-Guanidinobutanoate | C5H11N3 O2 | 1429 | 1224,458 | 6268 | SIGMA-ALDRICH-FLUKA |
| Butyro-betaine | C8H18N O | 1430 | 1223,534 | 9236 | As described by Goodfellow *et al.*, *Journal of Labelled Compounds and Radiopharmaceuticals* 19, 365 (2006) |
| 4-Guanidinobutanamide | C5H12N4 O | 1431 | 1207,474 | 9236 | HPLC purification from *Pseudomonas* sp. |
| Glycolaldehyde | C2H4O2 | 1432 | 1123,350 | 9222 | SIGMA-ALDRICH-FLUKA |
| Cyclohexa-3,5-diene-1,2-dicarboxylate | C8H8O4 | 1433 | 1247,445 | 9343 | As described by Mereyala and Pannala, *J Chem Soc., Perkin Trans* 1, 1755 (1997) |
| Glycolyl-D-mannosamine | C8H15N O7 | 1434 | 1316,507 | 9236 | As described by Yu *et al., Bioorganic & Medicinal Chemistry* 12, 6427 (2004) |
| Glycolyl-D-mannosaminolactone | C8H13N O7 | 1435 | 1298,491 | 6401 | As described by Yu *et al., Bioorganic & Medicinal Chemistry* 12, 6427 (2004) |
| Octanoic acid | C8H16O 2 | 1436 | 1223,511 | 7589 | SIGMA-ALDRICH-FLUKA |

| | | | | | |
|---|---|---|---|---|---|
| GDP-6-deoxy-D-mannose | C16H25N5O15P2 | 1437 | 1652,644 | 6334 | SIGMA-ALDRICH-FLUKA |
| GDP-6-deoxy-D-talose | C16H25N5O15P2 | 1438 | 1652,644 | 6615 | HPLC purification from *Actinobacillus actinomycetemcomitans* |
| GDP-4-dehydro-6-deoxy-D-mannose | C16H23N5O15P2 | 1439 | 1650,628 | 6428 | HPLC purification from *Actinobacillus actinomycetemcomitans* |
| GDP-D-mannose | C16H25N5O16P2 | 1440 | 1668,644 | 6321 | SIGMA-ALDRICH-FLUKA |
| GDP-L-fucose | C16H25N5O15P2 | 1441 | 1652,644 | 6335 | HPLC purification from *Actinobacillus actinomycetemcomitans* |
| 6-Deoxy-D-glucose | C6H12O5 | 1442 | 1227,455 | 11426 | SIGMA-ALDRICH-FLUKA |
| Gentiobiose | C12H22O11 | 1443 | 1405,597 | 11426 | SIGMA-ALDRICH-FLUKA |
| Gentisate | C7H6O4 | 1444 | 1247,445 | 6421 | SIGMA-ALDRICH-FLUKA |
| Geranyl acetate | C12H20O2 | 1445 | 1275,587 | 7001 | SIGMA-ALDRICH-FLUKA |
| Geranic acid | C10H16O2 | 1446 | 1247,533 | 8310 | SIGMA-ALDRICH-FLUKA |
| Geranial | C10H16O | 1447 | 1231,533 | 6507 | SIGMA-ALDRICH-FLUKA |
| Geraniol | C10H18O | 1448 | 1233,549 | 7245 | SIGMA-ALDRICH-FLUKA |
| trans-Geranyl-CoA | C31H50N7O17P3S | 1449 | 1997,053 | 7589 | trans-Geranic acid + CoA + ACETYLTRANSFERASA Arabidopsis thaliana → trans-Geranyl-CoA |
| cis-Geranyl-CoA | C31H50N7O17P3S | 1450 | 1997,053 | 6127 | cis-Geranic acid + CoA + ACETYLTRANSFERASA Arabidopsis thaliana → cis-Geranyl-CoA |
| Geranylate | C10H16O2 | 1451 | 1247,533 | 6614 | SIGMA-ALDRICH-FLUKA |
| 3-beta-D-Galactosyl-sn-glycerol | C9H18O8 | 1452 | 1317,535 | 6187 | HPLC purification from *Saccharomyces cerivisiae* |
| Geranylgeranyl diphosphate | C20H36O7P2 | 1453 | 1529,747 | 6601 | SIGMA-ALDRICH-FLUKA |
| beta-D-Glucose | C6H12O6 | 1454 | 1243,455 | 6281 | SIGMA-ALDRICH-FLUKA |

| | | | | | |
|---|---|---|---|---|---|
| gamma-Hexachlorocyclohexane | C6H6Cl6 | 1455 | 1370,128 | 6281 | Provided by Healthwise |
| 1-alpha-D-Galactosyl-myo-inositol | C12H22 O11 | 1456 | 1405,597 | 6180 | HPLC purification from *Saccharomyces cerivisiae* |
| Nitrobenzene | C6H5NO 2 | 1457 | 1202,409 | 6180 | SIGMA-ALDRICH-FLUKA |
| D-Gluconate | C6H12O 7 | 1458 | 1259,454 | 6820 | SIGMA-ALDRICH-FLUKA |
| Glutarate | C5H8O4 | 1459 | 1211,412 | 6480 | SIGMA-ALDRICH-FLUKA |
| D-Glucuronate | C6H10O 7 | 1460 | 1271,465 | 6761 | SIGMA-ALDRICH-FLUKA |
| D-Glutamine | C5H10N2 O3 | 1461 | 1209,443 | 18400 | SIGMA-ALDRICH-FLUKA |
| L-Glutamine | C5H10N2 O3 | 1462 | 1209,443 | 6507 | SIGMA-ALDRICH-FLUKA |
| L-Glutamate 1-semialdehyde | C5H9NO 3 | 1463 | 1194,429 | 6975 | SIGMA-ALDRICH-FLUKA |
| L-Glutamate 5-semialdehyde | C5H9NO 3 | 1464 | 1194,429 | 6287 | SIGMA-ALDRICH-FLUKA |
| L-Glutamate 5-phosphate | C5H10N O7P | 1465 | 1290,408 | 11265 | SIGMA-ALDRICH-FLUKA |
| D-Glucurono-6,2-lactone | C6H8O6 | 1466 | 1253,450 | 11185 | SIGMA-ALDRICH-FLUKA |
| gamma-L-Glutamyl-D-alanine | C8H14N2 O5 | 1467 | 1281,506 | 6480 | SIGMA-ALDRICH-FLUKA |
| beta-D-Glucan | C12H22 O11(C6H 10O5)10 | 1468 | 3027,021 | 5306 | SIGMA-ALDRICH-FLUKA |
| alpha-D-Glucose | C6H12O 6 | 1469 | 1243,455 | 6401 | SIGMA-ALDRICH-FLUKA |
| gamma-L-Glutamyl-L-cysteine | C8H14N2 O5S | 1470 | 1313,566 | 6287 | SIGMA-ALDRICH-FLUKA |
| D-Glutamate | C5H9NO 4 | 1471 | 1210,428 | 5800 | SIGMA-ALDRICH-FLUKA |
| 3-Hydroxy-5-methylhex-4-enoyl-CoA | C28H46N 7O18P3S | 1472 | 1972,987 | 6820 | HPLC purification from *Saccharomyces cerivisiae* |
| Glutaryl-CoA | C26H42N | 1473 | 1960,932 | 7080 | Glutaric acid+ CoA + ACETYLTRANSFERASA Arabidopsis thaliana → Glutaryl- |

| | | | | | |
|---|---|---|---|---|---|
| | 7O19P3S | | | | CoA |
| D-Glucurono-3,6-lactone | C6H8O6 | 1474 | 1253,450 | 5807 | Provided by Alfa Aesar (http://www.alfa.com/alf/laboratory_chemical_suppliers.htm) |
| Glucomannan | C24H42 O21 | 1475 | 1743,909 | 6421 | As described by Teleman *et al.*, *Carbohydrate Research* 338, 525 (2003) |
| 2-Hydroxycinnamate | C9H8O3 | 1476 | 1243,457 | 5907 | SIGMA-ALDRICH-FLUKA |
| L-Glutamate | C5H9NO 4 | 1477 | 1210,428 | 6547 | SIGMA-ALDRICH-FLUKA |
| Glyoxylate | C2H2O3 | 1478 | 1137,334 | 6381 | SIGMA-ALDRICH-FLUKA |
| alpha-D-Glutamyl phosphate | C5H10N O7P | 1479 | 1320,435 | 7774 | SIGMA-ALDRICH-FLUKA |
| 2,3-Bisphospho-D-glycerate | C3H8O1 0P2 | 1480 | 1359,362 | 7241 | SIGMA-ALDRICH-FLUKA |
| Glycine | C2H5NO 2 | 1481 | 1223,426 | 7241 | SIGMA-ALDRICH-FLUKA |
| 1-Hydroxy-2-naphthaldehyde | C11H8O 2 | 1482 | 1251,480 | 9751 | Provided by Jinan Haohua Industry Co., Ltd. |
| D-Glyceraldehyde | C3H6O3 | 1483 | 1153,376 | 6013 | SIGMA-ALDRICH-FLUKA |
| D-Glycerate 3-phosphate | C3H7O7 P | 1484 | 1249,355 | 5907 | SIGMA-ALDRICH-FLUKA |
| L-Glutamate methylester | C6H11N O4 | 1485 | 1224,455 | 7080 | SIGMA-ALDRICH-FLUKA |
| D-Glycero-D-manno-heptose 1,7-bisphosphate | C7H16O 13P2 | 1486 | 1447,469 | 5920 | HPLC purification from *Saccharomyces cerivisiae* |
| D-Glycero-D-manno-heptose 1-phosphate | C7H15O 10P | 1487 | 1367,488 | 5813 | HPLC purification from *Saccharomyces cerivisiae* |
| D-Glycero-D-manno-heptose 7-phosphate | C7H15O 10P | 1488 | 1367,488 | 6608 | HPLC purification from *Saccharomyces cerivisiae* |
| Glycogen | C24H42 O21 | 1489 | 1729,882 | 9618 | SIGMA-ALDRICH-FLUKA |
| (R)-Glycerate | C3H6O4 | 1490 | 1185,374 | 6440 | SIGMA-ALDRICH-FLUKA |
| Glycerone | C3H6O3 | 1491 | 1169,375 | 5800 | HPLC purification from *Saccharomyces cerivisiae* |
| 1,3-Bisphospho-D-glycerate | C3H8O1 0P2 | 1492 | 1329,335 | 6494 | HPLC purification from *Saccharomyces cerivisiae* |
| Glycerol | C3H8O3 | 1493 | 1155,392 | 11097 | SIGMA-ALDRICH-FLUKA |

| | | | | | |
|---|---|---|---|---|---|
| Glycolate | C2H4O3 | 1494 | 1139,350 | 7401 | SIGMA-ALDRICH-FLUKA |
| D-Glucosaminate | C6H13NO6 | 1495 | 1258,470 | 7195 | SIGMA-ALDRICH-FLUKA |
| D-Glucosaminide | C12H24N2O10(C6H11NO3)2 | 1496 | 1709,944 | 9391 | SIGMA-ALDRICH-FLUKA |
| Glycerone phosphate | C3H7O6S | 1497 | 1234,442 | 7355 | SIGMA-ALDRICH-FLUKA |
| Glyoxalate | C2H2O3 | 1498 | 1137,334 | 6447 | SIGMA-ALDRICH-FLUKA |
| D-Galactono-1,4-lactone | C6H10O6 | 1499 | 1241,439 | 8204 | As described by Zaliz and Varela , *Carbohydrate Research* 341, 2973 (2006) |
| P1,P4-Bis(5'-guanosyl) tetraphosphate | C20H28N10O21P4 | 1500 | 1931,689 | 9998 | HPLC purification from *Saccharomyces cerivisiae* |
| Geranyl diphosphate | C10H20O7P2 | 1501 | 1393,510 | 6087 | SIGMA-ALDRICH-FLUKA |
| Guanosine | C10H13N5O5 | 1502 | 1346,541 | 6134 | SIGMA-ALDRICH-FLUKA |
| Glutathione oxidized | C20H32N6O12S2 | 1503 | 1705,951 | 6507 | Boehringer Manheim |
| Glutathione reduced | C10H17N3O6S | 1504 | 1400,645 | 44415 | Boehringer Manheim |
| 2,3,4,5-Tetrahydrodipicolinate | C7H9NO4 | 1505 | 1264,476 | 43479 | HPLC purification from *Arabidopsis thaliana* |
| Glutathionylspermidine | C17H34N6O5S | 1506 | 1497,851 | 7054 | HPLC purification from *Saccharomyces cerivisiae* |
| Tetrahydropteroyltri-L-glutamate | C29H37N9O12 | 1507 | 1766,963 | 7054 | HPLC purification from *Arabidopsis thaliana* |
| Guanidoacetate | C3H7N3O2 | 1508 | 1210,431 | 11385 | As described by Arcelloni *et al., J Lab Comp and Radiophar* 31, 505 (2006) |
| Guaiacol | C7H8O2 | 1509 | 1203,436 | 7361 | SIGMA-ALDRICH-FLUKA |
| L-Gulose | C6H12O6 | 1510 | 1243,455 | 7809 | SIGMA-ALDRICH-FLUKA |
| Glucuronoxylan 4-O-methyl-D-glucuronate | (C22H34O18)10 | 1511 | 6944,308 | 5330 | HPLC purification from *Saccharomyces cerivisiae* |

| | | | | | |
|---|---|---|---|---|---|
| Glucuronoxylan D-glucuronate | (C21H32O18)10 | 1512 | 6802,066 | 5217 | HPLC purification from *Saccharomyces cerivisiae* |
| 1-Hydroxy-2-methyl-2-(E)-butenyl 4-diphosphate | C5H12O8P2 | 1513 | 1341,390 | 8464 | HPLC purification from *Saccharomyces cerivisiae* |
| (E)-4-Hydroxy-3-methylbut-2-en-1-yl diphosphate | C5H12O8P2 | 1514 | 1341,390 | 8464 | HPLC purification from *Saccharomyces cerivisiae* |
| (S)-3-Hydroxy-3-methylglutaryl-CoA | C27H44N7O20P3S | 1515 | 2004,986 | 13662 | HPLC purification from *Saccharomyces cerivisiae* |
| Galactarate | C6H10O8 | 1516 | 1303,464 | 8049 | SIGMA-ALDRICH-FLUKA |
| But-1-ene-1,2,4-tricarboxylate | C7H8O6 | 1517 | 1267,433 | 7873 | As in Journal of the American Chemical Society, 106, p. 3368, 1984 |
| Hydroxyacetone phosphate | C3H7O5P | 1518 | 1233,356 | 7600 | SIGMA-ALDRICH-FLUKA |
| cis-4-(2-hydroxynaph-3-yl)-2-oxobut-3-enoic acid | C14H10O42 | 1519 | 1913,505 | 13162 | HPLC purification from *Pseudomonas* sp. |
| 3-Hydroxy-2-naphthoate | C11H8O3 | 1520 | 1267,479 | 7873 | SIGMA-ALDRICH-FLUKA |
| 2,2',3,4',5,5',6-Heptachloro-4-biphenylol | C12H3Cl7O | 1521 | 1474,624 | 7673 | As described by Safe *et al.*, *Chemosphere* 31, 3017 (1995) |
| (15S)-15-Hydroxy-5,8,11-cis-13-trans-eicosatetraenoate | C20H32O3 | 1522 | 1399,769 | 8932 | HPLC purification from *Saccharomyces cerivisiae* |
| L-Homocysteine | C4H9NO2S | 1523 | 1198,478 | 7449 | SIGMA-ALDRICH-FLUKA |
| alpha-D-Glucose 6-phosphate | C6H13O9P | 1524 | 1337,462 | 8449 | SIGMA-ALDRICH-FLUKA |
| Hexadecenoic acid | C16H30O2 | 1525 | 1333,710 | 8185 | SIGMA-ALDRICH-FLUKA |
| trans-Hexadec-2-enoyl-CoA | C37H64N7O17P3S | 1526 | 2083,230 | 14401 | HPLC purification from *Saccharomyces cerivisiae* |
| cis-4-[2-(3-Hydroxy)-thionaphthenyl]-2-oxo-3-butenoate | C12H8O4S | 1527 | 1311,550 | 8346 | HPLC purification from *Pseudomonas* sp. |
| RNA Helicase substrate | # | 1528 | # | # | In house substrate |
| Heptane | C7H16 | 1529 | 1179,501 | 9217 | SIGMA-ALDRICH-FLUKA |
| Heptadecanoyldolichol | C37H50O2(C5H8 | 1530 | 2287,285 | 7511 | HPLC purification from *Arabidopsis thaliana* |

| | | | | | |
|---|---|---|---|---|---|
| | )10 | | | | |
| Heptadecanoic acid | C17H34 O2 | 1531 | 1349,753 | 8531 | SIGMA-ALDRICH-FLUKA |
| Heptadecane | C17H36 | 1532 | 1319,770 | 8291 | SIGMA-ALDRICH-FLUKA |
| Heptadecanoyl-CoA | C38H68N 7O17P3S | 1533 | 2099,273 | 14529 | SIGMA-ALDRICH-FLUKA |
| all-trans-Heptaprenyl diphosphate | C35H60 O7P2 | 1534 | 1734,102 | 11607 | HPLC purification from *Arabidopsis thaliana* |
| Heptanoic acid | C7H14O 2 | 1535 | 1209,484 | 7409 | SIGMA-ALDRICH-FLUKA |
| trans-2-Methyl-5-isopropylhexa-2,5-dienoyl-CoA | C31H50N 7O17P3S | 1536 | 1997,053 | 13711 | HPLC purification from *Arabidopsis thaliana* |
| Heptanal | C7H14O | 1537 | 1193,484 | 7281 | SIGMA-ALDRICH-FLUKA |
| Hexanoic acid | C6H12O 2 | 1538 | 1195,457 | 7297 | SIGMA-ALDRICH-FLUKA |
| Hexacosane | C26H54 | 1539 | 1446,012 | 16215 | SIGMA-ALDRICH-FLUKA |
| Hexadecane | C16H34 | 1540 | 1305,743 | 12532 | SIGMA-ALDRICH-FLUKA |
| 2,6-Dimethyl-5-methylene-3-oxo-heptanoyl-CoA | C31H50N 7O18P3S | 1541 | 2013,052 | 13839 | HPLC purification from *Arabidopsis thaliana* |
| Hexacosanoate | C26H52 O2 | 1542 | 1475,995 | 9542 | SIGMA-ALDRICH-FLUKA |
| Hexanal | C6H12O | 1543 | 1179,457 | 7169 | SIGMA-ALDRICH-FLUKA |
| Hexyl cinnamaldehyde | C15H20 O | 1544 | 1295,620 | 8098 | Provided by Betapharma(Shangai)Co., Ltd. |
| 2,2',4,4',5,5'-Hexachlorobiphenyl | C12H4Cl 6 | 1545 | 1440,179 | 7553 | SIGMA-ALDRICH-FLUKA |
| 2,2',4,4',6,6'-Hexachlorobiphenyl | C12H4Cl 6 | 1546 | 1440,179 | 7553 | SIGMA-ALDRICH-FLUKA |
| 2,2',3,3',5,5'-Hexachlorobiphenyl | C12H4Cl 6 | 1547 | 1440,179 | 7553 | SIGMA-ALDRICH-FLUKA |
| 2,2',3,3',6,6'-Hexachlorobiphenyl | C12H4Cl 6 | 1548 | 1440,179 | 7553 | SIGMA-ALDRICH-FLUKA |
| Hexanoyl-CoA | C27H46N 7O17P3S | 1549 | 1944,977 | 13294 | SIGMA-ALDRICH-FLUKA |
| Hexadecanoyl-CoA | C37H66N | 1550 | 2085,246 | 14417 | SIGMA-ALDRICH-FLUKA |

| | | | | | |
|---|---|---|---|---|---|
| | 7O17P3S | | | | |
| 1,2-Epoxyhexadecane | C16H32O | 1551 | 1319,726 | 8291 | SIGMA-ALDRICH-FLUKA |
| Hexadecanoylglycerone phosphate | C4H6O7P(C16H32O2) | 1552 | 1532,787 | 9996 | HPLC purification from *Saccharomyces cerivisiae* |
| Hexanoyldolichol | C21H35O2(C5H8)10C6H12O2 | 1553 | 2196,149 | 6023 | HPLC purification from *Arabidopsis thaliana* |
| L-Histidinol phosphate | C6H12N3O4P | 1554 | 1300,449 | 8137 | As described by Suga and Okabe, *Acta Cryst* C53, 134 (1997) |
| Histamine | C5H9N3 | 1555 | 1190,443 | 7257 | SIGMA-ALDRICH-FLUKA |
| (S)-3-Hydroxyisobutyrate | C4H8O3 | 1556 | 1183,402 | 7200 | Provided by TCI America |
| (S)-3-Hydroxyisobutyryl-CoA | C25H42N7O18P3S | 1557 | 1932,922 | 13198 | HPLC purification from *Saccharomyces cerivisiae* |
| Homoisocitrate | C7H10O7 | 1558 | 1299,476 | 8017 | SIGMA-ALDRICH-FLUKA |
| Histone-arginine | C7H13N5O2(CH3)2 | 1559 | 1349,631 | 8202 | SIGMA-ALDRICH-FLUKA |
| L-Histidine | C6H9N3O2 | 1560 | 1218,453 | 7609 | SIGMA-ALDRICH-FLUKA |
| Histone L-lysine | C7H13N3O2(CH3)2 | 1561 | 1264,566 | 7978 | SIGMA-ALDRICH-FLUKA |
| Histone N(omega)-methyl-L-arginine | C8H15N5O2(CH3)2 | 1562 | 1322,607 | 8314 | SIGMA-ALDRICH-FLUKA |
| 3,3',4',5,7,8-Hexahydroxyflavone | C15H10O8 | 1563 | 1381,537 | 8914 | Provided by Xiamen Bioshining Biotechnology Co.,ltd (http://www.bioshining.net) |
| D-Hexose | C6H12O6 | 1564 | 1243,455 | 7809 | SIGMA-ALDRICH-FLUKA |
| L-Histidinol | C6H11N3O | 1565 | 1204,470 | 7497 | SIGMA-ALDRICH-FLUKA |

| | | | | | |
|---|---|---|---|---|---|
| Histone | C6H11N3O | 1566 | # | 8250 | SIGMA-ALDRICH-FLUKA |
| 2-Hydroxy-6-oxonona-2,4-diene-1,9-dioate | C9H10O6 | 1567 | 1277,472 | 8081 | SIGMA-ALDRICH-FLUKA |
| 2-Oxohept-3-enedioate | C7H8O5 | 1568 | 1235,434 | 7745 | HPLC purification from *Saccharomyces cerivisiae* |
| 3-Hydroxy-L-kynurenine | C10H12N2O4 | 1569 | 1287,513 | 8161 | SIGMA-ALDRICH-FLUKA |
| Hydroxymethylbilane | C40H46N4O17 | 1570 | 1918,119 | 13207 | HPLC purification from *Saccharomyces cerivisiae* |
| (S)-3-Hydroxy-2-methylbutyryl-CoA | C26H44N7O18P3S | 1571 | 1791,793 | 13310 | Hexanoic acic + vinyl acetate + metagenomic lipase EL1 |
| trans-4-[2-(3-Hydroxy)-thionaphthenyl]-2-oxo-3-butenoate | C12H8O4S | 1572 | 1311,550 | 8346 | HPLC purification from *Saccharomyces cerivisiae* |
| L-Homoserine | C4H9NO3 | 1573 | 1182,418 | 7320 | SIGMA-ALDRICH-FLUKA |
| 2-Hydroxy-2H-benzo[h]chromene-2-carboxylate | C14H10O4 | 1574 | 1321,527 | 8306 | HPLC purification from *Saccharomyces cerivisiae* |
| Histone N6-methyl-L-lysine | C8H15N3O2(CH3)2 | 1575 | 1294,593 | 8090 | HPLC purification from *Saccharomyces cerivisiae* |
| Homogentisate | C8H8O4 | 1576 | 1231,446 | 7713 | SIGMA-ALDRICH-FLUKA |
| 3-Hydroxypropanoate | C3H6O3 | 1577 | 1177,398 | 7088 | SIGMA-ALDRICH-FLUKA |
| 3-Hydroxypropionyl-CoA | C24H40N7O18P3S | 1578 | 1902,896 | 13086 | SIGMA-ALDRICH-FLUKA |
| (S)-3-Hydroxybutanoate | C4H8O3 | 1579 | 1167,403 | 7200 | SIGMA-ALDRICH-FLUKA |
| trans-4-Hydroxy-L-proline | C5H9NO3 | 1580 | 1194,429 | 7417 | SIGMA-ALDRICH-FLUKA |
| all-trans-Hexaprenyl diphosphate | C30H52O7P2 | 1581 | 1665,984 | 11062 | HPLC purification from *Arabidopsis thaliana* |
| Hydroxypyruvate | C3H4O4 | 1582 | 1167,360 | 7200 | SIGMA-ALDRICH-FLUKA |
| cis-4-Hydroxy-L-proline | C5H9NO3 | 1583 | 1194,429 | 7417 | SIGMA-ALDRICH-FLUKA |
| 4-Hydroxy-2-oxohexanoate | C6H10O4 | 1584 | 1209,440 | 7537 | 4-Methylcatechol + *Pseudomonas putida* KT2440 cells |
| Hypoxanthine | C5H4N4 | 1585 | 1256,462 | 7456 | SIGMA-ALDRICH-FLUKA |

| | | | | | |
|---|---|---|---|---|---|
| | O | | | | |
| trans-Hexacos-2-enoyl-CoA | C49H88O19N7P3S | 1586 | 2283,551 | 8101 | HPLC purification from *Saccharomyces cerivisiae* |
| Hexadecanal | C16H32O | 1587 | 1319,726 | 8291 | SIGMA-ALDRICH-FLUKA |
| 4-Hydroxy-2-oxopentanoate | C5H8O4 | 1588 | 1195,413 | 7424 | Provided by Bright Chemicals |
| Hydroxyatrazine | C8H15N5O | 1589 | 1276,537 | 7946 | Provided by SYNCHEM |
| Hydantoin-5-propionate | C6H8N2O4 | 1590 | 1292,489 | 7745 | SIGMA-ALDRICH-FLUKA |
| 2-(Indol-3-yl)acetaldehyde | C10H9NO | 1591 | 1238,484 | 7641 | As described by G. Reed, *Archives of Biochemistry and Biophysics* 8, 480 (1959) |
| Indole-3-acetyl-beta-1-D-glucose | C16H19NO7 | 1592 | 1400,627 | 9067 | HPLC purification from *Arabidopsis thaliana* |
| Indole-3-acetyl-L-glutamine | C16H19NO7 | 1593 | 1416,627 | 9051 | HPLC purification from *Arabidopsis thaliana* |
| Indole-3-acetyl-myo-inositol | C16H19NO7 | 1594 | 1400,627 | 9067 | As described by Nowacki *et al., J Lab Comp and Radiophar* 15, 325 (2006) |
| 2-Phenylacetamide | C8H9NO | 1595 | 1214,462 | 7449 | SIGMA-ALDRICH-FLUKA |
| Indole-3-acetamide | C10H10N2O | 1596 | 1253,498 | 7761 | SIGMA-ALDRICH-FLUKA |
| (Indol-3-yl)pyruvate | C11H9NO3 | 1597 | 1282,494 | 7993 | SIGMA-ALDRICH-FLUKA |
| Pentadecanal | C15H30O | 1598 | 1305,699 | 8179 | SIGMA-ALDRICH-FLUKA |
| Indole-3-ethanol | C10H11NO | 1599 | 1240,500 | 7657 | SIGMA-ALDRICH-FLUKA |
| (Indol-3-yl)acetate | C10H9NO2 | 1600 | 1254,484 | 7769 | SIGMA-ALDRICH-FLUKA |
| Iminoaspartate | C4H5NO4 | 1601 | 1224,411 | 7304 | Oxidation of l-aspartate by L-aspartate oxidase from metagenomic origin |
| 2-Methylpropanoyl-CoA | C25H42N7O17P3S | 1602 | 1916,923 | 13070 | 2-Methylpropanoate + CoA + ACETYLTRANSFERASA Arabidopsis thaliana → 2-Methylpropanoyl-CoA |
| Isobutyryl-CoA | C25H42N | 1603 | 1916,923 | 13070 | Glutaric acid+ CoA + ACETYLTRANSFERASA Arabidopsis thaliana → 2- |

| | | | | | |
|---|---|---|---|---|---|
| | 7O17P3S | | | | ethylpropanoyl-CoA |
| 6-Aminohexanoate | C6H13NO2 | 1604 | 1194,472 | 7417 | Provided by Alfa Aesar (http://www.alfa.com/alf/laboratory_chemical_suppliers.htm) |
| Isochorismate | C10H10O6 | 1605 | 1319,509 | 8177 | As described by Gould and Eisenberg, *Tetrahedron* 47, 5979 (1991) |
| L-Idonate | C6H12O7 | 1606 | 1259,454 | 7937 | HPLC purification from *Pseudomonas putida* IFO3738 (Shibata et al. Journal of Bioscience and Bioengineering 2000, 90, 223-225) |
| Indole-3-acetaldehyde | C10H9NO | 1607 | 1238,484 | 7641 | SIGMA-ALDRICH-FLUKA |
| Isocitrate | C6H8O7 | 1608 | 1285,449 | 7905 | SIGMA-ALDRICH-FLUKA |
| Inosine 5'-diphosphate | C10H14N4O11P2 | 1609 | 1505,514 | 9794 | SIGMA-ALDRICH-FLUKA |
| Gentisate aldehyde | C7H6O3 | 1610 | 1201,420 | 7473 | SIGMA-ALDRICH-FLUKA |
| Indoleglycerol phosphate | C11H14NO6P | 1611 | 1366,506 | 8665 | Indoleglycerol phosphate + metagenomic sulfotransferase |
| cis-3,4-Dihydroxyphenanthrene-4-carboxylate | C15H12O4 | 1612 | 1335,554 | 8410 | HPLC purification from *Pseudomonas putida* KT2440 |
| L-Isoleucine | C6H13NO2 | 1613 | 1210,472 | 7417 | SIGMA-ALDRICH-FLUKA |
| 3-(Imidazol-4-yl)-2-oxopropyl phosphate | C6H9N2O5P | 1614 | 1340,471 | 8129 | HPLC purification from *Pseudomonas putida* KT2440 |
| L-Histidinal | C6H9N3O | 1615 | 1259,506 | 7481 | As described by Dancer *et al.*, *Bioorganic & Medicinal Chemistry Letters* 6, 2131 (1996) |
| Imidazo[1,5a]pyrimidine | C6H5N3 | 1616 | 1199,430 | 7361 | SIGMA-ALDRICH-FLUKA |
| 4-Imidazolone-5-propanoate | C6H8N2O3 | 1617 | 1249,464 | 7617 | HPLC purification from *Pseudomonas putida* KT2440 |
| Indoxazene | C7H5NO | 1618 | 1198,420 | 7320 | As described by Strakov *et al.*, *Chemistry of Heterocyclic Compounds* 10, 881 (1974) |
| Indane | C9H10 | 1619 | 1197,475 | 7313 | As described by Elwan *et al.*, *Heteroatom Chemistry* 13, 585 (2002) |
| Indene | C9H8 | 1620 | 1195,459 | 7297 | As described by Miller *et al.*, *J Org Chem* 39, 1955 (1974) |
| Methyl 3-hydroxybenzoate | C13H14O3 | 1621 | 1297,549 | 8114 | As in Synthetic Communications, 16, p. 331, 1986 |
| Indoline | C8H9N | 1622 | 1198,463 | 7321 | As described by Koo and Hillhouse, *Organometallics* 15, 2669 (1996) |
| 2,3-Benzopyrrole | C8H7N | 1623 | 1196,447 | 7305 | As in Nishio, T., Okuda, N., and Kashima, C. 1990. Reduction of indolin-2-ones and desulfurization of indoline-2-thiones to indoline and indole derivatives. Helv. |

| | | | | | |
|---|---|---|---|---|---|
| | | | | | Chim. Acta. 73:1719-1723. |
| myo-Inositol | C6H12O6 | 1624 | 1257,482 | 7809 | SIGMA-ALDRICH-FLUKA |
| Indole-3-pyruvate | C11H9NO3 | 1625 | 1282,494 | 7993 | SIGMA-ALDRICH-FLUKA |
| Inosine | C10H12N4O5 | 1626 | 1331,526 | 8514 | SIGMA-ALDRICH-FLUKA |
| Inosine 5'-tetraphosphate | C10H16N4O17P4 | 1627 | 1665,473 | 11073 | SIGMA-ALDRICH-FLUKA |
| Inosine 5'-triphosphate | C10H15N4O14P3 | 1628 | 1585,493 | 10434 | SIGMA-ALDRICH-FLUKA |
| Isopentenyl diphosphate | C5H12O7P2 | 1629 | 1325,391 | 8336 | As described by Leyes and Poulter, *Org. Lett* 1, 1067 (1999) |
| 2-Isopropylmalate | C7H12O5 | 1630 | 1255,466 | 7777 | As described by Bialy *et al.*, *European Journal of Organic Chemistry* 14, 2965 (2005) |
| Isothiazoline | C3H2NOS | 1631 | 1179,412 | 8820 | As described by Bell *et al.*, *Tetrahedron* 55, 12313 (1999) |
| Isobenzofuran | C8H6O | 1632 | 1197,431 | 7313 | As described by R. Rodrigo, *Tetrahedron* 44, 2093 (1988) |
| Isomaltose | C12H22O11 | 1633 | 1405,597 | 9106 | SIGMA-ALDRICH-FLUKA |
| Isochromen-3-one | C9H6O2 | 1634 | 1225,442 | 7537 | As described by Taylor *et al.*, *Tetrahedron* 63, 10889 (2007) |
| Isocoumarin | C9H6O2 | 1635 | 1225,442 | 7537 | Addition of a catalytic amount of Cy2NH·HX improves the rate and yields for cyclization reactions of o-(alkynyl)benzoates in the presence of a stoichiometric amount of copper(II)halide to give 4-haloisocoumarins. Under the standard reaction conditions, various 4-haloisocoumarins are provided in good yield. Y. Liang, Y.-X. Xie, J.-H. Li, Synthesis, 2007, 400-406 |
| 2-Methylpropanoate | C4H8O2 | 1636 | 1167,403 | 7072 | SIGMA-ALDRICH-FLUKA |
| Isoorientin | C21H20O11 | 1637 | 1511,681 | 9955 | As described by Kumazawa *et al.*, *Carbohydrate Research* 329, 507 (2000) |
| Isopentanoyl-CoA | C26H44N7O17P3S | 1638 | 1930,950 | 7168 | Isopentanoate + CoA + ACETYLTRANSFERASA Arabidopsis thaliana → Isopentanoyl-CoA |
| Isoprene | C5H8 | 1639 | 1188,468 | 6912 | SIGMA-ALDRICH-FLUKA |
| L-Carnitinamide | C7H17N2O2 | 1640 | 1224,521 | 7657 | HPLC purification from *Pseudomonas putida* KT2440 |
| 2-Benzazine | C9H7N | 1641 | 1208,458 | 7409 | Provided by Meta-Synthesis |

| | | | | | |
|---|---|---|---|---|---|
| Isoscoparin | C22H22O11 | 1642 | 1525,707 | 10067 | HPLC purification from *Arabidopsis thaliana* |
| Imidazolone | C3H4N2O | 1643 | 1163,374 | 7040 | SIGMA-ALDRICH-FLUKA |
| Ethyl isovalerate | C7H14O2 | 1644 | 1209,484 | 7409 | SIGMA-ALDRICH-FLUKA |
| Isovaleryl-CoA | C26H44N7O17P3S | 1645 | 1930,950 | 13182 | Isovalerate + CoA + ACETYLTRANSFERASA Arabidopsis thaliana → Isovaleryl-CoA |
| Isoxazolidine | C3H7NO | 1646 | 1152,391 | 6952 | SIGMA-ALDRICH-FLUKA |
| Isoxazole | C3H3NO | 1647 | 1148,360 | 6920 | SIGMA-ALDRICH-FLUKA |
| 7-Hydroxycoumarin | C9H6O3 | 1648 | 1241,441 | 7665 | SIGMA-ALDRICH-FLUKA |
| Carnitine | C7H16NO3 | 1649 | 1225,507 | 7665 | SIGMA-ALDRICH-FLUKA |
| (2S)-2-Isopropyl-3-oxosuccinate | C7H10O5 | 1650 | 1253,450 | 7761 | HPLC purification from *Pseudomonas putida* KT2440 |
| 5-Carboxymethyl-2-hydroxymuconate semialdehyde | C8H6O6 | 1651 | 1261,429 | 7953 | HPLC purification from *Pseudomonas putida* KT2440 |
| Hexose-1,5-lactone | C6H10O6 | 1652 | 1241,439 | 7793 | As described by Scaffidi *et al.*, *Australian Journal of Chemistry* 57, 723 (2004) |
| Kanosamine 6-Phosphate | C6H14NO8P | 1653 | 1336,478 | 8441 | As described by A. Kirschning *et al.*, *Carbohydr Res.* 256, 245 (1994) |
| 3-Deoxy-D-manno-2-octulosonate | C8H14O8 | 1654 | 1331,518 | 8273 | As described by Hongtao *et al.*, *Bioorganic and Medicinal Chemistry Letters* 7, 1419 (1997) |
| 3-Deoxy-D-manno-octulosonate 8-phosphate | C8H15O11P | 1655 | 1411,498 | 8913 | As described by Hongtao *et al.*, *Bioorganic and Medicinal Chemistry Letters* 7, 1419 (1997) |
| alpha-Ketoglutaric acid | C5H6O5 | 1656 | 1225,396 | 7536 | SIGMA-ALDRICH-FLUKA |
| Ketoprofen methyl ester | C17H16O3 | 1657 | 1347,609 | 8514 | SIGMA-ALDRICH-FLUKA |
| (R)-2-Methylmalate | C5H8O5 | 1658 | 1241,439 | 7552 | SIGMA-ALDRICH-FLUKA |
| Ketosamine | C13H16ClNO | 1659 | 1301,027 | 7038 | SIGMA-ALDRICH-FLUKA |
| Ketose | C6H12O6 | 1660 | 1243,455 | 7809 | SIGMA-ALDRICH-FLUKA |
| 5-Carboxymethyl-2-hydroxymuconate | C8H6O72 | 1661 | 2333,389 | 16394 | HPLC purification from *Pseudomonas putida* KT2440 |

| | | | | | |
|---|---|---|---|---|---|
| Jasmonate | C12H18O3 | 1662 | 1289,570 | 8050 | SIGMA-ALDRICH-FLUKA |
| Laminarin | C12H22O11(C6H10O5)10 | 1663 | 3027,021 | 22080 | SIGMA-ALDRICH-FLUKA |
| L-Arabinonate | C5H10O6 | 1664 | 1229,428 | 7697 | SIGMA-ALDRICH-FLUKA |
| Lactitol dihydrate | C11H22O11 | 1665 | 1393,586 | 9010 | SIGMA-ALDRICH-FLUKA |
| L-Lactaldehyde | C3H6O2 | 1666 | 1137,376 | 6960 | SIGMA-ALDRICH-FLUKA |
| D-Lactate | C3H6O3 | 1667 | 1153,376 | 7088 | SIGMA-ALDRICH-FLUKA |
| L-Arabinitol | C5H12O5 | 1668 | 1215,444 | 7585 | SIGMA-ALDRICH-FLUKA |
| L-Arabitol | C5H12O5 | 1669 | 1215,444 | 7585 | SIGMA-ALDRICH-FLUKA |
| L-Arabonate | C5H10O6 | 1670 | 1229,428 | 9831 | SIGMA-ALDRICH-FLUKA |
| L-Lactate | C3H6O3 | 1671 | 1153,376 | 7088 | SIGMA-ALDRICH-FLUKA |
| L-Arogenate | C10H13NO5 | 1672 | 1290,514 | 8185 | As described in L.O. Zamir *et al.*, *J Biol Chem* 258, 6492 (1983) |
| L-Ascorbic acid | C6H8O6 | 1673 | 1239,423 | 7777 | SIGMA-ALDRICH-FLUKA |
| L-Leucine | C6H13NO2 | 1674 | 1210,472 | 7417 | SIGMA-ALDRICH-FLUKA |
| Itaconyl-CoA | C26H40N7O19P3S | 1675 | 1972,943 | 13406 | HPLC purification from *Arabidopsis thaliana* |
| Itaconate | C5H6O4 | 1676 | 1223,423 | 7408 | SIGMA-ALDRICH-FLUKA |
| Lactose | C12H22O11 | 1677 | 1405,597 | 9106 | SIGMA-ALDRICH-FLUKA |
| 5-Aminoimidazole | C3H5N3 | 1678 | 1146,390 | 7032 | As described by T.N. Bhat *et al.*, *J. Am. Chem. Soc* 112, 4891 (1990) |
| Leucine p-nitroaniline | C12H17N3O3 | 1679 | 1330,582 | 8121 | SIGMA-ALDRICH-FLUKA |
| L-Formylkynurenine | C11H12N2O4 | 1680 | 1329,550 | 8258 | As described by I.Y. Filippova *et al.*, *Analytical Biochemistry* 143, 293 (1984) |
| L-Gulono-1,4-lactone | C6H10O6 | 1681 | 1255,466 | 7793 | As described by Gupta *et al.*, *Analytical Biochemistry* 38, 46 (1970) |

| | | | | | |
|---|---|---|---|---|---|
| L-Leucyl-glycyl-glycine | C10H19N3O4 | 1682 | 1324,575 | 8330 | SIGMA-ALDRICH-FLUKA |
| L-Homocitrulline | C7H15N3O3 | 1683 | 1252,512 | 7881 | SIGMA-ALDRICH-FLUKA |
| L-Gulonate | C6H12O7 | 1684 | 1259,454 | 7937 | SIGMA-ALDRICH-FLUKA |
| (R)-S-Lactoylglutathione | C13H21N3O8S | 1685 | 1456,709 | 9403 | As described by D.J. Clelland and P.J. Thornalley, *J. Chem. Soc* 1, 3009 (1991) |
| Licodione | C15H12O5 | 1686 | 1351,554 | 8546 | As described by J.C. Hubaud *et al.*, *Journal of Photochemistry and Photobiology B: Biology* 92, 103 (2008) |
| L-Iditol | C6H14O6 | 1687 | 1245,471 | 7825 | As described by M. Mancera *et al.*, *Carbohydrate Research* 337, 607 (2002) |
| Limonoate | C26H34O10 | 1688 | 1569,847 | 10421 | SIGMA-ALDRICH-FLUKA |
| Limonene-1,2-diol | C10H18O2 | 1689 | 1249,549 | 7730 | SIGMA-ALDRICH-FLUKA |
| (-)-(S)-Limonene | C10H16 | 1690 | 1215,534 | 7457 | SIGMA-ALDRICH-FLUKA |
| Limonene-1,2-epoxide | C10H16O | 1691 | 1231,533 | 7585 | SIGMA-ALDRICH-FLUKA |
| Lindane | C6H6Cl6 | 1692 | 1335,683 | 8694 | SIGMA-ALDRICH-FLUKA |
| Linolenate | C18H30O2 | 1693 | 1357,732 | 8595 | SIGMA-ALDRICH-FLUKA |
| Linoleate | C18H32O2 | 1694 | 1359,748 | 8611 | SIGMA-ALDRICH-FLUKA |
| Linoleyl-CoA | C39H66N7O17P3S | 1695 | 2109,268 | 14609 | SIGMA-ALDRICH-FLUKA |
| 2,3-Bis(3-Hydroxytetradecanoyl)-D-glucosaminyl-1,6-beta-D-2,3-bis(3-hydroxytetradecanoyl)-beta-D-glucosaminyl 1-phosphate | C68H129N2O20P | 1696 | 2405,044 | 16976 | HPLC purification from *Saccharomyces cerivisiae* |
| 2,3-Bis(3-hydroxytetradecanoyl)-beta-D-glucosaminyl 1-phosphate | C34H66NO12P | 1697 | 1791,169 | 12064 | HPLC purification from *Saccharomyces cerivisiae* |
| 3-Methylsalicylaldehyde | C8H8O2 | 1698 | 1215,447 | 7457 | SIGMA-ALDRICH-FLUKA |
| L-Kynurenine | C10H12N2O3 | 1699 | 1271,514 | 8033 | SIGMA-ALDRICH-FLUKA |

| | | | | | |
|---|---|---|---|---|---|
| DL-Leucine, benzyl ester | C13H19NO2 | 1700 | 1300,597 | 8138 | SIGMA-ALDRICH-FLUKA |
| 6-Lactoyl-5,6,7,8-tetrahydropterin | C9H13N5O3 | 1701 | 1302,532 | 8282 | HPLC purification from *Arabidopsis thaliana* |
| 2-Methyl-3-oxopropanoate | C4H6O3 | 1702 | 1222,439 | 7184 | HPLC purification from *Arabidopsis thaliana* |
| 2-Methylhomogentisate | C9H10O4 | 1703 | 1261,472 | 9056 | HPLC purification from *Saccharomyces cerivisiae* |
| (+)-(R)-Limonene | C10H16 | 1704 | 1215,534 | 9506 | SIGMA-ALDRICH-FLUKA |
| L-Palmitoylcarnitine | C23H45NO4 | 1705 | 1478,911 | 9565 | SIGMA-ALDRICH-FLUKA |
| L-Rhamno-1,4-lactone | C6H10O5 | 1706 | 1225,439 | 7665 | Modified from US Patent 5210089 |
| L-Xylono-1,4-lactone | C5H8O5 | 1707 | 1211,412 | 7552 | Modified from WO/2006/005070 |
| L-Xylo-Hex-3-ulono-1,4-lactone | C6H8O6 | 1708 | 1253,450 | 7777 | Modified from WO/2006/005070 |
| L-Xylonate | C5H10O6 | 1709 | 1229,428 | 7697 | As described by A.A. Edwards *et al., Tetrahedron* 62, 4110 (2006) |
| Methyl-2-bromopropionate | C4H7BrO2 | 1710 | 1246,299 | 7704 | SIGMA-ALDRICH-FLUKA |
| Methyl 2-hydroxybenzoate | C8H8O3 | 1711 | 1231,446 | 7585 | SIGMA-ALDRICH-FLUKA |
| N-Methyl-(R,S)-1-benzyl-1,2,3,4-tetrahydroisoquinoline | C17H19N | 1712 | 1316,642 | 8266 | Method modified from Baxendale, Heterocycles 60, 2707 (2003) |
| alpha-D-Mannosyl-beta-D-mannosyl-diacetylchitobiosyldiphosphodolichol | C48H84N2O27P2(C5H8)10 | 1713 | 2943,622 | 21285 | HPLC purification from soybean |
| Methyl 2,2-dimethyl-1,3-dioxane-4-carboxylate | C10H18O4 | 1714 | 1281,547 | 6240 | As described by Y. Yao and J.J. Lalonde, *Journal of Molecular Catalysis B: Enzymatic* 22, 55 (2003) |
| L-Lysine | C6H14N2O2 | 1715 | 1209,486 | 7537 | SIGMA-ALDRICH-FLUKA |
| 3-Methylsalicylate | C8H8O3 | 1716 | 1231,446 | 7585 | SIGMA-ALDRICH-FLUKA |
| (alpha-D-Mannosyl)4-beta-D-mannosyl-diacetylchitobiosyldiphosphodolichol | C66H114N2O42P2(C5H8)10 | 1717 | 3430,049 | 15177 | HPLC purification from soybean |
| Methyl-2-hydroxy-2-methylpropionate | C5H10O3 | 1718 | 1197,429 | 7313 | SIGMA-ALDRICH-FLUKA |
| Methyl-3-bromopropionate | C4H7BrO | 1719 | 1246,299 | 7704 | SIGMA-ALDRICH-FLUKA |

| | | | | | |
|---|---|---|---|---|---|
| | 2 | | | | |
| Melibiitol | C12H24 O11 | 1720 | 1407,613 | 9122 | SIGMA-ALDRICH-FLUKA |
| Inosine 5'-monophosphate | C10H13N 4O8P | 1721 | 1425,534 | 9154 | SIGMA-ALDRICH-FLUKA |
| 3-Oxohexanoate | C6H10O 3 | 1722 | 1209,440 | 7409 | Enzymatic hydrolysis: methyl-3-oxohexanoate (Chemistry Letters, 9, p. 257, 1980) + metagenomic lipase EL1 |
| cis-1,2-Dihydroxy-4-methylcyclohexa-3,5-diene-1-carboxylate | C8H10O 4 | 1723 | 1249,461 | 7729 | HPLC purification from *Saccharomyces cerivisiae* |
| 4-Maleylacetoacetate | C8H8O6 | 1724 | 1263,445 | 7969 | HPLC purification from *Arabidopsis thaliana* |
| Methyl 4-hydroxybenzoate | C8H8O3 | 1725 | 1231,446 | 7585 | SIGMA-ALDRICH-FLUKA |
| beta-D-Mannosyldiacetylchitobiosyldiphosphodolichol | C42H74N 2O22P2( C5H8)10 | 1726 | 2781,480 | 19988 | HPLC purification from soybean |
| 2-Methylacetoacetyl-CoA | C26H42N 7O18P3S | 1727 | 1944,933 | 13294 | HPLC purification from *Saccharomyces cerivisiae* |
| 2-Methylprop-2-enoyl-CoA | C25H40N 7O17P3S | 1728 | 1914,907 | 13054 | HPLC purification from *Saccharomyces cerivisiae* |
| 3-Methylmuconolactone | C7H8O4 | 1729 | 1235,434 | 7617 | As described by Cain et al., *J Chem Soc Perkin Trans.* 1, 202 (1989) |
| Malathion | C10H19 O6PS2 | 1730 | 1409,648 | 8763 | SIGMA-ALDRICH-FLUKA |
| Malonyl-CoA | C24H38N 7O19P3S | 1731 | 1946,905 | 13197 | As described by Roughan, *Biochem J* 300, 355 (1994) |
| Maleate | C4H4O4 | 1732 | 1179,371 | 7296 | SIGMA-ALDRICH-FLUKA |
| Melilotate | C9H10O 3 | 1733 | 1245,473 | 7697 | HPLC purification from *Saccharomyces cerivisiae* |
| Maltitol | C12H24 O11 | 1734 | 1407,613 | 9350 | SIGMA-ALDRICH-FLUKA |
| Malonate | C3H4O4 | 1735 | 1183,359 | 7200 | SIGMA-ALDRICH-FLUKA |
| L-Mannuronate | C6H10O 7 | 1736 | 1287,465 | 8940 | SIGMA-ALDRICH-FLUKA |
| Methylmalonate | C4H6O4 | 1737 | 1211,412 | 7312 | SIGMA-ALDRICH-FLUKA |
| alpha-Maltose | C12H22 O11 | 1738 | 1405,597 | 9106 | SIGMA-ALDRICH-FLUKA |

| | | | | | |
|---|---|---|---|---|---|
| Maltose 6'-phosphate | C12H23O14P | 1739 | 1485,577 | 9746 | SIGMA-ALDRICH-FLUKA |
| beta-Maltose | C12H22O11 | 1740 | 1405,597 | 9106 | SIGMA-ALDRICH-FLUKA |
| Isomaltotriose | C18H32O16 | 1741 | 1567,740 | 10404 | SIGMA-ALDRICH-FLUKA |
| Isomaltotetraose | C24H42O21 | 1742 | 1729,882 | 11701 | SIGMA-ALDRICH-FLUKA |
| Isomaltopentaose | C30H52O26 | 1743 | 1892,025 | 12999 | SIGMA-ALDRICH-FLUKA |
| Isomaltohexaose | C36H62O31 | 1744 | 2054,167 | 14296 | SIGMA-ALDRICH-FLUKA |
| Methyl-3-bromo-2-methylpropionate | C5H9BrO2 | 1745 | 1260,326 | 7816 | SIGMA-ALDRICH-FLUKA |
| Methylmalonate | C4H6O4 | 1746 | 1211,412 | 7312 | SIGMA-ALDRICH-FLUKA |
| 2-Ethyl-2-methyl-4-butyrolactone | C7H12O2 | 1747 | 1207,468 | 7393 | SIGMA-ALDRICH-FLUKA |
| Mannobiose | C12H22O11 | 1748 | 1405,597 | 9106 | SIGMA-ALDRICH-FLUKA |
| (R)-Mandelate | C8H8O3 | 1749 | 1215,447 | 7585 | SIGMA-ALDRICH-FLUKA |
| (-)-Menthol | C10H20O | 1750 | 1235,565 | 7618 | SIGMA-ALDRICH-FLUKA |
| D-Mannosamine | C6H13NO5 | 1751 | 1242,470 | 7801 | SIGMA-ALDRICH-FLUKA |
| D-Mannose | C6H12O6 | 1752 | 1257,482 | 7809 | SIGMA-ALDRICH-FLUKA |
| (S)-Mandelate | C8H8O3 | 1753 | 1215,447 | 7585 | SIGMA-ALDRICH-FLUKA |
| Mannotriose | C18H32O16 | 1754 | 1567,740 | 10404 | SIGMA-ALDRICH-FLUKA |
| Mannotetraose | C24H42O21 | 1755 | 1729,882 | 11701 | SIGMA-ALDRICH-FLUKA |
| 2-Methylbutyryl-CoA | C26H44N7O17P3S | 1756 | 1930,950 | 13182 | HPLC purification from *Saccharomyces cerivisiae* |
| Malonate semialdehyde | C3H4O3 | 1757 | 1181,386 | 7072 | As described by Lassaletta *et al.*, *J Org Chem* 68, 2698 (2003) |
| 2-Methylbut-2-enoyl-CoA | C26H42N | 1758 | 1928,934 | 13166 | HPLC purification from *Saccharomyces cerivisiae* |

| | | | | | |
|---|---|---|---|---|---|
| | 7O17P3S | | | | |
| m-Cresol | C7H8O | 1759 | 1187,436 | 7233 | SIGMA-ALDRICH-FLUKA |
| Melibiose | C12H22 O11 | 1760 | 1405,597 | 9106 | SIGMA-ALDRICH-FLUKA |
| 1,2-Dihydroxy-3-methylcyclohexa-3,5-dienecarboxylate | C8H10O 4 | 1761 | 1249,461 | 7729 | HPLC purification from *Pseudomonas putida* KT2440 |
| meso-2,6-Diaminopimelate | C7H14N2 O4 | 1762 | 1283,522 | 4931 | Provided by Alfa Aesar (http://www.alfa.com/alf/laboratory_chemical_suppliers.htm) |
| D-Mannose 1-phosphate | C6H13O 9P | 1763 | 1323,435 | 5281 | SIGMA-ALDRICH-FLUKA |
| D-Mannose 6-phosphate | C6H13O 9P | 1764 | 1337,462 | 5281 | SIGMA-ALDRICH-FLUKA |
| (-)-Menthyl acetate | C12H22 O2 | 1765 | 1277,602 | 4971 | SIGMA-ALDRICH-FLUKA |
| Mannan | C66H100 NO49 | 1766 | 2754,795 | 6820 | SIGMA-ALDRICH-FLUKA |
| (-)-Menthone | C10H18 O | 1767 | 1233,549 | 4751 | SIGMA-ALDRICH-FLUKA |
| Methyl 3-hydroxybutyrate | C5H10O 3 | 1768 | 1197,429 | 4570 | SIGMA-ALDRICH-FLUKA |
| o-Methylbenzoate | C8H8O2 | 1769 | 1215,447 | 4660 | SIGMA-ALDRICH-FLUKA |
| 2-Keto-3-deoxy-6-phosphogluconate | C6H11O 9P | 1770 | 1351,445 | 5270 | As described by Fong *et al., Chemistry & Biology* 7, 873 (2000) |
| Methyl cinnamate | C10H10 O2 | 1771 | 1241,485 | 4791 | Provided by Apin Chemicals Limited (UK) |
| D-Methionine | C5H11N O2S | 1772 | 1212,505 | 4726 | SIGMA-ALDRICH-FLUKA |
| 2-Amino-2-methylpropanoate | C4H9NO 2 | 1773 | 1182,418 | 4495 | As described by Isogai *et al., Bulletin of the Chemical Society of Japan* 59, 2839-2842, (1986) |
| 5,10-Methenyltetrahydrofolate | C20H22N 7O6 | 1774 | 1549,761 | 6262 | As described by Tatum *et al., Analytical Biochemistry* 103, 255 (1980) |
| Methylmalonate | C4H6O4 | 1775 | 1211,412 | 4730 | SIGMA-ALDRICH-FLUKA |
| L-Methionine | C5H11N O2S | 1776 | 1212,505 | 4565 | SIGMA-ALDRICH-FLUKA |
| 3-Methyl-cis,cis-muconate | C7H8O4 | 1777 | 1235,434 | 4760 | SIGMA-ALDRICH-FLUKA |

| | | | | | |
|---|---|---|---|---|---|
| Benzylparaben | C14H12 O3 | 1778 | 1307,544 | 5121 | SIGMA-ALDRICH-FLUKA |
| Methylparathion | C8H10N O5PS | 1779 | 1342,502 | 5296 | SIGMA-ALDRICH-FLUKA |
| m-Methylbenzoate | C8H8O2 | 1780 | 1215,447 | 4660 | SIGMA-ALDRICH-FLUKA |
| Xanthosine | C10H12N 4O6 | 1781 | 1347,526 | 5401 | SIGMA-ALDRICH-FLUKA |
| Mevalonic acid | C6H12O 4 | 1782 | 1211,456 | 4721 | HPLC purification from *Arabidopsis thaliana* |
| Methyl jasmonate | C13H20 O3 | 1783 | 1303,597 | 5101 | SIGMA-ALDRICH-FLUKA |
| 1D-myo-Inositol 1-phosphate | C6H13O 9P | 1784 | 1337,462 | 5281 | SIGMA-ALDRICH-FLUKA |
| 3-Methylglutaconyl-CoA | C27H42N 7O19P3S | 1785 | 1972,943 | 8449 | HPLC purification from *Arabidopsis thaliana* |
| 4-Methylmuconolactone | C7H8O4 | 1786 | 1235,434 | 4760 | SIGMA-ALDRICH-FLUKA |
| N-Methyl-L-histidine | C7H11N3 O2 | 1787 | 1262,507 | 4826 | SIGMA-ALDRICH-FLUKA |
| Mevaldate | C6H10O 4 | 1788 | 1209,440 | 4710 | SIGMA-ALDRICH-FLUKA |
| 7-Methylxanthine | C6H6N4 O2 | 1789 | 1286,489 | 4810 | SIGMA-ALDRICH-FLUKA |
| Methylisocitrate | C7H10O 7 | 1790 | 1269,449 | 5011 | SIGMA-ALDRICH-FLUKA |
| Limonoate D-ring-lactone | C26H30 O8 | 1791 | 1549,816 | 6333 | SIGMA-ALDRICH-FLUKA |
| 7-Methyluric acid | C6H6N4 O3 | 1792 | 1302,488 | 4890 | SIGMA-ALDRICH-FLUKA |
| 1,7-Dimethyluric acid | C7H8N4 O3 | 1793 | 1275,462 | 4961 | SIGMA-ALDRICH-FLUKA |
| L-Malate | C4H6O5 | 1794 | 1227,412 | 4650 | SIGMA-ALDRICH-FLUKA |
| 5,10-Methylenetetrahydrofolate | C20H23N 7O6 | 1795 | 1550,769 | 6267 | As described by Tatum *et al.*, *Analytical Biochemistry* 103, 255 (1980) |
| (R)-Methylmalonyl-CoA | C25H40N 7O19P3S | 1796 | 1960,932 | 8319 | (R)-Methylmalonate + CoA + ACETYLTRANSFERASA Arabidopsis thaliana → (R)-Methylmalonyl-CoA |

| | | | | | |
|---|---|---|---|---|---|
| (S)-Methylmalonyl-CoA | C25H40N7O19P3S | 1797 | 1960,932 | 8319 | (S)-Methylmalonate + CoA + ACETYLTRANSFERASA Arabidopsis thaliana → (S)-Methylmalonyl-CoA |
| (S)-Methylmalonate semialdehyde | C4H6O3 | 1798 | 1195,413 | 4490 | Provided by Alfa Aesar (http://www.alfa.com/alf/laboratory_chemical_suppliers.htm) |
| L-Met-L-Met-L-Met | C17H32N4P5S3 | 1799 | 1636,841 | 7350 | SIGMA-ALDRICH-FLUKA |
| N-Acetylmethionine | C7H13NO3S | 1800 | 1270,542 | 4936 | SIGMA-ALDRICH-FLUKA |
| D-Mannitol | C6H14O6 | 1801 | 1245,471 | 4891 | SIGMA-ALDRICH-FLUKA |
| D-Mannitol 1-phosphate | C6H15O9P | 1802 | 1325,451 | 5291 | SIGMA-ALDRICH-FLUKA |
| Tetrahydro-1,4-oxazine | C4H9NO | 1803 | 1166,418 | 4415 | SIGMA-ALDRICH-FLUKA |
| 4-Hydroxyphenylacetate | C8H8O3 | 1804 | 1231,446 | 4740 | SIGMA-ALDRICH-FLUKA |
| 2D-5-O-methyl-2,3,5/4,6-pentahydroxycyclohexanone | C7H12O6 | 1805 | 1255,466 | 4941 | HPLC purification from *Saccharomyces cerivisiae* |
| Menaquinol | C16H18O2(C5H8)10 | 1806 | 2002,800 | 8598 | HPLC purification from *Staphylococcus aureus* |
| Menaquinone | C16H16O2(C5H8)10 | 1807 | 2000,784 | 8588 | HPLC purification from *Staphylococcus aureus* |
| 5-Methyltetrahydropteroyltri-L-glutamate | C30H39N9O12 | 1808 | 1796,989 | 7569 | HPLC purification from *Arabidopsis thaliana* |
| Methylglyoxal | C3H4O2 | 1809 | 1192,413 | 4340 | SIGMA-ALDRICH-FLUKA |
| (S)-5-Oxo-2,5-dihydrofuran-2-acetate | C6H6O4 | 1810 | 1221,407 | 4690 | Provided by Alfa Aesar (http://www.alfa.com/alf/laboratory_chemical_suppliers.htm) |
| Mucic acid | C6H10O8 | 1811 | 1287,465 | 5030 | SIGMA-ALDRICH-FLUKA |
| (R)-Mevalonate | C6H12O4 | 1812 | 1227,455 | 4721 | HPLC purification from *Arabidopsis thaliana* |
| Phenanthrene-4-carboxylate | C15H10O2 | 1813 | 1315,567 | 5086 | SIGMA-ALDRICH-FLUKA |
| N1-Acetylspermidine | C9H21N3O | 1814 | 1250,583 | 4916 | HPLC purification from *Saccharomyces cerivisiae* |

| | | | | | |
|---|---|---|---|---|---|
| cis,cis-Muconatè | C6H6O4 | 1815 | 1205,408 | 4690 | SIGMA-ALDRICH-FLUKA |
| 4alpha-Methylzymosterol | C28H46O | 1816 | 1477,969 | 5973 | Prepared as described by A. V. Baranovskii *et al.*, *Bioorg Khim* 28, 277 (2002) |
| 3-Hydroxyisovaleryl-CoA | C26H44N7O18P3S | 1817 | 1946,949 | 8319 | HPLC purification from *Saccharomyces cerivisiae* |
| N4-Acetylaminobutanal | C6H11NO2 | 1818 | 1208,456 | 4625 | As described by Liu and Minich, *J Lab Comp and Radiophar* 18, 791 (2006) |
| 2-Oxopropanoate | C3H4O3 | 1819 | 1181,386 | 4420 | Enzymatic hydrolysis: methyl-2-oxohexanoate (Tetrahedron Letters, 27, p. 1947, 1986) + metagenomic lipase EL1 |
| N1-Acetylspermine | C12H28N4O | 1820 | 1307,678 | 5202 | SIGMA-ALDRICH-FLUKA |
| 2-Nonaprenyl-4-hydroxybenzoate | C52H78O3 | 1821 | 1830,486 | 7736 | HPLC purification from *Saccharomyces cerivisiae* |
| N-Acetyl-4-O-acetylneuraminate | C13H21NO10 | 1822 | 1414,608 | 5736 | HPLC purification from *Saccharomyces cerivisiae* |
| N-Acetyl-5-methoxytryptamine | C13H16N2O2 | 1823 | 1311,579 | 5141 | As described by Szmuszkovic and Heinzelman, *J Org Chem* 25, 857 (1960) |
| Nicotinate D-ribonucleoside | C11H14NO6 | 1824 | 1319,533 | 5261 | HPLC purification from *Saccharomyces cerivisiae* |
| N-Acetylarylamine | C8H9NO | 1825 | 1214,462 | 4656 | As described in *J Synt Org Chem* 24, 3907 (2007) |
| N-Benzoyl-D-arginine-4-nitroanilide | C19H22N6O4 | 1826 | 1477,717 | 5972 | SIGMA-ALDRICH-FLUKA |
| N-Acetyl-L-aspartate | C6H9NO5 | 1827 | 1268,465 | 4855 | SIGMA-ALDRICH-FLUKA |
| Nicotinate | C6H5NO2 | 1828 | 1202,409 | 4595 | SIGMA-ALDRICH-FLUKA |
| N-Acetylgalactosamine | C8H15NO6 | 1829 | 1284,508 | 8940 | SIGMA-ALDRICH-FLUKA |
| 1,2-Anthracenediol | C14H10O2 | 1830 | 1289,529 | 5031 | SIGMA-ALDRICH-FLUKA |
| Nicotinamide adenine dinucleotide | C21H28N7O14P2 | 1831 | 1743,736 | 7302 | SIGMA-ALDRICH-FLUKA |
| 2-Naphthaldehyde | C11H8O | 1832 | 1235,480 | 4761 | SIGMA-ALDRICH-FLUKA |
| N-Acetyl-D-galactosaminoglycan | # | 1833 | # | | HPLC purification from *Arabidopsis thaliana* |
| NADH | C21H29N | 1834 | 1728,745 | 7308 | SIGMA-ALDRICH-FLUKA |

| | | | | | |
|---|---|---|---|---|---|
| | 7O14P2 | | | | |
| Nicotinamide adenine dinucleotide phosphate | C21H29N 7O17P3 | 1835 | 1807,716 | 7702 | SIGMA-ALDRICH-FLUKA |
| N-Acetylglycinamide | C4H8N2 O2 | 1836 | 1195,416 | 4560 | SIGMA-ALDRICH-FLUKA |
| N-Acetyl-D-tryptophan | C13H14N 2O3 | 1837 | 1325,562 | 5211 | SIGMA-ALDRICH-FLUKA |
| NADPH | C21H30N 7O17P3 | 1838 | 1808,724 | 7707 | SIGMA-ALDRICH-FLUKA |
| N-Acetyl-N-hydroxy-L-lysine | C8H16N2 O4 | 1839 | 1267,523 | 7790 | SIGMA-ALDRICH-FLUKA |
| N-Ribosylnicotinamide | C11H15N 2O5 | 1840 | 1318,547 | 7359 | Provided by Alfa Aesar (http://www.alfa.com/alf/laboratory_chemical_suppliers.htm) |
| N-Acetylimidazole | C5H6N2 O | 1841 | 1189,411 | 6342 | SIGMA-ALDRICH-FLUKA |
| N6-Acetyl-L-lysine | C8H16N2 O3 | 1842 | 1251,524 | 6889 | SIGMA-ALDRICH-FLUKA |
| N-Acetyl-L-phenylalanine | C11H13N O3 | 1843 | 1286,526 | 7022 | SIGMA-ALDRICH-FLUKA |
| N-Acetyl-L-histidine | C8H11N3 O3 | 1844 | 1276,490 | 6952 | SIGMA-ALDRICH-FLUKA |
| Nonadecanoic acid methyl ester | C20H40 O2 | 1845 | 1391,833 | 7760 | SIGMA-ALDRICH-FLUKA |
| Nonadecanoyldolichol | C21H35 O2(C5H8 )5C19H3 8O2 | 1846 | 2037,906 | 12283 | SIGMA-ALDRICH-FLUKA |
| 2-Naphthalenesulfonate | C10H8O 3S | 1847 | 1287,528 | 6798 | SIGMA-ALDRICH-FLUKA |
| n-Butanol | C4H10O | 1848 | 1153,419 | 6090 | SIGMA-ALDRICH-FLUKA |
| 2-Naphthalenol | C10H8O | 1849 | 1223,469 | 6581 | SIGMA-ALDRICH-FLUKA |
| N-Acetylserotonin | C12H14N 2O2 | 1850 | 1297,552 | 7099 | SIGMA-ALDRICH-FLUKA |
| N-Acetyl-L-tyrosine ethyl ester | C13H17N O4 | 1851 | 1330,579 | 7331 | SIGMA-ALDRICH-FLUKA |

| | | | | | |
|---|---|---|---|---|---|
| N-Benzoyl-4-hydroxyanthranilate | C14H11NO4 | 1852 | 1336,542 | 7372 | Provided by SYNCHEM |
| N-Benzoyl-4-methoxyanthranilate | C15H13NO4 | 1853 | 1350,569 | 7471 | Provided by SYNCHEM |
| Ethyl benzoate | C9H10O2 | 1854 | 1229,474 | 7130 | SIGMA-ALDRICH-FLUKA |
| N-Benzoylglycine | C9H9NO3 | 1855 | 1258,472 | 6826 | SIGMA-ALDRICH-FLUKA |
| cis-2-Chlorodienelactone | C6H3ClO4 | 1856 | 1267,864 | 6793 | As described by Vollmer *et al.*, *Archives Microbiology* 159, 182 (1993) |
| N-Butyryl-L-homoserine lactone | C8H13NO3 | 1857 | 1250,493 | 6770 | Provided by Cayman Chemicals (http://www.caymanchem.com/) |
| N-(3-Oxododecanoyl)homoserine lactone | C16H27NO4 | 1858 | 1376,691 | 7654 | As described by Chhabra *et al.*, *Journal of Medicinal Chemistry* 46, 97 (2003) |
| N-(3-Oxooctanoyl)homoserine lactone | C12H19NO4 | 1859 | 1320,584 | 7261 | As described by Chhabra *et al.*, *Journal of Medicinal Chemistry* 46, 97 (2003) |
| Naphthalene | C10H8 | 1860 | 1207,470 | 6469 | SIGMA-ALDRICH-FLUKA |
| Nicotinic acid amide | C6H6N2O | 1861 | 1201,422 | 6426 | SIGMA-ALDRICH-FLUKA |
| N-Carbamoyl-beta-alanine | C4H8N2O3 | 1862 | 1225,442 | 6496 | SIGMA-ALDRICH-FLUKA |
| 3,6-Dichlorocatechol | C6H4Cl2O2 | 1863 | 1242,300 | 6328 | SIGMA-ALDRICH-FLUKA |
| N-Carbamoylbeta-glycine | C3H5N2O3(CH3) | 1864 | 1225,442 | 6496 | SIGMA-ALDRICH-FLUKA |
| N-Carbamoylputrescine | C5H13N3O | 1865 | 1194,475 | 6490 | As described by Venugopal and Adiga, *Analytical Biochemistry* 104, 440 (1980) |
| 2,5-Dichloro-cis,cis-muconate | C6H4Cl2O4 | 1866 | 1304,324 | 7049 | Purified from Pseudomonas sp. as described by Spain ad Nishino, *Appl Environ Microbiol* 53, 1010 (1987) |
| N-Dodecanoyl-glycine | C3H3NO3(C12H24O2) | 1867 | 1380,680 | 7681 | As described by Pal *et al.*, *Tetrahedron* 63, 7334 (2007) |
| N-Decanoyl-glycine | C3H3NO3(C10H20O2) | 1868 | 1352,626 | 7485 | As described by Pal *et al.*, *Tetrahedron* 63, 7334 (2007) |

| | | | | | |
|---|---|---|---|---|---|
| Nerol | C10H18O | 1869 | 1233,549 | 6651 | SIGMA-ALDRICH-FLUKA |
| 1,6-Dihydroxy-cis-2,4-cyclohexadiene-1-carboxylic acid | C7H8O4 | 1870 | 1219,435 | 6665 | HPLC purification from *Pseudomonas putida* KT2440 |
| Neral | C10H16O | 1871 | 1231,533 | 6637 | SIGMA-ALDRICH-FLUKA |
| (+)-Neomenthol | C10H20O | 1872 | 1235,565 | 6665 | SIGMA-ALDRICH-FLUKA |
| Neooctane | C8H18 | 1873 | 1193,528 | 8459 | SIGMA-ALDRICH-FLUKA |
| Neotetradecane | C14H30 | 1874 | 1277,689 | 9831 | SIGMA-ALDRICH-FLUKA |
| gamma-L-Glutamylputrescine | C9H19N3O3 | 1875 | 1280,566 | 7093 | HPLC purification from *Escherichia coli* K12 |
| 7-Methylxanthosine | C11H15N4O6 | 1876 | 1362,561 | 7667 | Isolated from tea plants as described by Negishi *et al., Agriculture and Biological Chemistry* 49, 251 (1985) |
| N-Heptadecanoyl-glycine | C3H3NO3(C17H34O2) | 1877 | 1450,815 | 8172 | As described by Pal *et al., Tetrahedron* 63, 7334 (2007) |
| L-4-Hydroxyphenylglycine | C8H9NO3 | 1878 | 1230,462 | 6742 | As described by Pal *et al., Tetrahedron* 63, 7334 (2007) |
| N-Hexadecanoyl-glycine | C3H3NO3(C16H32O2) | 1879 | 1436,788 | 8074 | As described by Pal *et al., Tetrahedron* 63, 7334 (2007) |
| (3R)-3-Isoprenyl-6-oxoheptanoyl-CoA | C31H50N7O18P3S | 1880 | 2013,052 | 6221 | HPLC purification from *Saccharomyces cerivisiae* |
| N-Hexanoyl-glycine | C3H3NO3(C6H12O2) | 1881 | 1296,519 | 7092 | As described by Pal *et al., Tetrahedron* 63, 7334 (2007) |
| N-Hydroxy-L-lysine | C3H3NO3(CH3) | 1882 | 1179,394 | 6384 | As described by Pal *et al., Tetrahedron* 63, 7334 (2007) |
| N-Heptanoyl-glycine | C3H3NO3(C7H14O2) | 1883 | 1310,546 | 7190 | As described by Pal *et al., Tetrahedron* 63, 7334 (2007) |
| (3R)-3-Isopropenyl-6-oxoheptanoate | C10H16O3 | 1884 | 1263,532 | 6854 | HPLC purification from *Saccharomyces cerivisiae* |
| Nicotinate D-ribonucleotide | C11H15N | 1885 | 1429,539 | 7925 | SIGMA-ALDRICH-FLUKA |

| | | | | | |
|---|---|---|---|---|---|
| | O9P | | | | |
| Nicotine | C10H14N2 | 1886 | 1241,531 | 6707 | SIGMA-ALDRICH-FLUKA |
| Nitroglycerin | C3H5N3O9 | 1887 | 1306,384 | 7161 | SIGMA-ALDRICH-FLUKA |
| N-Methylanthranilate | C8H9NO2 | 1888 | 1230,462 | 6630 | SIGMA-ALDRICH-FLUKA |
| D-Glucose | C6H12O6 | 1889 | 1243,455 | 6833 | SIGMA-ALDRICH-FLUKA |
| N-Malonylanthranilate | C10H9NO5 | 1890 | 1302,482 | 7134 | HPLC purification from *Srhodococcus erythropolis* (van der Werf et al. (1999) Appl Environ Microbiol 65, 2092-2102) |
| N-Methylaniline | C7H9N | 1891 | 1186,452 | 6322 | SIGMA-ALDRICH-FLUKA |
| N-Methylindole | C11H14N2 | 1892 | 1253,542 | 6791 | SIGMA-ALDRICH-FLUKA |
| N-Methylhistamine | C6H11N3 | 1893 | 1273,533 | 6448 | SIGMA-ALDRICH-FLUKA |
| N-Methyl-L-glutamate | C6H11NO4 | 1894 | 1254,481 | 6252 | SIGMA-ALDRICH-FLUKA |
| Pentadecan-1-ol | C15H32O | 1895 | 1307,715 | 7171 | SIGMA-ALDRICH-FLUKA |
| N-Methylphenylethanolamine | C9H13NO | 1896 | 1230,505 | 6630 | SIGMA-ALDRICH-FLUKA |
| N-Methyltyramine | C9H13NO | 1897 | 1230,505 | 6630 | As described by Mangino *et al.*, *IARC Sci Publ* 41, 57 (1982) |
| N,N-Dimethylaniline | C8H11N | 1898 | 1200,479 | 6420 | SIGMA-ALDRICH-FLUKA |
| N-Octadecanoyl-glycine | C3H3NO3(C18H36O2) | 1899 | 1464,841 | 7288 | As described by Pal *et al.*,*Tetrahedron* 63, 7334 (2007) |
| N-Octanoyl-glycine | C3H4NO3(C8H16O2) | 1900 | 1325,581 | 7296 | As described by Pal *et al.*,*Tetrahedron* 63, 7334 (2007) |
| N-(3-Oxooctanoyl)homoserine lactone | C12H19NO4 | 1901 | 1320,584 | 7261 | As described by Chhabra *et al.*, *Journal of Medicinal Chemistry* 46, 97 (2003) |
| 2,4-Diamino-6-nitrotoluene | C7H9N3O2 | 1902 | 1230,464 | 6742 | As described by Huang *et al.*, *Appl Environ Microbiol* 66, 1474 (2000) |
| Nonadecane | C19H40 | 1903 | 1347,823 | 7451 | SIGMA-ALDRICH-FLUKA |

| | | | | | |
|---|---|---|---|---|---|
| Nonadecanoic acid | C19H38 O2 | 1904 | 1377,807 | 7661 | SIGMA-ALDRICH-FLUKA |
| Nonanoyldolichol | C21H35 O2(C5H8 )5C19H3 8O2 | 1905 | 2037,906 | 12283 | SIGMA-ALDRICH-FLUKA |
| 4-Acetamido-2-amino-6-nitrotoluene | C9H11N3 O3 | 1906 | 1272,502 | 7036 | Provided by Hallochem Pharma Co., Ltd |
| Methyl nonylate | C10H20 O2 | 1907 | 1251,565 | 6778 | SIGMA-ALDRICH-FLUKA |
| Nonanal | C9H18O | 1908 | 1221,538 | 6567 | SIGMA-ALDRICH-FLUKA |
| Nonane | C9H20 | 1909 | 1207,555 | 6469 | SIGMA-ALDRICH-FLUKA |
| 2-Hydroxy-8-methylchromene-2-carboxylate | C11H10 O4 | 1910 | 1285,494 | 7008 | HPLC purification from *Pseudomonas putida* KT2440 |
| Nonanoate | C9H18O 2 | 1911 | 1221,538 | 6679 | SIGMA-ALDRICH-FLUKA |
| Nonylphenol | C15H24 O | 1912 | 1299,651 | 8511 | SIGMA-ALDRICH-FLUKA |
| Nicotinamide mononucleotide | C11H15N 2O8P | 1913 | 1411,547 | 6781 | SIGMA-ALDRICH-FLUKA |
| Oleoylglycerone phosphate | C21H39 O7P | 1914 | 1513,807 | 7383 | SIGMA-ALDRICH-FLUKA |
| Oleic acid methyl ester | C19H36 O2 | 1915 | 1375,791 | 6555 | SIGMA-ALDRICH-FLUKA |
| all-trans-Nonaprenyl diphosphate | C45H76 O7P2 | 1916 | 1870,339 | 9523 | HPLC purification from *Saccharomyces cerivisiae* |
| Phenanthrene-3,4-oxide | C14H10 O | 1917 | 1273,529 | 5941 | SIGMA-ALDRICH-FLUKA |
| 3-Amino-2-oxopropyl phosphate | C3H8NO 5P | 1918 | 1232,371 | 5790 | HPLC purification from *Saccharomyces cerivisiae* |
| N-Succinyl-Ala-Ala-Ala p-nitroanilide | C19H25N 5O8 | 1919 | # | 9531 | SIGMA-ALDRICH-FLUKA |
| N-Succinyl-Ala-Ala-Phe-7-amido-4-methyl coumarin | C32H39N 5O8 | 1920 | # | 6221 | SIGMA-ALDRICH-FLUKA |
| N-Succinyl-Ala-Ala-Pro-Phe p- | C30H36N | 1921 | # | 9415 | SIGMA-ALDRICH-FLUKA |

| | | | | | |
|---|---|---|---|---|---|
| nitroanilide | 6O9 | | | | |
| N-Heptanoylhomoserine lactone | C11H19N O3 | 1922 | 1292,574 | 6560 | SIGMA-ALDRICH-FLUKA |
| Methyloxaloacetate | C5H6O5 | 1923 | 1239,423 | 6123 | SIGMA-ALDRICH-FLUKA |
| O-Acetylcholine | C7H16N O2 | 1924 | 1225,507 | 6124 | SIGMA-ALDRICH-FLUKA |
| Oxaloacetate | C4H4O5 | 1925 | 1225,396 | 6032 | SIGMA-ALDRICH-FLUKA |
| O-Acetyl-L-homoserine | C6H11N O4 | 1926 | 1240,454 | 6221 | SIGMA-ALDRICH-FLUKA |
| 2-Oxohex-trans-4-enoate | C6H8O3 | 1927 | 1207,424 | 6006 | Provided by eMolecules (http://www.emolecules.com/) |
| 1-Octanal | C8H16O | 1928 | 1207,511 | 6007 | SIGMA-ALDRICH-FLUKA |
| Octadecanoic acid | C18H36 O2 | 1929 | 1363,780 | 7023 | SIGMA-ALDRICH-FLUKA |
| Octadecane | C18H38 | 1930 | 1333,797 | 6828 | SIGMA-ALDRICH-FLUKA |
| Octadecenoic acid | C18H34 O2 | 1931 | 1361,764 | 7010 | SIGMA-ALDRICH-FLUKA |
| 9-Octadecynoic acid | C18H32 O2 | 1932 | 1359,748 | 6997 | SIGMA-ALDRICH-FLUKA |
| 2-Butenoic acid | C4H6O2 | 1933 | 1165,387 | 5733 | SIGMA-ALDRICH-FLUKA |
| o-Cresol | C7H8O | 1934 | 1187,436 | 5876 | SIGMA-ALDRICH-FLUKA |
| Octane | C8H18 | 1935 | 1193,528 | 5916 | SIGMA-ALDRICH-FLUKA |
| Octane-1,8-diol | C8H18O 2 | 1936 | 1225,527 | 6124 | SIGMA-ALDRICH-FLUKA |
| Stearoyl-CoA | C39H70N 7O17P3S | 1937 | 2113,300 | 9151 | SIGMA-ALDRICH-FLUKA |
| Ethyl octanoate | C10H20 O2 | 1938 | 1251,565 | 6293 | SIGMA-ALDRICH-FLUKA |
| N1-(5-Phospho-D-ribosyl)glycinamide | C7H15N2 O8P | 1939 | 1349,476 | 7593 | HPLC purification from *Saccharomyces cerivisiae* |
| L-Octanoylcarnitine | C15H29N O4 | 1940 | 1366,696 | 6651 | SIGMA-ALDRICH-FLUKA |
| GDP | C10H15N 5O11P2 | 1941 | 1506,502 | 8055 | SIGMA-ALDRICH-FLUKA |
| Octadecanoyldolichol | C21H35 O2(C5H8 | 1942 | 2023,879 | 11052 | SIGMA-ALDRICH-FLUKA |

| | | | | | |
|---|---|---|---|---|---|
| | )5C18H36O2 | | | | |
| dAMP | C10H14N5O6P | 1943 | 1394,523 | 7327 | SIGMA-ALDRICH-FLUKA |
| Octanoyldolichol | C21H35O2(C5H8)5C8H16O2 | 1944 | 1883,610 | 10805 | SIGMA-ALDRICH-FLUKA |
| all-trans-Octaprenyl diphosphate | C40H68O7P2 | 1945 | 1802,221 | 9471 | Provided by Alfa Aesar (http://www.alfa.com/alf/laboratory_chemical_suppliers.htm) |
| cis-1,2-Dihydroxy-1,2-dihydro-7-hydroxymethylnaphthalene | C11H12O3 | 1946 | 1255,512 | 6423 | HPLC purification from *Pseudomonas putida* KT2440 |
| 2-Ethylphenol | C8H10O | 1947 | 1201,463 | 5968 | SIGMA-ALDRICH-FLUKA |
| 1-Octene | C8H16 | 1948 | 1191,512 | 5903 | SIGMA-ALDRICH-FLUKA |
| 1,2-Dihydroxy-7-hydroxymethylnaphthalene | C11H10O3 | 1949 | 1253,496 | 6410 | HPLC purification from *Pseudomonas putida* KT2440 |
| 2-Oxo-3-hydroxy-4-phosphobutanoate | C4H7O8P | 1950 | 1307,392 | 6565 | HPLC purification from *Saccharomyces cerivisiae* |
| Oleyldihydroxyacetonephosphate | C4H6O7P(C18H36O2) | 1951 | 1560,840 | 6388 | Modified from US Patent 6914056 |
| 3-(5-oxo-4,5-dihydro-3H-imidazol-4-yl)propanoate | C7H8N4O4 | 1952 | 1305,488 | 6250 | HPLC purification from *Streptomyces coelicor* |
| O-Demethylpuromycin | C21H27N7O5 | 1953 | 1520,786 | 12535 | HPLC purification from *Streptomyces coelicor* |
| O-Feruloylquinate | C17H20O9 | 1954 | 1447,637 | 11643 | As described by F. Ge *et al., PLoS Biol* 3:e316 (2005) |
| Octyl salicylate | C15H22O3 | 1955 | 1329,634 | 10463 | Provided by Dayang Chemicals Co., Ltd. |
| 4-Hydroxylamino-2,6-dinitrotoluene | C7H7N3O5 | 1956 | 1292,446 | 10091 | As described by Huang *et al., Appl Environ Microbiol* 66, 1474 (2000) |
| Oleyl alcohol | C18H36O | 1957 | 1347,780 | 10645 | SIGMA-ALDRICH-FLUKA |
| Oleic acid | C18H34O2 | 1958 | 1361,764 | 10784 | SIGMA-ALDRICH-FLUKA |

| | | | | | |
|---|---|---|---|---|---|
| 3-Methyl-2-oxobutanoate | C5H8O3 | 1959 | 1195,413 | 9121 | SIGMA-ALDRICH-FLUKA |
| 2-Hydroxylamino-4,6-dinitrotoluene | C7H7N3O5 | 1960 | 1292,446 | 10091 | As described by Huang *et al.*, *Appl Environ Microbiol* 66, 1474 (2000) |
| 2-Oxopent-4-enoate | C5H6O3 | 1961 | 1193,397 | 9100 | As described by Macritchie *et al.*, *Tetrahedron:Asummetry* 8, 3895 (1997) |
| N-Propionylglycine | C3H3NO3(C3H6O2) | 1962 | 1254,438 | 9711 | SIGMA-ALDRICH-FLUKA |
| alpha-N-Phenylacetyl-L-glutamine | C13H16N2O4 | 1963 | 1343,577 | 10602 | SIGMA-ALDRICH-FLUKA |
| Pantetheine 4'-phosphate | C11H23N2O7PS | 1964 | 1437,643 | 11543 | As described by Shimizu *et al.*, *Tetrahedron* 24, 5241 (1968) |
| L-Ornithine | C5H12N2O2 | 1965 | 1195,459 | 9281 | SIGMA-ALDRICH-FLUKA |
| (9Z,12Z)-Octadecadienoyl-CoA | C39H66N7O17P3S | 1966 | 2109,268 | 18261 | SIGMA-ALDRICH-FLUKA |
| Orotidine 5'-phosphate | C10H13N2O11P | 1967 | 1461,517 | 11642 | SIGMA-ALDRICH-FLUKA |
| O-Sinapoylglucarolactone | C17H20O10 | 1968 | 1463,637 | 11803 | HPLC purification from *Pseudomonas putida* KT2440 |
| Oxalureate | C3H4N2O4 | 1969 | 1225,398 | 9280 | Provided by Dayang Chemicals Co., Ltd. |
| Oxaloglutarate | C7H8O7 | 1970 | 1297,460 | 10001 | Provided by Nextbio |
| Oxaloglycolate | C4H4O6 | 1971 | 1241,395 | 9440 | Oxaloglycerate (Furuyoshi *et al.*, *Agriculture and Biological Chemistry* 53, 2101 (1989)) + vinyl acetate + metagenomic lipase EL1 |
| Oxalosuccinate | C6H6O7 | 1972 | 1283,433 | 9861 | As described by Ducrocq *et al.*, *J Lab Comp Radiophar* 22, 61 (2006) |
| Oxalic acid | C2H2O4 | 1973 | 1183,359 | 8860 | SIGMA-ALDRICH-FLUKA |
| 4-Oxobutanoate | C4H6O3 | 1974 | 1195,413 | 8980 | As described in Chemistry Letters, 11, p. 23, 1982 |
| Oxamate | C2H3NO3 | 1975 | 1182,375 | 8850 | SIGMA-ALDRICH-FLUKA |
| Oxazole | C3H3NO | 1976 | 1148,360 | 6559 | SIGMA-ALDRICH-FLUKA |
| Doxepin | C19H21NO | 1977 | 1358,679 | 10754 | SIGMA-ALDRICH-FLUKA |
| Oxirane | C2H2OR2 | 1978 | 560,435 | 8380 | SIGMA-ALDRICH-FLUKA |
| 3-Oxopropionyl-CoA | C24H38N | 1979 | 1916,880 | 16337 | 3-Oxopropionate (As described in Chemistry Letters, 11, p. 23, 1982) + CoA + |

| | | | | | |
|---|---|---|---|---|---|
| | 7O18P3S | | | | ACETYLTRANSFERASA Arabidopsis thaliana --- 3-Oxopropionyl-CoA |
| Oxomalonate | C3H2O5 | 1980 | 1211,370 | 9140 | Diethyl 2-oxomalate (Organic Syntheses, Coll. Vol. 1, p. 266, 1941) + metagenomic lipase EL1 |
| 2-Oxoadipate | C6H8O5 | 1981 | 1253,450 | 9561 | SIGMA-ALDRICH-FLUKA |
| Phenylacetaldehyde | C8H8O | 1982 | 1199,447 | 9161 | SIGMA-ALDRICH-FLUKA |
| 4-Hydroxyphenylacetyl-CoA | C29H42N7O18P3S | 1983 | 1980,966 | 16978 | 4-Hydroxyphenylacetate (Cepanec and Litvic ARKIVOC 2008 (ii) 19-24) + CoA + ACETYLTRANSFERASA Arabidopsis thaliana → 4-Hydroxyphenylacetyl-CoA |
| Phosphatidate | C5H7O8P(C4H8O2)2 | 1984 | 1481,589 | 11983 | SIGMA-ALDRICH-FLUKA |
| Orotate | C5H4N2O4 | 1985 | 1249,420 | 9520 | SIGMA-ALDRICH-FLUKA |
| Panose | C18H32O16 | 1986 | 1567,740 | 13005 | SIGMA-ALDRICH-FLUKA |
| (R)-Pantolactone | C6H10O3 | 1987 | 1209,440 | 9261 | SIGMA-ALDRICH-FLUKA |
| (R)-Pantothenate | C9H17NO5 | 1988 | 1298,535 | 10152 | SIGMA-ALDRICH-FLUKA |
| (R)-Pantoate | C6H12O4 | 1989 | 1227,455 | 9441 | SIGMA-ALDRICH-FLUKA |
| Adenosine 2',5'-bisphosphate | C10H15N5O10P2 | 1990 | 1490,502 | 12232 | SIGMA-ALDRICH-FLUKA |
| 3'-Phosphoadenylyl sulfate | C10H15N5O13P2S | 1991 | 1570,561 | 12712 | SIGMA-ALDRICH-FLUKA |
| Parathion | C10H14NO5PS | 1992 | 1370,556 | 10552 | SIGMA-ALDRICH-FLUKA |
| 3-sn-Phosphatidylcholine | C10H18NO8P(C4H8O2)2 | 1993 | 1566,739 | 12834 | SIGMA-ALDRICH-FLUKA |
| Choline phosphate | C5H15NO4P | 1994 | 1263,449 | 9801 | SIGMA-ALDRICH-FLUKA |
| p-Cumate | C10H12O2 | 1995 | 1243,501 | 9602 | SIGMA-ALDRICH-FLUKA |
| p-Coumaryl alcohol | C9H10O2 | 1996 | 1229,474 | 9461 | SIGMA-ALDRICH-FLUKA |

| | | | | | |
|---|---|---|---|---|---|
| Pentachlorophenol | C6HCl5O | 1997 | 1329,636 | 8850 | SIGMA-ALDRICH-FLUKA |
| p-Cresol | C7H8O | 1998 | 1187,436 | 9041 | SIGMA-ALDRICH-FLUKA |
| gamma-Phenyl-epsilon-caprolactam | C12H15ON | 1999 | 1268,554 | 7402 | SIGMA-ALDRICH-FLUKA |
| Pelargonic acid | C9H18O2 | 2000 | 1237,538 | 9542 | SIGMA-ALDRICH-FLUKA |
| Palmitoyldihydroxyacetonephosphate | C4H6O7P(C16H32O2) | 2001 | 1532,787 | 12494 | SIGMA-ALDRICH-FLUKA |
| Phosphatidyl-N-dimethylethanolamine | C9H16NO8P(C4H8O2)2 | 2002 | 1552,712 | 12694 | SIGMA-ALDRICH-FLUKA |
| Pyridoxine 5'-phosphate | C8H12NO6P | 2003 | 1312,458 | 10451 | SIGMA-ALDRICH-FLUKA |
| Phenethylamine | C8H11N | 2004 | 1200,479 | 9171 | As described in JOC 22, 332-333 (1957) |
| Pectin | (C26H36O24)10 | 2005 | # | 81231 | SIGMA-ALDRICH-FLUKA |
| Pentadecanoic acid | C15H30O2 | 2006 | 1321,699 | 10384 | SIGMA-ALDRICH-FLUKA |
| p-Cymene | C10H14 | 2007 | 1213,518 | 9302 | SIGMA-ALDRICH-FLUKA |
| Pentyl butyrate | C9H18O2 | 2008 | 1237,538 | 9542 | SIGMA-ALDRICH-FLUKA |
| Phosphatidylethanolamine | C7H12NO8P(C4H8O2)2 | 2009 | 1508,658 | 12413 | SIGMA-ALDRICH-FLUKA |
| Methyl pentadecanoate | C16H32O2 | 2010 | 1335,726 | 10524 | SIGMA-ALDRICH-FLUKA |
| Benzaldehyde | C7H6O | 2011 | 1185,420 | 9261 | SIGMA-ALDRICH-FLUKA |
| 2-Aminobenzoyl-CoA | C28H41N8O17P3S | 2012 | 1949,955 | 15578 | 2-Aminobenzoate (Advanced Synthesis Technologies) + CoA + ACETYLTRANSFERASA Arabidopsis thaliana → 2-Aminobenzoyl-CoA |
| Pentyl pentanoate | C10H20O2 | 2013 | 1251,565 | 9682 | SIGMA-ALDRICH-FLUKA |
| Pentanoic acid | C5H10O2 | 2014 | 1181,430 | 8981 | SIGMA-ALDRICH-FLUKA |
| Phosphoenolpyruvate | C3H5O6 | 2015 | 1261,366 | 9330 | SIGMA-ALDRICH-FLUKA |

| | | | | | |
|---|---|---|---|---|---|
| | P | | | | |
| Pentadecane | C15H32 | 2016 | 1291,716 | 10084 | SIGMA-ALDRICH-FLUKA |
| 5-Methylhexa-2,4-dienoyl-CoA | C28H44N7O17P3S | 2017 | 1954,972 | 16718 | 5-Methylhexa-2,4-dienoate (J Am Chem Soc 96, 1133-1974) + CoA + ACETYLTRANSFERASA Arabidopsis thaliana → 5-Methylhexa-2,4-dienoyl-CoA |
| cis-2-Oxohept-3-ene-1,7-dioate | C7H7O52 | 2018 | 2002,398 | 17182 | HPLC purification from *Saccharomyces cerivisiae* |
| n-Pentane | C5H12 | 2019 | 1151,447 | 8681 | SIGMA-ALDRICH-FLUKA |
| Perdeuterobenzene | C6H6 | 2020 | 1157,410 | 8740 | As described in Bellabarba *et al.*, *Journal of Organometallic Chemistry* 640, 93 (2001) |
| Perillyl aldehyde | C10H14O | 2021 | 1229,517 | 9462 | As described by Chen and Chan, *J lab comp radiophar* 39, 369 (1997) |
| Pentacen | C22H14 | 2022 | 1357,650 | 6250 | SIGMA-ALDRICH-FLUKA |
| Perillyl alcohol | C10H16O | 2023 | 1231,533 | 9482 | SIGMA-ALDRICH-FLUKA |
| Ethanolamine phosphate | C2H8NO4P | 2024 | 1204,361 | 9370 | SIGMA-ALDRICH-FLUKA |
| p-Formyltoluene | C8H8O | 2025 | 1199,447 | 9161 | SIGMA-ALDRICH-FLUKA |
| Phenol | C6H6O | 2026 | 1173,409 | 8900 | SIGMA-ALDRICH-FLUKA |
| Phenylboronic acid | C6H7BO2 | 2027 | 1201,227 | 9071 | SIGMA-ALDRICH-FLUKA |
| Phosphatidylglycerophosphate | C8H14O13P2(C4H8O2)2 | 2028 | 1635,649 | 13523 | SIGMA-ALDRICH-FLUKA |
| dATP | C10H16N5O12P3 | 2029 | 1554,482 | 12872 | SIGMA-ALDRICH-FLUKA |
| Phenylacetyl-CoA | C29H42N7O17P3S | 2030 | 1964,967 | 16818 | SIGMA-ALDRICH-FLUKA |
| 2,4,6/3,5-Pentahydroxycyclohexanone | C6H10O6 | 2031 | 1255,466 | 9741 | As described by Husson *et al.*, *Carbohydrate Research* 307, 163 (1998) |
| L-1-Pyrroline-3-hydroxy-5-carboxylate | C5H7NO3 | 2032 | 1222,439 | 9251 | HPLC purification from *Saccharomyces cerivisiae* |
| Phenylethanolamine | C8H11NO | 2033 | 1216,478 | 9331 | SIGMA-ALDRICH-FLUKA |
| Phenethyl alcohol | C8H10O | 2034 | 1201,463 | 9181 | SIGMA-ALDRICH-FLUKA |

| | | | | | |
|---|---|---|---|---|---|
| 1-Phenylethanol | C8H10O | 2035 | 1201,463 | 9181 | SIGMA-ALDRICH-FLUKA |
| L-Phenylalanine | C9H11NO2 | 2036 | 1244,489 | 9611 | SIGMA-ALDRICH-FLUKA |
| 2-Phenylglycine | C8H9NO2 | 2037 | 1230,462 | 9471 | SIGMA-ALDRICH-FLUKA |
| Quercetin | C15H10O7 | 2038 | 1365,537 | 10982 | SIGMA-ALDRICH-FLUKA |
| Phenanthrene | C14H10 | 2039 | 1257,530 | 9742 | SIGMA-ALDRICH-FLUKA |
| Phenazine | C12H8N2O | 2040 | 1275,504 | 9922 | SIGMA-ALDRICH-FLUKA |
| Phenetole | C8H10O | 2041 | 1201,463 | 9181 | SIGMA-ALDRICH-FLUKA |
| Phosphatidylglycerol | C8H13O10P(C4H8O2)2 | 2042 | 1539,669 | 12723 | SIGMA-ALDRICH-FLUKA |
| L-Phe-Gly-Gly | C13H17N3O4 | 2043 | 1342,593 | 10753 | SIGMA-ALDRICH-FLUKA |
| Phenothiazine | C17H20N2S | 2044 | 1363,716 | 10804 | SIGMA-ALDRICH-FLUKA |
| Phloroglucinol | C6H6O3 | 2045 | 1189,409 | 9220 | SIGMA-ALDRICH-FLUKA |
| O-Phospho-L-homoserine | C4H10NO6P | 2046 | 1262,398 | 9951 | SIGMA-ALDRICH-FLUKA |
| Phosphatidylglycerol | C8H13O10PR2 | 2047 | 1511,615 | 10961 | SIGMA-ALDRICH-FLUKA |
| Phosphatidylinositol | C11H17O13P(C4H8O2)2 | 2048 | 1641,759 | 13604 | SIGMA-ALDRICH-FLUKA |
| 2-Hydroxyphenylacetate | C8H8O3 | 2049 | 1231,446 | 9481 | SIGMA-ALDRICH-FLUKA |
| 3-Hydroxyphenylacetate | C8H8O3 | 2050 | 1231,446 | 9481 | SIGMA-ALDRICH-FLUKA |
| Phenylpyruvate | C9H8O3 | 2051 | 1243,457 | 9601 | SIGMA-ALDRICH-FLUKA |
| cis-3,4-Dihydroxy-3,4-dihydrophenanthrene | C14H12O2 | 2052 | 1291,545 | 10082 | As described by Balani et al., *J. Chem. Soc. Perkin Trans.* 1, 1091 (2001) |
| cis-1,2-Dihydroxy-1,2-dihydro-8-methylnaphthalene | C11H10O2 | 2053 | 1253,496 | 9701 | Phenol + vinyl hexanoate + metagenomic lipase EL1 |
| 1,2-Dihydroxy-8-methylnaphthale | C11H10O2 | 2054 | 1253,496 | 9701 | HPLC purification from *Pseudomonas putida* KT2440 |

| | | | | | |
|---|---|---|---|---|---|
| 2,4-Dihydroxy-hept-trans-2-ene-1,7-dioate | C7H9O6 2 | 2055 | 2178,435 | 9201 | HPLC purification from *Saccharomyces cerivisiae* |
| 1-Phosphatidyl-D-myo-inositol | C11H17 O13P(C4 H8O2)2 | 2056 | 1641,759 | 13324 | HPLC purification from *Saccharomyces cerivisiae* |
| Pimelic acid | C7H12O 4 | 2057 | 1253,493 | 11323 | SIGMA-ALDRICH-FLUKA |
| O-Phospho-4-hydroxy-L-threonine | C4H10N O7P | 2058 | 1308,424 | 10111 | SIGMA-ALDRICH-FLUKA |
| 1-Phosphatidyl-1D-myo-inositol 3-phosphate | C11H18 O16P2(C 4H8O2)2 | 2059 | 1721,739 | 12164 | SIGMA-ALDRICH-FLUKA |
| 1-Phosphatidyl-1D-myo-inositol 4-phosphate | C11H18 O16P2(C 4H8O2)2 | 2060 | 1721,739 | 12164 | SIGMA-ALDRICH-FLUKA |
| Piperideine | C5H9N | 2061 | 1162,430 | 8791 | As described by Harada *et al., Tetrahedron* 26, 1579 (1970) |
| Piperazine | C4H10N2 | 2062 | 1165,433 | 8821 | As described by Subba Rao, Chemical Communication 21, 2706 (2003) |
| Piperidine | C5H11N | 2063 | 1164,446 | 8811 | SIGMA-ALDRICH-FLUKA |
| 2,6-Diamino-4-nitrotoluene | C7H9N3 O2 | 2064 | 1230,464 | 9631 | As described by Huang *et al., Appl Environ Microbiol* 66, 1474 (2000) |
| (R)-5-Phosphomevalonate | C6H13O 7P | 2065 | 1307,436 | 9931 | HPLC purification from *Arabidopsis thaliana* |
| 4-Amino-2-hydroxylamino-6-nitrotoluene | C7H9N3 O3 | 2066 | 1262,463 | 9791 | As described by Johnson *et al., Appl Environ Microbiol* 67, 5460 (2001) |
| Pimeloyl-CoA | C28H46N 7O19P3S | 2067 | 2003,013 | 17058 | HPLC purification from *Arabidopsis thaliana* |
| 2,4-Dihydroxylamino-6-nitrotoluene | C7H9N3 O4 | 2068 | 1278,462 | 9951 | As described by Johnson *et al., Appl Environ Microbiol* 67, 5460 (2001) |
| 2-Amino-4,6-dinitrotoluene | C7H7N3 O4 | 2069 | 1276,446 | 9931 | SIGMA-ALDRICH-FLUKA |
| p-Nitrophenyl alpha-D-arabinofuranoside | C11H13N O7 | 2070 | 1334,524 | 10672 | SIGMA-ALDRICH-FLUKA |
| 2-Phenyl-1,3-propanediol monocarbamate | C10H13N O3 | 2071 | 1258,516 | 9912 | SIGMA-ALDRICH-FLUKA |
| p-Nitrophenyl galactoside | C12H15N | 2072 | 1364,551 | 10972 | SIGMA-ALDRICH-FLUKA |

| | | | | | |
|---|---|---|---|---|---|
| | O8 | | | | |
| (3S)-3-Hydroxyadipyl-CoA | C27H44N 7O20P3S | 2073 | 2004,986 | 17078 | HPLC purification from *Arabidopsis thaliana* |
| beta-D-Glucosyl-2-coumarinate | C15H18 O8 | 2074 | 1389,601 | 11223 | HPLC purification from *Arabidopsis thaliana* |
| p-Nitrophenyl cellobioside | C18H25N O13 | 2075 | 1526,693 | 12594 | SIGMA-ALDRICH-FLUKA |
| p-Nitrophenyl glucopyranoside | C12H15N O8 | 2076 | 1364,551 | 10972 | SIGMA-ALDRICH-FLUKA |
| 4-Fluorocatechol | C6H5FO 2 | 2077 | 1191,400 | 9240 | SIGMA-ALDRICH-FLUKA |
| Propenoyl-CoA | C24H38N 7O17P3S | 2078 | 1900,881 | 16177 | SIGMA-ALDRICH-FLUKA |
| p-Nitrophenyl myristate | C20H31N O4 | 2079 | 1428,767 | 11455 | SIGMA-ALDRICH-FLUKA |
| 5-Hydroxyconiferyl alcohol | C10H12 O4 | 2080 | 1259,500 | 9922 | SIGMA-ALDRICH-FLUKA |
| p-Nitrophenyl phosphate | C6H6NO 6P | 2081 | 1298,387 | 10150 | SIGMA-ALDRICH-FLUKA |
| p-Nitrophenyl alpha-L-rhamnoside | C12H15N O7 | 2082 | 1364,551 | 8420 | SIGMA-ALDRICH-FLUKA |
| p-Nitrophenyl alpha-L-rhamnopyranoside | C12H15N O7 | 2083 | 1364,551 | 6250 | SIGMA-ALDRICH-FLUKA |
| Propionate | C3H6O2 | 2084 | 1153,376 | 8700 | SIGMA-ALDRICH-FLUKA |
| Propanoyl phosphate | C3H7O5 P | 2085 | 1233,356 | 9500 | SIGMA-ALDRICH-FLUKA |
| Phosphoramidate | PNH4O3 | 2086 | 1245,369 | 8480 | As described by Schultz and Gryaznov, *Tetrahedron Letters* 41, 1895 (2000) |
| 4-Hydroxystyrene | C8H8O | 2087 | 1183,448 | 9161 | SIGMA-ALDRICH-FLUKA |
| 3,5,7,3',4'-Pentahydroxyflavone | C15H10 O7 | 2088 | 1365,537 | 10982 | Provided by Xiamen Bioshining Biotechnology Co.,ltd (http://www.bioshining.net) |
| Propanoyl-CoA | C24H40N 7O17P3S | 2089 | 1902,896 | 16197 | SIGMA-ALDRICH-FLUKA |
| Prephenate | C10H10 O6 | 2090 | 1319,509 | 10221 | As described by Ramage and Macleod, *Tetrahedron* 42, 3251 (1986) |
| Guanosine 3'-diphosphate 5'- | C10H17N | 2091 | 1666,461 | 12753 | HPLC purification from *Saccharomyces cerivisiae* |

| | | | | | |
|---|---|---|---|---|---|
| diphosphate | 5O17P4 | | | | |
| Phosphatidylinositol-4,5-bisphosphate | C11H19O19P3(C4H8O2)2 | 2092 | 1803,691 | 15204 | HPLC purification from *Saccharomyces cerivisiae* |
| (R)-Diphosphomevalonate | C6H14O10P2 | 2093 | 1371,416 | 11041 | HPLC purification from *Arabidopsis thaliana* |
| Pseudouridine | C9H12N2O6 | 2094 | 1307,501 | 10402 | SIGMA-ALDRICH-FLUKA |
| 2-Hydroxy-4-hydroxymethylbenzalpyruvate | C11H10O5 | 2095 | 1285,494 | 10182 | HPLC purification from *Pseudomonas putida* KT2440 |
| all-trans-pentaprenyl diphosphate | C25H44O7P2 | 2096 | 1597,865 | 13146 | HPLC purification from *Saccharomyces cerevisiae* |
| 5'-Phosphoribosyl-N-formylglycinamidine | C8H16N3O8P | 2097 | 1376,502 | 11092 | SIGMA-ALDRICH-FLUKA |
| 5-Phospho-beta-D-ribosylamine | C5H12NO7P | 2098 | 1306,451 | 10251 | HPLC purification from *Saccharomyces cerevisiae* |
| N-(5-Phospho-D-ribosyl)anthranilate | C12H16NO9P | 2099 | 1412,532 | 11452 | HPLC purification from *Saccharomyces cerevisiae* |
| 1-(5-Phosphoribosyl)-AMP | C15H23N5O14P2 | 2100 | 1636,645 | 13554 | HPLC purification from *Saccharomyces cerevisiae* |
| 1-(5-Phosphoribosyl)-ATP | C15H25N5O20P4 | 2101 | 1796,604 | 15153 | HPLC purification from *Saccharomyces cerevisiae* |
| 1-(5'-Phosphoribosyl)-5-formamido-4-imidazolecarboxamide | C10H15N4O9P | 2102 | 1443,549 | 11622 | HPLC purification from *Saccharomyces cerevisiae* |
| 1-(5-Phosphoribosyl)-5-[(5-phosphoribosylamino)methylideneamino]imidazole-4-carboxamide | C15H25N5O15P2 | 2103 | 1654,660 | 13734 | HPLC purification from *Saccharomyces cerevisiae* |
| 5-(5-Phospho-D-ribosylaminoformimino)-1-(5-phosphoribosyl)-imidazole-4-carboxamide | C15H25N5O15P2 | 2104 | 1654,660 | 13734 | HPLC purification from *Saccharomyces cerevisiae* |
| 1-(5-Phosphoribosyl)-5-[(5-phosphoribosylamino)methylideneamino]imidazole-4-carboxamide | C15H25N5O15P2 | 2105 | 1654,660 | 13734 | HPLC purification from *Saccharomyces cerevisiae* |
| Quinate | C7H12O | 2106 | 1269,493 | 9881 | As described by Pierrer, *European Journal of Lipid Science and Technology* 104, |

| | | | | | |
|---|---|---|---|---|---|
| | 6 | | | | 394 (2002) |
| L-Proline | C5H9NO 2 | 2107 | 1194,429 | 8971 | SIGMA-ALDRICH-FLUKA |
| 1,4-Dipropoxybenzene | C12H18 O2 | 2108 | 1273,571 | 9902 | As described by Stalmach and Detert, Journal für praktische Chemie 342, 10 (2000) |
| Propionyldolichol | C21H35 O2(C5H8 )5C3H6O 2 | 2109 | 1813,476 | 15302 | HPLC purification from soybean |
| 3-Phenyl-2-propen-1-ol | C9H10O | 2110 | 1213,474 | 9301 | SIGMA-ALDRICH-FLUKA |
| L-Prolyl-AMP | C13H20N 5O7P | 2111 | 1466,629 | 9620 | HPLC purification from *Saccharomyces cerevisiae* |
| Propanoic acid | C3H6O2 | 2112 | 1153,376 | 8700 | SIGMA-ALDRICH-FLUKA |
| Propyl paraben | C10H12 O3 | 2113 | 1259,500 | 9762 | SIGMA-ALDRICH-FLUKA |
| Pentanoyl-CoA | C26H44N 7O17P3S | 2114 | 1930,950 | 16478 | SIGMA-ALDRICH-FLUKA |
| Propenoyl-CoA | C24H38N 7O17P3S | 2115 | 1900,881 | 16177 | SIGMA-ALDRICH-FLUKA |
| Protein substrate denatured 5 kDa | # | 2116 | # | # | In house (Ferrer et al., 2005 Mol Microbiol 53, 167-182) |
| Protein substrate denatured 10 kDa | # | 2117 | # | # | In house (Ferrer et al., 2005 Mol Microbiol 53, 167-182) |
| Protein substrate denatured 15 kDa | # | 2118 | # | # | In house (Ferrer et al., 2005 Mol Microbiol 53, 167-182) |
| Protein substrate denatured 20 kDa | # | 2119 | # | # | In house (Ferrer et al., 2005 Mol Microbiol 53, 167-182) |
| Protein substrate denatured 30 kDa | # | 2120 | # | # | In house (Ferrer et al., 2005 Mol Microbiol 53, 167-182) |
| Protein substrate denatured 40 kDa | # | 2121 | # | # | In house (Ferrer et al., 2005 Mol Microbiol 53, 167-182) |
| Protein substrate denatured 60 kDa | # | 2122 | # | # | In house (Ferrer et al., 2005 Mol Microbiol 53, 167-182) |
| trans-O-Hydroxybenzylidenepyruvate | C10H8O 4 | 2123 | 1285,494 | 9881 | Provided by Alfa Aesar (http://www.alfa.com/alf/laboratory_chemical_suppliers.htm) |
| 5-Phospho-alpha-D-ribose 1-diphosphate | C5H13O 14P3 | 2124 | 1467,395 | 11861 | Purified as described by Goitein and Parsons, *Anal Biochem* 87, 636 (1978) |
| Pseudocumene | C9H12 | 2125 | 1199,491 | 9161 | SIGMA-ALDRICH-FLUKA |
| Pseudouridine 5'-phosphate | C9H13N2 O9P | 2126 | 1401,508 | 11202 | SIGMA-ALDRICH-FLUKA |
| Phosphatidylserine | C8H12N | 2127 | 1494,588 | 11972 | SIGMA-ALDRICH-FLUKA |

| | | | | | |
|---|---|---|---|---|---|
| | O10P(C4 H8O2) | | | | |
| O-Phospho-L-serine | C3H8NO 6P | 2128 | 1278,397 | 9810 | SIGMA-ALDRICH-FLUKA |
| Heptylparaben | C14H20 O3 | 2129 | 1315,608 | 10323 | SIGMA-ALDRICH-FLUKA |
| 1-Phosphatidyl-1D-myo-inositol 3,4-bisphosphate | C11H19 O19P3(C 3H6O2)2 | 2130 | 1773,665 | 14924 | HPLC purification from *Saccharomyces cerevisiae* |
| 1-Phosphatidyl-D-myo-inositol 4,5-bisphosphate | C11H19 O19P3R2 | 2131 | 400,150 | 14924 | 3-Hydroxybutyrate (SIGMA-ALDRICH-FLUKA) + vinyl propanoate + metagenomic lipase EL1 |
| Phosphatidyl-N-methylethanolamine | C8H14N O8P(C3H 6O2)2 | 2132 | 1510,631 | 12133 | As described by Pajouhesh and Hancock, *Journal of Lipid Research* 25, 310 (1984) |
| 1-Phosphatidyl-D-myo-inositol | C11H17 O13P(C3 H6O2)2 | 2133 | 1613,705 | 13324 | HPLC purification from *Saccharomyces cerevisiae* |
| 1-(5-Phospho-D-ribosyl)-5-amino-4-imidazolecarboxylate | C9H14N3 O9P | 2134 | 1432,522 | 11352 | HPLC purification from *Saccharomyces cerevisiae* |
| Pteridine | C6H4N4 | 2135 | 1211,422 | 9281 | As described by Pfleiderer and Blank, *Angewandte Chemie International Edition* 7, 535 (2003) |
| Putrescine | C4H12N2 | 2136 | 1167,449 | 8841 | SIGMA-ALDRICH-FLUKA |
| Pullulan | (C36H60 O30)10 | 2137 | 10805,868 | 105266 | SIGMA-ALDRICH-FLUKA |
| Purine | C5H4N4 | 2138 | 1199,411 | 9161 | SIGMA-ALDRICH-FLUKA |
| Puromycin | C22H29N 7O5 | 2139 | 1534,813 | 12675 | SIGMA-ALDRICH-FLUKA |
| Quercitrin | C21H20 O11 | 2140 | 1527,680 | 12444 | SIGMA-ALDRICH-FLUKA |
| Pyrano[3,4-b]pyrrole | C7H7NO | 2141 | 1200,435 | 8150 | SIGMA-ALDRICH-FLUKA |
| Pyridoxal | C8H9NO 3 | 2142 | 1246,461 | 9631 | SIGMA-ALDRICH-FLUKA |
| Pyrimidine | C4H4N2 | 2143 | 1159,386 | 8760 | SIGMA-ALDRICH-FLUKA |
| Pyridoxine | C8H11N O3 | 2144 | 1232,478 | 9651 | SIGMA-ALDRICH-FLUKA |

| | | | | | |
|---|---|---|---|---|---|
| (R)-Phenyllactate | C9H10O3 | 2145 | 1245,473 | 9621 | SIGMA-ALDRICH-FLUKA |
| Pyruvate | C3H4O3 | 2146 | 1181,386 | 8840 | SIGMA-ALDRICH-FLUKA |
| Pyrazine | C4H4N2 | 2147 | 1159,386 | 8760 | SIGMA-ALDRICH-FLUKA |
| Pyrazolidine | C3H8N2 | 2148 | 1151,406 | 8680 | As described by Ahn et al., *Bioorganis & Medicinal Chemistry Letters* 15, 1337 (2005) |
| Pyrazole | C3H4N2 | 2149 | 1147,375 | 8640 | SIGMA-ALDRICH-FLUKA |
| Pyrazolo[1,5-a]pyrimidine | C5H3N4 | 2150 | 1198,403 | 7440 | SIGMA-ALDRICH-FLUKA |
| Pyrene | C16H10 | 2151 | 1281,552 | 9982 | SIGMA-ALDRICH-FLUKA |
| Pyridazine | C4H4N2 | 2152 | 1159,386 | 8760 | SIGMA-ALDRICH-FLUKA |
| Pyridine | C5H5N | 2153 | 1158,399 | 8750 | SIGMA-ALDRICH-FLUKA |
| Pyridoxal 5'-phosphate | C8H10NO6P | 2154 | 1310,442 | 10431 | SIGMA-ALDRICH-FLUKA |
| Pyrogallol | C6H6O3 | 2155 | 1205,408 | 9220 | SIGMA-ALDRICH-FLUKA |
| Pyrrole | C4H5N | 2156 | 1146,388 | 8630 | SIGMA-ALDRICH-FLUKA |
| Pyrrolidine | C4H9N | 2157 | 1150,419 | 8670 | SIGMA-ALDRICH-FLUKA |
| Ubiquinone | C14H18O4(C5H8)5 | 2158 | 1670,183 | 13869 | SIGMA-ALDRICH-FLUKA |
| Ubiquinol | C14H20O4(C5H8)5 | 2159 | 1672,199 | 13889 | SIGMA-ALDRICH-FLUKA |
| Pyridine-2,3-dicarboxylate | C7H5NO4 | 2160 | 1246,418 | 9631 | SIGMA-ALDRICH-FLUKA |
| Pyridoxamine 5'-phosphate | C8H13N2O5P | 2161 | 1311,472 | 10441 | SIGMA-ALDRICH-FLUKA |
| dUTP | C9H15N2O14P3 | 2162 | 1547,440 | 12642 | SIGMA-ALDRICH-FLUKA |
| alpha-D-Ribose 5-phosphate | C5H11O8P | 2163 | 1307,436 | 9951 | SIGMA-ALDRICH-FLUKA |
| N-(5'-Phospho-D-1'-ribulosylformimino)-5-amino-1-(5"-phospho-D-ribosyl)-4-imidazolecarboxamide | C15H25N5O15P2 | 2164 | 1640,633 | 13734 | HPLC purification from *Saccharomyces cerevisiae* |
| D-Ribose 1,5-bisphosphate | C5H12O | 2165 | 1387,416 | 10441 | SIGMA-ALDRICH-FLUKA |

| | | | | | |
|---|---|---|---|---|---|
| | 11P2 | | | | |
| alpha-D-Ribose 1-phosphate | C5H11O8P | 2166 | 1293,409 | 10261 | SIGMA-ALDRICH-FLUKA |
| Quinone | C6H4O2 | 2167 | 1187,394 | 9040 | SIGMA-ALDRICH-FLUKA |
| Quinoxaline | C8H6N2 | 2168 | 1209,445 | 9261 | SIGMA-ALDRICH-FLUKA |
| Quinuclidine | C7H13N | 2169 | 1190,484 | 9071 | SIGMA-ALDRICH-FLUKA |
| Quinolinate | C7H5NO4 | 2170 | 1246,418 | 9631 | SIGMA-ALDRICH-FLUKA |
| L-Rhamnono-1,4-lactone | C6H10O5 | 2171 | 1225,439 | 9581 | SIGMA-ALDRICH-FLUKA |
| 2,5-Dihydroxybenzoate | C7H6O4 | 2172 | 1247,445 | 9501 | SIGMA-ALDRICH-FLUKA |
| Quinoline-3,4-diol | C9H7NO2 | 2173 | 1240,457 | 9571 | SIGMA-ALDRICH-FLUKA |
| 2-Phenylbutanoic acid | C10H12O2 | 2174 | 1243,501 | 9602 | SIGMA-ALDRICH-FLUKA |
| (R)-4-Hydroxymandelate | C8H8O4 | 2175 | 1231,446 | 9641 | SIGMA-ALDRICH-FLUKA |
| Pyridoxamine | C8H12N2O2 | 2176 | 1231,492 | 9641 | SIGMA-ALDRICH-FLUKA |
| alpha-Ribazole 5'-phosphate | C14H19N2O7P | 2177 | 1437,585 | 11543 | SIGMA-ALDRICH-FLUKA |
| N1-(alpha-D-Ribosyl)-5,6-dimethylbenzimidazole | C14H18N2O4 | 2178 | 1357,604 | 10743 | HPLC purification from *Saccharomyces cerevisiae* |
| Resorufin acetate | C14H9NO4 | 2179 | 1334,526 | 10512 | SIGMA-ALDRICH-FLUKA |
| 4-(1-D-Ribitylamino)-5-amino-2,6-dihydroxypyrimidine | C9H16N4O6 | 2180 | 1339,547 | 10621 | As described by Morieux *et al., Bioorganic & Medicinal Chemistry* 16, 8968 (2008) |
| Raffinose | C18H32O16 | 2181 | 1581,767 | 13005 | SIGMA-ALDRICH-FLUKA |
| L-Ribulose | C5H10O5 | 2182 | 1213,428 | 9461 | SIGMA-ALDRICH-FLUKA |
| Resorcinol | C6H6O2 | 2183 | 1189,409 | 9060 | SIGMA-ALDRICH-FLUKA |
| Quinaldate | C10H7NO2 | 2184 | 1252,468 | 9691 | SIGMA-ALDRICH-FLUKA |
| Folate | C19H19N7O6 | 2185 | 1520,700 | 12374 | SIGMA-ALDRICH-FLUKA |

| | | | | | |
|---|---|---|---|---|---|
| Dihydroresveratrol | C14H14 O3 | 2186 | 1309,560 | 10262 | As described by Wang *et al., Journal of Chromatography B* 829, 97 (2005) |
| Retinol propionate | C23H34 O2 | 2187 | 1421,819 | 11385 | Retinol + ethyl propionate + EL1 LIPASE METAGENOMIC → retinol propionate |
| Retinoate | C20H28 O2 | 2188 | 1379,738 | 10964 | SIGMA-ALDRICH-FLUKA |
| Retinol acetate | C22H32 O2 | 2189 | 1407,792 | 11245 | Retinol + ethyl acetate + EL1 LIPASE METAGENOMIC → retinol acetate |
| Retinol butyrate | C24H38 O2 | 2190 | 1437,862 | 7290 | Retinol + ethyl butyrate + EL1 LIPASE METAGENOMIC → retinol butyrate |
| Retinal | C20H28 O | 2191 | 1363,738 | 10804 | SIGMA-ALDRICH-FLUKA |
| Retinol | C20H30 O | 2192 | 1365,754 | 10824 | SIGMA-ALDRICH-FLUKA |
| Retinol hexanoate | C26H42 O2 | 2193 | 1465,915 | 7220 | Retinol + ethyl hexanoate + EL1 LIPASE METAGENOMIC → retinol hexanoate |
| Retinol octanoate | C28H46 O2 | 2194 | 1493,969 | 9930 | Retinol + ethyl octanoate + EL1 LIPASE METAGENOMIC → retinol octanoate |
| Resorufin-beta-D-galactopyranoside | C18H17N O8 | 2195 | 1438,633 | 11713 | SIGMA-ALDRICH-FLUKA |
| Resorufin-beta-D-glucopyranoside | C18H17N O8 | 2196 | 1438,633 | 11713 | SIGMA-ALDRICH-FLUKA |
| L-Rhamnonate | C6H12O 6 | 2197 | 1257,482 | 9761 | SIGMA-ALDRICH-FLUKA |
| gamma-Glutamyl-gamma-aminobutyraldehyde | C9H16N2 O4 | 2198 | 1309,560 | 10122 | SIGMA-ALDRICH-FLUKA |
| (R)-Hydroxybutanoyl-CoA | C25H42N 7O18P3S | 2199 | 1916,923 | 16497 | (R)-Hydroxybutyrate (SIGMA-ALDRICH-FLUKA) + CoA + ACETYLTRANSFERASA Arabidopsis thaliana → (R)-Hydroxybutanoyl-CoA |
| (R)-2-Hydroxystearate | C18H36 O3 | 2200 | 1379,779 | 10965 | SIGMA-ALDRICH-FLUKA |
| (S)-Ribosyl-L-homocysteine | C9H17N O6S | 2201 | 1360,621 | 10633 | SIGMA-ALDRICH-FLUKA |
| D-Ribose | C5H10O 5 | 2202 | 1213,428 | 9461 | SIGMA-ALDRICH-FLUKA |
| (R)-3-Hydroxybutanoate | C4H8O3 | 2203 | 1197,429 | 9000 | SIGMA-ALDRICH-FLUKA |

| | | | | | |
|---|---|---|---|---|---|
| Riboflavin | C17H20N4O6 | 2204 | 1455,666 | 11724 | SIGMA-ALDRICH-FLUKA |
| Ribitol | C5H12O5 | 2205 | 1229,471 | 9481 | SIGMA-ALDRICH-FLUKA |
| L-Rhamnulose | C6H12O5 | 2206 | 1227,455 | 9601 | SIGMA-ALDRICH-FLUKA |
| L-Rhamnulose 1-phosphate | C6H13O8P | 2207 | 1307,436 | 10401 | SIGMA-ALDRICH-FLUKA |
| L-Rhamnofuranose | C6H12O5 | 2208 | 1227,455 | 6686 | SIGMA-ALDRICH-FLUKA |
| L-Rhamnose | C6H12O5 | 2209 | 1227,455 | 6686 | SIGMA-ALDRICH-FLUKA |
| Resorufin butyrate | C16H13NO4 | 2210 | 1362,580 | 7516 | SIGMA-ALDRICH-FLUKA |
| R-S-Alanylglycine | C5H9N2O3SR | 2211 | 180,161 | 6582 | SIGMA-ALDRICH-FLUKA |
| (1R,2S)-Naphthalene 1,2-oxide | C10H8O | 2212 | 1223,469 | 6547 | As described by Boyd *et al., Chem Commun* 1201 (1999) |
| D-Ribulose 5-phosphate | C5H11O8P | 2213 | 1307,436 | 7146 | SIGMA-ALDRICH-FLUKA |
| 2-Hydroxy-3-methyl benzal pyruvate | C11H10O4 | 2214 | 1285,494 | 6972 | As described in Chemistry of Heterocyclic Compounds: The Pyrazines, Volume 41 (In: Chemistry of Heterocyclic Compounds: A Series Of Monographs; Ed. G.B. Barlin; John Wiley and Sons, Inc.) (1982) |
| Sedoheptulose 1,7-bisphosphate | C7H16O13P2 | 2215 | 1447,469 | 6768 | HPLC purification from *Saccharomyces cerevisiae* |
| L-Ribulose 5-phosphate | C5H11O8P | 2216 | 1307,436 | 7955 | SIGMA-ALDRICH-FLUKA |
| cis-1,2-Dihydroxy-1,2-dihydro-7-methylnaphthalene | C11H12O2 | 2217 | 1255,512 | 7642 | HPLC purification from *Pseudomonas putida* KT2440 |
| (S)-2,3-Epoxysqualene | C30H50O | 2218 | 1506,023 | 7096 | As described by Xiao and Prestwich, *J Lab Comp Radiophar* 29, 883 (2006) |
| (S)-(+)-2-Phenyl-butyrate | C10H12O2 | 2219 | 1243,501 | 6572 | SIGMA-ALDRICH-FLUKA |
| (S)-3-Hydroxyhexanoyl-CoA | C27H46N7O18P3S | 2220 | 1960,976 | 9737 | (S)-3-Hydroxyhexanoate (SIGMA-ALDRICH-FLUKA) + CoA + ACETYLTRANSFERASA Arabidopsis thaliana → (S)-3-Hydroxyhexanoyl-CoA |
| (S)-3-Hydroxyoctanoyl-CoA | C29H50N | 2221 | 1989,030 | 9900 | (S)-3-Hydroxyoctanoate (SIGMA-ALDRICH-FLUKA) + CoA + |

| | | | | | |
|---|---|---|---|---|---|
| | 7O18P3S | | | | ACETYLTRANSFERASA Arabidopsis thaliana → (S)-3-Hydroxyoctanoyl-CoA |
| 1-(5'-Phosphoribosyl)-5-amino-4-(N-succinocarboxamide)-imidazole | C13H19N4O12P | 2222 | 1547,612 | 7239 | HPLC purification from *Saccharomyces cerevisiae* |
| 2-Carboxybenzaldehyde | C8H6O3 | 2223 | 1213,431 | 6589 | Provided by Alfa Aesar (http://www.alfa.com/alf/laboratory_chemical_suppliers.htm) |
| (+)-cis-Sabinol | C10H16O | 2224 | 1231,533 | 6603 | Provided by ISCA Technologies |
| (+)-Sabinone | C10H14O | 2225 | 1229,517 | 6589 | As described by V. Mamane, *J Am Chem Soc* 126, 8654 (2004) |
| Sedoheptulose 7-phosphate | C7H15O10P | 2226 | 1367,488 | 7564 | As described by Lee *et al., Journal of Molecular Catalysis B* 6, 369 (1999) |
| 3-Chloro-2-hydroxymuconic semialdehyde | C6H5ClO4 | 2227 | 1239,854 | 6526 | HPLC purification from *Pseudomonas putida* KT2440 |
| L-Saccharopine | C11H20N2O6 | 2228 | 1369,612 | 7468 | SIGMA-ALDRICH-FLUKA |
| (S)-4-Hydroxymandelate | C8H8O4 | 2229 | 1231,446 | 6714 | SIGMA-ALDRICH-FLUKA |
| Salicin 6-phosphate | C13H19O10P | 2230 | 1429,559 | 8769 | HPLC purification from *Arabidopsis thaliana* |
| Salicylic acid | C7H6O3 | 2231 | 1217,419 | 7047 | SIGMA-ALDRICH-FLUKA |
| Salicylaldehyde | C7H6O2 | 2232 | 1185,420 | 6926 | SIGMA-ALDRICH-FLUKA |
| Salicin | C13H18O7 | 2233 | 1349,579 | 8165 | SIGMA-ALDRICH-FLUKA |
| S-Adenosyl-(5')-3-methylthiopropylamine | C14H23N6O3S | 2234 | 1434,731 | 8687 | HPLC purification from *Saccharomyces cerevisiae* |
| N-Succinyl-2-amino-6-oxopimelate | C11H15NO8 | 2235 | 1382,566 | 8188 | HPLC purification from *Saccharomyces cerevisiae* |
| Sarcosine | C3H7NO2 | 2236 | 1209,443 | 6677 | SIGMA-ALDRICH-FLUKA |
| D-Sorbitol 6-phosphate | C6H15O9P | 2237 | 1339,478 | 7983 | SIGMA-ALDRICH-FLUKA |
| D-Sorbitol | C6H14O6 | 2238 | 1259,498 | 7380 | SIGMA-ALDRICH-FLUKA |
| L-Sorbitol | C6H14O6 | 2239 | 1259,498 | 7380 | SIGMA-ALDRICH-FLUKA |
| sec-Butylbenzene | C10H14 | 2240 | 1213,518 | 7018 | SIGMA-ALDRICH-FLUKA |

| | | | | | |
|---|---|---|---|---|---|
| O-Succinylbenzoyl-CoA | C32H44N7O20P3S | 2241 | 2051,014 | 10260 | (R)-Hydroxybutyrate (SIGMA-ALDRICH-FLUKA) + CoA + ACETYLTRANSFERASA Arabidopsis thaliana → (R)-Hydroxybutanoyl-CoA |
| Scytalone | C10H10O4 | 2242 | 1257,484 | 6746 | As described by Bell *et al.*, *Tetrahedron* 32, 1353 (1976) |
| N-Succinyl-L-2,6-diaminopimelate | C11H18N2O7 | 2243 | 1383,597 | 6304 | HPLC purification from *Saccharomyces cerevisiae* |
| 2-Hydroxy-4-hydroxymethylbenzalpyruvate | C11H10O5 | 2244 | 1285,494 | 7909 | HPLC purification from *Saccharomyces cerevisiae* |
| Sedoheptulose | C7H14O7 | 2245 | 1273,481 | 6839 | SIGMA-ALDRICH-FLUKA |
| 2-Amino-5-oxocyclohex-1-enecarbonyl-CoA | C28H43N8O18P3S | 2246 | 1983,970 | 9871 | HPLC purification from *Saccharomyces cerevisiae* |
| 1,4-Cyclohexanedione | C6H8O2 | 2247 | 1191,425 | 6270 | SIGMA-ALDRICH-FLUKA |
| L-Seryl-AMP | C13H19N6O9P | 2248 | 1497,600 | 7140 | SIGMA-ALDRICH-FLUKA |
| D-Serine | C3H7NO3 | 2249 | 1198,417 | 7229 | SIGMA-ALDRICH-FLUKA |
| L-Serine | C3H7NO3 | 2250 | 1198,417 | 7225 | SIGMA-ALDRICH-FLUKA |
| S-Formylglutathione | C11H17N3O7S | 2251 | 1398,629 | 6566 | SIGMA-ALDRICH-FLUKA |
| (S)-Hydroxybutanoyl-CoA | C25H42N7O18P3S | 2252 | 1932,922 | 9574 | (S)-Hydroxybutyrate (SIGMA-ALDRICH-FLUKA) + CoA + ACETYLTRANSFERASA Arabidopsis thaliana → (S)-Hydroxybutanoyl-CoA |
| S-Succinyldihydrolipoamide | C12H21NO4S2 | 2253 | 1386,720 | 6031 | HPLC purification from *Arabidopsis thaliana*. |
| 4-Hydroxymethylsalicylaldehyde | C8H8O3 | 2254 | 1215,447 | 6502 | HPLC purification from *Arabidopsis thaliana*. |
| Sphinganine | C18H39NO2 | 2255 | 1380,810 | 6369 | As described by Masui and Shioiri, *Tetrahedron Letters* 39, 5199 (1998) |
| Sphingenine | C18H37NO2 | 2256 | 1378,795 | 6358 | As described by Chun *et al., J Org Chem* 68, 348 (2003) |
| S-(Hydroxymethyl)glutathione | C11H19N3O7S | 2257 | 1430,672 | 6577 | As described by Okumura *et al., Bioscience, Biotechnology, and Biochemistry* 67, 434 (2003) |
| (S)-2-Hydroxyoctadecanoate | C2H3O3R | 2258 | 632,428 | 7055 | SIGMA-ALDRICH-FLUKA |

| | | | | | |
|---|---|---|---|---|---|
| Styrene cis-glycol | C8H10O2 | 2259 | 1217,463 | 741 | SIGMA-ALDRICH-FLUKA |
| Sinapate | C11H12O5 | 2260 | 1303,510 | 5921 | SIGMA-ALDRICH-FLUKA |
| Sinapoyl-CoA | C32H46N7O20P3S | 2261 | 2053,030 | 10272 | HPLC purification from *Arabidopsis thaliana*. |
| Sinapyl alcohol | C11H14O4 | 2262 | 1289,526 | 5839 | SIGMA-ALDRICH-FLUKA |
| Sinapoyl-(S)-malate | C15H16O9 | 2263 | 1419,583 | 6594 | SIGMA-ALDRICH-FLUKA |
| Sinapaldehyde | C11H12O4 | 2264 | 1287,510 | 6828 | SIGMA-ALDRICH-FLUKA |
| Sinapoyltartronate | C14H14O9 | 2265 | 1405,556 | 6513 | HPLC purification from *Arabidopsis thaliana*. |
| Shikimate | C7H10O5 | 2266 | 1267,477 | 6630 | SIGMA-ALDRICH-FLUKA |
| N-Succinyl-LL-2,6-diaminoheptanedioate | C11H18N2O7 | 2267 | 1383,597 | 6223 | SIGMA-ALDRICH-FLUKA |
| N-Succinyl-2-L-amino-6-oxoheptanedioate | C11H15NO8 | 2268 | 1382,566 | 6298 | SIGMA-ALDRICH-FLUKA |
| (S)-2-Methyl-3-oxopropanoyl-CoA | C25H40N7O19P3S | 2269 | 1987,958 | 9655 | HPLC purification from *Arabidopsis thaliana*. |
| Shikimate 3-phosphate | C7H11O8P | 2270 | 1333,430 | 6094 | As described by Sharps, *Analytical Biochemistry* 140, 183 (1984) |
| Shikimate 5-phosphate | C7H11O8P | 2271 | 1347,457 | 6094 | As described by Campbell *et al.*, *Tetrahedron* 40, 5063 (1984) |
| (S)-beta-Methylindolepyruvate | C12H11NO3 | 2272 | 1296,521 | 5880 | HPLC purification from *Saccharomyces cerevisiae* |
| (S)-Methylthioglycolate | C3H6O2S | 2273 | 1199,463 | 7049 | As described by Tong *et al.*, *Tetrahedron* 46, 3037 (1990) |
| 2-Hydroxychromene-2-carboxylate | C10H8O4 | 2274 | 1255,468 | 7735 | As described by Asenstorfer and Jones, *Tetrahedron* 63, 4788 (2007) |
| L-Sorbose | C6H12O6 | 2275 | 1274,489 | 7665 | SIGMA-ALDRICH-FLUKA |
| Sorbose 1-phosphate | C6H13O | 2276 | 1323,435 | 6129 | SIGMA-ALDRICH-FLUKA |

| | | | | | |
|---|---|---|---|---|---|
| | 9P | | | | |
| Citronellate | C10H18 O2 | 2277 | 1249,549 | 8607 | SIGMA-ALDRICH-FLUKA |
| Sphinganine 1-phosphate | C18H40N O5P | 2278 | 1460,790 | 6834 | As described by Schick *et al.*, *Tetrahedron* 51, 11207 (1995) |
| Spermidine | C7H19N3 | 2279 | 1208,545 | 8462 | SIGMA-ALDRICH-FLUKA |
| Spermine | C10H26N 4 | 2280 | 1265,640 | 8794 | SIGMA-ALDRICH-FLUKA |
| Squalene | C30H50 | 2281 | 1490,024 | 7003 | SIGMA-ALDRICH-FLUKA |
| Sphinganine | C18H39N O2 | 2282 | 1380,810 | 6369 | As described by Wright *et al.*, *Arch-Biochem-Biophys* 415, 184 (2003) |
| (1S,2R)-Naphthalene 1,2-oxide | C10H8O | 2283 | 1223,469 | 9456 | SIGMA-ALDRICH-FLUKA |
| (S)-Squalene-2,3-epoxide | C30H50 O | 2284 | 1506,023 | 7096 | As described by Xiao and Prestwich, *J Lab Comp Radiophar* 29, 883 (2006) |
| Stachyose | C24H42 O21 | 2285 | 1743,909 | 8489 | SIGMA-ALDRICH-FLUKA |
| Starch | (C12H20 O10)20 | 2286 | # | 42268 | SIGMA-ALDRICH-FLUKA |
| Stearyl heptanoate | C25H50 O2 | 2287 | 1461,968 | 6840 | SIGMA-ALDRICH-FLUKA |
| 2,5-Dichloroaniline | C6H5Cl2 N | 2288 | 1225,316 | 9560 | SIGMA-ALDRICH-FLUKA |
| (1R,4R)-Dihydrocarvone | C10H16 O | 2289 | 1231,533 | 9553 | As described by Cramarossa *et al.*, *Comptes Rendus Chemie* 8, 849 (2005) |
| (1S,4S)-Dihydrocarvone | C10H16 O | 2290 | 1231,533 | 8503 | As described by Cramarossa *et al.*, *Comptes Rendus Chemie* 8, 849 (2005) |
| Succinate semialdehyde | C4H6O3 | 2291 | 1181,386 | 8212 | SIGMA-ALDRICH-FLUKA |
| N2-Succinyl-L-arginine | C10H18N 4O5 | 2292 | 1337,574 | 6211 | SIGMA-ALDRICH-FLUKA |
| (1S,4R)-1-Hydroxy-2-oxolimonene | C10H16 O2 | 2293 | 1247,533 | 6596 | As described by van der Merf *et al.*, *Appl Environ Microbiol* 65, 2092 (1999) |
| Sucrose 6-phosphate | C12H23 O14P | 2294 | 1499,604 | 7070 | SIGMA-ALDRICH-FLUKA |
| O-Succinylbenzoate-CoA | C32H46N 7O21P3S | 2295 | 2069,029 | 6810 | 2-Succinylbenzoate (SIGMA-ALDRICH-FLUKA) + CoA + ACETYLTRANSFERASA Arabidopsis thaliana → O-Succinylbenzoate-CoA |

| | | | | | |
|---|---|---|---|---|---|
| 2-Succinylbenzoate | C11H10 O5 | 2296 | 1301,494 | 7091 | Provided by Nextbio |
| m-Tolualdehyde | C8H8O | 2297 | 1199,447 | 6380 | SIGMA-ALDRICH-FLUKA |
| o-Tolualdehyde | C8H8O | 2298 | 1199,447 | 6380 | SIGMA-ALDRICH-FLUKA |
| Hydroxycinnamoyl-CoA | C30H42N 7O18P3S | 2299 | 1992,977 | 6260 | Methyl-4-hydroxycinnamate (Chemical and Pharmaceutical Bulletin, 28, p. 3662, 1980) + metagenomic lipase EL1 + CoA + ACETYLTRANSFERASA Arabidopsis thaliana → Hydroxycinnamoyl-CoA |
| 1,3,4,6-Tetrachloro-1,4-cyclohexadiene | C6H4Cl4 | 2300 | 1297,207 | 7061 | As described by L. Trantirek *et al.*, *J Biol Chem* 276, 7734 (2001) |
| O-Succinyl-L-homoserine | C8H13N O6 | 2301 | 1282,492 | 7070 | SIGMA-ALDRICH-FLUKA |
| N2-Succinyl-L-ornithine | C9H16N2 O5 | 2302 | 1295,533 | 7161 | HPLC purification from *Saccharomyces cerevisiae* |
| Sucrose | C12H22 O11 | 2303 | 1419,624 | 7927 | SIGMA-ALDRICH-FLUKA |
| Succinic semialdehyde | C4H6O3 | 2304 | 1181,386 | 6254 | SIGMA-ALDRICH-FLUKA |
| Syringaldehyde | C9H10O 4 | 2305 | 1261,472 | 6812 | SIGMA-ALDRICH-FLUKA |
| trans-2,3-epoxysuccinate | C4H4O5 | 2306 | 1225,396 | 6463 | As described by Korn *et al.*, *Tetrahedron* 50, 8381 (1994) |
| trans-Cinnamate | C9H8O2 | 2307 | 1227,458 | 7123 | SIGMA-ALDRICH-FLUKA |
| cis,cis-2-Hydroxy-6-oxohept-2,4-dienoate | C7H8O4 | 2308 | 1235,434 | 7176 | 3-methylcatechol + metagenomic Catechol 2,3-Dioxygenase |
| D-Tagatose | C6H12O 6 | 2309 | 1243,455 | 7364 | SIGMA-ALDRICH-FLUKA |
| D-Tagatose 6-phosphate | C6H13O 9P | 2310 | 1323,435 | 7968 | SIGMA-ALDRICH-FLUKA |
| D-Tagatose 1,6-bisphosphate | C6H14O 12P2 | 2311 | 1417,442 | 8571 | SIGMA-ALDRICH-FLUKA |
| D-Tagaturonate | C6H10O 7 | 2312 | 1271,465 | 7470 | SIGMA-ALDRICH-FLUKA |
| D-Talose | C6H12O 6 | 2313 | 1243,455 | 7364 | SIGMA-ALDRICH-FLUKA |
| D-Tartrate | C4H6O6 | 2314 | 1243,411 | 7137 | SIGMA-ALDRICH-FLUKA |
| L-Tartaric acid | C4H6O6 | 2315 | 1227,412 | 7137 | SIGMA-ALDRICH-FLUKA |

| | | | | | |
|---|---|---|---|---|---|
| 2,3,4,5-Tetrachlorophenol | C6H2Cl4O | 2316 | 1326,225 | 6685 | SIGMA-ALDRICH-FLUKA |
| trans-1,2-Dihydrobenzene-1,2-diol | C6H8O2 | 2317 | 1191,425 | 6851 | As described by Shindo *et al.*, *Appl Microbiol Biotechnol* 75, 1063 (2007) |
| GMP | C10H14N5O8P | 2318 | 1426,522 | 6728 | SIGMA-ALDRICH-FLUKA |
| N-Succinyl-L-glutamate | C9H13NO7 | 2319 | 1326,502 | 6054 | SIGMA-ALDRICH-FLUKA |
| N-Succinyl-L-glutamate 5-semialdehyde | C9H13NO6 | 2320 | 1310,502 | 6961 | SIGMA-ALDRICH-FLUKA |
| Taurine | C2H7NO3S | 2321 | 1218,466 | 6346 | SIGMA-ALDRICH-FLUKA |
| 2',6,3',4'-Tetrachlorobiphenyl | C12H6Cl4 | 2322 | 1371,288 | 6314 | SIGMA-ALDRICH-FLUKA |
| 2,4,4',6-Tetrachlorobiphenyl | C12H6Cl4 | 2323 | 1371,288 | 6314 | SIGMA-ALDRICH-FLUKA |
| 2,2',5,5'-Tetrachlorobiphenyl | C12H6Cl4 | 2324 | 1371,288 | 6314 | SIGMA-ALDRICH-FLUKA |
| 2,3,4,4'-Tetrachlorobiphenyl | C12H6Cl4 | 2325 | 1372,296 | 6314 | SIGMA-ALDRICH-FLUKA |
| 2,3,4,5-Tetrachlorobiphenyl | C12H6Cl4 | 2326 | 1371,288 | 6314 | SIGMA-ALDRICH-FLUKA |
| 2,3,4,4'-Tetrachlorobiphenyl | C12H6Cl4 | 2327 | 1371,288 | 6314 | SIGMA-ALDRICH-FLUKA |
| Succinate | C4H6O4 | 2328 | 1197,385 | 6305 | SIGMA-ALDRICH-FLUKA |
| Tetrahydrothiophene | C4H8S | 2329 | 1167,464 | 6810 | SIGMA-ALDRICH-FLUKA |
| D-Threose | C4H8O4 | 2330 | 1183,402 | 7316 | SIGMA-ALDRICH-FLUKA |
| (2E)-Tetradecenoyl-CoA | C35H60N7O17P3S | 2331 | 2056,184 | 10284 | SIGMA-ALDRICH-FLUKA |
| Toluene-cis-dihydrodiol | C7H10O2 | 2332 | 1205,452 | 7351 | SIGMA-ALDRICH-FLUKA |
| 2-Hydroxy-7-hydroxymethylchromene-2-carboxylate | C11H10O5 | 2333 | 1301,494 | 7903 | HPLC purification from *Pseudomonas putida* KT2440 |
| Tetracosane | C24H50 | 2334 | 1417,958 | 6322 | SIGMA-ALDRICH-FLUKA |
| N-Tetradecanoyl-glycine | C36H31 | 2335 | 1604,944 | 7961 | As described by Pal *et al.*, *Tetrahedron* 63, 7334 (2007) |

| | | | | | |
|---|---|---|---|---|---|
| | O3N | | | | |
| Tetradecanoyldolichol | C21H35O2(C5H8)5C14H28O2 | 2336 | 1967,772 | 9776 | SIGMA-ALDRICH-FLUKA |
| Succinyl-CoA | C25H40N7O19P3S | 2337 | 1946,905 | 9655 | Succinate (SIGMA-ALDRICH-FLUKA) + CoA + ACETYLTRANSFERASA Arabidopsis thaliana → Succinyl-CoA |
| Tetrahydrofuran | C4H8O | 2338 | 1151,403 | 9038 | SIGMA-ALDRICH-FLUKA |
| trans-Tetradec-2-enoyl-CoA | C35H60N7O17P3S | 2339 | 2055,176 | 10284 | HPLC purification from *Saccharomyces cerevisiae* |
| Tetradecanoyl-CoA | C35H62N7O17P3S | 2340 | 2057,192 | 10296 | SIGMA-ALDRICH-FLUKA |
| 3',4',5,6-Tetrahydroxy-3,7-dimethoxyflavone | C17H14O8 | 2341 | 1425,590 | 6629 | Provided by Xiamen Bioshining Biotechnology Co.,ltd (http://www.bioshining.net) |
| Deoxycytidine | C9H13N3O4 | 2342 | 1306,516 | 8938 | SIGMA-ALDRICH-FLUKA |
| GTP | C10H16N5O14P3 | 2343 | 1586,481 | 7656 | SIGMA-ALDRICH-FLUKA |
| 5,6,7,8-Tetrahydrofolate | C19H23N7O6 | 2344 | 1524,731 | 7205 | SIGMA-ALDRICH-FLUKA |
| THF-L-glutamate | C24H30N8O9 | 2345 | 1653,847 | 7954 | HPLC purification from *Arabidopsis thaliana*. |
| Thymidine | C10H14N2O5 | 2346 | 1321,528 | 6025 | SIGMA-ALDRICH-FLUKA |
| 1,3-Thiazole | C3H3NS | 2347 | 1164,421 | 6136 | SIGMA-ALDRICH-FLUKA |
| Thiomorpholine | C4H9NS | 2348 | 1182,479 | 6262 | SIGMA-ALDRICH-FLUKA |
| Thiophene | C4H4S | 2349 | 1163,433 | 6129 | SIGMA-ALDRICH-FLUKA |
| Thiamin monophosphate | C12H18N4O4PS | 2350 | 1424,630 | 7948 | SIGMA-ALDRICH-FLUKA |
| Thiamin | C12H17N4OS | 2351 | 1344,650 | 7168 | SIGMA-ALDRICH-FLUKA |
| Thiamine diphosphate | C12H19N4O7P2S | 2352 | 1504,611 | 8506 | SIGMA-ALDRICH-FLUKA |
| 3-Ethylphenol | C8H10O | 2353 | 1201,463 | 6394 | SIGMA-ALDRICH-FLUKA |
| dUDP | C9H14N2 | 2354 | 1467,461 | 8934 | SIGMA-ALDRICH-FLUKA |

| | | | | | |
|---|---|---|---|---|---|
| | O11P2 | | | | |
| 1,3,6,8-Tetrahydroxynaphthalene | C10H8O4 | 2355 | 1271,467 | 7455 | As described by Fujii et al., *Biochemistry* 39, 8853 (2000) |
| Guanine | C5H5N5O | 2356 | 1214,426 | 7145 | SIGMA-ALDRICH-FLUKA |
| L-Threonine | C4H9NO3 | 2357 | 1182,418 | 6904 | SIGMA-ALDRICH-FLUKA |
| L-Threonate | C4H8O5 | 2358 | 1199,401 | 7032 | SIGMA-ALDRICH-FLUKA |
| alpha-Tocopherol | C29H50O2 | 2359 | 1510,012 | 9255 | SIGMA-ALDRICH-FLUKA |
| 5,7,3',4'-Tetrahydroxyflavone | C15H10O6 | 2360 | 1365,537 | 8165 | Provided by Xiamen Bioshining Biotechnology Co.,ltd (http://www.bioshining.net) |
| Thiepin | C6H12S | 2361 | 1195,518 | 8448 | As described by Hofmann and Westernacher, *Angewandte Chemie International Edition* 5, 958 (2003) |
| 3-Hydroxy-3-isohexeneylglutaryl-CoA | C32H52N7O20P3S | 2362 | 2059,078 | 10301 | HPLC purification from *Arabidopsis thaliana*. |
| 2-Chlorohexane | C6H13Cl | 2363 | 1199,919 | 7319 | SIGMA-ALDRICH-FLUKA |
| N,N,N-Trimethyl-L-histidine | C9H15N3O2 | 2364 | 1290,561 | 6540 | SIGMA-ALDRICH-FLUKA |
| 2,4,6-Trinitrotoluene | C7H5N3O6 | 2365 | 1347,482 | 7125 | SIGMA-ALDRICH-FLUKA |
| Thymine | C5H6N2O2 | 2366 | 1205,411 | 6421 | SIGMA-ALDRICH-FLUKA |
| gamma-Tocopherol | C28H48O2 | 2367 | 1495,985 | 8446 | SIGMA-ALDRICH-FLUKA |
| Toluene-4-sulfonate | C7H8O3S | 2368 | 1251,495 | 6742 | SIGMA-ALDRICH-FLUKA |
| Toluene | C7H8 | 2369 | 1171,437 | 6185 | SIGMA-ALDRICH-FLUKA |
| 4-Ethylphenol | C8H10O | 2370 | 1201,463 | 6394 | SIGMA-ALDRICH-FLUKA |
| D-myo-Inositol 1,4,5-trisphosphate | C6H15O15P3 | 2371 | 1483,394 | 8469 | SIGMA-ALDRICH-FLUKA |
| 1-Bromohexane | C6H13Br | 2372 | 1244,370 | 7251 | SIGMA-ALDRICH-FLUKA |
| 1-Chlorohexane | C6H13Cl | 2373 | 1199,919 | 6915 | SIGMA-ALDRICH-FLUKA |
| 7-Methyl-3-oxo-6-octenoyl-CoA | C30H48N7O18P3S | 2374 | 1999,025 | 12945 | HPLC purification from *Arabidopsis thaliana*. |

| | | | | | |
|---|---|---|---|---|---|
| 1,2-Dibromohexane | C6H12Br 2 | 2375 | 1323,266 | 9860 | SIGMA-ALDRICH-FLUKA |
| Oxidized thioredoxin | C6H7NO 2S2(Prot ein)2 | 2376 | 1268,543 | # | SIGMA-ALDRICH-FLUKA |
| Reduced thioredoxin | C6H9NO 2S2(Prot ein(2) | 2377 | 1270,559 | # | SIGMA-ALDRICH-FLUKA |
| Trehalose | C12H22 O11 | 2378 | 1405,597 | 6606 | SIGMA-ALDRICH-FLUKA |
| Trehalose 6-phosphate | C12H23 O14P | 2379 | 1485,577 | 7070 | SIGMA-ALDRICH-FLUKA |
| Benzoylformate | C8H6O3 | 2380 | 1229,430 | 5491 | As described by Shozo *et al., J Org Chem* 37, 3174 (1972) |
| Triacetin | C6H5O6( C2H4O2) 3 | 2381 | 1432,557 | 6670 | SIGMA-ALDRICH-FLUKA |
| Glyceryl tributyrate | C15H26 O6 | 2382 | 1381,664 | 6374 | SIGMA-ALDRICH-FLUKA |
| Isohexenyl-glutaconyl-CoA | C32H50N 7O19P3S | 2383 | 2041,062 | 10202 | HPLC purification from *Arabidopsis thalian* |
| Citronellyl-CoA | C31H52N 7O17P3S | 2384 | 1999,069 | 9958 | HPLC purification from *Arabidopsis thalian* |
| Glyceryl tridecanoate | C6H5O6( C10H20 O2)3 | 2385 | 1769,202 | 8623 | SIGMA-ALDRICH-FLUKA |
| Glyceryl tridodecanoate | C6H5O6( C12H24 O2)3 | 2386 | 1853,363 | 6749 | SIGMA-ALDRICH-FLUKA |
| 3-Fluoro-cis,cis-muconate | C6H5FO 4 | 2387 | 1239,398 | 6658 | See Natarajan et al., *Journal of Fluorine Chemistry* 126, 424 (2004) |
| 3-Fluorocatechol | C6H5FO 2 | 2388 | 1207,400 | 6435 | Provided by Maybridge |
| 4,5,6-Trinitrotriazine | C3N6O6 | 2389 | 1323,420 | 6463 | As described by A.A. Korkin and R.J. Bartlett, *J Am Chem Soc* 118, 12244 (1996) |
| Glyceryl trihexanoate | C6H5O6( C6H12O | 2390 | 1600,879 | 7646 | SIGMA-ALDRICH-FLUKA |

| | | | | | |
|---|---|---|---|---|---|
| | 2)3 | | | | |
| Glyceryl trioctanoate | C6H5O6(C8H16O2)3 | 2391 | 1685,041 | 8135 | SIGMA-ALDRICH-FLUKA |
| Glycerol trioleate | C6H5O6(C18H36O2)3 | 2392 | 2105,847 | 10577 | SIGMA-ALDRICH-FLUKA |
| 1,4-Lactone | C4H6O2 | 2393 | 1165,387 | 7119 | SIGMA-ALDRICH-FLUKA |
| Glyceryl trihexadecanoate | C6H5O6(C16H32O2)3 | 2394 | 2021,686 | 10089 | SIGMA-ALDRICH-FLUKA |
| Glyceryl tripropionate | C6H5O6(C3H6O2)3 | 2395 | 1474,637 | 6914 | SIGMA-ALDRICH-FLUKA |
| Tropate | C9H10O3 | 2396 | 1245,473 | 7584 | SIGMA-ALDRICH-FLUKA |
| Glyceryl tritetradecanoate | C6H5O6(C14H28O2)3 | 2397 | 1937,524 | 9600 | SIGMA-ALDRICH-FLUKA |
| Tropine | C8H15NO | 2398 | 1220,510 | 8439 | SIGMA-ALDRICH-FLUKA |
| dUMP | C9H13N2O8P | 2399 | 1387,481 | 6408 | SIGMA-ALDRICH-FLUKA |
| L-Tryptophan | C11H12N2O2 | 2400 | 1283,525 | 8805 | SIGMA-ALDRICH-FLUKA |
| D-Tryptophan | C11H12N2O2 | 2401 | 1283,525 | 10058 | SIGMA-ALDRICH-FLUKA |
| Tryptamine | C10H12N2 | 2402 | 1239,515 | 9455 | SIGMA-ALDRICH-FLUKA |
| 1-Methyl-1-phenylethylene | C9H10 | 2403 | 1197,475 | 6305 | Modified from US Patent 6639083 |
| Tetracosanoic acid | C24H48O2 | 2404 | 1447,941 | 6759 | SIGMA-ALDRICH-FLUKA |
| Tetracosanoyl-CoA | C45H82N7O17P3S | 2405 | 2197,461 | 11110 | Tetracosanoate (SIGMA-ALDRICH-FLUKA) + CoA + ACETYLTRANSFERASA Arabidopsis thaliana → Tetracosanoyl-CoA |
| Tylactone | C23H38 | 2406 | 1473,849 | 6909 | HPLC purification from *Streptomyces fradiae* GS14 |

| | | | | | |
|---|---|---|---|---|---|
| | O5 | | | | |
| Tetradecane | C14H30 | 2407 | 1277,689 | 7009 | SIGMA-ALDRICH-FLUKA |
| Methyl tetradecenoate | C15H28 O2 | 2408 | 1319,683 | 7134 | (9Z)-Tetradecenoic acid + EL1 LIPASE METAGENOMIC → Methyl tetradecenoate |
| (9Z)-Tetradecenoic acid | C14H26 O2 | 2409 | 1305,656 | 7120 | SIGMA-ALDRICH-FLUKA |
| Tetradecanoate | C14H28 O2 | 2410 | 1307,672 | 7134 | SIGMA-ALDRICH-FLUKA |
| UDP-2,3-bis(3-hydroxytetradecanoyl)glucosamine | C43H77N 3O20P2 | 2411 | 2097,337 | 10529 | HPLC purification from *Pseudomonas putida KT2440* |
| L-Tyrosine | C9H11N O3 | 2412 | 1260,488 | 6671 | SIGMA-ALDRICH-FLUKA |
| Tyramine | C8H11N O | 2413 | 1216,478 | 7415 | SIGMA-ALDRICH-FLUKA |
| UDP-3-O-(3-hydroxytetradecanoyl)-N-acetylglucosamine | C31H53N 3O19P2 | 2414 | 1913,015 | 9459 | HPLC purification from *Pseudomonas putida KT2440* |
| UDP-3-O-(3-hydroxytetradecanoyl)-D-glucosamine | C29H55N 3O20P2 | 2415 | 1907,009 | 9164 | HPLC purification from *Pseudomonas putida KT2440* |
| UDP-N-acetyl-2-amino-2-deoxy-D-glucuronate | C17H25N 3O18P2 | 2416 | 1714,666 | 8226 | HPLC purification from *Pseudomonas putida KT2440* |
| UDP-N-Acetyl-3-O-(1-carboxyvinyl)-D-glucosamine | C20H29N 3O19P2 | 2417 | 1740,704 | 8551 | HPLC purification from *Pseudomonas putida KT2440* |
| Undecaprenyl-diphospho-N-acetylmuramoyl-(N-acetylglucosamine)-L-alanyl-D-glutamyl-meso-2,6-diaminopimeloyl-D-alanyl-D-alanine | C95H156 N8O28P2 | 2418 | 2999,565 | 15766 | HPLC purification from *Pseudomonas putida KT2440* |
| UDP-N-Acetyl-D-glucosamine | C17H27N 3O17P2 | 2419 | 1670,656 | 8145 | HPLC purification from *Pseudomonas putida KT2440* |
| UDP-N-Acetyl-D-mannosamine | C17H27N 3O17P2 | 2420 | 1670,656 | 8145 | HPLC purification from *Pseudomonas putida KT2440* |
| UDP-N-Acetyl-D-mannosaminouronate | C17H25N 3O18P2 | 2421 | 1714,666 | 8226 | HPLC purification from *Pseudomonas putida KT2440* |
| Undecaprenyl-diphospho-N-acetylmuramoyl-L-alanyl-D- | C87H143 N7O23P2 | 2422 | 2796,370 | 14586 | HPLC purification from *Pseudomonas putida KT2440* |

| | | | | | |
|---|---|---|---|---|---|
| glutamyl-meso-2,6-diaminopimeloyl-D-alanyl-D-alanine | | | | | |
| UDP-N-Acetylmuramoyl-L-alanine | C23H36N4O20P2 | 2423 | 1844,833 | 8976 | HPLC purification from *Pseudomonas putida KT2440* |
| UDP-N-Acetylmuramoyl-L-alanyl-D-glutamate | C28H43N5O23P2 | 2424 | 1942,915 | 9725 | HPLC purification from *Pseudomonas putida KT2440* |
| UDP-N-Acetylmuramate | C20H31N3O19P2 | 2425 | 1742,720 | 8563 | SIGMA-ALDRICH-FLUKA |
| Uridine 5'-diphosphate | C9H14N2O12P2 | 2426 | 1483,461 | 6965 | SIGMA-ALDRICH-FLUKA |
| Undecaprenyl phosphate | C55H91O4P | 2427 | 1926,595 | 9538 | HPLC purification from *Saccharomyces cerevisiae* |
| Undecaprenyl diphosphate | C55H92O7P2 | 2428 | 2006,576 | 10002 | HPLC purification from *Saccharomyces cerevisiae* |
| UDP-D-glucose | C15H24N2O17P2 | 2429 | 1645,603 | 7906 | SIGMA-ALDRICH-FLUKA |
| Methylitaconate | C6H8O4 | 2430 | 1223,423 | 6545 | As described by Ciceri *et al.*, *Helvetica Chimica Acta* 83, 2550 (2000) |
| UDP-D-galactose | C15H24N2O17P2 | 2431 | 1629,603 | 7906 | SIGMA-ALDRICH-FLUKA |
| UDP-D-Galacto-1,4-furanose | C15H24N2O17P2 | 2432 | 1629,603 | 7906 | HPLC purification from *Saccharomyces cerevisiae* |
| UDP-D-xylose | C14H22N2O16P2 | 2433 | 1630,611 | 7732 | UDP+ xylose + UDP-XYLOSYLTRANSEFERASE METAGENOMIC → UDP-D-Xylose |
| UDP-D-glucuronate | C15H22N2O18P2 | 2434 | 1657,614 | 7988 | HPLC purification from *Saccharomyces cerevisiae* |
| UDP-3-Ketoglucose | C15H22N2O17P2 | 2435 | 1627,587 | 7557 | HPLC purification from *Saccharomyces cerevisiae* |
| UDP-L-Rhamnose | C15H24N2O16P2 | 2436 | 1644,638 | 7480 | UDP+ rhamnose + UDP-RHAMNOSYLTRANSEFERASE METAGENOMIC → UDP-L-Rhamnose |
| UDP-N-Acetyl-D-galactosamine | C17H27N3O17P2 | 2437 | 1670,656 | 7797 | SIGMA-ALDRICH-FLUKA |
| UDP-N-acetylmuramoyl-L-alanyl-D-gamma-glutamyl-meso-2,6-diaminopimelate | C35H55N7O26P2 | 2438 | 2115,099 | 10266 | HPLC purification from *Saccharomyces cerevisiae* |
| UDP-N-acetylmuramoyl-L-alanyl-D- | C41H65N | 2439 | 2287,282 | 11056 | HPLC purification from *Saccharomyces cerevisiae* |

| | | | | | |
|---|---|---|---|---|---|
| glutamyl-meso-2,6-diaminopimeloyl-D-alanyl-D-alanine | 9O28P2 | | | | |
| UMP | C9H13N2 O9P | 2440 | 1403,480 | 6051 | SIGMA-ALDRICH-FLUKA |
| 2,3-Didehydro-pimeloyl-CoA | C28H44N 7O19P3S | 2441 | 1986,970 | 9465 | HPLC purification from *Saccharomyces cerevisiae* |
| 3-Oxopimeloyl-CoA | C28H44N 7O20P3S | 2442 | 2002,970 | 9554 | HPLC purification from *Saccharomyces cerevisiae* |
| Undecanoic acid | C11H22 O2 | 2443 | 1265,591 | 5457 | SIGMA-ALDRICH-FLUKA |
| 3-Hydroxy-5-oxohexanoyl-CoA | C27H44N 7O19P3S | 2444 | 1974,959 | 9399 | HPLC purification from *Saccharomyces cerevisiae* |
| 4-Hydroxy-2-oxovalerate | C5H8O4 | 2445 | 1211,412 | 5156 | As described by Macritchie *et al, Tetrahedron:Asummetry* 8, 3895 (1997) |
| Cyclohexaamylose | C36H60 O30 | 2446 | 2036,151 | 9828 | SIGMA-ALDRICH-FLUKA |
| Uracil | C4H4N2 O2 | 2447 | 1191,385 | 5045 | SIGMA-ALDRICH-FLUKA |
| 5-Ureido-4-imidazole carboxylate | C5H6N4 O3 | 2448 | 1263,451 | 5367 | HPLC purification from *Saccharomyces cerevisiae* |
| 3',5'-Cyclic IMP | C10H11N 4O7P | 2449 | 1409,491 | 6023 | HPLC purification from *Saccharomyces cerevisiae* |
| Urea-1-carboxylate | C2H4N2 O3 | 2450 | 1197,388 | 6000 | SIGMA-ALDRICH-FLUKA |
| Butanoyl phosphate | C4H9O5 P | 2451 | 1247,383 | 6427 | 2-Butanoate + metagenomic pyruvate decarboxylase |
| Urocanate | C6H6N2 O2 | 2452 | 1217,422 | 6227 | Histidine + HISTIDINE AMMONIA-LYASE METAGENOMIC → Urocanate |
| 2-Oxovalerate | C5H8O3 | 2453 | 1195,413 | 6080 | SIGMA-ALDRICH-FLUKA |
| Cyclopentanol | C5H10O | 2454 | 1165,430 | 5880 | SIGMA-ALDRICH-FLUKA |
| p-Nitrophenol octanoate | C14H21 O4N | 2455 | 1346,622 | 5306 | SIGMA-ALDRICH-FLUKA |
| Vanillin | C8H8O3 | 2456 | 1231,446 | 6321 | SIGMA-ALDRICH-FLUKA |
| L-Valine | C5H11N O2 | 2457 | 1196,445 | 6594 | SIGMA-ALDRICH-FLUKA |
| Vanillate | C8H8O4 | 2458 | 1247,445 | 6963 | SIGMA-ALDRICH-FLUKA |

| | | | | | |
|---|---|---|---|---|---|
| Neuraminic acid | C9H17N O8 | 2459 | 1330,534 | 7679 | Pyruvic acid and D-mannosamine (ALDOL-CONDENSATION) → Neuraminic acid |
| delta-Valerolactone | C5H8O2 | 2460 | 1179,414 | 6471 | SIGMA-ALDRICH-FLUKA |
| gamma-Undecalactone | C11H20 O2 | 2461 | 1263,576 | 7079 | SIGMA-ALDRICH-FLUKA |
| 5-Methyl-3-oxo-4-hexenoyl-CoA | C28H44N 7O18P3S | 2462 | 1970,971 | 12190 | SIGMA-ALDRICH-FLUKA |
| Vermelone | C10H10 O3 | 2463 | 1257,484 | 7035 | HPLC purification from *Curvularia lunata* |
| 1,3,7-Trimethylxanthine | C8H10N4 O2 | 2464 | 1273,490 | 7151 | SIGMA-ALDRICH-FLUKA |
| Methyl salicylate | C8H8O3 | 2465 | 1231,446 | 6847 | SIGMA-ALDRICH-FLUKA |
| Xanthosine 5'-phosphate | C10H13N 4O9P | 2466 | 1427,506 | 8379 | SIGMA-ALDRICH-FLUKA |
| o-Xylene | C8H10 | 2467 | 1185,464 | 6515 | SIGMA-ALDRICH-FLUKA |
| Xanthene | C13H10 O | 2468 | 1261,518 | 7065 | SIGMA-ALDRICH-FLUKA |
| o-Xylene | C8H10 | 2469 | 1185,464 | 6515 | SIGMA-ALDRICH-FLUKA |
| L-Xylulose 1-phosphate | C5H11O 8P | 2470 | 1293,409 | 7411 | SIGMA-ALDRICH-FLUKA |
| 1,3-beta-D-Xylan | (C5H8O5 )20 | 2471 | 4039,634 | 10883 | SIGMA-ALDRICH-FLUKA |
| L-Xylulose 5-phosphate | C5H11O 8P | 2472 | 1324,443 | 6840 | SIGMA-ALDRICH-FLUKA |
| D-Xylulose 5-phosphate | C5H11O 8P | 2473 | 1324,443 | 6840 | SIGMA-ALDRICH-FLUKA |
| D-Xylose | C5H10O 5 | 2474 | 1244,463 | 6307 | SIGMA-ALDRICH-FLUKA |
| Chloroxylenol | C8H9ClO | 2475 | 1235,908 | 6114 | SIGMA-ALDRICH-FLUKA |
| m-Xylene | C8H10 | 2476 | 1185,464 | 6014 | SIGMA-ALDRICH-FLUKA |
| L-Xylose | C5H10O 5 | 2477 | 1213,428 | 6307 | SIGMA-ALDRICH-FLUKA |
| Xyloglucan | (C5H10O 5)20 | 2478 | # | 5306 | SIGMA-ALDRICH-FLUKA |

| | | | | | |
|---|---|---|---|---|---|
| p-Xylene | C8H10 | 2479 | 1185,464 | 6515 | SIGMA-ALDRICH-FLUKA |
| Xylitol | C5H12O 5 | 2480 | 1215,444 | 6847 | SIGMA-ALDRICH-FLUKA |
| Xylitol 5-phosphate | C5H13O 8P | 2481 | 1326,459 | 7425 | SIGMA-ALDRICH-FLUKA |
| L-Xylulose | C5H10O 5 | 2482 | 1213,428 | 6833 | SIGMA-ALDRICH-FLUKA |
| Zymosterol | C27H44 O | 2483 | 1463,942 | 6559 | Prepared as described by A. V. Baranovskii *et al.*, *Bioorg Khim* 28, 277 (2002) |
| | | | | | |

| N° | Substrate | Chemical Structure | N° | Substrate | Chemical Structure |
|---|---|---|---|---|---|
| 1 | γ-Nonalactone | 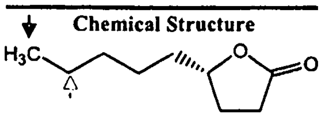 | 16 | 1,2,3-Oxadiazole | 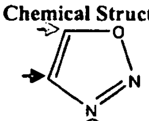 |
| 2 | ε-Caprolactone | 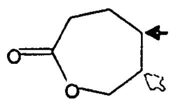 | 17 | 1,2,3-Triazine | 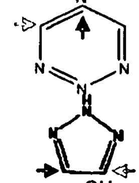 |
| 3 | 10-Oxodecanoate | 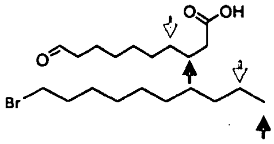 | 18 | v-Triazole | |
| 4 | 1-Bromodecane | | 19 | (R)-1,2,4-Butanetriol | 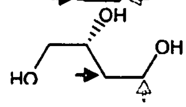 |
| 5 | 2-Bromodecane | 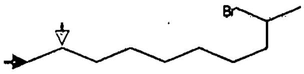 | 20 | 1,2,4-Dioxazole | 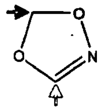 |
| 6 | 10-Formyltetrahydrofolate | 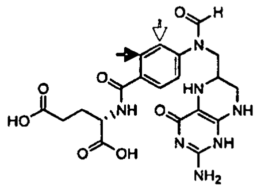 | 21 | 1,3,4-Oxadiazine | 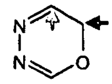 |
| 7 | 4-Hydroxy-5-phenyltetrahydro-1,3-oxazin-2-one | 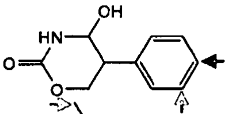 | 22 | 2-Methyl-1,3,4-oxadiazole | 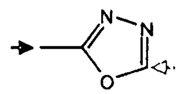 |
| 8 | 5-Chloro-3-methylcatechol | 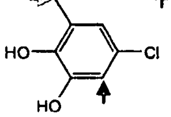 | 23 | 1,3,5-Triazine | 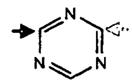 |
| 9 | 3-Methylcrotonyl-CoA | 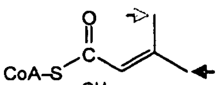 | 24 | (1R,2S)-1,2-Dihydronaphthalene-1,2-diol | 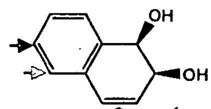 |
| 10 | 2-Chloromaleylacetate | 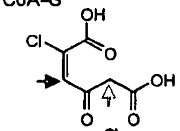 | 25 | 1,2,4-Trimethylbenzene | 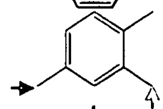 |
| 11 | 1,3-Dichloropentane | 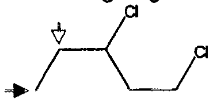 | 26 | Oxathiazine | 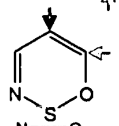 |
| 12 | 11-Hydroxyundecanoate | 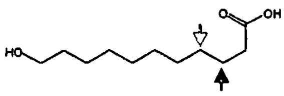 | 27 | 1,2,5-Oxadiazole | 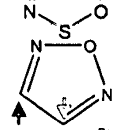 |
| 13 | 11-Oxoundecanoate | 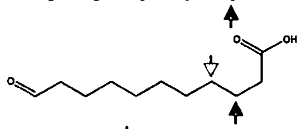 | 28 | 2,4,6-Tribromophenol | 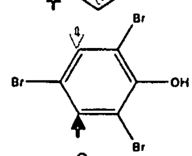 |
| 14 | Oxatriazole | 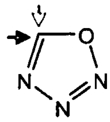 | 29 | 1,2-Benzoquinone | 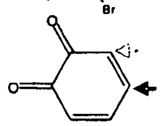 |
| 15 | 1,2,3,5-Oxatriazole | 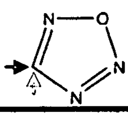 | 30 | 1,4-Dichloro-2-butene | 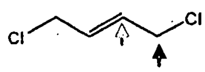 |

| N° | Substrate | Chemical Structure | N° | Substrate | Chemical Structure |
|---|---|---|---|---|---|
| 31 | 1,2-Didecanoyl-β-D-galactosyl-glycerol | 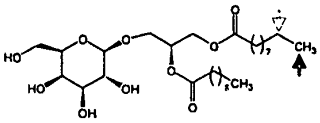 | 42 | 1,1-Dibromopentane | 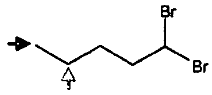 |
| 32 | Decane-1,2-diol | 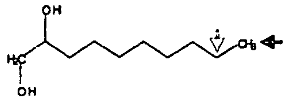 | 43 | 1,2-Dichlorobenzene | 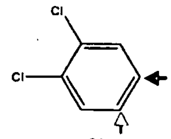 |
| 33 | Dodecane-1,2-diol | 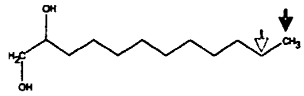 | 44 | 1,2-Dichloropentane | 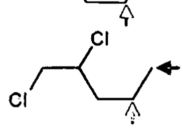 |
| 34 | 1,2,4-Triazole | 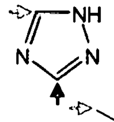 | 45 | 1,1-Dichloropentane | 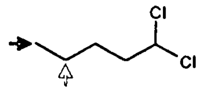 |
| 35 | 1,2-Dipropionyl-3-β-D-galactosyl-sn-glycerol | 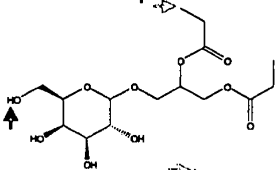 | 46 | 3-Hydroxy-1-indanone | 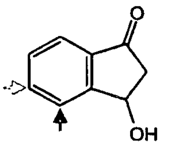 |
| 36 | 1,2-Dibutyryl-3-β-D-galactosyl-sn-glycerol | 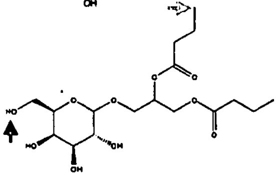 | 47 | 1,5-Diaminopentane | 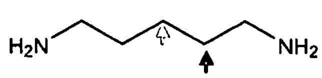 |
| 37 | 1,2-Dihydroxy-1,2-dihydronaphthalene | 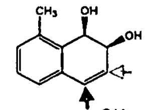 | 48 | (1S,2S)-1,2-Dihydronaphthalene-1,2-diol | 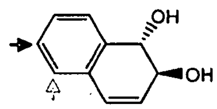 |
| 38 | 1,2-Butanediol | 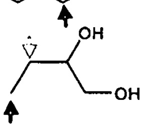 | 49 | 1,2-Didodecanoyl-3-beta-D-galactosyl-sn-glycerol | 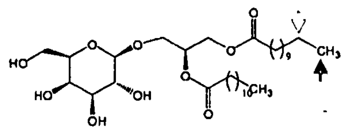 |
| 39 | 1,2-Dihexanoyl-3-β-D-galactosyl-sn-glycerol | 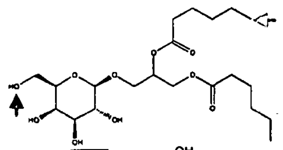 | 50 | 1,2-Dihydroxydibenzothiophene | 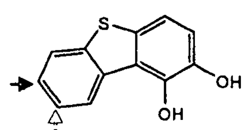 |
| 40 | 1,2-Dihydroxynaphthalene | 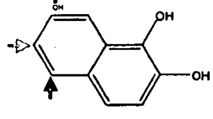 | 51 | cis-1,2-Dihydroxy-1,2-dihydrodibenzothiophene | 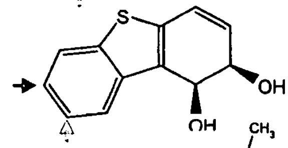 |
| 41 | 1,2-Diazole | 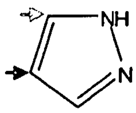 | 52 | 1,2-Diacyl-3-O-(alpha-D-glucopyranosyl(1->2)-O-alpha-D-glucopyranosyl)sn-glycerol | 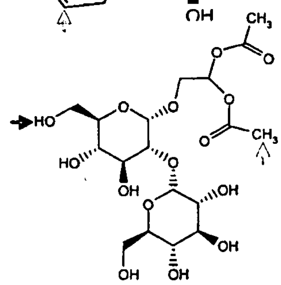 |

| N° | Substrate | Chemical Structure | N° | Substrate | Chemical Structure |
|---|---|---|---|---|---|
| 53 | 1,2-dioleyl-3-O-(α-D-glucopyranosyl)-sn-glycerol | 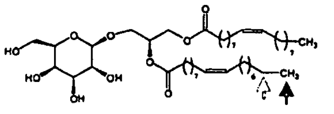 | 64 | Hexacosane-1,2-diol | 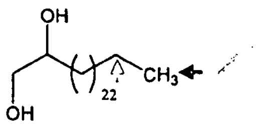 |
| 54 | 1,2-Didodecanoyl-sn-glycerol | 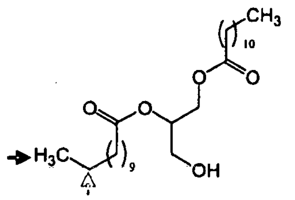 | 65 | 1,2-Hexadecanodiol | 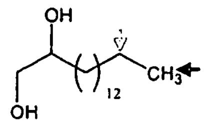 |
| 55 | 1,2-Oxazine | 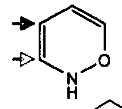 | 66 | 1,2-Hexanodiol | 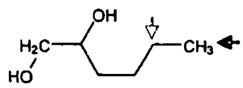 |
| 56 | 1,2-Dioctanoyl-3-β-D-galactosyl-sn-glycerol | 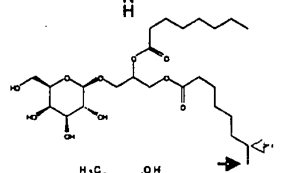 | 67 | 1,2-Nonanediol | 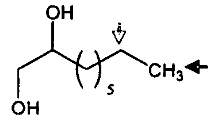 |
| 57 | 6-Deoxyerythronolide | 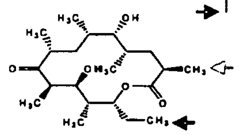 | 68 | 1,2-Nonadecanodiol | 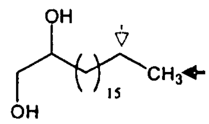 |
| 58 | 1,2-Dipalmitoyl-β-D-galactosyl-glycerol | 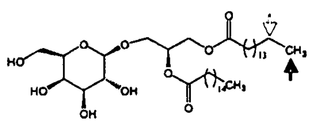 | 69 | 3-Isopropylbut-3-enoyl-CoA | 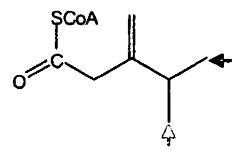 |
| 59 | 1,2-Dipalmitoyl-3-O-[α-D-glucopyranosyl(1->2)-O-α-D-glucopyranosyl]-sn-glycerol | 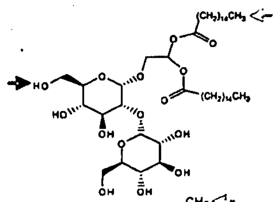 | 70 | 1,2-Octadecanodiol | 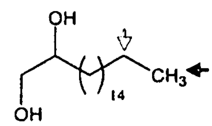 |
| 60 | 1,2-dipalmitoyl-3-O-α-D-glucopyranosyl-sn-glycerol | 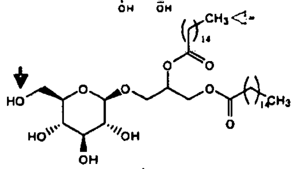 | 71 | 1,2-Octanodiol | 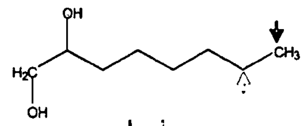 |
| 61 | 1,2-Epoxycyclohexane | 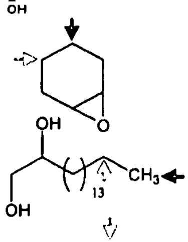 | 72 | (+)-1,2-O-Isopropylidene-sn-glycerol propyl ester | 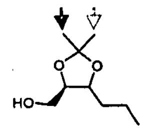 |
| 62 | 1,2-Heptadecanodiol | | 73 | 1,3-Oxazole | 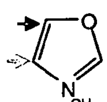 |
| 63 | 1,7-Heptanediol | 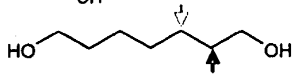 | 74 | 1,2-Pentanodiol | 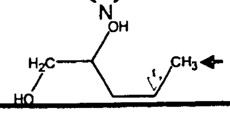 |

| N° | Substrate | Chemical Structure | N° | Substrate | Chemical Structure |
|---|---|---|---|---|---|
| 75 | 1,2-Dibutyryl-sn-glycerol | 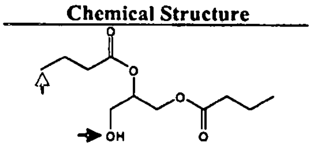 | 87 | Dithiazine | 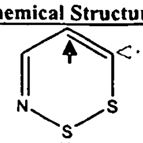 |
| 76 | Propane-1,2-diol-1-phosphate | 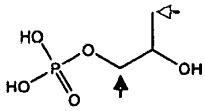 | 88 | 1,2,5-Oxadiazine | 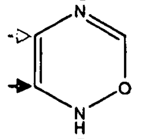 |
| 77 | Propane-1,2-diol | 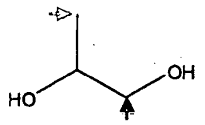 | 89 | 1,3,5-Oxadithiane | 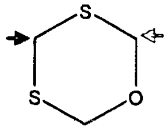 |
| 78 | (R)-Propane-1,2-diol | 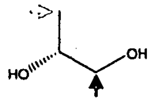 | 90 | Thiadiazine | 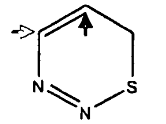 |
| 79 | (S)-Propane-1,2-diol | 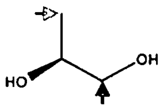 | 91 | (2S)-2-Isopropylmalate | 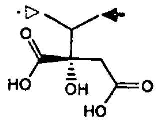 |
| 80 | 1,2-bis-O-Sinapoyl-?-D-glucoside | 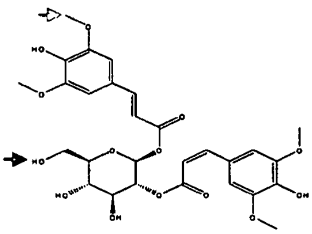 | 92 | 3-Phospho-D-glyceroyl phosphate | 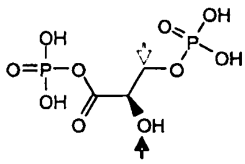 |
| 81 | 1,2-Tetracosanodiol | 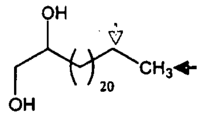 | 93 | 1,3,8-Trihydroxynaphthalene | 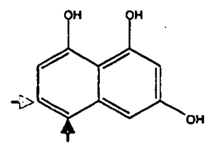 |
| 82 | 1,2-Thiazole | 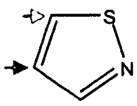 | 94 | 1,3-Bisphospho-D-glycerate | 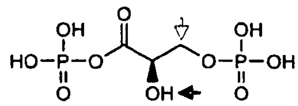 |
| 83 | 1,2-Undecanodiol | 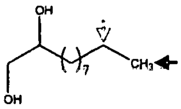 | 95 | 1,3-Butanediol | 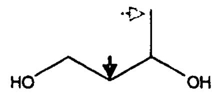 |
| 84 | 2-Chloro-4-hydroxy-6-amino-1,3,5-triazine | 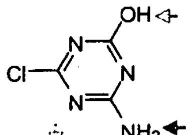 | 96 | 2,4-Dichloroaniline | 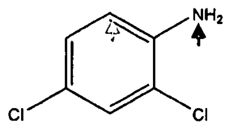 |
| 85 | 1,3,4-Oxadiazole | 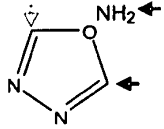 | 97 | 1,3-Diazole | 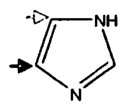 |
| 86 | 1,3,4-Thiadiazole | 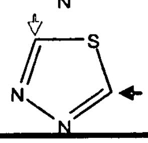 | 98 | 1,3-Dibromobenzene | 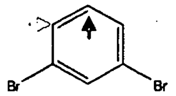 |

| N° | Substrate | Chemical Structure | N° | Substrate | Chemical Structure |
|---|---|---|---|---|---|
| 99 | 1,2-Dihexanoyl-sn-glycerol | 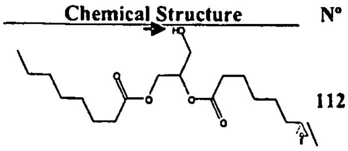 | 112 | 1,3-Dithiane | 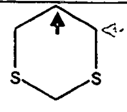 |
| 100 | Deoxyuridine | 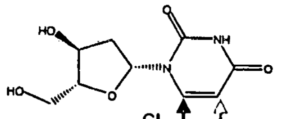 | 113 | 1,2-Dioleyl-sn-glycerol | 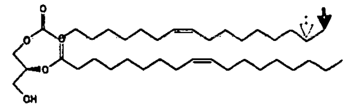 |
| 101 | 1,3-Dichloropentane | 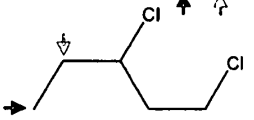 | 114 | 3-Hydroxy-3-methyl-2-oxobutanoate | 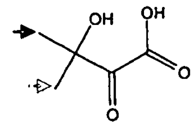 |
| 102 | 1,3-Dichlorobenzene | 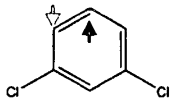 | 115 | Pentadecane-1,2-diol | 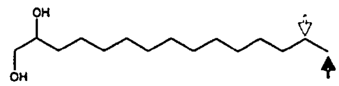 |
| 103 | 1,3-Didecanoyl-sn-glycerol | 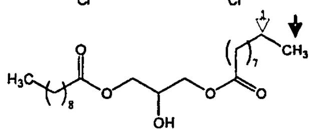 | 116 | Propane-1,3-diol | 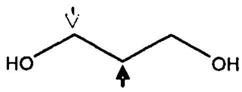 |
| 104 | 1,3-Dimethylbenzene | 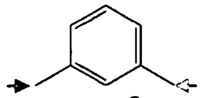 | 117 | 2,6-Dihydroxycyclohexane-1-carboxyl-CoA | 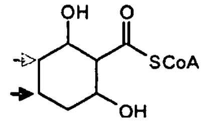 |
| 105 | 1,7-Dimethyluric acid | 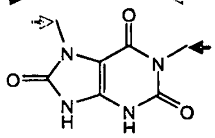 | 118 | 1,3-Oxathiole | 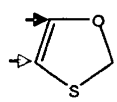 |
| 106 | 1,3-Diolein | 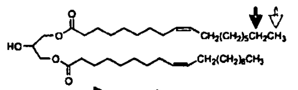 | 119 | 1,3-Oxazole | 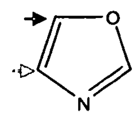 |
| 107 | 1,3,2-Dioxazole | 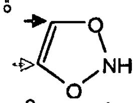 | 120 | 1,3-Pentanediol | 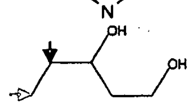 |
| 108 | (R)-2-Methylmalate | 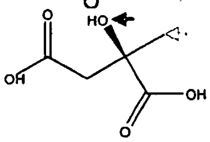 | 121 | dTTP | 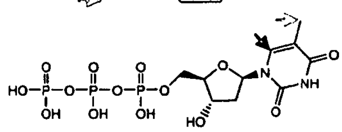 |
| 109 | 1,3,5-Trithiane | 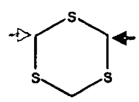 | 122 | 6-Hydroxycyclohex-1-ene-1-carboxyl-CoA | 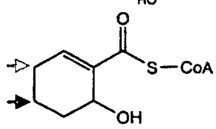 |
| 110 | 1,2-Dipalmitoyl-3-myristoylglycerol | 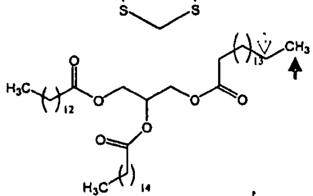 | 123 | 1,4-Butanediol | 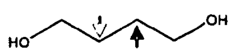 |
| 111 | 1,3-Dioctadecanoyl-sn-glycerol | 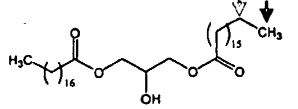 | 124 | 1-Butanoyl-sn-glycerol 3-phosphate | 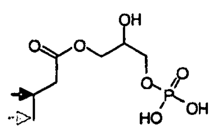 |

| N° | Substrate | Chemical Structure | N° | Substrate | Chemical Structure |
|---|---|---|---|---|---|
| 125 | 1,4-Dichloro-2-butene | 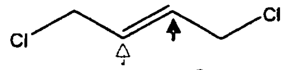 | 139 | 1,5-Anhydro-D-mannitol | 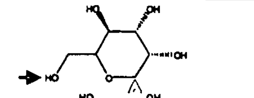 |
| 126 | D-Erythrose 4-phosphate | 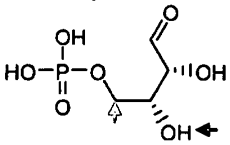 | 140 | 1,5-Anhydro-D-glucitol | 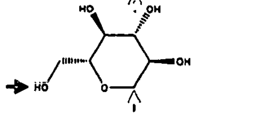 |
| 127 | 1,4-Dichlorobutane | 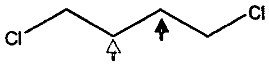 | 141 | 1,2-Dihydroxycyclohexa-3,5-diene-1-carboxylate | 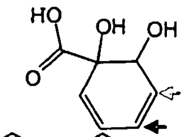 |
| 128 | 1,3-Dichloropropane | 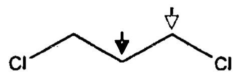 | 142 | 1,5-Dichloropentane | 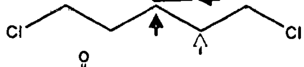 |
| 129 | 1,4-Dimethylbenzene | 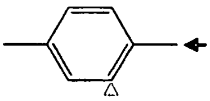 | 143 | 1-Heptadecanoyl-sn-glycero-phosphocholine | 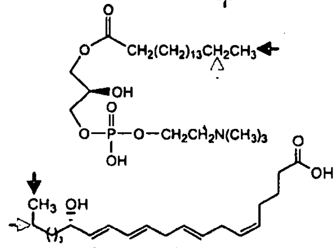 |
| 130 | 1,4-Dioxane | 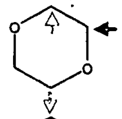 | 144 | (15S)-15-Hydroxy-5,8,11-cis-13-trans-icosatetraenoate | |
| 131 | 1,4-Dithiane | 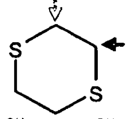 | 145 | 1,5-Pentanediol | 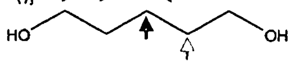 |
| 132 | Maltose | 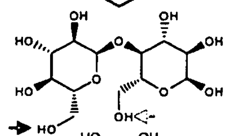 | 146 | 2-Pentadecanol | 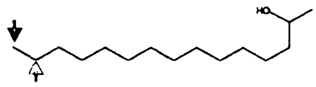 |
| 133 | Maltotriose | 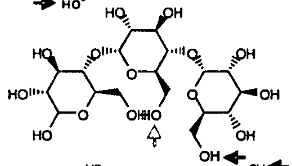 | 147 | 1,1-Dichlorohexane | 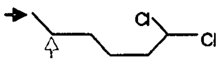 |
| 134 | Maltotetraose | 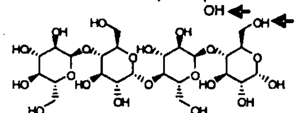 | 148 | 1,6-Napththyridine | 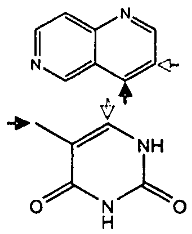 |
| 135 | Maltopentaose | 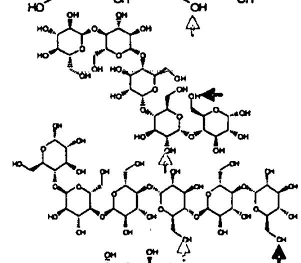 | 149 | 5-Methyluracil | |
| 136 | Maltohexaose | | 150 | Methyl viologen | 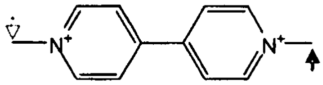 |
| 137 | Maltoheptaose | 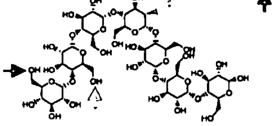 | 151 | 1,7-Naphthyridine | 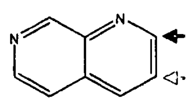 |
| 138 | 1,5-Anhydro-D-fructose | 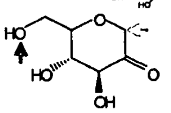 | 152 | 1,8-Dichlorooctane | 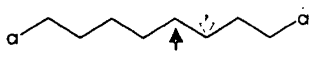 |

| N° | Substrate | Chemical Structure | N° | Substrate | Chemical Structure |
|---|---|---|---|---|---|
| 153 | 1,8-Naphthyridine | 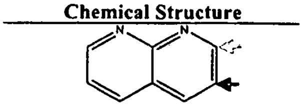 | 166 | 1-Butanoyl-sn-glycerol-3-phosphocholine | 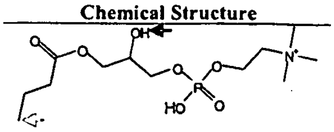 |
| 154 | 1-Aminocyclopropane-1-carboxylate | 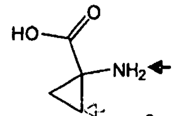 | 167 | 2-Chloro-4-methylphenol | 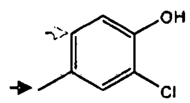 |
| 155 | 1-Acetyl-sn-glycerol 3-phosphate | 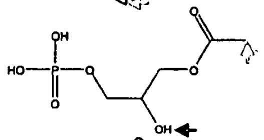 | 168 | 1-Chlorobutane | 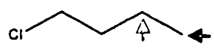 |
| 156 | 1-Acetyl-5-Hydroxy-1,5-dihydro-pyrrol-2-one | 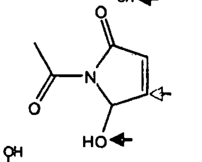 | 169 | 1-Chlorodecane | 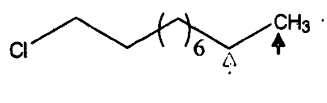 |
| 157 | 1-Butyrylglycerol | 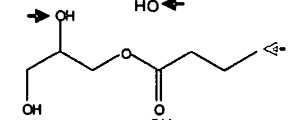 | 170 | 3-Chloropropanoic acid | 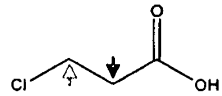 |
| 158 | 1-Octanoyl-sn-glycerol-3-phosphate | 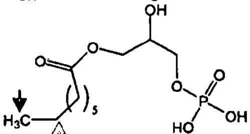 | 171 | Chlorotoluene | 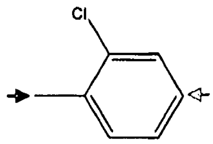 |
| 159 | 3,5-Dibromo-4-hydroxybenzoate | 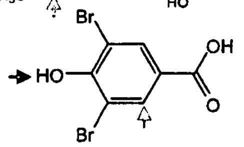 | 172 | 1-Deoxy-D-altro-heptulose 7-phosphate | 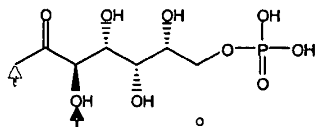 |
| 160 | 4-Bromophenyl acetate | 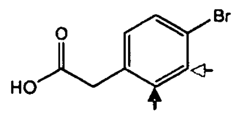 | 173 | trans-1-Decalone | 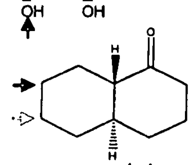 |
| 161 | 1-Bromobutane | 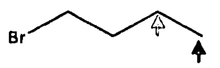 | 174 | 1-Decanol | 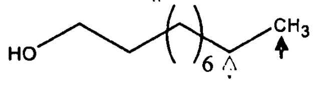 |
| 162 | 1-Bromodecane | 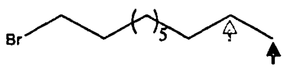 | 175 | 1-Decanoyl-sn-glycerol 3-phosphate | 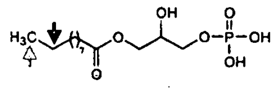 |
| 163 | 1,4-Dichlorobenzene | 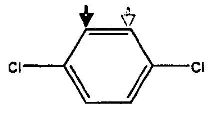 | 176 | 1-Decanoyl-sn-glycero-phosphocholine | 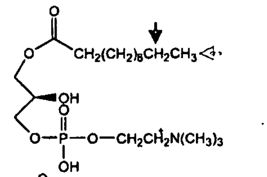 |
| 164 | beta-D-Fructose 6-phosphate | 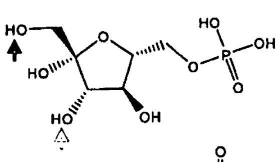 | 177 | 1-Dodecanoyl-sn-glycero-phosphocholine | 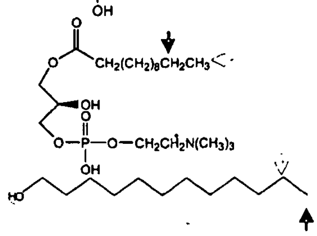 |
| 165 | Dicarbomethoxynaphthalene | 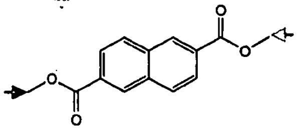 | 178 | 1-Dodecanol | |

| N° | Substrate | Chemical Structure | N° | Substrate | Chemical Structure |
|---|---|---|---|---|---|
| 179 | 1,5-Naphthyridine | 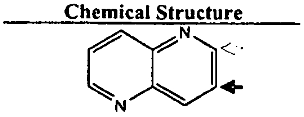 | 192 | 1-Methylnicotinamide | 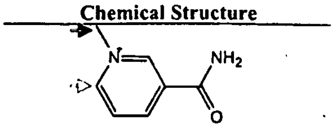 |
| 180 | 2,6-Dichlorophenol | 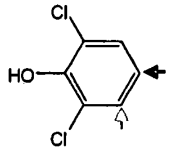 | 193 | 4-Imidazolone-5-propanoate | 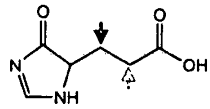 |
| 181 | 3,4-Dichlorophenol | 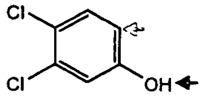 | 194 | 1-Methylnaphthalene | 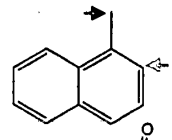 |
| 182 | 1H-2-Benzopyran-1-one | 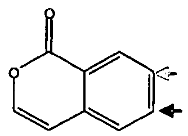 | 195 | 5-Hydroxyindoleacetaldehyde | 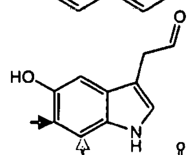 |
| 183 | 1-Hydroxybutane-1,2,4-tricarboxylate | 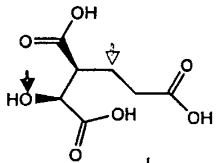 | 196 | 2,5-Diaminopyrimidine nucleoside triphosphate | 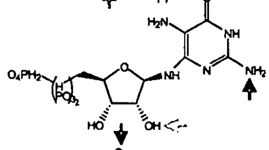 |
| 184 | 1-Heptadecanol | 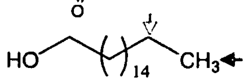 | 197 | Indan-1-ol | 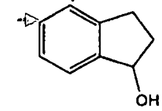 |
| 185 | 1-Dodecanoyl-sn-glycerol 3-phosphate | 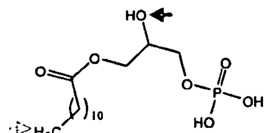 | 198 | 1-Indanone | 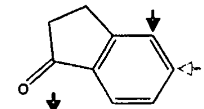 |
| 186 | 2-Heptanol | 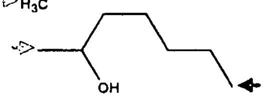 | 199 | 1R-Indenol | 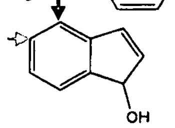 |
| 187 | 1-Heptanol | 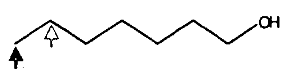 | 200 | 1-Methoxynaphtalene | 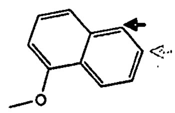 |
| 188 | 1-Hexacosanol | 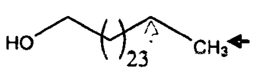 | 201 | 1-Monododecanoylglycerol | 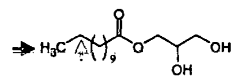 |
| 189 | 1-Palmitoylglycerophosphocholine | 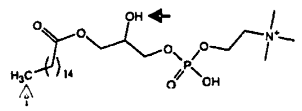 | 202 | 1-Monotetradecanoylglycerol | 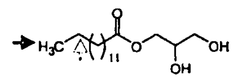 |
| 190 | beta-D-Fructose 1,6-bisphosphate | 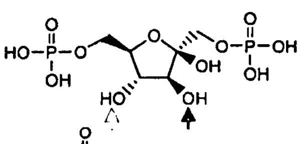 | 203 | 2-Carboxy-2,3-dihydro-5,6-dihydroxyindole | 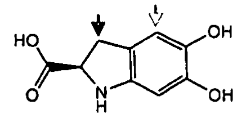 |
| 191 | 1-Hexanoyl-sn-glycero-phosphocholine | 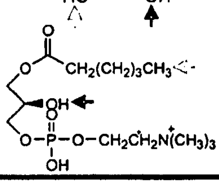 | 204 | Methyl pyridine-3-carboxylate | 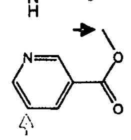 |

| N° | Substrate | Chemical Structure | N° | Substrate | Chemical Structure |
|---|---|---|---|---|---|
| 205 | 2-Hydroxymethylnaphthalene | 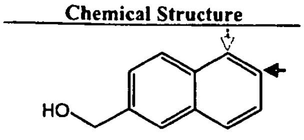 | 218 | 6-Oxo-2-hydroxycyclohexane-1-carboxyl-CoA | 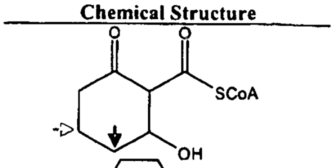 |
| 206 | 1-Hexanol | 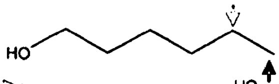 | 219 | 2-Octanol | 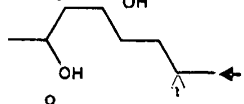 |
| 207 | 1-Monohexanoylglycerol | 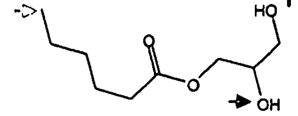 | 220 | 1-Octadecanoyl-sn-glycero-phosphocholine | 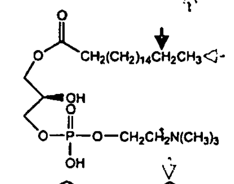 |
| 208 | 1-Monooctanoylglycerol | 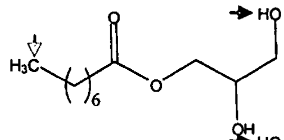 | 221 | 1-Octadecanol | 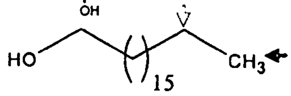 |
| 209 | 1-Monodecanoylglycerol | 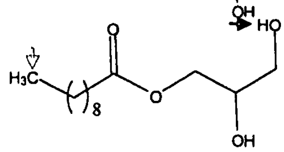 | 222 | 1-Octanoyl-sn-glycero-phosphocholine | 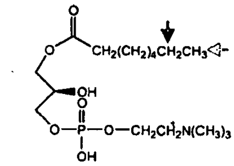 |
| 210 | 1-Nitrosonaphthalene | 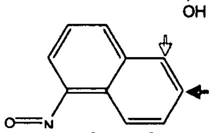 | 223 | 1-Octanol | 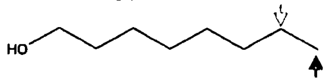 |
| 211 | 1-Nitronaphthalene | 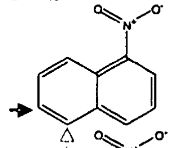 | 224 | 1-Palmitoyl-3-stearoyl-rac-glycerol | 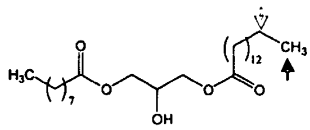 |
| 212 | 1-Nitronaphthalene-5,6-oxide | 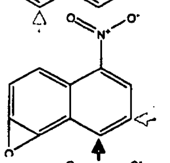 | 225 | 1-Pentanol | 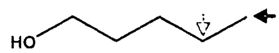 |
| 213 | 1-Nitronaphthalene-7,8-oxide | 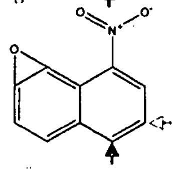 | 226 | 1-O-Hexadecanoyl-glycerol | 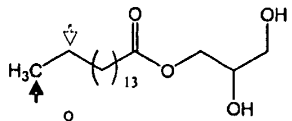 |
| 214 | 1-Nonadecanol | 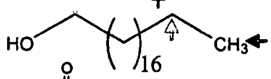 | 227 | 1-Propionyl-sn-glycero-3-phosphocholine | 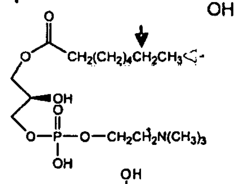 |
| 215 | 1-Nonanoyl-sn-glycero-phosphocholine | 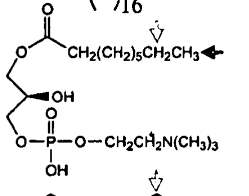 | 228 | 1-Hexadecanoyl-sn-glycerol 3-phosphate | 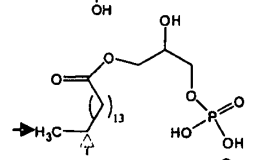 |
| 216 | 1-Nonanol | 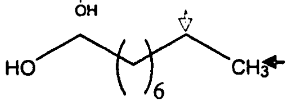 | 229 | Phenylacetate | 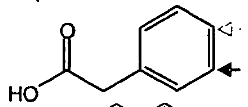 |
| 217 | 1-Oleyl-sn-glycerol 3-phosphate | 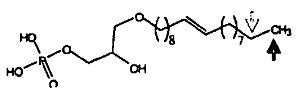 | 230 | 12-Pyren-1-yldodecanoic acid | 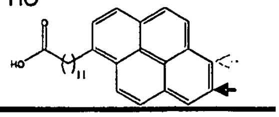 |

| N° | Substrate | Chemical Structure | N° | Substrate | Chemical Structure |
|---|---|---|---|---|---|
| 231 | 2,3,4-Trichlorobiphenyl | 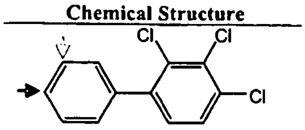 | 244 | 1-Pyrroline-5-carboxylate | 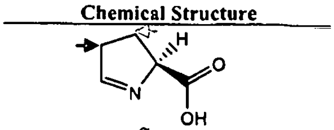 |
| 232 | 1-Pyrroline | 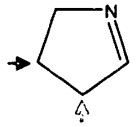 | 245 | 2,3,5,6-Tetrachlorobiphenyl | 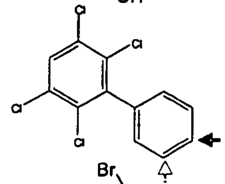 |
| 233 | 1-O-Sinapoyl-β-D-glucoside | 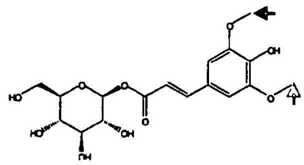 | 246 | 2-(2,6-Dibromophenyl)-1H-pyrrole | 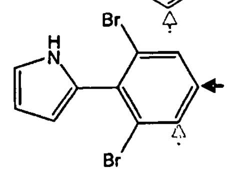 |
| 234 | 1-Tetracosanol | 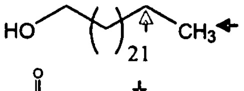 | 247 | 2,4,5-Trichlorophenol | 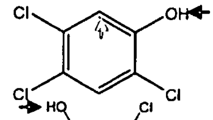 |
| 235 | 1-Tetradecanoyl-sn-glycero-phosphocholine | 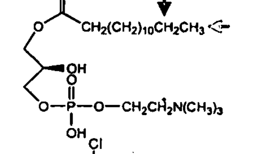 | 248 | 2,3,6-Trichlorophenol | 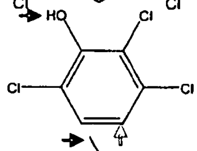 |
| 236 | 2,2',5-Trichlorobiphenyl | 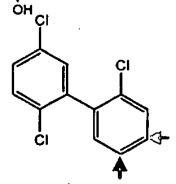 | 249 | 2,3-Butanediol | 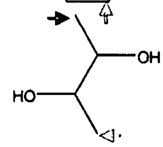 |
| 237 | 1-Undecanol | 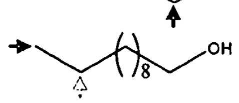 | 250 | 1-Phenyl-1,3-butanedion | 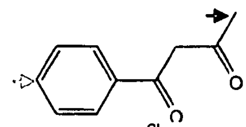 |
| 238 | 2,1-Benzoxazole | 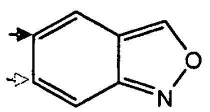 | 251 | 2,4,4'-Trichlorobiphenyl | 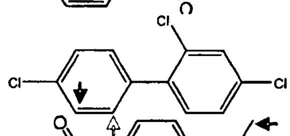 |
| 239 | 1-Tetradecanol | 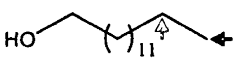 | 252 | cis-2,3-Dihydroxy-2,3-dihydro-p-cumate | 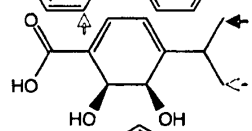 |
| 240 | 1-Bromo-2-(2-bromoethyl)benzene | 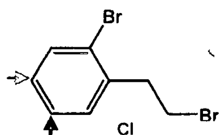 | 253 | 2,3-Dihydro-2,3-dihydroxybiphenyl | 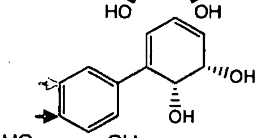 |
| 241 | 1-Chloro-2-(chlorophenylmethyl)benzene | 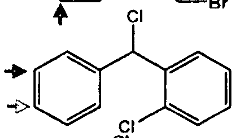 | 254 | 3-(4-Chlorophenyl)benzene-1,2-diol | 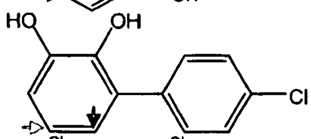 |
| 242 | 2,2-Dichloropropanoic acid | 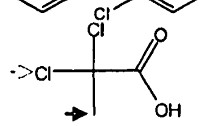 | 255 | 1-Chloro-3-(2-chlorophenyl)benzene | 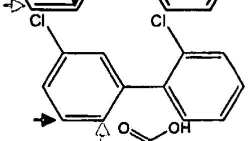 |
| 243 | 2,3,4-Trichlorophenol | 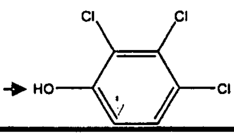 | 256 | 2,3-Dihydro-2,3-dihydroxybenzoate | 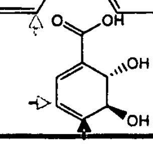 |

| N° | Substrate | Chemical Structure | N° | Substrate | Chemical Structure |
|---|---|---|---|---|---|
| 257 | 1,3-Thiazole | 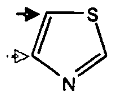 | 270 | 2,3-Diketo-L-gulonate | 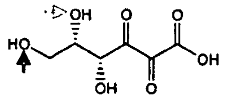 |
| 258 | 2,3-Dihydroxybenzoate | 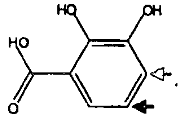 | 271 | 2,3-Dihydroxyisovalerate | 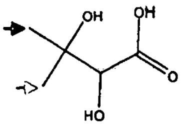 |
| 259 | 2,3-Dihydroxybenzoyladenylate | 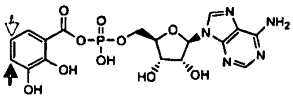 | 272 | 2,3-Dihydroxy-p-cumate | 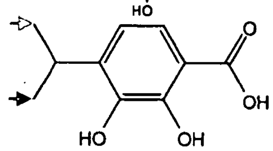 |
| 260 | 2,3-Dihydroxybiphenyl | 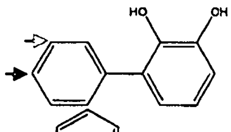 | 273 | cis-2,3-Dihydro-2,3-dihydroxy-4'-chlorobiphenyl | 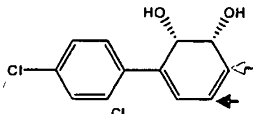 |
| 261 | trans-2,3-Dihydroxycinnamate | 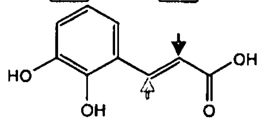 | 274 | 2,4,5-Trichlorobiphenyl | 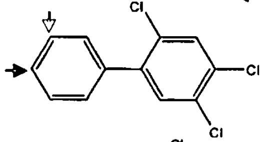 |
| 262 | 2,4,6-Trichlorobiphenyl | 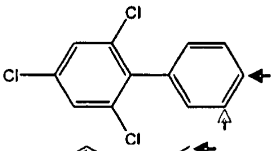 | 275 | 2,4',5-Trichlorobiphenyl | 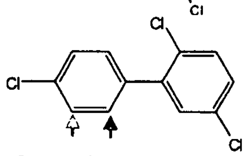 |
| 263 | 2,2'-Dimethoxybiphenyl | 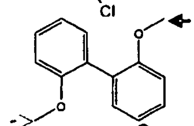 | 276 | 4-Hydroxyphenylpyruvate | 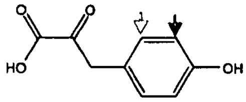 |
| 264 | 2,3-Dihydroxy-3-methylpentanoate | 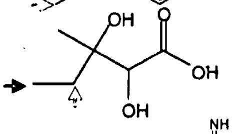 | 277 | 2,3-Dihydrodipicolinate | 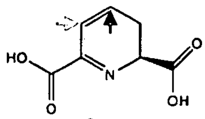 |
| 265 | 2-(Formamido)-N1-(5-phospho-D-ribosyl)acetamidine | 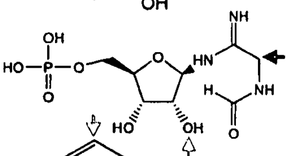 | 278 | 2',2,4-Trichlorobiphenyl | 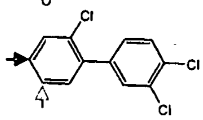 |
| 266 | 2,3-Dihydroxyphenylpropanoate | 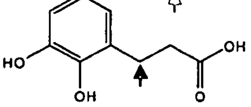 | 279 | 2,4,6-Trichlorophenol | 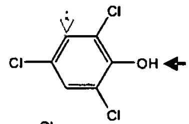 |
| 267 | 2,3-Dihydrofuran | 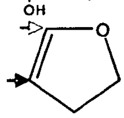 | 280 | 2,4-Dichlorobenzoate | 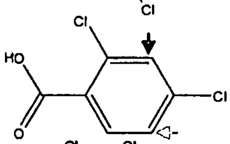 |
| 268 | (R)-2,3-Dihydroxy-3-methylbutanoate | 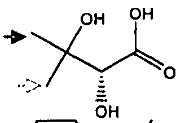 | 281 | 1-Chloro-4-(2,2-dichloro-1-phenylethyl)benzene | 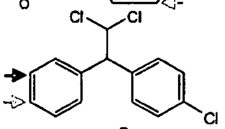 |
| 269 | 1,2-Dimethylnaphthalene | 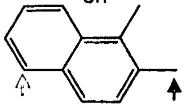 | 282 | 2-Bromo-1,3-dichlorobenzene | 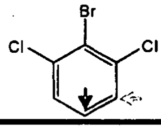 |

| N° | Substrate | Chemical Structure | N° | Substrate | Chemical Structure |
|---|---|---|---|---|---|
| 283 | 2,4-Dichlorophenoxyacetic acid | 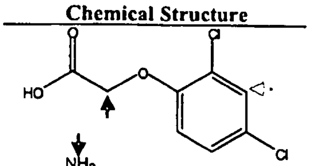 | 296 | 2,5-Didehydro-D-gluconate | 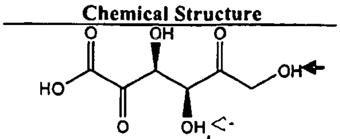 |
| 284 | gamma-Glutamyl-gamma-aminobutyraldehyde | 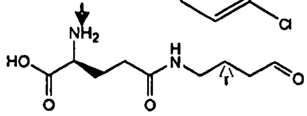 | 297 | 2,5-Dihydroxyphenyl acetate | 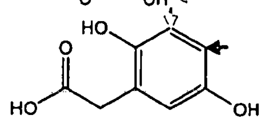 |
| 285 | 2,4-Dichlorophenol | 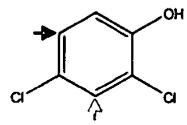 | 298 | 2-cis-6-trans-farnesol | 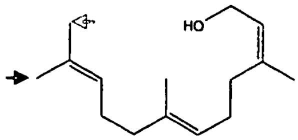 |
| 286 | 2,4-Dinitroaniline | 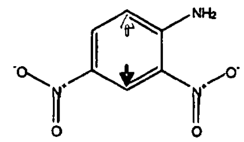 | 299 | 2-cis-6-trans-farnesal | 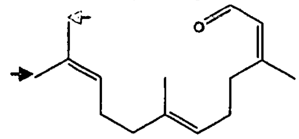 |
| 287 | 2,4,5-Trichlorophenol | 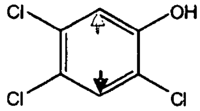 | 300 | LL-2,6-Diamino heptanedioate | 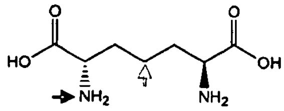 |
| 288 | S-2-[5-Amino-1-5-phospho-D-ribosylimidazole-4-carboxamido]succinate | 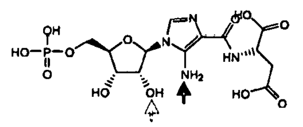 | 301 | meso-2,6-Diaminoheptanedioate | 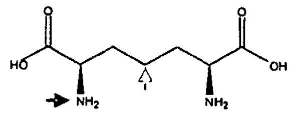 |
| 289 | 2,5-Dichloro-2,5-cyclohexadiene-1,4-diol | 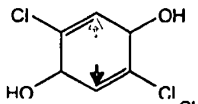 | 302 | 2,6-Dimethylnaphthalene | 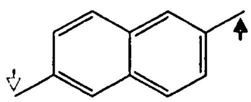 |
| 290 | 1,4-Dichloro-2-phenylbenzene | 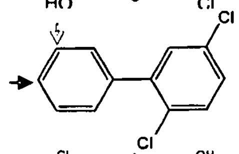 | 303 | 2-trans-6-trans-farnesol | 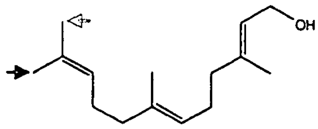 |
| 291 | 2,5-Dichlorohydroquinone | 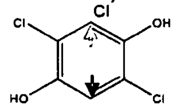 | 304 | 2-trans-6-trans-farnesal | 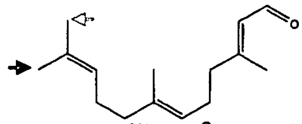 |
| 292 | 2,5-Dichlorophenol | 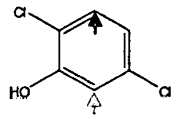 | 305 | 2-Amino-3-oxobutanoate | 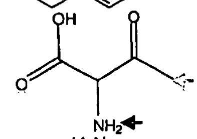 |
| 293 | 2,5-Didehydro-D-gluconate | 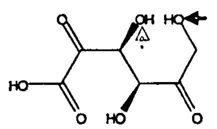 | 306 | 2-Amino benzene sulfonate | 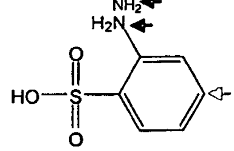 |
| 294 | 2,5-Diamino-6-(5'-phosphoribosylamino)-4-pyrimidineone | 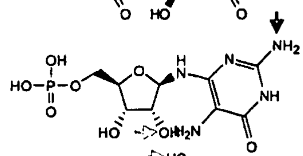 | 307 | cis-2,3-Dihydroxy-2,3-dihydroethylbenzene | 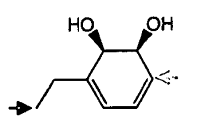 |
| 295 | 2,5-Diamino-6-hydroxy-4-(5'-phosphoribosylamino)-pyrimidine | 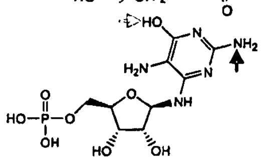 | 308 | Methyl 3-hydroxydecanoate | 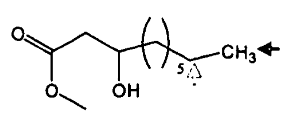 |

| N° | Substrate | Chemical Structure | N° | Substrate | Chemical Structure |
|---|---|---|---|---|---|
| 309 | 2-Amino-4-hydroxy-6-hydroxymethyl-7,8-dihydropteridine diphosphate | 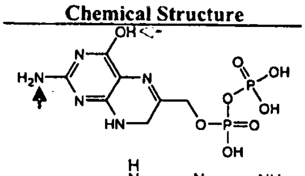 | 322 | Bromobenzene-3,4-dihydrodiol | 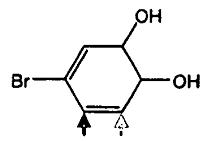 |
| 310 | 2-Amino-4-hydroxy-6-hydroxymethyl-7,8-dihydropteridine | 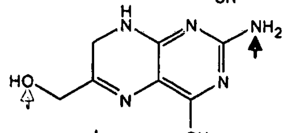 | 323 | 2-Butyne-1,4-diol | 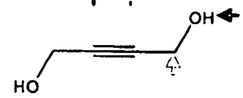 |
| 311 | 2-Amino-7,8-dihydro-4-hydroxy-6-(diphosphooxymethyl)pteridine | 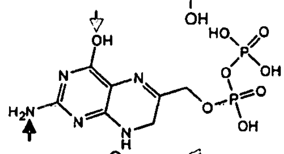 | 324 | Bromobenzene | 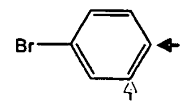 |
| 312 | 2-Aceto-2-hydroxybutanoate | 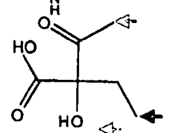 | 325 | 2-Bromobutane | 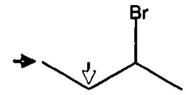 |
| 313 | (S)-2-Aceto-2-hydroxybutanoate | 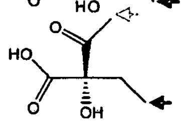 | 326 | 2-Bromoethylbenzene | 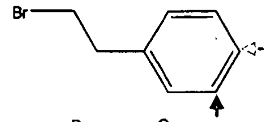 |
| 314 | 2-Aminoebenzenesulfonate | 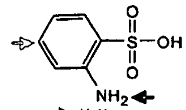 | 327 | 2-Bromopropanoic acid | 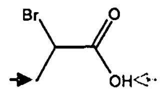 |
| 315 | 2-Aminobenzoate | 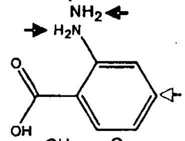 | 328 | 1-Bromo-2-ethenylbenzene | 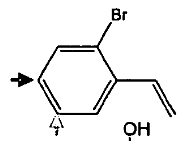 |
| 316 | 2-Amino-3-oxobutanoate | 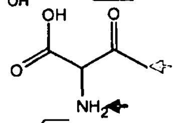 | 329 | 2-Butanol | 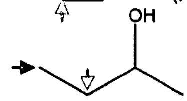 |
| 317 | 2-Arachidonoylglycerol | 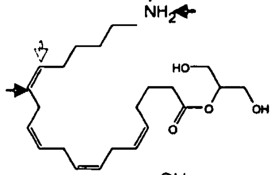 | 330 | cis-2-Chloro-2-butene | 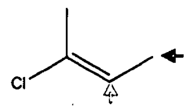 |
| 318 | 2,3-Dihydroxyethylbenzene | 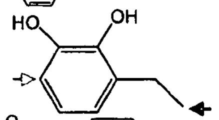 | 331 | 2-Chlorobiphenyl | 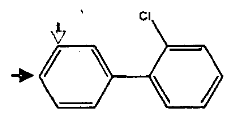 |
| 319 | 4-Fluorobenzoate | 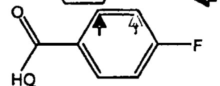 | 332 | 2-Chlorobutane | 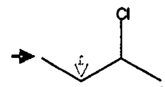 |
| 320 | 2-Hydroxymuconate | 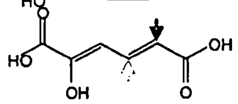 | 333 | 2-Chlorodecane | 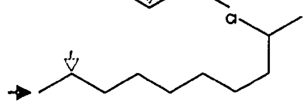 |
| 321 | 2,3-Bisphospho-D-glycerate | 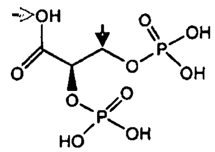 | 334 | 1-(2-Carboxyphenylamino)-1'-deoxy-D-ribulose 5'-phosphate | 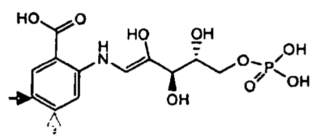 |

| N° | Substrate | Chemical Structure | N° | Substrate | Chemical Structure |
|---|---|---|---|---|---|
| 335 | 2-Dehydro-3-deoxy-D-arabino-heptonate 7-phosphate | 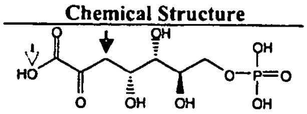 | 348 | 5-Dehydro-2-deoxy-D-gluconate | 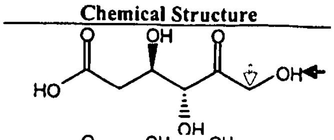 |
| 336 | 2-Chloropropanoic acid | 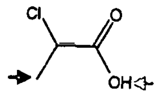 | 349 | 2-Dehydro-D-gluconate | 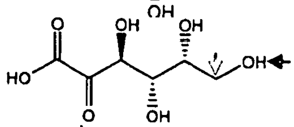 |
| 337 | cis-2-Hydroxypenta-2,4-dienoate | 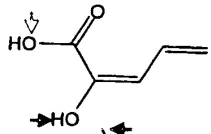 | 350 | 2-Deoxy-D-glucose | 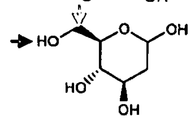 |
| 338 | 1-Chloro-2-methylbenzene | 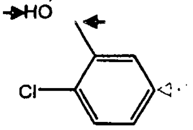 | 351 | 2-Dodecanoyl-glycerol | 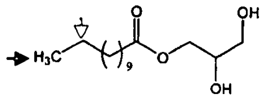 |
| 339 | 3-(p-Chlorophenyl)coumarin | 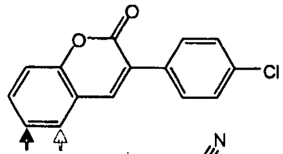 | 352 | 2-Decanoyl-glycerol | 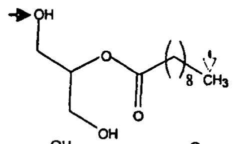 |
| 340 | 2-Phenylpropanenitrile | 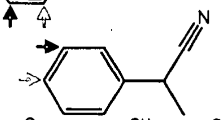 | 353 | 2-Dehydro-3-deoxy-D-galactonate | 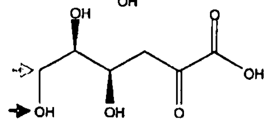 |
| 341 | 2-Dehydro-3-deoxy-D-gluconate 6-phosphate | 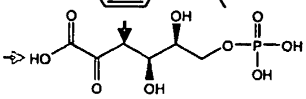 | 354 | 2-Dehydro-3-deoxy-D-galactonate 6-phosphate | 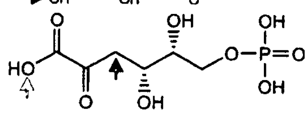 |
| 342 | 2-Dehydro-3-deoxy-L-arabinonate | 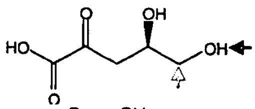 | 355 | Decan-2-ol | 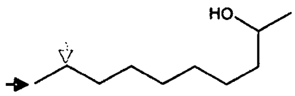 |
| 343 | 2-Dehydro-3-deoxy-D-gluconate | 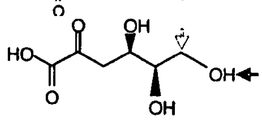 | 356 | 2-Keto-L-gulonate | 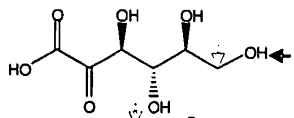 |
| 344 | 2-Chlorophenol | 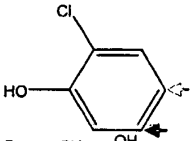 | 357 | 2-Dehydropantoate | 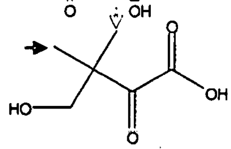 |
| 345 | 2-Deoxy-D-gluconate | 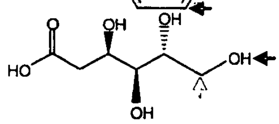 | 358 | 2-Dehydropantolactone | 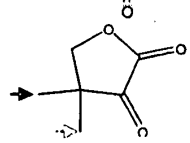 |
| 346 | 2-Deoxy-D-glucose | 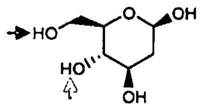 | 359 | 2H-1,2,4-Benzoxadiazine | 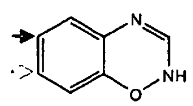 |
| 347 | 2-Dehydro-3-deoxy-D-xylonate | 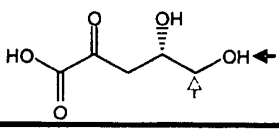 | 360 | Demethylmenaquinone | 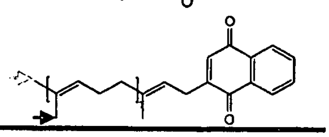 |

| N° | Substrate | Chemical Structure | N° | Substrate | Chemical Structure |
|---|---|---|---|---|---|
| 361 | 2-Dodecanol | 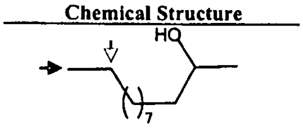 | 374 | 3H-1,2,4-dioxazole | 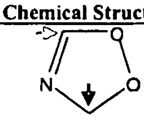 |
| 362 | 2-Deoxy-D-ribose 5-phosphate | 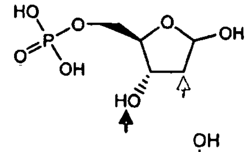 | 375 | (S)-2-Hydroxy-2-methyl-3-oxobutanoate | 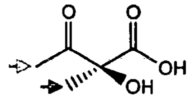 |
| 363 | 2-Deoxy-D-ribose 1-phosphate | 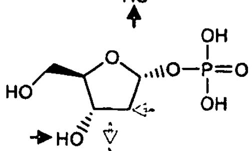 | 376 | 2-Phenyl-4-propyl-tetrahydro-1,4-oxazine | 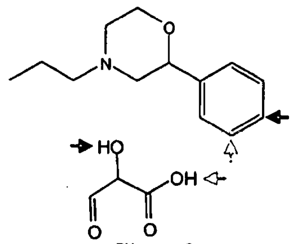 |
| 364 | 2-Ethylhexan-1-ol | 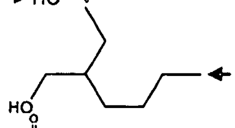 | 377 | 2-Hydroxy-3-oxopropanoate | |
| 365 | 2-Demethylmenaquinone | 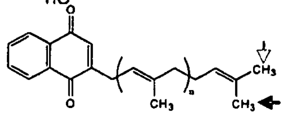 | 378 | 2-Hydroxy-3-oxosuccinate | 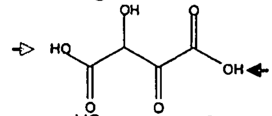 |
| 366 | 2H-1,2-Oxazine | 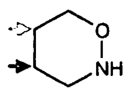 | 379 | 2-Hydroxy-5-methyl-cis,cis-muconic semialdehyde | 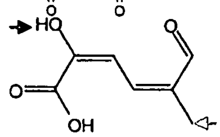 |
| 367 | 2-Hydroxy-2-hydropyrone-4,6-dicarboxylate | 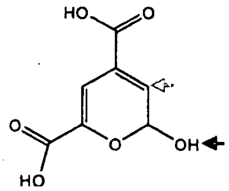 | 380 | 2-Hexadecanoyl-glycerol | 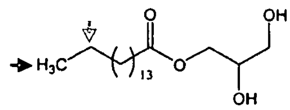 |
| 368 | 2H-1,2-Benzoxazine | 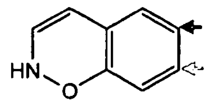 | 381 | 2-Hydroxy-6-keto-2,4-heptadienoate | 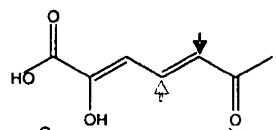 |
| 369 | 4-Methyl-1,3-oxazinane-2-thione | 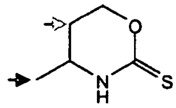 | 382 | 2-Hydroxy-6-oxo-7-methylocta-2,4-dienoate | 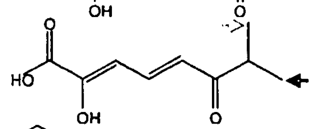 |
| 370 | 3-Methyl-2H-1,4-benzoxazine | 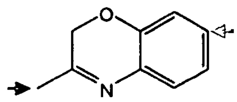 | 383 | 2-Hydroxy-6-oxo-6-phenylhexa-2,4-dienoate | 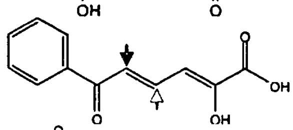 |
| 371 | 2-Hydroxy-3-carboxy-6-oxo-7-methylocta-2,4-dienoate | 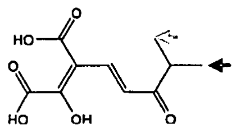 | 384 | 2-Hydroxyadipate | 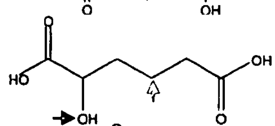 |
| 372 | 2H-1-Benzopyran-2-one | 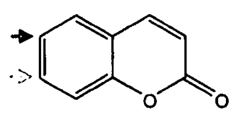 | 385 | 2-Hydroxybutane-1,2,4-tricarboxylate | 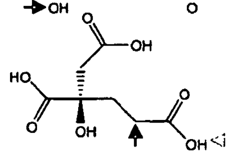 |
| 373 | 2H-1-Benzopyran | 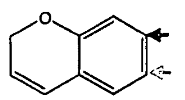 | 386 | 2-Hydroxycyclohexan-1-one | 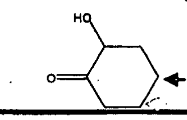 |

| N° | Substrate | Chemical Structure | N° | Substrate | Chemical Structure |
|---|---|---|---|---|---|
| 387 | 2-Heptadecanol | 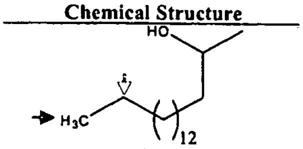 | 401 | 1-chloro-2-ethenylbenzene | 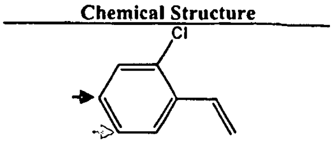 |
| 388 | 2-Heptadecanoyl-glycerol | 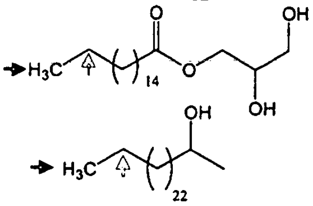 | 402 | Pyran-2-one | 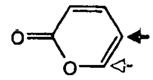 |
| 389 | 2-Hexacosanol | | 403 | 2H-Pyran | 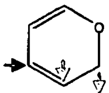 |
| 390 | L-Argininosuccinate | 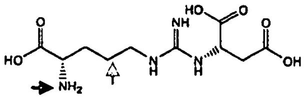 | 404 | p-Tolualdehyde | 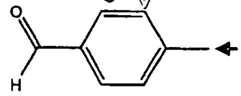 |
| 391 | 2-Hexadecanol | 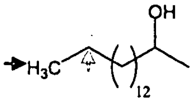 | 405 | 2-Hydroxybiphenyl | 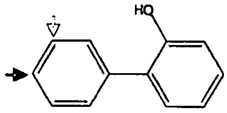 |
| 392 | 2-Hexanoyl-glycerol | 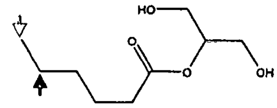 | 406 | 2-Hydroxybiphenyl-2-sulfinate | 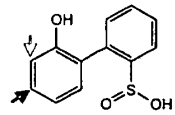 |
| 393 | 2-Hexanol | 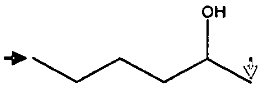 | 407 | 2-Isothiocyanatoethylbenzene | 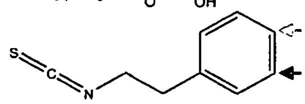 |
| 394 | (R)-2-Hydroxyglutarate | 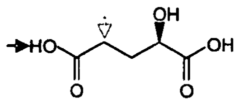 | 408 | N-Methylimidazolidine-2,4-dione | 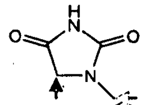 |
| 395 | 2,4-Dichlorobenzoyl-CoA | 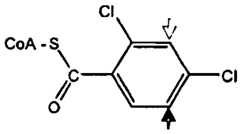 | 409 | 2-Indanone | 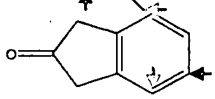 |
| 396 | 2H-Imidazole | 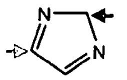 | 410 | 2-Isopropylmaleate | 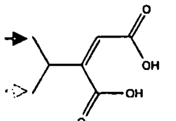 |
| 397 | 2-Hydroxymalonate | 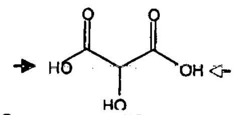 | 411 | Benzoyl acetyl-CoA | 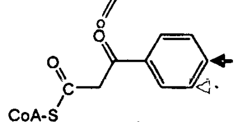 |
| 398 | 2-Hydroxy muconate semialdehyde | 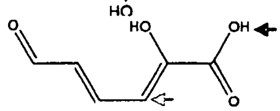 | 412 | 2H-Pyrrole | 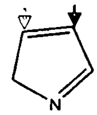 |
| 399 | 2-Hydroxyhexadecanal | 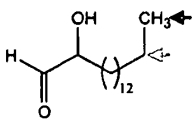 | 413 | 2-Keto-3-deoxy-gluconate | 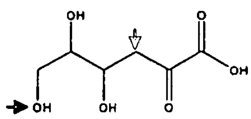 |
| 400 | 2-Bromo-1-chloropropane | 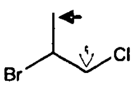 | 414 | 2-Keto-3-methylbutyrate | 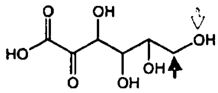 |

| N° | Substrate | Chemical Structure | N° | Substrate | Chemical Structure |
|---|---|---|---|---|---|
| 415 | 2-Keto-3-methylbutyric acid | 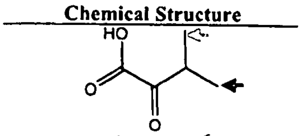 | 429 | 2-Methylcitrate | 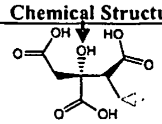 |
| 416 | 2-Oxoisovalerate | 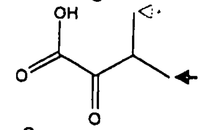 | 430 | 2-C-Methyl-D-erythritol 4-phosphate | 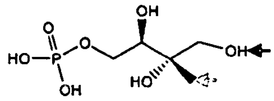 |
| 417 | 4-Methylthio-2-oxobutanoate | 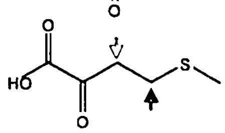 | 431 | 2-C-Methyl-D-erythritol 2,4-cyclodiphosphate | 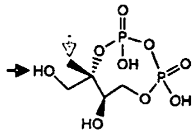 |
| 418 | 2-Oxopent-4-enoic acid | 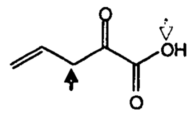 | 432 | 1-Methoxy-2-phenylbenzene | 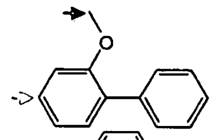 |
| 419 | 2-Methyl-1,3-cyclopentaion | 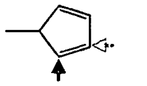 | 433 | 1-Hydroxymethylnaphthalene | 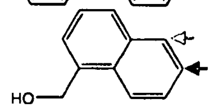 |
| 420 | 2-Methyl-4-amino-5-hydroxymethylpyrimidine diphosphate | 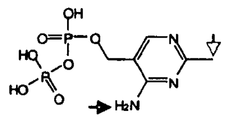 | 434 | 2-Methylphenol | 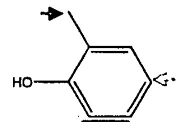 |
| 421 | 2-Methyl-1-propanol | 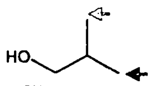 | 435 | 2-Methylpyridine | 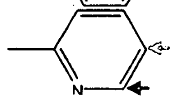 |
| 422 | 2-Maleylacetate | 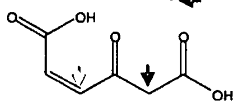 | 436 | [(2S)-Oxiran-2-yl]methyl acetate | 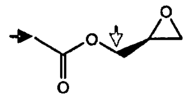 |
| 423 | 2-Methyl-1,3-dioxolan | 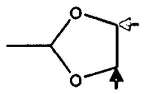 | 437 | 2-O-Methyllicodione | 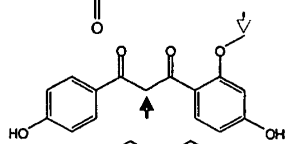 |
| 424 | 2-Methyl benzyl alcohol | 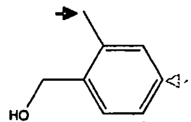 | 438 | 2-Methylnaphtalene | 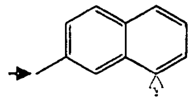 |
| 425 | 2-Methylbenzo-1,3-dithiole | 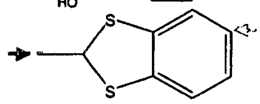 | 439 | 2-Hexaprenyl-3-methyl-6-methoxy-1,4-benzoquinone | 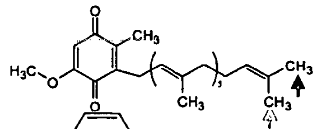 |
| 426 | 2-Methylbutanoyl-CoA | 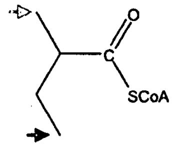 | 440 | 2-Naphthoic acid | 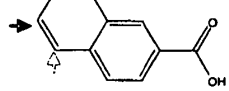 |
| 427 | S-Glutaryldihydrolipoamide | 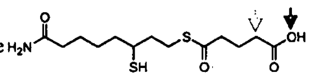 | 441 | 2-Nitroaniline | 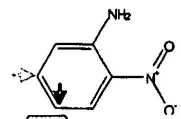 |
| 428 | cis-2-Methylaconitate | 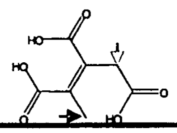 | 442 | 2-Nitronaphthalene | 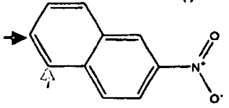 |

| N° | Substrate | Chemical Structure | N° | Substrate | Chemical Structure |
|---|---|---|---|---|---|
| 443 | 1-Methyl-2-nitrobenzene | 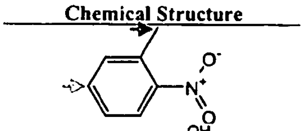 | 457 | 2-Octanoyl-glycerol | 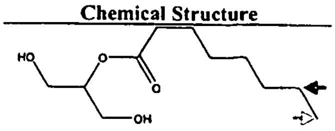 |
| 444 | 2-Methylserine | 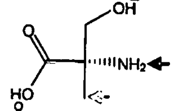 | 458 | 2-Octanol | 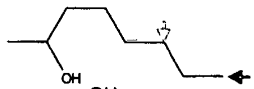 |
| 445 | 2-Octaprenyl-3-methyl-5-hydroxy-6-methoxy-1,4-benzoquinone | 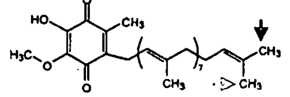 | 459 | 2-Phenylphenol | 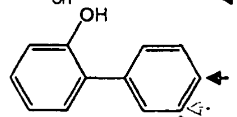 |
| 446 | 2-Octaprenyl-6-methoxyphenol | 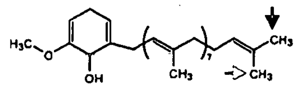 | 460 | 2-Thiocyanatoethylbenzene | 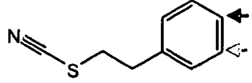 |
| 447 | 2-Nonadecanol | 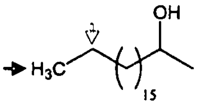 | 461 | 1-Monobutanoyl-glycerol | 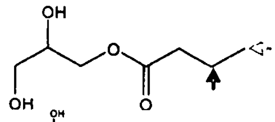 |
| 448 | 2-Nonadecanoyl-glycerol | 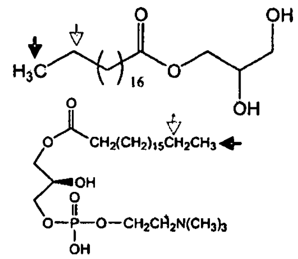 | 462 | 2-Octaprenyl-6-methoxy-1,4-benzoquinol | 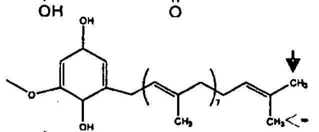 |
| 449 | 1-Nonadecanoyl-sn-glycero-phosphocholine | | 463 | 2-Octaprenyl-3-methyl-5-hydroxy-6-methoxy-1,4-benzoquinol | 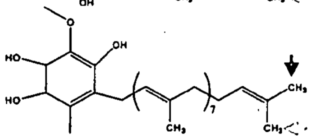 |
| 450 | 2-Nonanoyl-glycerol | 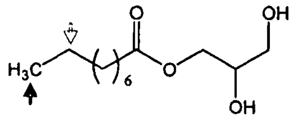 | 464 | 2-Octaprenyl-3-methyl-6-methoxy- 1,4-benzoquinol | 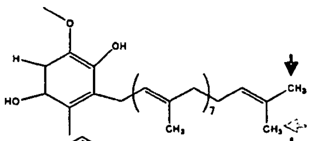 |
| 451 | 2-Nonanol | 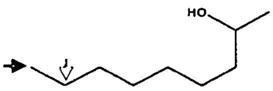 | 465 | 2-Octaprenyl-6-methoxyphenol | 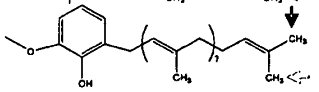 |
| 452 | 2-Nonaprenyl-6-hydroxyphenol | 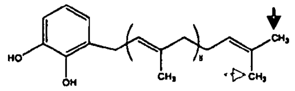 | 466 | 2-Oxooctadecanoate | 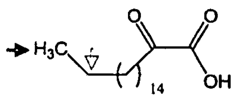 |
| 453 | 2-Nonaprenylphenol | 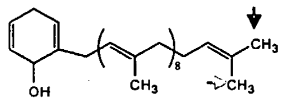 | 467 | 2-Octaprenylphenol | 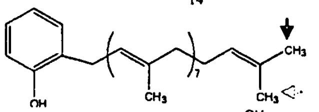 |
| 454 | 2-Oxobutanoate | 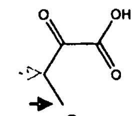 | 468 | 3-Phenylbutan-1-ol | 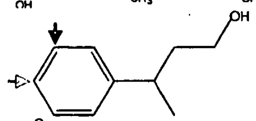 |
| 455 | 2-Octadecanoyl-glycerol | 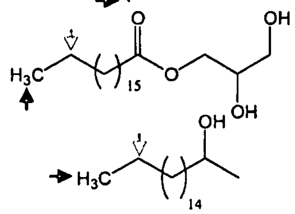 | 469 | 2-Phospho-4-(cytidine 5'-diphospho)-2-C-methyl-D-erythritol | 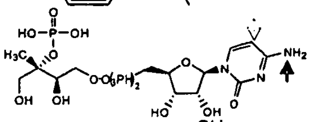 |
| 456 | 2-Octadecanol | | 470 | 2-Pentanol | 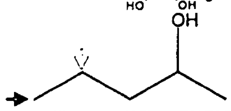 |

| N° | Substrate | Chemical Structure | N° | Substrate | Chemical Structure |
|---|---|---|---|---|---|
| 471 | D-Glycerate 2-phosphate | 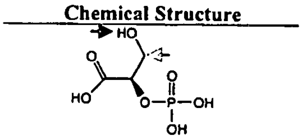 | 486 | 2-Tetradecanoyl-glycerol | 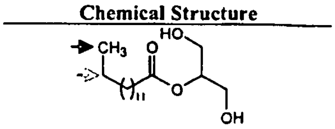 |
| 472 | 2-Propyl-glycerol | 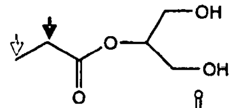 | 487 | 2-Tetracosanol | 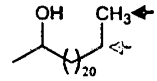 |
| 473 | 2-Phosphoglycolate | 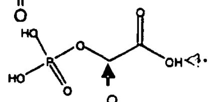 | 488 | 2-Tetradecanol | 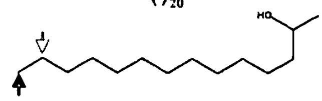 |
| 474 | 2-Pyrone-4,6-dicarboxylate | 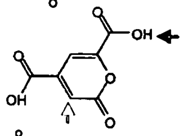 | 489 | 2-Methyl-4,5-dihydro-1,3-thiazole | 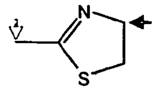 |
| 475 | 2-Octaprenyl-6-methoxy-1,4-benzoquinone | 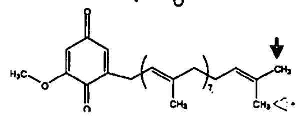 | 490 | 2-Undecanol | 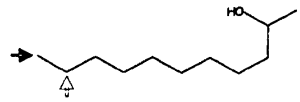 |
| 476 | Propionaldehyde | 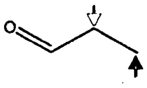 | 491 | 1-Chloro-2-[1-(4-chlorophenyl)ethenyl]benzene | 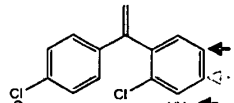 |
| 477 | 2-Propen-1-ol | 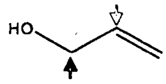 | 492 | L-Dicysteine | 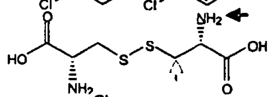 |
| 478 | 4,5-Dihydro-1H-pyrazole | 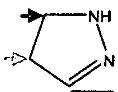 | 493 | 3,4,5-Trichlorophenol | 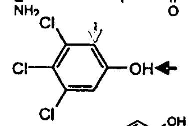 |
| 479 | 2-Pyrone | 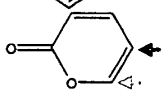 | 494 | 3,4,5-Trihydroxy-3,7-dimethoxyflavone | 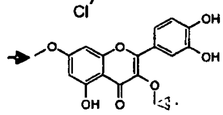 |
| 480 | 2,3-Dihydro-1H-pyrrole | 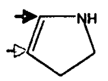 | 495 | 3,4-Dichloroaniline | 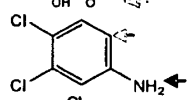 |
| 481 | 2S-Flavanone | 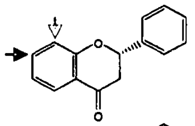 | 496 | 1,3-Dichloro-2-phenylbenzene | 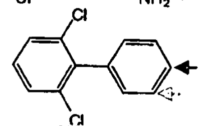 |
| 482 | 2S-Flavan-4-ol | 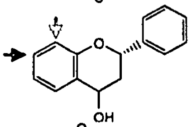 | 497 | 3,4-Dihydroxymandelate | 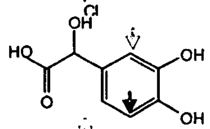 |
| 483 | (1R,6R)-2-Succinyl-6-hydroxy-2,4-cyclohexadiene-1-carboxylate | 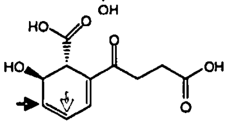 | 498 | 3,4-Dihydroxyphenylacetaldehyde | 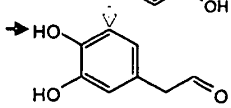 |
| 484 | 2-S-isocapryloyl-3R-hydroxymethyl-?-butyrolactone | 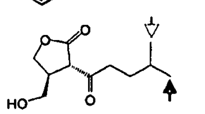 | 499 | 3,4-Dihydroxy-trans-cinnamate | 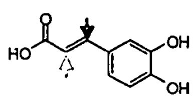 |
| 485 | 2-Octaprenyl-6-hydroxyphenol | 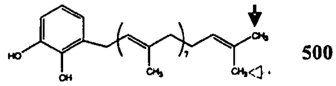 | 500 | 3,4-Dihydroxybenzoate | 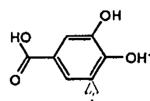 |

| N° | Substrate | Chemical Structure | N° | Substrate | Chemical Structure |
|---|---|---|---|---|---|
| 501 | Cystathionine | 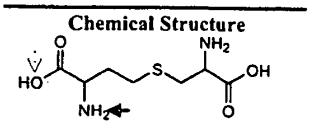 | 516 | 3-Chloro-4-hydroxy-phenylacetate | 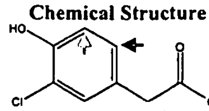 |
| 502 | 3,4-Dihydroxyphenylacetate | 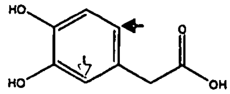 | 517 | 3-Carboxy-4-methyl-2-oxopentanoate | 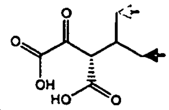 |
| 503 | 3,4-Dichloro-1-butene | 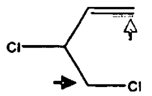 | 518 | 4-Chlorobenzoate | 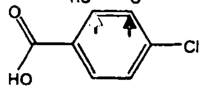 |
| 504 | 3,4-Dihydro-1 (2H) naphthalene | 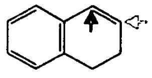 | 519 | 1-Chloro-3-phenylbenzene | 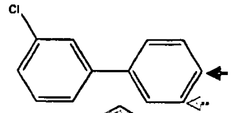 |
| 505 | 3-(3,4-Dihydroxyphenyl)lactate | 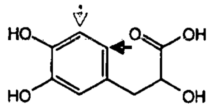 | 520 | 1-Chloro-3-ethenylbenzene | 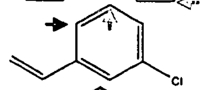 |
| 506 | 3-(4 Hydroxyphenyl)pyruvate | 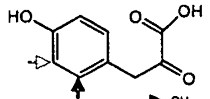 | 521 | 3-Chlorotoluene | 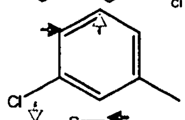 |
| 507 | 3,5-Dihydroxybenzoic acid | 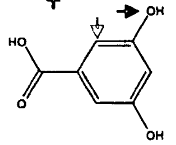 | 522 | 3-Carbamoyloxymethyl cephem | 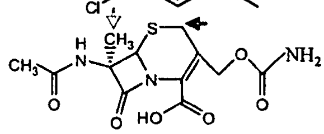 |
| 508 | 1,3-Dichloro-5-phenylbenzene | 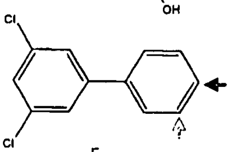 | 523 | 3-Deoxy-arabino-heptulonate 7-phosphate | 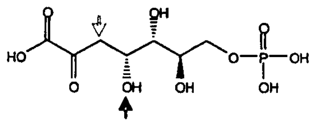 |
| 509 | 2-Fluorobenzoate | 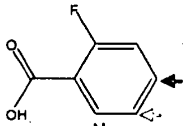 | 524 | 3-Dehydrocarnitine | 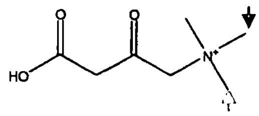 |
| 510 | 3-Amino-1,2,4-triazole | 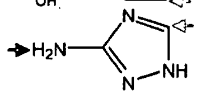 | 525 | 3-Dehydro-L-gulonate 6-phosphate | 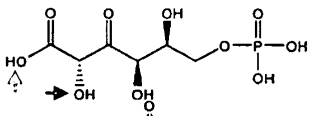 |
| 511 | 3-Amino-5-deoxyfructose 6-phosphate | 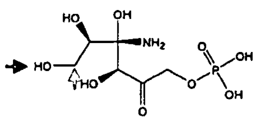 | 526 | 3-Dehydroquinate | 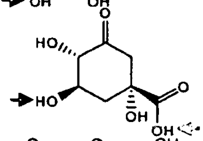 |
| 512 | 3-Fluorobenzoate | 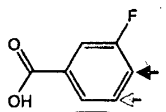 | 527 | 3-Dehydro-L-gulonate | 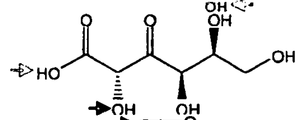 |
| 513 | Monochlorobenzene | 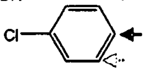 | 528 | 1,4,2-Oxathiazole | 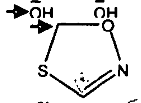 |
| 514 | 3-Carboxy-2-hydroxy-4-methylpentanoate | 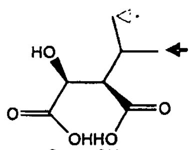 | 529 | 2,3-Dichloropentane | 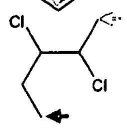 |
| 515 | 3-Carboxy-3-hydroxy-4-methylpentanoate | 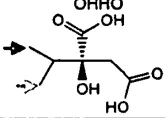 | 530 | 3-Dehydro-L-threonate | 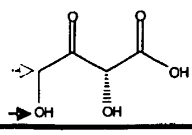 |

| N° | Substrate | Chemical Structure | N° | Substrate | Chemical Structure |
|---|---|---|---|---|---|
| 531 | 3-Demethylubiquinone-9 | 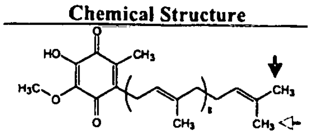 | 545 | 3-Hydroxybenzaldehyde | 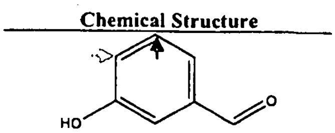 |
| 532 | 3-Ethylcatechol | 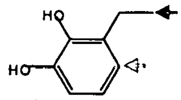 | 546 | 2-Hydroxybenzyl alcohol | 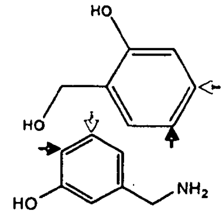 |
| 533 | 3H-Dioxazole | 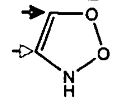 | 547 | 3-Hydroxybenzyl amine | |
| 534 | 3H-1,2-Dithiole | 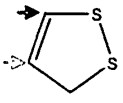 | 548 | 3-Hydroxybenzoate | 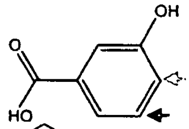 |
| 535 | 3-Dehydroshikimate | 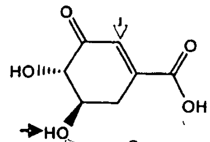 | 549 | 3-Hydroxycinnamate | 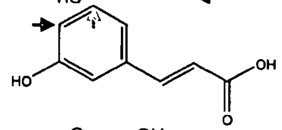 |
| 536 | 2H-1,3-Dithiole | 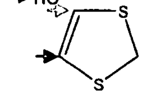 | 550 | (3S)-3-Hydroxyhexacosyl-CoA | 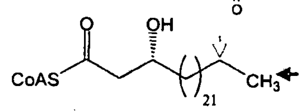 |
| 537 | 3-Hydroxy-2-benzopyrone | 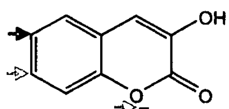 | 551 | 3-Hydroxybutan-2-one | 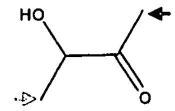 |
| 538 | (2S,3S)-3-Hydroxy-2-methylbutanoyl-CoA | 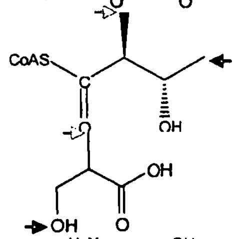 | 552 | (3S)-3-Hydroxydodecanoyl-CoA | 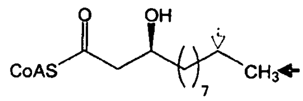 |
| 539 | 3-Hydroxy-2-methylpropanoate | | 553 | (3S)-3-Hydroxyhexadecanoyl-CoA | 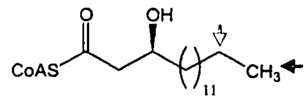 |
| 540 | 3-Hydroxyanthranilate | 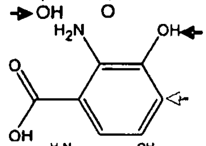 | 554 | 3H-Indole | 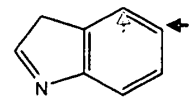 |
| 541 | 3-Hydroxy-4-methylanthranilate | 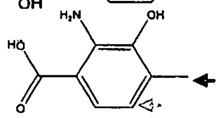 | 555 | 3-Hydroxymethylceph-3-em-4-carboxylate | 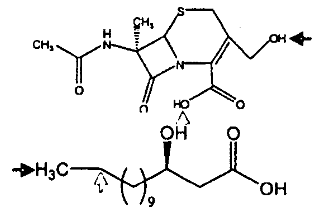 |
| 542 | 3-Hydroxyoxolan-2-one | 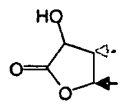 | 556 | (3R)-3-Hydroxytetradecanoic acid | |
| 543 | (2S)-2,4-Diamino-3-hydroxy-4-oxobutanoic acid | 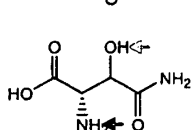 | 557 | 3-Hydroxy-muconate | 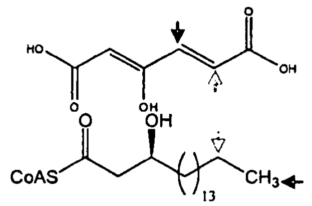 |
| 544 | (3S)-3-Hydroxydecanoyl-CoA | 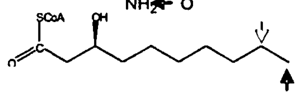 | 558 | (3S)-3-Hydroxyoctadecanoyl-CoA | |

| N° | Substrate | Chemical Structure | N° | Substrate | Chemical Structure |
|---|---|---|---|---|---|
| 559 | (3R)-3-Hydroxypalmitoyl-CoA | 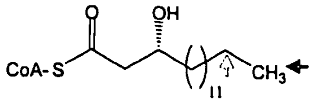 | 570 | 3-β-Hydroxysterol hexadecanoate | 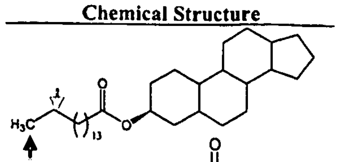 |
| 560 | 3-Hydroxyhexadecanoic acid | 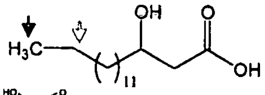 | 571 | 3-Hydroxypropanoate | 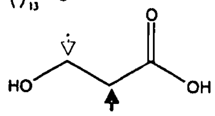 |
| 561 | 3-Hexaprenyl-4,5-dihydroxybenzoate | 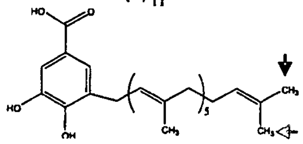 | 572 | 3-β-Hydroxysterol oleate | 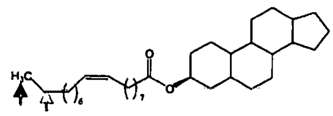 |
| 562 | 3-Hydroxypimeloyl-CoA | 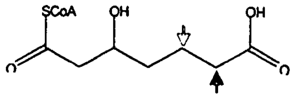 | 573 | 3-β-Hydroxysterol tetradecanoate | 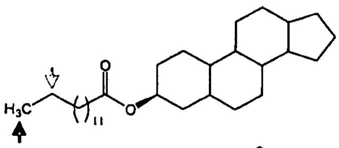 |
| 563 | 3-Hexaprenyl-4-hydroxy-5-methoxybenzoate | 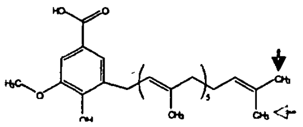 | 574 | 3-beta-Hydroxysterol decanoate | 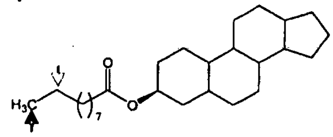 |
| 564 | 3-Hydroxypropanal | 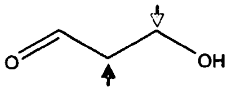 | 575 | 3-beta-Hydroxysterol octanoate | 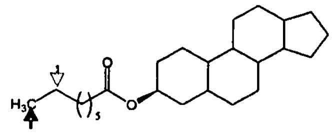 |
| 565 | 3-(3-Hydroxy-phenyl)-propanoic acid | 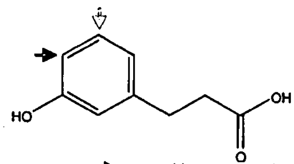 | 576 | S-3-Imidazol-5-yllactate | 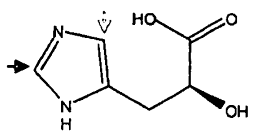 |
| 566 | 3H-Pyrrole | 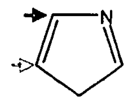 | 577 | 3-Imidazol-5-ylpyruvate | 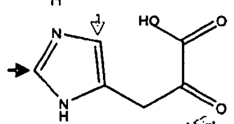 |
| 567 | (3S)-3-Hydroxytetradecanoyl-CoA | 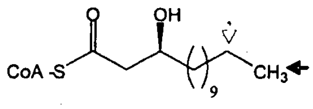 | 578 | 3-Methylbutanol | 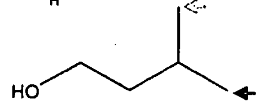 |
| 568 | 3-β-Hydroxysterol dodecanoate | 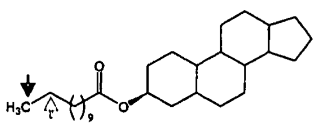 | 579 | 3-Indoleacetonitrile | 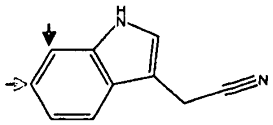 |
| 569 | 3-β-Hydroxysterol hexanoate | 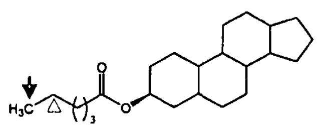 | 580 | 3-Methylbutan-2-ol | 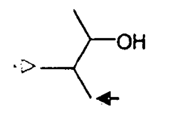 |

| N° | Substrate | Chemical Structure | N° | Substrate | Chemical Structure |
|---|---|---|---|---|---|
| 581 | 3-Methoxy-4-hydroxy-trans-cinnamate | 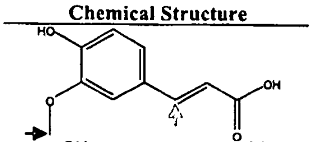 | 593 | (S)-3-Methyl-2-oxopentanoate | 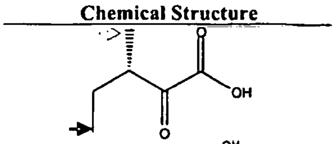 |
| 582 | Glutaconyl-CoA | 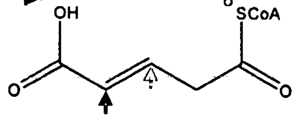 | 594 | 3-Methoxy-5,7,3',4'-tetrahydroxyflavone | 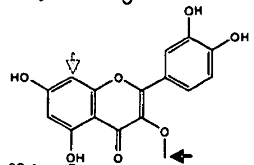 |
| 583 | 3-Methyl benzyl alcohol | 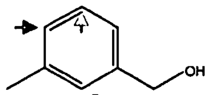 | 595 | 3-Oxodecanoyl-CoA | 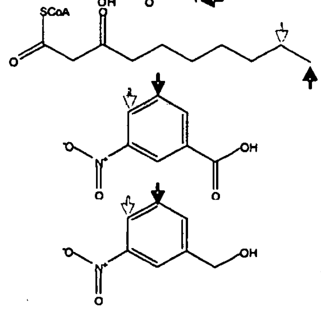 |
| 584 | 3-Methylbenzothiophene | 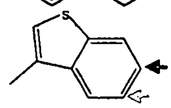 | 596 | 3-Nitrobenzoic acid | |
| 585 | C1-(3-Indolyl)-glycerol 3-phosphate | 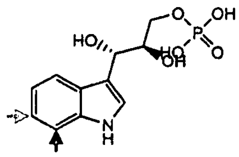 | 597 | 3-Nitrobenzyl alcohol | |
| 586 | 3-Methylbutanoyl-CoA | 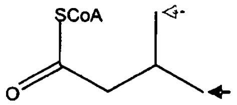 | 598 | 2,4,6-Trinitrotoluene | 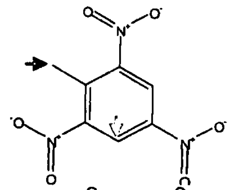 |
| 587 | 3-Hydroxytoluene | 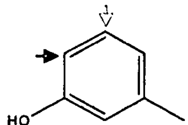 | 599 | 3-Oxobutyryl-CoA | 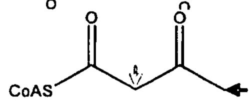 |
| 588 | 3-(Methoxycarbonyl)pent-2-enedioate | 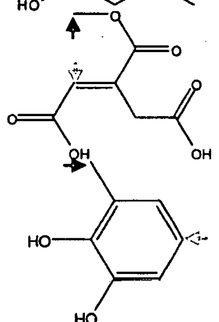 | 600 | 4-Nitroanilide | 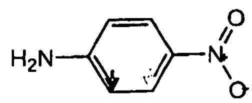 |
| 589 | 3-Methylcatechol | | 601 | 3-Oxododecanoyl-CoA | 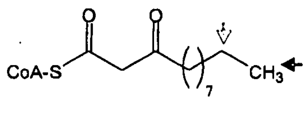 |
| 590 | trans-2-(4-Nitrophenyl)-3-phenyloxirane | 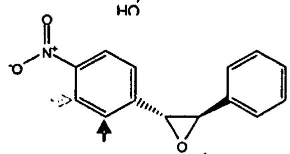 | 602 | 3-Oxohexanoyl-CoA | 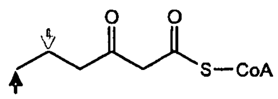 |
| 591 | 3-Methyl-2-oxobutanoate | 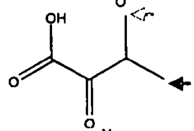 | 603 | 3-Oxohexadecanoyl-CoA | 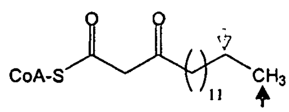 |
| 592 | 3-Methylquinoline | 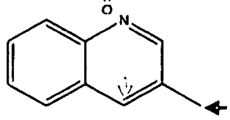 | 604 | 3-Oxooctadecanoyl-CoA | 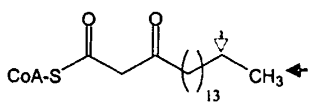 |

| N° | Substrate | Chemical Structure | N° | Substrate | Chemical Structure |
|---|---|---|---|---|---|
| 605 | 3-Oxohexacosyl-CoA | 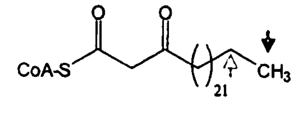 | 618 | 5-O-(1-Carboxyvinyl)-3-phosphoshikimate | 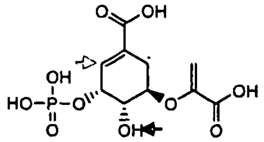 |
| 606 | 3-Oxooctanoyl-CoA | 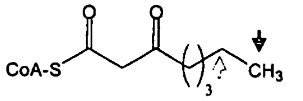 | 619 | 3-Phosphonooxypyruva te | 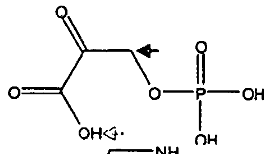 |
| 607 | 3-Oxopropanoate | 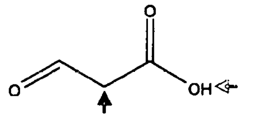 | 620 | 2,5-dihydro-1H-pyrrole | 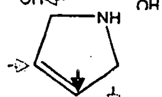 |
| 608 | 2,3-Didehydro-pimeloyl-CoA | 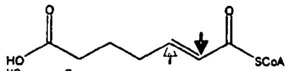 | 621 | (3R)-3-Hydroxy-butanoyl-CoA | 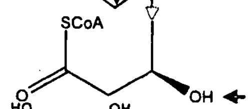 |
| 609 | 3-Octaprenyl-4-hydroxybenzoate | 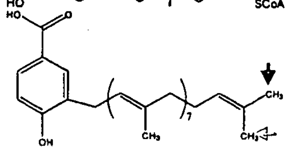 | 622 | 3-Sulfo-catechol | 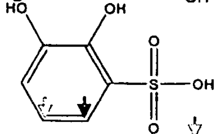 |
| 610 | 3-Oxotetradecanoyl-CoA | 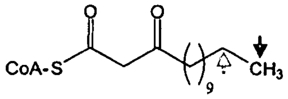 | 623 | 4,4'-Dichlorobiphenyl | 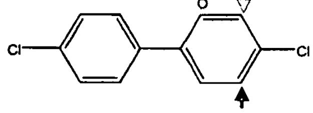 |
| 611 | 3-Oxoadipyl-CoA | 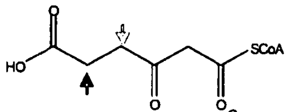 | 624 | 3-Thiooctanoyl-CoA | 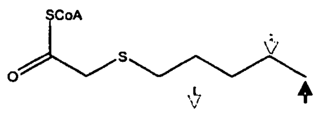 |
| 612 | 3-Oxoadipate | 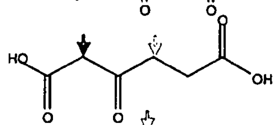 | 625 | (3S)-3-Hydroxy-butanoyl-CoA | 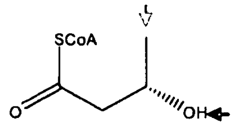 |
| 613 | 3-Oxoadipate enol-lactone | 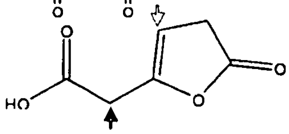 | 626 | 4-Acetamidobutanoate | 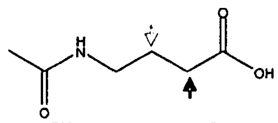 |
| 614 | 3-Phospho-D-glycerate | 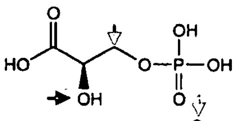 | 627 | (4S,5S)-4,5-Dihydroxy-2,6-dioxohexanoate | 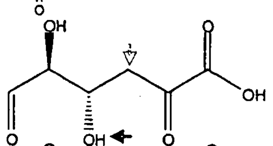 |
| 615 | 3-Phenyl-1-propanol | 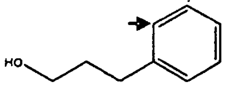 | 628 | (4S)-4,6-Dihydroxy-2,5-dioxohexanoate | 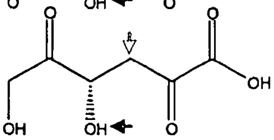 |
| 616 | 3-Phosphohydroxypyruvat e | 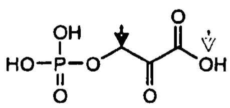 | 629 | 4-Acetamidobutanoate | 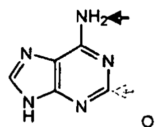 |
| 617 | 3-Phenyl-propionic acid | 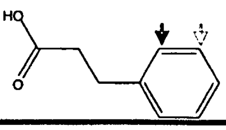 | 630 | 4-Aminobutanoate | 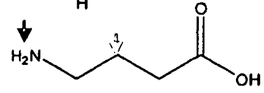 |

| N° | Substrate | Chemical Structure | N° | Substrate | Chemical Structure |
|---|---|---|---|---|---|
| 631 | 4-Aminobutanal | 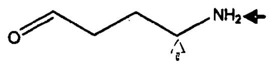 | 644 | 4-Carboxy-2-hydroxy-cis,cis-muconate | 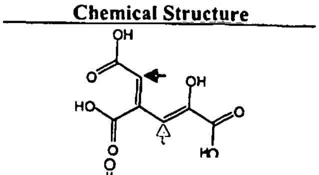 |
| 632 | 4-Aminobenzoate | 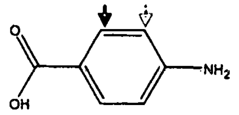 | 645 | 4-Carboxy-2-hydroxy-muconate semialdehyde | 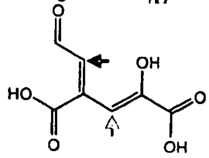 |
| 633 | 4-Amino-4-deoxychorismate | 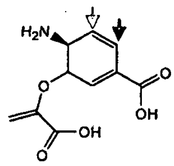 | 646 | 4-(Cytidine 5'-diphospho)-2-C-methyl-D-erythritol | 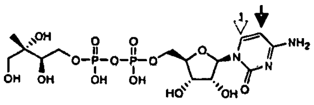 |
| 634 | 4-Amino-5-hydroxymethyl-2-methylpyrimidine | 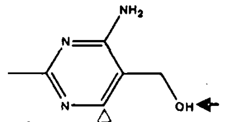 | 647 | 4-Carboxy-2-oxo-4-pentanoate | 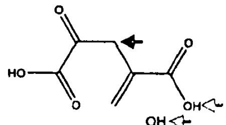 |
| 635 | 4-Amino-2-methyl-5-phosphomethylpyrimidine | 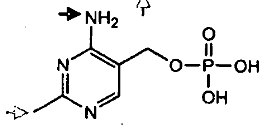 | 648 | 4-Carboxy-4-hydroxy-2-oxoadipate | 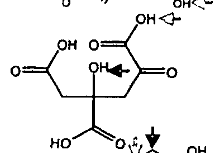 |
| 636 | 4-Bromo-1-butanol | 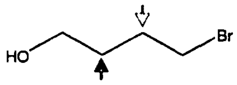 | 649 | 5,7,4'-Trihydroxy-3'-methoxyflavone | 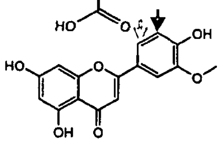 |
| 637 | 4-Bromobenzoate | 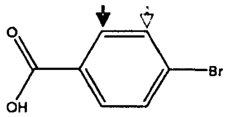 | 650 | 4-Chlorobenzoyl-CoA | 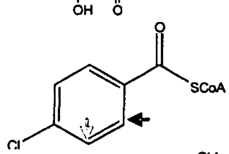 |
| 638 | Bromobenzene-2,3-dihydrodiol | 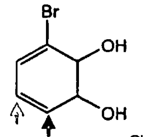 | 651 | 1-Chloro-4-ethenylbenzene | 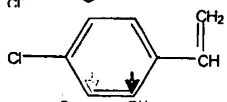 |
| 639 | 4-Bromocatechol | 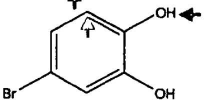 | 652 | 4-Dehydropantoate | 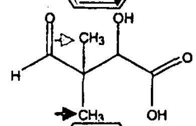 |
| 640 | 4-Bromophenol | 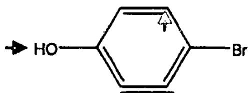 | 653 | 4-Chlorotoluene | 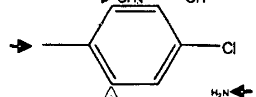 |
| 641 | 1-Bromo-4-methylbenzene | 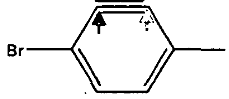 | 654 | 5-Amino-6-(ribosylamino)-2,4-(1H,3H)-pyrimidinedione 5'-phosphate | 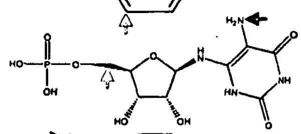 |
| 642 | 4-Dimethylaminophenol | 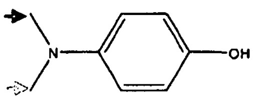 | 655 | 4-Ethyl phenol | 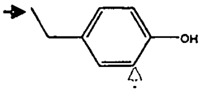 |
| 643 | 4-Carboxy-2-hydroxyhexa-2,4-dienedioate | 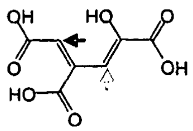 | 656 | 1-Fluoro-4-methylbenzene | 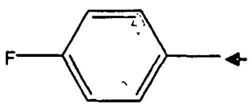 |

| N° | Substrate | Chemical Structure | N° | Substrate | Chemical Structure |
|---|---|---|---|---|---|
| 657 | Diazepine | 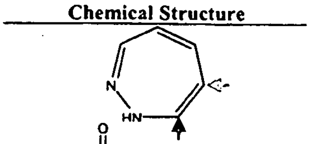 | 671 | 4-(Hydroxymethyl)phenol | 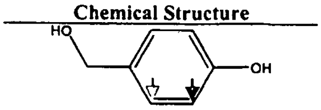 |
| 658 | 4-Hydroxy-2-oxopentanoate | 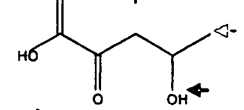 | 672 | 4-Hydroxybenzoylformate | 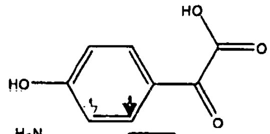 |
| 659 | 4-Hydroxy-2-oxoglutarate | 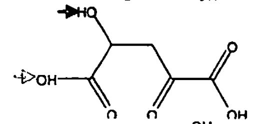 | 673 | 4-(Aminomethyl)phenol | 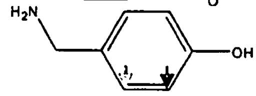 |
| 660 | 4-Hydroxymandelate | 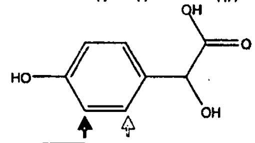 | 674 | 4-Hydroxybenzoyl-CoA | 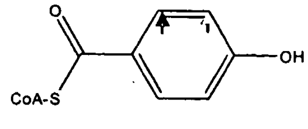 |
| 661 | Biphenyl | 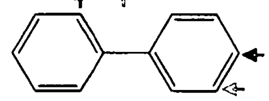 | 675 | 4-Hydroxybenzaldehyde | 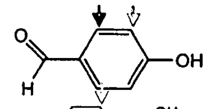 |
| 662 | 4-Hydroxy-2-butynal | 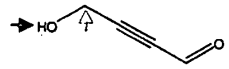 | 676 | 3-Methoxy-4-hydroxyman- | 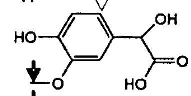 |
| 663 | 3-Methyl-1,3-oxazinane | 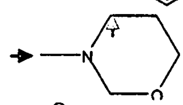 | 677 | 4-Hydroxybutanoate | 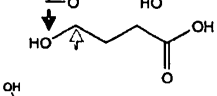 |
| 664 | 6-Methyl-3,4-dihydro-2H-1,4-benzoxazine | 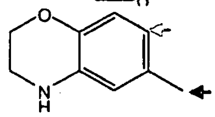 | 678 | 4-Hydroxycinnamate | 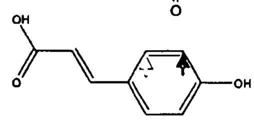 |
| 665 | 4-Phenyl-1,3-oxazinane-2-thione | 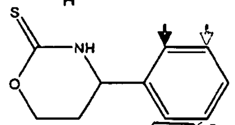 | 679 | 4-Hydroxy-L-threonine | 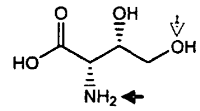 |
| 666 | 1,3-Benzoxazine-2,4-dione | 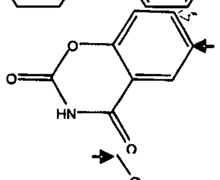 | 680 | D-4-Hydroxyphenylglycine | 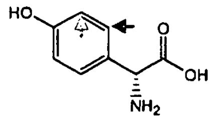 |
| 667 | 4-Hydroxy-3-methoxybenzoate | 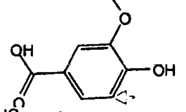 | 681 | 3-Hydroxybenzyl alcohol | 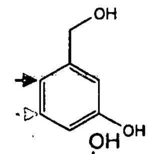 |
| 668 | 4-Hydroxy-3-methoxycinnamate | 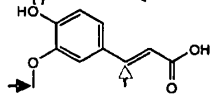 | 682 | 3-Hydroxy-2-formylbenzothiophene | 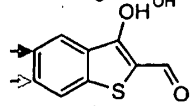 |
| 669 | (1S,4S)-4-Hydroxy-3-oxocyclohexane-1-carboxylate | 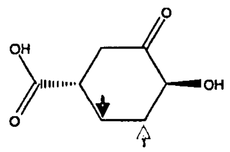 | 683 | L-4-Hydroxyglutamate semialdehyde | 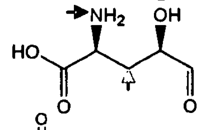 |
| 670 | 4-Hydroxy-4-methyl-2-oxoglutarate | 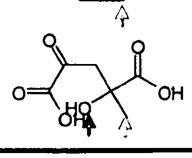 | 684 | 4-Methyl-2-oxopentanoate | 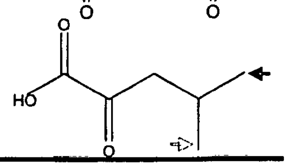 |

| N° | Substrate | Chemical Structure | N° | Substrate | Chemical Structure |
|---|---|---|---|---|---|
| 685 | 4-Hydroxybenzoate | 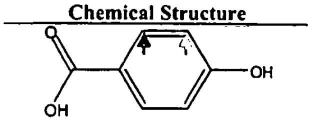 | 699 | 2-Hydroxy-6-oxo-octa-2,4-dienoate | 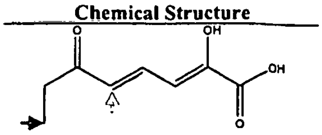 |
| 686 | 3-Hydroxy-3-methyl-2-oxopentanoate | 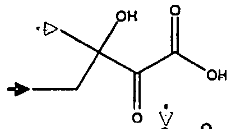 | 700 | 4-Methyl-5-2-phosphoethyl-thiazole | 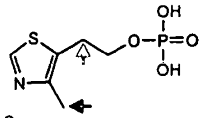 |
| 687 | 4-Methoxy-57-dihydroxyflavone | 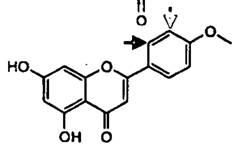 | 701 | 4-Methylumbelliferyl glucuronide | 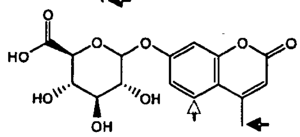 |
| 688 | 4-Methylbenzaldehyde | 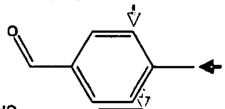 | 702 | 4-Nitrophenol | 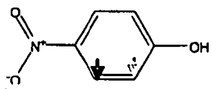 |
| 689 | 4-Methyl benzyl alcohol | 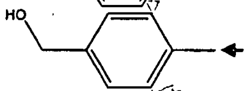 | 703 | 4-Nitroaniline | 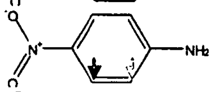 |
| 690 | 3-Methylbutanal | 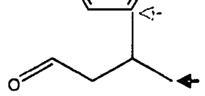 | 704 | 4-Nitrotoluene | 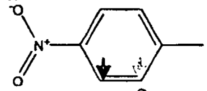 |
| 691 | 4-Methylcatechol | 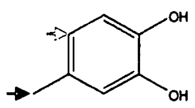 | 705 | 4-Oxalocrotonate | 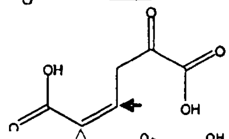 |
| 692 | Methyl-cyclohexane | 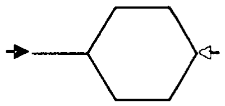 | 706 | 4-Oxalomesaconate | 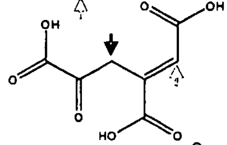 |
| 693 | 4-Methyl-cyclohexanol | 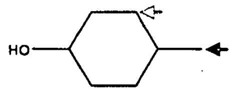 | 707 | 4-Oxoproline | 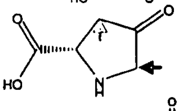 |
| 694 | 4-Methylcyclohexan-1-one | 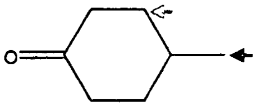 | 708 | 4-Phospho-L-aspartate | 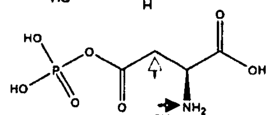 |
| 695 | 4-Methylcycloheptan-1-ol | 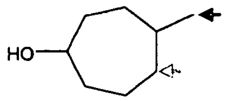 | 709 | 4-Phospho-D-erythronate | 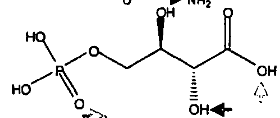 |
| 696 | 4-Methylphenol | 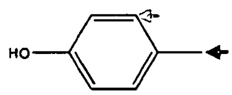 | 710 | D-4-Phosphopantothenate | 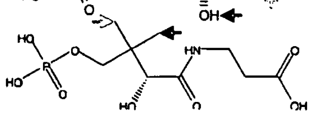 |
| 697 | 5-(2-Hydroxyethyl)-4-methylthiazole | 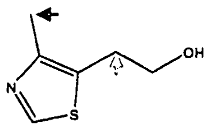 | 711 | (R)-4'-Phosphopantothenoyl-L-cysteine | 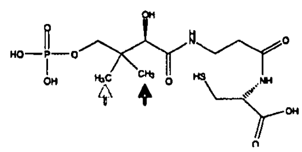 |
| 698 | 4-O-Methylisoflavone | 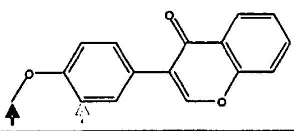 | 712 | Pyran-4-one | 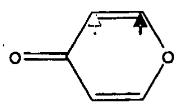 |

| N° | Substrate | Chemical Structure | N° | Substrate | Chemical Structure |
|---|---|---|---|---|---|
| 713 | 5-O-Methyl-myo-inositol | 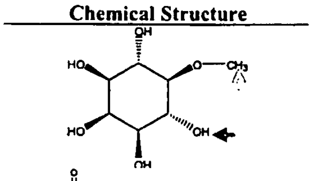 | 725 | Benzoyl acetyl-CoA | 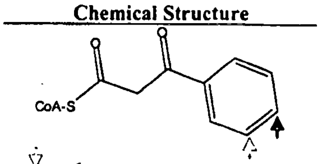 |
| 714 | 4-(1-D-Ribitylamino)-5-aminouracil | 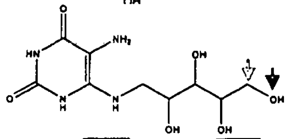 | 726 | 5-Benzamido-2-benzyl-4-oxopentanoate | 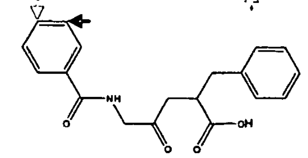 |
| 715 | 4-Chlorobiphenyl | 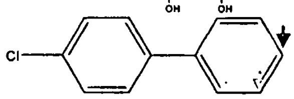 | 727 | 5-Bromo-4-chloro-3-indoyl phosphate | 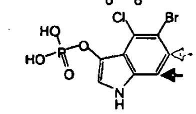 |
| 716 | 5,7,4'-Trihydroxy-flavone | 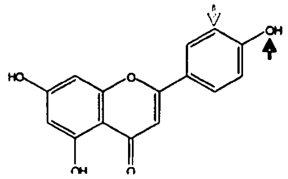 | 728 | 5-Phosphoribosyl-5-carboxyaminoimidazole | 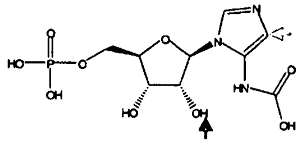 |
| 717 | 2-Aminodecanoic acid | 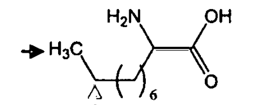 | 729 | 5-Dehydro-D-fructose | 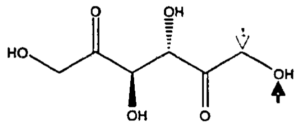 |
| 718 | 1-(5-Phospho-D-ribosyl)-5-amino-4-imidazolecarboxylate | 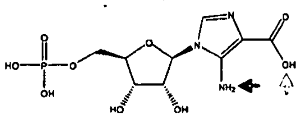 | 730 | 2-Dehydro-D-glucono-1,5-lactone | 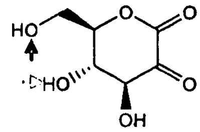 |
| 719 | 2,2'-Dihydroxybiphenyl | 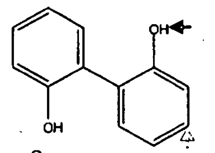 | 731 | 5-Dehydro-D-gluconate | 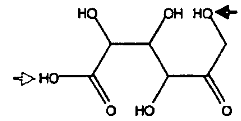 |
| 720 | 5-Amino-4-oxopentanoate | 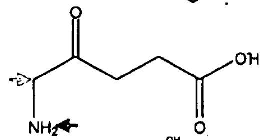 | 732 | 5'-Fluoro-5'-deoxyadenosine | 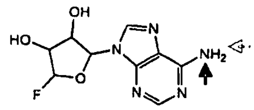 |
| 721 | 5-Amino-6-(5'-phosphoribitylamino)uracil | 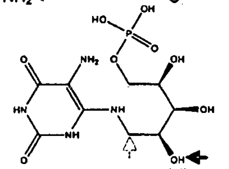 | 733 | 5-Dehydro-4-deoxy-D-glucarate | 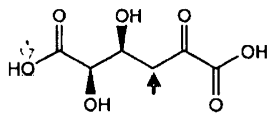 |
| 722 | 5-Amino-6-(5'-phosphoribosylamino)uracil | 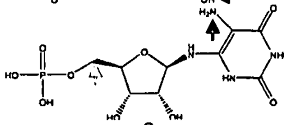 | 734 | 1-(5'-Phosphoribosyl)-5-formamido-4-imidazolecarboxamide | 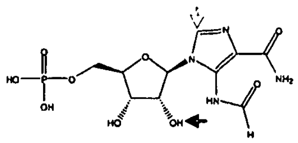 |
| 723 | 5-Amino-4-oxooctanoate | 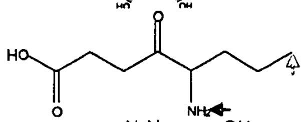 | 735 | 5H-1,2,5-Oxathiazole | 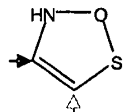 |
| 724 | 2-Aminohexadecanoic acid | 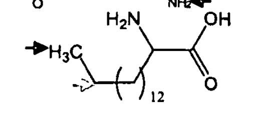 | 736 | 5-Hydroxyfuranocoumarin | 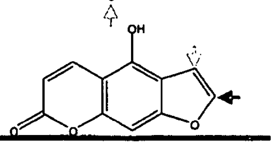 |

| N° | Substrate | Chemical Structure | N° | Substrate | Chemical Structure |
|---|---|---|---|---|---|
| 737 | S-Methyl-5-thio-5-deoxy-D-ribose 1-phosphate | 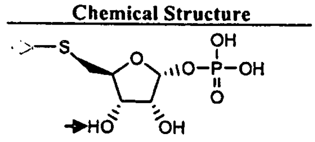 | 750 | 6-Acetyl-β-D-galactoside | 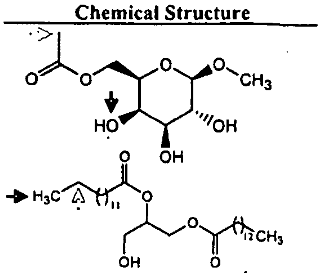 |
| 738 | 2,5-Diamino-6-(5'-triphosphoryl-3',4'-trihydroxy-2'-oxopentyl)-amino-4-oxopyrimidine | 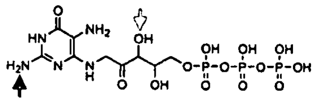 | 751 | 1,2-Ditetradecanoyl-sn-glycerol | |
| 739 | 5-Methylthio-D-ribose-1-phosphate | 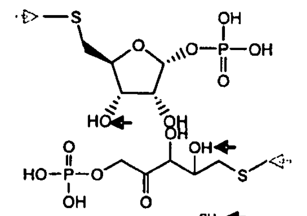 | 752 | 6-Chloro-3-indolyl -D-galactopyranoside | 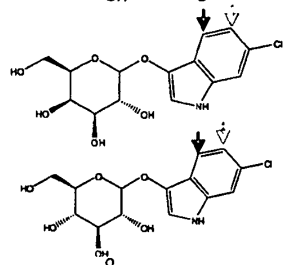 |
| 740 | 5-Methylthio-5-deoxy-D-ribulose 1-phosphate | | 753 | 6-Chloro-3-indolyl -D-glucopyranoside | |
| 741 | 8-Methoxy furanocoumarin | 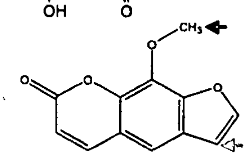 | 754 | 6-S-Acetyldihydrolipoamide | 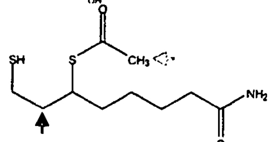 |
| 742 | 5-Methoxyfuranocoumarin | 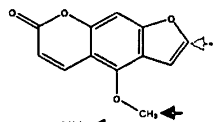 | 755 | Melibiose | 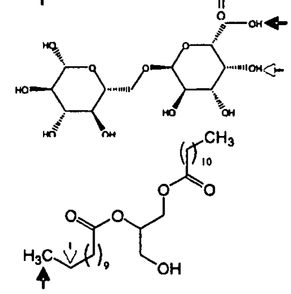 |
| 743 | 5-Methylthioadenosine | 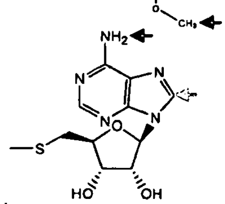 | 756 | 1,2-Didodecanoyl-sn-glycerol | |
| 744 | 5-Methyltetrahydrofolate | 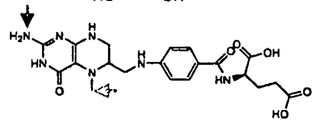 | 757 | 2,3,6-trimethyl-1,3-oxazinane | 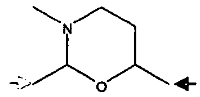 |
| 745 | 6-Hydroxyhexanoate | 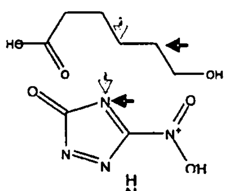 | 758 | 5-Methylthio-D-ribose | 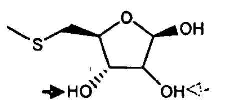 |
| 746 | 5-Nitro-1,2,4-triazol-3-one | | 759 | 6-Hydroxymethyl 7,8-dihydropterin | 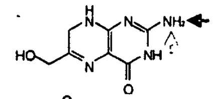 |
| 747 | 2(3H)-oxazolone | 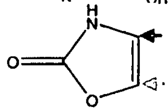 | 760 | 6-Hydroxymethyl-dihydropterin pyrophosphate | 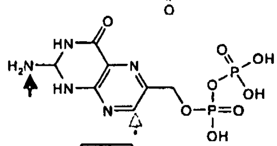 |
| 748 | N1-(5-Phospho-α-D-ribosyl)-5,6-dimethylbenzimidazole | 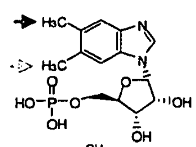 | 761 | 6-Oxohexanoate | 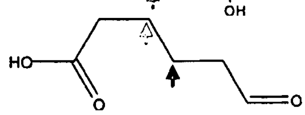 |
| 749 | 4-Pyridoxolactone | 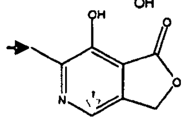 | 762 | 6-Phospho-2-dehydro-D-gluconate | 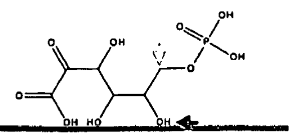 |

| N° | Substrate | Chemical Structure | N° | Substrate | Chemical Structure |
|---|---|---|---|---|---|
| 763 | 2'-Aminobiphenyl-2,3-diol | 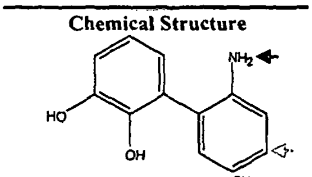 | 775 | 9,10-Dihydrophenanthrene | 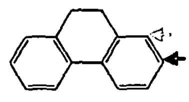 |
| 764 | 6-Phospho-D-glucono-1,5-lactone | 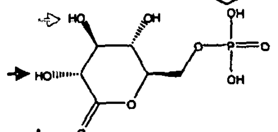 | 776 | Acyclic-7-deazapurine | 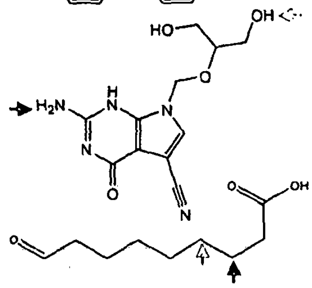 |
| 765 | 6-Pyruvoyltetrahydropterin | 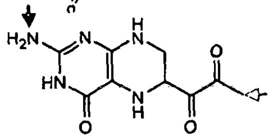 | 777 | 9-Oxononanoate | |
| 766 | 5-Hydroxy-L-tryptophan | 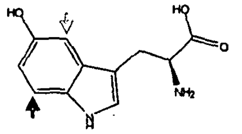 | 778 | 8-Amino-7-oxononanoate | 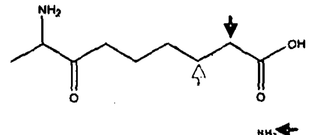 |
| 767 | 1-Deaza-5-azapurine | 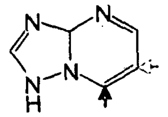 | 779 | Adenosine 5'-phosphosulfate | 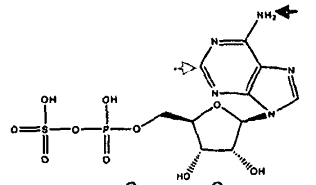 |
| 768 | 1-Deazapurine | 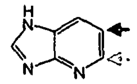 | 780 | 3-Acetoacetyl-CoA | 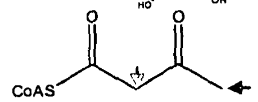 |
| 769 | 6-Phospho-D-gluconate | 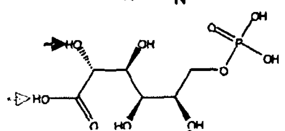 | 781 | Tri-L-Alanine | 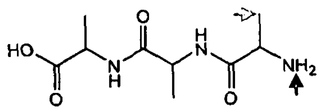 |
| 770 | 7H-pyrrolo[2,3-d]pyrimidine | 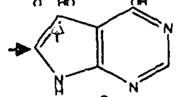 | 782 | Aminoacetaldehyde | 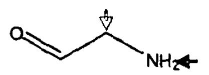 |
| 771 | Xanthen-9-one | 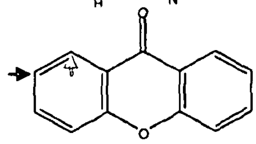 | 783 | (S)-5-Oxo-2,5-dihydrofuran-2-acetate | 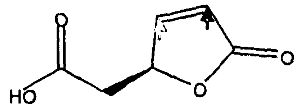 |
| 772 | 8-Hydroxyfuranocoumarin | 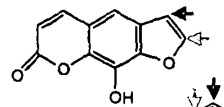 | 784 | 2,2-Azino-bis3-ethylbenzthiazoline-6-sulfonic acid | 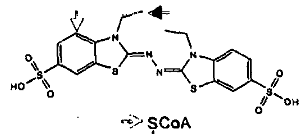 |
| 773 | Uridine 5'-triphosphate | 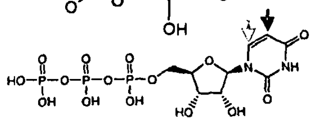 | 785 | Acetyl-CoA | 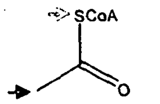 |
| 774 | 1,2-Anthracenediol | 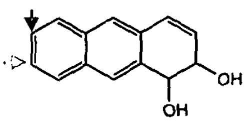 | 786 | N-Acetyl-D-glucosamine | 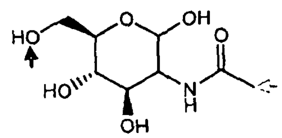 |

| N° | Substrate | Chemical Structure | N° | Substrate | Chemical Structure |
|---|---|---|---|---|---|
| 787 | Acetoacetate | 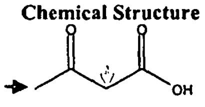 | 799 | 2-Hydroxy-3-oxoadipate | 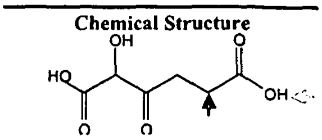 |
| 788 | Acetaldehyde | 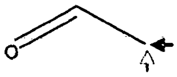 | 800 | N-Acetyl-D-glucosamine 6-phosphate | 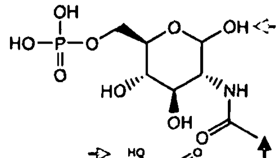 |
| 789 | O-Acetyl-carnitine | 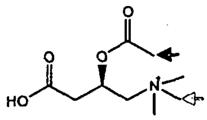 | 801 | N-Acetyl-L-glutamate | 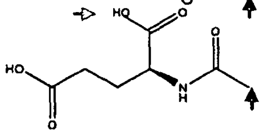 |
| 790 | Acetate | 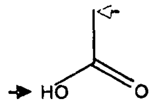 | 802 | 2-Amino-3-oxoadipate | 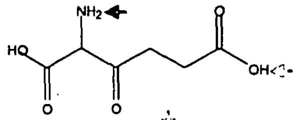 |
| 791 | Acenaphthalen-1-ol | 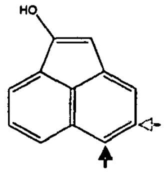 | 803 | Acenaphthalene | 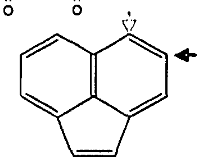 |
| 792 | N-Acetyl-D-mannosamine 6-phosphate | 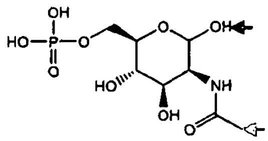 | 804 | N-Acetylneuraminate | 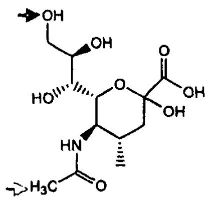 |
| 793 | Naphthyl-2-methyl-succinyl-CoA | 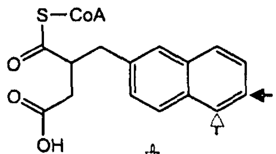 | 805 | 3-Carboxy-2-hydroxy-4-methylpentanoate | 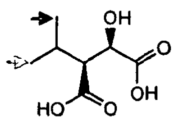 |
| 794 | 1-Phenylethanone | 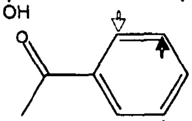 | 806 | cis-Aconitate | 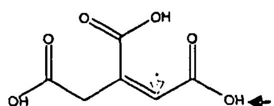 |
| 795 | Acetoin | 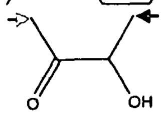 | 807 | N2-Acetyl-L-ornithine | 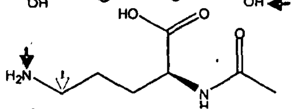 |
| 796 | N-Acetyl-L-glutamyl 5-phosphate | 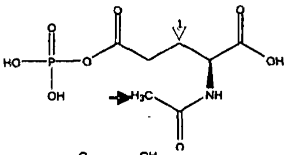 | 808 | N5-Propyl-L-ornithine ester | 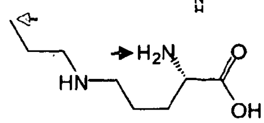 |
| 797 | N-Acetyl-L-glutamate 5-semialdehyde | 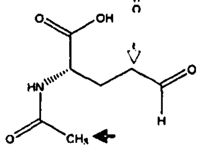 | 809 | Acryloyl-CoA | 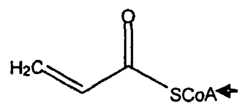 |
| 798 | Adenosylcobalamin 5'-phosphate | Too big to be incorporated | 810 | Acridine | 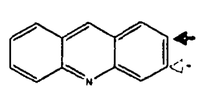 |

| N° | Substrate | Chemical Structure | N° | Substrate | Chemical Structure |
|---|---|---|---|---|---|
| 811 | Acrolein | 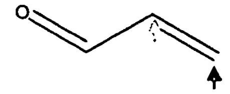 | 821 | Adenosyl cobinamide phosphate | 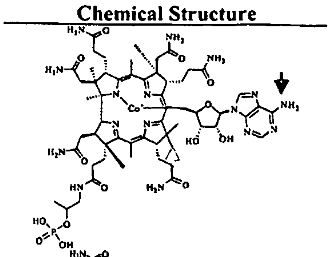 |
| 812 | O-Acetyl-L-serine | 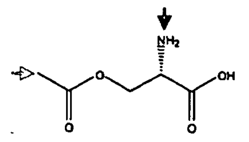 | 822 | Adenosylcobalamin | 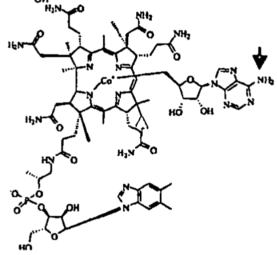 |
| 813 | Adenosine | 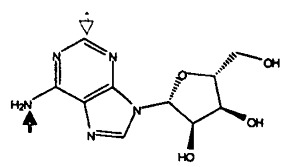 | 823 | ADP | 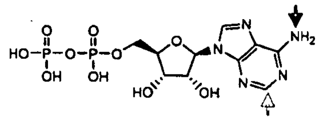 |
| 814 | N-Acetyl-D-glucosamine 1-phosphate | 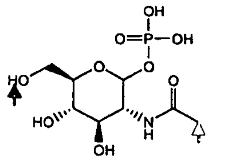 | 824 | ADP-L-glycero-D-manno-heptose | 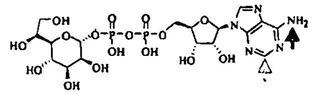 |
| 815 | N-Acetyl-D-mannosamine | 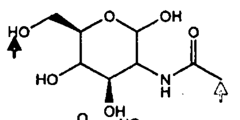 | 825 | ADP-D-glycero-D-manno-heptose | 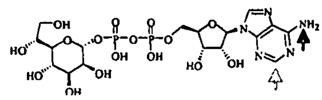 |
| 816 | Acetyl phosphate | 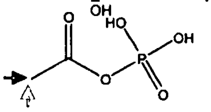 | 826 | Alginate | 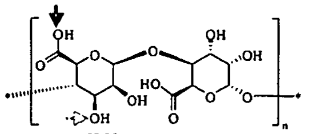 |
| 817 | Agmatine | 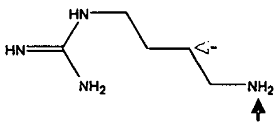 | 827 | 2-amino-4,6-dimethoxypyrimidine | 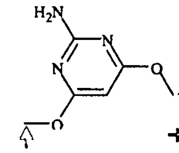 |
| 818 | 6-Aminopurine | 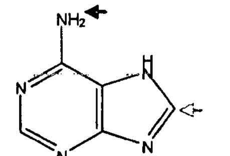 | 828 | ADP-ribose | 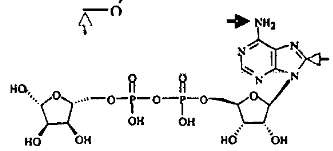 |
| 819 | 4-Aminobiphenyl | 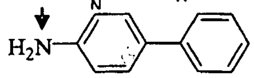 | 829 | Hexanedinitrile | 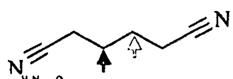 |
| 820 | Adenosyl cobinamide | 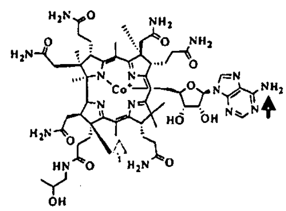 | 830 | Adenosine-GDP-cobinamide | 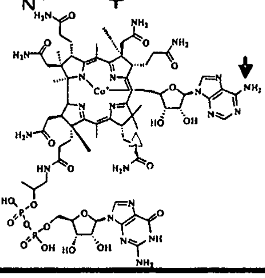 |

| N° | Substrate | Chemical Structure | N° | Substrate | Chemical Structure |
|---|---|---|---|---|---|
| 831 | Undecane | 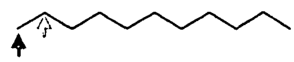 | 843 | S-2-Acetolactate | 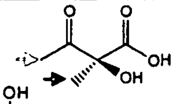 |
| 832 | 3,4-Dihydroxymandelaldehyde | 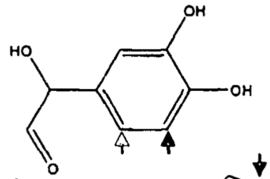 | 844 | 2-Ketoglutaric acid | 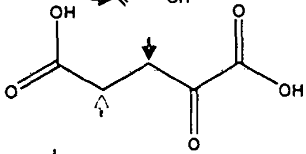 |
| 833 | 1-Acetyl-sn-glycero-3-phosphoethanolamine | 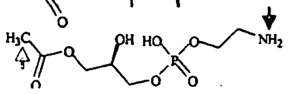 | 845 | N-Acetyl-beta-alanine | 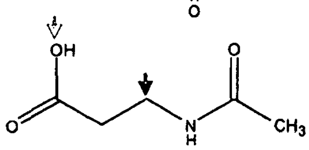 |
| 834 | S-Adenosyl-L-homocysteine | 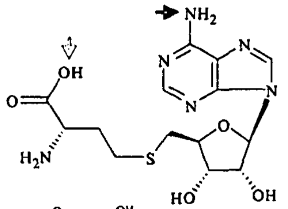 | 846 | L-Alanine | 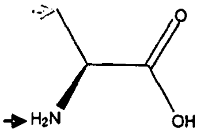 |
| 835 | 2-Amino-4-hydroxy-6-(erythro-1,2,3-trihydroxypropyl)dihydropteridine triphosphate | 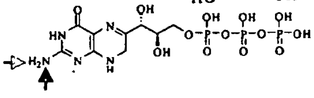 | 847 | D-Alanyl-D-lactate | 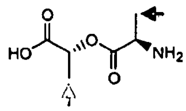 |
| 836 | 1-Acetyl-sn-glycero-3-phosphocholine | 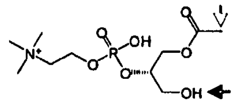 | 848 | N-Methyl-L-alanine | 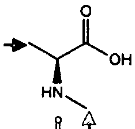 |
| 837 | 1-Acetyl-sn-glycerol 3-phosphate | 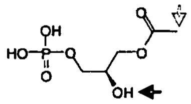 | 849 | 5-Aminolevulinate | 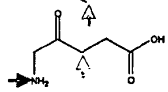 |
| 838 | Acetylhistone | Not available | 850 | Alditol | 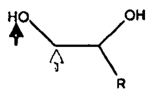 |
| 839 | D-Alanine | 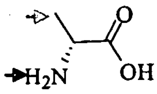 | 851 | D-Gluconic acid | 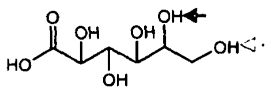 |
| 840 | 1-(5'-Phosphoribosyl)-5-aminoimidazole | 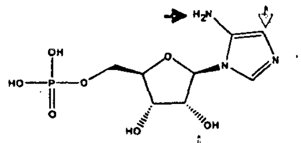 | 852 | D-Aldose | 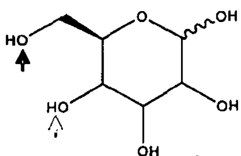 |
| 841 | 5-Amino-1-(5-phospho-D-ribosyl)imidazole-4-carboxamide | 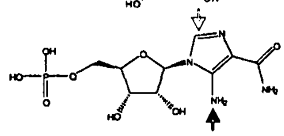 | 853 | ADP-glucose | 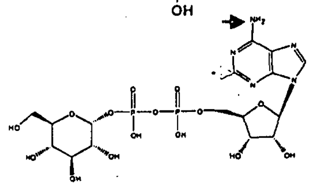 |
| 842 | D-Alanyl-D-alanine | 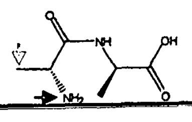 | 854 | Allyl alcohol | 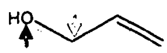 |

| N° | Substrate | Chemical Structure | N° | Substrate | Chemical Structure |
|---|---|---|---|---|---|
| 855 | Allose | 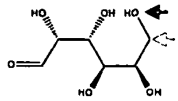 | 867 | Acetyl-maltose | 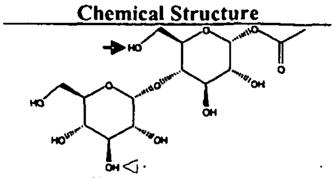 |
| 856 | Allophanate | 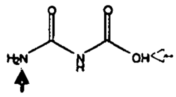 | 868 | S-Adenosyl-L-threonine | 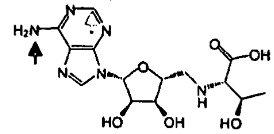 |
| 857 | S-Adenosyl-L-methionine | 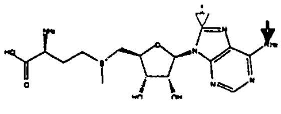 | 869 | S-Adenosylmethioninamine | 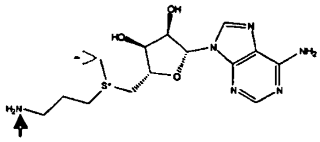 |
| 858 | 5-Amino-4-imidazole carboxylate | 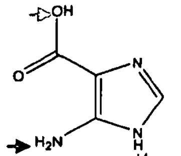 | 870 | Allantoate | 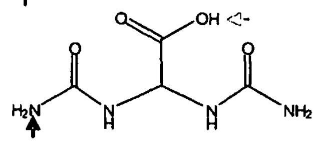 |
| 859 | (S)-Allantoin | 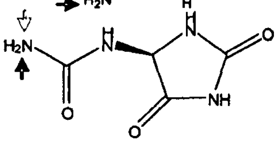 | 871 | Methyl-D-glucoside | 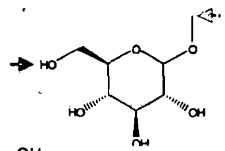 |
| 860 | L-2-Aminoadipate 6-semialdehyde | 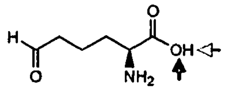 | 872 | 5-Aminovaleric acid | 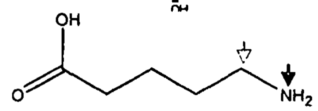 |
| 861 | Altrose | 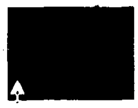 | 873 | Aminoacetone | 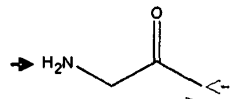 |
| 862 | (R)-Allantoin | 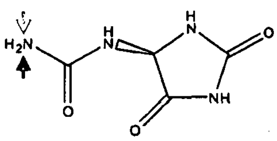 | 874 | Aminoimidazole ribotide | 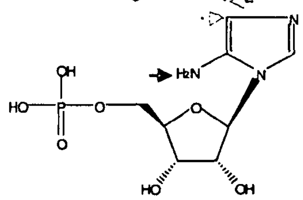 |
| 863 | Aminofructose 6-phosphate | 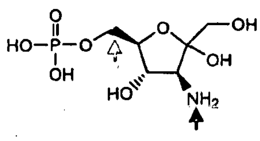 | 875 | 2-Aminophenol | 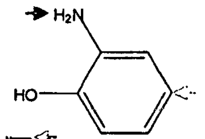 |
| 864 | 2-Aminomuconate 6-semialdehyde 2-aminomuconic semialdehyde (2*E*,4*Z*)-2-amino-6-oxohexa-2,4-dienoic acid | 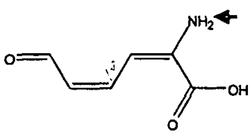 | 876 | S-Adenosyl-4-methylthio-2-oxobutanoate | 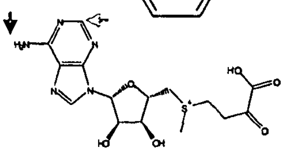 |
| 865 | L-2-Aminoadipate | 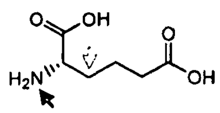 | 877 | Naphthyl-2-methylene-succinyl-CoA | 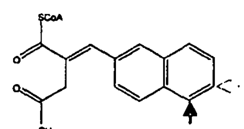 |
| 866 | D-Altronate | 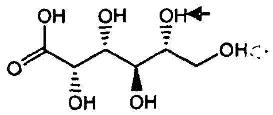 | 878 | Naphthyl-2-hydroxymethyl-succinyl CoA | 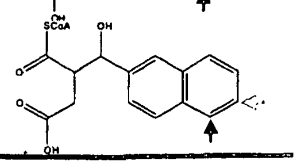 |

| N° | Substrate | Chemical Structure | N° | Substrate | Chemical Structure |
|---|---|---|---|---|---|
| 879 | 2-Aminomuconate | 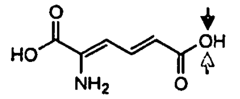 | 892 | Anserine | 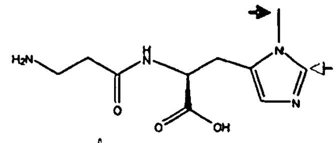 |
| 880 | Amylose | 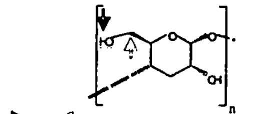 | 893 | Anthracene | 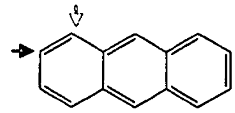 |
| 881 | Amylopectin | 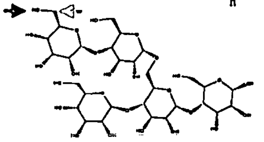 | 894 | D-Arabinono-1,4-lactone | 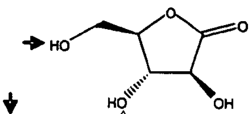 |
| 882 | 5-Carboxymethyl-2-oxohex-3-ene-1,6-dioate | 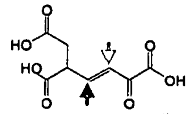 | 895 | P1,P5-Bis(5'-adenosyl) pentaphosphate | 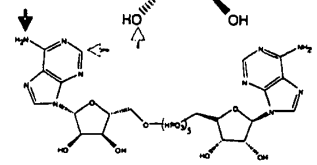 |
| 883 | AMP | 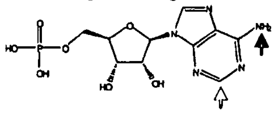 | 896 | 3-O-L-Alanyl-1-O-phosphatidylglycerol | 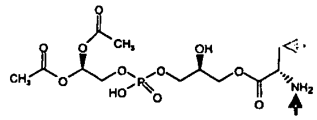 |
| 884 | 1,3-Cyclohexanedione | 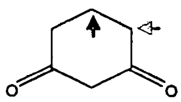 | 897 | N-Acetylputrescine | 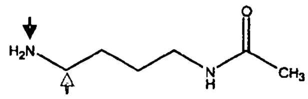 |
| 885 | 3-Hydroxycyclohexanone | 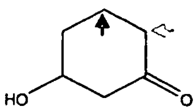 | 898 | 3-Aminopropanal | 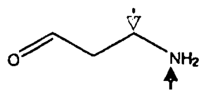 |
| 886 | Naphthyl-2-oxomethyl-succinyl-CoA | 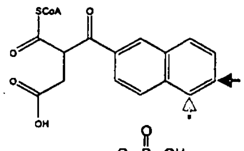 | 899 | α-Aminopropiononitrile | 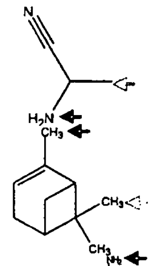 |
| 887 | Naphthalen-1-yl hydrogen phosphate | 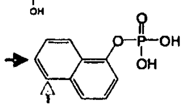 | 900 | alpha-Pinene | |
| 888 | Anisole | 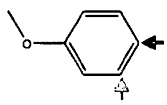 | 901 | 5'-Adenylyl sulfate | 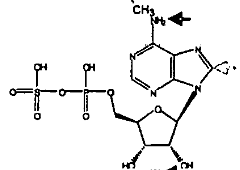 |
| 889 | Naphthalen-1-yl acetate | 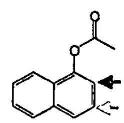 | 902 | 3'-Phosphoadenylyl sulfate | 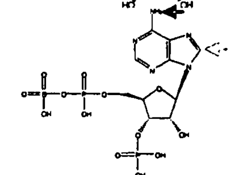 |
| 890 | Aniline | 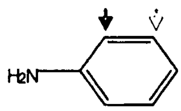 | 903 | L-Arabinono-1,5-lactone | 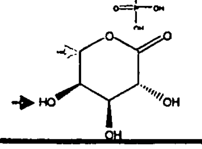 |
| 891 | Anthranilate | 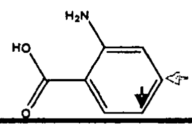 | | | |

| N° | Substrate | Chemical Structure | N° | Substrate | Chemical Structure |
|---|---|---|---|---|---|
| 904 | D-Arabinose 5-phosphate | 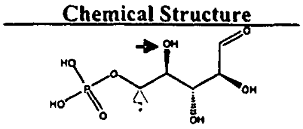 | 917 | L-Aspartate 4-semialdehyde | 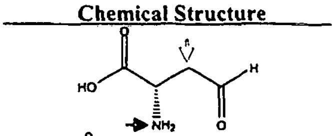 |
| 905 | D-Arabinose | 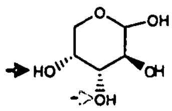 | 918 | Ascorbyl butyrate | 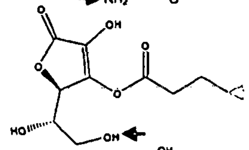 |
| 906 | L-Arabinose | 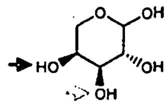 | 919 | Ascorbyl propionate | 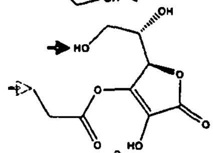 |
| 907 | P1,P4-Bis(5'-adenosyl) tetraphosphate | 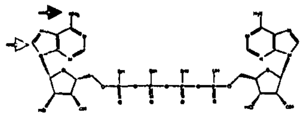 | 920 | Ascorbyl acetate | 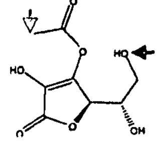 |
| 908 | L-Arginine | 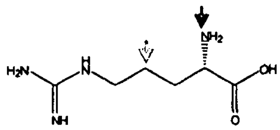 | 921 | Ascorbyl hexanoate | 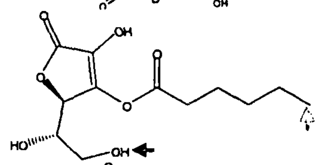 |
| 909 | Biphenyl-2,3-diol | 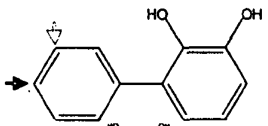 | 922 | L-Asparagine | 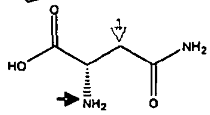 |
| 910 | Arbutin 6-phosphate | 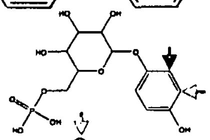 | 923 | Ascorbyl octanoate | 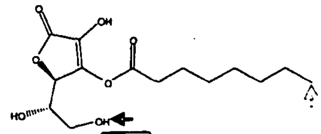 |
| 911 | Arene oxide | 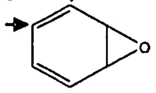 | 924 | Arylamine | 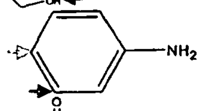 |
| 912 | Arachidic acid | 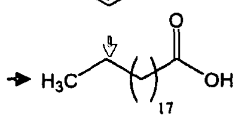 | 925 | L-Aspartate | 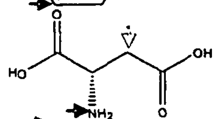 |
| 913 | Arginine methyl ester | 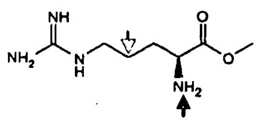 | 926 | Atropine | 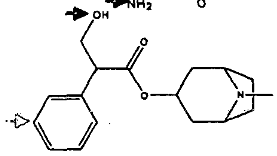 |
| 914 | Arg-Pro-Lys-Pro-Gln-Gln-Phe-Phe-Gly-Leu-Met-NH2 | Too big | 927 | 2-Hydroxyliminostilbene | 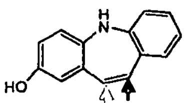 |
| 915 | Arginine p-nitroaniline | 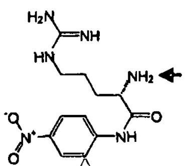 | 928 | Atrazine | 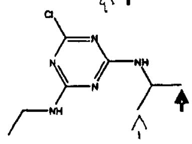 |
| 916 | L-Arginosuccinic acid | 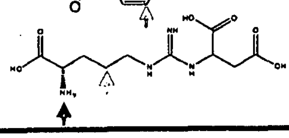 | 929 | 4-Hydroxymethylsalicylate | 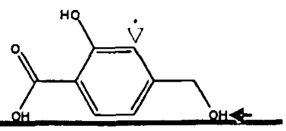 |

| N° | Substrate | Chemical Structure |
|---|---|---|
| 930 | ATP | 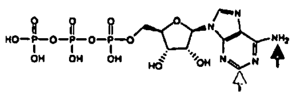 |
| 931 | Azetidine | 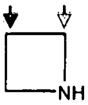 |
| 932 | Aziridine | 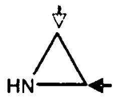 |
| 933 | 1-(5'-Phosphoribosyl)-5-amino-4-imidazolecarboxamide | 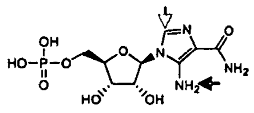 |
| 934 | γ-Butyrobetainyl-CoA | Too big |
| 935 | β-Cyclodextrin | 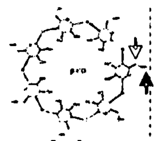 |
| 936 | Butanoyldolichol | 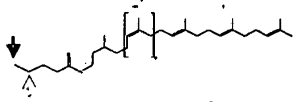 |
| 937 | Benzyl-2-methyl-3-oxobutanoate | 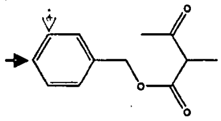 |
| 938 | Benzyl-(2R,3S)-2-methyl-3-hydroxybutanoate | 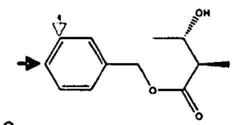 |
| 939 | 4-Phenylbutan-2-one | 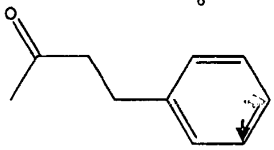 |
| 940 | Phenylmethyl benzoate | 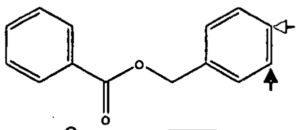 |
| 941 | Benzoyl-CoA | 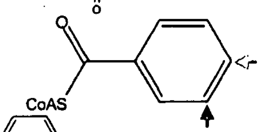 |
| 942 | Phenylmethyl hexanoate | 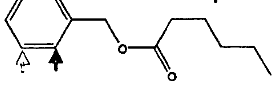 |

| N° | Substrate | Chemical Structure |
|---|---|---|
| 943 | Phenylmethyl acetate | 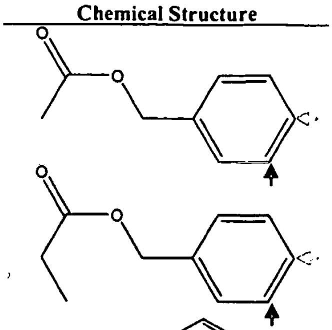 |
| 944 | Phenylmethyl propanoate | |
| 945 | (R,S)-Tetrahydrobenzylisoquinoline | 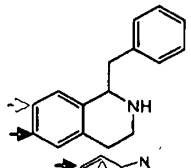 |
| 946 | Benzamidazole | 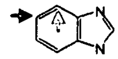 |
| 947 | Benzyl alcohol | 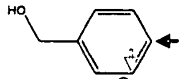 |
| 948 | 2,1-Benzoxazole | 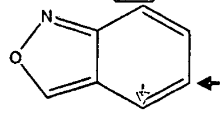 |
| 949 | Benzene | 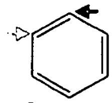 |
| 950 | Poly-beta-Hydroxybutyrate | 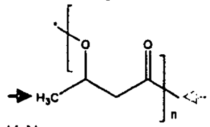 |
| 951 | Benzamide | 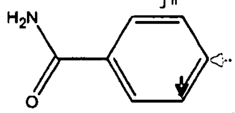 |
| 952 | 1,2-Benzophenanthrene | 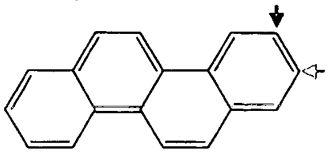 |
| 953 | 1-Benzothiophene | 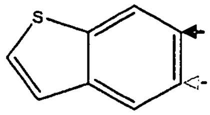 |
| 954 | 2-Benzothiophene | 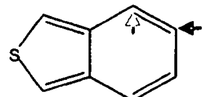 |
| 955 | Betaine aldehyde | 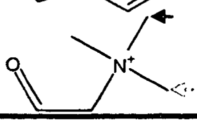 |

| N° | Substrate | Chemical Structure | N° | Substrate | Chemical Structure |
|---|---|---|---|---|---|
| 956 | Benzocyclobutene | 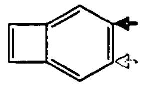 | 969 | Bromobenzene-2,3-oxide | 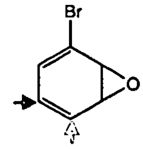 |
| 957 | Benzofuran | 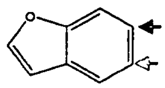 | 970 | Nicofuranose | 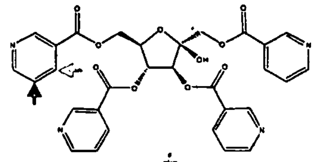 |
| 958 | 2,6-Dichlorobenzonitrile | 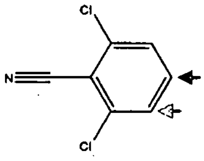 | 971 | Bromobenzene3,4-oxide | 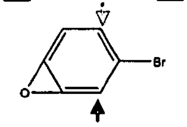 |
| 959 | Benzoate | 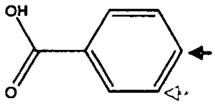 | 972 | 2-Bromomaleylacetate | 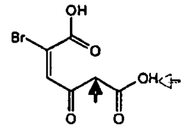 |
| 960 | 2-Methylthiobenzothiazole | 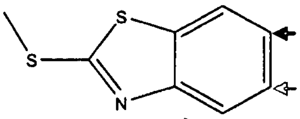 | 973 | m-Bromobenzotrifluoride | 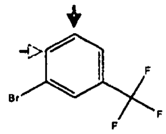 |
| 961 | 2H-Benzotriazole | 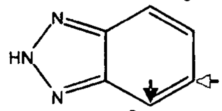 | 974 | Butanoyl-CoA | 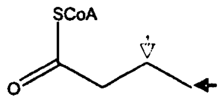 |
| 962 | Benzoxazole | 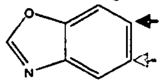 | 975 | 4-Aminophenol | 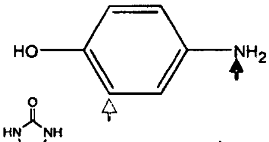 |
| 963 | Benzocycloheptene | 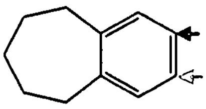 | 976 | epsilon-N-Biotinyl-L-lysine | 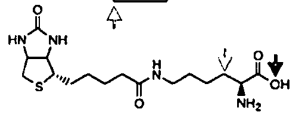 |
| 964 | Betaine | 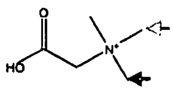 | 977 | Butylamine | 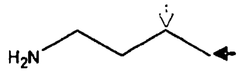 |
| 965 | Benzonitrile | 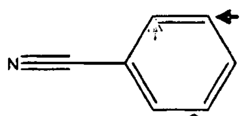 | 978 | Butyrate | 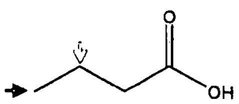 |
| 966 | N-Benzoylanthranilate | 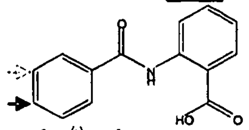 | 979 | Butyl butyrate | 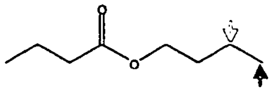 |
| 967 | CDP-1,2-Dihexadecanoylglycerol | 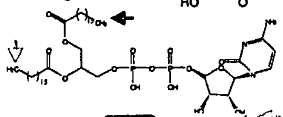 | 980 | Butyryl-CoA | 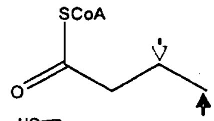 |
| 968 | Myrcene | 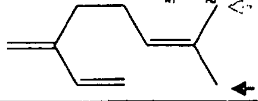 | 981 | Uridine | 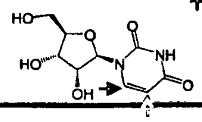 |

| N° | Substrate | Chemical Structure | N° | Substrate | Chemical Structure |
|---|---|---|---|---|---|
| 982 | 1-(5-Phospho-D-ribosyl)-5-amino-4-imidazolecarboxylate | 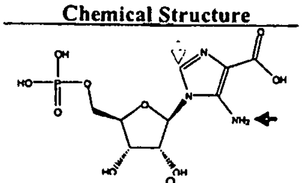 | 995 | epsilon-Caprolactam | 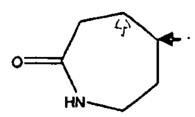 |
| 983 | Cyclohexa-1,5-diene-1-carbonyl-CoA | 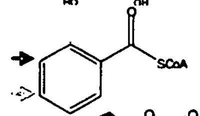 | 996 | Hexanoyl-CoA | 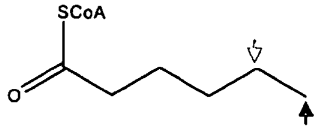 |
| 984 | Cellobiose-1,5-lactone | 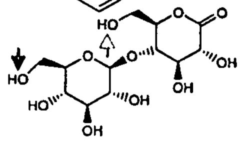 | 997 | 5-Carboxy-2-pentenoyl-CoA | 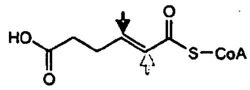 |
| 985 | 1-Hexadecene | 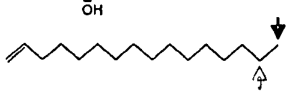 | 998 | Carbazole | 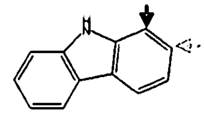 |
| 986 | Cadaverine | 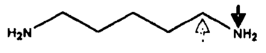 | 999 | 8-Hydroxy-2-methyl-α-carboline | 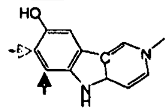 |
| 987 | Caffeate | 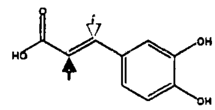 | 1000 | N-Butyl-beta-carboline-3-carboxylate | 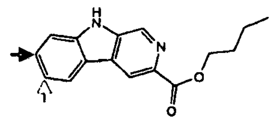 |
| 988 | Caffeoyl-CoA | 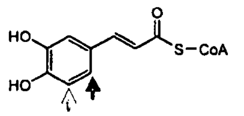 | 1001 | 8-Hydroxy-2-methyl-beta-carboline | 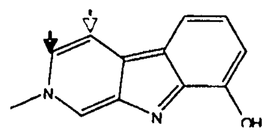 |
| 989 | Butyl paraben | 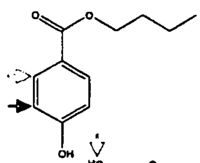 | 1002 | 8-Hydroxy-2-methyl-γ-carboline | 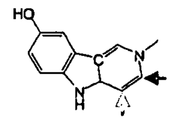 |
| 990 | N-Carbamyl-L-glutamate | 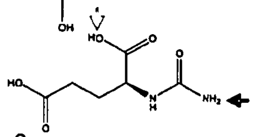 | 1003 | Carnosine | 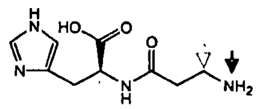 |
| 991 | Methyl caffeate | 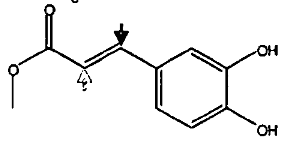 | 1004 | Carvone | 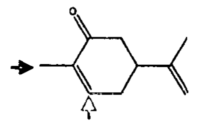 |
| 992 | Catechol | 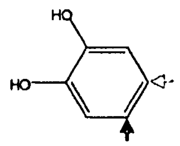 | 1005 | 2-(3-Carboxy-3-aminopropyl)-L-histidine | 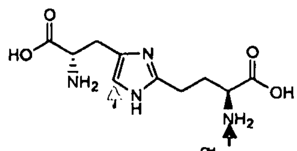 |
| 993 | (-)-trans-Carveol | 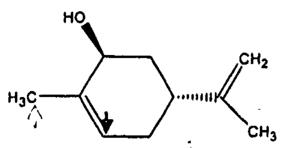 | 1006 | Catechin | 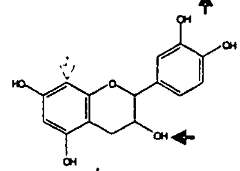 |
| 994 | 3',5'-Cyclic AMP | 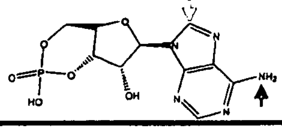 | 1007 | N-Carbamoyl-L-aspartate | 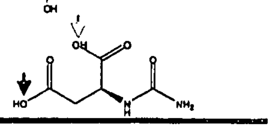 |

| N° | Substrate | Chemical Structure | N° | Substrate | Chemical Structure |
|---|---|---|---|---|---|
| 1008 | 3-Isopropylmalate | 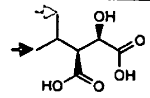 | 1020 | CDP-butyrylglycerol | 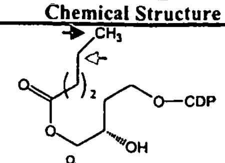 |
| 1009 | Cobinamide | 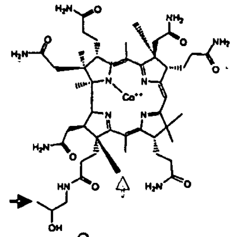 | 1021 | Benzyl-L-cysteinamide | 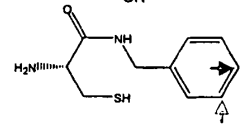 |
| 1010 | Carbamoyl phosphate | 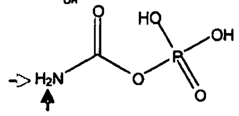 | 1022 | CDP-Hexadecanoylglycerol | 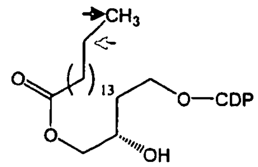 |
| 1011 | m-Aminophenol | 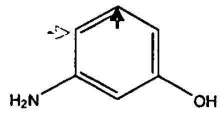 | 1023 | CDP-1,2-Dibutyrylglycerol | 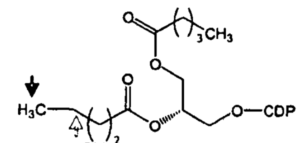 |
| 1012 | 4-Hydroxyphenylglyoxylate | 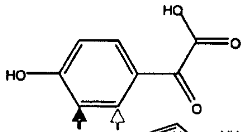 | 1024 | CDP-choline | 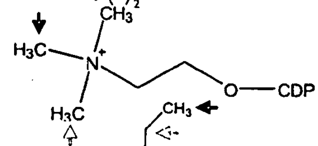 |
| 1013 | 3',5'-Cyclic CMP | 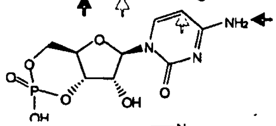 | 1025 | CDP-Dodecanoylglycerol | 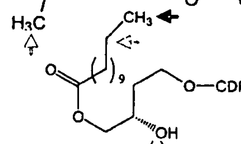 |
| 1014 | 3',5'-Cyclic dAMP | 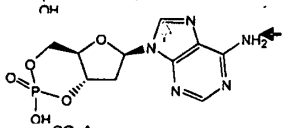 | 1026 | CDP-1,2-Didodecanoylglycerol | 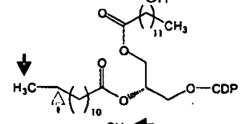 |
| 1015 | trans-Hex-2-enoyl-CoA | 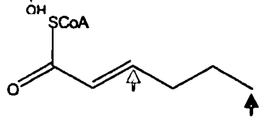 | 1027 | CDP-Tetradecanoylglycerol | 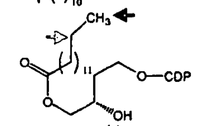 |
| 1016 | cis-1,2-Dihydrobenzene-1,2-diol | 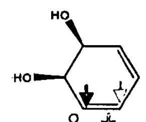 | 1028 | CDP-1,2-Ditetradecanoylglycerol | 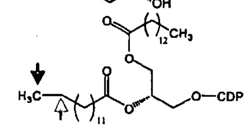 |
| 1017 | Capryldihydroxyacetone posphate | 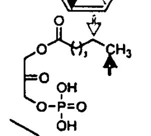 | 1029 | CDP-1,2-Dihexanoylglycerol | 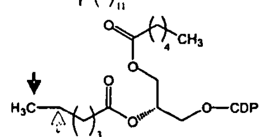 |
| 1018 | cis-1,2-dihydro-ethylcatechol | 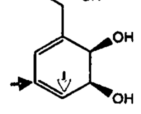 | 1030 | CDP-1,2-Dioleylglycerol | 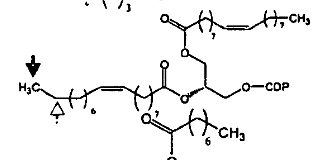 |
| 1019 | CDP-1,2-Dinonadecanoylglycerol | 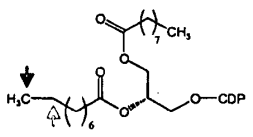 | 1031 | CDP-1,2-Dioctanoylglycerol | 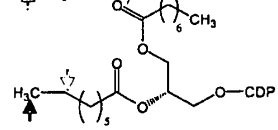 |

| N° | Substrate | Chemical Structure | N° | Substrate | Chemical Structure |
|---|---|---|---|---|---|
| 1032 | CDP-Octanoylglycerol | 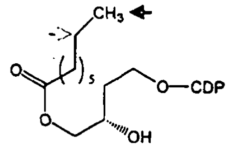 | 1046 | Cellobiose | 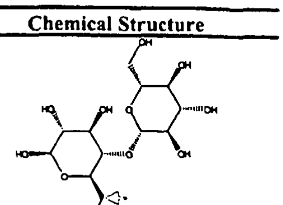 |
| 1033 | Cytidine diphosphate (CDP) | 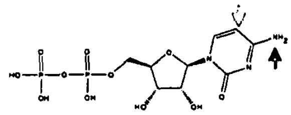 | 1047 | Cellotriose | 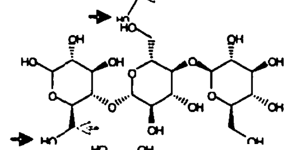 |
| 1034 | CDP-1,2-Dinonanoylglycerol | 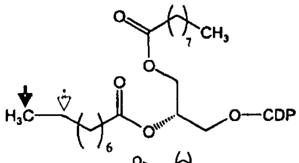 | 1048 | Cellotetraose | 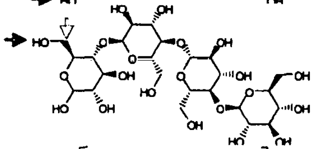 |
| 1035 | CDP-1,2-Dioctadecanoylglycerol | 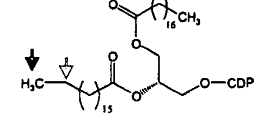 | 1049 | Cellulose | 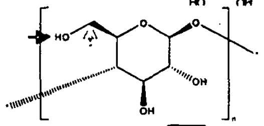 |
| 1036 | CDP-1,2-Dipropionylglycerol | 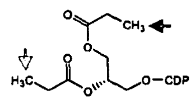 | 1050 | cis-3-(3-Carboxyethenyl)-3,5-cyclohexadiene-1,2-diol | 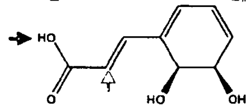 |
| 1037 | CDP-Decanoylglycerol | 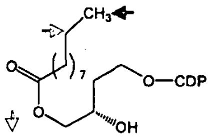 | 1051 | CDP-1,2-Didecanoylglycerol | 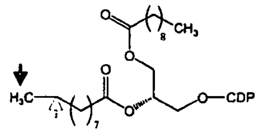 |
| 1038 | CDP-ethanolamine | 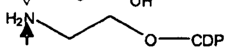 | 1052 | L-Cysteinylglycine | 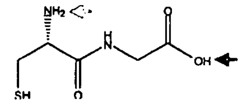 |
| 1039 | 3-Hydroxyaminophenol | 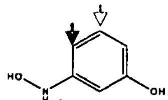 | 1053 | Cetyl alcohol | 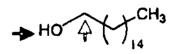 |
| 1040 | CDP-glycerol | 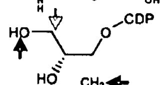 | 1054 | 4-Chlorocatechol | 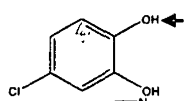 |
| 1041 | CDP-hexanoylglycerol | 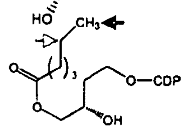 | 1055 | 3',5'-Cyclic GMP | 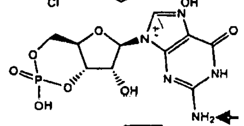 |
| 1042 | CDP-Nonanoylglycerol | 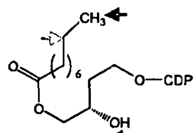 | 1056 | Cyclohex-2-enone | 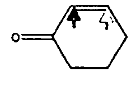 |
| 1043 | CDP-Nonadecanoylglycerol | 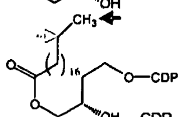 | 1057 | 6-Carboxyhexanoyl-CoA | 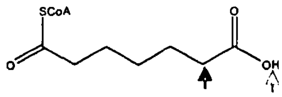 |
| 1044 | CDP-propionylglycerol | 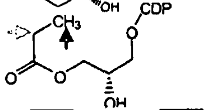 | 1058 | Cyclohexane-1,2-dione | 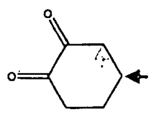 |
| 1045 | 4-Hydroxybiphenyl | 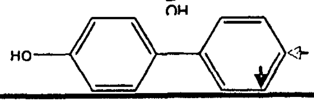 | 1059 | Cyclohexanone | 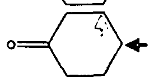 |

| N° | Substrate | Chemical Structure | N° | Substrate | Chemical Structure |
|---|---|---|---|---|---|
| 1060 | Chitin | 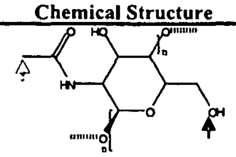 | 1073 | CMP | 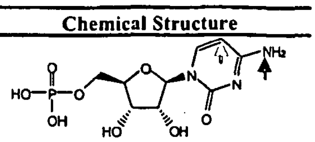 |
| 1061 | Chitosan | 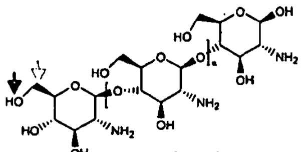 | 1074 | Cinnamoyl-CoA | 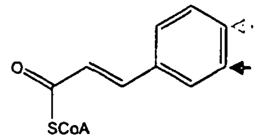 |
| 1062 | Chitobiose | 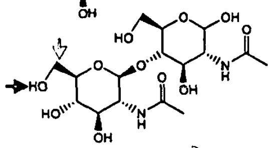 | 1075 | L-Citrulline | 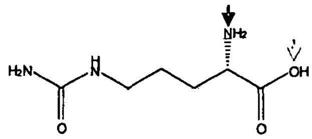 |
| 1063 | Choline | 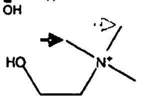 | 1076 | Cinnamaldehyde | 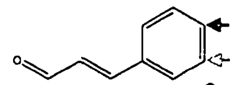 |
| 1064 | Phenyl acetate | 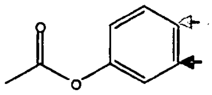 | 1077 | Cinnamyl acetate | 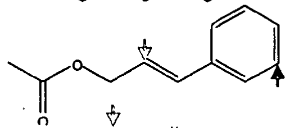 |
| 1065 | Ethyl acetate | 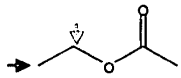 | 1078 | Cinnoline | 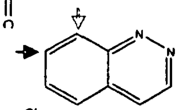 |
| 1066 | Tolylacetate | 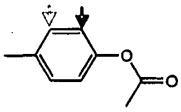 | 1079 | 4-Chloro-m-cresol | 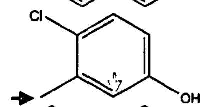 |
| 1067 | Ethyl butyryl acetate | 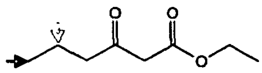 | 1080 | 1-Bromopentane | 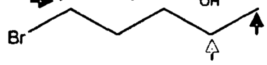 |
| 1068 | N-Acetyl-D-glucosamine 1,6-bisphosphate | 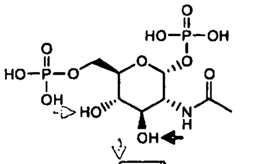 | 1081 | 1-Chloropentane | 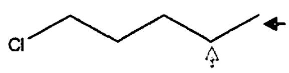 |
| 1069 | 4-Chlorophenol | 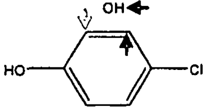 | 1082 | 2-Hydroxy-2,4-pentadienoate | 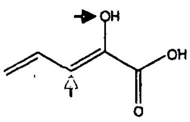 |
| 1070 | Chorismate | 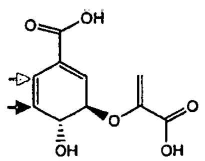 | 1083 | Styrene oxide | 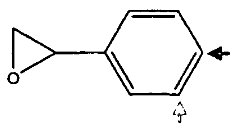 |
| 1071 | 5,7-Dihydroxychromone | 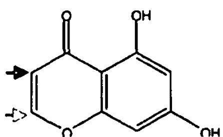 | 1084 | 2,3-Dibromopentane | 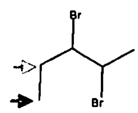 |
| 1072 | Citrate | 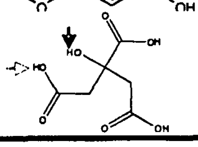 | 1085 | 1,2,3-Trimethylbenzene | 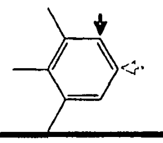 |

| N° | Substrate | Chemical Structure | N° | Substrate | Chemical Structure |
|---|---|---|---|---|---|
| 1086 | trans-Aconitate | 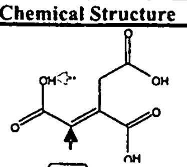 | 1099 | 2-Amino-3-carboxymuconate semialdehyde | 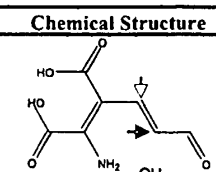 |
| 1087 | (R)-Citronellal | 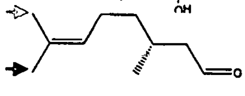 | 1100 | gamma-Carboxymuconolactone | 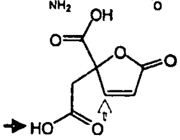 |
| 1088 | (-)-Citronellol | 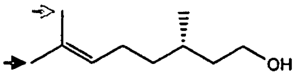 | 1101 | Coenzyme A | 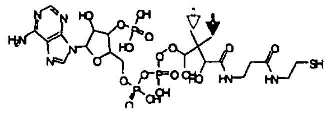 |
| 1089 | Cinnamyl alcohol | 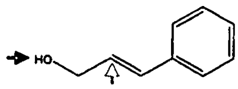 | 1102 | Coniferyl alcohol | 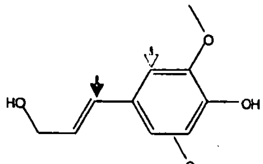 |
| 1090 | CMP-3-deoxy-D-manno-octulosonate | 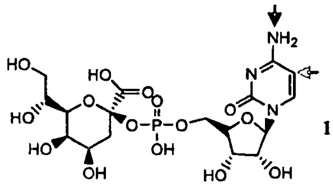 | 1103 | Coniferaldehyde | 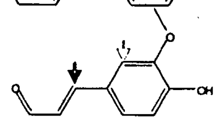 |
| 1091 | Chlorobenzene | 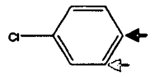 | 1104 | 3-Chloro-cis,cis-muconate | 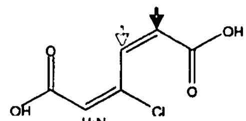 |
| 1092 | 2-Chlorobenzoate | 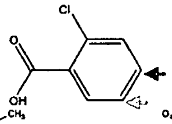 | 1105 | Coronamic acid | 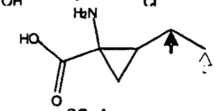 |
| 1093 | Cardiolipin | 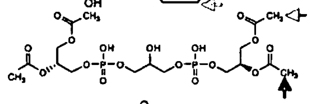 | 1106 | Crotonoyl-CoA | 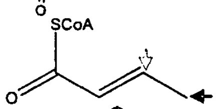 |
| 1094 | 3-Carboxy-cis,cis-muconate | 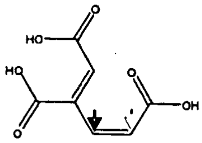 | 1107 | 3-Ethyltoluene | 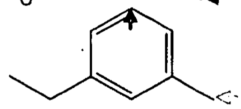 |
| 1095 | 4-Chlorobutan-1-ol | 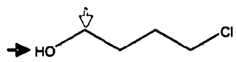 | 1108 | 4-Coumarate | 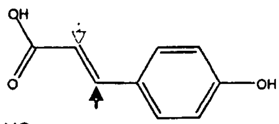 |
| 1096 | Chloroxylenol | 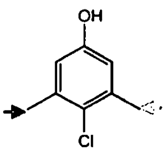 | 1109 | p-Coumaroyl-CoA | 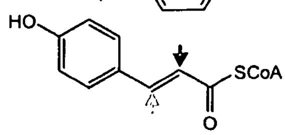 |
| 1097 | Styrene | 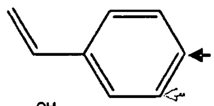 | 1110 | Coumaran | 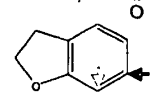 |
| 1098 | Chlorogenate | 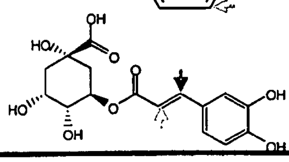 | 1111 | Coumarin | 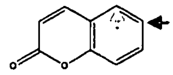 |

| N° | Substrate | Chemical Structure | N° | Substrate | Chemical Structure |
|---|---|---|---|---|---|
| 1112 | Methyl p-coumarate | 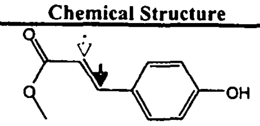 | 1125 | p-Cumic aldehyde | 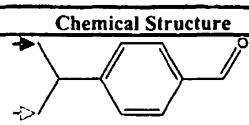 |
| 1113 | CDP-Octadecanoylglycerol | 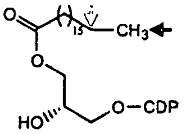 | 1126 | Cumene | 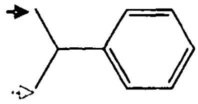 |
| 1114 | 2-Coumarinate | 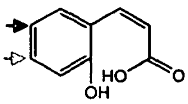 | 1127 | p-Cumic alcohol | 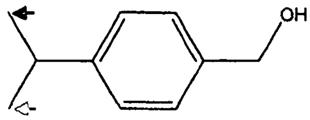 |
| 1115 | Creatine | 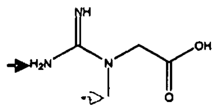 | 1128 | N6-(1,2-Dicarboxyethyl)-AMP | 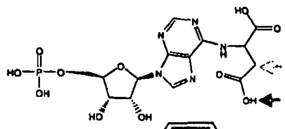 |
| 1116 | Ethylglycocyamine | 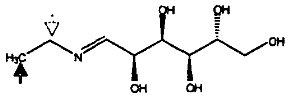 | 1129 | Cyanobenzene | 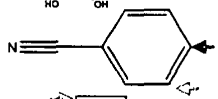 |
| 1117 | 3-Chlorocatechol | 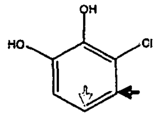 | 1130 | Cyclobutane | 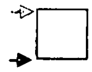 |
| 1118 | CTP | 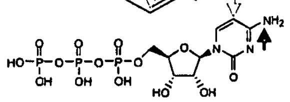 | 1131 | Cycloheptane | 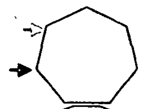 |
| 1119 | 2-Chloro-cis,cis-muconate | 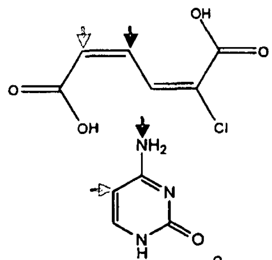 | 1132 | Cycloheptadecane | 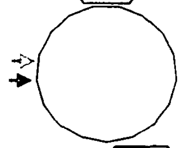 |
| 1120 | Cytosine | | 1133 | Cyclohexanol | 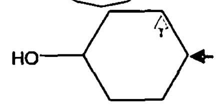 |
| 1121 | Coronafacic acid | 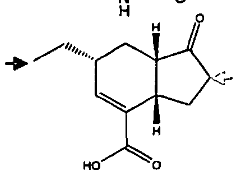 | 1134 | Cyclohexylamine | 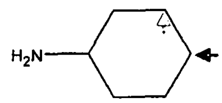 |
| 1122 | Crotono-betaine | 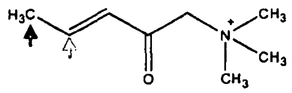 | 1135 | Cyclohexane | 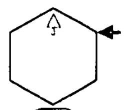 |
| 1123 | 3,5-Dichlorocatechol | 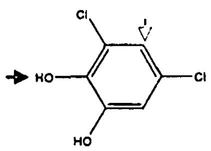 | 1136 | Cyclohexadccane | 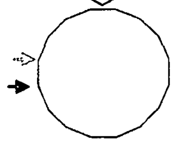 |
| 1124 | Coronatine | 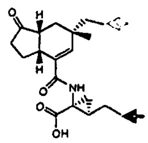 | 1137 | Cyclohexene oxide | 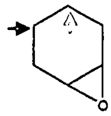 |

| N° | Substrate | Chemical Structure | N° | Substrate | Chemical Structure |
|---|---|---|---|---|---|
| 1138 | Cyclononane | 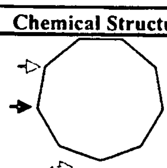 | 1151 | Acetylcysteine | 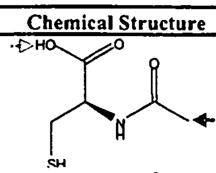 |
| 1139 | Cyclopentane | 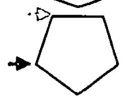 | 1152 | Cysteine p-nitroaniline | 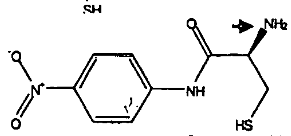 |
| 1140 | Urea | 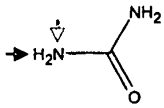 | 1153 | Cystinyl p-nitroalinine | 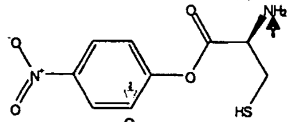 |
| 1141 | Cyclopropane | 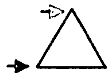 | 1154 | Methyl 2-phenylacetate | 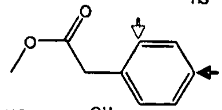 |
| 1142 | dADP | 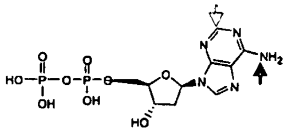 | 1155 | Cytidine | 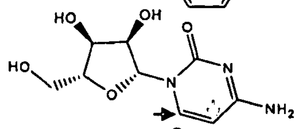 |
| 1143 | D-Cycloserine | 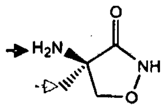 | 1156 | 2,4-Dihydrofuran | 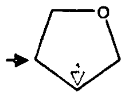 |
| 1144 | Cycloundecane | 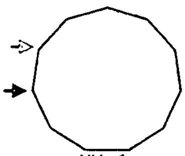 | 1157 | Diethyl-2-methyl-3-oxosuccinate | 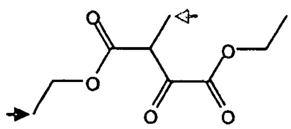 |
| 1145 | L-Cysteine | 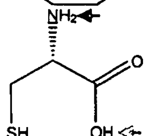 | 1158 | D-4-Hydroxyphenyl-glycine methyl ester | 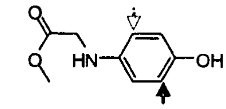 |
| 1146 | trans-Cyclohexane-1,2-diol | 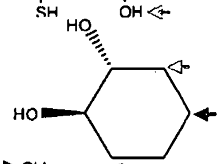 | 1159 | L-Cystathionine | 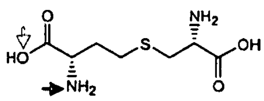 |
| 1147 | Decanoic acid | 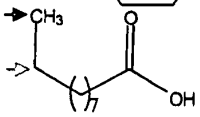 | 1160 | Deoxyadenosine | 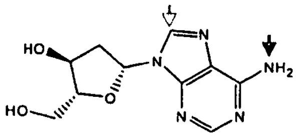 |
| 1148 | Cyclopentanone | 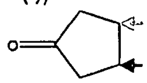 | 1161 | D-Galactono-1,5-lactone | 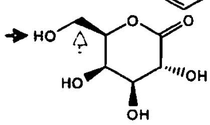 |
| 1149 | Caproyldihydroxyaceton eposphate | 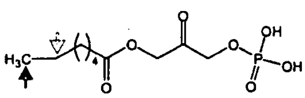 | 1162 | 6,7-Dimethyl-8-(1-D-ribityl)lumazine | 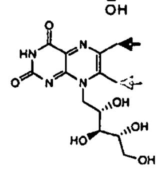 |
| 1150 | L-Cystine | 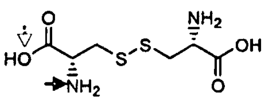 | 1163 | Deoxy-5-methylcytidylate | 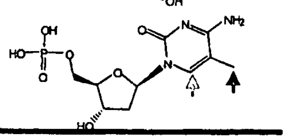 |

| N° | Substrate | Chemical Structure | N° | Substrate | Chemical Structure |
|---|---|---|---|---|---|
| 1164 | Cyclopenta[b]pyridine | 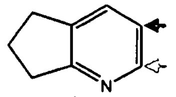 | 1178 | Decanoyl-CoA | 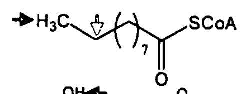 |
| 1165 | D-Aldonolactone | 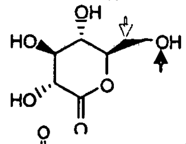 | 1179 | D-Galacturonate 1-phosphate | 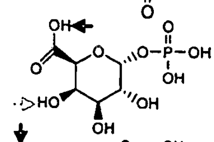 |
| 1166 | Octadecanoylglycerone phosphate | 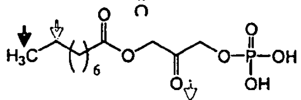 | 1180 | N6-(L-1,3-Dicarboxypropyl)-L-lysine | 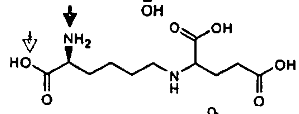 |
| 1167 | D-Arabinitol | 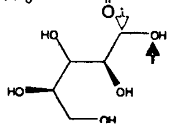 | 1181 | 2'-Deoxy-5-hydroxymethylcytidine-5'-triphosphate | 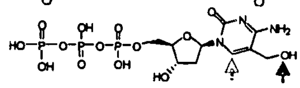 |
| 1168 | 1,3-Diaminopropane | 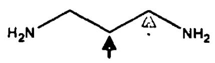 | 1182 | dCMP | 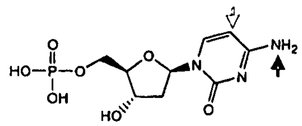 |
| 1169 | 7,8-Diaminononanoate | 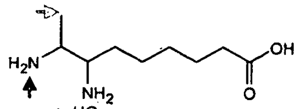 | 1183 | dCDP | 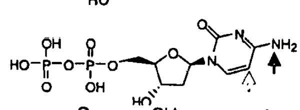 |
| 1170 | 3,4-Dihydroxy-2-butanone 4-phosphate | 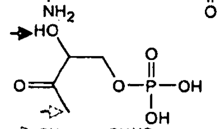 | 1184 | 3-Hydroxy-4-trimethylammoniobutanoate | 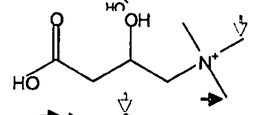 |
| 1171 | D-Arabonate | 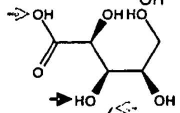 | 1185 | Dihydrothymine | 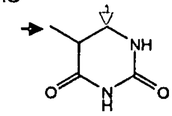 |
| 1172 | 2-Ethyltoluene | 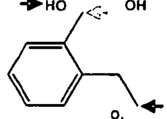 | 1186 | dCTP | 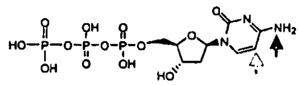 |
| 1173 | 2'-Deoxy-5-hydroxymethylcytidine-5'-diphosphate | 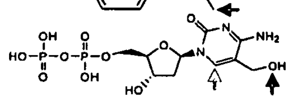 | 1187 | O-Butanoylcarnitine | 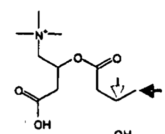 |
| 1174 | D-Arabitol | 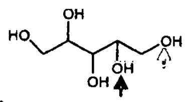 | 1188 | 3',4',5,7-Tetrahydroxy-3-methoxyflavone | 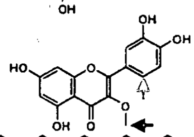 |
| 1175 | Methyl decanoate | 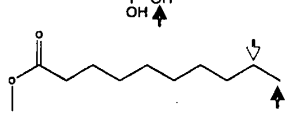 | 1189 | trans-Dodec-2-Enoyl-CoA | 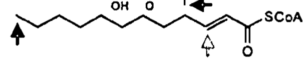 |
| 1176 | trans-Dec-2-Enoyl-CoA | 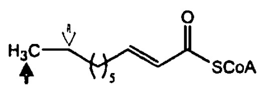 | 1190 | Methyl dodecanoate | 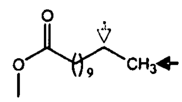 |
| 1177 | beta-Alanyl-L-lysine | 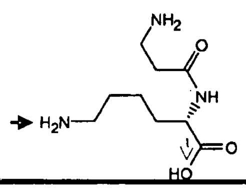 | 1191 | Dodecanoate | 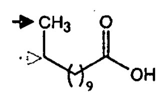 |

| N° | Substrate | Chemical Structure | N° | Substrate | Chemical Structure |
|---|---|---|---|---|---|
| 1192 | Dodecanoyl-CoA | 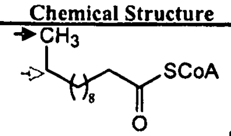 | 1206 | dGDP | 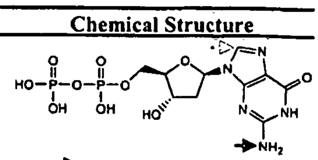 |
| 1193 | 1-Tetradecanoyl-sn-glycerol 3-phosphate | 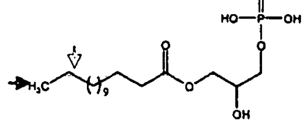 | 1207 | D-Glucarate | 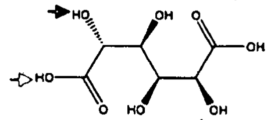 |
| 1194 | Dodecanoyldolichol | 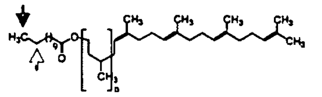 | 1208 | dGMP | 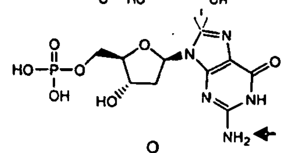 |
| 1195 | Decanoyldihydroxyacetonephosphate | 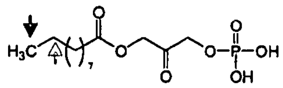 | 1209 | 4-Bromophenol-2,3-epoxide | 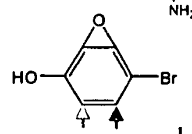 |
| 1196 | Decanoyldolichol | 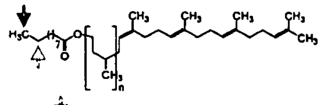 | 1210 | dGTP | 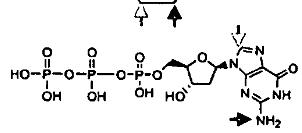 |
| 1197 | Decane | 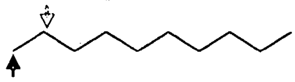 | 1211 | 1-Hydroxy-2-naphthoate | 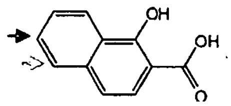 |
| 1198 | Decanal | 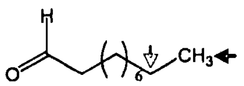 | 1212 | Dihydroxyacetone | 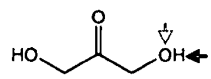 |
| 1199 | Octadecanal | 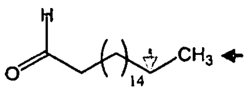 | 1213 | Dihydroxyacetone phosphate | 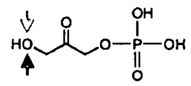 |
| 1200 | 2'-Deoxy-5-hydroxymethylcytidine 5'-phosphate | 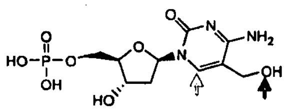 | 1214 | (1S,3R,4S)-3,4-Dihydroxycyclohexane 1-carboxylate | 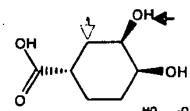 |
| 1201 | 2-Deoxy-D-ribose | 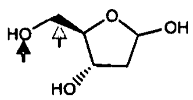 | 1215 | 7,8-Dihydrofolate | 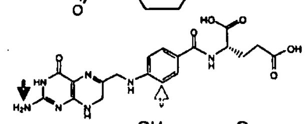 |
| 1202 | Dihydrouracil | 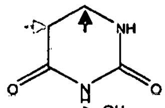 | 1216 | trans-2,3-Dihydroxycinnamate | 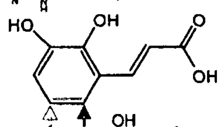 |
| 1203 | (S)-2-Hydroxyglutarate | 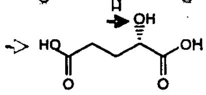 | 1217 | 1,4-Dihydroxy-2-naphthoate | 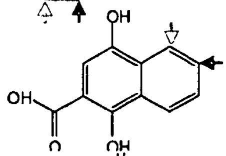 |
| 1204 | D-Fucose | 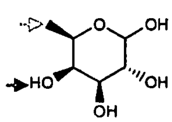 | 1218 | 2-Amino-4-hydroxy-6-(D-erythro-1,2,3-trihydroxypropyl)-7,8-dihydropteridine | 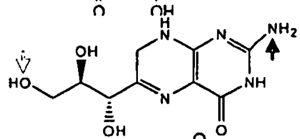 |
| 1205 | D-Glucono-1,5-lactone | 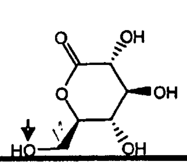 | 1219 | S-Dihydroorotate | 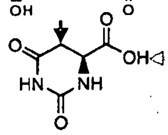 |

| N° | Substrate | Chemical Structure | N° | Substrate | Chemical Structure |
|---|---|---|---|---|---|
| 1220 | Dihydroneopterin phosphate | 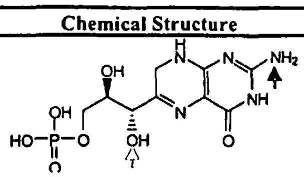 | 1232 | dTMP | 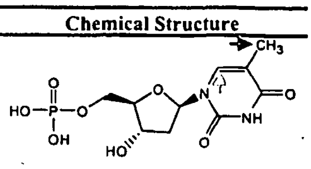 |
| 1221 | 3-(2,3-Dihydroxyphenyl)propanoate | 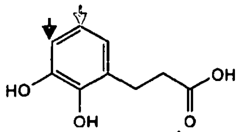 | 1233 | 4-Chlorophenylacetate | 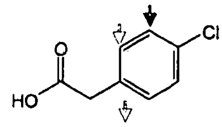 |
| 1222 | Dihydropteroate | 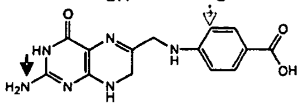 | 1234 | D-Iditol | 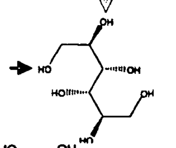 |
| 1223 | (S)-4,5-dihydroxypentan-2,3-dione | 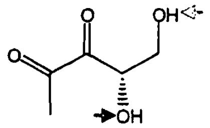 | 1235 | Digallate | 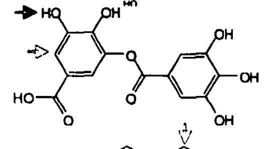 |
| 1224 | 3-Dehydrosphinganine | 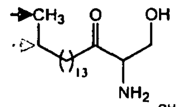 | 1236 | Dihydrocoumarin | 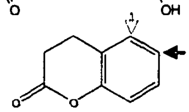 |
| 1225 | 3,5-Dihydroxy-3,4,7-trimethoxyflavone | 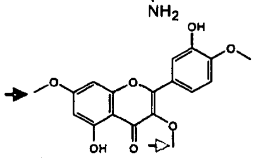 | 1237 | 9-Hydroxynonanoate | 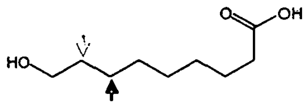 |
| 1226 | (5R)-3,4-Dihydroxy-5-(hydroxymethyl)furan-2(5H)-one | 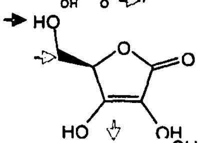 | 1238 | 10-Hydroxydecanoate | 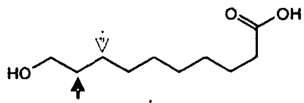 |
| 1227 | 2,4-Dibromophenol | 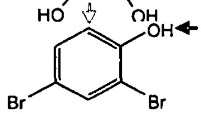 | 1239 | 2,4-Dinitrotoluene | 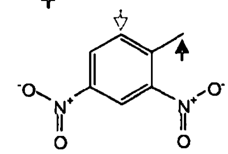 |
| 1228 | 2,6-Dibromophenol | 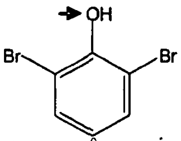 | 1240 | Deoxyinosine | 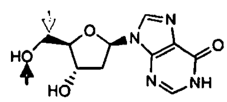 |
| 1229 | Deoxyguanosine | 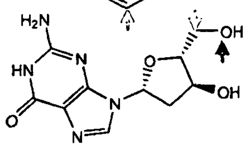 | 1241 | Dioxybenzone | 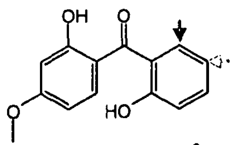 |
| 1230 | Dibenzofuran | 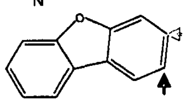 | 1242 | 2,3-Bis(3-Hydroxytetradecanoyl)-D-glucosaminyl-1,6-beta-D-2,3-bis(3-hydroxytetradecanoyl)-beta-D-glucosaminyl 1-phosphate | 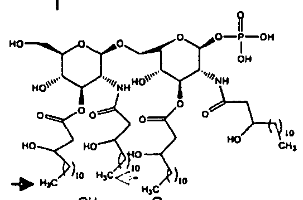 |
| 1231 | Dibenzo-p-dioxin | 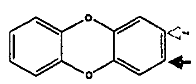 | 1243 | 2,3-Diketo-5-methylthio-1-phosphopentane | 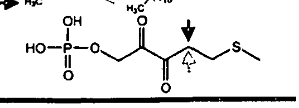 |

| N° | Substrate | Chemical Structure | N° | Substrate | Chemical Structure |
|---|---|---|---|---|---|
| 1244 | 4-Carboxymethylenebut-2-en-4-olide | 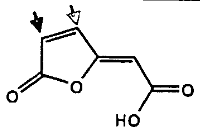 | 1258 | 6,7-Dimethyl-8-(1-D-ribityl)lumazine | 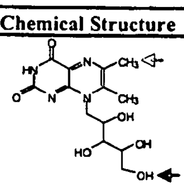 |
| 1245 | cis-2-Methyl-5-isopropylhexa-2,5-dienoic acid | 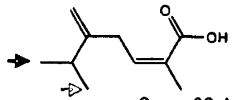 | 1259 | Dimethylallyl diphosphate | 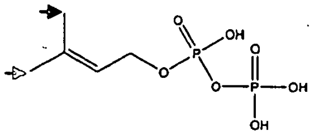 |
| 1246 | cis-2-Methyl-5-isopropylhexa-2,5-dienoyl-CoA | 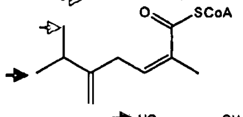 | 1260 | DNA substrate lambda DNA + HindIII | Not available |
| 1247 | D-Mannuronate | 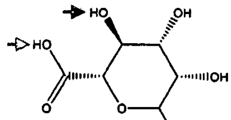 | 1261 | DNA substrate | 5'-TAAGCTCCGGATTGTCCGGG AGGTAAAGCCCTGAT-3' |
| 1248 | D-Lyxose | 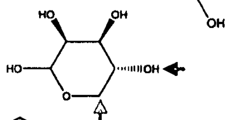 | 1262 | DNA substrate | 5'-CACAGGAAGCTCTACAGGT ACTCCG-3' |
| 1249 | Dihydrolipoamide | 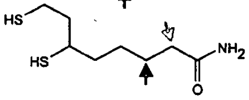 | 1263 | DNA substrate | 5'-TGGTCATCAGGGCTTTACCT CCCGGACAATCCGGAGCTTA CGGAGTACCTGTAGAGCTTC CTGTGCAAGC-3' |
| 1250 | D-Mannonate | 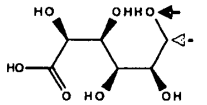 | 1264 | DNA substrate SspI X174 DNA | Not available |
| 1251 | Dimethoxybenzidine | 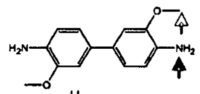 | 1265 | DNA Helicase substrate dsDNA: | 5'-GCACTGGCCGTCGTTTTACC-3' |
| 1252 | 5,6-Dimethylbenzimidazole | 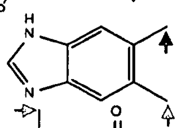 | 1266 | DNA Gyrase substrate relaxed pBR322 | Not available |
| 1253 | Dimethylglycine | 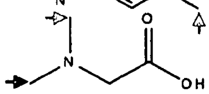 | 1267 | DNA Topoisomerase substrate | 40-base single-stranded oligodeoxyribonucleotides |
| 1254 | Nalpha,Nalpha-Dimethyl-L-histidine | 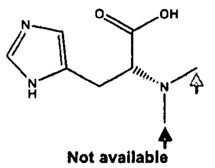 | 1268 | Deamino-NAD$^+$ | 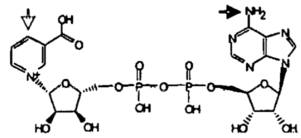 |
| 1255 | DNA substrate lambda DNA + Sau3A | Not available | 1269 | n-Dodecyl-D-maltoside | 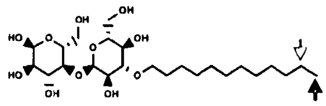 |
| 1256 | DNA resolvase substrate | 5-GACGCTGCCGAATTCTGGC TTGCTAGGACATCTTTGCC CACGTTGACCC-3 | 1270 | Docosan-1-ol | 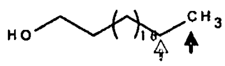 |
| 1257 | 4alpha-Methylzymosterol | 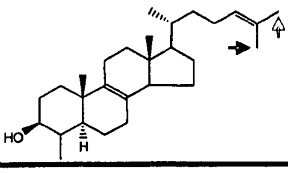 | 1271 | Dodecane | 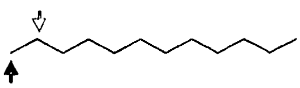 |

| N° | Substrate | Chemical Structure | N° | Substrate | Chemical Structure |
|---|---|---|---|---|---|
| 1272 | Dodecanoyldihydoxyacetonephosphate | 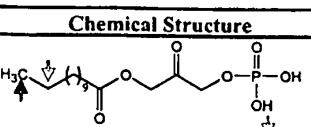 | 1286 | D-Ribulose | 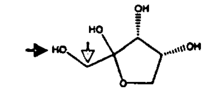 |
| 1273 | Dolichol | 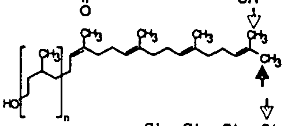 | 1287 | Diethyl (2S,3R)-2-methyl-3-hydroxysuccinate | 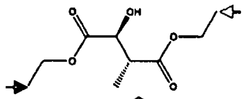 |
| 1274 | Dolichyl D-mannosyl phosphate | 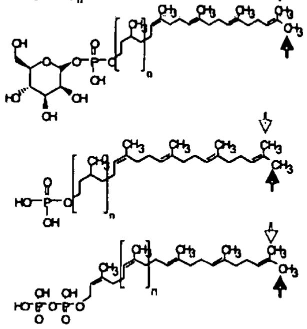 | 1288 | Phenanthrene-4,5-dicarboxylate | 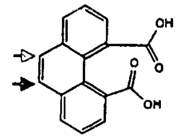 |
| 1275 | Dolichyl phosphate | | 1289 | Deoxyribose | 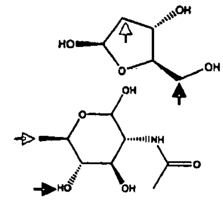 |
| 1276 | Dehydrodolichol diphosphate | | 1290 | 2-Acetamido-2,6-dideoxy-D-glucose | |
| 1277 | O-Decanoyl-L-carnitine | 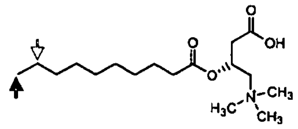 | 1291 | dTDP-4-Amino-4,6-dideoxy-D-glucose | 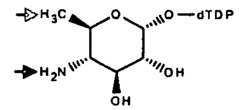 |
| 1278 | O-Propanoylcarnitine | 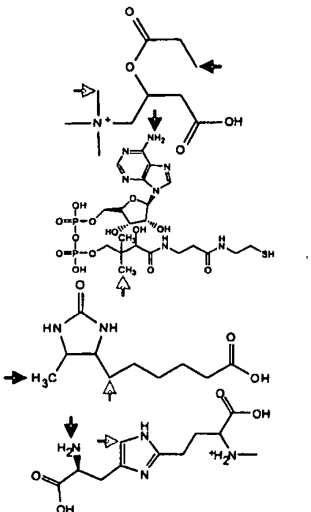 | 1292 | dTDP-4-Dehydro-6-deoxy-D-glucose | 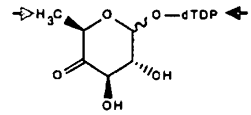 |
| 1279 | Dephospho-CoA | | 1293 | dTDP-4-Dehydro-6-deoxy-L-mannose | 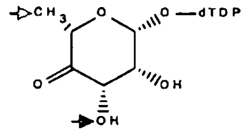 |
| 1280 | Dethiobiotin | | 1294 | dTDP-4-oxo-6-deoxy alpha-D-glucose | 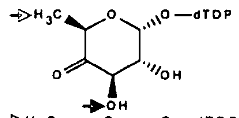 |
| 1281 | 2-[3-Carboxy-3-(methylammonio)propyl]-L-histidine | | 1295 | dTDP-6-deoxy-L-mannose | 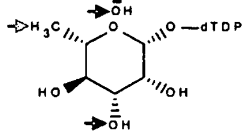 |
| 1282 | Diethyl (2R,3R)-2-methyl-3-hydroxysuccinate | 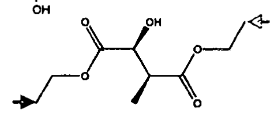 | 1296 | dTDP-6-deoxy-L-talose | 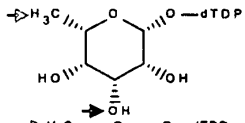 |
| 1283 | dTDP | 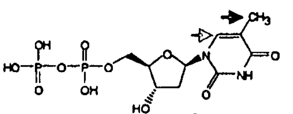 | 1297 | dTDP-alpha-D-desosamine | 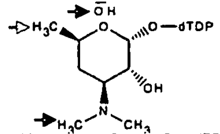 |
| 1284 | D-Ribitol 5-phosphate | 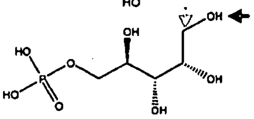 | 1298 | dTDP-glucose | 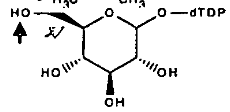 |
| 1285 | D-Ribonate | 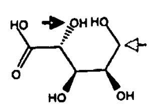 | 1299 | dTDP-D-galactose | 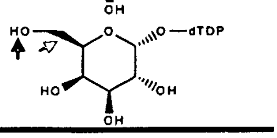 |

| N° | Substrate | Chemical Structure | N° | Substrate | Chemical Structure |
|---|---|---|---|---|---|
| 1300 | dTDP-D-fucose | 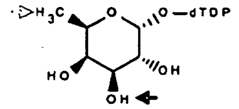 | 1313 | D-Xylonate | 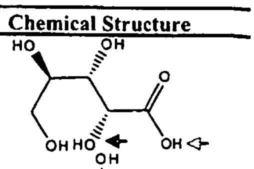 |
| 1301 | dTDP-L-rhamnose | 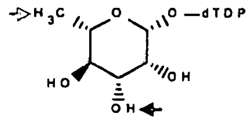 | 1314 | 4,5-Dihydroxypyrene | 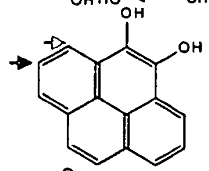 |
| 1302 | Dibenzothiophene | 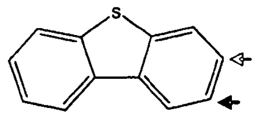 | 1315 | Ethyl 3-oxohexanoate | 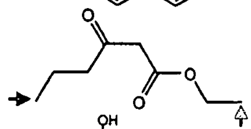 |
| 1303 | 4,5-Dihydroxy-4,5-dihydropyrene | 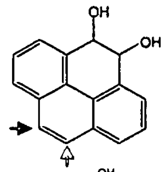 | 1316 | 3,4-Dihydroxyphenanthrene | 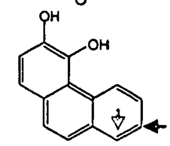 |
| 1304 | D-Xylulose | 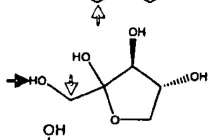 | 1317 | 3,6-Dichloro-cis-1,2-dihydroxycyclohexa 3,5-diene | 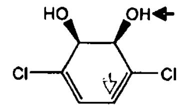 |
| 1305 | dTDP-D-galacturonate | 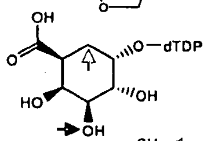 | 1318 | L-Erythro-4-Hydroxyglutamate | 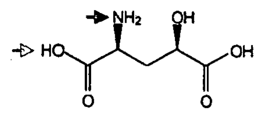 |
| 1306 | 1-Deoxy-D-xylulose | 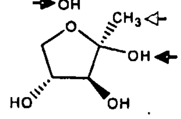 | 1319 | 1,2-Epoxypropane | 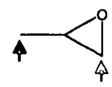 |
| 1307 | Thiamin triphosphate | 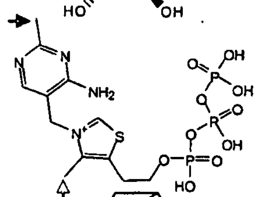 | 1320 | Docosapentaenoic acid methyl ester | 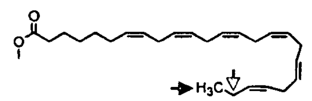 |
| 1308 | Tropinone | 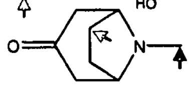 | 1321 | Docosapentaenoic acid | 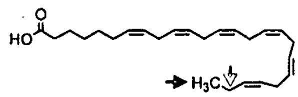 |
| 1309 | 1,2-Dioctanoyl-sn-glycerol | 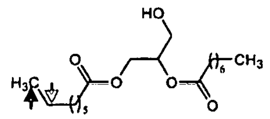 | 1322 | D-erythro-1-(Imidazol-4-yl)glycerol 3-phosphate | 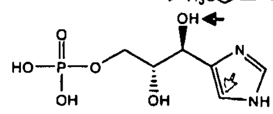 |
| 1310 | Quinazoline | 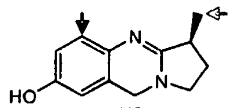 | 1323 | 2-Indanone oxime | 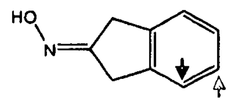 |
| 1311 | 1,2-Dihexadecanoyl-sn-glycerol | 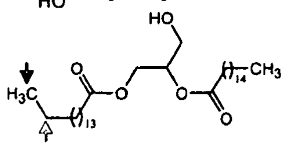 | 1324 | (+)-Epicatechin | 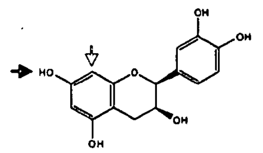 |
| 1312 | 1-Deoxy-D-xylulose 5-phosphate | 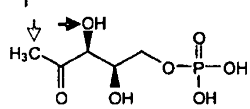 | 1325 | 2,3-Dihydroxy-4'-chlorobiphenyl | 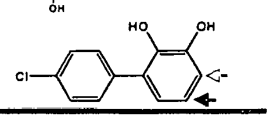 |

| N° | Substrate | Chemical Structure | N° | Substrate | Chemical Structure |
|---|---|---|---|---|---|
| 1326 | Epimelibiose | 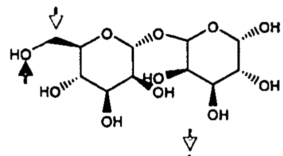 | 1339 | N-Isopropylammelide | 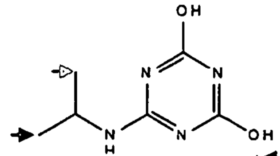 |
| 1327 | Epichlorohydrin | 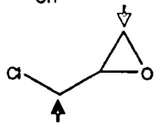 | 1340 | Ethyl benzoate | 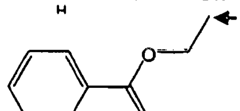 |
| 1328 | 1,2-Didecanoyl-sn-glycerol | 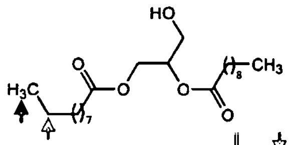 | 1341 | Ethyl butyrate | 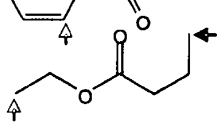 |
| 1329 | Episterol | 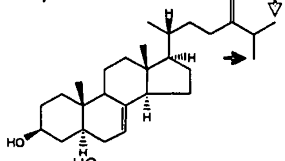 | 1342 | Ethyl cinnamate | 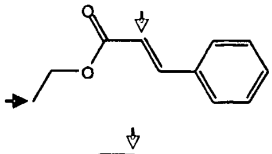 |
| 1330 | Ethyl (R)-3-Hydroxyhexanoate | 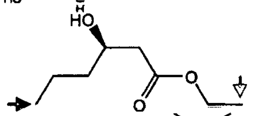 | 1343 | Ethylguaiacol | 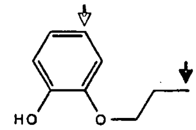 |
| 1331 | trans-2-Methyl-5-isopropylhexa-2,5-dienoic acid | 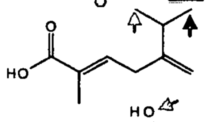 | 1344 | Ethanolamine | 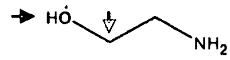 |
| 1332 | trans-2-Chlorodienelactone | 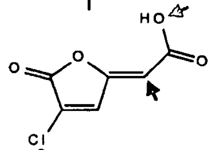 | 1345 | Ethylhexyl palmitate | 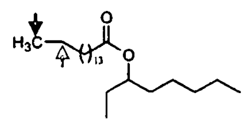 |
| 1333 | 1-Formyl-2-indanone | 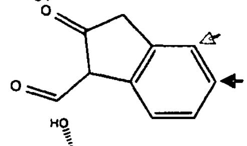 | 1346 | Ethyl heptanoate | 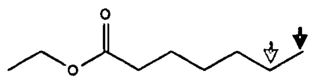 |
| 1334 | Erythrose | 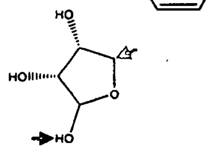 | 1347 | Ethylphenyl sulfide | 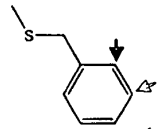 |
| 1335 | (R)-2-Hydroxystearate | 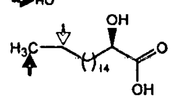 | 1348 | Ethylbenzene | 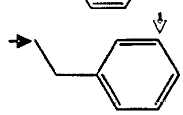 |
| 1336 | D-Erythrulose | 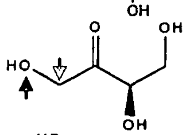 | 1349 | Ethyl lactate | 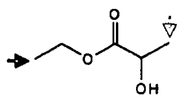 |
| 1337 | Erythritol | 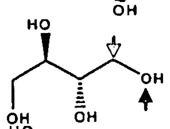 | 1350 | Ethynylbenzene | 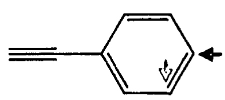 |
| 1338 | Ethyl (S)-3-Hydroxyhexanoate | 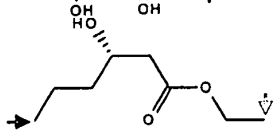 | 1351 | o-Hydroxybenzoic acid | 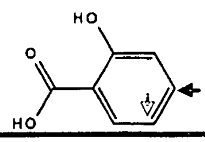 |

| N° | Substrate | Chemical Structure | N° | Substrate | Chemical Structure |
|---|---|---|---|---|---|
| 1352 | Ethyl paraben | 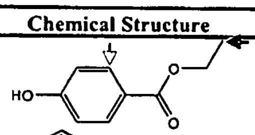 | 1365 | 1-Hexadecanol | 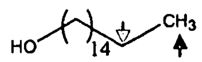 |
| 1353 | Ethyl salicylate | 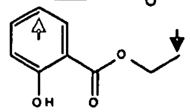 | 1366 | Feruloyl-CoA | 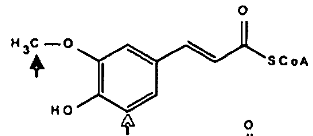 |
| 1354 | D-Fructose 1-phosphate | 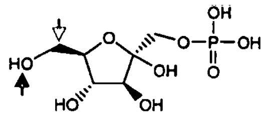 | 1367 | Ferulate | 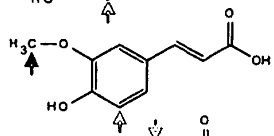 |
| 1355 | Formaldehyde | 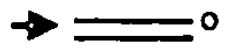 | 1368 | Methyl ferulate | 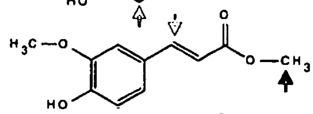 |
| 1356 | D-Fructose 2,6-bisphosphate | 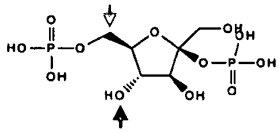 | 1369 | 5-Hydroxyferulate | 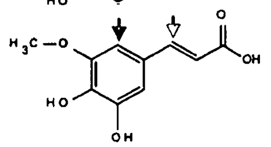 |
| 1357 | FAD | 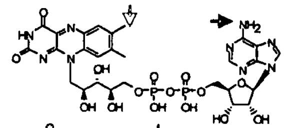 | 1370 | Diphenylenemethane | 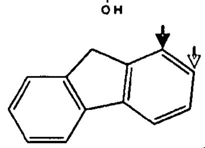 |
| 1358 | FADH2 | 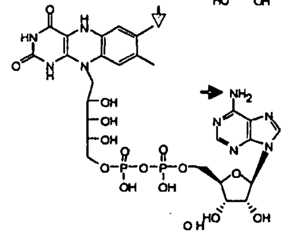 | 1371 | Flavone | 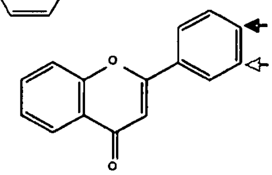 |
| 1359 | 1-Hexanoyl-sn-glycerol 3-phosphate | 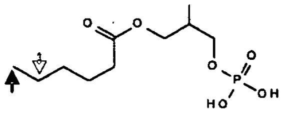 | 1372 | Fluorobenzene | 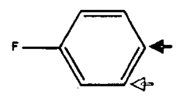 |
| 1360 | N-Formylanthranilate | 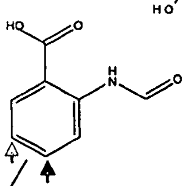 | 1373 | Formiminoglycine | 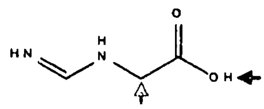 |
| 1361 | Farnesol | 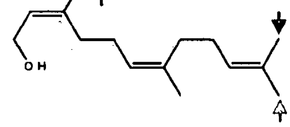 | 1374 | N2-Formyl-N1-(5-phospho-D-ribosyl)glycinamide | 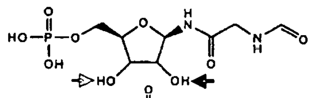 |
| 1362 | FAXX | Not available | 1375 | FMN | 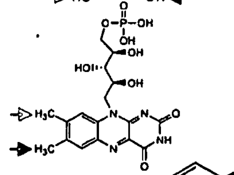 |
| 1363 | L-Fuculose 1-phosphate | 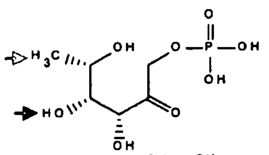 | 1376 | Flavonol | 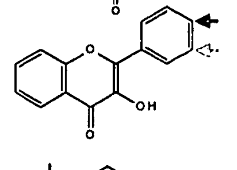 |
| 1364 | L-Fuculose | 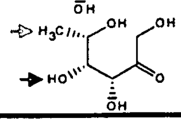 | 1377 | Formate | 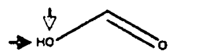 |

| N° | Substrate | Chemical Structure | N° | Substrate | Chemical Structure |
|---|---|---|---|---|---|
| 1378 | 2-Hydroxy-6-oxo-6-(4'-chlorophenyl)-hexa-2,4-dienoate | 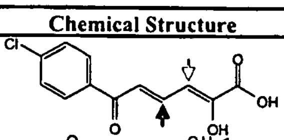 | 1391 | D-Fuconate | 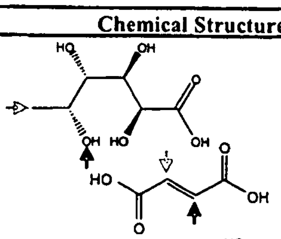 |
| 1379 | 2-Dehydro-3-deoxy-D-fuconate | 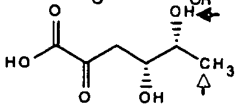 | 1392 | Fumarate | |
| 1380 | N-Formimidoyl-L-glutamate | 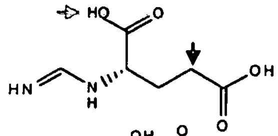 | 1393 | L-Fucose | 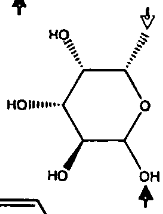 |
| 1381 | (R)-2,3-Dihydroxy-3-methylpentanoate | 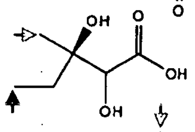 | 1394 | 4-Formylsalicylic acid | 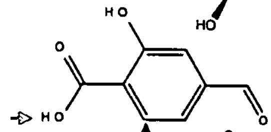 |
| 1382 | 5-Formamido-1-(5-phospho-D-ribosyl)imidazole-4-carboxamide | 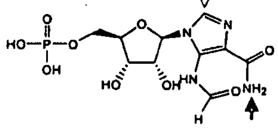 | 1395 | D-Glucose 1-phosphate | 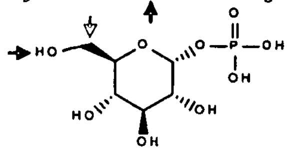 |
| 1383 | 4'-O-beta-D-Glucosyl-cis-p-coumarate | 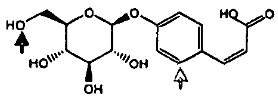 | 1396 | Glutathionyl-OH-1,2-dihydronaphthalene | 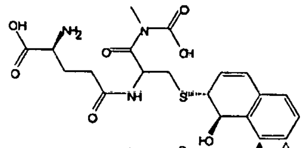 |
| 1384 | Farnesyl diphosphate | 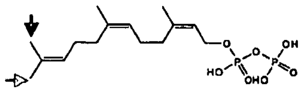 | 1397 | Glutathionyl-1,2-dihydronaphthalene | 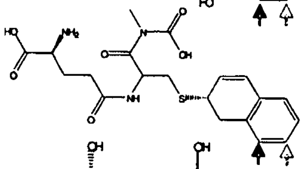 |
| 1385 | D-Fructose | 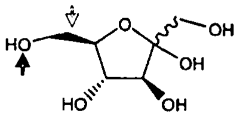 | 1398 | D-Glucono-1,5-lactate | 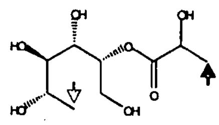 |
| 1386 | Fructosamine | 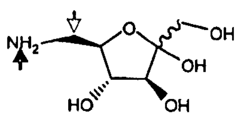 | 1399 | Furazan-3,4-diol | 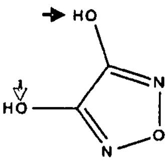 |
| 1387 | D-Fructuronate | 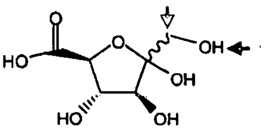 | 1400 | Glycerol-3-phosphate | 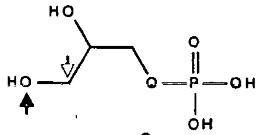 |
| 1388 | Farnesal | 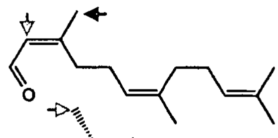 | 1401 | Glyceraldehyde 3-phosphate | 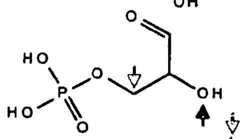 |
| 1389 | L-Fucose 1-phosphate | 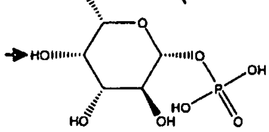 | 1402 | sn-Glycero-3-phosphocholine | 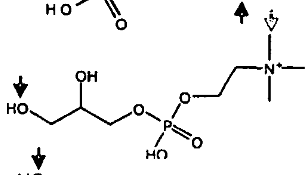 |
| 1390 | Fumarylacetoacetate | 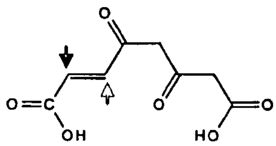 | 1403 | Glycerophosphoglycerol | 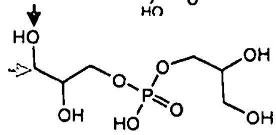 |

| N° | Substrate | Chemical Structure | N° | Substrate | Chemical Structure |
|---|---|---|---|---|---|
| 1404 | sn-Glycero-3-phosphoethanolamine | 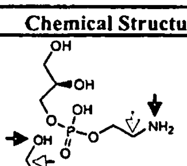 | 1417 | D-Galactonate | 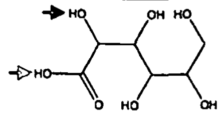 |
| 1405 | sn-Glycero-3-phospho-1-inositol | 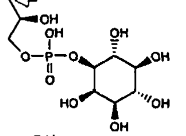 | 1418 | 3-O-Methylgallate | 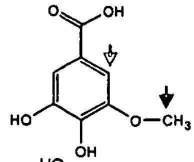 |
| 1406 | beta-D-Glucose 6-phosphate | 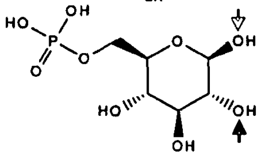 | 1419 | Gallate | 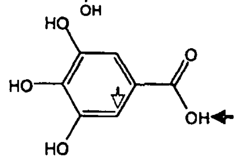 |
| 1407 | Hexadecanoate | 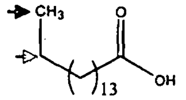 | 1420 | n-Propyl gallate | 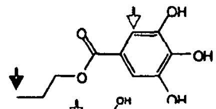 |
| 1408 | 4-Aminobutyraldehyde | 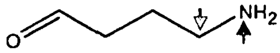 | 1421 | Galactitol | 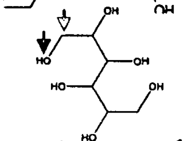 |
| 1409 | gamma-Amino-gamma-cyanobutanoate | 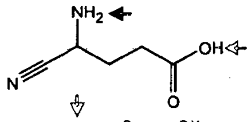 | 1422 | Galactitol 1-phosphate | 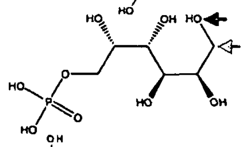 |
| 1410 | D-Galactose | 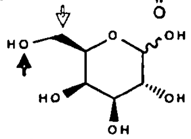 | 1423 | D-Glucosamine 6-phosphate | 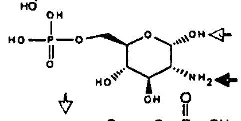 |
| 1411 | alpha-D-Galactose 1-phosphate | 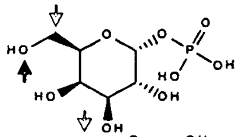 | 1424 | D-Glucosamine 1-phosphate | 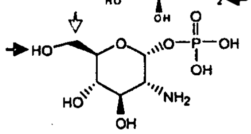 |
| 1412 | D-Galactosamine | 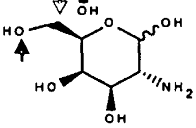 | 1425 | D-Galacturonate | 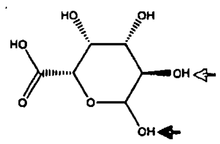 |
| 1413 | Galactomannan | 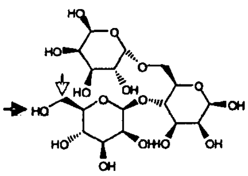 | 1426 | D-Glucosamine | 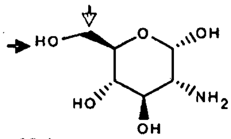 |
| 1414 | D-Galactosaminoglycan | Not available | 1427 | Octanoyl-CoA | 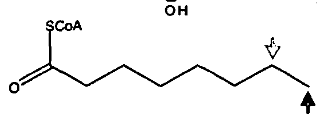 |
| 1415 | Galactinol | 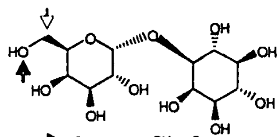 | 1428 | gamma-Butyrolactone | 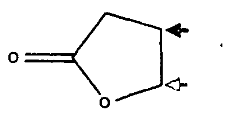 |
| 1416 | D-Galactarate | 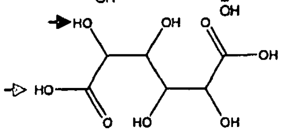 | 1429 | 4-Guanidinobutanoate | 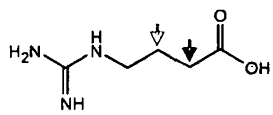 |

| N° | Substrate | Chemical Structure | N° | Substrate | Chemical Structure |
|---|---|---|---|---|---|
| 1430 | Butyro-betaine | 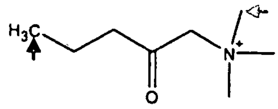 | 1443 | Gentiobiose | 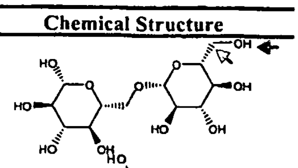 |
| 1431 | 4-Guanidinobutanamide | 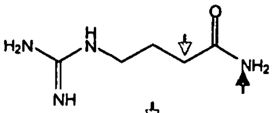 | 1444 | Gentisate | 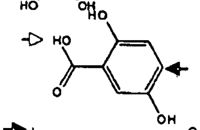 |
| 1432 | Glycolaldehyde | 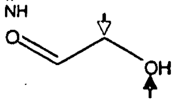 | 1445 | Geranyl acetate | 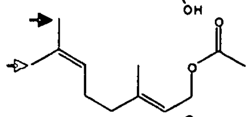 |
| 1433 | Cyclohexa-3,5-diene-1,2-dicarboxylate | 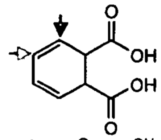 | 1446 | Geranic acid | 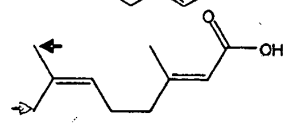 |
| 1434 | Glycolyl-D-mannosamine | 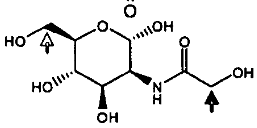 | 1447 | Geranial | 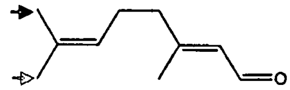 |
| 1435 | Glycolyl-D-mannosaminolactone | 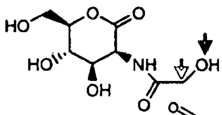 | 1448 | Geraniol | 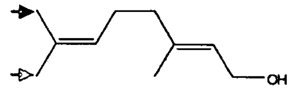 |
| 1436 | Octanoic acid | 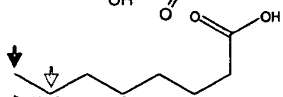 | 1449 | trans-Geranyl-CoA | 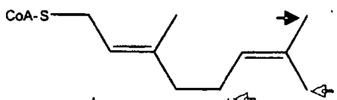 |
| 1437 | GDP-6-deoxy-D-mannose | 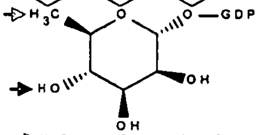 | 1450 | cis-Geranyl-CoA | 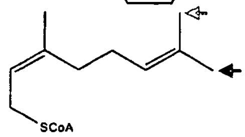 |
| 1438 | GDP-6-deoxy-D-talose | 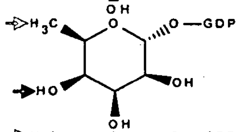 | 1451 | Geranylate | 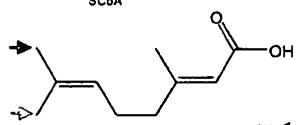 |
| 1439 | GDP-4-dehydro-6-deoxy-D-mannose | 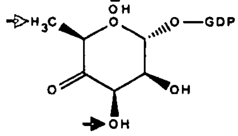 | 1452 | 3-beta-D-Galactosyl-sn-glycerol | 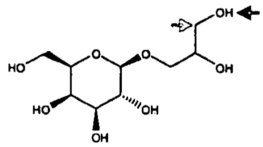 |
| 1440 | GDP-D-mannose | 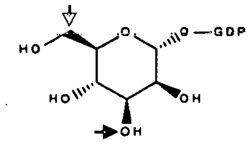 | 1453 | Geranylgeranyl diphosphate | 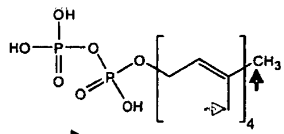 |
| 1441 | GDP-L-fucose | 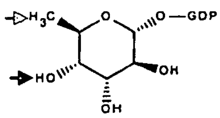 | 1454 | beta-D-Glucose | 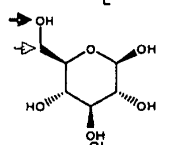 |
| 1442 | 6-Deoxy-D-glucose | 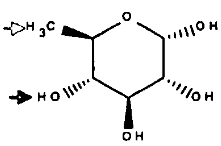 | 1455 | gamma-Hexachlorocyclohexane | 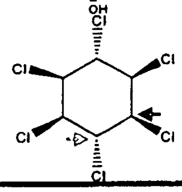 |

| N° | Substrate | Chemical Structure | N° | Substrate | Chemical Structure |
|---|---|---|---|---|---|
| 1456 | 1-alpha-D-Galactosyl-myo-inositol | 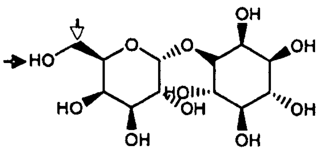 | 1469 | alpha-D-Glucose | 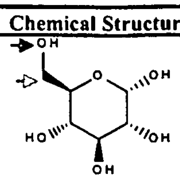 |
| 1457 | Nitrobenzene | 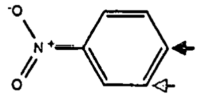 | 1470 | gamma-L-Glutamyl-L-cysteine | 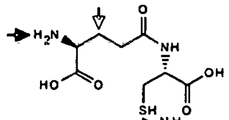 |
| 1458 | D-Gluconate | 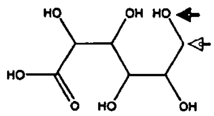 | 1471 | D-Glutamate | 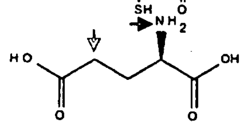 |
| 1459 | Glutarate | 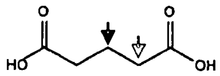 | 1472 | 3-Hydroxy-5-methylhex-4-enoyl-CoA | 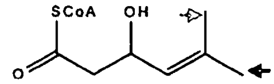 |
| 1460 | D-Glucuronate | 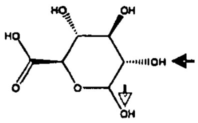 | 1473 | Glutaryl-CoA | 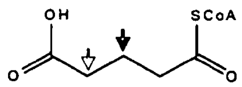 |
| 1461 | D-Glutamine | 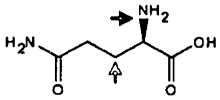 | 1474 | D-Glucurono-3,6-lactone | 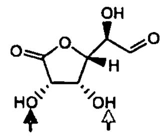 |
| 1462 | L-Glutamine | 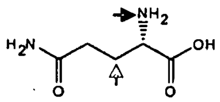 | 1475 | Glucomannan | 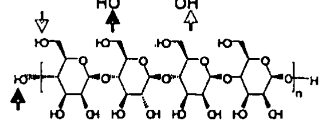 |
| 1463 | L-Glutamate 1-semialdehyde | 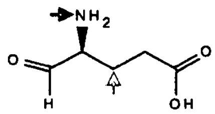 | 1476 | 2-Hydroxycinnamate | 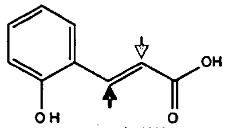 |
| 1464 | L-Glutamate 5-semialdehyde | 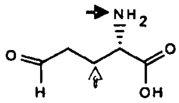 | 1477 | L-Glutamate | 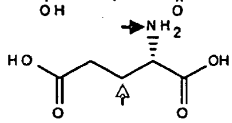 |
| 1465 | L-Glutamate 5-phosphate | 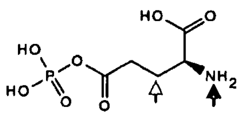 | 1478 | Glyoxylate | 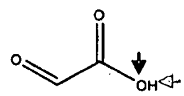 |
| 1466 | D-Glucurono-6,2-lactone | 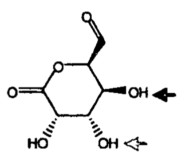 | 1479 | alpha-D-Glutamyl phosphate | 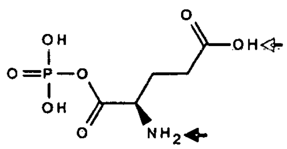 |
| 1467 | gamma-L-Glutamyl-D-alanine | 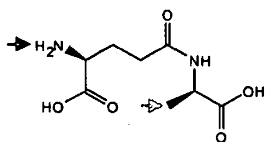 | 1480 | 2,3-Bisphospho-D-glycerate | 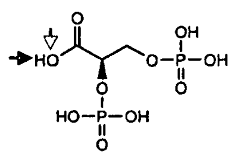 |
| 1468 | beta-D-Glucan | 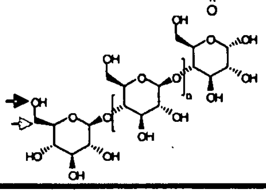 | 1481 | Glycine | 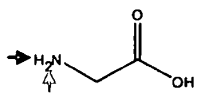 |

| N° | Substrate | Chemical Structure | N° | Substrate | Chemical Structure |
|---|---|---|---|---|---|
| 1482 | 1-Hydroxy-2-naphthaldehyde | 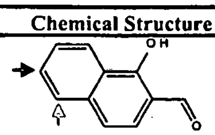 | 1495 | D-Glucosaminate | 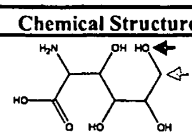 |
| 1483 | D-Glyceraldehyde | 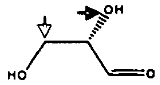 | 1496 | D-Glucosaminide | 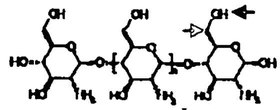 |
| 1484 | D-Glycerate 3-phosphate | 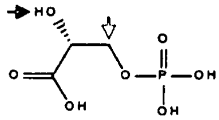 | 1497 | Glycerone phosphate | 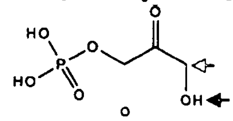 |
| 1485 | L-Glutamate methylester | 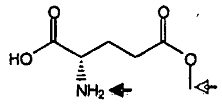 | 1498 | Glyoxalate | 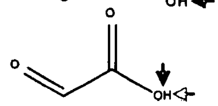 |
| 1486 | D-Glycero-D-manno-heptose 1,7-bisphosphate | 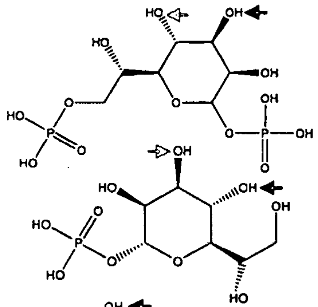 | 1499 | D-Galactono-1,4-lactone | 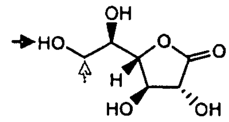 |
| 1487 | D-Glycero-D-manno-heptose 1-phosphate | | 1500 | P1,P4-Bis(5'-guanosyl) tetraphosphate | 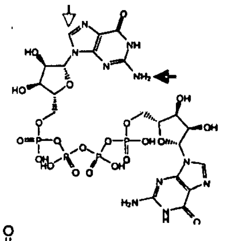 |
| 1488 | D-Glycero-D-manno-heptose 7-phosphate | 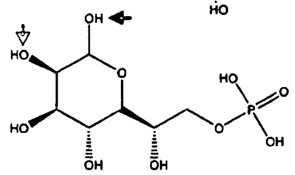 | 1501 | Geranyl diphosphate | 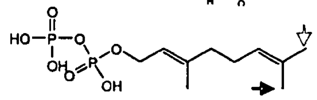 |
| 1489 | Glycogen | 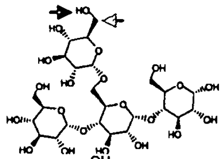 | 1502 | Guanosine | 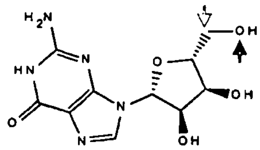 |
| 1490 | (R)-Glycerate | 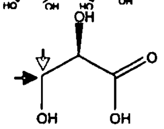 | 1503 | Glutathione oxidized | 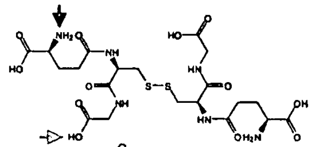 |
| 1491 | Glycerone | 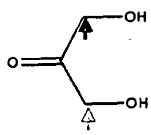 | 1504 | Glutathione reduced | 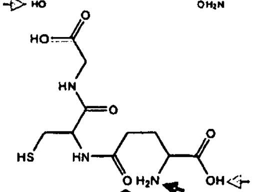 |
| 1492 | 1,3-Bisphospho-D-glycerate | 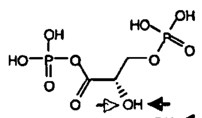 | 1505 | 2,3,4,5-Tetrahydrodipicolinate | 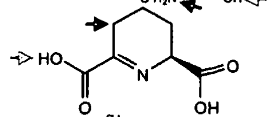 |
| 1493 | Glycerol | 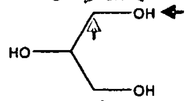 | 1506 | Glutathionylspermidine | 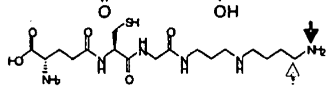 |
| 1494 | Glycolate | 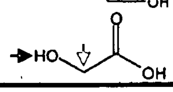 | 1507 | Tetrahydropteroyltri-L-glutamate | 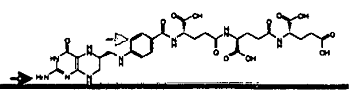 |

| N° | Substrate | Chemical Structure | N° | Substrate | Chemical Structure |
|---|---|---|---|---|---|
| 1508 | Guanidoacetate | 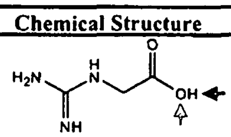 | 1521 | 2,2',3,4',5,5',6-Heptachloro-4-biphenylol | 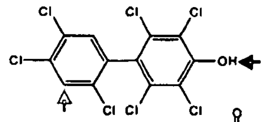 |
| 1509 | Guaiacol | 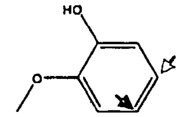 | 1522 | (15S)-15-Hydroxy-5,8,11-cis-13-trans-eicosatetraenoate | 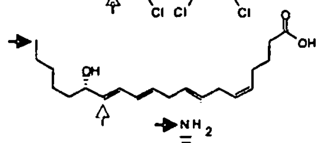 |
| 1510 | L-Gulose | 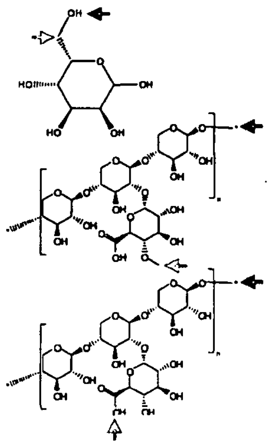 | 1523 | L-Homocysteine | 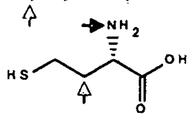 |
| 1511 | Glucuronoxylan 4-O-methyl-D-glucuronate | | 1524 | alpha-D-Glucose 6-phosphate | 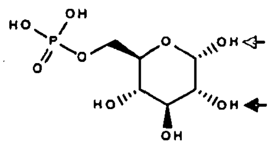 |
| 1512 | Glucuronoxylan D-glucuronate | | 1525 | Hexadecenoic acid | 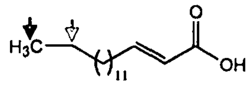 |
| 1513 | 1-Hydroxy-2-methyl-2-(E)-butenyl 4-diphosphate | 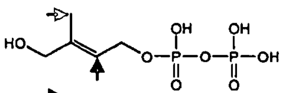 | 1526 | trans-Hexadec-2-enoyl-CoA | 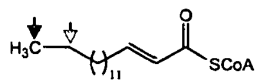 |
| 1514 | (E)-4-Hydroxy-3-methylbut-2-en-1-yl diphosphate | 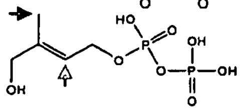 | 1527 | cis-4-[2-(3-Hydroxy)-thionaphthenyl]-2-oxo-3-butenoate | 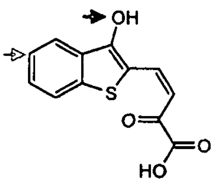 |
| 1515 | (S)-3-Hydroxy-3-methylglutaryl-CoA | 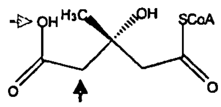 | 1528 | RNA Helicase substrate | Not available |
| 1516 | Galactarate | 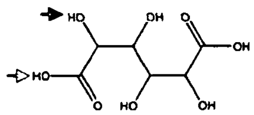 | 1529 | Heptane | 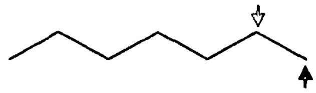 |
| 1517 | But-1-ene-1,2,4-tricarboxylate | 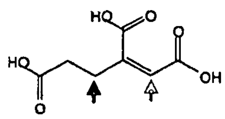 | 1530 | Heptadecanoyldolichol | 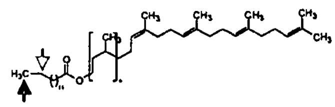 |
| 1518 | Hydroxyacetone phosphate | 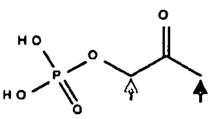 | 1531 | Heptadecanoic acid | 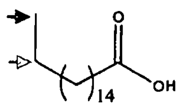 |
| 1519 | cis-4-(2-hydroxynaphth-3-yl)-2-oxobut-3-enoic acid | 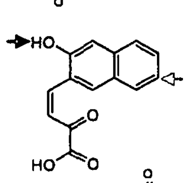 | 1532 | Heptadecane | 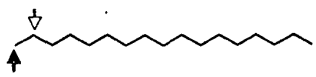 |
| 1520 | 3-Hydroxy-2-naphthoate | 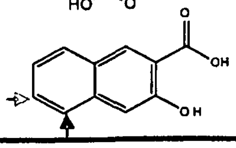 | 1533 | Heptadecanoyl-CoA | 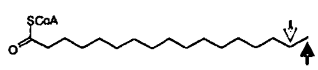 |

| N° | Substrate | Chemical Structure | N° | Substrate | Chemical Structure |
|---|---|---|---|---|---|
| 1534 | all-trans-Heptaprenyl diphosphate | 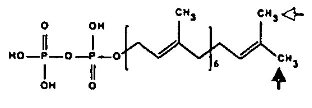 | 1547 | 2,2',3,3',5,5'-Hexachlorobipheny l | 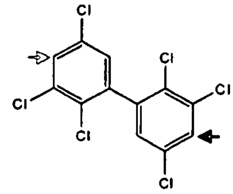 |
| 1535 | Heptanoic acid | 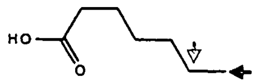 | 1548 | 2,2',3,3',6,6'-Hexachlorobipheny l | 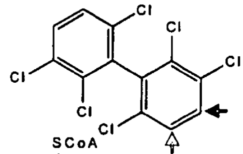 |
| 1536 | trans-2-Methyl-5-isopropylhexa-2,5-dienoyl-CoA | 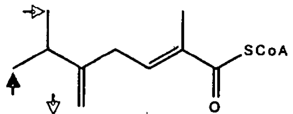 | 1549 | Hexanoyl-CoA | 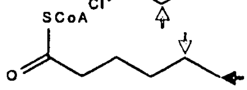 |
| 1537 | Heptanal | 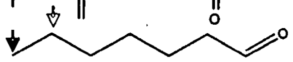 | 1550 | Hexadecanoyl-CoA | 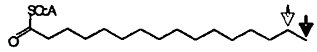 |
| 1538 | Hexanoic acid | 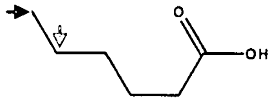 | 1551 | 1,2-Epoxyhexadecane | 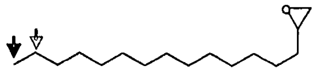 |
| 1539 | Hexacosane | 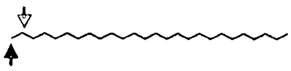 | 1552 | Hexadecanoylglyce rone phosphate | 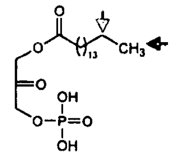 |
| 1540 | Hexadecane | 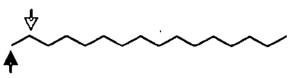 | 1553 | Hexanoyldolichol | 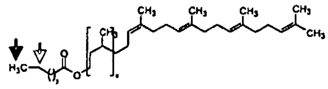 |
| 1541 | 2,6-Dimethyl-5-methylene-3-oxo-heptanoyl-CoA | 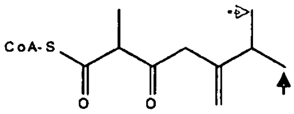 | 1554 | L-Histidinol phosphate | 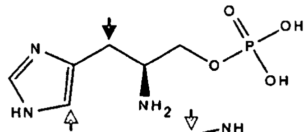 |
| 1542 | Hexacosanoate | 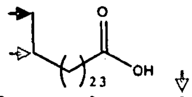 | 1555 | Histamine | 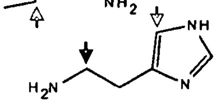 |
| 1543 | Hexanal | 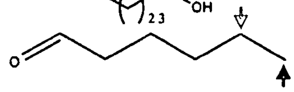 | 1556 | (S)-3-Hydroxyisobutyrat e | 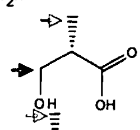 |
| 1544 | Hexyl cinnamaldehyde | 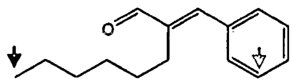 | 1557 | (S)-3-Hydroxyisobutyryl -CoA | 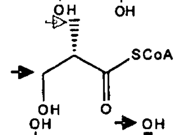 |
| 1545 | 2,2',4,4',5,5'-Hexachlorobiphenyl | 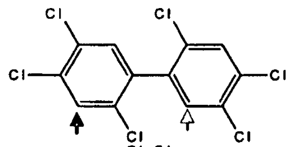 | 1558 | Homoisocitrate | 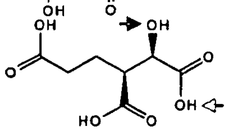 |
| 1546 | 2,2',4,4',6,6'-Hexachlorobiphenyl | 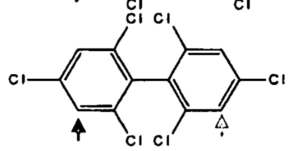 | 1559 | Histone-arginine | 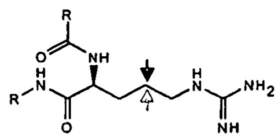 |

| N° | Substrate | Chemical Structure | N° | Substrate | Chemical Structure |
|---|---|---|---|---|---|
| 1560 | L-Histidine | 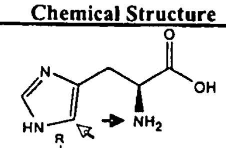 | 1574 | 2-Hydroxy-2H-benzo[h]chromene 2-carboxylate | 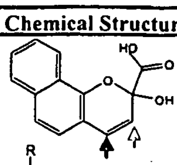 |
| 1561 | Histone L-lysine | 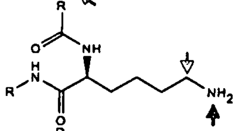 | 1575 | Histone N6-methyl-L-lysine | 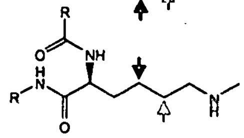 |
| 1562 | Histone N(omega)-methyl-L-arginine | 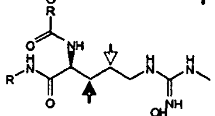 | 1576 | Homogentisate | 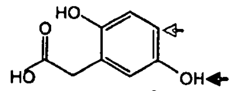 |
| 1563 | 3,3',4',5,7,8-Hexahydroxyflavone | 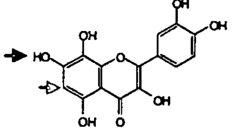 | 1577 | 3-Hydroxypropanoate | 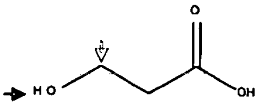 |
| 1564 | D-Hexose | 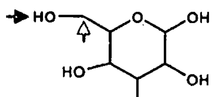 | 1578 | 3-Hydroxypropionyl CoA | 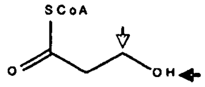 |
| 1565 | L-Histidinol | 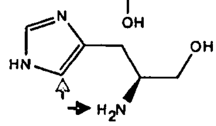 | 1579 | (S)-3-Hydroxybutanoate | 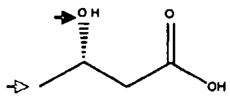 |
| 1566 | Histone | Not available | 1580 | trans-4-Hydroxy-L-proline | 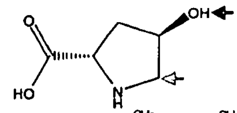 |
| 1567 | 2-Hydroxy-6-oxonona-2,4-diene-1,9-dioate | 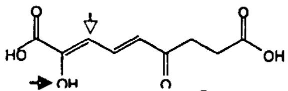 | 1581 | all-trans-Hexaprenyl diphosphate | 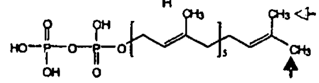 |
| 1568 | 2-Oxohept-3-enedioate | 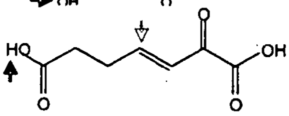 | 1582 | Hydroxypyruvate | 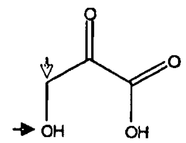 |
| 1569 | 3-Hydroxy-L-kynurenine | 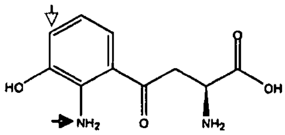 | 1583 | cis-4-Hydroxy-L-proline | 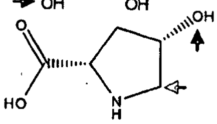 |
| 1570 | Hydroxymethylbilane | 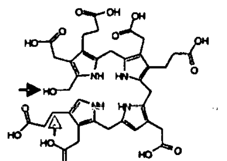 | 1584 | 4-Hydroxy-2-oxohexanoate | 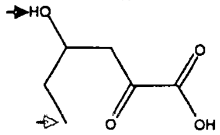 |
| 1571 | (S)-3-Hydroxy-2-methylbutyryl-CoA | 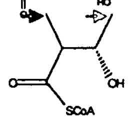 | 1585 | Hypoxanthine | 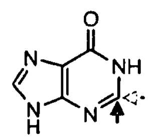 |
| 1572 | trans-4-[2-(3-Hydroxy)-thionaphthenyl]-2-oxo-3-butenoate | 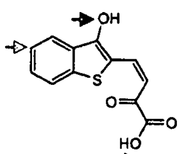 | 1586 | trans-Hexacos-2-enoyl-CoA | 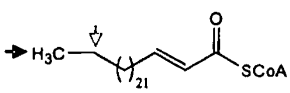 |
| 1573 | L-Homoserine | 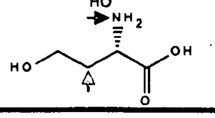 | 1587 | Hexadecanal | 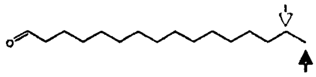 |

| N° | Substrate | Chemical Structure | N° | Substrate | Chemical Structure |
|---|---|---|---|---|---|
| 1588 | 4-Hydroxy-2-oxopentanoate | 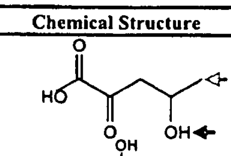 | 1601 | Iminoaspartate | 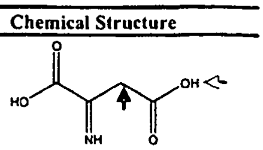 |
| 1589 | Hydroxyatrazine | 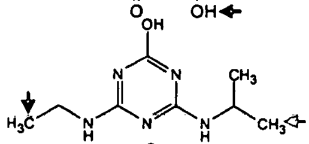 | 1602 | 2-Methylpropanoyl-CoA | 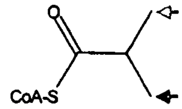 |
| 1590 | Hydantoin-5-propionate | 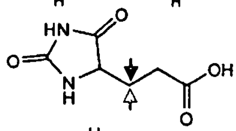 | 1603 | Isobutyryl-CoA | 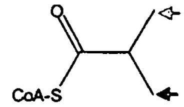 |
| 1591 | 2-(Indol-3-yl)acetaldehyde | 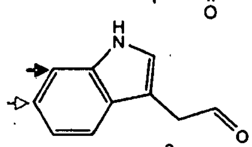 | 1604 | 6-Aminohexanoate | 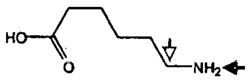 |
| 1592 | Indole-3-acetyl-beta-1-D-glucose | 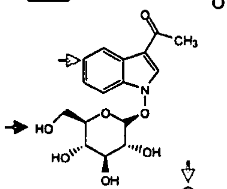 | 1605 | Isochorismate | 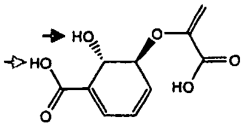 |
| 1593 | Indole-3-acetyl-L-glutamine | 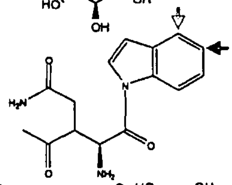 | 1606 | L-Idonate | 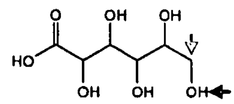 |
| 1594 | Indole-3-acetyl-myo-inositol | 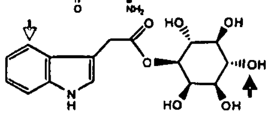 | 1607 | Indole-3-acetaldehyde | 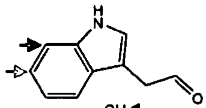 |
| 1595 | 2-Phenylacetamide | 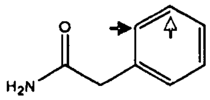 | 1608 | Isocitrate | 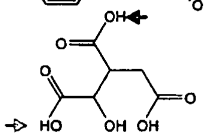 |
| 1596 | Indole-3-acetamide | 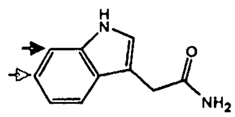 | 1609 | Inosine 5'-diphosphate | 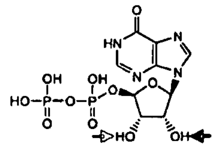 |
| 1597 | (Indol-3-yl)pyruvate | 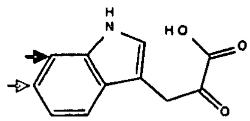 | 1610 | Gentisate aldehyde | 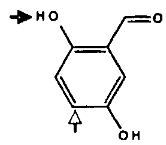 |
| 1598 | Pentadecanal | 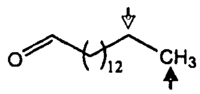 | 1611 | Indoleglycerol phosphate | 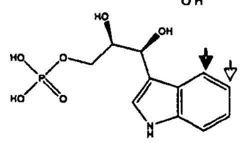 |
| 1599 | Indole-3-ethanol | 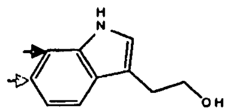 | 1612 | cis-3,4-Dihydroxyphenanthrene-4-carboxylate | 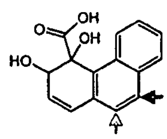 |
| 1600 | (Indol-3-yl)acetate | 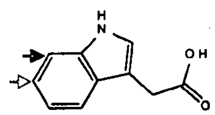 | 1613 | L-Isoleucine | 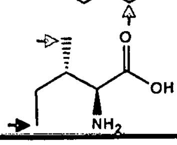 |

| N° | Substrate | Chemical Structure | N° | Substrate | Chemical Structure |
|---|---|---|---|---|---|
| 1614 | 3-(Imidazol-4-yl)-2-oxopropyl phosphate | 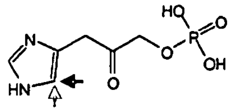 | 1627 | Inosine 5'-tetraphosphate | 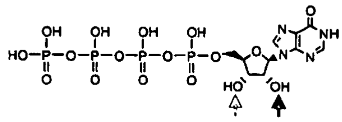 |
| 1615 | L-Histidinal | 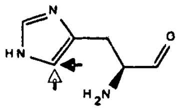 | 1628 | Inosine 5'-triphosphate | 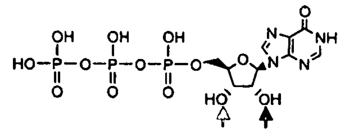 |
| 1616 | Imidazo[1,5a]pyrimidine | 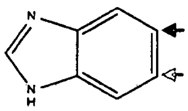 | 1629 | Isopentenyl diphosphate | 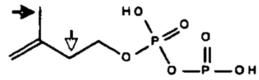 |
| 1617 | 4-Imidazolone-5-propanoate | 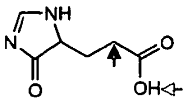 | 1630 | 2-Isopropylmalate | 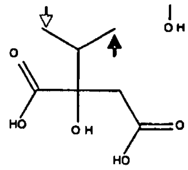 |
| 1618 | Indoxazene | 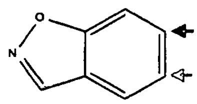 | 1631 | Isothiazoline | 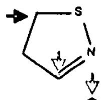 |
| 1619 | Indane | 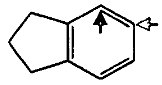 | 1632 | Isobenzofuran | 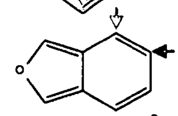 |
| 1620 | Indene | 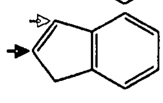 | 1633 | Isomaltose | 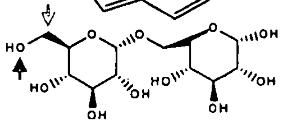 |
| 1621 | Methyl 3-hydroxybenzoate | 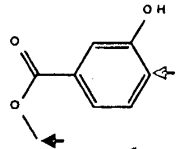 | 1634 | Isochromen-3-one | 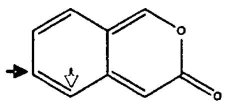 |
| 1622 | Indoline | 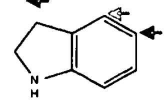 | 1635 | Isocoumarin | 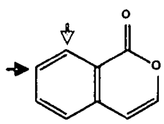 |
| 1623 | 2,3-Benzopyrrole | 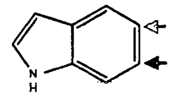 | 1636 | 2-Methylpropanoate | 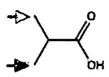 |
| 1624 | myo-Inositol | 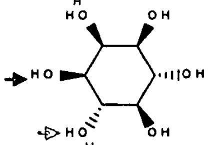 | 1637 | Isoorientin | 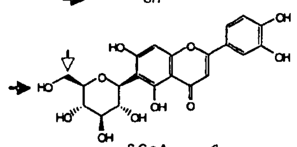 |
| 1625 | Indole-3-pyruvate | 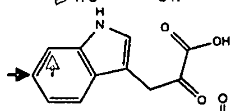 | 1638 | Isopentanoyl-CoA | 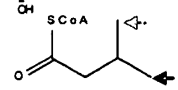 |
| 1626 | Inosine | 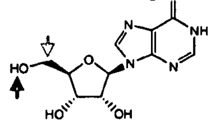 | 1639 | Isoprene | 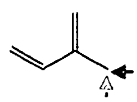 |

| N° | Substrate | Chemical Structure | N° | Substrate | Chemical Structure |
|---|---|---|---|---|---|
| 1640 | L-Carnitinamide | 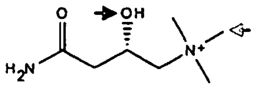 | 1653 | Kanosamine 6 Phosphate | 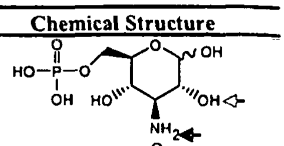 |
| 1641 | 2-Benzazine | 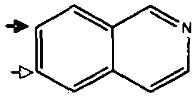 | 1654 | 3-Deoxy-D-manno-2-octulosonate | 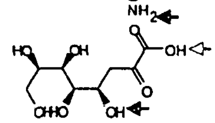 |
| 1642 | Isoscoparin | 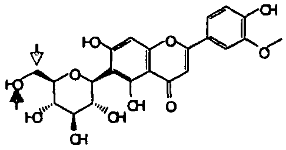 | 1655 | 3-Deoxy-D-manno-octulosonate 8-phosphate | 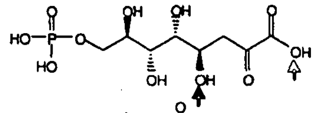 |
| 1643 | Imidazolone | 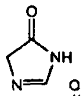 | 1656 | alpha-Ketoglutaric acid | 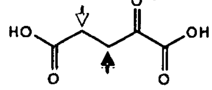 |
| 1644 | Ethyl isovalerate | 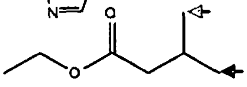 | 1657 | Ketoprofen methyl ester | 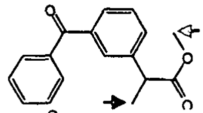 |
| 1645 | Isovaleryl-CoA | 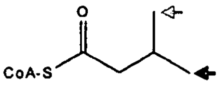 | 1658 | (R)-2-Methylmalate | 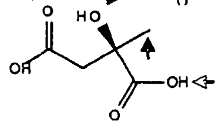 |
| 1646 | Isoxazolidine | 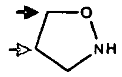 | 1659 | (2R,3S,4R,5R)-2-(aminomethyl)oxane 2,3,4,5-tetrol | 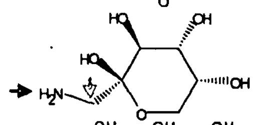 |
| 1647 | Isoxazole | 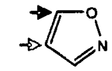 | 1660 | Ketose | 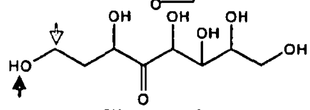 |
| 1648 | 7-Hydroxycoumarin | 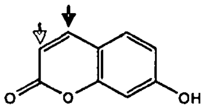 | 1661 | 5-Carboxymethyl-2-hydroxymuconate | 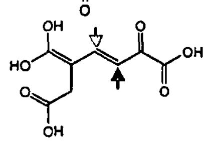 |
| 1649 | Carnitine | 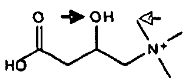 | 1662 | Jasmonate | 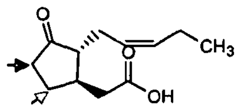 |
| 1650 | (2S)-2-Isopropyl-3-oxosuccinate | 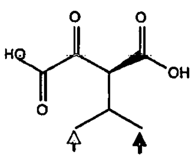 | 1663 | Laminarin | 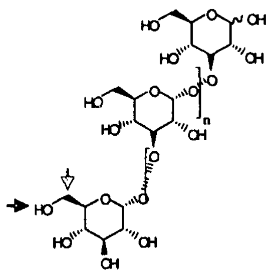 |
| 1651 | 5-Carboxymethyl-2-hydroxymuconate semialdehyde | 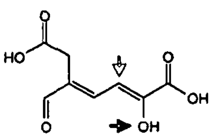 | 1664 | L-Arabinonate | 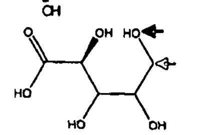 |
| 1652 | Hexose-1,5-lactone | 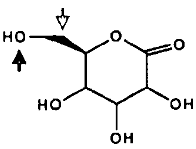 | 1665 | Lactitol | 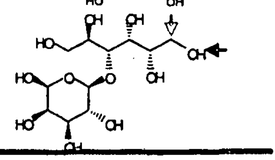 |

| N° | Substrate | Chemical Structure | N° | Substrate | Chemical Structure |
|---|---|---|---|---|---|
| 1666 | L-Lactaldehyde | 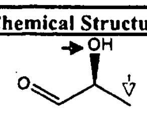 | 1679 | Leucine p-nitroaniline | 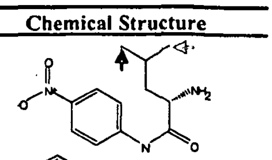 |
| 1667 | D-Lactate | 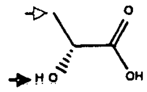 | 1680 | L-Formylkynurenine | 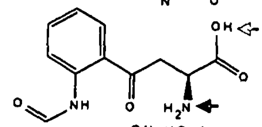 |
| 1668 | L-Arabinitol | 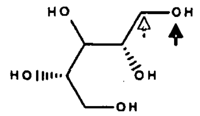 | 1681 | L-Gulono-1,4-lactone | 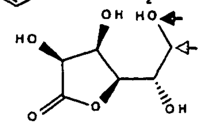 |
| 1669 | L-Arabitol | 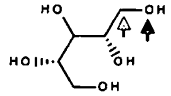 | 1682 | L-Leucyl-glycyl-glycine | 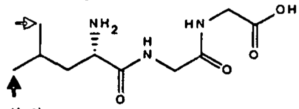 |
| 1670 | L-Arabonate | 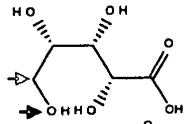 | 1683 | L-Homocitrulline | 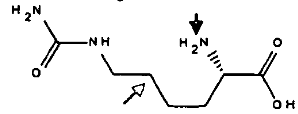 |
| 1671 | L-Lactate | 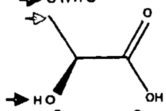 | 1684 | L-Gulonate | 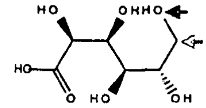 |
| 1672 | L-Arogenate | 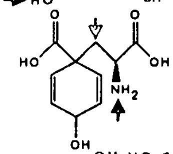 | 1685 | (R)-S-Lactoylglutathione | 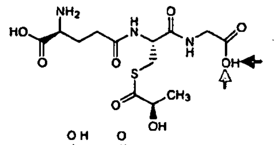 |
| 1673 | L-Ascorbic acid | 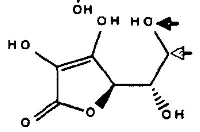 | 1686 | Licodione | 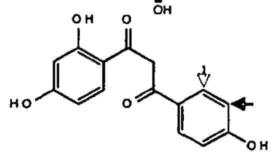 |
| 1674 | L-Leucine | 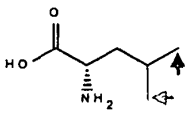 | 1687 | L-Iditol | 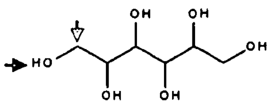 |
| 1675 | Itaconyl-CoA | 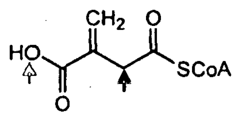 | 1688 | Limonoate | 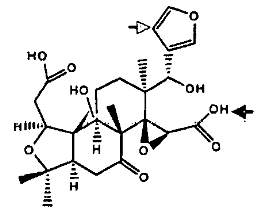 |
| 1676 | Itaconate | 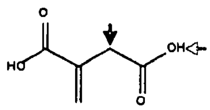 | 1689 | Limonene-1,2-diol | 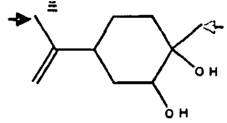 |
| 1677 | Lactose | 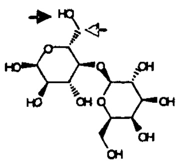 | 1690 | (-)-(S)-Limonene | 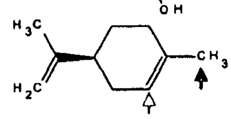 |
| 1678 | 5-Aminoimidazole | 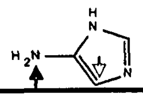 | 1691 | Limonene-1,2-epoxide | 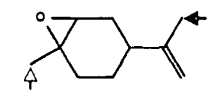 |

| N° | Substrate | Chemical Structure | N° | Substrate | Chemical Structure |
|---|---|---|---|---|---|
| 1692 | Lindane | 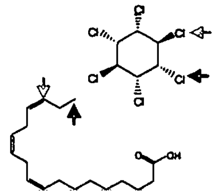 | 1706 | L-Rhamno-1,4-lactone | 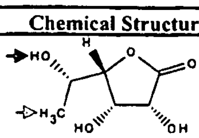 |
| 1693 | Linolenate | | 1707 | L-Xylono-1,4-lactone | 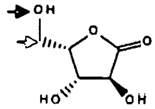 |
| 1694 | Linoleate | 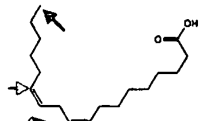 | 1708 | L-Xylo-Hex-3-ulono-1,4-lactone | 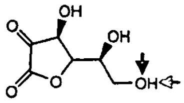 |
| 1695 | Linoleyl-CoA | 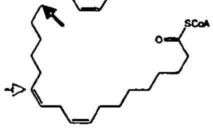 | 1709 | L-Xylonate | 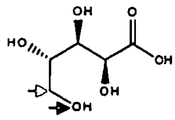 |
| 1696 | 2,3-Bis(3-Hydroxytetradecanoyl)-D-glucosaminyl-1,6-beta-D-2,3-bis(3-hydroxytetradecanoyl)-beta-D-glucosaminyl 1-phosphate | 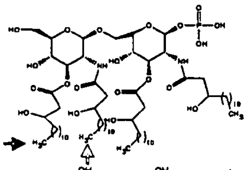 | 1710 | Methyl-2-bromopropionate | 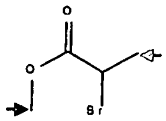 |
| 1697 | 2,3-Bis(3-hydroxytetradecanoyl)-beta-D-glucosaminyl 1-phosphate | 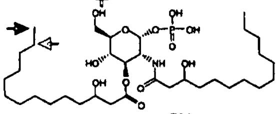 | 1711 | Methyl 2-hydroxybenzoate | 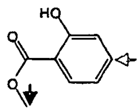 |
| 1698 | 3-Methylsalicylaldehyde | 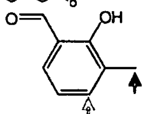 | 1712 | N-Methyl-(R,S)-1-benzyl-1,2,3,4-tetrahydroisoquinoline | 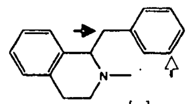 |
| 1699 | L-Kynurenine | 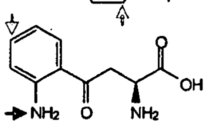 | 1713 | alpha-D-Mannosyl-beta-D-mannosyl-diacetylchitobiosyldiphosphodolichol | 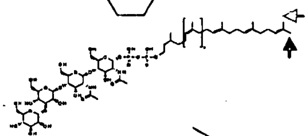 |
| 1700 | DL-Leucine, benzyl ester | 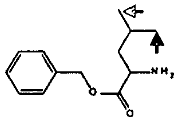 | 1714 | Methyl 2,2-dimethyl-1,3-dioxane-4-carboxylate | 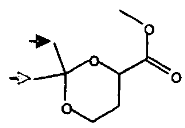 |
| 1701 | 6-Lactoyl-5,6,7,8-tetrahydropterin | 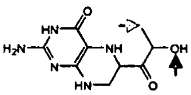 | 1715 | L-Lysine | 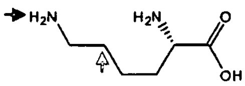 |
| 1702 | 2-Methyl-3-oxopropanoate | 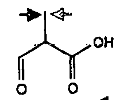 | 1716 | 3-Methylsalicylate | 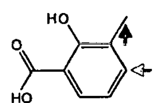 |
| 1703 | 2-Methylhomogentisate | 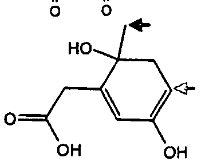 | 1717 | (alpha-D-Mannosyl)4-beta-D-mannosyl-diacetylchitobiosyldiphosphodolichol | 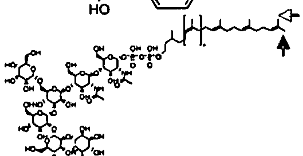 |
| 1704 | (+)-(R)-Limonene | 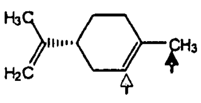 | 1718 | Methyl-2-hydroxy-2-methylpropionate | 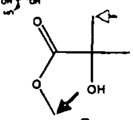 |
| 1705 | L-Palmitoylcarnitine | 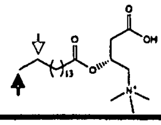 | 1719 | Methyl-3-bromopropionate | 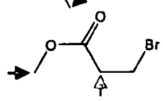 |

| N° | Substrate | Chemical Structure | N° | Substrate | Chemical Structure |
|---|---|---|---|---|---|
| 1720 | Melibiitol | 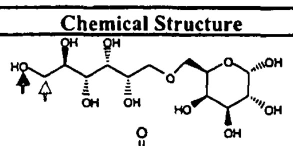 | 1734 | Maltitol | 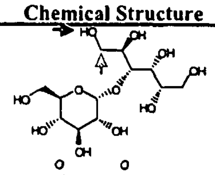 |
| 1721 | Inosine 5'-monophosphate | 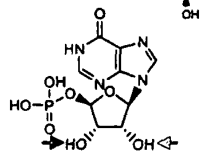 | 1735 | Malonate | 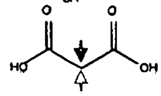 |
| 1722 | 3-Oxohexanoate | 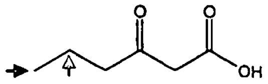 | 1736 | L-Mannuronate | 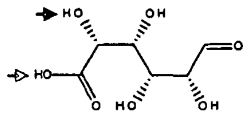 |
| 1723 | cis-1,2-Dihydroxy-4-methylcyclohexa3,5-diene-1-carboxylate | 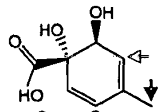 | 1737 | Methylmalonate | 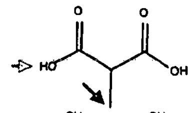 |
| 1724 | 4-Maleylacetoacetate | 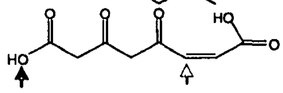 | 1738 | alpha-Maltose | 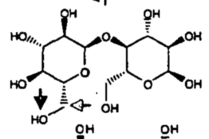 |
| 1725 | Methyl 4-hydroxybenzoate | 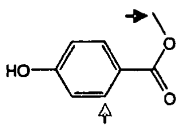 | 1739 | Maltose 6'-phosphate | 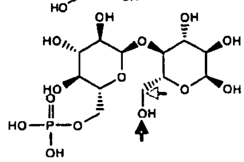 |
| 1726 | beta-D-Mannosyldiacetylchitobiosyldiphosphodolichol | 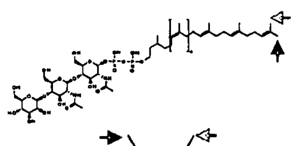 | 1740 | beta-Maltose | 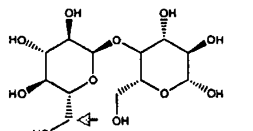 |
| 1727 | 2-Methylacetoacetyl-CoA | 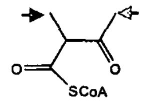 | 1741 | Isomaltotriose | 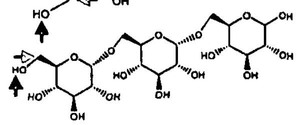 |
| 1728 | 2-Methylprop-2-enoyl-CoA | 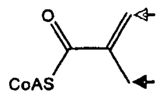 | 1742 | Isomaltotetraose | Too big |
| 1729 | 3-Methylmuconolactone | 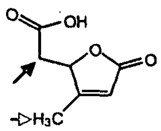 | 1743 | Isomaltopentaose | Too big |
| 1730 | Malathion | 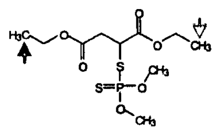 | 1744 | Isomaltohexaose | Too big |
| 1731 | Malonyl-CoA | 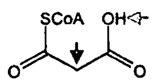 | 1745 | Methyl-3-bromo-2-methylpropionate | 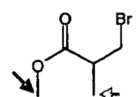 |
| 1732 | Maleate | 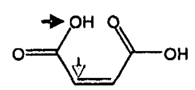 | 1746 | Methylmalonate | 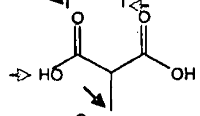 |
| 1733 | Melilotate | 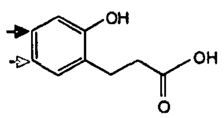 | 1747 | 2-Ethyl-2-methyl-4-butyrolactone | 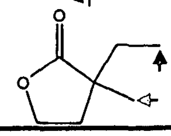 |

| N° | Substrate | Chemical Structure | N° | Substrate | Chemical Structure |
|---|---|---|---|---|---|
| 1748 | Mannobiose | 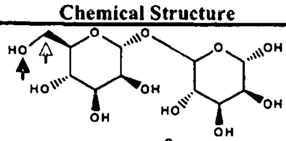 | 1761 | 1,2-Dihydroxy-3-methylcyclohexa-3,5-dienecarboxylate | 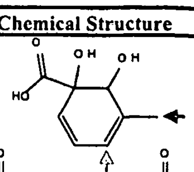 |
| 1749 | (R)-Mandelate | 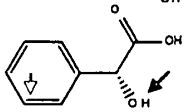 | 1762 | meso-2,6-Diaminopimelate | 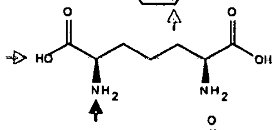 |
| 1750 | (-)-Menthol | 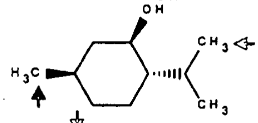 | 1763 | D-Mannose 1-phosphate | 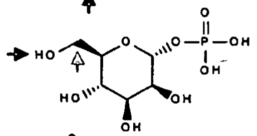 |
| 1751 | D-Mannosamine | 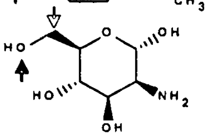 | 1764 | D-Mannose 6-phosphate | 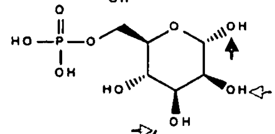 |
| 1752 | D-Mannose | 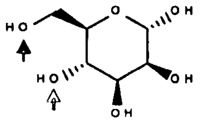 | 1765 | (-)-Menthyl acetate | 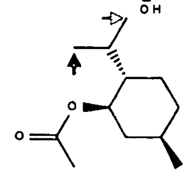 |
| 1753 | (S)-Mandelate | 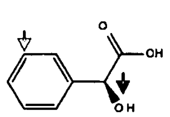 | 1766 | Mannan | Not available |
| 1754 | Mannotriose | 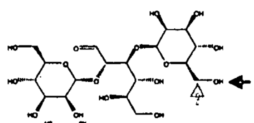 | 1767 | (-)-Menthone | 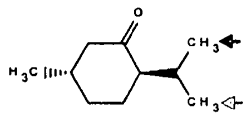 |
| 1755 | Mannotetraose | 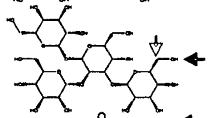 | 1768 | Methyl 3-hydroxybutyrate | 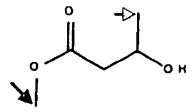 |
| 1756 | 2-Methylbutyryl-CoA | 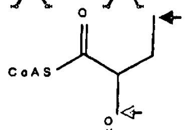 | 1769 | o-Methylbenzoate | 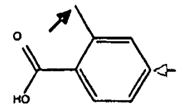 |
| 1757 | Malonate semialdehyde | 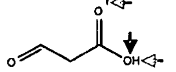 | 1770 | 2-Keto-3-deoxy-6-phosphogluconate | 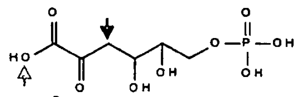 |
| 1758 | 2-Methylbut-2-enoyl-CoA | 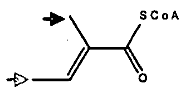 | 1771 | Methyl cinnamate | 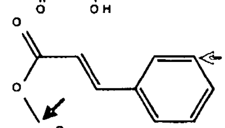 |
| 1759 | m-Cresol | 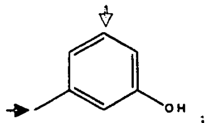 | 1772 | D-Methionine | 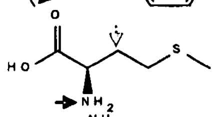 |
| 1760 | Melibiose | 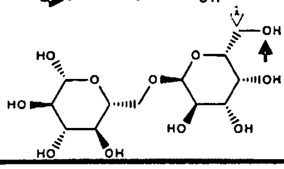 | 1773 | 2-Amino-2-methylpropanoate | 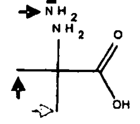 |

| N° | Substrate | Chemical Structure | N° | Substrate | Chemical Structure |
|---|---|---|---|---|---|
| 1774 | 5,10-Methenyltetrahydrofolate | 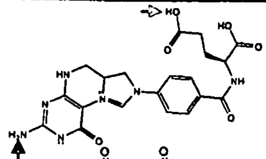 | 1788 | Mevaldate | 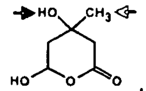 |
| 1775 | Methylmalonate | 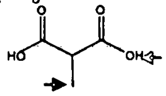 | 1789 | 7-Methylxanthine | 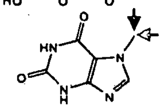 |
| 1776 | L-Methionine | 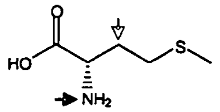 | 1790 | Methylisocitrate | 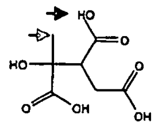 |
| 1777 | 3-Methyl-cis,cis-muconate | 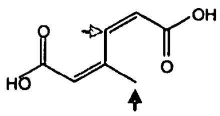 | 1791 | Limonoate D-ring-lactone | 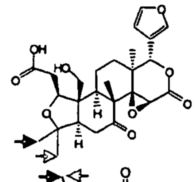 |
| 1778 | Benzylparaben | 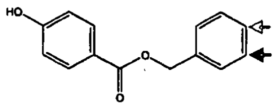 | 1792 | 7-Methyluric acid | 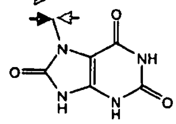 |
| 1779 | Methylparathion | 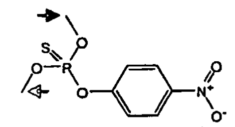 | 1793 | 1,7-Dimethyluric acid | 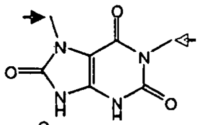 |
| 1780 | m-Methylbenzoate | 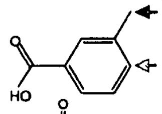 | 1794 | L-Malate | 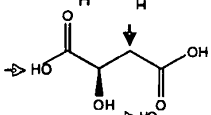 |
| 1781 | Xanthosine | 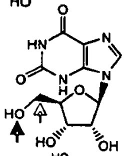 | 1795 | 5,10-Methylenetetrahydrofolate | 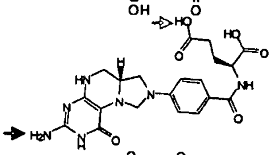 |
| 1782 | Mevalonic acid | 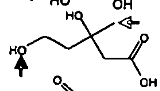 | 1796 | (R)-Methylmalonyl-CoA | 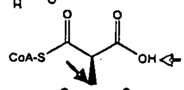 |
| 1783 | Methyl jasmonate | 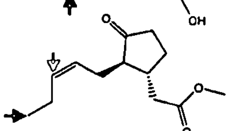 | 1797 | (S)-Methylmalonyl-CoA | 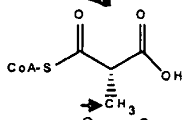 |
| 1784 | 1D-myo-Inositol 1-phosphate | 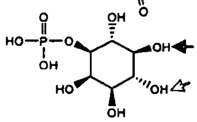 | 1798 | (S)-Methylmalonate semialdehyde | 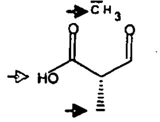 |
| 1785 | 3-Methylglutaconyl-CoA | 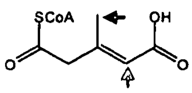 | 1799 | L-Met-L-Met-L-Met | 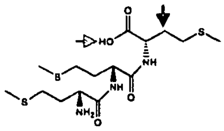 |
| 1786 | 4-Methylmuconolactone | 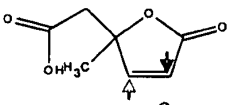 | 1800 | N-Acetylmethionine | 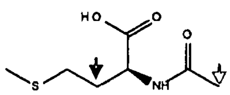 |
| 1787 | N-Methyl-L-histidine | 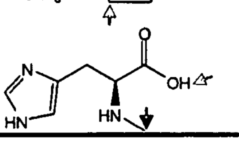 | 1801 | D-Mannitol | 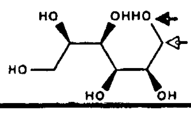 |

| N° | Substrate | Chemical Structure | N° | Substrate | Chemical Structure |
|---|---|---|---|---|---|
| 1802 | D-Mannitol l-phosphate | 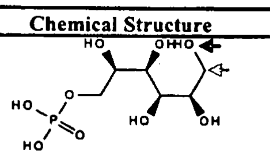 | 1816 | 4alpha-Methylzymosterol | 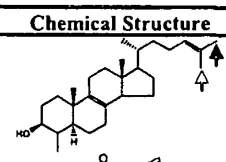 |
| 1803 | Tetrahydro-1,4-oxazine | 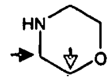 | 1817 | 3-Hydroxyisovaleryl-CoA | 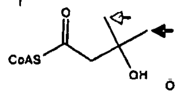 |
| 1804 | 4-Hydroxyphenylacetate | 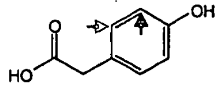 | 1818 | N4-Acetylaminobutanal | 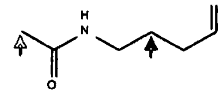 |
| 1805 | 2D-5-O-methyl-2,3,5/4,6-pentahydroxycyclohexanone | 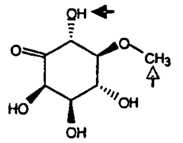 | 1819 | 2-Oxopropanoate | 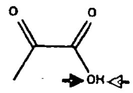 |
| 1806 | Menaquinol | 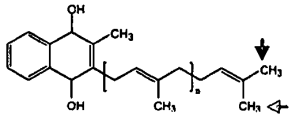 | 1820 | N1-Acetylspermine | 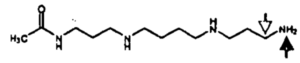 |
| 1807 | Menaquinone | 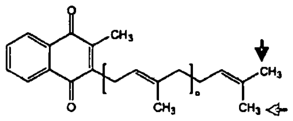 | 1821 | 2-Nonaprenyl-4-hydroxybenzoate | 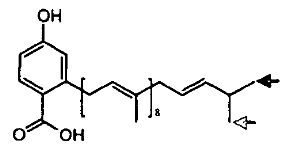 |
| 1808 | 5-Methyltetrahydropteroyl tri-L-glutamate | 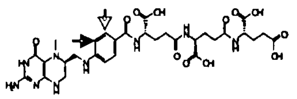 | 1822 | N-Acetyl-4-O-acetylneuraminate | 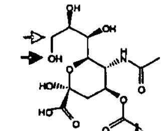 |
| 1809 | Methylglyoxal | 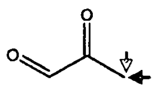 | 1823 | N-Acetyl-5-methoxytryptamine | 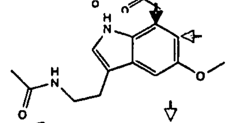 |
| 1810 | (S)-5-Oxo-2,5-dihydrofuran-2-acetate | 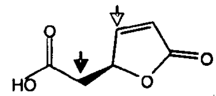 | 1824 | Nicotinate D-ribonucleoside | 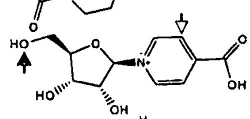 |
| 1811 | Mucic acid | 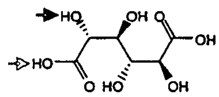 | 1825 | N-Acetylarylamine | 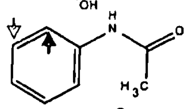 |
| 1812 | (R)-Mevalonate | 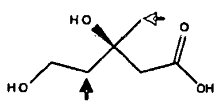 | 1826 | N-Benzoyl-D-arginine-4-nitroanilide | 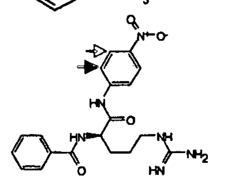 |
| 1813 | Phenanthrene-4-carboxylate | 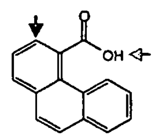 | 1827 | N-Acetyl-L-aspartate | 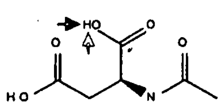 |
| 1814 | N1-Acetylspermidine | 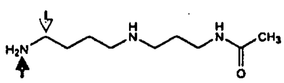 | 1828 | Nicotinate | 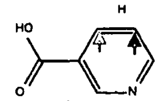 |
| 1815 | cis,cis-Muconate | 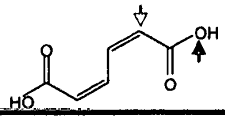 | 1829 | N-Acetylgalactosamine | 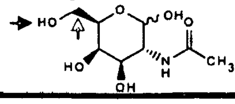 |

| N° | Substrate | Chemical Structure | N° | Substrate | Chemical Structure |
|---|---|---|---|---|---|
| 1830 | 1,2-Anthracenediol | 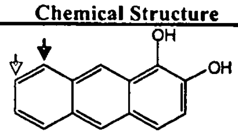 | 1843 | N-Acetyl-L-phenylalanine | 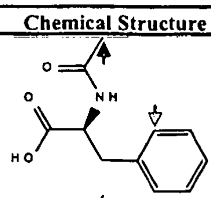 |
| 1831 | Nicotinamide adenine dinucleotide | 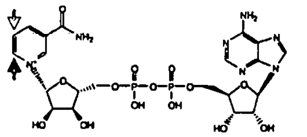 | 1844 | N-Acetyl-L-histidine | 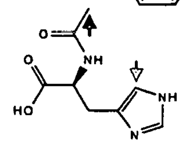 |
| 1832 | 2-Naphthaldehyde | 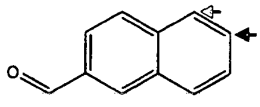 | 1845 | Nonadecanoic acid methyl ester | 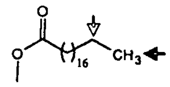 |
| 1833 | N-Acetyl-D-galactosaminoglycan | Not available | 1846 | Nonadecanoyldolichol | 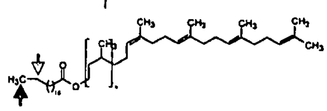 |
| 1834 | NADH | 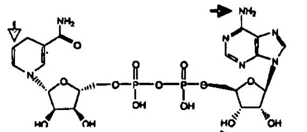 | 1847 | 2-Naphthalenesulfonate | 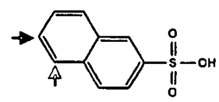 |
| 1835 | Nicotinamide adenine dinucleotide phosphate | 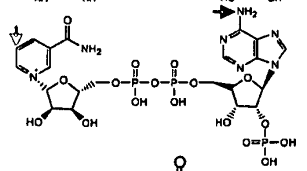 | 1848 | n-Butanol | 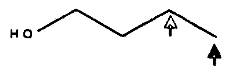 |
| 1836 | N-Acetylglycinamide | 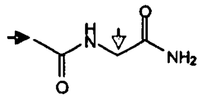 | 1849 | 2-Naphthalenol | 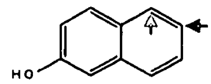 |
| 1837 | N-Acetyl-D-tryptophan | 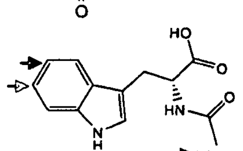 | 1850 | N-Acetylserotonin | 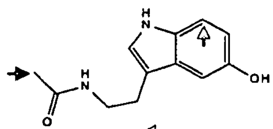 |
| 1838 | NADPH | 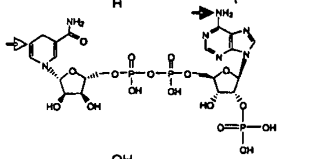 | 1851 | N-Acetyl-L-tyrosine ethyl ester | 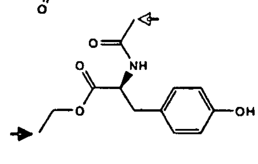 |
| 1839 | N-Acetyl-N-hydroxy-L-lysine | 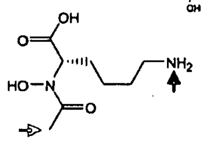 | 1852 | N-Benzoyl-4-hydroxyanthranilate | 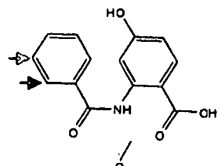 |
| 1840 | N-Ribosylnicotinamide | 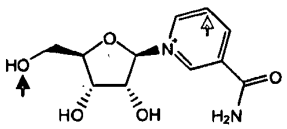 | 1853 | N-Benzoyl-4-methoxyanthranilate | 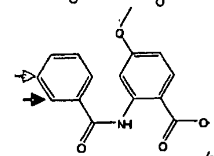 |
| 1841 | N-Acetylimidazole | 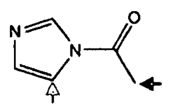 | 1854 | Ethyl benzoate | 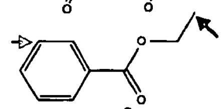 |
| 1842 | N6-Acetyl-L-lysine | 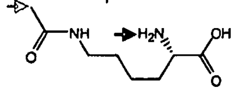 | 1855 | N-Benzoylglycine | 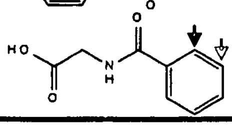 |

| N° | Substrate | Chemical Structure | N° | Substrate | Chemical Structure |
|---|---|---|---|---|---|
| 1856 | cis-2-Chlorodienelactone | 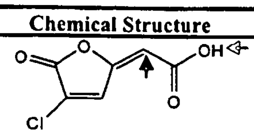 | 1869 | Nerol | 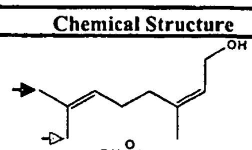 |
| 1857 | N-Butyryl-L-homoserine lactone | 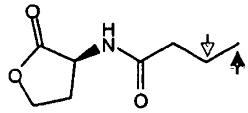 | 1870 | 1,6-Dihydroxy-cis-2,4-cyclohexadiene-1-carboxylic acid | 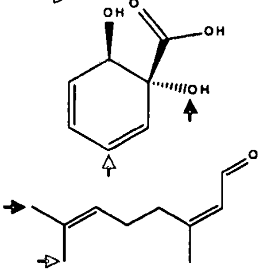 |
| 1858 | N-(3-Oxododecanoyl)homoserine lactone | 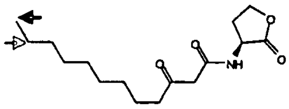 | 1871 | Neral | |
| 1859 | N-(3-Oxooctanoyl)homoserine lactone | 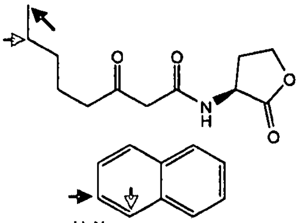 | 1872 | (+)-Neomenthol | 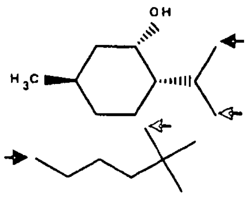 |
| 1860 | Naphthalene | | 1873 | Neooctane | |
| 1861 | Nicotinic acid amide | 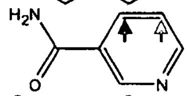 | 1874 | Neotetradecane | 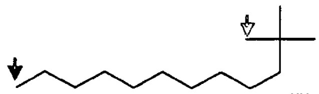 |
| 1862 | N-Carbamoyl-beta-alanine | 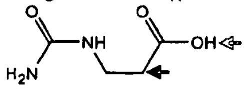 | 1875 | gamma-L-Glutamylputrescine | 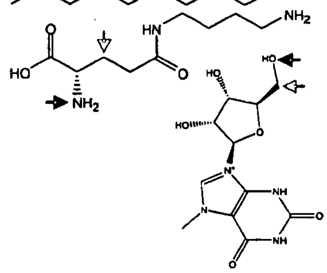 |
| 1863 | 3,6-Dichlorocatechol | 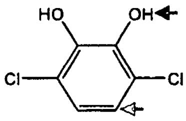 | 1876 | 7-Methylxanthosine | |
| 1864 | N-Carbamoylbeta-glycine | 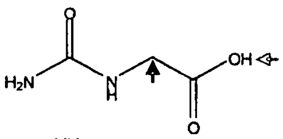 | 1877 | N-Heptadecanoyl-glycine | 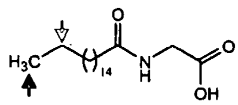 |
| 1865 | N-Carbamoylputrescine | 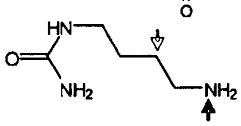 | 1878 | L-4-Hydroxyphenylglycine | 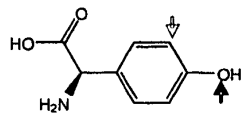 |
| 1866 | 2,5-Dichloro-cis,cis-muconate | 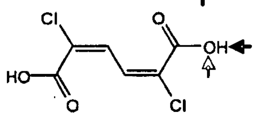 | 1879 | N-Hexadecanoyl-glycine | 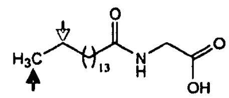 |
| 1867 | N-Dodecanoyl-glycine | 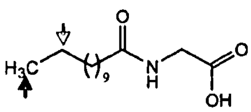 | 1880 | (3R)-3-Isoprenyl-6-oxoheptanoyl-CoA | 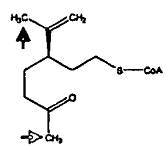 |
| 1868 | N-Decanoyl-glycine | 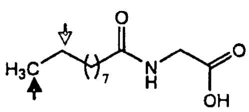 | 1881 | N-Hexanoyl-glycine | 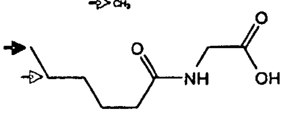 |

| N° | Substrate | Chemical Structure | N° | Substrate | Chemical Structure |
|---|---|---|---|---|---|
| 1882 | N-Hydroxy-L-lysine | 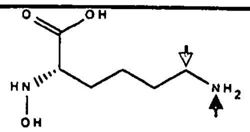 | 1895 | Pentadecan-1-ol | 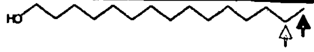 |
| 1883 | N-Heptanoyl-glycine | 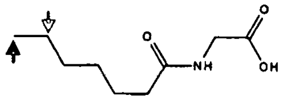 | 1896 | N-Methylphenylethanol amine | 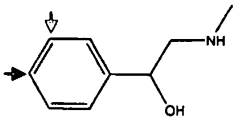 |
| 1884 | (3R)-3-Isopropenyl-6-oxoheptanoate | 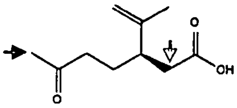 | 1897 | N-Methyltyramine | 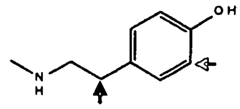 |
| 1885 | Nicotinate D-ribonucleotide | 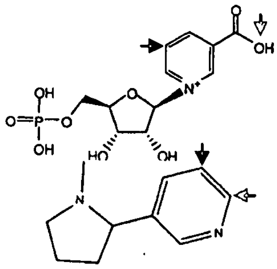 | 1898 | N,N-Dimethylaniline | 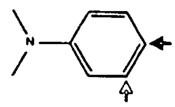 |
| 1886 | Nicotine | | 1899 | N-Octadecanoyl-glycine | 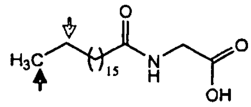 |
| 1887 | Nitroglycerin | 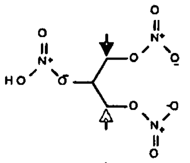 | 1900 | N-Octanoyl-glycine | 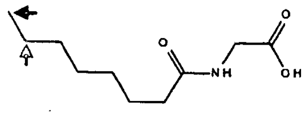 |
| 1888 | N-Methylanthranilate | 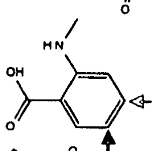 | 1901 | N-(3-Oxooctanoyl)homoserine lactone | 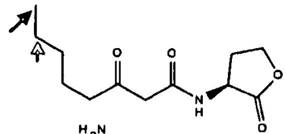 |
| 1889 | D-Glucose | 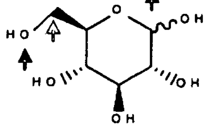 | 1902 | 2,4-Diamino-6-nitrotoluene | 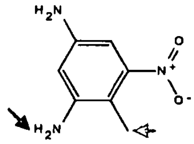 |
| 1890 | N-Malonylanthranilate | 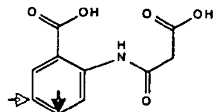 | 1903 | Nonadecane | 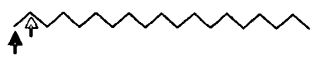 |
| 1891 | N-Methylaniline | 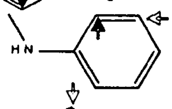 | 1904 | Nonadecanoic acid | 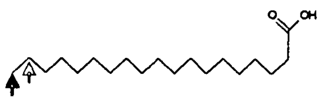 |
| 1892 | N-Methylindole | 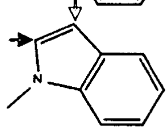 | 1905 | Nonanoyldolichol | 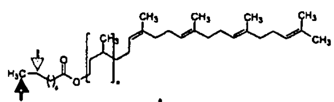 |
| 1893 | N-Methylhistamine | 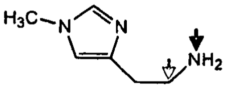 | 1906 | 4-Acetamido-2-amino-6-nitrotoluene | 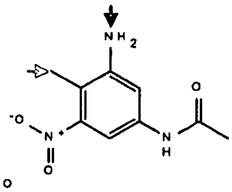 |
| 1894 | N-Methyl-L-glutamate | 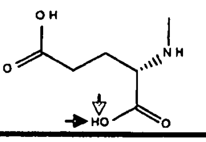 | 1907 | Methyl nonylate | 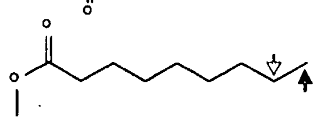 |

| N° | Substrate | Chemical Structure | N° | Substrate | Chemical Structure |
|---|---|---|---|---|---|
| 1908 | Nonanal | 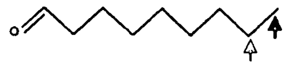 | 1921 | N-Succinyl-Ala-Ala-Pro-Phe p-nitroanilide | Too big |
| 1909 | Nonane | 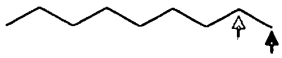 | 1922 | N-Heptanoylhomoserine lactone | 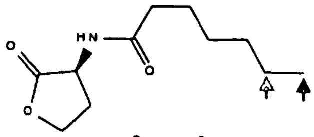 |
| 1910 | 2-Hydroxy-8-methylchromene-2-carboxylate | 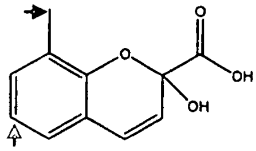 | 1923 | Methyloxaloacetate | 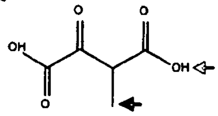 |
| 1911 | Nonanoate | 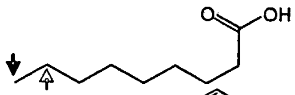 | 1924 | O-Acetylcholine | 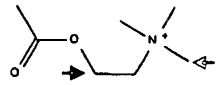 |
| 1912 | Nonylphenol | 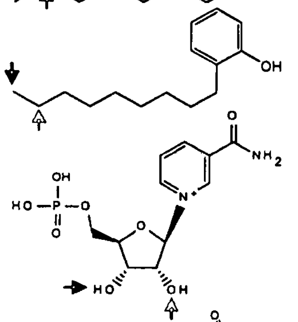 | 1925 | Oxaloacetate | 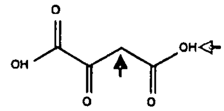 |
| 1913 | Nicotinamide mononucleotide | | 1926 | O-Acetyl-L-homoserine | 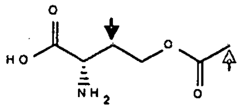 |
| 1914 | Oleoylglycerone phosphate | 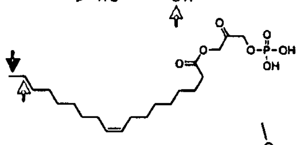 | 1927 | 2-Oxohex-trans-4-enoate | 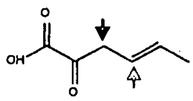 |
| 1915 | Oleic acid methyl ester | 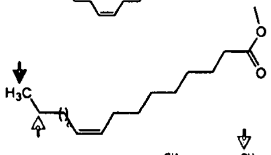 | 1928 | 1-Octanal | 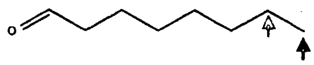 |
| 1916 | all-trans-Nonaprenyl diphosphate | 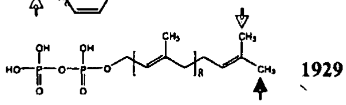 | 1929 | Octadecanoic acid | 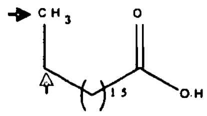 |
| 1917 | Phenanthrene-3,4-oxide | 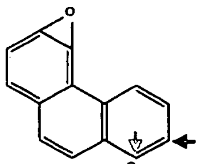 | 1930 | Octadecane | 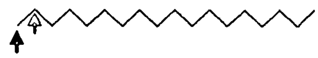 |
| 1918 | 3-Amino-2-oxopropyl phosphate | 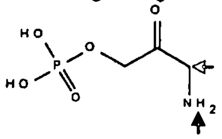 | 1931 | Octadecenoic acid | 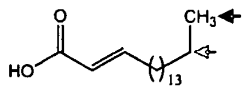 |
| 1919 | N-Succinyl-Ala-Ala-Ala p-nitroanilide | Too big | 1932 | 9-Octadecynoic acid | 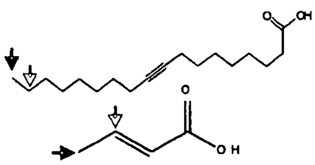 |
| 1920 | N-Succinyl-Ala-Ala-Phe-7-amido-4-methyl coumarin | Too big | 1933 | 2-Butenoic acid | |

| N° | Substrate | Chemical Structure | N° | Substrate | Chemical Structure |
|---|---|---|---|---|---|
| 1934 | o-Cresol | 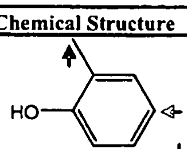 | 1947 | 2-Ethylphenol | 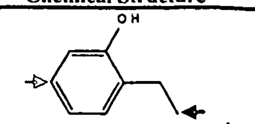 |
| 1935 | Octane | 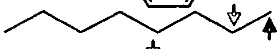 | 1948 | 1-Octene | 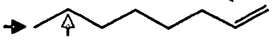 |
| 1936 | Octane-1,8-diol | 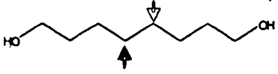 | 1949 | 1,2-Dihydroxy-7-hydroxymethylnaphthalene | 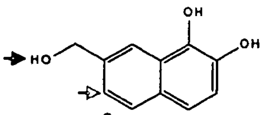 |
| 1937 | Stearoyl-CoA | 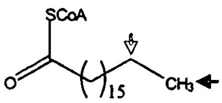 | 1950 | 2-Oxo-3-hydroxy-4-phosphobutanoate | 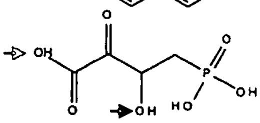 |
| 1938 | Ethyl octanoate | 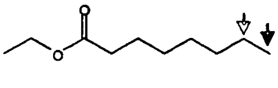 | 1951 | Oleyldihydroxyacetone phosphate | 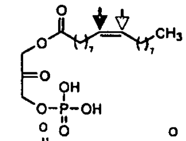 |
| 1939 | N1-(5-Phospho-D-ribosyl)glycinamide | 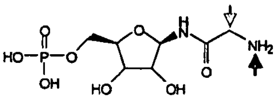 | 1952 | 3-(5-oxo-4,5-dihydro-3H-imidazol-4-yl)propanoate | 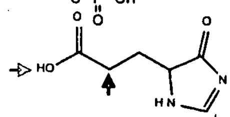 |
| 1940 | L-Octanoylcarnitine | 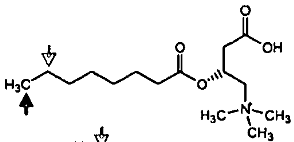 | 1953 | O-Demethylpuromycin | 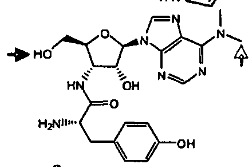 |
| 1941 | GDP | 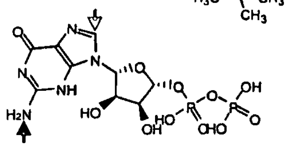 | 1954 | O-Feruloylquinate | 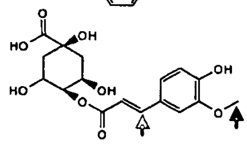 |
| 1942 | Octadecanoyldolichol | 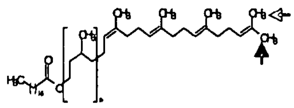 | 1955 | Octyl salicylate | 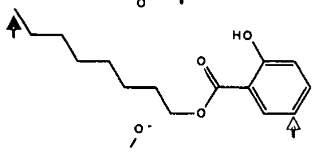 |
| 1943 | dAMP | 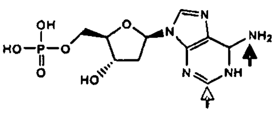 | 1956 | 4-Hydroxylamino-2,6-dinitrotoluene | 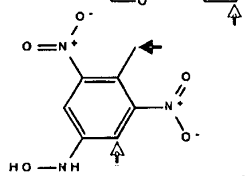 |
| 1944 | Octanoyldolichol | 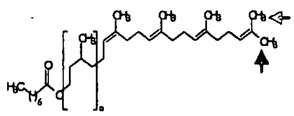 | 1957 | Oleyl alcohol | 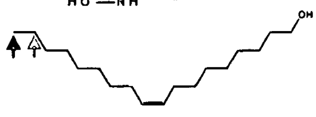 |
| 1945 | all-trans-Octaprenyl diphosphate | 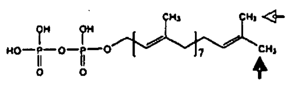 | 1958 | Oleic acid | 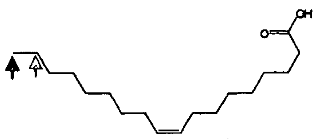 |
| 1946 | cis-1,2-Dihydroxy-1,2-dihydro-7-hydroxymethylnaphthalene | 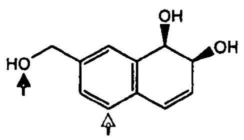 | 1959 | 3-Methyl-2-oxobutanoate | 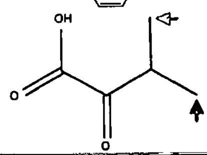 |

| N° | Substrate | Chemical Structure | N° | Substrate | Chemical Structure |
|---|---|---|---|---|---|
| 1960 | 2-Hydroxylamino-4,6-dinitrotoluene | 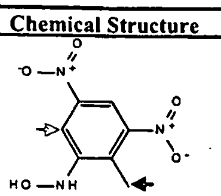 | 1973 | Oxalic acid | 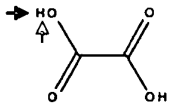 |
| 1961 | 2-Oxopent-4-enoate | 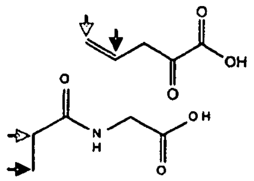 | 1974 | 4-Oxobutanoate | 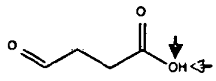 |
| 1962 | N-Propionylglycine | | 1975 | Oxamate | 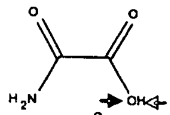 |
| 1963 | alpha-N-Phenylacetyl-L-glutamine | 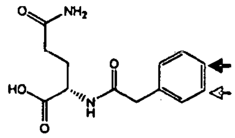 | 1976 | Oxazole | 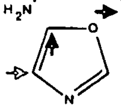 |
| 1964 | Pantetheine 4'-phosphate | 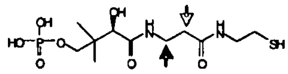 | 1977 | Doxepin | 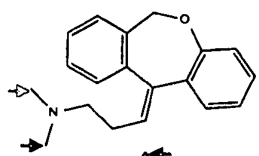 |
| 1965 | L-Ornithine | 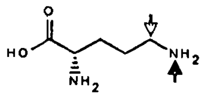 | 1978 | Oxirane | 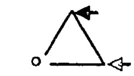 |
| 1966 | (9Z,12Z)-Octadecadienoyl-CoA | 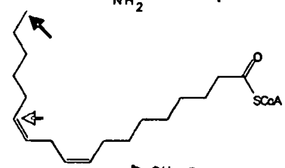 | 1979 | 3-Oxopropionyl-CoA | 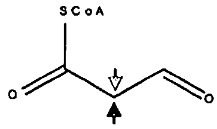 |
| 1967 | Orotidine 5'-phosphate | 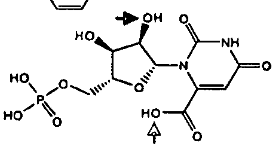 | 1980 | Oxomalonate | 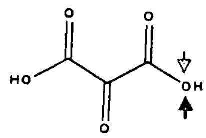 |
| 1968 | O-Sinapoylglucarolactone | 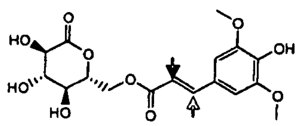 | 1981 | 2-Oxoadipate | 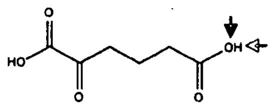 |
| 1969 | Oxalureate | 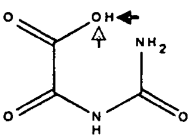 | 1982 | Phenylacetaldehyde | 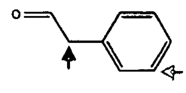 |
| 1970 | Oxaloglutarate | 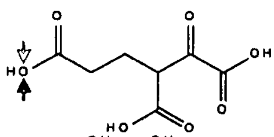 | 1983 | 4-Hydroxyphenylacetyl-CoA | 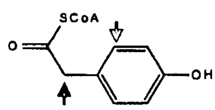 |
| 1971 | Oxaloglycolate | 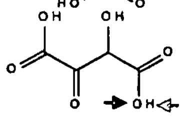 | 1984 | Phosphatidate | 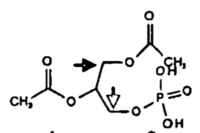 |
| 1972 | Oxalosuccinate | 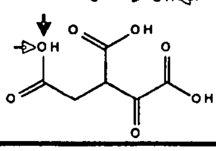 | 1985 | Orotate | 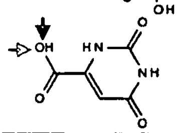 |

| N° | Substrate | Chemical Structure | N° | Substrate | Chemical Structure |
|---|---|---|---|---|---|
| 1986 | Panose | 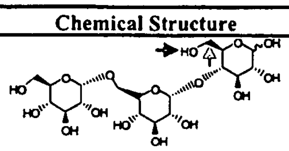 | 1999 | gamma-Phenyl-epsilon-caprolactam | 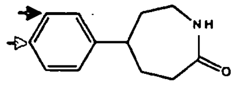 |
| 1987 | (R)-Pantolactone | 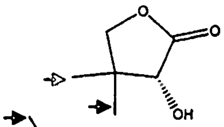 | 2000 | Pelargonic acid | 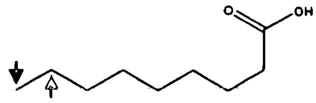 |
| 1988 | (R)-Pantothenate | 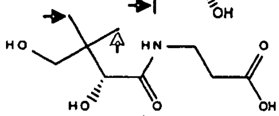 | 2001 | Palmitoyldihydroxyacetone phosphate | 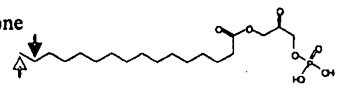 |
| 1989 | (R)-Pantoate | 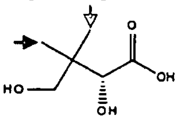 | 2002 | Phosphatidyl-N-dimethylethanolamine | 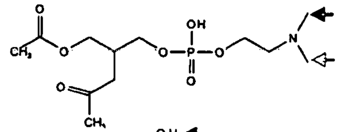 |
| 1990 | Adenosine 2',5'-bisphosphate | 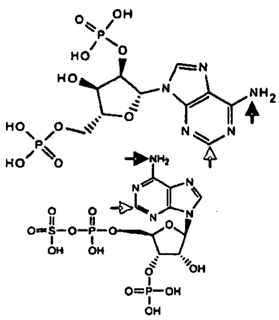 | 2003 | Pyridoxine 5'-phosphate | 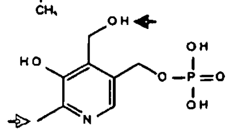 |
| 1991 | 3'-Phosphoadenylyl sulfate | | 2004 | Phenethylamine | 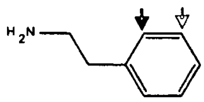 |
| 1992 | Parathion | 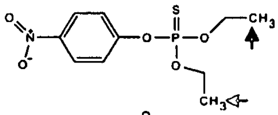 | 2005 | Pectin | Too big |
| 1993 | 3-sn-Phosphatidylcholine | 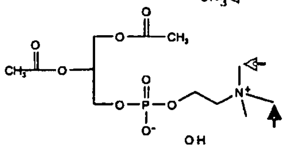 | 2006 | Pentadecanoic acid | 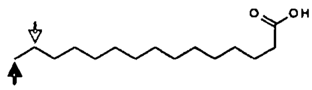 |
| 1994 | Choline phosphate | 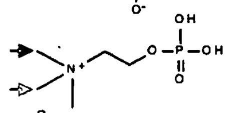 | 2007 | p-Cymene | 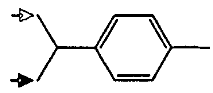 |
| 1995 | p-Cumate | 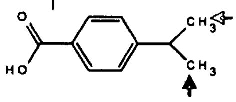 | 2008 | Pentyl butyrate | 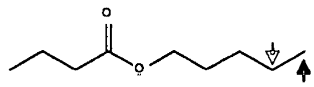 |
| 1996 | p-Coumaryl alcohol | 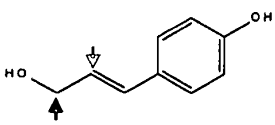 | 2009 | Phosphatidylethanolamine | 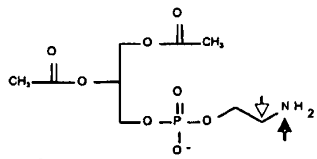 |
| 1997 | Pentachlorophenol | 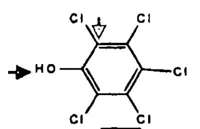 | 2010 | Methyl pentadecanoate | 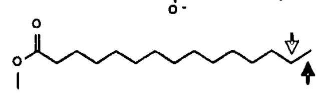 |
| 1998 | p-Cresol | 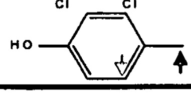 | 2011 | Benzaldehyde | 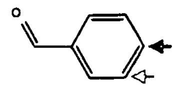 |

| N° | Substrate | Chemical Structure | N° | Substrate | Chemical Structure |
|---|---|---|---|---|---|
| 2012 | 2-Aminobenzoyl-CoA | 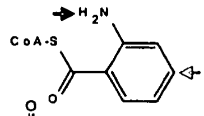 | 2025 | p-Formyltoluene | 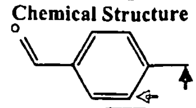 |
| 2013 | Pentyl pentanoate | 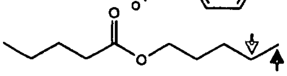 | 2026 | Phenol | 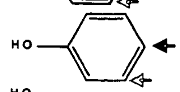 |
| 2014 | Pentanoic acid | 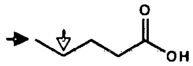 | 2027 | Phenylboronic acid | 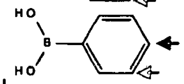 |
| 2015 | Phosphoenolpyruvate | 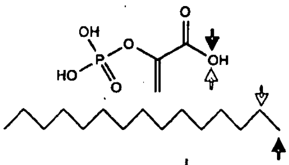 | 2028 | Phosphatidylglycerophosphate | 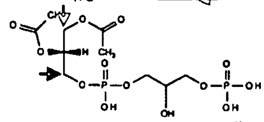 |
| 2016 | Pentadecane | | 2029 | dATP | 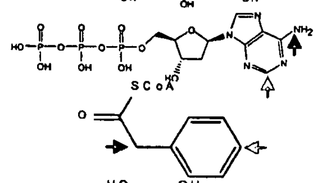 |
| 2017 | 5-Methylhexa-2,4-dienoyl-CoA | 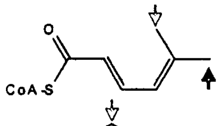 | 2030 | Phenylacetyl-CoA | |
| 2018 | cis-2-Oxohept-3-ene-1,7-dioate | 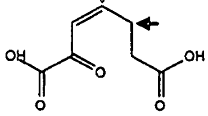 | 2031 | 2,4,6/3,5-Pentahydroxycyclohexanone | 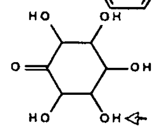 |
| 2019 | *n*-Pentane | 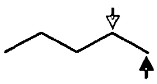 | 2032 | L-1-Pyrroline-3-hydroxy-5-carboxylate | 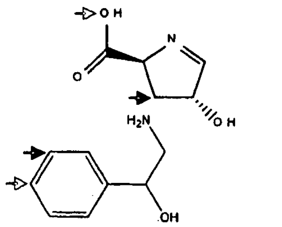 |
| 2020 | Perdeuterobenzene | 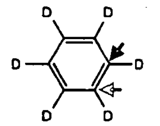 | 2033 | Phenylethanolamine | |
| 2021 | Perillyl aldehyde | 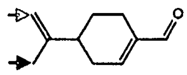 | 2034 | Phenethyl alcohol | 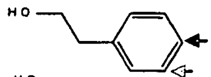 |
| 2022 | Pentacen | 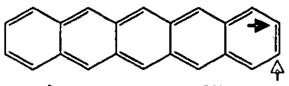 | 2035 | 1-Phenylethanol | 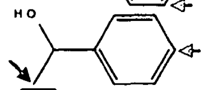 |
| 2023 | Perillyl alcohol | 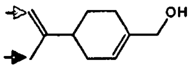 | 2036 | L-Phenylalanine | 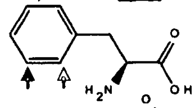 |
| 2024 | Ethanolamine phosphate | 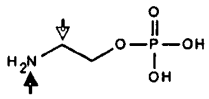 | 2037 | 2-Phenylglycine | 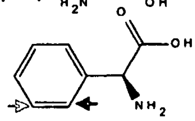 |

| N° | Substrate | Chemical Structure | N° | Substrate | Chemical Structure |
|---|---|---|---|---|---|
| 2038 | Quercetin | 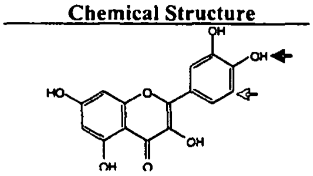 | 2052 | cis-3,4-Dihydroxy-3,4-dihydrophenanthrene | 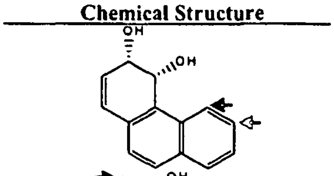 |
| 2039 | Phenanthrene | 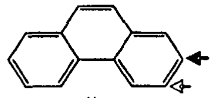 | 2053 | cis-1,2-Dihydroxy-1,2-dihydro-8-methylnaphthalene | 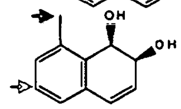 |
| 2040 | Phenazine | 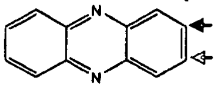 | 2054 | 1,2-Dihydroxy-8-methylnaphthale | 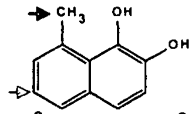 |
| 2041 | Phenetole | 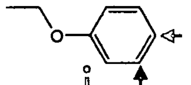 | 2055 | 2,4-Dihydroxy-hept-trans-2-ene-1,7-dioate | 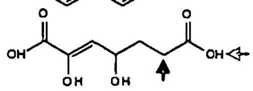 |
| 2042 | Phosphatidylglycerol | 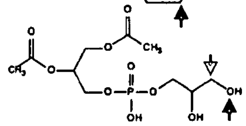 | 2056 | 1-Phosphatidyl-D-myo-inositol | 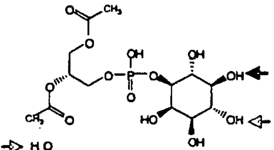 |
| 2043 | L-Phe-Gly-Gly | 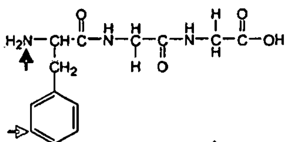 | 2057 | Pimelic acid | 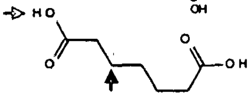 |
| 2044 | Phenothiazine | 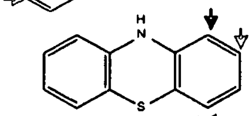 | 2058 | O-Phospho-4-hydroxy-L-threonine | 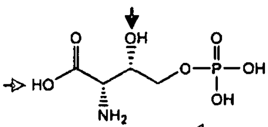 |
| 2045 | Phloroglucinol | 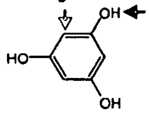 | 2059 | 1-Phosphatidyl-1D-myo-inositol 3-phosphate | 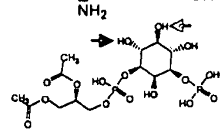 |
| 2046 | O-Phospho-L-homoserine | 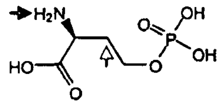 | 2060 | 1-Phosphatidyl-1D-myo-inositol 4-phosphate | 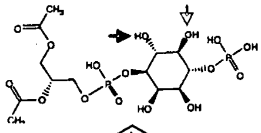 |
| 2047 | Phosphatidylglycerol | 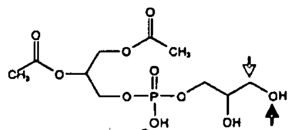 | 2061 | Piperideine | 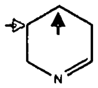 |
| 2048 | Phosphatidylinositol | 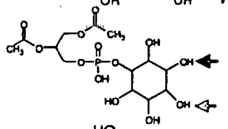 | 2062 | Piperazine | 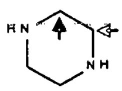 |
| 2049 | 2-Hydroxyphenylacetate | 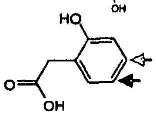 | 2063 | Piperidine | 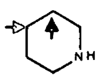 |
| 2050 | 3-Hydroxyphenylacetate | 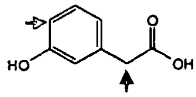 | 2064 | 2,6-Diamino-4-nitrotoluene | 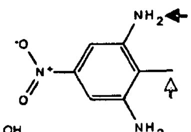 |
| 2051 | Phenylpyruvate | 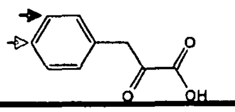 | 2065 | (R)-5-Phosphomevalonate | 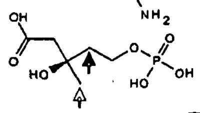 |

| N° | Substrate | Chemical Structure | N° | Substrate | Chemical Structure |
|---|---|---|---|---|---|
| 2066 | 4-Amino-2-hydroxylamino-6-nitrotoluene | 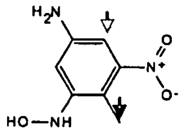 | 2079 | p-Nitrophenyl myristate | 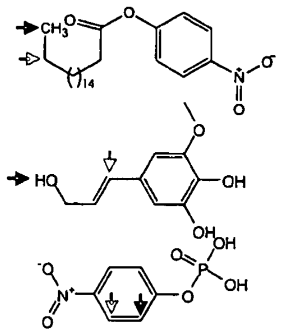 |
| 2067 | Pimeloyl-CoA | 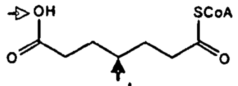 | 2080 | 5-Hydroxyconiferyl alcohol | |
| 2068 | 2,4-Dihydroxylamino-6-nitrotoluene | 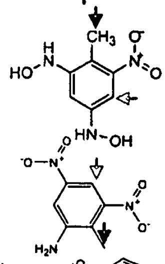 | 2081 | p-Nitrophenyl phosphate | |
| 2069 | 2-Amino-4,6-dinitrotoluene | | 2082 | p-Nitrophenyl alpha-L-rhamnoside | 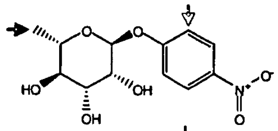 |
| 2070 | p-Nitrophenyl alpha-D-arabinofuranoside | 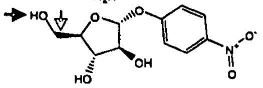 | 2083 | p-Nitrophenyl alpha-L-rhamnopyranoside | 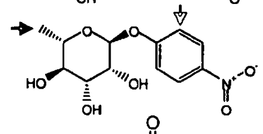 |
| 2071 | 2-Phenyl-1,3-propanediol monocarbamate | 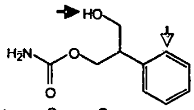 | 2084 | Propionate | 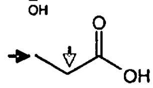 |
| 2072 | p-Nitrophenyl galactoside | 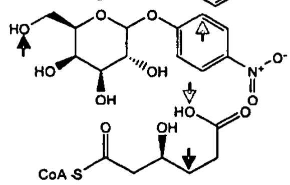 | 2085 | Propanoyl phosphate | 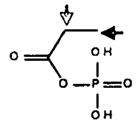 |
| 2073 | (3S)-3-Hydroxyadipyl-CoA | | 2086 | Phosphoramidate | 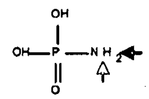 |
| 2074 | beta-D-Glucosyl-2-coumarinate | 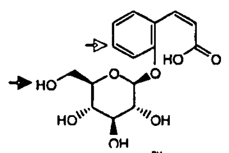 | 2087 | 4-Hydroxystyrene | 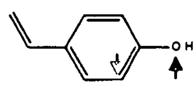 |
| 2075 | p-Nitrophenyl cellobioside | 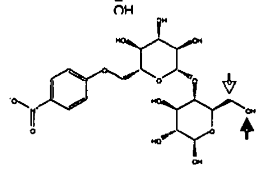 | 2088 | 3,5,7,3',4'-Pentahydroxyflavone | 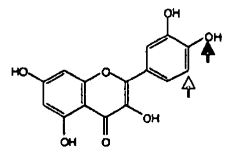 |
| 2076 | p-Nitrophenyl glucopyranoside | 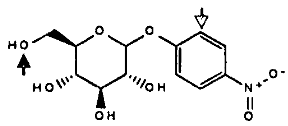 | 2089 | Propanoyl-CoA | 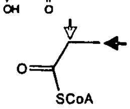 |
| 2077 | 4-Fluorocatechol | 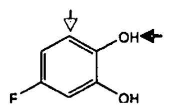 | 2090 | Prephenate | 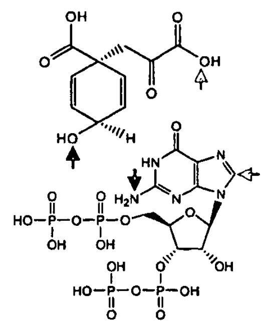 |
| 2078 | Propenoyl-CoA | 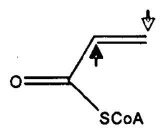 | 2091 | Guanosine 3'-diphosphate 5'-diphosphate | |

| N° | Substrate | Chemical Structure | N° | Substrate | Chemical Structure |
|---|---|---|---|---|---|
| 2092 | Phosphatidylinositol-4,5-bisphosphate | 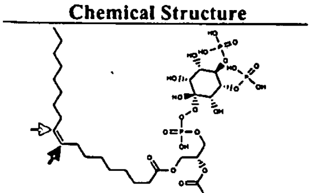 | 2105 | 1-(5-Phosphoribosyl)-5-[(5-phosphoribosylamino)methylideneamino]imidazole-4-carboxamide | 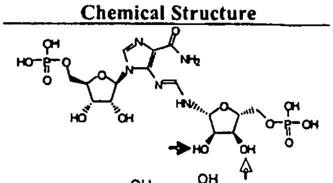 |
| 2093 | (R)-Diphosphomevalonate | 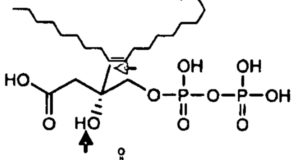 | 2106 | Quinate | 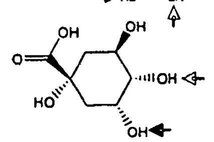 |
| 2094 | Pseudouridine | 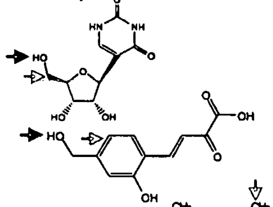 | 2107 | L-Proline | 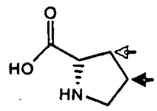 |
| 2095 | 2-Hydroxy-4-hydroxymethylbenzalpyruvate | | 2108 | 1,4-Dipropoxybenzene | 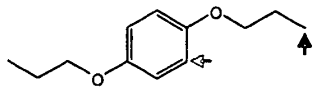 |
| 2096 | all-trans-pentaprenyl diphosphate | 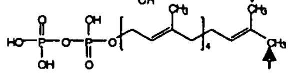 | 2109 | Propionyldolichol | 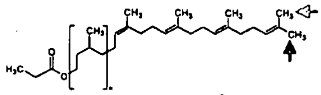 |
| 2097 | 5'-Phosphoribosyl-N-formylglycinamidine | 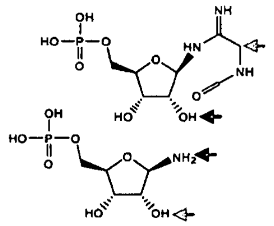 | 2110 | 3-Phenyl-2-propen-1-ol | 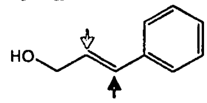 |
| 2098 | 5-Phospho-beta-D-ribosylamine | | 2111 | L-Prolyl-AMP | 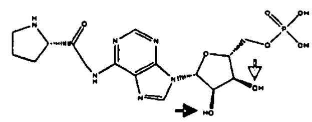 |
| 2099 | N-(5-Phospho-D-ribosyl)anthranilate | 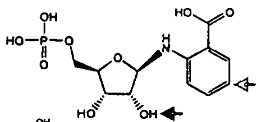 | 2112 | Propanoic acid | 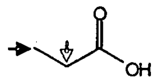 |
| 2100 | 1-(5-Phosphoribosyl)-AMP | 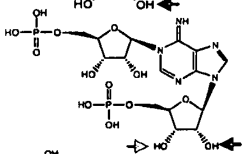 | 2113 | Propyl paraben | 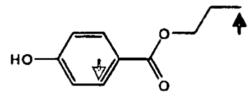 |
| 2101 | 1-(5-Phosphoribosyl)-ATP | 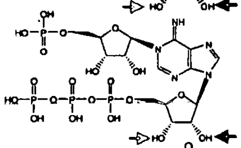 | 2114 | Pentanoyl-CoA | 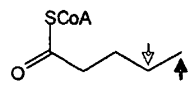 |
| 2102 | 1-(5'-Phosphoribosyl)-5-formamido-4-imidazolecarboxamide | 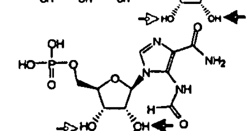 | 2115 | Propenoyl-CoA | 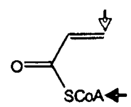 |
| 2103 | 1-(5-Phosphoribosyl)-5-[(5-phosphoribosylamino)methylideneamino]imidazole-4-carboxamide | 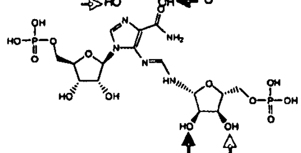 | 2116 | Protein substrate denatured 5 kDa | Not available |
| 2104 | 5-(5-Phospho-D-ribosylaminoformimino)-1-(5-phosphoribosyl)-imidazole-4-carboxamide | 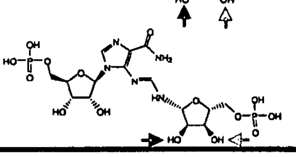 | 2117 | Protein substrate denatured 10 kDa | Not available |

| N° | Substrate | Chemical Structure | N° | Substrate | Chemical Structure |
|---|---|---|---|---|---|
| 2118 | Protein substrate denatured 15 kDa | Not available | 2131 | 1-Phosphatidyl-D-myo-inositol 4,5-bisphosphate | 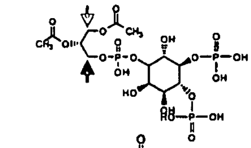 |
| 2119 | Protein substrate denatured 20 kDa | Not available | 2132 | Phosphatidyl-N-methylethanolamine | 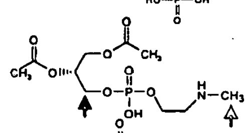 |
| 2120 | Protein substrate denatured 30 kDa | Not available | 2133 | 1-Phosphatidyl-D-myo-inositol | 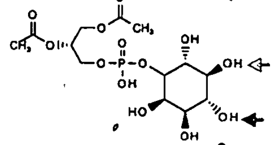 |
| 2121 | Protein substrate denatured 40 kDa | Not available | 2134 | 1-(5-Phospho-D-ribosyl)-5-amino-4-imidazolecarboxylate | 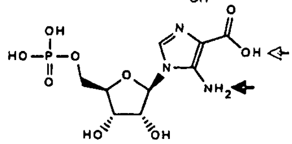 |
| 2122 | Protein substrate denatured 60 kDa | Not available | 2135 | Pteridine | 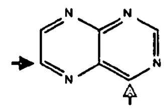 |
| 2123 | trans-O-Hydroxybenzylidenepyruvate | 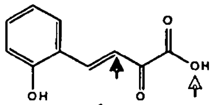 | 2136 | Putrescine | 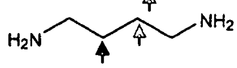 |
| 2124 | 5-Phospho-alpha-D-ribose 1-diphosphate | 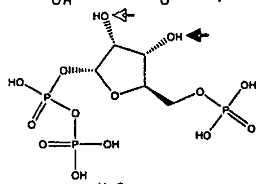 | 2137 | Pullulan | 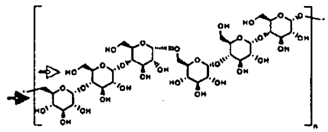 |
| 2125 | Pseudocumene | 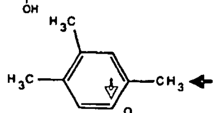 | 2138 | Purine | 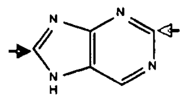 |
| 2126 | Pseudouridine 5'-phosphate | 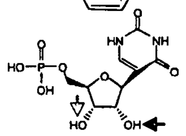 | 2139 | Puromycin | 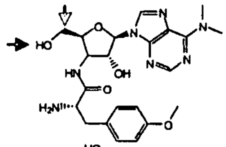 |
| 2127 | Phosphatidylserine | 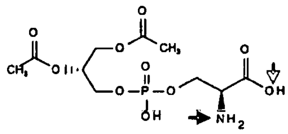 | 2140 | Quercitrin | 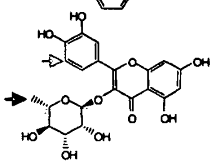 |
| 2128 | O-Phospho-L-serine | 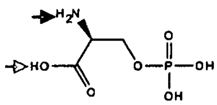 | 2141 | Pyrano[3,4-b]pyrrole | 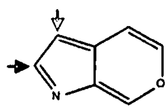 |
| 2129 | Heptylparaben | 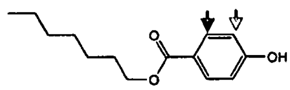 | 2142 | Pyridoxal | 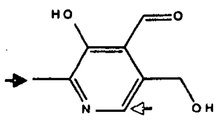 |
| 2130 | 1-Phosphatidyl-1D-myo-inositol 3,4-bisphosphate | 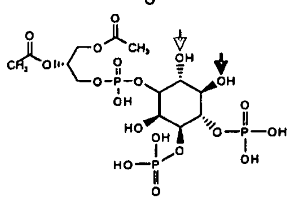 | 2143 | Pyrimidine | 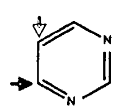 |

| N° | Substrate | Chemical Structure | N° | Substrate | Chemical Structure |
|---|---|---|---|---|---|
| 2144 | Pyridoxine | 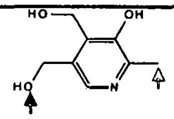 | 2157 | Pyrrolidine | 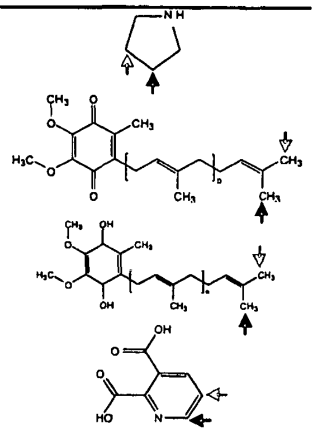 |
| 2145 | (R)-Phenyllactate | 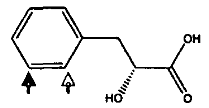 | 2158 | Ubiquinone | |
| 2146 | Pyruvate | 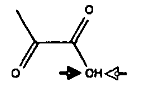 | 2159 | Ubiquinol | |
| 2147 | Pyrazine | 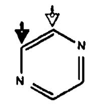 | 2160 | Pyridine-2,3-dicarboxylate | |
| 2148 | Pyrazolidine | 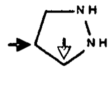 | 2161 | Pyridoxamine 5'-phosphate | 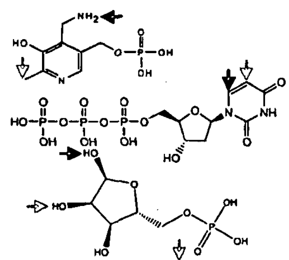 |
| 2149 | Pyrazole | 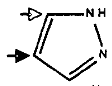 | 2162 | dUTP | |
| 2150 | Pyrazolo[1,5-a]pyrimidine | 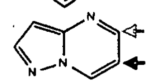 | 2163 | alpha-D-Ribose 5-phosphate | |
| 2151 | Pyrene | 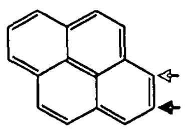 | 2164 | N-(5'-Phospho-D-1'-ribulosylformimino)-5-amino-1-(5"-phospho-D-ribosyl)-4-imidazolecarboxamide | 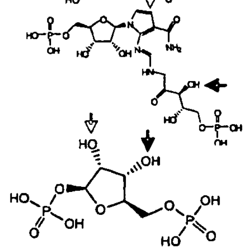 |
| 2152 | Pyridazine | 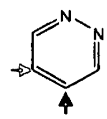 | 2165 | D-Ribose 1,5-bisphosphate | |
| 2153 | Pyridine | 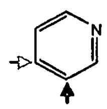 | 2166 | alpha-D-Ribose 1-phosphate | 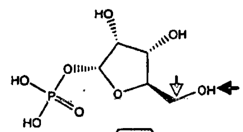 |
| 2154 | Pyridoxal 5'-phosphate | 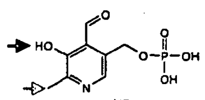 | 2167 | Quinone | 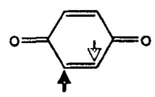 |
| 2155 | Pyrogallol | 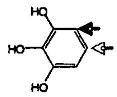 | 2168 | Quinoxaline | 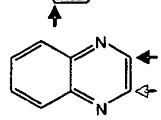 |
| 2156 | Pyrrole | 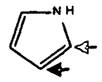 | 2169 | Quinuclidine | 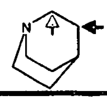 |

| N° | Substrate | Chemical Structure | N° | Substrate | Chemical Structure |
|---|---|---|---|---|---|
| 2170 | Quinolinate | 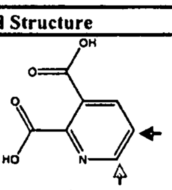 | 2184 | Quinaldate | 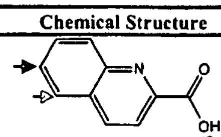 |
| 2171 | L-Rhamnono-1,4-lactone | 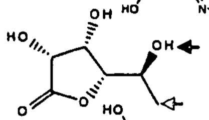 | 2185 | Folate | 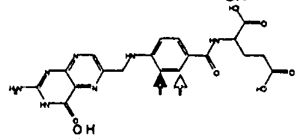 |
| 2172 | 2,5-Dihydroxybenzoate | 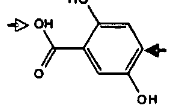 | 2186 | Dihydroresveratrol | 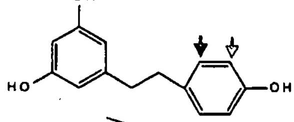 |
| 2173 | Quinoline-3,4-diol | 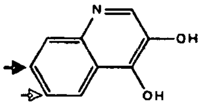 | 2187 | Retinol propionate | 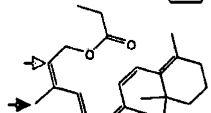 |
| 2174 | 2-Phenylbutanoic acid | 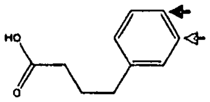 | 2188 | Retinoate | 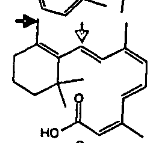 |
| 2175 | (R)-4-Hydroxymandelate | 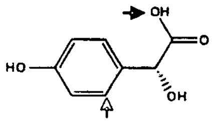 | 2189 | Retinol acetate | 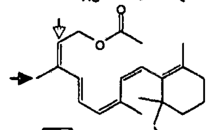 |
| 2176 | Pyridoxamine | 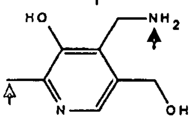 | 2190 | Retinol butyrate | 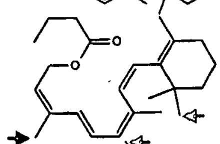 |
| 2177 | alpha-Ribazole 5'-phosphate | 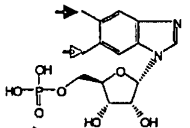 | 2191 | Retinal | 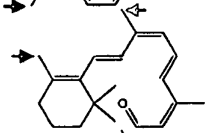 |
| 2178 | N1-(alpha-D-Ribosyl)-5,6-dimethylbenzimidazole | 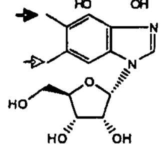 | 2192 | Retinol | 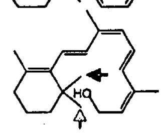 |
| 2179 | Resorufin acetate | 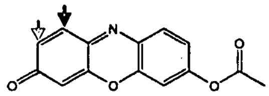 | 2193 | Retinol hexanoate | 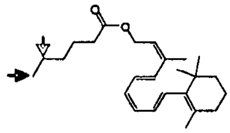 |
| 2180 | 4-(1-D-Ribitylamino)-5-amino-2,6-dihydroxypyrimidine | 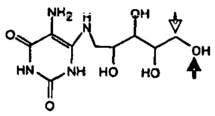 | 2194 | Retinol octanoate | 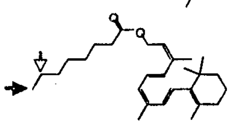 |
| 2181 | Raffinose | 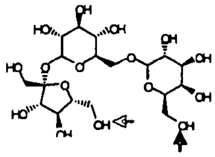 | 2195 | Resorufin-beta-D-galactopyranoside | 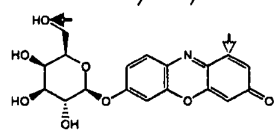 |
| 2182 | L-Ribulose | 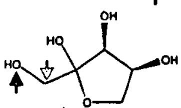 | 2196 | Resorufin-beta-D-glucopyranoside | 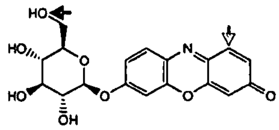 |
| 2183 | Resorcinol | 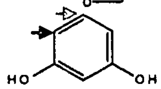 | 2197 | L-Rhamnonate | 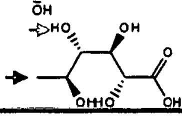 |

| N° | Substrate | Chemical Structure | N° | Substrate | Chemical Structure |
|---|---|---|---|---|---|
| 2198 | gamma-Glutamyl-gamma-aminobutyraldehyde | 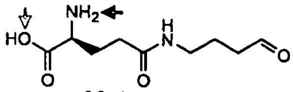 | 2212 | (1R,2S)-Naphthalene 1,2-oxide | 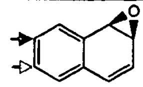 |
| 2199 | (R)-Hydroxybutanoyl-CoA | 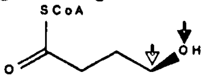 | 2213 | D-Ribulose 5-phosphate | 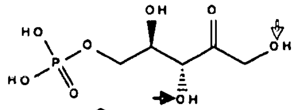 |
| 2200 | (R)-2-Hydroxystearate | 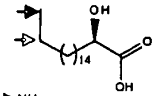 | 2214 | 2-Hydroxy-3-methyl benzal pyruvate | 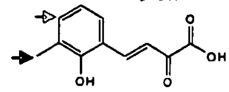 |
| 2201 | (S)-Ribosyl-L-homocysteine | 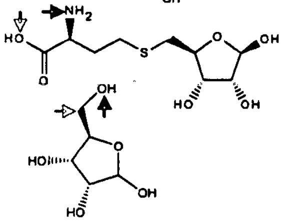 | 2215 | Sedoheptulose 1,7-bisphosphate | 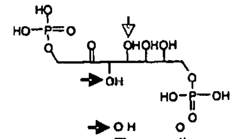 |
| 2202 | D-Ribose | | 2216 | L-Ribulose 5-phosphate | 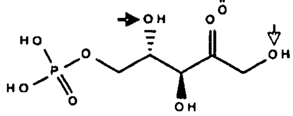 |
| 2203 | (R)-3-Hydroxybutanoate | 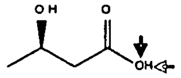 | 2217 | cis-1,2-Dihydroxy-1,2-dihydro-7-methylnaphthalene | 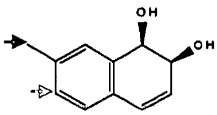 |
| 2204 | Riboflavin | 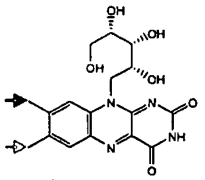 | 2218 | (S)-2,3-Epoxysqualene | 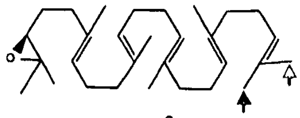 |
| 2205 | Ribitol | 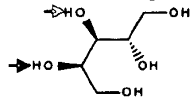 | 2219 | (S)-(+)-2-Phenyl-butyrate | 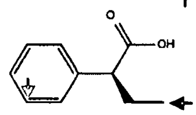 |
| 2206 | L-Rhamnulose | 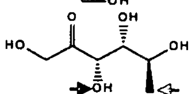 | 2220 | (S)-3-Hydroxyhexanoyl-CoA | 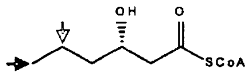 |
| 2207 | L-Rhamnulose 1-phosphate | 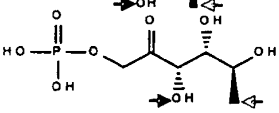 | 2221 | (S)-3-Hydroxyoctanoyl-CoA | 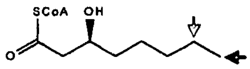 |
| 2208 | L-Rhamnofuranose | 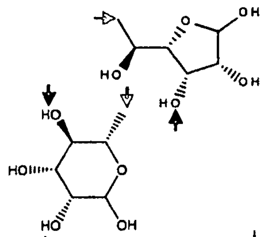 | 2222 | 1-(5'-Phosphoribosyl)-5-amino-4-(N-succinocarboxamide)-imidazole | 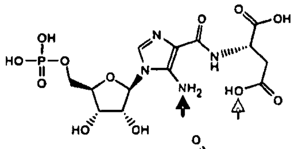 |
| 2209 | L-Rhamnose | | 2223 | 2-Carboxybenzaldehyde | 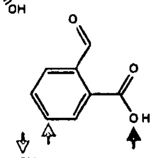 |
| 2210 | Resorufin butyrate | 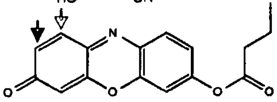 | 2224 | (+)-*cis*-Sabinol | 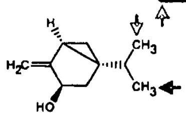 |
| 2211 | R-S-Alanylglycine | 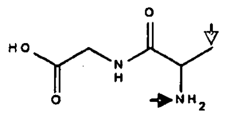 | 2225 | (+)-Sabinone | 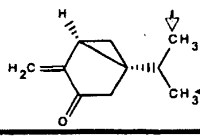 |

| N° | Substrate | Chemical Structure | N° | Substrate | Chemical Structure |
|---|---|---|---|---|---|
| 2226 | Sedoheptulose 7-phosphate | 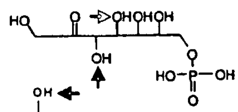 | 2239 | L-Sorbitol | 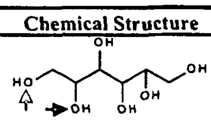 |
| 2227 | 3-Chloro-2-hydroxymuconic semialdehyde | 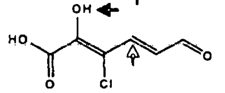 | 2240 | sec-Butylbenzene | 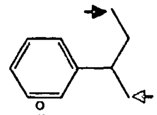 |
| 2228 | L-Saccharopine | 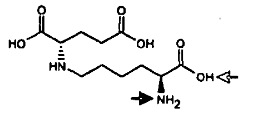 | 2241 | O-Succinylbenzoyl-CoA | 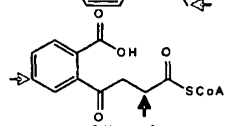 |
| 2229 | (S)-4-Hydroxymandelate | 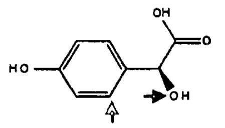 | 2242 | Scytalone | 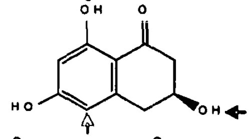 |
| 2230 | Salicin 6-phosphate | 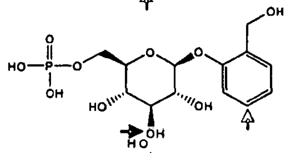 | 2243 | N-Succinyl-L-2,6-diaminopimelate | 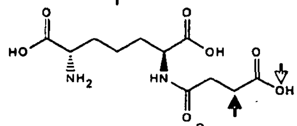 |
| 2231 | Salicylic acid | 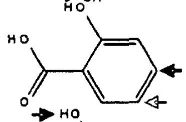 | 2244 | 2-Hydroxy-4-hydroxymethylbenzalpyruvate | 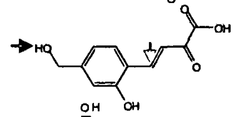 |
| 2232 | Salicylaldehyde | 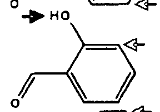 | 2245 | Sedoheptulose | 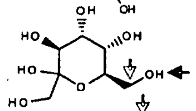 |
| 2233 | Salicin | 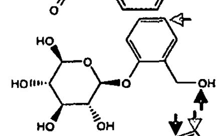 | 2246 | 2-Amino-5-oxocyclohex-1-enecarbonyl-CoA | 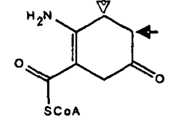 |
| 2234 | S-Adenosyl-(5')-3-methylthiopropylamine | 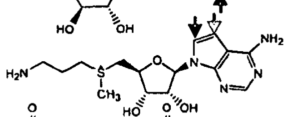 | 2247 | 1,4-Cyclohexanedione | 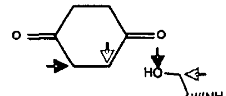 |
| 2235 | N-Succinyl-2-amino-6-oxopimelate | 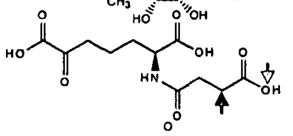 | 2248 | L-Seryl-AMP | 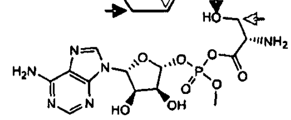 |
| 2236 | Sarcosine | 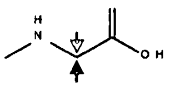 | 2249 | D-Serine | 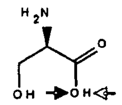 |
| 2237 | D-Sorbitol 6-phosphate | 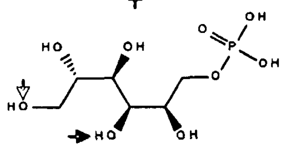 | 2250 | L-Serine | 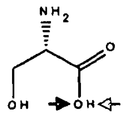 |
| 2238 | D-Sorbitol | 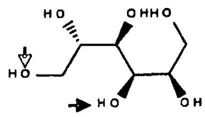 | 2251 | S-Formylglutathione | 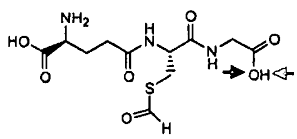 |

| N° | Substrate | Chemical Structure | N° | Substrate | Chemical Structure |
|---|---|---|---|---|---|
| 2252 | (S)-Hydroxybutanoyl-CoA | 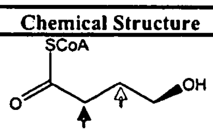 | 2266 | Shikimate | 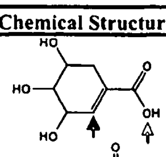 |
| 2253 | S-Succinyldihydrolipoamide | 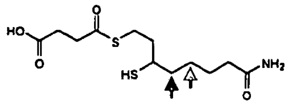 | 2267 | N-Succinyl-LL-2,6-diaminoheptanedioate | 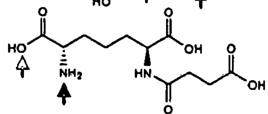 |
| 2254 | 4-Hydroxymethylsalicylaldehyde | 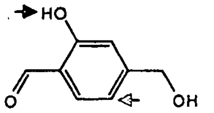 | 2268 | N-Succinyl-2-L-amino-6-oxoheptanedioate | 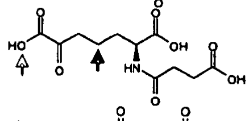 |
| 2255 | Sphinganine | 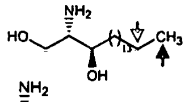 | 2269 | (S)-2-Methyl-3-oxopropanoyl-CoA | 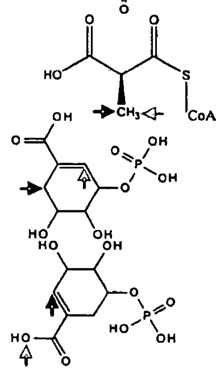 |
| 2256 | Sphingenine | 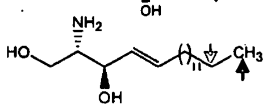 | 2270 | Shikimate 3-phosphate | |
| 2257 | S-(Hydroxymethyl)glutathione | 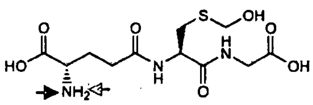 | 2271 | Shikimate 5-phosphate | |
| 2258 | (S)-2-Hydroxyoctadecanoate | 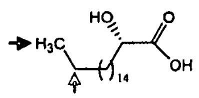 | 2272 | (S)-beta-Methylindolepyruvate | 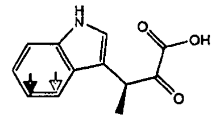 |
| 2259 | Styrene cis-glycol | 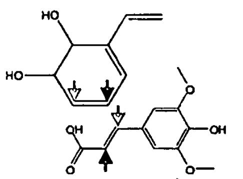 | 2273 | (S)-Methylthioglycolate | 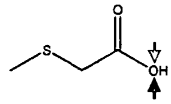 |
| 2260 | Sinapate | | 2274 | 2-Hydroxychromene-2-carboxylate | 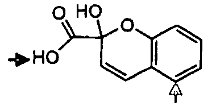 |
| 2261 | Sinapoyl-CoA | 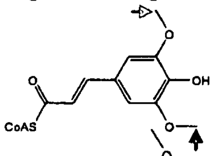 | 2275 | L-Sorbose | 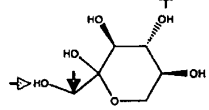 |
| 2262 | Sinapyl alcohol | 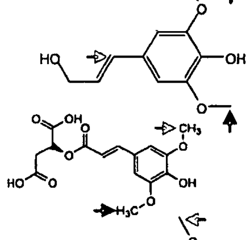 | 2276 | Sorbose 1-phosphate | 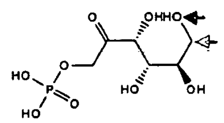 |
| 2263 | Sinapoyl-(S)-malate | | 2277 | Citronellate | 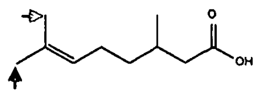 |
| 2264 | Sinapaldehyde | 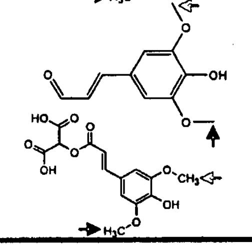 | 2278 | Sphinganine 1-phosphate | 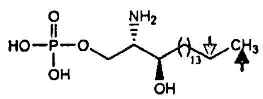 |
| 2265 | Sinapoyltartronate | | 2279 | Spermidine | 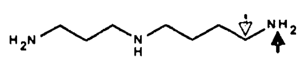 |

| N° | Substrate | Chemical Structure | N° | Substrate | Chemical Structure |
|---|---|---|---|---|---|
| 2280 | Spermine | 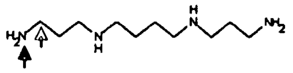 | 2294 | Sucrose 6-phosphate | 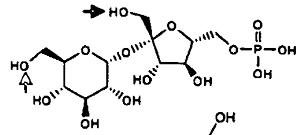 |
| 2281 | Squalene | 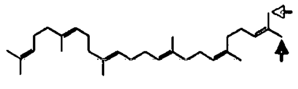 | 2295 | O-Succinylbenzoate-CoA | 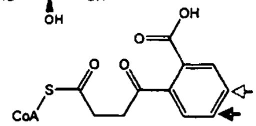 |
| 2282 | Sphinganine | 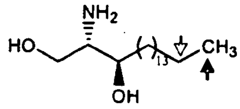 | 2296 | 2-Succinylbenzoate | 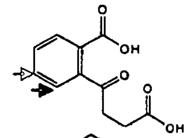 |
| 2283 | (1S,2R)-Naphthalene 1,2-oxide | 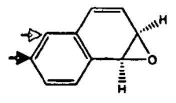 | 2297 | m-Tolualdehyde | 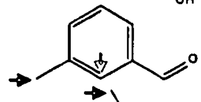 |
| 2284 | (S)-Squalene-2,3-epoxide | 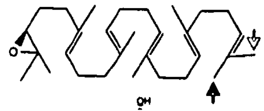 | 2298 | o-Tolualdehyde | 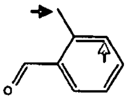 |
| 2285 | Stachyose | 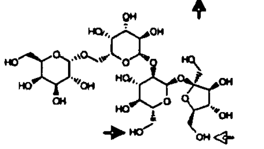 | 2299 | Hydroxycinnamoyl-CoA | 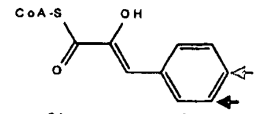 |
| 2286 | Starch | Too big | 2300 | 1,3,4,6-Tetrachloro-1,4-cyclohexadiene | 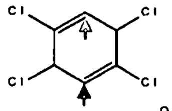 |
| 2287 | Stearyl heptanoate | 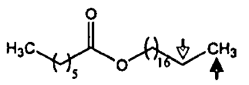 | 2301 | O-Succinyl-L-homoserine | 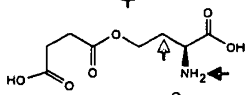 |
| 2288 | 2,5-Dichloroaniline | 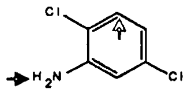 | 2302 | N2-Succinyl-L-ornithine | 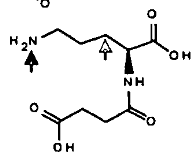 |
| 2289 | (1R,4R)-Dihydrocarvone | 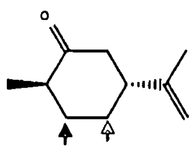 | 2303 | Sucrose | 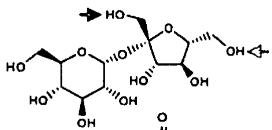 |
| 2290 | (1S,4S)-Dihydrocarvone | 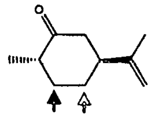 | 2304 | Succinic semialdehyde | 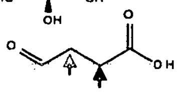 |
| 2291 | Succinate semialdehyde | 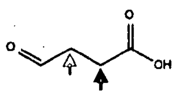 | 2305 | Syringaldehyde | 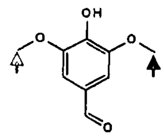 |
| 2292 | N2-Succinyl-L-arginine | 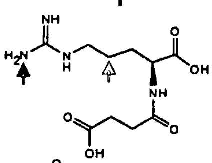 | 2306 | trans-2,3-epoxysuccinate | 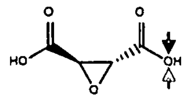 |
| 2293 | (1S,4R)-1-Hydroxy-2-oxolimonene | 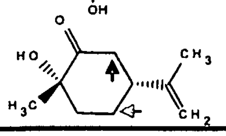 | 2307 | trans-Cinnamate | 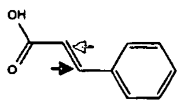 |

| N° | Substrate | Chemical Structure | N° | Substrate | Chemical Structure |
|---|---|---|---|---|---|
| 2308 | cis,cis-2-Hydroxy-6-oxohept-2,4-dienoate | 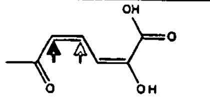 | 2321 | Taurine | 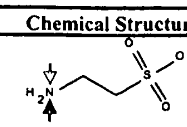 |
| 2309 | D-Tagatose | 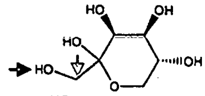 | 2322 | 2',6,3',4'-Tetrachlorobiphenyl | 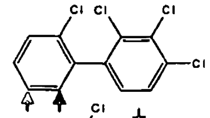 |
| 2310 | D-Tagatose 6-phosphate | 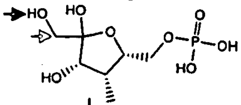 | 2323 | 2,4,4',6-Tetrachlorobiphenyl | 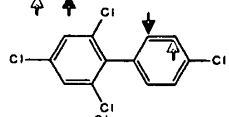 |
| 2311 | D-Tagatose 1,6-bisphosphate | 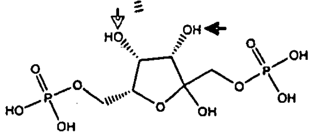 | 2324 | 2,2',5,5'-Tetrachlorobiphenyl | 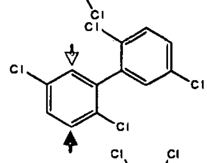 |
| 2312 | D-Tagaturonate | 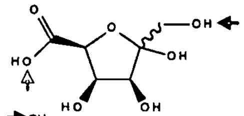 | 2325 | 2,3,4,4'-Tetrachlorobiphenyl | 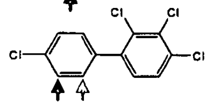 |
| 2313 | D-Talose | 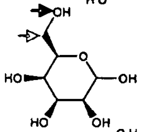 | 2326 | 2,3,4,5-Tetrachlorobiphenyl | 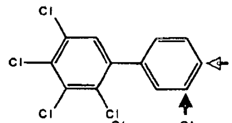 |
| 2314 | D-Tartrate | 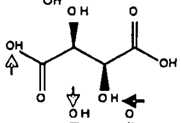 | 2327 | 2,3,4,4'-Tetrachlorobiphenyl | 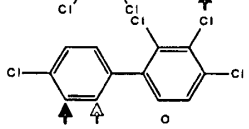 |
| 2315 | L-Tartaric acid | 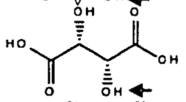 | 2328 | Succinate | 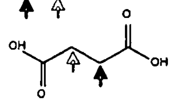 |
| 2316 | 2,3,4,5-Tetrachlorophenol | 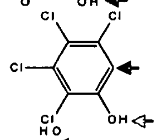 | 2329 | Tetrahydrothiophene | 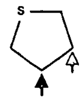 |
| 2317 | trans-1,2-Dihydrobenzene-1,2-diol | 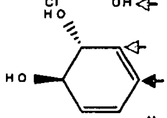 | 2330 | D-Threose | 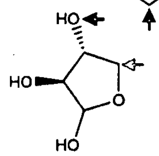 |
| 2318 | GMP | 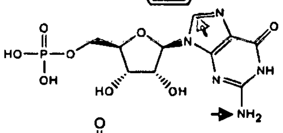 | 2331 | (2E)-Tetradecenoyl-CoA | 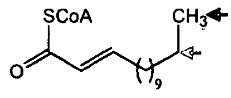 |
| 2319 | N-Succinyl-L-glutamate | 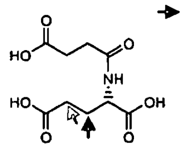 | 2332 | Toluene-cis-dihydrodiol | 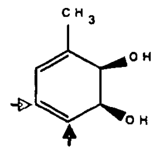 |
| 2320 | N-Succinyl-L-glutamate 5-semialdehyde | 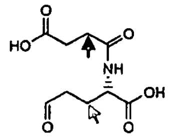 | 2333 | 2-Hydroxy-7-hydroxymethylchromene-2-carboxylate | 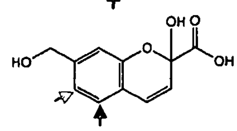 |

| N° | Substrate | Chemical Structure | N° | Substrate | Chemical Structure |
|---|---|---|---|---|---|
| 2334 | Tetracosane | 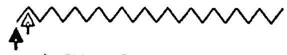 | 2347 | 1,3-Thiazole | 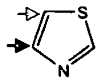 |
| 2335 | N-Tetradecanoyl-glycine | 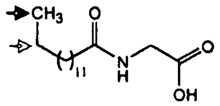 | 2348 | Thiomorpholine | 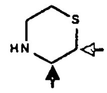 |
| 2336 | Tetradecanoyldolichol | 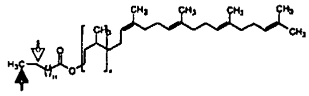 | 2349 | Thiophene | 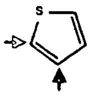 |
| 2337 | Succinyl-CoA | 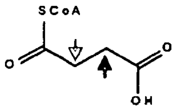 | 2350 | Thiamin monophosphate | 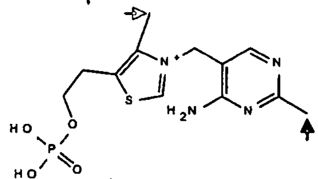 |
| 2338 | Tetrahydrofuran | 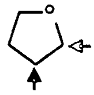 | 2351 | Thiamin | 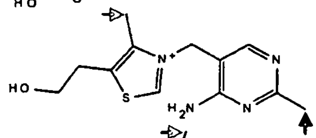 |
| 2339 | trans-Tetradec-2-enoyl-CoA | 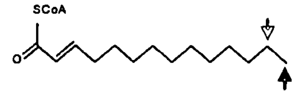 | 2352 | Thiamine diphosphate | 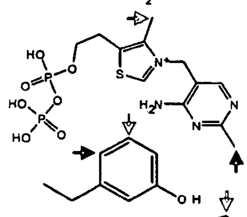 |
| 2340 | Tetradecanoyl-CoA | 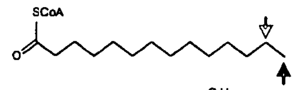 | 2353 | 3-Ethylphenol | |
| 2341 | 3',4',5,6-Tetrahydroxy-3,7-dimethoxyflavone | 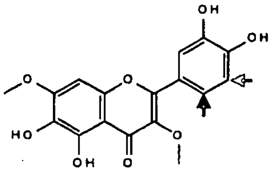 | 2354 | dUDP | 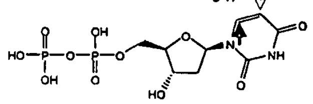 |
| 2342 | Deoxycytidine | 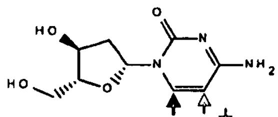 | 2355 | 1,3,6,8-Tetrahydroxynaphthalene | 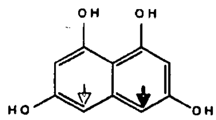 |
| 2343 | GTP | 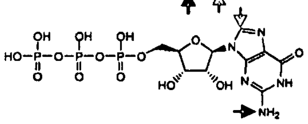 | 2356 | Guanine | 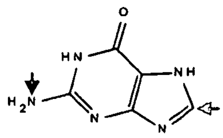 |
| 2344 | 5,6,7,8-Tetrahydrofolate | 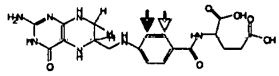 | 2357 | L-Threonine | 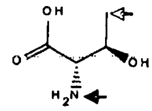 |
| 2345 | THF-L-glutamate | 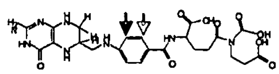 | 2358 | L-Threonate | 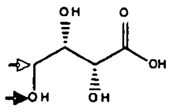 |
| 2346 | Thymidine | 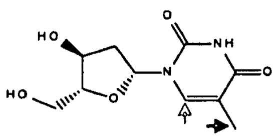 | 2359 | alpha-Tocopherol | 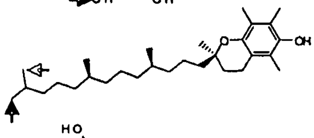 |
| | | | 2360 | 5,7,3',4'-Tetrahydroxyflavone | 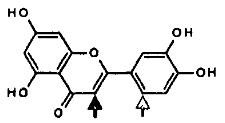 |

| N° | Substrate | Chemical Structure | N° | Substrate | Chemical Structure |
|---|---|---|---|---|---|
| 2361 | Thiepin | 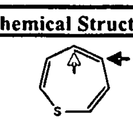 | 2374 | 7-Methyl-3-oxo-6-octenoyl-CoA | 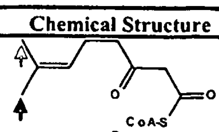 |
| 2362 | 3-Hydroxy-3-isohexeneylglutaryl-CoA | 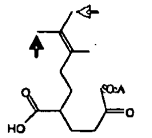 | 2375 | 1,2-Dibromohexane | 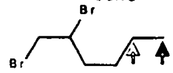 |
| 2363 | 2-Chlorohexane | 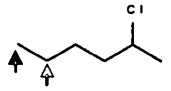 | 2376 | Oxidized thioredoxin | 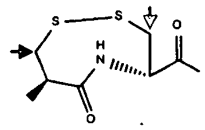 |
| 2364 | N,N,N-Trimethyl-L-histidine | 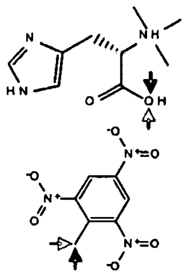 | 2377 | Reduced thioredoxin | 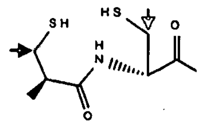 |
| 2365 | 2,4,6-Trinitrotoluene | | 2378 | Trehalose | 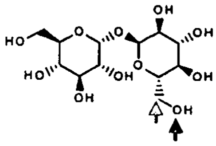 |
| 2366 | Thymine | 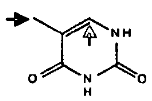 | 2379 | Trehalose 6-phosphate | 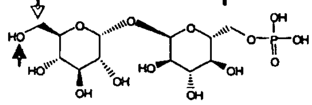 |
| 2367 | gamma-Tocopherol | 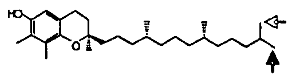 | 2380 | Benzoylformate | 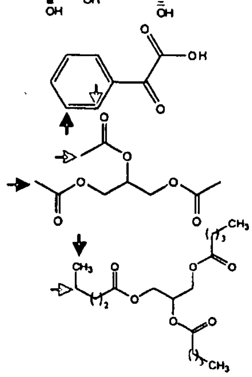 |
| 2368 | Toluene-4-sulfonate | 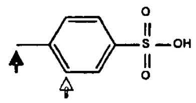 | 2381 | Triacetin | |
| 2369 | Toluene | 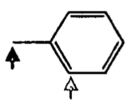 | 2382 | Glyceryl tributyrate | |
| 2370 | 4-Ethylphenol | 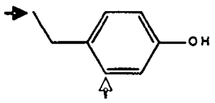 | 2383 | Isohexenyl-glutaconyl-CoA | 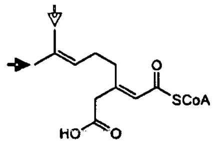 |
| 2371 | D-myo-Inositol 1,4,5-trisphosphate | 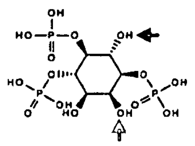 | 2384 | Citronellyl-CoA | 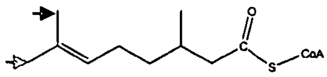 |
| 2372 | 1-Bromohexane | 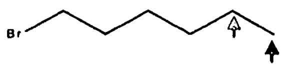 | 2385 | Glyceryl tridecanoate | 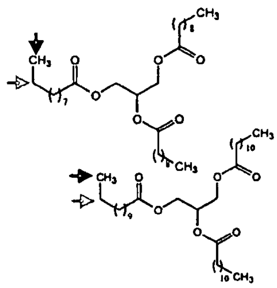 |
| 2373 | 1-Chlorohexane | 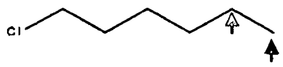 | 2386 | Glyceryl tridodecanoate | |

| N° | Substrate | Chemical Structure | N° | Substrate | Chemical Structure |
|---|---|---|---|---|---|
| 2387 | 3-Fluoro-cis,cis-muconate | 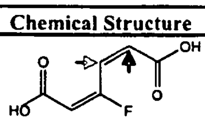 | 2400 | L-Tryptophan | 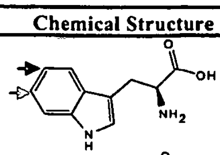 |
| 2388 | 3-Fluorocatechol | 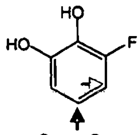 | 2401 | D-Tryptophan | 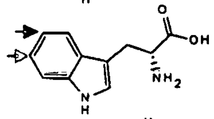 |
| 2389 | 4,5,6-Trinitrotriazine | 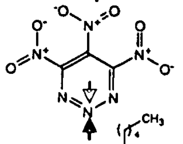 | 2402 | Tryptamine | 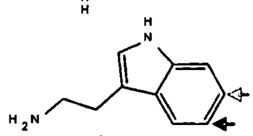 |
| 2390 | Glyceryl trihexanoate | 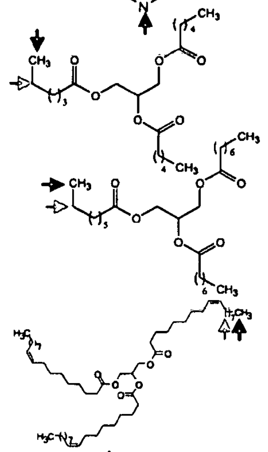 | 2403 | 1-Methyl-1-phenylethylene | 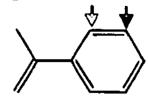 |
| 2391 | Glyceryl trioctanoate | | 2404 | Tetracosanoic acid | 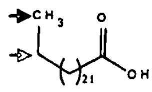 |
| 2392 | Glycerol trioleate | | 2405 | Tetracosanoyl-CoA | 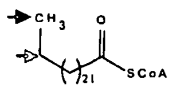 |
| 2393 | 1,4-Lactone | 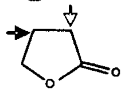 | 2406 | Tylactone | 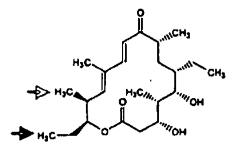 |
| 2394 | Glyceryl trihexadecanoate | 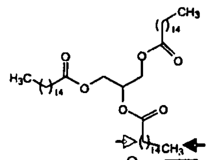 | 2407 | Tetradecane | 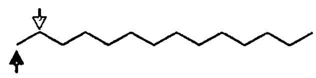 |
| 2395 | Glyceryl tripropionate | 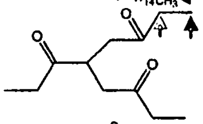 | 2408 | Methyl tetradecenoate | 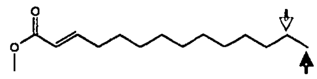 |
| 2396 | Tropate | 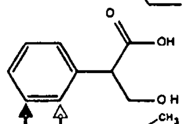 | 2409 | (9Z)-Tetradecenoic acid | 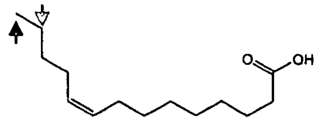 |
| 2397 | Glyceryl tritetradecanoate | 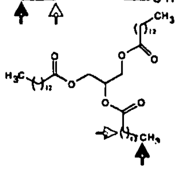 | 2410 | Tetradecanoate | 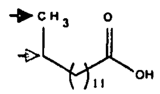 |
| 2398 | Tropine | 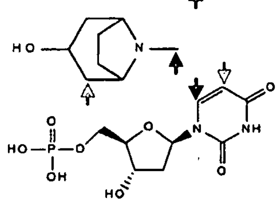 | 2411 | UDP-2,3-bis(3-hydroxytetradecanoyl)glucosamine | 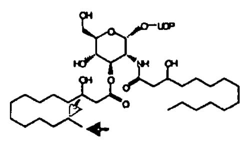 |
| 2399 | dUMP | | 2412 | L-Tyrosine | 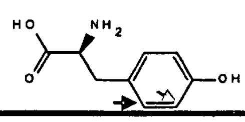 |

| N° | Substrate | Chemical Structure | N° | Substrate | Chemical Structure |
|---|---|---|---|---|---|
| 2413 | Tyramine | 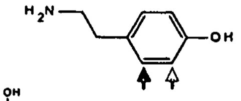 | 2426 | Uridine 5'-diphosphate | 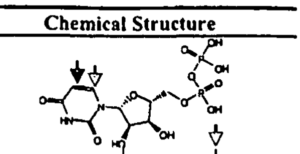 |
| 2414 | UDP-3-O-(3-hydroxytetradecanoyl)-N-acetylglucosa mine | 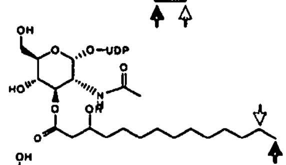 | 2427 | Undecaprenyl phosphate | 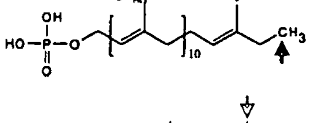 |
| 2415 | UDP-3-O-(3-hydroxytetradecanoyl)-D-glucosamine | 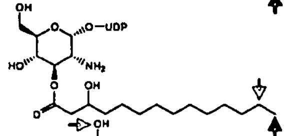 | 2428 | Undecaprenyl diphosphate | 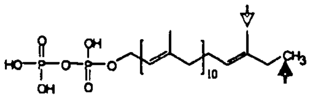 |
| 2416 | UDP-N-acetyl-2-amino-2-deoxy-D-glucuronate | 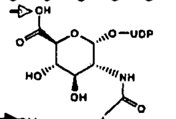 | 2429 | UDP-D-glucose | 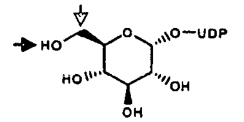 |
| 2417 | UDP-N-Acetyl-3-O-(1-carboxyvinyl)-D-glucosamine | 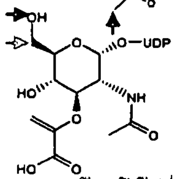 | 2430 | Methylitaconate | 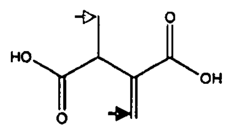 |
| 2418 | Undecaprenyl-diphospho-N-acetylmuramoyl-(N-acetylglucosamine)-L-alanyl-D-glutamyl-meso-2,6-diaminopimeloyl-D-alanyl-D-alanine | 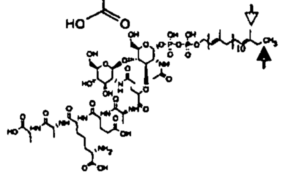 | 2431 | UDP-D-galactose | 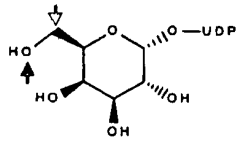 |
| 2419 | UDP-N-Acetyl-D-glucosamine | 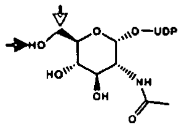 | 2432 | UDP-D-Galacto-1,4-furanose | 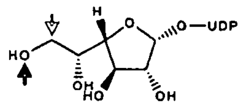 |
| 2420 | UDP-N-Acetyl-D-mannosamine | 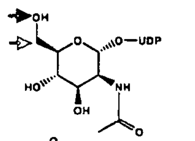 | 2433 | UDP-D-xylose | 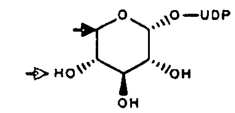 |
| 2421 | UDP-N-Acetyl-D-mannosaminouronate | 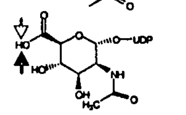 | 2434 | UDP-D-glucuronate | 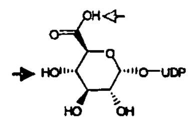 |
| 2422 | Undecaprenyl-diphospho-N-acetylmuramoyl-L-alanyl-D-glutamyl-meso-2,6-diaminopimeloyl-D-alanyl-D-alanine | 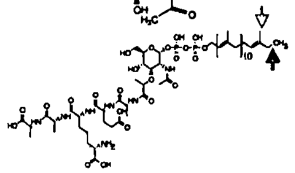 | 2435 | UDP-3-Ketoglucose | 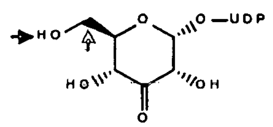 |
| 2423 | UDP-N-Acetylmuramoyl-L-alanine | 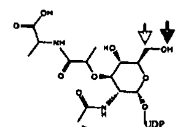 | 2436 | UDP-L-Rhamnose | 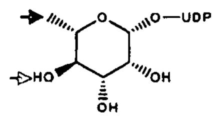 |
| 2424 | UDP-N-Acetylmuramoyl-L-alanyl-D-glutamate | 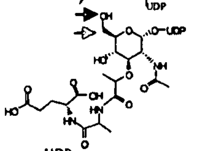 | 2437 | UDP-N-Acetyl-D-galactosamine | 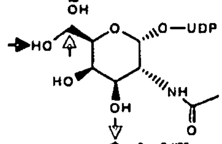 |
| 2425 | UDP-N-Acetylmuramate | 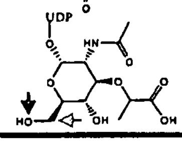 | 2438 | UDP-N-acetylmuramoyl-L-alanyl-D-gamma-glutamyl-meso-2,6-diaminopimela te | 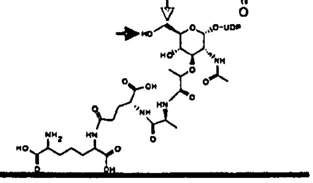 |

| N° | Substrate | Chemical Structure | N° | Substrate | Chemical Structure |
|---|---|---|---|---|---|
| 2439 | UDP-N-acetylmuramoyl-L-alanyl-D-glutamyl-meso-2,6-diaminopimeloyl-D-alanyl-D-alanine | 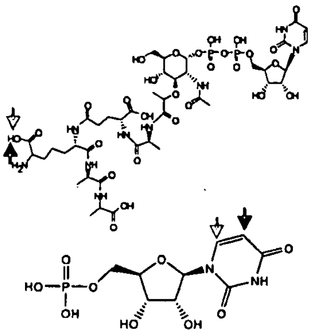 | 2452 | Urocanate | 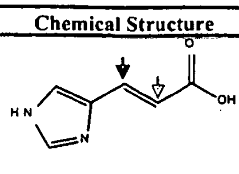 |
| 2440 | UMP | | 2453 | 2-Oxovalerate | 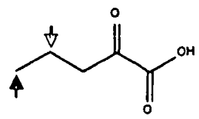 |
| 2441 | 2,3-Didehydro-pimeloyl-CoA | 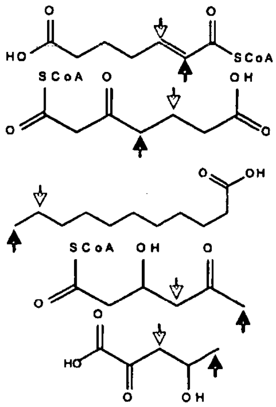 | 2454 | Cyclopentanol | 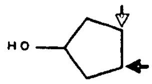 |
| 2442 | 3-Oxopimeloyl-CoA | | 2455 | p-Nitrophenol octanoate | 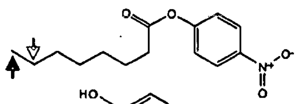 |
| 2443 | Undecanoic acid | | 2456 | Vanillin | 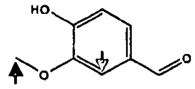 |
| 2444 | 3-Hydroxy-5-oxohexanoyl-CoA | | 2457 | L-Valine | 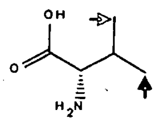 |
| 2445 | 4-Hydroxy-2-oxovalerate | | 2458 | Vanillate | 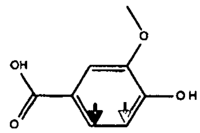 |
| 2446 | Cyclohexaamylose | 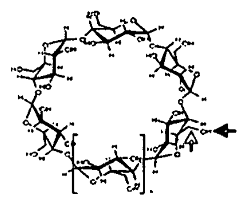 | 2459 | Neuraminic acid | 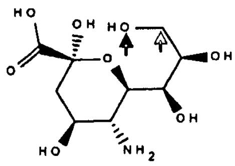 |
| 2447 | Uracil | 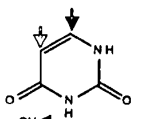 | 2460 | delta-Valerolactone | 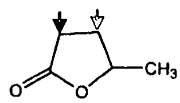 |
| 2448 | 5-Ureido-4-imidazole carboxylate | 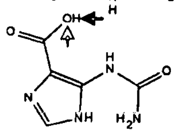 | 2461 | gamma-Undecalactone | 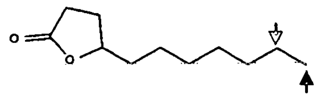 |
| 2449 | 3',5'-Cyclic IMP | 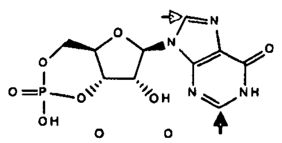 | 2462 | 5-Methyl-3-oxo-4-hexenoyl-CoA | 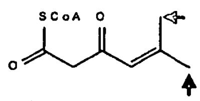 |
| 2450 | Urea-1-carboxylate | 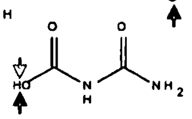 | 2463 | Vermelone | 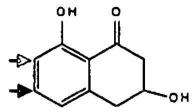 |
| 2451 | Butanoyl phosphate | 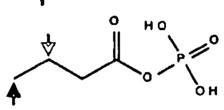 | 2464 | 1,3,7-Trimethylxanthine | 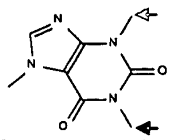 |

| N° | Substrate | Chemical Structure | N° | Substrate | Chemical Structure |
|---|---|---|---|---|---|
| 2465 | Methyl salicylate | 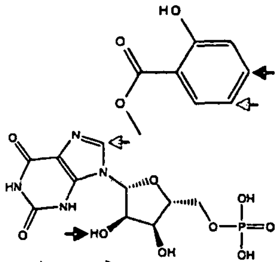 | 2475 | Chloroxylenol | 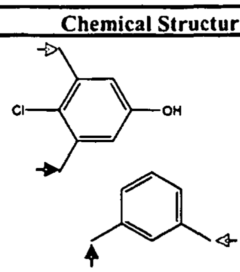 |
| 2466 | Xanthosine 5'-phosphate | | 2476 | m-Xylene | |
| 2467 | o-Xylene | 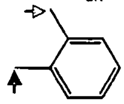 | 2477 | L-Xylose | 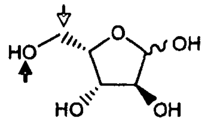 |
| 2468 | Xanthene | 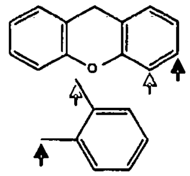 | 2478 | Xyloglucan | Too big |
| 2469 | o-Xylene | | 2479 | p-Xylene | 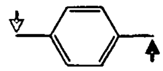 |
| 2470 | L-Xylulose 1-phosphate | 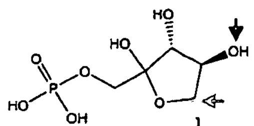 | 2480 | Xylitol | 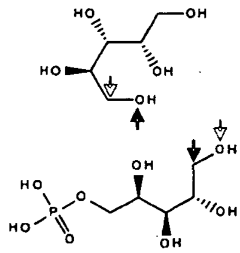 |
| 2471 | 1,3-beta-D-Xylan | 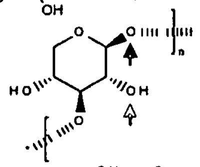 | 2481 | Xylitol 5-phosphate | |
| 2472 | L-Xylulose 5-phosphate | 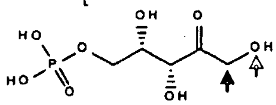 | 2482 | L-Xylulose | 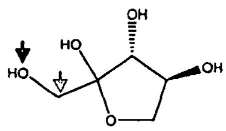 |
| 2473 | D-Xylulose 5-phosphate | 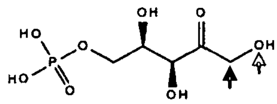 | 2483 | Zymosterol | 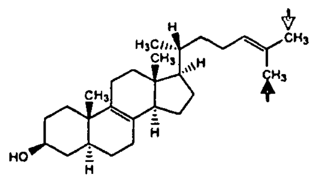 |
| 2474 | D-Xylose | 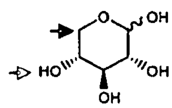 | | | |

**Table 3. Extensive description of microarray data**

| Compound | No. | Molecular Formula | KEGG Metabolite | Other Synonymous/ KEGG Analogous | PubChem Substance | KT2440 succinate (- background) | KOL (- background) | VUL (- background ) | L'A (- background) |
|---|---|---|---|---|---|---|---|---|---|
| gamma-Nonalactone | 1 | C9H16O2 | YES | | | 0 | 2263 | 0 | 1244,5 |
| epsilon-Caprolactone | 2 | C6H10O2 | YES | | | 0 | 0 | 0 | 203 |
| 10-Oxodecanoate | 3 | C10H18O3 | YES | | | 408 | 2383 | 4846 | 131,25 |
| 1-Bromodecane | 4 | C10H21Br | NO | n-Decyl bromide; Decyl bromide; 1-Bromo-decane | 8173 | 0 | 0 | 0 | 424,5 |
| 2-Bromodecane | 5 | C10H21Br | NO | 2-Bromo-decane | 98297 | 0 | 2834 | 0 | 311,25 |
| 10-Formyltetrahydrofolate | 6 | C20H23N7O7 | YES | | | 356 | 3150 | 0 | 461 |
| 4-Hydroxy-5-phenyltetrahydro-1,3-oxazin-2-one | 7 | C10H11NO3 | YES | | | 9157 | 0 | 0 | 4474,5 |
| 5-Chloro-3-methylcatechol | 8 | C7H7ClO2 | YES | | | 0 | 6596 | 0 | 0 |
| 3-Methylcrotonyl-CoA | 9 | C26H42N7O17P3S | YES | | | 5101 | 2729 | 0 | 0 |
| 2-Chloromaleylacetate | 10 | C6H5ClO5 | YES | | | 0 | 11633 | 0 | 329,25 |
| 1,3-Dichloropentane | 11 | C5H10Cl2 | NO | | 10517721 | 0 | 4421 | 0 | 686 |
| 11-Hydroxyundecanoate | 12 | C11H22O3 | NO | | 7004975 | 45 | 0 | 0 | 2800 |
| 11-Oxoundecanoate | 13 | C11H21O3 | NO | | 7004975 | 0 | 0 | 0 | 10011,3 |
| Oxatriazole | 14 | CHN3O | NO | | 21832123 | 0 | 2922 | 0 | 2083 |
| 1,2,3,5-Oxatriazole | 15 | CHN3O | NO | | 20165534 | 73 | 2564 | 0 | 1441,25 |
| 1,2,3-Oxadiazole | 16 | C2H2N2O | NO | Azoxime; 2,4-Diazafuran; 1,2,4-Oxadiazole | 6451463 | 0 | 29 | 19091 | 1553,75 |
| 1,2,3-Triazine | 17 | C3H3N3 | NO | Triazine; 1,2,3-Triazabenzene | 123047 | 0 | 0 | 7219 | 135 |
| v-Triazole | 18 | C2H3N3 | NO | Osotriazole; 1H-1,2,3- | 67516 | 216 | 0 | 0 | 2776,5 |

| | | | | | | | | | |
|---|---|---|---|---|---|---|---|---|---|
| | | | | Triazole; 2H-1,2,3-triazole; 1,2,3-1H-Triazole | | | | | |
| (R)-1,2,4-Butanetriol | 19 | C4H10O3 | NO | (2R)-butane-1,2,4-triol | 6994279 | 168 | 454 | 0 | 16,75 |
| 1,4,2-Dioxazole | 20 | C2H3NO2 | NO | | 21942687 | 40 | 89 | 0 | 1905 |
| 1,3,4-Oxadiazine | 21 | C3H4N2O | NO | 4H-1,3,4-oxadiazine | 21865302 | 80 | 0 | 2429 | 4764,25 |
| 2-Methyl-1,3,4-oxadiazole | 22 | C3H4N2O | NO | 2-Methyl[1,3,4]oxadiazole | 558447 | 0 | 0 | 4384 | 5887,75 |
| 1,3,5-Triazine | 23 | C3H3N3 | NO | s-Triazine; sym-Triazine; Cyanidine | 9262 | 128 | 4922 | 0 | 7121,5 |
| (1R,2S)-1,2-Dihydronaphthalene-1,2-diol | 24 | C10H10O2 | YES | | | 2886 | 0 | 0 | 1057,5 |
| 1,2,4-Trimethylbenzene | 25 | C9H12 | YES | | | 181 | 29 | 0 | 1187,5 |
| Oxathiazine | 26 | C3H3NOS | NO | | 21864897 | 128 | 0 | 0 | 6466,75 |
| 1,2,5-Oxadiazole | 27 | C2H2N2O | NO | Furazan; Azoxazole; 1-Oxa-2,5-diazacyclopentadiene | 67517 | 0 | 0 | 2555 | 5326,5 |
| 2,4,6-Tribromophenol | 28 | C6H3Br3O | YES | | | 75 | 0 | 0 | 0 |
| 1,2-Benzoquinone | 29 | C6H4O2 | YES | | | 116 | 340 | 0 | 5510,25 |
| 1,4-Dichloro-2-butene | 30 | C4H6Cl2 | NO | | 36255366 | 472 | 2998 | 0 | 1327,25 |
| 1,2-Didecanoyl-3-beta-D-galactosyl-sn-glycerol | 31 | C11H16O10(C20H40O4) | YES* | 1,2-Diacyl-3-beta-D-galactosyl-sn-glycerol | | 1 | 1772 | 0 | 572,25 |
| Decane-1,2-diol | 32 | C10H22O2 | NO | 1,2-Decanediol | 656165 | 22 | 1605 | 2995 | 282 |
| Dodecane-1,2-diol | 33 | C12H26O2 | NO | 1,2-Dodecanediol; Lauryl glycol | 669791 | 0 | 4238 | 10617 | 3649,5 |
| 1,2,4-Triazole | 34 | C2H3N3 | NO | s-Triazole | 9257 | 38 | 7155 | 0 | 7023,25 |
| 1,2-Dipropionyl-3-beta-D-galactosyl-sn-glycerol | 35 | C11H16O10(C6H12O4) | YES* | 1,2-Diacyl-3-beta-D-galactosyl-sn-glycerol | | 0 | 76 | 0 | 3014,5 |
| 1,2-Dibutyryl-3-beta-D-galactosyl-sn-glycerol | 36 | C17H30O10 | YES* | 1,2-Diacyl-3-beta-D-galactosyl-sn-glycerol | | 62 | 7033 | 0 | 527,75 |
| cis-1,2-Dihydroxy-1,2- | 37 | C11H12O2 | YES | | | 0 | 0 | 0 | 246,25 |

| | | | | | | | | | |
|---|---|---|---|---|---|---|---|---|---|
| dihydro-8-methylnaphthale | | | | | | | | | |
| 1,2-Butanediol | 38 | C4H10O2 | NO | Butane-1,2-diol | 641012 | 0 | 0 | 0 | 156 |
| 1,2-Dihexanoyl-3-beta-D-galactosyl-sn-glycerol | 39 | C11H16O10(C12H24O4) | YES* | 3-beta-D-Galactosyl-1,2-Dihexanoyl-glycerol; 1,2-Diacyl-3-beta-D-galactosyl-sn-glycerol | | 0 | 0 | 0 | 392 |
| 1,2-Dihydroxynaphthalene | 40 | C10H8O2 | YES | | | 0 | 5663 | 0 | 305 |
| 1,2-Diazole | 41 | C3H4N2 | YES | | | 0 | 5411 | 0 | 435 |
| 1,1-Dibromopentane | 42 | C5H10Br2 | NO | | 12003646 | 0 | 6396 | 0 | 848,25 |
| 1,2-Dichlorobenzene | 43 | C6H4Cl2 | YES | | | 0 | 3448 | 40 | 520,25 |
| 1,2-Dichloropentane | 44 | C5H10Cl2 | NO | | 158799 | 0 | 2 | 0 | 2292,75 |
| 1,1-Dichloropentane | 45 | C5H10Cl2 | NO | | 522765 | 216 | 8492 | 0 | 176 |
| 3-Hydroxy-1-indanone | 46 | C9H8O2 | YES | | | 0 | 3238 | 2861 | 880,5 |
| 1,5-Diaminopentane | 47 | C5H14N2 | YES | | | 244 | 3205 | 31 | 286 |
| (1S,2S)-1,2-Dihydronaphthalene-1,2-diol | 48 | C10H10O2 | YES | | | 25 | 2823 | 0 | 451,25 |
| 1,2-Didodecanoyl-3-beta-D-galactosyl-sn-glycerol | 49 | C11H16O10(C24H48O4) | YES* | 1,2-Diacyl-3-beta-D-galactosyl-sn-glycerol | | 0 | 3737 | 0 | 2775,25 |
| 1,2-Dihydroxydibenzothiophene | 50 | C12H8O2S | YES | | | 0 | 1985 | 0 | 291 |
| cis-1,2-Dihydroxy-1,2-dihydrodibenzothiophene | 51 | C12H10O2S | YES | | | 0 | 2734 | 0 | 4145 |
| 1,2-Didodecanoyl-3-O-(alpha-D-glucopyranosyl(1->2)-O-alpha-D-glucopyranosyl)sn- | 52 | C17H26O15(C24H48O4) | YES* | 1,2-Diacyl-3-O-(alpha-D-glucopyranosyl(1->2)-O-alpha-D-glucopyranosyl)sn-glycerol | | 25 | 2213 | 0 | 6401,75 |

| | | | | | | | | | |
|---|---|---|---|---|---|---|---|---|---|
| glycerol | | | | | | | | | |
| 1,2-Didodecanoyl-3-O-(alpha-D-glucopyranosyl)-sn-glycerol | 53 | C11H16O10(C24H48O4) | YES* | 1,2-Diacyl-3-O-(alpha-D-glucopyranosyl)-sn-glycerol | | 104 | 1336 | 0 | 152,75 |
| 1,2-Didodecanoyl-sn-glycerol | 54 | C5H6O5(C24H48O4) | YES* | 1,2-Diacyl-sn-glycerol | | 0 | 34 | 0 | 477,5 |
| 1,2-Oxazine | 55 | C4H9NO | NO | | 142102 | 114 | 0 | 0 | 3886,75 |
| 1,2-Dioctanoyl-3-beta-D-galactosyl-sn-glycerol | 56 | C11H16O10(C16H32O4) | YES* | 1,2-Diacyl-3-beta-D-galactosyl-sn-glycerol | | 0 | 0 | 18 | 1509 |
| 6-Deoxyerythronolide | 57 | C21H38O6 | YES | | | 177 | 4736 | 0 | 599,75 |
| 1,2-Dipalmitoyl-beta-D-galactosyl-sn-glycerol | 58 | C11H16O10(C32H64O4) | YES* | 1,2-Diacyl-beta-D-galactosyl-sn-glycerol | | 0 | 4740 | 3951 | 272,75 |
| 1,2-Dipalmitoyl-3-O-[alpha-D-glucopyranosyl(1->2)-O-alpha-D-glucopyranosyl]-sn-glycerol | 59 | C17H26O15(C32H64O4) | YES* | 1,2-Diacyl-3-O-(alpha-D-glucopyranosyl(1->2)-O-alpha-D-glucopyranosyl)sn-glycerol | | 0 | 817 | 0 | 263,75 |
| 1,2-Dipalmitoyl-3-O-alpha-D-glucopyranosyl-sn-glycerol | 60 | C11H16O10(C32H64O4) | YES* | 1,2-Diacyl-3-O-(alpha-D-glucopyranosyl)-sn-glycerol | | 0 | 0 | 26 | 47,25 |
| 1,2-Epoxycyclohexane | 61 | C6H10O | NO | Cyclohexene oxide; Cyclohexene epoxide; Cyclohexylene oxide; Cyclohexane oxide | 9246 | 0 | 0 | 0 | 157,75 |
| 1,2-Heptadecanediol | 62 | C17H36O2 | NO | Heptadecane-1,2-diol | 3738847 | 0 | 3853 | 0 | 76 |
| 1,7-Heptanediol | 63 | C7H16O2 | NO | Heptane-1,2-diol | 588135 | 0 | 38 | 96 | 332,25 |
| Hexacosane-1,2-diol | 64 | C26H54O2 | NO | 1,2-Hexacosanediol | 3802019 | 0 | 1431 | 863 | 126,75 |
| 1,2-Hexadecanediol | 65 | C16H34O2 | NO | Hexadecane-1,2-diol | 675284 | 0 | 3252 | 0 | 327 |
| 1,2-Hexanediol | 66 | C6H14O2 | NO | Hexane-1,2-diol | 724745 | 0 | 2277 | 44 | 1190 |

| | | | | | | | | | |
|---|---|---|---|---|---|---|---|---|---|
| 1,2-Nonanediol | 67 | C9H20O2 | NO | Nonane-1,2-diol | 100708 | 0 | 0 | 1812 | 6314,5 |
| 1,2-Nonadecanediol | 68 | C19H40O2 | NO | Nonadecane-1,2-diol | 10521486 | 0 | 122 | 70 | 410,75 |
| 3-Isopropylbut-3-enoyl-CoA | 69 | C28H46N7O17P3S | YES | | | 762 | 0 | 0 | 449,5 |
| 1,2-Octadecanediol | 70 | C18H38O2 | NO | Octadecane-1,2-diol; 1,2-Dihydroxyoctadecane | 114496 | 0 | 0 | 0 | 6605,5 |
| 1,2-Octanediol | 71 | C8H18O2 | NO | Octane-1,2-diol | 24852793 | 117 | 4601 | 3963 | 452 |
| D-()-Solketal propionate | 72 | C8H14O4 | NO | (+)-1,2-O-Isopropylidene-sn-glycerol propyl ester | | 0 | 1750 | 0 | 312 |
| 1,3-Oxazole | 73 | C3H3NO | NO | Oxazole | 9255 | 29 | 3188 | 2651 | 7015,5 |
| 1,2-Pentanediol | 74 | C5H12O2 | NO | Pentan-1,2-diol | 24887074 | 0 | 0 | 0 | 10203,3 |
| 1,2-Dibutyryl-sn-glycerol | 75 | C5H6O5(C8H16O4) | YES* | 1,2-Diacyl-sn-glycerol | | 960 | 0 | 0 | 9664,25 |
| Propane-1,2-diol 1-phosphate | 76 | C3H9O5P | YES | | | 161 | 1580 | 0 | 2399,5 |
| Propane-1,2-diol | 77 | C3H8O2 | YES | | | 0 | 0 | 0 | 4 |
| (R)-Propane-1,2-diol | 78 | C3H8O2 | YES | | | 0 | 4103 | 0 | 3905 |
| (S)-Propane-1,2-diol | 79 | C3H8O2 | YES | | | 0 | 0 | 0 | 10017 |
| 1,2-bis-O-Sinapoyl-beta-D-glucoside | 80 | C28H32O14 | YES | | | 141 | 23 | 0 | 2227,25 |
| 1,2-Tetracosanediol | 81 | C24H50O2 | NO | Tetracosane-1,2-diol | 11076928 | 90 | 868 | 2355 | 5063,5 |
| 1,2-Thiazole | 82 | C3H3NS | NO | 2-(2-Methylpropyl)-1,3-thiazole; Thiazole, 2-isobutyl-; 2-Isobutylthiazole; 2-Isobutyl-1,3-thiazole | 67515 | 120 | 4485 | 4548 | 0 |
| 1,2-Undecanediol | 83 | C11H24O2 | NO | Undecane-1,2-diol | 114435 | 100 | 88 | 0 | 5027,75 |
| 2-Chloro-4-hydroxy-6-amino-1,3,5-triazine | 84 | C3H3ClN4O | YES | | | 743 | 0 | 0 | 1530,75 |
| 1,3,4-Oxadiazole | 85 | C2H2N2O | NO | 1,3,4-Oxadiazole; 1-Oxa-3,4-diazacyclopentadiene | 97428 | 0 | 0 | 39 | 518 |
| 1,3,4-Thiadiazole | 86 | C2H2N2S | NO | Thiadiazole; 1,3,4-Thiadiazole | 119391 | 77 | 0 | 0 | 8697 |
| Dithiazine | 87 | C3H3NS2 | NO | | 21864925 | 0 | 0 | 0 | 33 |
| 1,2,5-Oxadiazine | 88 | C3H4N2O | NO | 2H-1,2,5-Oxadiazine | 21864916 | 136 | 5387 | 0 | 5278 |

| | | | | | | | | | |
|---|---|---|---|---|---|---|---|---|---|
| 1,3,5-Oxadithiane | 89 | C3H6OS2 | NO | | 19390256 | 437 | 2455 | 0 | 3409,25 |
| Thiadiazine | 90 | C16H7Cl4FN4S | NO | | 24203067 | 92 | 3004 | 2897 | 0 |
| (2S)-2-Isopropylmalate | 91 | C7H12O5 | YES | | | 1124 | 49 | 6138 | 770,25 |
| 3-Phospho-D-glyceroyl phosphate | 92 | C3H8O10P2 | YES | | | 1166 | 4819 | 0 | 4707,25 |
| 1,3,8-Trihydroxynaphthalene | 93 | C10H8O3 | YES | | | 112 | 3184 | 0 | 0 |
| 1,3-Bisphospho-D-glycerate | 94 | C3H8O10P2 | YES | | | 0 | 0 | 10 | 32,25 |
| 1,3-Butanediol | 95 | C4H10O2 | NO | Butane-1,3-diol; Butylene glycol; 1,3-Butylene glycol; 1,3-Dihydroxybutane; beta-Butylene glycol; beta-Butylene glycol; Methyltrimethylene glycol; 1-Methyl-1,3-propanediol | 7896 | 83 | 2296 | 0 | 4091,25 |
| 2,4-Dichloroaniline | 96 | C6H5Cl2N | NO | see http://umbbd.ahc.umn.edu | | 119 | 2376 | 0 | 682,75 |
| 1,3-Diazole | 97 | C3H4N2 | YES | | | 107 | 0 | 128 | 379,5 |
| 1,3-Dibromobenzene | 98 | C18H12Br6 | NO | | 46170822 | 0 | 0 | 0 | 182 |
| 1,2-Dihexanoyl-sn-glycerol | 99 | C5H6O5(C12H24O4) | YES* | 1,2-Diacyl-sn-glycerol | | 1567 | 0 | 0 | 5614,5 |
| Deoxyuridine | 100 | C9H12N2O5 | YES | | | 2492 | 447 | 8123 | 691 |
| 1,3-Dichloropentane | 101 | C5H10Cl2 | NO | | 10517721 | 192 | 5460 | 0 | 566 |
| 1,3-Dichlorobenzene | 102 | C6H4Cl2 | NO | | 46170822 | 0 | 4371 | 0 | 44,5 |
| 1,3-Didecanoyl-sn-glycerol | 103 | C5H6O5(C20H40O4) | YES* | 1,2-Diacyl-sn-glycerol | | 0 | 0 | 0 | 348,5 |
| 1,3-Dimethylbenzene | 104 | C8H10 | YES | | | 0 | 1822 | 0 | 0 |
| 1,7-Dimethyluric acid | 105 | C7H8N4O3 | YES | | | 0 | 4405 | 2595 | 291,25 |

| | | | | | | | | | |
|---|---|---|---|---|---|---|---|---|---|
| 1,3-Diolein | 106 | C39H72O5 | NO | 1,3-Diolein; Glycerol 1,3-dioleate; 1,3-Dioleoylglycerol; sn-1,3-Dioleoylglycerol; 2-Hydroxy-1,3-propanediyl dioleate; (Z,Z)-1,3-Dioctadecenoyl glycerol; 1,3-Di(cis-9-octadecenoyl)glycerol; Olein, 1,3-di- (7Cl,8Cl); 9-Octadecenoic acid (Z)-, 2-hydroxy-1,3-propanediyl ester; 9-Octadecenoic acid (9Z)-, 2-hydroxy-1,3-propanediyl ester (9Cl) | 841647 | 0 | 6666 | 0 | 5633,25 |
| 1,3,2-Dioxazole | 107 | C2H3NO2 | NO | | 21864914 | 0 | 0 | 26 | 394 |
| (R)-2-Methylmalate | 108 | C5H8O5 | YES | | | 0 | 0 | 0 | 148,25 |
| 1,3,5-Trithiane | 109 | C3H6S3 | NO | s-Trithiane; Trithioformaldehyde; Thioform; sym-Trithian; sym-Trithiane; Trimethylene trisulfide | 9264 | 0 | 81 | 0 | 260,25 |
| 1,2-Dipalmitoyl-3-myristoylglycerol | 110 | C49H94O6 | NO | 1,2-Dipalmitoyl-3-myristoylglycerol; 1,2-Dipalmitoyl-3-myristoyl-sn-glycerol; Hexadecanoic acid, 1-(((1-oxotetradecyl)oxy)methyl)-1,2-ethanediyl ester | 3745646 | 0 | 0 | 18360 | 3176,75 |
| 1,3-Dioctadecanoyl-sn-glycerol | 111 | C5H6O5(C36 H72O4) | YES* | 1,2-Diacyl-sn-glycerol | | 0 | 0 | 0 | 73,75 |
| 1,3-Dithiane | 112 | C4H8S2 | NO | m-Dithiane; 1,3-Dithiacyclohexane | 10451 | 0 | 0 | 127 | 3665,5 |
| 1,2-Dioleyl-sn- | 113 | C5H6O5(C36 | YES* | 1,2-Diacyl-sn-glycerol | | 0 | 2972 | 0 | 247,5 |

| | | | | | | | | | |
|---|---|---|---|---|---|---|---|---|---|
| glycerol | | H68O4) | | | | | | | |
| 3-Hydroxy-3-methyl-2-oxobutanoate | 114 | C5H8O4 | YES | | | 1287 | 5861 | 1974 | 2667,5 |
| Pentadecane-1,2-diol | 115 | C49H94O6 | NO | Pentadecane-1,2-diol | 118123 | 0 | 0 | 12074 | 241,75 |
| Propane-1,3-diol | 116 | C3H8O2 | YES | | | 161 | 3711 | 2394 | 11403,5 |
| 2,6-Dihydroxycyclohexane-1-carboxyl-CoA | 117 | C28H46N7O19P3S | YES | | | 10992 | 1154 | 279 | 397,75 |
| 1,3-Oxathiole | 118 | C3H4OS | NO | Oxathiole | 3080681 | 0 | 0 | 0 | 8378,5 |
| 1,3-Oxazole | 119 | C3H3NO | NO | Oxazole; 1,3-Oxazole- | 9255 | 0 | 2329 | 3291 | 10104,8 |
| 1,3-Pentanediol | 120 | C5H12O2 | NO | Pentane-1,3-diol | 50002645 | 0 | 0 | 0 | 5104,5 |
| dTTP | 121 | C10H17N2O14P3 | YES | | | 2203 | 4169 | 0 | 964,25 |
| 6-Hydroxycyclohex-1-ene-1-carboxyl-CoA | 122 | C28H44N7O18P3S | YES | | | 4055 | 0 | 0 | 2016,75 |
| 1,4-Butanediol | 123 | C4H10O2 | NO | Butanediol; Butane-1,4-diol | 8064 | 2 | 3039 | 0 | 207,25 |
| 1-Butanoyl-sn-glycerol 3-phosphate | 124 | C4H8O7P(C4H8O2) | YES* | 1-Acyl-sn-glycerol 3-phosphate | | 2053 | 0 | 0 | 970 |
| 1,4-Dichloro-2-butene | 125 | C4H6Cl2 | NO | (E)-1,4-dibromobut-2-ene | 36255366 | 14 | 5293 | 0 | 257,5 |
| D-Erythrose 4-phosphate | 126 | C4H9O7P | YES | | | 2384 | 3731 | 0 | 4735,25 |
| 1,4-Dichlorobutane | 127 | C4H8Cl2 | NO | 1,4-Dichloro-butane | 36678605 | 0 | 0 | 31 | 6168,25 |
| 1,3-Dichloropropane | 128 | C3H6Cl2 | NO | | 46170824 | 0 | 0 | 0 | 96 |
| 1,4-Dimethylbenzene | 129 | C8H10 | YES | | | 0 | 3512 | 0 | 6487 |
| 1,4-Dioxane | 130 | C4H8O2 | YES | | | 0 | 0 | 0 | 4750,25 |
| 1,4-Dithiane | 131 | C4H8S2 | YES | | | 0 | 4552 | 0 | 248,75 |
| Maltose | 132 | C12H22O11 | YES | | | 0 | 0 | 0 | 230,5 |
| Maltotriose | 133 | C18H32O16 | YES | | | 0 | 616 | 0 | 377 |
| Maltotetraose | 134 | C24H42O21 | YES | | | 0 | 4640 | 9677 | 418,75 |
| Maltopentaose | 135 | C30H52O26 | YES* | Maltodextrin | 700764 | 170 | 7467 | 7589 | 589,75 |
| Maltohexaose | 136 | C36H62O31 | YES | | | 0 | 1358 | 12777 | 682 |
| Maltoheptaose | 137 | C42H72O36 | YES* | Maltodextrin | 746463 | 0 | 26856 | 0 | 4708,25 |
| 1,5-Anhydro-D- | 138 | C6H10O5 | YES | | | 0 | 657 | 1357 | 2650,75 |

| | | | | | | | | | |
|---|---|---|---|---|---|---|---|---|---|
| fructose | | | | | | | | | |
| 1,5-Anhydro-D-mannitol | 139 | C6H12O5 | YES | | | 0 | 5255 | 0 | 132,25 |
| 1,5-Anhydro-D-glucitol | 140 | C6H12O5 | YES | | | 0 | 0 | 80 | 232 |
| 1,2-Dihydroxycyclohexa-3,5-diene-1-carboxylate | 141 | C7H8O4 | YES | | | 1656 | 4157 | 0 | 126,75 |
| 1,5-Dichloropentane | 142 | C5H10Cl2 | NO | | 155675 | 0 | 678 | 108 | 69 |
| 1-Heptadecanoyl-sn-glycero-phosphocholine | 143 | C9H20NO7P(C17H34O2) | YES* | 1-Acyl-sn-glycero-3-phosphocholine | | 4201 | 0 | 0 | 3028,75 |
| (15S)-15-Hydroxy-5,8,11-cis-13-trans-icosatetraenoate | 144 | C20H32O3 | YES | | | 0 | 0 | 739 | 5056,75 |
| 1,2-Pentanediol | 145 | C5H12O2 | NO | Pentane-1-2-diol | 93000 | 0 | 0 | 0 | 1442,75 |
| 2-Pentadecanol | 146 | C15H32O | NO | Pentadecan-2-ol; sec-Pentadecyl alcohol | 10510025 | 23 | 2 | 0 | 789 |
| 1,1-Dichlorohexane | 147 | C9H14Cl2N2O2 | NO | | 36594013 | 0 | 0 | 0 | 335,5 |
| 1,6-Naphthyridine | 148 | C8H6N2 | NO | | 209604 | 378 | 2206 | 0 | 2896,25 |
| 5-Methyluracil | 149 | C5H6N2O2 | YES | | | 112 | 502 | 0 | 237,5 |
| Methyl viologen | 150 | C12H14N2 | YES | | | 0 | 0 | 0 | 53 |
| 1,7-Naphthyridine | 151 | C8H6N2 | NO | 1,7-Diazanaphthalene | 10515748 | 0 | 143 | 0 | 7785,25 |
| 1,8-Dichlorooctane | 152 | C8H16Cl2 | NO | 1,8-Dicloro-octane | 75102 | 0 | 4485 | 0 | 5860,5 |
| 1,8-Naphthyridine | 153 | C8H6N2 | NO | 1,8-Naphthyridine; 1,8-Diazanaphthalene; 1,8-Pyridopyridine | 14717984 | 0 | 0 | 0 | 6134,5 |
| 1-Aminocyclopropane-1-carboxylate | 154 | C4H7NO2 | YES | | | 3160 | 74 | 7216 | 3819,75 |
| 1-Acetyl-sn-glycerol 3-phosphate | 155 | C4H8O7P(CH2O2) | YES* | 1-Acyl-sn-glycerol 3-phosphate | | 0 | 2713 | 0 | 80 |
| 1-Acetyl-5-Hydroxy- | 156 | C9H17NO3 | NO* | | | 0 | 3413 | 0 | 1369 |

| | | | | | | | | | |
|---|---|---|---|---|---|---|---|---|---|
| 1,5-dihydro-pyrrol-2-one | | | | | | | | | |
| 1-Butyrylglycerol | 157 | C4H7O4C4H8O2 | YES* | 1-Acylglycerol | | 0 | 0 | 0 | 63,75 |
| 1-Octanoyl-sn-glycerol-3-phosphate | 158 | C11H25O11P | YES* | 1-Acyl-sn-glycerol 3-phosphate | | 8069 | 465 | 0 | 2645,75 |
| 3,5-Dibromo-4-hydroxybenzoate | 159 | C7H4Br2O3 | YES | | | 37 | 0 | 0 | 0 |
| 4-Bromophenyl acetate | 160 | C8H7BrO2 | YES | | | 137 | 823 | 4 | 90 |
| 1-Bromobutane | 161 | C4H9Br | NO | 1-Bromobutane; Butyl bromide; 1-bromo-butane; n-Butylbromide | 49895557 | 0 | 0 | 5 | 155 |
| 1-Bromodecane | 162 | C10H21Br | NO | n-Decyl bromide; Decyl bromide; 1-Bromo-decane | 49960336 | 0 | 9528 | 0 | 4780 |
| 1,4-Dichlorobenzene | 163 | C6H4Cl2 | YES | | | 168 | 810 | 8156 | 12,25 |
| beta-D-Fructose 6-phosphate | 164 | C6H13O9P | YES | | | 520 | 2483 | 5566 | 0 |
| Dicarbomethoxynaphthalene | 165 | C14H12O4 | NO | Dimethyl naphthalene-2,6-dicarboxylate; Dimethyl 2,6-naphthalenedicarboxylate ; 2,6-Dicarbomethoxynaphthalene; Dimethyl naphthalene-2,6-dicarboxylate; Dimethyl-2,6-naphthalenedicarboxylate | 61225 | 0 | 2465 | 0 | 57 |
| 1-Butanoyl-sn-glycerol-3-phosphocholine | 166 | C9H20NO7P(C4H8O2) | YES* | 1-Acyl-sn-glycerol 3-phosphocholine | | 0 | 853 | 0 | 0 |
| 2-Chloro-4-methylphenol | 167 | C7H7ClO | YES | | | 0 | 4435 | 11 | 877,25 |

| | | | | | | | | | |
|---|---|---|---|---|---|---|---|---|---|
| 1-Chlorobutane | 168 | C4H9Cl | NO | 1-Chlorobutane; Butyl chloride; 1-Chloro-butane | 49854686 | 0 | 2819 | 4 | 1752,25 |
| 1-Chlorodecane | 169 | C10H21Cl | NO | 1-Chloro-Decane; n-Decyl chloride | 24892600 | 0 | 3704 | 0 | 412,25 |
| 3-Chloropropanoic acid | 170 | C3H5ClO2 | NO | | 24848067 | 0 | 253 | 0 | 259,75 |
| Chlorotoluene | 171 | C7H7Cl | NO | Chloromethylbenzene; ar-chloro-toluene; chloromethyl-benzene | 174753 | 0 | 3739 | 0 | 335,5 |
| 1-Deoxy-D-altro-heptulose 7-phosphate | 172 | C7H15O9P | YES | | | 0 | 6255 | 0 | 6210 |
| trans-1-Decalone | 173 | C10H16O | NO | trans-1-Decalone; trans-Decahydro-1-naphthalenone | 24849624 | 0 | 1403 | 0 | 3802 |
| 1-Decanol | 174 | C10H22O | YES | | | 1650 | 1612 | 0 | 84,5 |
| 1-Decanoyl-sn-glycerol 3-phosphate | 175 | C4H8O7P(C10H20O2) | YES* | 1-Acyl-sn-glycerol-3-phosphate | | 1 | 2693 | 31 | 3208,5 |
| 1-Decanoyl-sn-glycero-phosphocholine | 176 | C9H20NO7P(C10H20O2) | YES* | 1-Acyl-sn-glycerol-3-phosphocholine | | 0 | 0 | 0 | 77 |
| 1-Dodecanoyl-sn-glycero-phosphocholine | 177 | C9H20NO7P(C12H24O2) | YES* | 1-Acyl-sn-glycerol-3-phosphocholine | | 0 | 33 | 0 | 5019 |
| 1-Dodecanol | 178 | C12H26O | YES | | | 0 | 0 | 0 | 736,5 |
| 1,5-Naphthyridine | 179 | C8H6N2 | NO | 1,5-Naphthyridine; 1,5-Diazanaphthalene; 1,5-Pyridopyridine | 124190 | 50 | 3418 | 0 | 5227,5 |
| 2,6-Dichlorophenol | 180 | C6H4Cl2O | YES | | | 223 | 1019 | 701 | 409,25 |
| 3,4-Dichlorophenol | 181 | C6H4Cl2O | YES | | | 5 | 0 | 0 | 50 |
| 1H-2-Benzopyran-1-one | 182 | C9H6O2 | NO | Isochromen-1-one; Isocoumarin | 210232 | 0 | 0 | 0 | 76,5 |
| 1-Hydroxybutane-1,2,4-tricarboxylate | 183 | C7H10O7 | YES | | | 19 | 67 | 0 | 334,75 |

| | | | | | | | | | |
|---|---|---|---|---|---|---|---|---|---|
| 1-Heptadecanol | 184 | C17H36O | NO | Heptadecan-1-ol; n-Heptadecanol; Heptadecyl alcoho | 49975224 | 0 | 0 | 7435 | 2190,75 |
| 1-Dodecanoyl-sn-glycerol 3-phosphate | 185 | C4H8O7P(C12H24O2) | YES* | 1-Acyl-sn-glycerol 3-phosphate | | 925 | 1132 | 0 | 5457,75 |
| 2-Heptanol | 186 | C7H16O | NO | Heptan-2-ol; s-Heptyl alcohol; 2-Hydroxyheptane | 49856655 | 67 | 0 | 0 | 262,25 |
| 1-Heptanol | 187 | C7H16O | NO | Heptan-1-ol; Heptyl alcohol | 49856663 | 0 | 0 | 0 | 172,5 |
| 1-Hexacosanol | 188 | C26H54O | YES | | | 0 | 0 | 25 | 27 |
| 1-Palmitoylglycerophosphocholine | 189 | C24H51NO7P | YES | | | 0 | 0 | 0 | 88,25 |
| beta-D-Fructose 1,6-bisphosphate | 190 | C6H14O12P2 | YES | | | 623 | 4506 | 0 | 615,25 |
| 1-Hexanoyl-sn-glycero-phosphocholine | 191 | C9H20NO7P(C6H12O2) | YES* | 1-Acyl-sn-glycero-3-phosphocholine | | 0 | 4425 | 0 | 5056,25 |
| 1-Methylnicotinamide | 192 | C7H9N2O | YES | | | 0 | 30341 | 0 | 5442 |
| 4-Imidazolone-5-propanoate | 193 | C6H8N2O3 | YES | | | 0 | 7451 | 0 | 216,25 |
| 1-Methylnaphthalene | 194 | C11H10 | YES | | | 56 | 7176 | 3759 | 5466,25 |
| 5-Hydroxyindoleacetaldehyde | 195 | C10H9NO2 | YES | | | 119 | 0 | 0 | 585,25 |
| 2,5-Diaminopyrimidine nucleoside triphosphate | 196 | C9H18N5O14P3 | YES | | | 0 | 0 | 0 | 5785,75 |
| Indan-1-ol | 197 | C9H10O | YES | | | 0 | 20466 | 0 | 147,75 |
| 1-Indanone | 198 | C9H8O | YES | | | 21 | 144 | 0 | 10527,5 |
| 1R-Indenol | 199 | C9H8O | NO | 1H-inden-1-ol | 3745366 | 0 | 0 | 0 | 6519,5 |
| 1-Methoxynaphthalene | 200 | C11H10O | NO | PubChem Substance ID 49995766 | 49995766 | 0 | 0 | 0 | 2837,5 |

| | | | | | | | | | |
|---|---|---|---|---|---|---|---|---|---|
| 1-Monododecanoylglycerol | 201 | C4H7O4(C10H20O2) | YES* | 1-Monoacylglycerol | | 1016 | 5945 | 0 | 1331 |
| 1-Monotetradecanoylglycerol | 202 | C4H7O4(C14H28O2) | YES* | 1-Monoacylglycerol | | 537 | 0 | 0 | 422 |
| 2-Carboxy-2,3-dihydro-5,6-dihydroxyindole | 203 | C9H9NO4 | YES | | | 1496 | 0 | 0 | 309,5 |
| Methyl pyridine-3-carboxylate | 204 | C7H7O2N | NO | 1-Methoxy-naphthalene; Methyl 1-naphthyl ether; alpha-Methoxynaphthalene; alpha-Naphthyl methyl ether | 49700739 | 0 | 5099 | 678 | 9860,75 |
| 2-Hydroxymethylnaphthalene | 205 | C11H10O | YES | see http://umbbd.ahc.umn.edu; naphthalen-2-ylmethanol | 14709068 | 4063 | 0 | 0 | 906,75 |
| 1-Hexanol | 206 | C6H14O | NO | Hexyl alcohol; n-Hexanol; Hexan-1-ol | 151229 | 2782 | 15565 | 14 | 1053,25 |
| 1-Monohexanoylglycerol | 207 | C4H7O4(C6H12O2) | YES* | 1-Monoacylglycerol | | 1812 | 2781 | 0 | 11336,3 |
| 1-Monooctanoylglycerol | 208 | C4H7O4(C8H16O2) | YES* | 1-Monoacylglycerol | | 15795 | 6515 | 0 | 1151,5 |
| 1-Monodecanoylglycerol | 209 | C4H7O4(C10H20O2) | YES* | 1-Monoacylglycerol | | 3 | 0 | 0 | 3493,5 |
| 1-Nitrosonaphthalene | 210 | C10H7NO | YES | | | 0 | 2085 | 0 | 5730,5 |
| 1-Nitronaphthalene | 211 | C10H7NO2 | YES | | | 0 | 0 | 180 | 2117,5 |
| 1-Nitronaphthalene-5,6-oxide | 212 | C10H7NO3 | YES | | | 0 | 5001 | 0 | 16516,3 |
| 1-Nitronaphthalene-7,8-oxide | 213 | C10H7NO3 | YES | | | 98 | 0 | 0 | 9287,5 |
| 1-Nonadecanol | 214 | C19H40O | NO | Nonadecan-1-ol; | 24857252 | 0 | 0 | 0 | 437,5 |

| | | | | | | | | | |
|---|---|---|---|---|---|---|---|---|---|
| | | | | Nonadecyl alcohol | | | | | |
| 1-Nonanoyl-sn-glycero-3-phosphocholine | 215 | C9H20NO7P(C9H18O2) | YES* | 1-Acyl-sn-glycerol-3-phosphocholine | | 0 | 0 | 0 | 6061,5 |
| 1-Nonanol | 216 | C9H20O | NO | Nonan-1-ol; Nonanol | 49856860 | 0 | 0 | 0 | 829 |
| 1-Oleyl-sn-glycerol 3-phosphate | 217 | C4H8O7P(C18H36O2) | YES* | 1-Acyl-sn-glycerol 3-phosphate | | 0 | 0 | 0 | 1134 |
| 6-Oxo-2-hydroxycyclohexane-1-carboxyl-CoA | 218 | C28H44N7O19P3S | YES | | | 11775 | 2208 | 2618 | 7840,25 |
| 2-Octanol | 219 | C8H18O | NO | Octan-2-ol; Capryl alcohol | 49856866 | 140 | 0 | 0 | 107,25 |
| 1-Octadecanoyl-sn-glycero-phosphocholine | 220 | C9H20NO7P(C18H36O2) | YES* | 1-Acyl-sn-glycerol-3-phosphocholine | | 266 | 0 | 0 | 6153 |
| 1-Octadecanol | 221 | C18H38O | NO | Stearyl alcohol; Octadecan-1-ol; Octadecyl alcohol | 49998720 | 0 | 7344 | 3580 | 9085,25 |
| 1-Octanoyl-sn-glycero-3-phosphocholine | 222 | C9H20NO7P(C8H16O2) | YES* | 1-Acyl-sn-glycerol-3-phosphocholine | | 68 | 0 | 8 | 579,25 |
| 1-Octanol | 223 | C8H18O | YES | | | 4 | 0 | 3178 | 625,25 |
| 1-Palmitoyl-3-stearoyl-rac-glycerol | 224 | C37H72O5 | NO | (3-Hexadecanoyloxy-2-hydroxypropyl) octadecanoate | 24898427 | 0 | 2987 | 17629 | 6770,75 |
| 1-Pentanol | 225 | C5H12O | NO | Pentan-1-ol; Pentyl alcohol; Amyl alcohol | 49855733 | 134 | 0 | 0 | 7906,5 |
| 1-O-Hexadecanoyl-glycerol | 226 | C4H7O4(C16H32O2) | YES* | 1-Monoacylglycerol | | 198 | 0 | 0 | 623 |
| 1-Propionyl-sn-glycero-3-phosphocholine | 227 | C9H20NO7P(C3H6O2) | YES* | 1-Acyl-sn-glycerol-3-phosphocholine | | 0 | 0 | 15460 | 5257,5 |
| 1-Hexadecanoyl-sn-glycerol 3-phosphate | 228 | C4H8O7P(C16H32O2) | YES* | 1-Acyl-sn-glycerol 3-phosphate | | 412 | 3295 | 0 | 6252,75 |
| Phenylacetate | 229 | C8H8O2 | YES | | | 2033 | 0 | 0 | 5248,5 |

| | | | | | | | | | |
|---|---|---|---|---|---|---|---|---|---|
| 12-Pyren-1-yldodecanoic acid | 230 | C28H32O2 | NO | 1-Pyrenedodecanoic acid; 12-(1-Pyrene)dodecanoic acid | 692163 | 0 | 0 | 16 | 2973 |
| 2,3,4-Trichlorobiphenyl | 231 | C12H7Cl3 | NO | 2,3,4-Trichlorobiphenyl; 2,3,4-trichloro-1,1'-Biphenyl | 14718402 | 1 | 0 | 0 | 1661,75 |
| 1-Pyrroline | 232 | C4H7N | YES | | | 45 | 4255 | 120 | 2093 |
| 1-O-Sinapoyl beta-D-glucoside | 233 | C17H22O10 | YES | | | 68 | 3995 | 0 | 2989,5 |
| 1-Tetracosanol | 234 | C24H50O | NO | Tetracosan-1-ol; n-Tetracosanol | 48417558 | 0 | 0 | 0 | 0 |
| 1-Tetradecanoyl-sn-glycero-phosphocholine | 235 | C9H20NO7P(C14H28O2) | YES* | 1-Acyl-sn-glycerol-3-phosphocholine | | 13 | 0 | 0 | 7868 |
| 2,2',5-Trichlorobiphenyl | 236 | C12H7Cl3 | NO | 1,4-dichloro-2-(2-chlorophenyl)benzene | 49894501 | 72 | 145 | 0 | 1442,75 |
| 1-Undecanol | 237 | C11H24O | NO | Undecan-1-ol; Undecyl alcohol; n-Undecanol | 49857016 | 83 | 3244 | 0 | 1265 |
| 2,1-Benzisoxazole | 238 | C7H5NO | NO | 2,1-Benzoxazole; Anthranil; 2,1-Benzisoxazole; Benz(c)isoxazole | 403050 | 0 | 0 | 8658 | 206,25 |
| 1-Tetradecanol | 239 | C14H30O | NO | Tetradecan-1-ol; Myristyl alcohol | 49856841 | 2541 | 0 | 0 | 0 |
| 1-Bromo-2-(2-bromoethyl)benzene | 240 | C8H8Br2 | NO | | 5837137 | 0 | 2183 | 0 | 164,75 |
| 1-Chloro-2-(chlorophenylmethyl)benzene | 241 | C13H10Cl2 | YES | | | 0 | 9563 | 0 | 784,25 |
| 2,2-Dichloropropanoic acid | 242 | C3H4Cl2O2 | NO | | 105772 | 188 | 0 | 6865 | 326,5 |
| 2,3,4-Trichlorophenol | 243 | C6H3Cl3O | NO | | 49855072 | 0 | 4298 | 0 | 0 |
| 1-Pyrroline-5-carboxylate | 244 | C5H7NO2 | YES | | | 652 | 0 | 0 | 0 |
| 2,3,5,6- | 245 | C12H6Cl4 | NO | 1,2,4,5-Tetrachloro-3- | 177912 | 195 | 0 | 0 | 19052,8 |

| | | | | | | | | | |
|---|---|---|---|---|---|---|---|---|---|
| Tetrachlorobiphenyl | | | | phenylbenzene | | | | | |
| 2-(2,6-Dibromophenyl)-1H-pyrrole | 246 | C10H7Br2N | NO | | 20089117 | 0 | 0 | 0 | 773,5 |
| 2,4,5-Trichlorophenol | 247 | C16H16Cl6N2O2 | NO | Piperazine | 164654 | 0 | 13 | 0 | 539 |
| 2,3,6-Trichlorophenol | 248 | C6H3Cl3O | NO | | 49856057 | 0 | 2182 | 0 | 4335 |
| 2,3-Butanediol | 249 | C4H10O2 | NO | Butane-2,3-diol | 262 | 0 | 2610 | 0 | 0 |
| 1-Phenyl-1,3-butanedion | 250 | C10H10O2 | NO | 1-Phenylbutane-1,3-dione | 7993343 | 0 | 2424 | 7902 | 2866 |
| 2,4,4'-Trichlorobiphenyl | 251 | C12H7Cl3 | NO | | 166026 | 0 | 0 | 4239 | 272 |
| cis-2,3-Dihydroxy-2,3-dihydro-p-cumate | 252 | C10H14O4 | YES | | | 6931 | 0 | 71 | 1136,5 |
| 2,3-Dihydro-2,3-dihydroxybiphenyl | 253 | C12H12O2 | YES | | | 0 | 17 | 0 | 5343,75 |
| 3-(4-Chlorophenyl)benzene-1,2-diol | 254 | C12H9ClO2 | NO | 2,3-Dihydroxy-4'-chlorobiphenyl; 4'-chlorobiphenyl-2,3-diol; 4'-chloro-[1,1'-biphenyl]-2,3-diol | 8144889 | 0 | 2796 | 0 | 355 |
| 1-Chloro-3-(2-chlorophenyl)benzene | 255 | C12H8Cl2 | NO | 2,3'-Dichlorobiphenyl; 2,3'-Dichloro-1,1'-biphenyl; 2,3'-dichloro-1,1'-Biphenyl | 174990 | 0 | 2509 | 0 | 78,75 |
| 2,3-Dihydro-2,3-dihydroxybenzoate | 256 | C7H8O4 | YES | | | 17 | 0 | 0 | 5172,5 |
| 1,3-Thiazole | 257 | C3H3NS | NO | | 9256 | 0 | 0 | 0 | 111,5 |
| 2,3-Dihydroxybenzoate | 258 | C7H6O4 | YES | | | 0 | 3504 | 3330 | 3987,75 |
| (2,3-Dihydroxybenzoyl)adenylate | 259 | C17H18N5O10P | YES | | | 0 | 3366 | 0 | 191,5 |
| 2,3-Dihydroxybiphenyl | 260 | C12H10O2 | YES | | | 0 | 3407 | 7 | 312 |

| | | | | | | | | | |
|---|---|---|---|---|---|---|---|---|---|
| trans-2,3-Dihydroxycinnamate | 261 | C9H8O4 | YES | | | 0 | 39 | 0 | 294,75 |
| 2,4,6-Trichlorobiphenyl | 262 | C12H7Cl3 | NO | 1,3,5-Trichloro-2-phenylbenzene | 24860585 | 138 | 2610 | 0 | 410,5 |
| 2,2'-Dimethoxybiphenyl | 263 | C14H14O2 | NO | 1-Methoxy-2-(2-methoxyphenyl)benzene | 611816 | 0 | 0 | 0 | 239 |
| 2,3-Dihydroxy-3-methylpentanoate | 264 | C6H12O4 | YES | | | 117 | 6905 | 0 | 399,25 |
| 2-(Formamido)-N1-(5-phospho-D-ribosyl)acetamidine | 265 | C8H16N3O8P | YES | | | 1574 | 5631 | 0 | 276,75 |
| 2,3-Dihydroxyphenylpropanoate | 266 | C9H10O4 | YES | | | 188 | 0 | 0 | 353,25 |
| 2,3-Dihydrofuran | 267 | C4H6O | NO | | 24863187 | 205 | 11643 | 287 | 23 |
| (R)-2,3-Dihydroxy-3-methylbutanoate | 268 | C5H10O4 | YES | | | 2441 | 1841 | 0 | 3426,75 |
| 1,2-Dimethylnaphthalene | 269 | C12H12 | YES | | | 0 | 53 | 0 | 164,25 |
| 2,3-Diketo-L-gulonate | 270 | C6H8O7 | YES | | | 62 | 3494 | 0 | 368,75 |
| 2,3-Dihydroxy-isovalerate | 271 | C5H10O4 | YES | | | 0 | 43 | 9846 | 333,5 |
| 2,3-Dihydroxy-p-cumate | 272 | C10H12O4 | YES | | | 4672 | 0 | 8192 | 31 |
| cis-2,3-Dihydro-2,3-dihydroxy-4'-chlorobiphenyl | 273 | C12H11ClO2 | YES | | | 1319 | 0 | 0 | 0 |
| 2,4,5-Trichlorobiphenyl | 274 | C12H7Cl3 | NO | 1,2,4-Trichloro-5-phenylbenzene | 170103 | 0 | 0 | 3420 | 0 |
| 2,4',5-Trichlorobiphenyl | 275 | C12H7Cl3 | NO | 1,4-Dichloro-2-(4-chlorophenyl)benzene | 49894500 | 22 | 5585 | 0 | 0 |
| 4-Hydroxyphenylpyruvate | 276 | C9H8O4 | YES | | | 9074 | 2157 | 0 | 109,75 |
| 2,3- | 277 | C7H7NO4 | YES | | | 3391 | 3475 | 0 | 1179 |

| | | | | | | | | | |
|---|---|---|---|---|---|---|---|---|---|
| Dihydrodipicolinate | | | | | | | | | |
| 2',2,4-Trichlorobiphenyl | 278 | C12H7Cl3 | NO | 2,4-Dichloro-1-(2-chlorophenyl)benzene | 179114 | 0 | 10 | 0 | 0 |
| 2,4,6-Trichlorophenol | 279 | C6H3Cl3O | YES | | | 0 | 3021 | 0 | 0 |
| 2,4-Dichlorobenzoate | 280 | C7H4Cl2O2 | YES | | | 0 | 1797 | 0 | 33 |
| 1-Chloro-4-(2,2-dichloro-1-phenylethyl)benzene | 281 | C14H11Cl3 | YES | | | 0 | 0 | 0 | 7198,75 |
| 2-Bromo-1,3-dichlorobenzene | 282 | C6H3BrCl2 | NO | 2,6-Dichlorobromobenzene; 2-Bromo-1,3-dichlorobenzene; 1-BROMO-2,6-DICHLOROBENZENE; 1,3-Dichloro-2-bromobenzene | 171870 | 0 | 4210 | 0 | 1251,75 |
| 2,4-Dichlorophenoxyacetate | 283 | C8H6Cl2O3 | YES | | | 0 | 2583 | 0 | 272 |
| gamma-Glutamyl-gamma-aminobutyraldehyde | 284 | C9H16N2O4 | YES | | | 2131 | 494 | 0 | 19 |
| 2,4-Dichlorophenol | 285 | C6H4Cl2O | YES | | | 0 | 0 | 0 | 286 |
| 2,4-Dinitroaniline | 286 | C6H5N3O4 | YES | | | 0 | 0 | 0 | 117,75 |
| 2,4,5-Trichlorophenol | 287 | C6H3Cl3O | YES | | | 27 | 4626 | 47 | 5695,5 |
| S)-2-[5-Amino-1-(5-phospho-D-ribosyl)imidazole-4-carboxamido]succinate | 288 | C13H19N4O12P | YES | | | 57 | 2032 | 0 | 88 |
| 2,5-Dichloro-2,5-cyclohexadiene-1,4-diol | 289 | C6H6Cl2O2 | YES | | | 0 | 52 | 0 | 330,5 |
| 1,4-Dichloro-2-phenylbenzene | 290 | C12H8Cl2 | NO | 2,5-Dichlorobiphenyl | 17395354 | 33 | 1704 | 0 | 250,75 |

| | | | | | | | | | |
|---|---|---|---|---|---|---|---|---|---|
| 2,5-Dichlorohydroquinone | 291 | C6H4Cl2O2 | YES | | | 0 | 0 | 3584 | 0 |
| 2,5-Dichlorophenol | 292 | C6H4Cl2O | YES | | | 0 | 0 | 0 | 2341 |
| 2,5-Didehydro-D-gluconate | 293 | C6H8O7 | YES | | | 37 | 26 | 0 | 6768,25 |
| 2,5-Diamino-6-(5'-phosphoribosylamino)-4-pyrimidineone | 294 | C9H16N5O8P | YES | | | 1180 | 2507 | 0 | 42,25 |
| 2,5-Diamino-6-hydroxy-4-(5'-phosphoribosylamino)-pyrimidine | 295 | C9H16NO7P | NO* | | | 0 | 0 | 5183 | 3904,25 |
| 2,5-Didehydro-D-gluconate | 296 | C6H8O7 | YES | | | 0 | 0 | 6926 | 335 |
| 2,5-Dihydroxyphenyl acetate | 297 | C8H8O4 | YES | | | 0 | 0 | 0 | 2723 |
| 2-cis,6-trans-Farnesol | 298 | C15H26O | YES | | | 0 | 0 | 0 | 502 |
| (2Z,6E)-3,7,11-Trimethyldodeca-2,6,10-trienal | 299 | C15H24O | NO | (2-cis,6-trans)-Farnesal | 14718291 | 0 | 3 | 0 | 128,25 |
| LL-2,6-Diaminoheptanedioate | 300 | C7H14N2O4 | YES | | | 0 | 0 | 0 | 228,75 |
| meso-2,6-Diaminoheptanedioate | 301 | C7H14N2O4 | YES | | | 0 | 7 | 4632 | 329,75 |
| 2,6-Dimethylnaphthalene | 302 | C12H12 | YES | | | 9 | 0 | 0 | 4151,25 |
| 2-trans,6-trans-Farnesol | 303 | C15H26O | YES | | | 0 | 7 | 0 | 3880 |
| 2-trans,6-trans-Farnesal | 304 | C15H26O | YES | | | 0 | 0 | 0 | 368 |
| 2-Amino-3-oxobutanoate | 305 | C4H7NO3 | YES | | | 0 | 0 | 0 | 250,25 |
| 2-Amino benzene | 306 | C6H7NO3S | YES | | | 130 | 0 | 0 | 0 |

| | | | | | | | | | |
|---|---|---|---|---|---|---|---|---|---|
| sulfonate | | | | | | | | | |
| cis-2,3-Dihydroxy-2,3-dihydroethylbenzene | 307 | C8H12O2 | YES | | | 0 | 6647 | 0 | 204,25 |
| Methyl 3-hydroxydecanoate | 308 | C11H22O3 | NO | Methyl ester of 3-hydroxydecanoic acid | 521747 | 0 | 0 | 0 | 5950,5 |
| 2-Amino-4-hydroxy-6-hydroxymethyl-7,8-dihydropteridine diphosphate | 309 | C7H11N5O8P2 | YES | | | 1150 | 0 | 6044 | 291,75 |
| 2-Amino-4-hydroxy-6-hydroxymethyl-7,8-dihydropteridine | 310 | C7H9N5O2 | YES | | | 2282 | 3807 | 12632 | 134,5 |
| 2-Amino-7,8-dihydro-4-hydroxy-6-(diphosphooxymethyl)pteridine | 311 | C7H11N5O8P2 | YES | | | 470 | 0 | 0 | 0 |
| 2-Aceto-2-hydroxybutanoate | 312 | C6H10O4 | YES | | | 42 | 3843 | 0 | 3297,75 |
| (S)-2-Aceto-2-hydroxybutanoate | 313 | C6H10O4 | YES | | | 0 | 4014 | 9043 | 3416,75 |
| 2-Aminobenzenesulfonate | 314 | C6H7NO3S | YES | | | 0 | 3582 | 0 | 2650,5 |
| 2-Aminobenzoate | 315 | C7H7NO2 | YES | | | 70 | 2321 | 0 | 5060,5 |
| 2-Amino-3-oxobutanoate | 316 | C4H5NO3 | YES | | | 0 | 1040 | 0 | 1328,25 |
| 2-Arachidonoylglycerol | 317 | C23H38O4 | YES | | | 86 | 0 | 13520 | 390,25 |
| 2,3-Dihydroxyethylbenzene | 318 | C8H10O2 | YES | | | 0 | 4466 | 0 | 4212 |
| 4-Fluorobenzoate | 319 | C7H5FO2 | YES | | | 1877 | 2694 | 0 | 267,5 |
| 2-Hydroxymuconate | 320 | C6H6O5 | YES | | | 759 | 14410 | 31915 | 98,5 |
| 2,3-Bisphospho-D-glycerate | 321 | C3H8O10P2 | YES | | | 0 | 3442 | 89 | 3194,5 |

| | | | | | | | | | |
|---|---|---|---|---|---|---|---|---|---|
| Bromobenzene-3,4-dihydrodiol | 322 | C6H7BrO2 | YES | | | 7 | 7691 | 3 | 0 |
| 2-Butyne-1,4-diol | 323 | C4H6O2 | YES | | | 0 | 0 | 0 | 185,5 |
| Bromobenzene | 324 | C6H5Br | YES | | | 66 | 4295 | 72 | 108,5 |
| 2-Bromobutane | 325 | C4H9Br | NO | 2-Butyl bromide | 149518 | 179 | 2567 | 5 | 3171 |
| 2-Bromoethylbenzene | 326 | C8H9Br | NO | Phenethyl bromide; Phenylethyl bromide; 2-Phenylethyl bromide; 2-Phenethyl bromide; beta-Bromoethylbenzene; 1-Bromo-2-phenylethane; 2-Phenyl-1-bromoethane | 150748 | 0 | 0 | 3035 | 4467 |
| 2-Bromopropanoic acid | 327 | C3H5BrO2 | NO | | 155032 | 0 | 7614 | 0 | 6197,5 |
| 1-Bromo-2-ethenylbenzene | 328 | C8H7Br | NO | 2-Bromostyrene | 159524 | 0 | 0 | 0 | 379,5 |
| Butan-2-ol | 329 | C4H10O | NO | 2-Butanol; sec-Butanol | 149532 | 55 | 0 | 0 | 451,25 |
| (E)-2-Chlorobut-2-ene | 330 | C4H7Cl | NO | cis-2-Chloro-2-butene | 713828 | 0 | 1433 | 0 | 1727,5 |
| 2-Chlorobiphenyl | 331 | C12H9Cl | YES | | | 0 | 942 | 76 | 2008,25 |
| 2-Chlorobutane | 332 | C4H9Cl | NO | sec-Butyl chloride; 2-chloro-butane; 2-chlorobutane; 1-Methylpropyl chloride | 149527 | 0 | 0 | 0 | 253,5 |
| 2-Chlorodecane | 333 | C10H21Cl | NO | | 713064 | 0 | 1918 | 3294 | 5759,75 |
| 1-(2-Carboxyphenylamino)-1'-deoxy-D-ribulose 5'-phosphate | 334 | C12H16NO9P | YES | | | 621 | 3014 | 0 | 187,5 |
| 2-Dehydro-3-deoxy-D-arabino-heptonate 7-phosphate | 335 | C7H13O10P | YES | | | 3830 | 2237 | 0 | 1890 |
| 2-Chloropropanoic acid | 336 | C3H5ClO2 | NO | | 155037 | 0 | 1686 | 0 | 2408,25 |
| cis-2-Hydroxypenta-2,4-dienoate | 337 | C5H6O3 | YES | | | 3392 | 2574 | 0 | 973 |

| | | | | | | | | | |
|---|---|---|---|---|---|---|---|---|---|
| 1-Chloro-2-methylbenzene | 338 | C7H7Cl | NO | o-Chlorotoluene; 2-Chlorotoluene; o-Tolyl chloride; ortho-Chlorotoluene; 1-Chloro-2-methylbenzene; 1-chloro-2-methyl- benzene; 2-Methylchlorobenzene; 2-Chloro-1-methylbenzene | 150288 | 0 | 109 | 3688 | 1290,25 |
| 3-(p-Chlorophenyl)coumarin | 339 | C15H9ClO2 | YES | | | 0 | 0 | 13743 | 578,5 |
| 2-Phenylpropanenitrile | 340 | C9H9N | NO | alpha-Cyanoethylbenzene; Benzeneacetonitrile | 10393027 | 169 | 2180 | 1891 | 28 |
| 2-Dehydro-3-deoxy-D-gluconate 6-phosphate | 341 | C6H11O9P | YES | | | 1540 | 89 | 0 | 147,25 |
| 2-Dehydro-3-deoxy-L-arabinonate | 342 | C5H8O5 | YES | | | 67 | 5026 | 11014 | 4771,75 |
| 2-Dehydro-3-deoxy-D-gluconate | 343 | C6H10O6 | YES | | | 833 | 2415 | 0 | 441 |
| 2-Chlorophenol | 344 | C6H5ClO | YES | | | 0 | 10289 | 7703 | 7146,25 |
| 2-Deoxy-D-gluconate | 345 | C6H12O6 | YES | | | 26 | 628 | 0 | 224,5 |
| 2-Dehydro-D-glucose | 346 | C6H10O6 | YES | | | 0 | 0 | 10704 | 4417,5 |
| 2-Dehydro-3-deoxy-D-xylonate | 347 | C5H8O5 | YES | | | 85 | 170 | 10915 | 587,75 |
| 5-Dehydro-2-deoxy-D-gluconate | 348 | C6H10O6 | YES | | | 31 | 0 | 12311 | 337 |
| 2-Dehydro-D-gluconate | 349 | C6H10O7 | YES | | | 13231 | 0 | 0 | 453,25 |
| 2-Deoxy-D-glucose | 350 | C6H12O5 | YES | | | 0 | 2195 | 0 | 25,25 |
| 2-Dodecanoylglycerol | 351 | C4H7O4(C12H24O2) | YES* | 2-Acylglycerol | | 0 | 2039 | 18 | 4924,5 |
| 2-Decanoylglycerol | 352 | C4H7O4(C10 | YES* | 2-Acylglycerol | | 64 | 5464 | 0 | 294,75 |

| | | | | | | | | | |
|---|---|---|---|---|---|---|---|---|---|
| | | H20O2) | | | | | | | |
| 2-Dehydro-3-deoxy-D-galactonate | 353 | C6H10O6 | YES | | | 258 | 24 | 1064 | 4571 |
| 2-Dehydro-3-deoxy-D-galactonate 6-phosphate | 354 | C6H11O9P | YES | | | 6 | 0 | 5012 | 394,25 |
| Decan-2-ol | 355 | C10H22O | NO | 2-Hydroxydecane | 157538 | 0 | 8722 | 6744 | 6349,25 |
| 2-Keto-L-gulonate | 356 | C6H10O7 | YES | | | 0 | 5524 | 0 | 5259,25 |
| 2-Dehydropantoate | 357 | C6H10O4 | YES | | | 4647 | 2026 | 0 | 4175,5 |
| 2-Dehydropantolactone | 358 | C6H8O3 | YES | | | 0 | 2969 | 4093 | 4948,75 |
| 2H-1,2,4-Benzoxadiazine | 359 | C7H6N2O | NO | | 39951878 | 0 | 172 | 66 | 1 |
| Demethylmenaquinone | 360 | C50H70O2 | NO | | 5376004 | 0 | 0 | 0 | 2895 |
| Dodecan-2-ol | 361 | C12H26O | NO | 2-Decanol | 167827 | 0 | 4551 | 0 | 821,75 |
| 2-Deoxy-D-ribose 5-phosphate | 362 | C5H11O7P | YES | | | 128 | 0 | 0 | 2465,75 |
| 2-Deoxy-D-ribose 1-phosphate | 363 | C5H11O7P | YES | | | 0 | 422 | 0 | 1544,75 |
| 2-Ethylhexan-1-ol | 364 | C8H18O | NO | Ethylhexanol; 2-Ethylhexanol; Octyl alcohol; 2-Ethylhexan-1-ol; 2-ethyl-1-hexanol; 2-Ethylhexyl alcohol | 150810 | 0 | 0 | 34243 | 1812,25 |
| 2-Demethylmenaquinone | 365 | C15H14O2(C25H40) | YES | | | 2483 | 3567 | 24 | 11,75 |
| 2H-1,2-Oxazine | 366 | C4H9NO | NO | Oxazinane | 10520377 | 0 | 0 | 13 | 4518 |
| 2-Hydroxy-2-hydropyrone-4,6-dicarboxylate | 367 | C7H6O6 | YES | | | 1615 | 16399 | 0 | 6265,5 |
| 2H-1,2-Benzoxazine | 368 | C12H17N3O | NO | | 22918850 | 0 | 0 | 0 | 6052 |
| 4-Methyl-1,3-oxazinane-2-thione | 369 | C5H9NOS | NO | 4-Methyltetrahydro-1,3-oxazine-2-thione | 5371781 | 104 | 0 | 0 | 1869,75 |

| | | | | | | | | | |
|---|---|---|---|---|---|---|---|---|---|
| 3-Methyl-2H-1,4-benzoxazine | 370 | C9H9NO | NO | | 16851504 | 0 | 0 | 0 | 76,25 |
| 2-Hydroxy-3-carboxy-6-oxo-7-methylocta-2,4-dienoate | 371 | C10H12O6 | YES | | | 1087 | 0 | 4180 | 501,25 |
| 2H-1-Benzopyran-2-one | 372 | C9H6O2 | YES | Coumarin | | 40 | 2726 | 0 | 156 |
| 2H-1-Benzopyran | 373 | C9H8O | NO | 2H-Chromene; delta-3-Chromene; 1,2-Benzopyran; 1,2-Chromene; 2H-1-benzopyran | 152402 | 0 | 3507 | 0 | 194,25 |
| 3H-1,2,4-dioxazole | 374 | C2H3NO2 | NO | | 20393311 | 0 | 1850 | 0 | 1214 |
| (S)-2-Hydroxy-2-methyl-3-oxobutanoate | 375 | C5H8O4 | YES | | | 0 | 0 | 0 | 2972 |
| 2-Phenyl-4-propyl-tetrahydro-1,4-oxazine | 376 | C13H19NO | NO | 2-phenyl-4-propylmorpholine | 6430941 | 89 | 0 | 39 | 683,75 |
| 2-Hydroxy-3-oxopropanoate | 377 | C3H4O4 | YES | | | 0 | 10796 | 0 | 6964,75 |
| 2-Hydroxy-3-oxosuccinate | 378 | C4H4O6 | YES | | | 0 | 5997 | 117 | 1177,25 |
| 2-Hydroxy-5-methyl-cis,cis-muconic semialdehyde | 379 | C7H8O4 | YES | | | 0 | 0 | 156 | 5319 |
| 2-Hexadecanoyl-glycerol | 380 | C4H7O4(C16 H32O2) | YES* | 2-Acylglycerol | | 0 | 6276 | 0 | 824,5 |
| 2-Hydroxy-6-keto-2,4-heptadienoate | 381 | C7H8O4 | YES | | | 0 | 0 | 0 | 6241 |
| 2-Hydroxy-6-oxo-7-methylocta-2,4-dienoate | 382 | C9H12O4 | YES | | | 1439 | 1862 | 0 | 3536,25 |
| 2-Hydroxy-6-oxo-6-phenylhexa-2,4- | 383 | C12H10O4 | YES | | | 0 | 43 | 0 | 428,5 |

| | | | | | | | | | |
|---|---|---|---|---|---|---|---|---|---|
| dienoate | | | | | | | | | |
| 2-Hydroxyadipate | 384 | C6H10O5 | YES | | | 0 | 9717 | 0 | 606,25 |
| 2-Hydroxybutane-1,2,4-tricarboxylate | 385 | C7H10O7 | YES | | | 2 | 1636 | 0 | 99,25 |
| 2-Hydroxycyclohexan-1-one | 386 | C6H10O2 | YES | | | 0 | 2379 | 0 | 613,5 |
| Heptadec-2-nol | 387 | C17H36O | NO | 2-Heptadecanol | 10510276 | 0 | 5045 | 7487 | 1326,5 |
| 2-Heptadecanoylglycerol | 388 | C20H40O4 | YES* | 2-Acylglycerol, 2-Glyceride, 2-Monoacylglycerol | | 0 | 0 | 0 | 5300,25 |
| Hexacosan-1-ol | 389 | C46H96O2 | NO | 1-Hexacosanol | 18702115 | 0 | 4298 | 0 | 170 |
| L-Argininosuccinate | 390 | C10H18N4O6 | YES | | | 1070 | 56 | 0 | 7095,25 |
| Hexadecan-2-ol | 391 | C16H34O | NO | 2-Headecanol | 10508538 | 0 | 0 | 0 | 271,75 |
| 2-Hexanoylglycerol | 392 | C4H7O4(C6H12O2) | YES | 2-Acylglycerol, 2-Glyceride, 2-Monoacylglycerol | | 0 | 7115 | 0 | 5556,5 |
| Hexan-2-ol | 393 | C6H14O | NO | Methylamyl alcohol; Methyl amyl alcohol; Methyl-1-Pentanol | 198589 | 0 | 97 | 66 | 3710,25 |
| (R)-2-Hydroxyglutarate | 394 | C5H8O5 | YES | | | 0 | 0 | 0 | 248 |
| 2,4-Dichlorobenzoyl-CoA | 395 | C28H38Cl2N7O17P3S | YES | | | 497 | 0 | 0 | 1004,5 |
| 2H-Imidazole | 396 | C3H4N2 | NO | | 37492577 | 0 | 1672 | 0 | 210,25 |
| 2-Hydroxymalonate | 397 | C3H4O5 | YES | | | 0 | 0 | 0 | 1771,75 |
| 2-Hydroxymuconate semialdehyde | 398 | C6H6O4 | YES | | | 0 | 4155 | 221 | 277,25 |
| 2-Hydroxyhexadecanal | 399 | C16H32O2 | NO | | 31537087 | 0 | 2198 | 79 | 3760,25 |
| 2-Bromo-1-chloropropane | 400 | C3H6BrCl | NO | | 161246 | 0 | 2494 | 0 | 4478,5 |
| 1-chloro-2-ethenylbenzene | 401 | C8H7Cl | NO | 2-Chlorostyrene; o-Chlorostyrene; Chlorostyrene | 159523 | 0 | 2728 | 0 | 386,25 |

| | | | | | | | | | |
|---|---|---|---|---|---|---|---|---|---|
| Pyran-2-one | 402 | C5H4O2 | NO | | 210283 | 0 | 11 | 0 | 1754,25 |
| 2H-Pyran | 403 | C5H6O | NO | | 763609 | 122 | 819 | 0 | 1673,25 |
| p-Tolualdehyde | 404 | C8H8O | YES | | | 61 | 2002 | 3032 | 3219 |
| 2-Hydroxybiphenyl | 405 | C12H10O | YES | | | 0 | 5966 | 0 | 339,5 |
| 2'-Hydroxybiphenyl-2-sulfinate | 406 | C12H10O3S | YES | | | 0 | 3326 | 0 | 247,25 |
| 2-Isothiocyanatoethylbenzene | 407 | C9H9NS | NO | Phenethyl isothiocyanate | 159961 | 0 | 1916 | 28 | 364,25 |
| N-Methylimidazolidine-2,4-dione | 408 | C4H6N2O2 | YES | | | 0 | 493 | 0 | 5624,25 |
| 2-Indanone | 409 | C9H8O | YES | | | 0 | 3464 | 0 | 7463,5 |
| 2-Isopropylmaleate | 410 | C7H10O4 | YES | | | 0 | 50 | 0 | 12684,3 |
| Benzoyl acetyl-CoA | 411 | C30H42N7O18P3S | YES | | | 0 | 5322 | 4585 | 873,5 |
| 2H-Pyrrole | 412 | C4H5N | NO | | 11533617 | 0 | 0 | 4426 | 1941,75 |
| 2-Dehydro-3-deoxy-D-gluconate | 413 | C6H10O6 | YES | | | 0 | 0 | 0 | 370,5 |
| 2-Keto-D-gluconic acid | 414 | C6H10O7 | YES | | | 37 | 0 | 0 | 1201,75 |
| 2-Keto-3-methylbutyric acid | 415 | C5H8O3 | YES | | | 0 | 0 | 0 | 6721,75 |
| 2-Oxoisovalerate | 416 | C5H8O3 | YES | | | 0 | 59 | 121 | 708,5 |
| 4-Methylthio-2-oxobutanoate | 417 | C5H8O3S | YES | | | 0 | 0 | 16 | 3094,75 |
| 2-Oxopent-4-enoic acid | 418 | C5H6O3 | NO | Oxopent-4-enoate; 2-keto-4-pentenoic acid; Oxopentanoate; 2-oxopent-4-enoate; 2-keto-4-pentenoate; 2-ketopent-4-enoic acid; 2-hydroxy-2,4-pentadienoate; 2-hydroxypenta-2,4-dienoate; cis-2- | 3040 | 0 | 169 | 0 | 1534,75 |

| | | | | | | | | | |
|---|---|---|---|---|---|---|---|---|---|
| | | | | Hydroxypenta-2,4-dienoate | | | | | |
| 2-Methyl-1,3-cyclopentaion | 419 | C6H10 | NO* | | | 0 | 5956 | 0 | 905,75 |
| 2-Methyl-4-amino-5-hydroxymethylpyrimidine diphosphate | 420 | C6H11N3O7P2 | YES | | | 1354 | 2087 | 0 | 445,75 |
| 2-Methyl-1-propanol | 421 | C4H10O | YES | | | 144 | 2414 | 133 | 1617,5 |
| 2-Maleylacetate | 422 | C6H6O5 | YES | | | 0 | 4144 | 0 | 5072,5 |
| 2-Methyl-1,3-dioxolan | 423 | C7H12O4 | NO* | | | 0 | 3191 | 391 | 4272 |
| 2-Methylbenzyl alcohol | 424 | C8H10O | YES | | | 293 | 3772 | 0 | 777 |
| 2-Methylbenzo-1,3-dithiole | 425 | C8H8S2 | NO* | | | 0 | 3993 | 0 | 498,5 |
| 2-Methylbutanoyl-CoA | 426 | C26H44N7O17P3S | YES | | | 15 | 67 | 28 | 3768,75 |
| S-Glutaryldihydrolipoamide | 427 | C13H23NO4S2 | YES | | | 2494 | 24 | 4749 | 5021,5 |
| cis-2-Methylaconitate | 428 | C7H8O6 | YES | | | 0 | 5080 | 1119 | 9162,75 |
| 2-Methylcitrate | 429 | C7H10O7 | YES | | | 0 | 0 | 0 | 6805,75 |
| 2-C-Methyl-D-erythritol 4-phosphate | 430 | C5H13O7P | YES | | | 0 | 0 | 0 | 9032,75 |
| 2-C-Methyl-D-erythritol 2,4-cyclodiphosphate | 431 | C5H12O9P2 | YES | | | 53 | 4011 | 0 | 5047 |
| 1-Methoxy-2-phenylbenzene | 432 | C13H12O | NO | 2-Phenylanisole; o-Phenylanisole; 2-Methoxybiphenyl; o-Phenyl anisole | 149836 | 181 | 4820 | 45 | 2890 |
| 1-Hydroxymethylnaphthalene | 433 | C11H10O | YES | | | 170 | 2381 | 0 | 4491,25 |

| | | | | | | | | | |
|---|---|---|---|---|---|---|---|---|---|
| o-Methylphenol | 434 | C7H8O | YES | | | 458 | 2216 | 0 | 4397,25 |
| 2-Methylpyridine | 435 | C6H7N | YES | | | 26 | 3764 | 1409 | 1076,75 |
| [(2S)-Oxiran-2-yl]methyl acetate | 436 | C5H8O3 | NO | S-Glycidyl acetate | 49834467 | 20 | 0 | 0 | 4497 |
| 2'-O-Methyllicodione | 437 | C16H14O5 | YES | | | 0 | 0 | 0 | 12455,8 |
| 2-Methylnaphthalene | 438 | C11H10 | YES | | | 0 | 7699 | 1148 | 12950,5 |
| 2-Hexaprenyl-3-methyl-6-methoxy-1,4-benzoquinone | 439 | C38H56O3 | YES | | | 218 | 3958 | 254 | 5339,25 |
| 2-Naphthoic acid | 440 | C11H8O2 | YES | | | 145 | 2360 | 297 | 2061,5 |
| 2-Nitroaniline | 441 | C6H6N2O2 | NO | o-Nitroaniline; o-Nitraniline; Benzenamine, 2-nitroaniline; 2-Nitrobenzenamine; o-Aminonitrobenzene; 2-Aminonitrobenzene | 149956 | 287 | 0 | 0 | 8386,25 |
| 2-Nitronaphthalene | 442 | C10H7NO2 | NO | 2-Nitronaphthalene, beta-Nitronaphthalene | 154696 | 25 | 0 | 0 | 7127,25 |
| 1-Methyl-2-nitrobenzene | 443 | C7H7NO2 | NO | | 149954 | 0 | 0 | 0 | 3443 |
| 2-Methylserine | 444 | C4H9NO3 | YES | | | 0 | 0 | 0 | 4341,25 |
| 2-Octaprenyl-3-methyl-5-hydroxy-6-methoxy-1,4-benzoquinone | 445 | C48H72O4 | YES | | | 267 | 0 | 0 | 8734,25 |
| 2-Octaprenyl-6-methoxyphenol | 446 | C47H72O2 | YES | | | 18 | 3362 | 382 | 5262,25 |
| Nonadecan-2-ol | 447 | C19H40O | NO | 2-Nonadecanol | 10516268 | 0 | 0 | 246 | 4486,75 |
| 2-Nonadecanoylglycerol | 448 | C4H7O4(C19H38O2) | YES* | 2-Acylglycerol | | 0 | 0 | 0 | 4563 |
| 1-Nonadecanoyl-sn-glycero-phosphocholine | 449 | C9H20NO7P(C19H38O2) | YES* | 1-Acyl-sn-glycerol-3-phosphocholine | | 0 | 43 | 0 | 1734,5 |
| 2-Nonanoylglycerol | 450 | C4H7O4(C9H | YES* | 2-Acylglycerol | | 110 | 0 | 829 | 2319,25 |

| | | | | | | | | | |
|---|---|---|---|---|---|---|---|---|---|
| | | 18O2) | | | | | | | |
| Nonan-2-ol | 451 | C9H20O | NO | 2-Nonanol; 1-Methyl-1-octanol | 155688 | 274 | 0 | 5012 | 1200,75 |
| 2-Nonaprenyl-6-hydroxyphenol | 452 | C52H80O2 | NO* | | | 0 | 0 | 0 | 8566,25 |
| 2-Nonaprenylphenol | 453 | C52H80O | NO* | | | 139 | 0 | 0 | 4073,75 |
| 2-Oxobutanoate | 454 | C4H6O3 | YES | | | 343 | 0 | 0 | 1314,25 |
| 2-Octadecanoylglycerol | 455 | C4H7O4(C18O36O2) | YES* | 2-Acylglycerol | | 457 | 563 | 0 | 5698 |
| Octadecan-2-ol | 456 | C18H38O | NO | 2-Octanol | 728308 | 0 | 3588 | 4872 | 12275,8 |
| 2-Octanoylglycerol | 457 | C4H7O4(C8H16O2) | YES* | 2-Acylglycerol, 2-Glyceride, 2-Monoacylglycerol | | 0 | 0 | 1586 | 4000 |
| 2-Octanol | 458 | C8H18O | NO | Octan-2-ol; Capryl alcohol; 2-Hydroxyoctane; 2-Octyl alcohol | 173324 | 19 | 0 | 1898 | 776 |
| 2-Phenylphenol | 459 | C12H10O | YES | | | 11 | 123 | 0 | 1630,75 |
| 2-Thiocyanatoethylbenzene | 460 | C9H9NS | NO | | 32928505 | 0 | 0 | 0 | 1505,25 |
| 1-Monobutanoyl-glycerol | 461 | C4H7O4(C4H8O2) | YES* | 1-Monoacylglycerol | | 145 | 0 | 0 | 5312,5 |
| 2-Octaprenyl-6-methoxy-1,4-benzoquinol | 462 | C47H70O3 | NO* | | | 78 | 0 | 2698 | 1471,75 |
| 2-Octaprenyl-3-methyl-5-hydroxy-6-methoxy-1,4-benzoquinol | 463 | C47H71NO2 | NO* | | | 0 | 0 | 0 | 4758,25 |
| 2-Octaprenyl-3-methyl-6-methoxy-1,4-benzoquinol | 464 | C46H70O2 | NO* | | | 0 | 2178 | 0 | 1601 |
| 2-Octaprenyl-6-methoxyphenol | 465 | C47H72O2 | YES | | | 0 | 0 | 0 | 769,25 |
| 2-Oxooctadecanoic | 466 | C18H34O3 | YES | | | 0 | 3592 | 2739 | 5068,25 |

| | | | | | | | | | |
|---|---|---|---|---|---|---|---|---|---|
| acid | | | | | | | | | |
| 2-Octaprenylphenol | 467 | C46H70O | YES | | | 0 | 0 | 0 | 657,75 |
| 3-Phenylbutan-1-ol | 468 | C10H14O | NO | | 6841864 | 0 | 2642 | 3484 | 1425,75 |
| 2-Phospho-4-(cytidine 5'-diphospho)-2-C-methyl-D-erythritol | 469 | C14H26N3O17P3 | YES | | | 1567 | 0 | 0 | 2689,5 |
| Pentan-2-ol | 470 | C5H12O | NO | 2-Pentanol; Methyl butanol | 198476 | 0 | 0 | 0 | 7808,75 |
| D-Glycerate 2-phosphate | 471 | C3H7O7P | YES | | | 0 | 2492 | 143 | 3277,75 |
| 2-Propylglycerol | 472 | C4H7O4(C3H6O2) | YES* | 2-Acylglycerol | | 0 | 1112 | 0 | 4701,75 |
| 2-Phosphoglycolate | 473 | C2H5O6P | YES | | | 10251 | 4811 | 0 | 963,5 |
| 2-Pyrone-4,6-dicarboxylate | 474 | C7H4O6 | YES | | | 5130 | 0 | 0 | 1822,5 |
| 2-Octaprenyl-6-methoxy-1,4-benzoquinone | 475 | C47H70O3 | YES | | | 2164 | 0 | 0 | 1263 |
| Propionaldehyde | 476 | C3H6O | YES | | | 0 | 4174 | 0 | 2444,5 |
| 2-Propen-1-ol | 477 | C3H6O | YES | | | 19 | 1178 | 0 | 6895,5 |
| 4,5-Dihydro-1H-pyrazole | 478 | C3H6N2 | NO | 2-Pyrazoline | 209064 | 0 | 9429 | 0 | 4818,25 |
| Pyran-2-one | 479 | C5H4O2 | NO | 2H-Pyran-2-one; alpha-Pyrone; Coumalin; 2-Pyranone; pyran-2-one; 2-Pyrone | 24434777 | 0 | 1751 | 25361 | 2886,5 |
| 2,3-Dihydro-1H-pyrrole | 480 | C4H7N | NO | 2-pyrroline; Delta(2)-pyrroline | 8147300 | 0 | 776 | 0 | 2154 |
| (2S)-Flavanone | 481 | C15H12O2 | YES | | | 0 | 4804 | 0 | 46,75 |
| (2S)-Flavan-4-ol | 482 | C15H14O2 | YES | | | 0 | 257 | 0 | 185,75 |
| (1R,6R)-2-Succinyl-6-hydroxy-2,4-cyclohexadiene-1-carboxylate | 483 | C11H12O6 | YES | | | 211 | 0 | 0 | 3310 |
| 2-S-isocapryloyl-3R- | 484 | C13H22O4 | NO* | | | 0 | 47 | 0 | 2926 |

| | | | | | | | | | |
|---|---|---|---|---|---|---|---|---|---|
| hydroxymethyl-gamma-butyrolactone | | | | | | | | | |
| 2-Octaprenyl-6-hydroxyphenol | 485 | C46H70O2 | YES | | | 865 | 0 | 3427 | 8232,75 |
| 2-Tetradecanoylglycerol | 486 | C4H7O4(C14H28O2) | YES* | 2-Acylglycerol | | 29 | 0 | 0 | 5513,75 |
| Tetracosan-2-ol | 487 | C24H50O | NO | 2-Tetracosanol | 31665760 | 0 | 4001 | 1214 | 1873,75 |
| Tetradecan-2-ol | 488 | C14H30O | NO | 2-Tetradecanol; sec-Tetradecyl alcohol | 163625 | 0 | 2680 | 0 | 907,75 |
| 2-Methyl-4,5-dihydro-1,3-thiazole | 489 | C4H7NS | NO | 2-Methylthiazoline; Methyl-2-thiazoline; 2-Methyl-2-thiazoline | 50051884 | 0 | 0 | 0 | 5536,75 |
| Undecan-2-ol | 490 | C11H24O | NO | | 158767 | 41 | 0 | 0 | 538,75 |
| 1-Chloro-2-[1-(4-chlorophenyl)ethenyl] benzene | 491 | C14H10Cl2 | YES | | | 0 | 30439 | 0 | 217,75 |
| L-Dicysteine | 492 | C6H12N2O4S2 | YES | | | 0 | 0 | 0 | 5472,75 |
| 3,4,5-Trichlorophenol | 493 | C6H3Cl3O | NO | 3,4,5-Trichlorophenol; 3,4,5.Trichloro-phenol | 49855590 | 0 | 0 | 0 | 5908,5 |
| 3',4',5-Trihydroxy-3,7-dimethoxyflavone | 494 | C17H14O7 | YES | | | 0 | 4333 | 42 | 3791,5 |
| 3,4-Dichloroaniline | 495 | C6H5Cl2N | YES | | | 0 | 4441 | 0 | 322,75 |
| 1,3-Dichloro-2-phenylbenzene | 496 | C18H13Cl2 | NO | | 21402031 | 0 | 0 | 0 | 2616 |
| 3,4-Dihydroxymandelate | 497 | C8H8O5 | YES | | | 0 | 328 | 0 | 5646 |
| 3,4-Dihydroxyphenylacetaldehyde | 498 | C8H8O3 | YES | Protocatechualdehyde | | 0 | 0 | 0 | 6370,75 |
| 3,4-Dihydroxy-trans-cinnamate | 499 | C9H8O4 | YES | trans-Caffeate | | 0 | 2002 | 0 | 293,75 |
| 3,4-Dihydroxybenzoate | 500 | C7H6O4 | YES | Protocatechuate | | 1377 | 0 | 0 | 514,75 |
| Cystathionine | 501 | C7H14N2O4S | YES | | | 1331 | 0 | 3 | 5237,25 |

| | | | | | | | | | |
|---|---|---|---|---|---|---|---|---|---|
| 3,4-Dihydroxyphenylacetate | 502 | C8H8O4 | YES | | | 111 | 2157 | 0 | 2627,5 |
| 3,4-Dichlorobut-1-ene | 503 | C4H6Cl2 | NO | 3,4-Dichlorobut-1-ene; 3,4-dichloro-1-butene | 156320 | 0 | 3635 | 0 | 543 |
| 3,4-Dihydro-1 (2H) naphthalene | 504 | C14H16O2 | NO* | | | 30 | 1449 | 5 | 7328,25 |
| 3-(3,4-Dihydroxyphenyl)lactate | 505 | C9H10O5 | YES | | | 112 | 0 | 4263 | 707,75 |
| 3-(4-Hydroxyphenyl)pyruvate | 506 | C9H8O4 | YES | | | 4110 | 1232 | 0 | 393,5 |
| 3,5-Dihydroxybenzoic acid | 507 | C7H6O4 | NO | | 50044369 | 0 | 8475 | 0 | 7215,5 |
| 1,3-Dichloro-5-phenylbenzene | 508 | C12H8Cl2 | NO | 3,5-Dichlorobiphenyl | 17395356 | 0 | 5028 | 0 | 185 |
| 2-Fluorobenzoate | 509 | C7H5FO2 | YES | | | 660 | 4353 | 0 | 5037,25 |
| 3-Amino-1,2,4-triazole | 510 | C2H4N4 | YES | | | 0 | 2406 | 0 | 3567 |
| 3-Amino-5-deoxyfructose 6-phosphate | 511 | C6H14O5NP | NO* | | | 0 | 0 | 0 | 140,75 |
| 3-Fluorobenzoate | 512 | C7H5FO2 | YES | | | 0 | 5118 | 0 | 3633,75 |
| Monochlorobenzene | 513 | C6H5Cl | YES | | | 0 | 6087 | 50 | 203,5 |
| 3-Carboxy-2-hydroxy-4-methylpentanoate | 514 | C7H12O5 | YES | | | 0 | 964 | 27 | 54,5 |
| 3-Carboxy-3-hydroxy-4-methylpentanoate | 515 | C7H12O5 | YES | | | 53 | 4963 | 0 | 236 |
| 3-Chloro-4-hydroxy-phenylacetate | 516 | C9H10O3 | NO* | | | 40 | 0 | 0 | 3717,75 |
| 3-Carboxy-4-methyl-2-oxopentanoate | 517 | C8H12O5 | YES | | | 0 | 1068 | 0 | 2803,5 |
| 4-Chlorobenzoate | 518 | C7H5ClO2 | YES | | | 435 | 3123 | 0 | 1621 |

| | | | | | | | | | |
|---|---|---|---|---|---|---|---|---|---|
| 1-Chloro-3-phenylbenzene | 519 | C12H9Cl | NO | m-Chlorobiphenyl; 3-Chlorodiphenyl; o-Chlorobiphenyl | 49894213 | 0 | 44 | 0 | 326,75 |
| 1-Chloro-3-ethenylbenzene | 520 | C8H7Cl | NO | 3-Chlorostyrene; m-chloro-styrene; m-chlorostyrene | 159522 | 0 | 4130 | 213 | 3002,75 |
| 3-Chlorotoluene | 521 | C7H7Cl | NO | m-Tolyl chloride; 1-chloro-3-methylbenzene | 151041 | 6 | 0 | 7723 | 55,5 |
| 3-Carbamoyloxymethyl cephem | 522 | C10H9N3O6S (CH3)2 | YES | | | 320 | 2133 | 0 | 2292,5 |
| 3-Deoxy-arabino-heptulonate 7-phosphate | 523 | C7H13O10P | YES | | | 0 | 6256 | 4 | 4636,75 |
| 3-Dehydrocarnitine | 524 | C7H14NO3 | YES | | | 0 | 1366 | 260 | 4484,25 |
| 3-Dehydro-L-gulonate 6-phosphate | 525 | C6H11O10P | YES | | | 0 | 50 | 0 | 6629,75 |
| 3-Dehydroquinate | 526 | C7H10O6 | YES | | | 586 | 3565 | 0 | 3924,5 |
| 3-Dehydro-L-gulonate | 527 | C6H10O7 | YES | | | 0 | 1900 | 84 | 44,5 |
| 1,4,2-Oxathiazole | 528 | C2H3NOS | NO | | 21865251 | 0 | 0 | 0 | 70,75 |
| 2,3-Dichloropentane | 529 | C5H10Cl2 | NO | 2,3-Dichloroprop-1-ene; 2,3-Dichloropropylene; 2,3-Dichloropropene; 2,3-dichloro-propene | 679908 | 0 | 0 | 0 | 126,5 |
| 3-Dehydro-L-threonate | 530 | C4H6O5 | YES | | | 0 | 47 | 0 | 2153,75 |
| 3-Demethylubiquinone-9 | 531 | C53H80O4 | YES | | | 69 | 6634 | 8 | 3859,25 |
| 3-Ethylcatechol | 532 | C8H10O2 | YES | | | 0 | 0 | 0 | 521,25 |
| 3H-Dioxazole | 533 | C2H3NO2 | NO | | 21931167 | 17 | 3111 | 0 | 5421,75 |
| 3H-1,2-Dithiole | 534 | C5H8 | NO* | | | 0 | 1638 | 0 | 500,25 |
| 3-Dehydroshikimate | 535 | C7H8O5 | YES | | | 4191 | 0 | 0 | 3582 |
| 2H-1,3-Dithiole | 536 | C5H8 | NO* | | | 0 | 1685 | 23 | 333,5 |
| 3-Hydroxy-2- | 537 | C9H6O3 | NO | 3-Hydroxycoumarin; 3- | 156990 | 0 | 0 | 0 | 0 |

| | | | | | | | | | |
|---|---|---|---|---|---|---|---|---|---|
| benzopyrone | | | | Hydroxy-2-benzopyrone | | | | | |
| (2S,3S)-3-Hydroxy-2-methylbutanoyl-CoA | 538 | C26H44N7O18P3S | YES | | | 0 | 5451 | 0 | 315,75 |
| 3-Hydroxy-2-methylpropanoate | 539 | C4H8O3 | YES | | | 0 | 13847 | 19 | 8295,75 |
| 3-Hydroxyanthranilate | 540 | C7H7NO3 | YES | | | 6519 | 0 | 3531 | 1554,75 |
| 3-Hydroxy-4-methylanthranilate | 541 | C8H9NO3 | YES | | | 0 | 1205 | 0 | 2297,75 |
| 3-Hydroxyoxolan-2-one | 542 | C8H12O6 | NO | | 32721888 | 30 | 0 | 0 | 457,75 |
| (2S)-2,4-Diamino-3-hydroxy-4-oxobutanoic acid | 543 | C4H8N2O4 | NO | 3-Hydroxyasparagine; beta-Hydroxyasparagine | 729721 | 3 | 2527 | 0 | 810 |
| (3S)-3-Hydroxydecanoyl-CoA | 544 | C24H39N7O18P3S(C10H20O2) | YES* | (3S)-3-Hydroxyacyl-CoA; (S)-3-Hydroxyacyl-CoA | | 295 | 2773 | 0 | 635,5 |
| 3-Hydroxybenzaldehyde | 545 | C7H6O2 | YES | | | 0 | 0 | 0 | 245,25 |
| 2-Hydroxybenzyl alcohol | 546 | C7H8O2 | YES | | | 0 | 6160 | 10164 | 473,5 |
| 3-Hydroxybenzyl amine | 547 | C13H7NO3 | NO | | 10464491 | 0 | 7310 | 0 | 491,25 |
| 3-Hydroxybenzoate | 548 | C7H6O3 | YES | | | 0 | 20159 | 0 | 3518 |
| 3-Hydroxycinnamate | 549 | C9H8O3 | YES | | | 0 | 1949 | 0 | 4324,75 |
| (3S)-3-Hydroxyhexacosyl-CoA | 550 | C24H39N7O18P3S(C6H12O2) | YES* | (3S)-3-Hydroxyacyl-CoA; (S)-3-Hydroxyacyl-CoA | | 0 | 2904 | 0 | 534,25 |
| 3-Hydroxybutan-2-one | 551 | C4H8O2 | YES | | | 29 | 2283 | 0 | 6397,5 |
| (3S)-3-Hydroxydodecanoyl-CoA | 552 | C24H39N7O18P3S(C12O24O2) | YES* | (3S)-3-Hydroxyacyl-CoA; (S)-3-Hydroxyacyl-CoA | | 0 | 0 | 52 | 266,75 |
| (3S)-3-Hydroxyhexadecanoy | 553 | C24H39N7O18P3S(C16H32 | YES* | (3S)-3-Hydroxyacyl-CoA; (S)-3-Hydroxyacyl-CoA | | 237 | 2176 | 7551 | 105 |

| | | | | | | | | | |
|---|---|---|---|---|---|---|---|---|---|
| l-CoA | | O2) | | | | | | | |
| 3H-Indole | 554 | C8H7N | NO | | 3884887 | 0 | 9385 | 1545 | 3818,25 |
| 3-Hydroxymethyl ceph-3-em-4-carboxylate | 555 | C9H8N2O5S(CH3)2 | YES | | | 0 | 2064 | 9499 | 9765 |
| (3R)-3-Hydroxytetradecanoic acid | 556 | C14H28O3 | NO | 3-Hydroxy-myristic acid; 3-Hydroxy-tetradecanoic acid | 7887606 | 0 | 0 | 0 | 551,75 |
| 3-Hydroxy-cis,cis-muconate | 557 | C6H6O5 | YES | | | 98 | 4206 | 0 | 8665 |
| (3S)-3-Hydroxyoctadecanoyl-CoA | 558 | C24H39N7O18P3S(C18H36O2) | YES* | (3S)-3-Hydroxyacyl-CoA; (S)-3-Hydroxyacyl-CoA | | 0 | 4659 | 0 | 207,5 |
| (3R)-3-Hydroxypalmitoyl-CoA | 559 | C24H39N7O18P3S(C16H32O2) | YES* | (3S)-3-Hydroxyacyl-CoA; (S)-3-Hydroxyacyl-CoA | | 0 | 2192 | 0 | 297,75 |
| 3-Hydroxyhexadecanoic acid | 560 | C16H32O3 | NO | 3-Hydroxy-hexadecanoic acid | 7982567 | 3 | 6355 | 0 | 345,75 |
| 3-Hexaprenyl-4,5-dihydroxybenzoate | 561 | C37H54O4 | YES | | | 136 | 4220 | 0 | 3770,5 |
| 3-Hydroxypimeloyl-CoA | 562 | C28H46N7O20P3S | YES | | | 1587 | 7 | 0 | 218,75 |
| 3-Hexaprenyl-4-hydroxy-5-methoxybenzoate | 563 | C38H56O4 | YES | | | 18 | 0 | 0 | 714,75 |
| 3-Hydroxypropanal | 564 | C3H6O2 | YES | | | 207 | 4487 | 3649 | 1385,25 |
| 3-(3-Hydroxy-phenyl)-propanoic acid | 565 | C9H10O3 | YES | | | 0 | 0 | 0 | 3823,75 |
| 3H-Pyrrole | 566 | C4H5N | NO | | 11533618 | 2 | 0 | 1461 | 1747,25 |
| (3S)-3-Hydroxytetradecanoyl-CoA | 567 | C24H39N7O18P3S(C14H28O2) | YES* | (3S)-3-Hydroxyacyl-CoA; (S)-3-Hydroxyacyl-CoA | | 2507 | 4292 | 0 | 262 |
| 3-beta-Hydroxysterol | 568 | C18H27O2(C1 | YES* | 3beta-Hydroxysterol ester | | 54 | 0 | 0 | 4045,25 |

| | | | | | | | | | |
|---|---|---|---|---|---|---|---|---|---|
| dodecanoate | | 0H20O2) | | | | | | | |
| 3-beta-Hydroxysterol hexanoate | 569 | C18H27O2(C6H12O2) | YES* | 3beta-Hydroxysterol ester | | 0 | 3961 | 0 | 711,5 |
| 3-beta-Hydroxysterol hexadecanoate | 570 | C18H27O2(C16H32O2) | YES* | 3beta-Hydroxysterol ester | | 0 | 5407 | 0 | 578,5 |
| 3-Hydroxypropanoate | 571 | C3H6O3 | YES | | | 3253 | 5 | 0 | 5281,75 |
| 3-beta-Hydroxysterol oleate | 572 | C18H27O2(C18H34O2) | YES* | 3beta-Hydroxysterol ester | | 0 | 5544 | 0 | 491,25 |
| 3-beta-Hydroxysterol tetradecanoate | 573 | C18H27O2(C14H28O2) | YES* | 3beta-Hydroxysterol ester | | 0 | 6401 | 223 | 5238,75 |
| 3-beta-Hydroxysterol decanoate | 574 | C18H27O2(C10H20O2) | YES* | 3beta-Hydroxysterol ester | | 0 | 1354 | 34948 | 221,75 |
| 3-beta-Hydroxysterol octanoate | 575 | C18H27O2(C8H16O2) | YES* | 3beta-Hydroxysterol ester | | 0 | 3683 | 0 | 6629 |
| (S)-3-(Imidazol-5-yl)lactate | 576 | C6H8N2O3 | YES | | | 0 | 47 | 8381 | 6978 |
| 3-(Imidazol-5-yl)pyruvate | 577 | C6H6N2O3 | YES | | | 0 | 3291 | 6475 | 579,5 |
| 3-Methylbutanol | 578 | C5H12O | YES | | | 0 | 6927 | 4309 | 10070,5 |
| 3-Indoleacetonitrile | 579 | C10H8N2 | YES | | | 0 | 0 | 0 | 3704,5 |
| 3-Methylbutan-2-ol | 580 | C5H12O | NO | sec-Isoamyl alcohol; Methylisopropylcarbinol; 2-Methyl-3-butanol; 3-Methylbutan-2-ol; Isopropylmethylcarbinol; 1,2-Dimethylpropanol; 1,2-Dimethyl-1-propanol | 155035 | 0 | 0 | 0 | 7369,5 |
| 3-Methoxy-4-hydroxy-trans-cinnamate | 581 | C10H10O4 | YES | | | 0 | 0 | 97 | 12535,3 |
| Glutaconyl-CoA | 582 | C26H39N7O19P3S | YES | see http://umbbd.ahc.umn.edu | | 6040 | 4657 | 2924 | 2071,5 |
| 3-Methylbenzyl alcohol | 583 | C8H10O | YES | | | 43 | 6755 | 0 | 1640,5 |
| 3-Methyl-1-benzothiophene | 584 | C9H8S | NO | 3-Methylbenzothiophene; 3-Methylthioindene; 3- | 216054 | 159 | 7072 | 0 | 455 |

| | | | | | | | | | |
|---|---|---|---|---|---|---|---|---|---|
| | | | | Methylthianaphthene; 3-Methylbenzylthiophene | | | | | |
| C1-(3-Indolyl)-glycerol 3-phosphate | 585 | C11H14NO6P | YES | | | 1674 | 0 | 0 | 3063,25 |
| 3-Methylbutanoyl-CoA | 586 | C26H44N7O17P3S | YES | | | 0 | 90 | 509 | 5260,25 |
| 3-Hydroxytoluene | 587 | C7H8O | YES | m-cresol; 3-Cresol | | 88 | 0 | 0 | 344,5 |
| 3-(Methoxycarbonyl)pent-2-enedioate | 588 | C7H8O6 | YES | | | 0 | 128 | 0 | 1272,25 |
| 3-Methylcatechol | 589 | C7H8O2 | YES | | | 2367 | 4414 | 0 | 287 |
| trans-2-(4-Nitrophenyl)-3-phenyloxirane | 590 | C14H11NO3 | YES | | | 18 | 18215 | 1262 | 1527,25 |
| 3-Methyl-2-oxobutanoate | 591 | C5H8O3 | YES | | | 1656 | 101 | 447 | 5294 |
| 3-Methylquinoline | 592 | C10H9N | NO | 3-Methyl-quinoline; beta-Methylquinoline | 155238 | 0 | 242 | 0 | 3481,25 |
| (S)-3-Methyl-2-oxopentanoate | 593 | C6H10O3 | YES | | | 2856 | 107 | 4072 | 6258 |
| 3-Methoxy-5,7,3',4'-tetrahydroxyflavone | 594 | C16H12O7 | YES | | | 36 | 487 | 0 | 5001,25 |
| 3-Oxodecanoyl-CoA | 595 | C31H52N7O18P3S | YES | | | 1492 | 0 | 0 | 7384,75 |
| 3-Nitrobenzoic acid | 596 | C7H5NO4 | NO | m-Nitrobenzoic acid | 151659 | 0 | 0 | 0 | 649 |
| (3-Nitrophenyl)methanol | 597 | C7H7NO3 | NO | 3-Nitrobenzyl alcohol; m-Nitrobenzyl alcohol; m-nitro-benzyl alcohol; (3-nitrophenyl)methanol | 211433 | 61 | 3271 | 74 | 1299 |
| 2,4,6-Trinitrotoluene | 598 | C7H5N3O6 | YES | | | 101 | 0 | 32 | 487,5 |
| 3-Oxobutyryl-CoA | 599 | C24H37N7O18P3S(C4H8O2) | YES* | 3-Oxoacyl-CoA | | 0 | 0 | 39226 | 8122 |
| 4-Nitroanilide | 600 | C6H7N2O2 | YES | | | 129 | 10 | 0 | 233,5 |
| 3-Oxododecanoyl- | 601 | C33H56N7O1 | YES | | | 24 | 0 | 0 | 7618,25 |

| | | | | | | | | | |
|---|---|---|---|---|---|---|---|---|---|
| CoA | | 8P3S | | | | | | | |
| 3-Oxohexanoyl-CoA | 602 | C27H44N7O18P3S | YES | | | 4359 | 57 | 0 | 554,5 |
| 3-Oxohexadecanoyl-CoA | 603 | C37H64N7O18P3S | YES | | | 193 | 4344 | 0 | 1473,25 |
| 3-Oxooctadecanoyl-CoA | 604 | C39H68N7O18P3S | YES | | | 116 | 0 | 62 | 4789,75 |
| 3-Oxohexacosyl-CoA | 605 | C24H37N7O18P3S(C6H12O2) | YES* | 3-Oxoacyl-CoA | | 0 | 0 | 21990 | 4649,25 |
| 3-Oxooctanoyl-CoA | 606 | C29H48N7O18P3S | YES | | | 5010 | 0 | 0 | 4475,5 |
| 3-Oxopropanoate | 607 | C3H4O3 | YES | | | 0 | 3664 | 0 | 705 |
| 2,3-Didehydro-pimeloyl-CoA | 608 | C28H44N7O19P3S | NO | | | 0 | 0 | 4189 | 523,75 |
| 3-Octaprenyl-4-hydroxybenzoate | 609 | C47H70O3 | YES | | | 2371 | 0 | 0 | 5033,5 |
| 3-Oxotetradecanoyl-CoA | 610 | C35H60N7O18P3S | YES | | | 2 | 0 | 0 | 1065,75 |
| 3-Oxoadipyl-CoA | 611 | C27H42N7O20P3S | YES | | | 0 | 4728 | 4436 | 664,25 |
| 3-Oxoadipate | 612 | C6H8O5 | YES | | | 76 | 0 | 3364 | 7552,75 |
| 3-Oxoadipate enol-lactone | 613 | C6H6O4 | YES | | | 242 | 0 | 1997 | 746,5 |
| 3-Phospho-D-glycerate | 614 | C3H7O7P | YES | | | 66 | 0 | 2382 | 1414,75 |
| 3-Phenylpropan-1-ol | 615 | C9H12O | NO | 3-phenylpropan-1-ol; Benzenepropanol; Phenylpropanol; 3-Phenylpropanol | 173270 | 0 | 3689 | 0 | 247,75 |
| 3-Phosphohydroxypyruvate | 616 | C3H5O7P | YES | | | 1058 | 0 | 0 | 781,5 |
| 3-Phenyl-propionic acid | 617 | C9H10O2 | YES | | | 0 | 0 | 0 | 287,25 |

| | | | | | | | | | |
|---|---|---|---|---|---|---|---|---|---|
| 5-O-(1-Carboxyvinyl)-3-phosphoshikimate | 618 | C10H13O10P | YES | | | 1244 | 3821 | 0 | 2153 |
| 3-Phosphonooxypyruvate | 619 | C3H5O7P | YES | | | 0 | 0 | 0 | 162 |
| 2,5-dihydro-1H-pyrrole | 620 | C4H7N | NO | 3-Pyrroline; 2,5-Dihydropyrrole | 208129 | 144 | 0 | 1666 | 725,75 |
| (3R)-3-Hydroxy-butanoyl-CoA | 621 | C24H39N7O18P3S(C4H8O2) | YES* | (3R)-3-Hydroxyacyl-CoA | | 0 | 3241 | 6581 | 4312,5 |
| 3-Sulfocatechol | 622 | C6H6O5S | YES | | | 0 | 56 | 0 | 2649 |
| 4,4'-Dichlorobiphenyl | 623 | C12H8Cl2 | YES | | | 25 | 686 | 0 | 5333,5 |
| 3-Thiaoctanoyl-CoA | 624 | C29H51N7O18P3S | NO* | | | 140 | 0 | 0 | 7873 |
| (3S)-3-Hydroxy-butanoyl-CoA | 625 | C24H39N7O18P3S(C4H8O2) | YES* | (3S)-3-Hydroxyacyl-CoA | | 1399 | 3236 | 0 | 75,5 |
| 4-Acetamidobutanoate | 626 | C6H11NO3 | YES | | | 10 | 48 | 0 | 2356,25 |
| (4S,5S)-4,5-Dihydroxy-2,6-dioxohexanoate | 627 | C6H8O6 | YES | | | 0 | 721 | 566 | 4257,5 |
| (4S)-4,6-Dihydroxy-2,5-dioxohexanoate | 628 | C6H8O6 | YES | | | 0 | 4 | 4559 | 893,25 |
| Adenine | 629 | C5H5N5 | YES | | | 1303 | 2445 | 8044 | 0 |
| 4-Aminobutanoate | 630 | C4H9NO2 | YES | | | 1276 | 25 | 0 | 119,25 |
| 4-Aminobutanal | 631 | C4H9NO | YES | | | 570 | 91 | 0 | 218,25 |
| 4-Aminobenzoate | 632 | C7H7NO2 | YES | | | 1180 | 1901 | 0 | 9,5 |
| 4-Amino-4-deoxychorismate | 633 | C10H11NO5 | YES | | | 0 | 3674 | 0 | 363,75 |
| 4-Amino-5-hydroxymethyl-2-methylpyrimidine | 634 | C6H9N3O | YES | | | 1709 | 2943 | 0 | 54,75 |
| 4-Amino-2-methyl-5-phosphomethylpyrimi | 635 | C6H10N3O4P | YES | | | 1496 | 2545 | 10 | 321,25 |

| | | | | | | | | | |
|---|---|---|---|---|---|---|---|---|---|
| dine | | | | | | | | | |
| 4-Bromobutan-1-ol | 636 | C4H9BrO | NO | 4-Bromo-1-butanol | 695081 | 0 | 0 | 0 | 178,25 |
| 4-Bromobenzoic acid | 637 | C7H5BrO2 | NO | 4-Bromobenzoic acid; p-Carboxybromobenzene; P-Bromobenzoic acid; p-Bromobenzenecarboxylic acid | 154771 | 45 | 0 | 0 | 446,25 |
| Bromobenzene-2,3-dihydrodiol | 638 | C6H7BrO2 | YES | | | 0 | 7165 | 0 | 151 |
| 4-Bromocatechol | 639 | C6H5BrO2 | YES | | | 33 | 3548 | 0 | 273 |
| 4-Bromophenol | 640 | C6H5BrO | YES | | | 18 | 0 | 0 | 3237,75 |
| 1-Bromo-4-methylbenzene | 641 | C7H7Br | NO | p-Bromotoluene; p-Tolyl bromide | 150899 | 0 | 2832 | 0 | 3318,25 |
| 4-Dimethylaminophenol | 642 | C8H11NO | NO | 4-(Dimethylamino)phenol | 213793 | 0 | 0 | 21 | 36 |
| 4-Carboxy-2-hydroxyhexa-2,4-dienedioate | 643 | C7H6O7 | YES | | | 0 | 4548 | 0 | 872,75 |
| 4-Carboxy-2-hydroxy-cis,cis-muconate | 644 | C7H6O7 | YES | | | 75 | 0 | 0 | 59 |
| 4-Carboxy-2-hydroxymuconate semialdehyde | 645 | C7H6O7 | YES | | | 0 | 3822 | 0 | 264,25 |
| 4-(Cytidine 5'-diphospho)-2-C-methyl-D-erythritol | 646 | C14H25N3O14P2 | YES | | | 1899 | 2317 | 19382 | 3250,5 |
| 4-Carboxy-2-oxo-4-pentenoate | 647 | C6H6O5 | YES | | | 0 | 4559 | 0 | 120,5 |
| 4-Carboxy-4-hydroxy-2-oxoadipate | 648 | C7H8O8 | YES | | | 0 | 3053 | 110 | 2412,25 |
| 5,7,4'-Trihydroxy-3'-methoxyflavone | 649 | C16H12O6 | YES | | | 366 | 2927 | 5487 | 2879,75 |
| 4-Chlorobenzoyl-CoA | 650 | C28H39ClN7O17P3S | YES | | | 0 | 3944 | 0 | 1736 |

| | | | | | | | | | |
|---|---|---|---|---|---|---|---|---|---|
| 1-Chloro-4-ethenylbenzene | 651 | C8H7Cl | NO | 4-Chlorostyrene; Parachlorostyrene; p-Chloro-styrene | 157386 | 0 | 1637 | 0 | 2850,5 |
| 4-Dehydropantoate | 652 | C6H10O4 | YES | | | 2062 | 5436 | 4281 | 370,25 |
| 4-Chlorotoluene | 653 | C7H7Cl | YES | 4-Chlorotoluene; p-Tolyl chloride; P-Chlorotoluene; 1-Chloro-4-methylbenzene; 1-Methyl-4-chlorobenzene; 4-Chloro-1-methylbenzene; p-Chlorophenyl bromide | 474350 | 0 | 2396 | 0 | 2391,75 |
| 5-Amino-6-(ribosylamino)-2,4-(1H,3H)-pyrimidinedione 5'-phosphate | 654 | C9H15N4O9P | YES | | | 158 | 3737 | 0 | 385,5 |
| 4-Ethyl phenol | 655 | C8H10O | YES | | | 0 | 0 | 0 | 112,5 |
| 1-Fluoro-4-methylbenzene | 656 | C7H7F | NO | 4-Fluorotoluene; p-fluoro-toluene; p-Fluoromethylbenzene | 152819 | 0 | 3906 | 0 | 375 |
| Diazepine | 657 | C5H6N2 | NO | | 166734 | 3 | 5556 | 9351 | 95 |
| 4-Hydroxy-2-oxopentanoate | 658 | C5H8O4 | YES | | | 133 | 2940 | 0 | 3610,25 |
| 4-Hydroxy-2-oxoglutarate | 659 | C5H6O6 | YES | | | 876 | 0 | 0 | 3576 |
| 4-Hydroxymandelate | 660 | C8H8O4 | YES | | | 29 | 0 | 0 | 1497,25 |
| Biphenyl | 661 | C12H10 | YES | | | 0 | 1320 | 5754 | 2059,5 |
| 4-Hydroxy-2-butynal | 662 | C4H4O2 | YES | | | 0 | 42 | 0 | 1015,25 |
| 3-Methyl-1,3-oxazinane | 663 | C5H11NO | NO | 2H-1,3-oxazine | 641502 | 2 | 3 | 2435 | 1713,5 |
| 6-Methyl-3,4-dihydro-2H-1,4-benzoxazine | 664 | C9H11NO | NO | | 649409 | 0 | 4849 | 0 | 330,75 |
| 4-Phenyl-1,3-oxazinane-2-thione | 665 | C10H11NOS | NO | 4-Phenyltetrahydro-1,3-oxazine-2-thione | 5369431 | 0 | 4451 | 0 | 174,5 |
| 1,3-Benzoxazine-2,4- | 666 | C8H5NO3 | NO | | 16258 | 0 | 0 | 0 | 63,25 |

| | | | | | | | | | |
|---|---|---|---|---|---|---|---|---|---|
| dione | | | | | | | | | |
| 4-Hydroxy-3-methoxybenzoate | 667 | C8H8O4 | YES | | | 0 | 3250 | 8 | 278 |
| 4-Hydroxy-3-methoxycinnamic acid | 668 | C10H10O4 | YES | | | 17 | 2335 | 0 | 1589,75 |
| (1S,4S)-4-Hydroxy-3-oxocyclohexane-1-carboxylate | 669 | C7H10O4 | YES | | | 74 | 3497 | 0 | 1507,75 |
| 4-Hydroxy-4-methyl-2-oxoglutarate | 670 | C6H8O6 | YES | | | 0 | 59 | 0 | 1474 |
| 4-(Hydroxymethyl)phenol | 671 | C7H8O2 | NO | p-Methylolphenol; 4-Methylolphenol; 4-hydroxybenzyl alcohol | 155483 | 0 | 0 | 1191 | 5744,25 |
| 4-Hydroxybenzoylformate | 672 | C8H6O4 | YES | | | 2088 | 3509 | 0 | 254 |
| 4-(Aminomethyl)phenol | 673 | C7H9NO | NO | 4-Hydroxybenzylamine; para-Hydroxybenzylamine | 674693 | 273 | 0 | 75 | 590,5 |
| 4-Hydroxybenzoyl-CoA | 674 | C28H40N7O18P3S | YES | | | 0 | 4 | 0 | 3669,25 |
| 4-Hydroxybenzaldehyde | 675 | C7H6O2 | YES | | | 50 | 996 | 0 | 201,5 |
| 3-Methoxy-4-hydroxymandelate | 676 | C9H10O5 | YES | | | 251 | 0 | 5320 | 6873,5 |
| 4-Hydroxybutanoate | 677 | C4H8O3 | YES | | | 0 | 5396 | 0 | 1940,25 |
| 4-Hydroxycinnamate | 678 | C9H8O3 | YES | | | 0 | 1971 | 0 | 475,5 |
| 4-Hydroxy-L-threonine | 679 | C4H9NO4 | YES | | | 0 | 1234 | 0 | 2831,75 |
| D-4-Hydroxyphenylglycine | 680 | C8H9NO3 | YES | | | 196 | 0 | 0 | 500,75 |
| 3-Hydroxybenzyl alcohol | 681 | C7H8O2 | YES | | | 0 | 0 | 0 | 589 |
| 3-Hydroxy-2- | 682 | C9H6O2S | NO | see | | 0 | 1042 | 0 | 7161,25 |

| | | | | | | | | | |
|---|---|---|---|---|---|---|---|---|---|
| formylbenzothiophene | | | | http://umbbd.ahc.umn.edu | | | | | |
| L-4-Hydroxyglutamate semialdehyde | 683 | C5H9NO4 | YES | | | 1550 | 2874 | 2516 | 429,75 |
| 4-Methyl-2-oxopentanoate | 684 | C6H10O3 | YES | | | 1396 | 3645 | 0 | 273,75 |
| 4-Hydroxybenzoate | 685 | C7H6O3 | YES | | | 1862 | 3128 | 0 | 752,75 |
| 3-Hydroxy-3-methyl-2-oxopentanoate | 686 | C6H10O4 | YES | | | 817 | 0 | 0 | 2968 |
| 4'-Methoxy-5,7-dihydroxyflavone | 687 | C16H12O5 | YES | | | 0 | 1900 | 0 | 5862,5 |
| p-Methylbenzaldehyde | 688 | C8H8O | YES | | | 0 | 371 | 0 | 1261,25 |
| 4-Methyl benzyl alcohol | 689 | C8H10O | YES | | | 0 | 3798 | 0 | 2522,75 |
| 3-Methylbutanal | 690 | C5H10O | YES | | | 0 | 0 | 0 | 2716,75 |
| 4-Methylcatechol | 691 | C7H8O2 | YES | | | 0 | 0 | 307 | 563,5 |
| Methyl-cyclohexane | 692 | C7H14 | NO | Cyclohexylmethane; Hexahydrotoluene; Hexahydroxytoluene; Methylcyclohexane | 151074 | 0 | 6306 | 4536 | 6288,75 |
| 4-Methylcyclohexan-1-ol | 693 | C7H14O | NO | Hexahydro-p-cresol; 4-Methylcylcohexanol | 24884646 | 277 | 0 | 0 | 1373,75 |
| 4-Methylcyclohexan-1-one | 694 | C7H12O | NO | 4-Methylcyclohexanone; 4-Methyl-cyclohexanone | 154829 | 67 | 2138 | 2470 | 1938,75 |
| 4-Methylcycloheptan-1-ol | 695 | C8H16O | NO | | 38645936 | 0 | 4020 | 0 | 8363,75 |
| 4-Methylphenol | 696 | C7H8O | YES | | | 39 | 0 | 77 | 984 |
| 5-(2-Hydroxyethyl)-4-methylthiazole | 697 | C6H9NOS | YES | | | 0 | 375 | 115 | 6720,25 |
| 4'-O-Methylisoflavone | 698 | C16H12O3 | YES | | | 0 | 0 | 0 | 7235,75 |
| 2-Hydroxy-6-oxo-octa-2,4-dienoate | 699 | C8H10O4 | YES | | | 0 | 4772 | 0 | 9288 |
| 4-Methyl-5-(2- | 700 | C6H10NO4PS | YES | | | 840 | 0 | 3012 | 8100,75 |

| | | | | | | | | | |
|---|---|---|---|---|---|---|---|---|---|
| phosphoethyl)-thiazole | | | | | | | | | |
| 4-Methylumbelliferyl glucuronide | 701 | C16H16O9 | YES | | | 0 | 2364 | 1848 | 472 |
| 4-Nitrophenol | 702 | C6H5NO3 | YES | | | 85 | 0 | 161 | 8318,5 |
| 4-Nitroaniline | 703 | C6H6N2O2 | YES | | | 31 | 1889 | 0 | 6508,25 |
| 4-Nitrotoluene | 704 | C7H7NO2 | YES | | | 0 | 3702 | 0 | 827,75 |
| 4-Oxalocrotonate | 705 | C6H6O5 | YES | | | 0 | 0 | 0 | 1291,5 |
| 4-Oxalomesaconate | 706 | C7H6O7 | YES | | | 63 | 0 | 0 | 4030,75 |
| 4-Oxoproline | 707 | C5H7NO3 | YES | | | 468 | 7892 | 0 | 4385,25 |
| 4-Phospho-L-aspartate | 708 | C4H8NO7P | YES | | | 1904 | 0 | 4402 | 1522 |
| 4-Phospho-D-erythronate | 709 | C4H9O8P | YES | | | 612 | 3659 | 0 | 389,75 |
| D-4'-Phosphopantothenate | 710 | C9H18NO8P | YES | | | 75 | 0 | 0 | 1848 |
| (R)-4'-Phosphopantothenoyl-L-cysteine | 711 | C12H23N2O9PS | YES | | | 2121 | 5448 | 0 | 1730,25 |
| Pyran-4-one | 712 | C5H4O2 | NO | Pyran-4-one; gamma-Pyrone | 24434902 | 0 | 1081 | 4825 | 5342,5 |
| 5-O-Methyl-myo-inositol | 713 | C7H14O6 | YES | | | 0 | 5641 | 0 | 3056,25 |
| 4-(1-D-Ribitylamino)-5-aminouracil | 714 | C9H16N4O6 | YES | | | 1966 | 4583 | 0 | 2168,75 |
| 4-Chlorobiphenyl | 715 | C12H9Cl | YES | | | 3071 | 0 | 0 | 0 |
| 5,7,4'-Trihydroxyflavone | 716 | C15H10O5 | YES | | | 0 | 0 | 3743 | 3767,5 |
| 2-Aminodecanoic acid | 717 | C10H21NO2 | NO | 2-Aminocapric acid | 24846092 | 9 | 1074 | 0 | 599 |
| 1-(5-Phospho-D-ribosyl)-5-amino-4-imidazolecarboxylate | 718 | C9H14N3O9P | YES | | | 0 | 0 | 10127 | 1538 |
| 2,2'-Dihydroxybiphenyl | 719 | C12H10O2 | YES | | | 108 | 430 | 0 | 285,75 |

| | | | | | | | | | |
|---|---|---|---|---|---|---|---|---|---|
| 5-Amino-4-oxopentanoate | 720 | C5H9NO3 | YES | | | 0 | 1051 | 0 | 1686 |
| 5-Amino-6-(5'-phosphoribitylamino)uracil | 721 | C9H17N4O9P | YES | | | 645 | 3890 | 0 | 0 |
| 5-Amino-6-(5'-phosphoribosylamino)uracil | 722 | C9H15N4O9P | YES | | | 1010 | 84 | 37 | 3678,25 |
| 5-Amino-4-oxooctanoate | 723 | C8H12O7N2 | NO* | | | 234 | 77 | 9399 | 230,5 |
| 2-Aminohexadecanoic acid | 724 | C16H33NO2 | NO | | 7521675 | 53 | 2933 | 4126 | 212,75 |
| Benzoyl acetyl-CoA | 725 | C30H42N7O18P3S | YES | | | 786 | 1189 | 700 | 0 |
| 5-Benzamido-2-benzyl-4-oxopentanoate | 726 | C12H16ClNO3 | NO* | | | 0 | 2164 | 0 | 419,25 |
| 5-Bromo-4-chloro-3-indoyl phosphate | 727 | C8H4BrClNO4P | NO* | | | 0 | 8169 | 5912 | 160,75 |
| 5-carboxyamino-1-(5-phospho-D-ribosyl)imidazole | 728 | C9H14N3O9P | YES | | | 0 | 0 | 6363 | 428,5 |
| 5-Dehydro-D-fructose | 729 | C6H10O6 | YES | | | 0 | 2293 | 2931 | 1214,75 |
| 2-Dehydro-D-glucono-1,5-lactone | 730 | C6H8O6 | YES | | | 302 | 3429 | 0 | 101 |
| 5-Dehydro-D-gluconate | 731 | C6H10O7 | YES | | | 436 | 7434 | 0 | 3980,5 |
| 5'-Fluoro-5'-deoxyadenosine | 732 | C10H12FN5O3 | NO | | 8148180 | 0 | 4887 | 0 | 19,25 |
| 5-Dehydro-4-deoxy-D-glucarate | 733 | C6H8O7 | YES | | | 604 | 3892 | 0 | 4517,75 |
| 1-(5'-Phosphoribosyl)-5-formamido-4-imidazolecarboxamid | 734 | C10H15N4O9P | YES | | | 0 | 4055 | 0 | 488,5 |

| | | | | | | | | | |
|---|---|---|---|---|---|---|---|---|---|
| e | | | | | | | | | |
| 5H-1,2,5-Oxathiazole | 735 | C2H3NOS | NO | | 21221324 | 0 | 7510 | 133 | 8333,5 |
| 5-Hydroxyfuranocoumarin | 736 | C11H6O4 | YES | | | 0 | 0 | 0 | 247,75 |
| S-Methyl-5-thio-5-deoxy-D-ribose 1-phosphate | 737 | C6H13O7S2 | YES | | | 153 | 66 | 0 | 1258,25 |
| 2,5-Diamino-6-(5'-triphosphoryl-3',4'-trihydroxy-2'-oxopentyl)-amino-4-oxopyrimidine | 738 | C9H18N5O14P3 | YES | | | 7859 | 4056 | 0 | 837,25 |
| S-Methyl-5-thio-D-ribose 1-phosphate | 739 | C6H13O7PS | YES | | | 1923 | 2083 | 523 | 6916 |
| S-Methyl-5-thio-D-ribulose 1-phosphate | 740 | C6H13O7PS | YES | | | 0 | 99 | 13 | 530,75 |
| 8-Methoxyfuranocoumarin | 741 | C12H8O4 | YES | | | 0 | 6235 | 0 | 6102,75 |
| 5-Methoxyfuranocoumarin | 742 | C12H8O4 | YES | | | 65 | 2058 | 0 | 4972,75 |
| 5-Methylthioadenosine | 743 | C11H15N5O3S | YES | | | 0 | 0 | 0 | 7933,75 |
| 5-Methyltetrahydrofolate | 744 | C20H25N7O6 | YES | | | 0 | 0 | 0 | 8701,25 |
| 6-Hydroxyhexanoate | 745 | C6H12O3 | YES | | | 2666 | 0 | 416 | 7926,25 |
| 5-Nitro-1,2,4-triazol-3-one | 746 | C2H2N4O3 | NO* | | | 31 | 0 | 0 | 6050 |
| 2(3H)-oxazolone | 747 | C3H3NO2 | NO | 3H-1,3-oxazol-2-one | 186114 | 54 | 0 | 0 | 12087,8 |
| N1-(5-Phospho-alpha-D-ribosyl)-5,6-dimethylbenzimidazol | 748 | C14H19N2O7P | YES | | | 2861 | 0 | 0 | 1594,5 |

| | | | | | | | | | |
|---|---|---|---|---|---|---|---|---|---|
| e | | | | | | | | | |
| 4-Pyridoxolactone | 749 | C8H7NO3 | YES | | | 141 | 0 | 3560 | 8331,25 |
| 6-Acetyl-beta-D-galactoside | 750 | C8H14O7 | YES | | | 0 | 6228 | 0 | 0 |
| 1,2-Ditetradecanoyl-sn-glycerol | 751 | C5H6O5(C28H56O4) | YES* | 1,2-Diacyl-sn-glycerol | | 501 | 3248 | 8541 | 0 |
| 6-Chloro-3-indolyl-beta-D-galactoside | 752 | C14H15ClNO6 | NO* | | | 0 | 0 | 0 | 4555,25 |
| 6-Chloro-3-indolyl-beta-D-glucoside | 753 | C14H15ClNO6 | NO* | | | 0 | 2357 | 0 | 1702,75 |
| 6-S-Acetyldihydrolipoamide | 754 | C10H19NO2S2 | YES | | | 4053 | 6854 | 22 | 4367 |
| Melibiose | 755 | C12H22O11 | YES | | | 0 | 5216 | 0 | 327 |
| 1,2-Didodecanoyl-sn-glycerol | 756 | C5H6O5(C24H48O4) | YES* | 1,2-Diacyl-glycerol | | 11201 | 0 | 0 | 2586,5 |
| 2,3,6-trimethyl-1,3-oxazinane | 757 | C7H15NO | NO | 2H-1,3-Oxazine | 575121 | 11 | 0 | 0 | 486,75 |
| 5-Methylthio-D-ribose | 758 | C6H12O4S | YES | | | 0 | 262 | 0 | 239,5 |
| 6-Hydroxymethyl 7,8-dihydropterin | 759 | C7H9N5O2 | NO* | | | 0 | 0 | 22560 | 5858,25 |
| 6-Hydroxymethyl 7,8-dihydropterin pyrophosphate | 760 | C7H9N5O9P2 | NO* | | | 0 | 5997 | 7853 | 2104,5 |
| 6-Oxohexanoate | 761 | C6H10O3 | YES | | | 166 | 0 | 0 | 966 |
| 6-Phospho-2-dehydro-D-gluconate | 762 | C6H11O10P | YES | | | 12994 | 2713 | 355 | 6658,5 |
| 2'-Aminobiphenyl-2,3-diol | 763 | C12H11NO2 | YES | | | 0 | 61 | 5562 | 6456 |
| 6-Phospho-D-glucono-1,5-lactone | 764 | C6H11O9P | YES | | | 2695 | 119 | 103 | 7124,75 |
| 6-Pyruvoyltetrahydropterin | 765 | C9H11N5O3 | YES | | | 168 | 13 | 0 | 2017,75 |
| 5-Hydroxy-L- | 766 | C11H12N2O3 | YES | | | 2361 | 5480 | 0 | 3271,25 |

| | | | | | | | | | |
|---|---|---|---|---|---|---|---|---|---|
| tryptophan | | | | | | | | | |
| 1-Deaza-5-azapurine | 767 | C5H6N4 | NO | | 466064 | 82 | 2294 | 0 | 950 |
| 1-Deazapurine | 768 | C6H5N3 | NO | 4-Azabenzimidazole; 1H-imidazo[4,5-b]pyridine; 3,4-Diazaindole | 67504 | 0 | 4554 | 0 | 511,5 |
| 6-Phospho-D-gluconate | 769 | C6H13O10P | YES | | | 1649 | 1304 | 2836 | 5922,25 |
| 7H-pyrrolo[2,3-d]pyrimidine | 770 | C7H6N2 | NO* | | | 73 | 0 | 0 | 1137,25 |
| Xanthen-9-one | 771 | C13H8O2 | NO | Xanthone; Genicide; 9H-Xanthen-9-one; 9-Xanthenone; Xanthenone; 9-Oxoxanthene; Benzophenone oxide; Xanthen-9-one; 9-Xanthone; Dibenzo-gamma-pyrone | 150040 | 0 | 0 | 0 | 2200 |
| 8-Hydroxyfuranocoumarin | 772 | C11H6O4 | YES | | | 0 | 0 | 0 | 274,25 |
| Uridine 5'-triphosphate | 773 | C9H15N2O15P3 | YES | | | 1104 | 3700 | 0 | 1125,75 |
| 1,2-Anthracenediol | 774 | C14H12O2 | NO | See htpp://umbdd.ahc.umn.edu | | 0 | 9149 | 0 | 325,75 |
| 9,10-Dihydrophenanthrene | 775 | C14H12 | NO | 9,10-Dihydro-phenanthrene | 156411 | 1568 | 0 | 0 | 100,75 |
| Acyclic-7-deazapurine | 776 | C11H13N5O4 | NO | | 451812 | 0 | 2782 | 0 | 5084 |
| 9-Oxononanoic acid | 777 | C9H16O3 | NO | 9-Ketononanoic acid; 9-oxo-nonanoic acid | 217925 | 0 | 0 | 0 | 2579,75 |
| 8-Amino-7-oxononanoate | 778 | C9H17NO3 | YES | | | 1464 | 0 | 0 | 0 |
| Adenosine 5'-phosphosulfate | 779 | C10H14N5O10PS | YES | | | 30 | 0 | 0 | 3325,25 |

| | | | | | | | | | |
|---|---|---|---|---|---|---|---|---|---|
| 3-Acetoacetyl-CoA | 780 | C25H40N7O18P3S | YES | | | 931 | 3650 | 3425 | 5253,5 |
| Tri-L-Alanine | 781 | C9H17N3O4 | NO | Ala-Ala-Ala; D-Ala-Ala-Ala | 24891479 | 138 | 4520 | 0 | 2,25 |
| Aminoacetaldehyde | 782 | C2H5NO | YES | | | 0 | 3330 | 0 | 176,5 |
| (S)-5-Oxo-2,5-dihydrofuran-2-acetate | 783 | C6H6O4 | YES | | | 771 | 0 | 47541 | 3434,75 |
| 2,2-Azino-bis-3-ethylbenzthiazoline-6-sulfonic acid | 784 | C18H24N6O6S6 | NO* | ABTS | | 182 | 1826 | 6033 | 0 |
| Acetyl-CoA | 785 | C23H38N7O17P3S | YES | | | 1277 | 2797 | 2619 | 5833 |
| N-Acetyl-D-glucosamine | 786 | C8H15NO6 | YES | | | 1269 | 0 | 4338 | 3495 |
| Acetoacetate | 787 | C4H6O3 | YES | | | 640 | 6597 | 6842 | 612 |
| Acetaldehyde | 788 | C2H4O | YES | | | 1275 | 6670 | 6141 | 5115,5 |
| O-Acetylcarnitine | 789 | C9H18NO4 | YES | | | 0 | 3213 | 0 | 165,25 |
| Acetate | 790 | C2H4O2 | YES | | | 4 | 2983 | 0 | 4761,25 |
| Acenaphthalen-1-ol | 791 | C12H10O | NO* | | | 270 | 5166 | 10642 | 4090,75 |
| N-Acetyl-D-mannosamine 6-phosphate | 792 | C8H16NO9P | YES | | | 1009 | 1497 | 2533 | 7799,5 |
| Naphthyl-2-methyl-succinyl-CoA | 793 | C36H48N7O19P3S | YES | | | 2645 | 0 | 2139 | 0 |
| 1-Phenylethanone | 794 | C8H8O | YES | | | 0 | 7016 | 0 | 374,5 |
| Acetoin | 795 | C4H8O2 | YES | | | 0 | 5013 | 0 | 4082,5 |
| N-Acetyl-L-glutamyl 5-phosphate | 796 | C7H12NO8P | YES | | | 1073 | 0 | 0 | 3517,25 |
| N-Acetyl-L-glutamate 5-semialdehyde | 797 | C7H11NO4 | YES | | | 2511 | 4349 | 5280 | 23 |
| Adenosylcobalamin 5'-phosphate | 798 | C72H101CoN18O20P2 | NO | Cobamamide 5'-phosphate | 766614 | 0 | 3561 | 92 | 4605,25 |
| 2-Hydroxy-3-oxoadipate | 799 | C6H8O6 | YES | | | 145 | 2494 | 0 | 4336,5 |
| N-Acetyl-D- | 800 | C8H16NO9P | YES | | | 2639 | 17 | 0 | 3516,5 |

| | | | | | | | | | |
|---|---|---|---|---|---|---|---|---|---|
| glucosamine 6-phosphate | | | | | | | | | |
| N-Acetyl-L-glutamate | 801 | C7H11NO5 | YES | | | 4199 | 8707 | 13 | 0 |
| 2-Amino-3-oxoadipate | 802 | C6H9NO5 | YES | | | 424 | 11604 | 5356 | 449 |
| Acenaphthalene | 803 | C12H8 | NO | Cyclopenta(de)naphthalene | 152352 | 0 | 46 | 4395 | 1285 |
| N-Acetylneuraminate | 804 | C11H19NO9 | YES | | | 0 | 0 | 3249 | 2516,75 |
| 3-Carboxy-2-hydroxy-4-methylpentanoate | 805 | C7H12O5 | YES | | | 0 | 2476 | 0 | 121,25 |
| cis-Aconitate | 806 | C6H6O6 | YES | | | 1498 | 4000 | 0 | 37 |
| N2-Acetyl-L-ornithine | 807 | C7H14N2O3 | YES | | | 179 | 2623 | 0 | 1742,75 |
| N5-Propyl-L-ornithine ester | 808 | C6H10N2O3(C3H6O2) | YES | | | 0 | 0 | 9281 | 4098 |
| Acryloyl-CoA | 809 | C24H38N7O17P3S | YES | | | 0 | 0 | 6303 | 0 |
| Acridine | 810 | C13H9N | NO | 9-Azaanthracene | 152406 | 0 | 2413 | 6027 | 2565,75 |
| Acrolein | 811 | C3H4O | YES | | | 0 | 1217 | 5665 | 114,75 |
| O-Acetyl-L-serine | 812 | C5H9NO4 | YES | | | 0 | 2868 | 0 | 0 |
| Adenosine | 813 | C10H13N5O4 | YES | | | 1231 | 5089 | 7634 | 3923,5 |
| N-Acetyl-D-glucosamine 1-phosphate | 814 | C8H16NO9P | YES | | | 5847 | 3703 | 0 | 255 |
| N-Acetyl-D-mannosamine | 815 | C8H15NO6 | YES | | | 728 | 4085 | 0 | 451,75 |
| Acetyl phosphate | 816 | C2H5O5P | YES | | | 7268 | 0 | 0 | 3689,75 |
| Agmatine | 817 | C5H14N4 | YES | | | 3451 | 0 | 0 | 104 |
| 6-Aminopurine | 818 | C5H5N5 | YES | | | 0 | 3921 | 0 | 147,25 |
| 4-Aminobiphenyl | 819 | C12H11N | YES | | | 44 | 0 | 0 | 6090,5 |
| Adenosyl cobinamide | 820 | C58H84CoN16O11 | YES | | | 0 | 2886 | 9109 | 0 |
| Adenosyl cobinamide phosphate | 821 | C58H85CoN16O14P | YES | | | 0 | 3165 | 15357 | 3765,75 |
| Adenosylcobalamin | 822 | C72H100CoN | YES | | | 2 | 1605 | 24 | 101,25 |

| | | | | | | | | | |
|---|---|---|---|---|---|---|---|---|---|
| | | 18O17P | | | | | | | |
| ADP | 823 | C10H15N5O10P2 | YES | | | 1075 | 1353 | 0 | 2685,75 |
| ADP-L-glycero-D-manno-heptose | 824 | C17H27N5O16P2 | YES | | | 0 | 1134 | 199 | 6744,75 |
| ADP-D-glycero-D-manno-heptose | 825 | C17H27N5O16P2 | YES | | | 0 | 2916 | 0 | 5129,5 |
| Alginate | 826 | (C120H160O120) | YES | | | 89 | 1623 | 0 | 2841,25 |
| 2-amino-4,6-dimethoxypyrimidine | 827 | C6H8N3O2 | NO* | ADPM | | 0 | 0 | 812 | 2642,5 |
| ADP-ribose | 828 | C15H23N5O14P2 | YES | | | 1695 | 2076 | 0 | 1604,5 |
| Hexanedinitrile | 829 | C6H8N2 | NO | Adiponitrile; Adipyldinitrile | 151257 | 0 | 0 | 0 | 0 |
| Adenosine-GDP-cobinamide | 830 | C68H97CoN21O21P2 | YES | | | 9 | 3837 | 0 | 193,25 |
| Undecane | 831 | C11H24 | NO | | 157541 | 332 | 6345 | 69 | 3913,75 |
| 3,4-Dihydroxymandelaldehyde | 832 | C8H8O4 | YES | | | 2228 | 3029 | 10202 | 132,25 |
| 1-Acetyl-sn-glycero-3-phosphoethanolamine | 833 | C6H13NO7P(C2H4O2) | YES* | 1-Acyl-sn-glycero-3-phosphoethanolamine | | 0 | 0 | 0 | 0 |
| S-Adenosyl-L-homocysteine | 834 | C14H20N6O5S | YES | | | 1907 | 2519 | 0 | 2111 |
| 2-Amino-4-hydroxy-6-(erythro-1,2,3-trihydroxypropyl)dihydropteridine triphosphate | 835 | C9H16N5O13P3 | YES | | | 1533 | 15 | 11 | 5446 |
| 1-Acetyl-sn-glycero-3-phosphocholine | 836 | C9H20NO7P(C2H4O2) | YES* | 1-Acyl-sn-glycero-3-phosphocholine | | 0 | 1266 | 1 | 345,25 |
| 1-Acetyl-sn-glycerol 3-phosphate | 837 | C4H8O7P(C2H4O2) | YES* | 1-Acyl-sn-glycerol 3-phosphate | | 0 | 3022 | 0 | 3694,75 |
| Acetylhistone | 838 | C7H16NO2 | YES | | | 0 | 1920 | 0 | 0 |

| | | | | | | | | | |
|---|---|---|---|---|---|---|---|---|---|
| D-Alanine | 839 | C3H7NO2 | YES | | | 42 | 1638 | 27 | 6979 |
| 1-(5'-Phosphoribosyl)-5-aminoimidazole | 840 | C8H14N3O7P | YES | | | 78 | 1631 | 0 | 3698,75 |
| 5-Amino-1-(5-phospho-D-ribosyl)imidazole-4-carboxamide | 841 | C9H15N4O8P | YES | | | 0 | 16 | 0 | 5565,5 |
| D-Alanyl-D-alanine | 842 | C6H12N2O3 | YES | | | 0 | 3081 | 11775 | 6,5 |
| (S)-2-Acetolactate | 843 | C5H8O4 | YES | | | 8748 | 2816 | 0 | 4054,25 |
| 2-Ketoglutaric acid | 844 | C5H6O5 | YES | | | 624 | 6999 | 0 | 153 |
| N-Acetyl-beta-alanine | 845 | C5H9NO3 | YES | | | 0 | 6388 | 0 | 3524 |
| L-Alanine | 846 | C3H7NO2 | YES | | | 799 | 0 | 580 | 3311,25 |
| Alanyllactate | 847 | C6H11NO4 | NO | (2R)-2-[(2R)-2-Aminopropanoyl]oxypropanoic acid; Alanyl-lactate; D-Alanyl-D-lactate | 702946 | 40 | 4490 | 4404 | 179,25 |
| N-Methyl-L-alanine | 848 | C4H9NO2 | YES | | | 2027 | 2638 | 49 | 88,5 |
| 5-Aminolevulinate | 849 | C5H9NO3 | YES | | | 0 | 2257 | 770 | 1480,75 |
| Alditol | 850 | C2H5O2(C4H8O2) | YES | | | 0 | 3419 | 0 | 0 |
| D-Gluconic acid | 851 | C7H12O7 | YES | | | 1959 | 4471 | 0 | 46,5 |
| D-Aldose | 852 | C6H12O6 | YES | | | 1500 | 1312 | 63 | 2726,75 |
| ADP-glucose | 853 | C16H25N5O15P2 | YES | | | 2210 | 1593 | 0 | 153,25 |
| Allyl alcohol | 854 | C3H6O | YES | | | 0 | 1571 | 1473 | 1774,5 |
| D-Allose | 855 | C6H12O6 | YES | | | 0 | 0 | 0 | 2519,25 |
| Allophanate | 856 | C2H4N2O3 | YES | | | 0 | 2495 | 0 | 0 |
| S-Adenosyl-L-methionine | 857 | C15H22N6O5S | YES | | | 1199 | 3291 | 0 | 197 |
| 5-Amino-4-imidazole carboxylate | 858 | C4H5N3O2 | YES | | | 2455 | 2781 | 0 | 170 |
| (S)-Allantoin | 859 | C4H6N4O3 | YES | | | 934 | 0 | 4805 | 1 |
| L-2-Aminoadipate 6-semialdehyde | 860 | C6H11NO3 | YES | | | 0 | 1772 | 0 | 1760,75 |

| | | | | | | | | | |
|---|---|---|---|---|---|---|---|---|---|
| Altrose | 861 | C6H12O6 | YES | | | 0 | 486 | 0 | 4544,25 |
| (R)-Allantoin | 862 | C4H6N4O3 | YES | | | 291 | 856 | 0 | 3676,25 |
| Aminofructose 6-phosphate | 863 | C6H14NO8P | YES | | | 10562 | 4460 | 0 | 2230 |
| 2-Aminomuconate 6-semialdehyde | 864 | C6H7NO3 | YES | | | 0 | 4473 | 0 | 143 |
| L-2-Aminoadipate | 865 | C6H11NO4 | YES | | | 0 | 11013 | 103 | 1338,25 |
| D-Altronate | 866 | C6H12O7 | YES | | | 1494 | 0 | 0 | 0 |
| Acetyl-maltose | 867 | C14H24O12 | YES | | | 0 | 82 | 0 | 11,25 |
| S-Adenosyl-L-threonine | 868 | C14H20N7O6 | NO* | | | 719 | 0 | 6547 | 0 |
| S-Adenosylmethioninamine | 869 | C14H23N6O3S | YES | | | 1276 | 2719 | 5076 | 900 |
| Allantoate | 870 | C4H8N4O4 | YES | | | 78 | 1512 | 37 | 528,75 |
| Methyl D-glucoside | 871 | C7H14O6 | NO | Methyl D-glucopyranoside; Methyl-D-Glucopyranoside | 668350 | 0 | 5504 | 1850 | 9095,5 |
| 5-Aminovaleric acid | 872 | C5H11NO2 | YES | | | 0 | 7517 | 0 | 291 |
| Aminoacetone | 873 | C3H7NO | YES | | | 902 | 1243 | 0 | 3736,25 |
| Aminoimidazole ribotide | 874 | C8H14N3O7P | YES | | | 1947 | 0 | 959 | 193 |
| 2-Aminophenol | 875 | C6H7NO | YES | | | 0 | 2625 | 0 | 351,5 |
| S-Adenosyl-4-methylthio-2-oxobutanoate | 876 | C15H20N5O6S | YES | | | 690 | 0 | 0 | 3438,5 |
| Naphthyl-2-methylene-succinyl-CoA | 877 | C36H46N7O19P3S | YES | | | 1561 | 5706 | 0 | 723,5 |
| Naphthyl-2-hydroxymethyl-succinyl CoA | 878 | C36H48N7O20P3S | YES | | | 2110 | 873 | 46 | 0 |
| 2-Aminomuconate | 879 | C6H7NO4 | YES | | | 0 | 6102 | 0 | 5504,25 |
| Amylose | 880 | (C120H200O100) | YES | | | 295 | 0 | 0 | 200,75 |

| | | | | | | | | | |
|---|---|---|---|---|---|---|---|---|---|
| Amylopectin | 881 | C30H52O26 | YES | | | 0 | 4025 | 0 | 80,5 |
| 5-Carboxymethyl-2-oxohex-3-ene-1,6-dioate | 882 | C8H5O7 | NO | see http://umbbd.ahc.umn.edu | | 157 | 0 | 0 | 1155 |
| AMP | 883 | C10H14N5O7P | YES | | | 89 | 3375 | 0 | 4789,5 |
| 1,3-Cyclohexanedione | 884 | C6H8O2 | YES | | | 151 | 3300 | 0 | 6738,75 |
| 3-Hydroxycyclohexanone | 885 | C6H10O2 | YES | | | 0 | 5206 | 0 | 1991,5 |
| Naphthyl-2-oxomethyl-succinyl-CoA | 886 | C36H46N7O20P3S | YES | | | 1275 | 5 | 173 | 64,75 |
| Naphthalen-1-yl hydrogen phosphate | 887 | C10H10NaO5P | NO | | 24897801 | 0 | 1747 | 0 | 122,5 |
| Anisole | 888 | C7H8O | YES | | | 0 | 6 | 0 | 238 |
| Naphthalen-1-yl acetate | 889 | C12H10O2 | NO | 1-Naphthyl acetate; a-Naphthyl acetate | 156600 | 73 | 2926 | 0 | 291,5 |
| Aniline | 890 | C6H7N | YES | | | 161 | 3694 | 0 | 236,5 |
| Anthranilate | 891 | C7H7NO2 | YES | | | 433 | 2364 | 0 | 93,25 |
| Anserine | 892 | C10H16N4O3 | YES | | | 0 | 1907 | 86 | 178 |
| Anthracene | 893 | C14H10 | YES | | | 94 | 0 | 0 | 105 |
| D-Arabinono-1,4-lactone | 894 | C5H8O5 | YES | | | 344 | 0 | 0 | 1006 |
| P1,P5-Bis(5'-adenosyl) pentaphosphate | 895 | C20H29N10O22P5 | YES | | | 107 | 0 | 0 | 285,25 |
| 3-O-L-Alanyl-1-O-phosphatidylglycerol | 896 | C10H19NO10P(CH3) | YES | | | 0 | 1032 | 0 | 0 |
| N-Acetylputrescine | 897 | C6H14N2O | YES | | | 2085 | 56 | 0 | 731 |
| 3-Aminopropanal | 898 | C3H7NO | YES | | | 68 | 4007 | 0 | 3497,5 |
| alpha-Aminopropiononitrile | 899 | C3H6N2 | YES | | | 3054 | 1877 | 0 | 104,25 |
| alpha-Pinene | 900 | C10H16 | YES | | | 4910 | 4584 | 0 | 4380 |

| | | | | | | | | | |
|---|---|---|---|---|---|---|---|---|---|
| 5'-Adenylyl sulfate | 901 | C10H14N5O10PS | YES | | | 7005 | 1 | 11614 | 360 |
| 3'-Phosphoadenylyl sulfate | 902 | C10H15N5O13P2S | YES | | | 0 | 0 | 0 | 5717,75 |
| L-Arabinono-1,5-lactone | 903 | C5H8O5 | YES | | | 41 | 1655 | 5519 | 0 |
| D-Arabinose 5-phosphate | 904 | C5H11O8P | YES | | | 0 | 4113 | 12075 | 101,25 |
| D-Arabinose | 905 | C5H10O5 | YES | | | 0 | 1184 | 227 | 55,25 |
| L-Arabinose | 906 | C5H10O5 | YES | | | 484 | 1814 | 0 | 259,5 |
| P1,P4-Bis(5'-adenosyl) tetraphosphate | 907 | C20H28N10O19P4 | YES | | | 790 | 6167 | 4628 | 241,25 |
| L-Arginine | 908 | C6H14N4O2 | YES | | | 1301 | 0 | 4594 | 373 |
| Biphenyl-2,3-diol | 909 | C12H10O2 | YES | | | 0 | 5772 | 0 | 6296,25 |
| Arbutin 6-phosphate | 910 | C12H17O10P | YES | | | 15 | 0 | 0 | 3831 |
| Arene oxide | 911 | C6H6O | YES | | | 467 | 4054 | 0 | 310,5 |
| Arachidic acid | 912 | C20H40O2 | YES | | | 0 | 13466 | 59 | 2676 |
| L-Arginine methyl ester | 913 | C6H13N4O2(CH3) | YES* | L-Arginine ester | | 120 | 5114 | 0 | 5597,5 |
| Arg-Pro-Lys-Pro-Gln-Gln-Phe-Phe-Gly-Leu-Met-NH2 | 914 | C75H108N20O13 | NO* | | | 0 | 1654 | 0 | 305 |
| Arginine p-nitroaniline | 915 | C12H18N6O3 | NO* | | | 248 | 3542 | 0 | 494 |
| L-Arginosuccinic acid | 916 | C10H18N4O6 | YES | | | 165 | 0 | 14411 | 5032 |
| L-Aspartate 4-semialdehyde | 917 | C4H7NO3 | YES | | | 4349 | 0 | 32 | 264,75 |
| Ascorbyl butyrate | 918 | C10H16O6 | NO* | | | 170 | 0 | 0 | 3041,75 |
| Ascorbyl propionate | 919 | C9H14O2 | NO* | | | 161 | 120 | 0 | 1445,75 |
| Ascorbyl acetate | 920 | C8H12O2 | NO* | | | 0 | 0 | 0 | 3105,5 |
| Ascorbyl hexanoate | 921 | C12H20O2 | NO* | | | 66 | 5401 | 0 | 628 |
| L-Asparagine | 922 | C4H8N2O3 | YES | | | 19012 | 0 | 0 | 278,5 |
| Ascorbyl octanoate | 923 | C14H24O2 | NO | | | 23 | 3953 | 0 | 3331,5 |
| Arylamine | 924 | C6H7N | YES | | | 0 | 121 | 0 | 144 |

| | | | | | | | | | |
|---|---|---|---|---|---|---|---|---|---|
| L-Aspartate | 925 | C4H7NO4 | YES | | | 1940 | 5035 | 6439 | 1320,25 |
| Atropine | 926 | C17H23NO3 | YES | | | 0 | 0 | 0 | 3305,5 |
| 2-Hydroxylminostilbene | 927 | C14H11NO | YES | | | 0 | 0 | 6173 | 0 |
| Atrazine | 928 | C8H14ClN5 | YES | | | 392 | 2407 | 0 | 99,75 |
| 4-Hydroxymethylsalicylate | 929 | C8H7O4 | NO | see http://umbbd.ahc.umn.edu | | 359 | 254 | 43 | 1900,25 |
| ATP | 930 | C10H16N5O13P3 | YES | | | 502 | 2410 | 0 | 1461,75 |
| Azetidine | 931 | C3H7N | NO | Azacyclobutane; Trimethylenimine | 10524348 | 852 | 2014 | 0 | 3729,5 |
| Aziridine | 932 | C2H5N | YES | | | 0 | 28 | 56 | 97,25 |
| 1-(5'-Phosphoribosyl)-5-amino-4-imidazolecarboxamide | 933 | C9H15N4O8P | YES | | | 0 | 2575 | 0 | 42,75 |
| gamma-Butyrobetainyl-CoA | 934 | C28H50N8O17P3S | NO | gamma-Butyrobetaine-CoA | 2734 | 118 | 0 | 8 | 3452 |
| Cycloheptaamylose | 935 | C42H70O35 | NO | beta-Cyclodextrin | 26758515 | 0 | 3595 | 0 | 62,5 |
| Butanoyldolichol | 936 | C21H35O2(C50H80) | YES* | Acyldolichol | | 0 | 3353 | 0 | 463,25 |
| Benzyl 2-methyl-3-oxobutanoate | 937 | C12H14O3 | YES | | | 0 | 3728 | 87 | 326,25 |
| Benzyl (2R,3S)-2-methyl-3-hydroxybutanoate | 938 | C12H16O3 | YES | | | 280 | 0 | 0 | 79,75 |
| 4-Phenylbutan-2-one | 939 | C10H12O | NO | | 6060129 | 144 | 1293 | 0 | 738 |
| Phenylmethyl benzoate | 940 | C14H12O2 | NO | Benzyl benzoate | 151586 | 363 | 1953 | 86 | 1589 |
| Benzoyl-CoA | 941 | C28H40N7O17P3S | YES | | | 16 | 4511 | 0 | 4801,25 |
| Phenylmethyl hexanoate | 942 | C13H18O2 | NO | Benzyl caproate; Benzyl hexanoate | 165943 | 132 | 0 | 0 | 114,25 |
| Phenylmethyl acetate | 943 | C9H10O2 | NO | Benzyl acetate | 151938 | 0 | 5325 | 19 | 3525,25 |

| | | | | | | | | | |
|---|---|---|---|---|---|---|---|---|---|
| Phenylmethyl propanoate | 944 | C10H12O2 | NO | Benzyl propionate; Benzyl propanoate | 173253 | 0 | 2849 | 8527 | 0 |
| (R,S)-Tetrahydrobenzylisoquinoline | 945 | C16H17N | YES | | | 0 | 0 | 3344 | 3189,25 |
| Benzimidazole | 946 | C7H6N2 | YES | | | 347 | 2991 | 0 | 355,75 |
| Benzyl alcohol | 947 | C7H8O | YES | | | 0 | 4617 | 0 | 154 |
| 2,1-Benzoxazole | 948 | C7H5NO | NO | Anthranil; 2,1-Benzisoxazole | 222079 | 354 | 0 | 0 | 5245 |
| Benzene | 949 | C6H6 | YES | | | 1287 | 3743 | 38 | 0 |
| Poly-beta-Hydroxybutyrate | 950 | (C40H60O20) | YES | | | 1713 | 0 | 56 | 52,75 |
| Benzamide | 951 | C7H7NO | YES | | | 1314 | 744 | 21 | 46,5 |
| 1,2-Benzophenanthrene | 952 | C18H12 | YES | | | 177 | 0 | 5382 | 303,75 |
| 1-Benzothiophene | 953 | C8H6S | NO | Benzothiofuran; Thianaphthene | 150269 | 0 | 1736 | 0 | 20,5 |
| 2-Benzothiophene | 954 | C8H6S | NO | Benzo(c)thiophene | 712306 | 0 | 1427 | 3150 | 146,75 |
| Betaine aldehyde | 955 | C5H12NO | YES | | | 1050 | 5859 | 9825 | 3417,5 |
| Benzocyclobutene | 956 | C8H6 | NO | Bicyclo[4.2.0]octa-1,3,5-triene | 655043 | 0 | 98 | 171 | 169,5 |
| Benzofuran | 957 | C8H6O | YES | | | 0 | 0 | 66 | 133,25 |
| 2,6-Dichlorobenzonitrile | 958 | C7H3Cl2N | YES | | | 462 | 4118 | 0 | 44 |
| Benzoate | 959 | C7H6O2 | YES | | | 1200 | 2444 | 8 | 72,25 |
| 2-Methylthiobenzothiazole | 960 | C8H7NS2 | YES | | | 77 | 0 | 0 | 3206,75 |
| 2H-Benzotriazole | 961 | C6H5N3 | NO | 1H-Benzotriazole; Azimidobenzene; Benzotriazole | 150268 | 0 | 2249 | 18 | 4544,5 |
| Benzoxazole | 962 | C7H5NO | NO | 1,3-Benzoxazole; 1-Oxa-3-azaindene | 152419 | 0 | 0 | 0 | 0 |
| Benzocycloheptene | 963 | C11H14 | NO | | 136842 | 0 | 29 | 68 | 131,5 |
| Betaine | 964 | C5H11NO2 | YES | | | 0 | 0 | 0 | 142 |

| | | | | | | | | | |
|---|---|---|---|---|---|---|---|---|---|
| Benzonitrile | 965 | C8H6O | YES | | | 177 | 0 | 0 | 6600 |
| N-Benzoylanthranilate | 966 | C14H11NO3 | YES | | | 0 | 0 | 0 | 60,75 |
| CDP-1,2-Dihexadecanoylglycerol | 967 | C14H19N3O15P2(C32H64O4) | YES* | CDP-1,2-diacylglycerol | | 1843 | 4546 | 0 | 1605,75 |
| Myrcene | 968 | C10H16 | YES | | | 60 | 0 | 0 | 2671,25 |
| Bromobenzene-2,3-oxide | 969 | C6H5BrO | YES | | | 580 | 5182 | 685 | 6262,5 |
| Nicofuranose | 970 | C30H24N4O10 | NO | | 519112 | 211 | 0 | 23 | 0 |
| Bromobenzene-3,4-oxide | 971 | C6H5BrO | YES | | | 0 | 2315 | 0 | 2509,5 |
| 2-Bromomaleylacetate | 972 | C6H5BrO5 | YES | | | 79 | 2727 | 7499 | 2598,25 |
| m-Bromobenzotrifluoride | 973 | C7H4BrF3 | NO | 1-Bromo-3-(trifluoromethyl)benzene; m-Bromobenzotrifluoride; | 153036 | 12 | 0 | 10811 | 341,75 |
| Butanoyl-CoA | 974 | C25H42N7O17P3S | YES | | | 0 | 1933 | 0 | 2996 |
| 4-Aminophenol | 975 | C6H7NO | YES | | | 0 | 1493 | 0 | 3965,5 |
| epsilon-N-Biotinyl-L-lysine | 976 | C16H28N4O4S | YES | | | 0 | 0 | 0 | 115 |
| Butylamine | 977 | C4H11N | YES | Butylamine; 1-Butanamine; 1-Aminobutane | 151121 | 0 | 2567 | 0 | 5317,25 |
| Butyrate | 978 | C4H8O2 | YES | | | 2391 | 0 | 0 | 3137 |
| Butyl butyrate | 979 | C8H16O2 | NO | Butyl butanoate; Butyl butylate | 151097 | 308 | 5279 | 6635 | 2453,5 |
| Butyryl-CoA | 980 | C25H42N7O17P3S | YES | | | 0 | 5450 | 0 | 2193,25 |
| Uridine | 981 | C9H12N2O6 | YES | | | 2087 | 13647 | 7719 | 0 |
| 1-(5-Phospho-D-ribosyl)-5-amino-4-imidazolecarboxylate | 982 | C9H14N3O9P | YES | | | 1067 | 2328 | 11177 | 157,75 |

| | | | | | | | | | |
|---|---|---|---|---|---|---|---|---|---|
| Cyclohexa-1,5-diene-1-carbonyl-CoA | 983 | C28H42N7O17P3S | YES | | | 217 | 3124 | 8332 | 233 |
| Cellobiose-1,5-lactone | 984 | C12H20O11 | YES | | | 0 | 0 | 0 | 24 |
| Hexadec-1-ene | 985 | C16H32 | NO | 1-Hexadecene; Cetene; Hexadecylene-1 | 155716 | 0 | 1343 | 0 | 1965,5 |
| Cadaverine | 986 | C5H14N2 | YES | | | 0 | 0 | 5256 | 49,5 |
| Caffeate | 987 | C9H8O4 | YES | | | 0 | 1734 | 4359 | 0 |
| Caffeoyl-CoA | 988 | C30H42N7O19P3S | YES | | | 0 | 1756 | 0 | 273,5 |
| Butyl paraben | 989 | C11H14O3 | NO | Butyl 4-hydroxybenzoate | 150227 | 1944 | 0 | 0 | 1407,75 |
| N-Carbamyl-L-glutamate | 990 | C6H10N2O5 | YES | | | 0 | 0 | 0 | 357,5 |
| Methyl caffeate | 991 | C10H10O4 | YES | | | 0 | 0 | 0 | 578,75 |
| Catechol | 992 | C6H6O2 | YES | | | 1203 | 0 | 0 | 67 |
| (-)-trans-Carveol | 993 | C10H16O | YES | | | 0 | 0 | 1189 | 3253,25 |
| 3',5'-Cyclic AMP | 994 | C10H12N5O6P | YES | | | 1884 | 4140 | 0 | 4723 |
| epsilon-Caprolactam | 995 | C6H11NO | YES | | | 0 | 1019 | 0 | 1696,75 |
| Hexanoyl-CoA | 996 | C27H46N7O17P3S | YES | | | 2002 | 2669 | 0 | 52,5 |
| 5-Carboxy-2-pentenoyl-CoA | 997 | C27H42N7O19P3S | YES | | | 918 | 2959 | 11209 | 208,5 |
| Carbazole | 998 | C12H9N | YES | | | 745 | 2660 | 0 | 2305,75 |
| 8-Hydroxy-2-methyl-alpha-carboline | 999 | C12H10N2O | NO* | | | 81 | 0 | 3284 | 4971,25 |
| N-Butyl-beta-carboline-3-carboxylate | 1000 | C16H16N2O2 | YES | | | 0 | 2947 | 0 | 685,25 |
| 8-Hydroxy-2-methyl-beta-carboline | 1001 | C12H10N2O | NO* | | | 391 | 64 | 0 | 3874,5 |
| 8-Hydroxy-2-methyl-gamma-carboline | 1002 | C12H10N2O | NO* | | | 95 | 0 | 122 | 3815 |
| Carnosine | 1003 | C9H14N4O3 | YES | | | 449 | 0 | 9995 | 3581 |
| Carvone | 1004 | C10H14O | YES | | | 110 | 0 | 1141 | 242,75 |

| | | | | | | | | | |
|---|---|---|---|---|---|---|---|---|---|
| 2-(3-Carboxy-3-aminopropyl)-L-histidine | 1005 | C10H16N4O4 | YES | | | 81 | 1128 | 3586 | 3897,75 |
| Catechin | 1006 | C15H14O6 | YES | | | 146 | 181 | 5552 | 93,5 |
| N-Carbamoyl-L-aspartate | 1007 | C5H8N2O5 | YES | | | 1153 | 2361 | 0 | 0 |
| 3-Isopropylmalate | 1008 | C7H12O5 | YES | | | 0 | 5960 | 12 | 2998,25 |
| Cobinamide | 1009 | C48H72CoN11O8 | YES | | | 0 | 2212 | 72 | 7087,75 |
| Carbamoyl phosphate | 1010 | CH4NO5P | YES | | | 1297 | 2310 | 0 | 358,75 |
| m-Aminophenol | 1011 | C6H7NO | YES | | | 0 | 1022 | 0 | 0 |
| 4-Hydroxyphenylglyoxylate | 1012 | C8H6O4 | YES | | | 1516 | 89 | 0 | 4,25 |
| 3',5'-Cyclic CMP | 1013 | C9H12N3O7P | YES | | | 93 | 0 | 10 | 7306,5 |
| 3',5'-Cyclic dAMP | 1014 | C10H12N5O5P | YES | | | 0 | 2123 | 0 | 317,75 |
| trans-Hex-2-enoyl-CoA | 1015 | C27H44N7O17P3S | YES | | | 1019 | 0 | 0 | 3646,5 |
| cis-1,2-Dihydrobenzene-1,2-diol | 1016 | C6H8O2 | YES | | | 0 | 5367 | 0 | 257,5 |
| Capryldihydroxyacetonephosphate | 1017 | C4H6O7P(C8H16O2) | YES* | Dihydroxyacetone phosphate acyl ester | | 10 | 2 | 0 | 0 |
| cis-1,2-Dihydro-3-ethylcatechol | 1018 | C8H12O2 | YES | | | 0 | 8914 | 9933 | 4705,75 |
| CDP-1,2-Dinonadecanoylglycerol | 1019 | C14H19N3O15P2(C9H18O2) | YES* | CDP-1,2-diacylglycerol | | 0 | 6106 | 4271 | 261,75 |
| CDP-butyrylglycerol | 1020 | C13H20N3O14P2(C4H8O2) | YES* | CDP-acylglycerol | | 0 | 35 | 9 | 322,75 |
| Benzyl-L-cysteinamide | 1021 | C33H32N2O5S | NO* | | | 190 | 1521 | 753 | 0 |
| CDP-Hexadecanoylglycerol | 1022 | C13H20N3O14P2(C16H32O | YES* | CDP-acylglycerol | | 75 | 0 | 4619 | 396,25 |

| | | | | | | | | | |
|---|---|---|---|---|---|---|---|---|---|
| | | 2) | | | | | | | |
| CDP-1,2-Dibutyrylglycerol | 1023 | C14H19N3O15P2(C4H8O2)2 | YES* | CDP-1,2-diacylglycerol | | 1366 | 574 | 2087 | 3237,25 |
| CDP-choline | 1024 | C14H26N4O11P2 | YES | | | 0 | 3965 | 0 | 621,5 |
| CDP-Dodecanoylglycerol | 1025 | C13H20N3O14P2(C12H24O2) | YES* | CDP-acylglycerol | | 0 | 2933 | 0 | 313,5 |
| CDP-1,2-Dinonanoylglycerol | 1026 | C14H19N3O15P2(C9H18O2)2 | YES* | CDP-1,2-diacylglycerol | | 0 | 0 | 0 | 99 |
| CDP-Tetradecanoylglycerol | 1027 | C13H20N3O14P2(C14H28O2) | YES* | CDP-acylglycerol | | 0 | 1803 | 0 | 3194,25 |
| CDP-1,2-Ditetradecanoylglycerol | 1028 | C14H19N3O15P2(C14H28O2)2 | YES* | CDP-1,2-diacylglycerol | | 0 | 3791 | 0 | 2669,25 |
| CDP-1,2-Dihexanoylglycerol | 1029 | C14H19N3O15P2(C16H32O2)2 | YES* | CDP-1,2-diacylglycerol | | 10121 | 2775 | 0 | 3504,25 |
| CDP-1,2-Dioleylglycerol | 1030 | C14H19N3O15P2(C18H36O2)2 | YES* | CDP-1,2-diacylglycerol | | 85 | 2499 | 6312 | 414 |
| CDP-1,2-Dioctanoylglycerol | 1031 | C14H19N3O15P2(C8H16O2)2 | YES* | CDP-1,2-diacylglycerol | | 217 | 5095 | 0 | 485,5 |
| CDP-Octanoylglycerol | 1032 | C13H20N3O14P2(C8H16O2) | YES* | CDP-acylglycerol | | 0 | 2446 | 8074 | 69,5 |
| Cytidine diphosphate | 1033 | C9H15N3O11P2 | YES | | | 396 | 2787 | 5585 | 4655,5 |
| CDP-1,2-Didodecanoylglycerol | 1034 | C14H19N3O15P2(C12H24O2)2 | YES* | CDP-1,2-diacylglycerol | | 1435 | 2516 | 0 | 146,25 |

| | | | | | | | | | |
|---|---|---|---|---|---|---|---|---|---|
| CDP-1,2-Dioctadecanoylglycerol | 1035 | C14H19N3O15P2(C18H34O2)2 | YES* | CDP-1,2-diacylglycerol | | 0 | 3400 | 8796 | 3560,5 |
| CDP-1,2-Dipropionylglycerol | 1036 | C14H19N3O15P2(C3H6O2)2 | YES* | CDP-1,2-diacylglycerol | | 0 | 304 | 0 | 513,75 |
| CDP-Decanoylglycerol | 1037 | C13H20N3O14P2(C10H20O2) | YES* | CDP-acylglycerol | | 28 | 0 | 0 | 295,25 |
| CDP-ethanolamine | 1038 | C11H20N4O11P2 | YES* | CDP-acylglycerol | | 0 | 0 | 38 | 4395,25 |
| 3-Hydroxyaminophenol | 1039 | C6H7NO2 | YES | | | 406 | 4697 | 4677 | 3459,75 |
| CDP-glycerol | 1040 | C12H21N3O13P2 | YES | | | 0 | 2411 | 3437 | 0 |
| CDP-hexanoylglycerol | 1041 | C13H20N3O14P2(C6H12O2) | YES* | CDP-acylglycerol | | 0 | 0 | 0 | 594,25 |
| CDP-Nonanoylglycerol | 1042 | C13H20N3O14P2(C9H18O2) | YES* | CDP-acylglycerol | | 0 | 0 | 0 | 329,25 |
| CDP-Nonadecanoylglycerol | 1043 | C13H20N3O14P2(C19H38O2) | YES* | CDP-acylglycerol | | 0 | 8192 | 2455 | 279 |
| CDP-propionylglycerol | 1044 | C13H20N3O14P2(C3H6O2) | YES* | CDP-acylglycerol | | 0 | 2586 | 0 | 311,25 |
| 4-Hydroxybiphenyl | 1045 | C12H10O | YES | | | 431 | 0 | 0 | 4022,5 |
| Cellobiose | 1046 | C12H22O11 | YES | | | 0 | 682 | 0 | 0 |
| Cellotriose | 1047 | C18H32O16 | YES | | | 0 | 39 | 5 | 3594 |
| Cellotetraose | 1048 | C24H42O21 | YES | | | 0 | 61 | 0 | 652,25 |
| Cellulose | 1049 | (C6H10O5)20 | YES | | | 76 | 0 | 0 | 3299 |
| cis-3-(3-Carboxyethenyl)-3,5-cyclohexadiene-1,2-diol | 1050 | C9H10O4 | YES | | | 0 | 4153 | 6841 | 2104,75 |

| | | | | | | | | | |
|---|---|---|---|---|---|---|---|---|---|
| CDP-1,2-Didecanoylglycerol | 1051 | C14H19N3O15P2(C12H24O2)2 | YES* | CDP-1,2-diacylglycerol | | 1761 | 0 | 0 | 1647,25 |
| L-Cysteinylglycine | 1052 | C5H10N2O3S | YES | Cys-Gly | | 0 | 0 | 55 | 1566,25 |
| Cetyl alcohol | 1053 | C16H34O | YES | | | 1281 | 0 | 0 | 1580,25 |
| 4-Chlorocatechol | 1054 | C6H5ClO2 | YES | | | 418 | 3455 | 0 | 207 |
| 3',5'-Cyclic GMP | 1055 | C10H12N5O7P | YES | | | 458 | 0 | 10304 | 2455 |
| Cyclohex-2-enone | 1056 | C6H8O | YES | | | 0 | 3863 | 9406 | 5824,75 |
| 6-Carboxyhexanoyl-CoA | 1057 | C28H46N7O19P3S | YES | | | 0 | 0 | 0 | 17 |
| Cyclohexan-1,2-dione | 1058 | C6H8O2 | YES | | | 135 | 2085 | 6002 | 3607,25 |
| Cyclohexanone | 1059 | C6H10O | YES | | | 278 | 230 | 104 | 1726,5 |
| Chitin | 1060 | (C8H13NO5)20 | YES | | | 316 | 4091 | 0 | 5975 |
| Chitosan | 1061 | C12H24N2O9(C6H11NO4)20 | YES | | | 1304 | 4917 | 46 | 3271,25 |
| Chitobiose | 1062 | C16H28N2O11 | YES | | | 2464 | 0 | 0 | 470,75 |
| Choline | 1063 | C5H14NO | YES | | | 4051 | 0 | 0 | 4357,25 |
| Phenyl acetate | 1064 | C8H8O2 | YES | | | 0 | 0 | 0 | 11008,3 |
| Ethyl acetate | 1065 | C4H8O2 | YES | | | 0 | 57 | 0 | 2055,5 |
| Tolylacetate | 1066 | C9H10O2 | YES | | | 0 | 2171 | 0 | 136,25 |
| Ethyl butyryl acetate | 1067 | C16H30O4 | YES | | | 0 | 1883 | 0 | 563,75 |
| N-Acetyl-D-glucosamine 1,6-bisphosphate | 1068 | C8H17NO12P2 | YES | | | 0 | 0 | 0 | 3631,75 |
| 4-Chlorophenol | 1069 | C6H5ClO | YES | | | 54 | 0 | 0 | 278,75 |
| Chorismate | 1070 | C10H10O6 | YES | | | 1792 | 973 | 432 | 1907,25 |
| 5,7-Dihydroxychromone | 1071 | C9H6O4 | YES | | | 1972 | 0 | 0 | 3941,5 |
| Citrate | 1072 | C6H8O7 | YES | | | 2128 | 0 | 0 | 297,25 |
| CMP | 1073 | C9H14N3O8P | YES | | | 2808 | 0 | 0 | 146 |
| Cinnamoyl-CoA | 1074 | C30H42N7O1 | YES | | | 4316 | 0 | 8607 | 201,75 |

| | | | | | | | | | |
|---|---|---|---|---|---|---|---|---|---|
| | | 7P3S | | | | | | | |
| L-Citrulline | 1075 | C6H13N3O3 | YES | | | 31412 | 4820 | 0 | 378,5 |
| Cinnamaldehyde | 1076 | C9H8O | YES | | | 348 | 0 | 0 | 4753 |
| Cinnamyl acetate | 1077 | C11H12O2 | YES | | | 0 | 3623 | 0 | 2017 |
| Cinnoline | 1078 | C8H6N2 | NO | Benzo[c]pyridazine; 1,2-Benzodiazine | 152399 | 527 | 0 | 6471 | 4018,75 |
| 4-Chloro-m-cresol | 1079 | C7H7ClO | YES | | | 0 | 3231 | 0 | 861 |
| 1-Bromopentane | 1080 | C5H11Br | NO | n-Amyl bromide; Pentyl bromide; 1-Bromoprop-1-ene | 151176 | 0 | 2116 | 0 | 230,75 |
| 1-Chloropentane | 1081 | C5H11Cl | NO | n-Amyl chloride; Pentyl chloride; Amyl chloride; 1-Chloroprop-1-ene; 1-Chloro-propene; 1-Chloropropene; Propenyl chloride | 154280 | 34 | 0 | 10 | 2846,25 |
| 2-Hydroxy-2,4-pentadienoate | 1082 | C5H6O3 | YES | | | 0 | 0 | 0 | 2463,25 |
| Styrene oxide | 1083 | C8H8O | YES | | | 262 | 1276 | 0 | 1739,25 |
| 2,3-Dibromopentane | 1084 | C5H10Br2 | NO | | 672518 | 67 | 5052 | 0 | 45,5 |
| 1,2,3-Trimethylbenzene | 1085 | C9H12 | YES | | | 0 | 0 | 7029 | 73,25 |
| trans-Aconitate | 1086 | C6H6O6 | YES | | | 1953 | 2875 | 0 | 384,25 |
| (R)-(+)-Citronellal | 1087 | C10H18O | YES | | | 96 | 211 | 613 | 2974,25 |
| (-)-Citronellol | 1088 | C10H20O | YES | | | 0 | 0 | 0 | 8063,5 |
| Cinnamyl alcohol | 1089 | C9H10O | YES | | | 196 | 2518 | 3540 | 5323,75 |
| CMP-3-deoxy-D-manno-octulosonate | 1090 | C17H26N3O15P | YES | | | 0 | 5827 | 0 | 4176,75 |
| Chlorobenzene | 1091 | C6H5Cl | YES | | | 41 | 0 | 0 | 398,75 |
| 2-Chlorobenzoate | 1092 | C7H5ClO2 | YES | | | 0 | 2879 | 37 | 141,25 |
| Cardiolipin | 1093 | C13H18O17P2(C3H6O2)4 | YES* | | | 2326 | 0 | 0 | 141,25 |
| 3-Carboxy-cis,cis-muconate | 1094 | C7H6O6 | YES | | | 1664 | 1974 | 0 | 2082,75 |

| | | | | | | | | | |
|---|---|---|---|---|---|---|---|---|---|
| 4-Chlorobutan-1-ol | 1095 | C4H9ClO | NO | 4-Chloro-1-butanol; 4-Chlorobutanol; 4-Chlorobutan-1 | 156908 | 0 | 9 | 0 | 244,25 |
| Chloroxylenol | 1096 | C8H9ClO | YES | | | 3245 | 1236 | 0 | 1615,5 |
| Styrene | 1097 | C8H8 | YES | | | 96 | 2442 | 7989 | 189,75 |
| Chlorogenate | 1098 | C16H18O9 | YES | | | 0 | 4455 | 0 | 2579,75 |
| 2-Amino-3-carboxymuconate semialdehyde | 1099 | C7H7NO5 | YES | | | 0 | 0 | 0 | 4825 |
| gamma-Carboxymuconolactone | 1100 | C7H6O6 | YES | | | 1684 | 0 | 0 | 36,75 |
| Coenzyme A | 1101 | C21H36N7O16P3S | YES | | | 159 | 2065 | 0 | 2827 |
| Coniferyl alcohol | 1102 | C10H12O3 | YES | | | 1807 | 2368 | 0 | 486,25 |
| Coniferaldehyde | 1103 | C10H10O3 | YES | | | 100 | 1972 | 0 | 5760 |
| 3-Chloro-cis,cis-muconate | 1104 | C6H5ClO4 | YES | | | 5148 | 120 | 0 | 5950,75 |
| Coronamic acid | 1105 | C6H11NO2 | NO | 1-amino-2-ethylcyclopropane-1-carboxylic acid | 133485 | 0 | 73 | 0 | 9177,5 |
| Crotonoyl-CoA | 1106 | C25H40N7O17P3S | YES | | | 3876 | 1375 | 0 | 3915,75 |
| 3-Ethyltoluene | 1107 | C9H12 | YES | | | 93 | 0 | 3 | 6344,5 |
| 4-Coumarate | 1108 | C9H8O3 | YES | | | 9 | 4496 | 0 | 8231,5 |
| p-Coumaroyl-CoA | 1109 | C30H42N7O18P3S | YES | | | 46 | 2520 | 0 | 4028 |
| Coumaran | 1110 | C8H8O | NO | 2,3-Dihydro-1-benzofuran; Benzofuran | 153600 | 168 | 23784 | 0 | 221,75 |
| Coumarin | 1111 | C9H6O2 | YES | 2H-1-Benzopyran-2-one; 1,2-Benzopyrone | 24902108 | 0 | 41 | 270 | 205,75 |
| Methyl p-coumarate | 1112 | C10H10O3 | NO | Methyl 3-(4-hydroxyphenyl)prop-2-enoate; Methyl p-hydroxycinnamate; Methyl | 669055 | 0 | 19660 | 0 | 4514,25 |

| | | | | | | | | | |
|---|---|---|---|---|---|---|---|---|---|
| | | | | 4-hydroxycinnamate | | | | | |
| CDP-Octadecanoylglycerol | 1113 | C13H20N3O14P2(C8H16O2) | YES* | CDP-acylglycerol | | 16 | 0 | 0 | 279,75 |
| 2-Coumarinate | 1114 | C9H8O3 | YES | | | 0 | 47 | 0 | 264,25 |
| Creatine | 1115 | C4H9N3O2 | YES | | | 14 | 1173 | 0 | 41 |
| Ethylglycocyamine | 1116 | C4H9N3O2 | YES | | | 0 | 182 | 0 | 30,75 |
| 3-Chlorocatechol | 1117 | C6H5ClO2 | YES | | | 626 | 0 | 0 | 397 |
| CTP | 1118 | C9H16N3O14P3 | YES | | | 3452 | 3522 | 3159 | 2732 |
| 2-Chloro-cis,cis-muconate | 1119 | C6H5ClO4 | YES | | | 0 | 4669 | 0 | 3675,75 |
| Cytosine | 1120 | C4H5N3O | YES | | | 939 | 5129 | 0 | 422 |
| Coronafacic acid | 1121 | C12H16O3 | YES | | | 121 | 2057 | 0 | 256,25 |
| Crotono-betaine | 1122 | C8H16NO | YES | | | 0 | 3196 | 0 | 4059,75 |
| 3,5-Dichlorocatechol | 1123 | C6H4Cl2O2 | YES | | | 5 | 2531 | 48 | 5978,75 |
| Coronatine | 1124 | C18H25NO4 | NO | | 668482 | 0 | 2173 | 0 | 42,75 |
| p-Cumic aldehyde | 1125 | C10H12O | YES | | | 1062 | 81 | 10092 | 557,5 |
| Cumene | 1126 | C9H12 | YES | | | 0 | 3749 | 0 | 5920,25 |
| p-Cumic alcohol | 1127 | C10H14O | YES | | | 1889 | 3465 | 0 | 6168,5 |
| N6-(1,2-Dicarboxyethyl)-AMP | 1128 | C14H18N5O11P | YES | | | 2686 | 1700 | 0 | 388 |
| Cyanobenzene | 1129 | C7H5N | YES | | | 3019 | 0 | 0 | 2864,75 |
| Cyclobutane | 1130 | C4H8 | NO | Tetramethylene | 152440 | 1780 | 3917 | 9617 | 3970,5 |
| Cycloheptane | 1131 | C7H14 | NO | Heptamethylene | 152457 | 0 | 1465 | 0 | 3230,25 |
| Cycloheptadecane | 1132 | C17H34 | NO | | 10517498 | 92 | 5453 | 14280 | 370,75 |
| Cyclohexanol | 1133 | C6H12O | YES | | | 5883 | 911 | 3659 | 2421,25 |
| Cyclohexylamine | 1134 | C6H13N | YES | | | 479 | 2311 | 0 | 176,75 |
| Cyclohexane | 1135 | C6H12 | YES | | | 29 | 1431 | 20353 | 354,5 |
| Cyclohexadecane | 1136 | C16H32 | NO | | 209642 | 1917 | 3135 | 9871 | 125 |
| Cyclohexene oxide | 1137 | C6H10O | NO | 7-oxabicyclo[4.1.0]heptane; Cyclohexene epoxide | 152436 | 1713 | 3195 | 20644 | 162 |

| | | | | | | | | | |
|---|---|---|---|---|---|---|---|---|---|
| Cyclononane | 1138 | C9H18 | NO | | 712369 | 85 | 662 | 0 | 251,25 |
| Cyclopentane | 1139 | C5H10 | NO | Pentamethylene | 152443 | 3174 | 2003 | 0 | 96 |
| Urea | 1140 | CON2H4 | YES | | | 2506 | 23816 | 0 | 628,25 |
| Cyclopropane | 1141 | C3H6 | NO | Trimethylene | 149297 | 2186 | 3505 | 0 | 2658,25 |
| dADP | 1142 | C10H15N5O9 P2 | YES | | | 1846 | 18 | 15108 | 411,25 |
| D-Cycloserine | 1143 | C3H6N2O2 | YES | | | 77 | 6182 | 0 | 6862,75 |
| Cycloundecane | 1144 | C11H22 | NO | | 712370 | 0 | 5626 | 0 | 4066,75 |
| L-Cysteine | 1145 | C3H7NO2S | YES | | | 1273 | 1903 | 0 | 2052,25 |
| trans-Cyclohexane-1,2-diol | 1146 | C6H12O2 | YES | | | 0 | 2243 | 4647 | 2942,25 |
| Decanoic acid | 1147 | C10H20O2 | YES | | | 2373 | 0 | 0 | 2587 |
| Cyclopentanone | 1148 | C5H8O | YES | | | 0 | 2690 | 0 | 3562,75 |
| Caproyldihydroxyacetoneposphate | 1149 | C4H6O7P(C8 H16O2) | YES* | Dihydroxyacetone phosphate acyl ester | | 206 | 0 | 0 | 440,25 |
| L-Cystine | 1150 | C6H12N2O4S 2 | YES | | | 0 | 3432 | 21 | 106,25 |
| Acetylcysteine | 1151 | C5H9NO3S | YES | | | 0 | 5001 | 0 | 37 |
| Cysteine p-nitroaniline | 1152 | C9H14O4SN | NO* | | | 0 | 0 | 0 | 940 |
| Cystinyl p-nitroalinine | 1153 | C12H18O6N3 S2 | NO* | | | 0 | 807 | 0 | 135 |
| Methyl 2-phenylacetate | 1154 | C9H10O2 | NO | Methyl benzeneacetate; Methyl phenylacetate; Methyl alpha-toluate | 7559 | 0 | 5020 | 9608 | 619 |
| Cytidine | 1155 | C9H13N3O5 | YES | | | 490 | 4380 | 0 | 327,5 |
| 2,4-Dihydrofuran | 1156 | C4H6O | NO | 3-Oxolene | 15570 | 0 | 6387 | 0 | 569,5 |
| Diethyl 2-methyl-3-oxosuccinate | 1157 | C9H14O5 | YES | | | 25 | 0 | 0 | 1016,25 |
| D-4-Hydroxyphenyl-glycine methyl ester | 1158 | C9H11NO3 | NO | | 13215089 | 23 | 7981 | 2082 | 9480,75 |
| L-Cystathionine | 1159 | C7H14N2O4S | YES | | | 3056 | 8129 | 0 | 6464 |
| Deoxyadenosine | 1160 | C10H13N5O3 | YES | | | 940 | 0 | 22 | 2394 |
| D-Galactono-1,5- | 1161 | C6H10O6 | YES | | | 158 | 0 | 0 | 7864,25 |

| | | | | | | | | | |
|---|---|---|---|---|---|---|---|---|---|
| lactone | | | | | | | | | |
| 6,7-Dimethyl-8-(1-D-ribityl)lumazine | 1162 | C13H18N4O6 | YES | | | 0 | 0 | 0 | 234,75 |
| Deoxy-5-methylcytidylate | 1163 | C10H16N3O7P | YES | | | 0 | 2980 | 0 | 3976 |
| Cyclopenta[b]pyridine | 1164 | C8H9N | NO | | 210415 | 0 | 0 | 0 | 958 |
| D-Aldonolactone | 1165 | C6H10O6 | YES | | | 162 | 2874 | 12 | 80,5 |
| Octadecanoylglycerone phosphate | 1166 | C4H6O7P(C18H36O2) | YES* | Dihydroxyacetone phosphate acyl ester | | 30 | 4294 | 0 | 403,75 |
| D-Arabinitol | 1167 | C5H12O5 | YES | | | 59 | 0 | 0 | 199,25 |
| 1,3-Diaminopropane | 1168 | C3H10N2 | YES | | | 0 | 1732 | 4662 | 282,25 |
| 7,8-Diaminononanoate | 1169 | C9H20N2O2 | YES | | | 7539 | 0 | 6948 | 5251 |
| 3,4-Dihydroxy-2-butanone 4-phosphate | 1170 | C4H9O6P | YES | | | 2289 | 0 | 0 | 229,75 |
| D-Arabonate | 1171 | C5H10O6 | NO | 2,3,4,5-Tetrahydroxypentanoic acid; D-Arabonate; D-Arabonic acid; D-Arabinonate | 153524 | 18 | 2307 | 17316 | 89,75 |
| 2-Ethyltoluene | 1172 | C9H12 | YES | | | 0 | 26 | 0 | 990 |
| 2'-Deoxy-5-hydroxymethylcytidine-5'-diphosphate | 1173 | C10H17N3O11P2 | YES | | | 373 | 4493 | 9033 | 403,75 |
| D-Arabitol | 1174 | C5H12O5 | YES | | | 29 | 1994 | 0 | 193,25 |
| Methyl decanoate | 1175 | C11H22O2 | NO | Methyl caprate | 151168 | 0 | 4551 | 98 | 4497 |
| trans-Dec-2-Enoyl-CoA | 1176 | C31H52N7O17P3S | YES | | | 2903 | 5318 | 0 | 5226,5 |
| beta-Alanyl-L-lysine | 1177 | C9H19N3O3 | YES | | | 0 | 3088 | 0 | 283,5 |
| Decanoyl-CoA | 1178 | C37H66N7O17P3S | YES | | | 0 | 77 | 7481 | 300,333 |
| D-Galacturonate 1-phosphate | 1179 | C6H11O10P | YES | | | 152 | 4190 | 0 | 2987,75 |
| N6-(L-1,3- | 1180 | C11H20N2O6 | YES | | | 0 | 0 | 0 | 306,75 |

| | | | | | | | | | |
|---|---|---|---|---|---|---|---|---|---|
| Dicarboxypropyl)-L-lysine | | | | | | | | | |
| 2'-Deoxy-5-hydroxymethylcytidine-5'-triphosphate | 1181 | C10H18N3O14P3 | YES | | | 103 | 3747 | 0 | 0 |
| dCMP | 1182 | C9H14N3O7P | YES | | | 5279 | 1415 | 0 | 335 |
| dCDP | 1183 | C9H15N3O10P2 | YES | | | 1645 | 3049 | 0 | 336 |
| 3-Hydroxy-4-trimethylammoniobutanoate | 1184 | C7H16NO3 | YES | | | 0 | 388 | 0 | 2041 |
| Dihydrothymine | 1185 | C5H8N2O2 | YES | | | 0 | 2148 | 0 | 1481,75 |
| dCTP | 1186 | C9H16N3O13P3 | YES | | | 615 | 2338 | 0 | 4221,75 |
| O-Butanoylcarnitine | 1187 | C11H21NO4 | YES | | | 502 | 0 | 0 | 0 |
| 3',4',5,7-Tetrahydroxy-3-methoxyflavone | 1188 | C16H12O7 | YES | | | 0 | 2644 | 0 | 101,75 |
| trans-Dodec-2-Enoyl-CoA | 1189 | C33H56N7O17P3S | NO | (2E)-Dodecenoyl-CoA; 2-trans-Dodecenoyl-CoA | 8143198 | 59 | 2034 | 0 | 6655,25 |
| Methyl dodecanoate | 1190 | C13H26O2 | NO | Methyl dodecanoate; Methyl laurate | 151268 | 108 | 804 | 0 | 348,25 |
| Dodecanoate | 1191 | C12H24O2 | YES | | | 156 | 3814 | 0 | 561,75 |
| Dodecanoyl-CoA | 1192 | C33H58N7O17P3S | YES | | | 0 | 866 | 0 | 440,5 |
| 1-Tetradecanoyl-sn-glycerol 3-phosphate | 1193 | C4H8O7P(C14H28O2) | YES* | 1-Acyl-sn-glycerol 3-phosphate | | 0 | 8080 | 24 | 6166,25 |
| Dodecanoyldolichol | 1194 | C21H35O2(C5H8)5C12H24O2 | YES* | Acyldolichol | | 0 | 30700 | 0 | 218 |
| Decanoyldihydroxyacetonephosphate | 1195 | C4H6O7P(C12H24O2) | YES* | Dihydroxyacetone phosphate acyl ester | | 0 | 2333 | 0 | 3404,75 |
| Decanoyldolichol | 1196 | C21H35O2(C5H8)5C10H20O2 | YES* | Acyldolichol | | 4481 | 2527 | 0 | 179,5 |

| | | | | | | | | | |
|---|---|---|---|---|---|---|---|---|---|
| Decane | 1197 | $C_{10}H_{22}$ | NO | n-Decane | 173339 | 0 | 4199 | 29 | 3287,5 |
| Decanal | 1198 | $C_{10}H_{20}O$ | YES | | | 0 | 0 | 0 | 139 |
| Octadecanal | 1199 | $C_{18}H_{36}O$ | YES | | | 99 | 1514 | 0 | 228,5 |
| 2'-Deoxy-5-hydroxymethylcytidine 5'-phosphate | 1200 | $C_{10}H_{16}N_3O_8P$ | YES | | | 0 | 3771 | 12 | 3432,75 |
| 2-Deoxy-D-ribose | 1201 | $C_5H_{12}O_{10}P_2$ | YES | | | 0 | 1603 | 0 | 98 |
| Dihydrouracil | 1202 | $C_4H_6N_2O_2$ | YES | | | 146 | 5455 | 0 | 4615,5 |
| (S)-2-Hydroxyglutarate | 1203 | $C_5H_8O_5$ | YES | | | 20 | 7078 | 0 | 2269,75 |
| D-Fucose | 1204 | $C_6H_{12}O_5$ | YES | | | 0 | 0 | 4 | 3269,25 |
| D-Glucono-1,5-lactone | 1205 | $C_6H_{10}O_6$ | YES | | | 0 | 0 | 0 | 2489,5 |
| dGDP | 1206 | $C_{10}H_{15}N_5O_{10}P_2$ | YES | | | 1001 | 2736 | 0 | 373,5 |
| D-Glucarate | 1207 | $C_6H_{10}O_8$ | YES | | | 0 | 7 | 0 | 818 |
| dGMP | 1208 | $C_{10}H_{14}N_5O_7P$ | YES | | | 873 | 0 | 0 | 230,75 |
| 4-Bromophenol-2,3-epoxide | 1209 | $C_6H_5BrO_2$ | YES | | | 46 | 1045 | 0 | 368 |
| dGTP | 1210 | $C_{10}H_{16}N_5O_{13}P_3$ | YES | | | 82 | 4599 | 0 | 67,5 |
| 1-Hydroxy-2-naphthoate | 1211 | $C_{11}H_8O_3$ | YES | | | 227 | 3633 | 0 | 470 |
| Dihydroxyacetone | 1212 | $C_3H_6O_2$ | YES | | | 0 | 2549 | 0 | 4589,25 |
| Dihydroxyacetone phosphate | 1213 | $C_3H_7O_6P$ | YES | | | 0 | 0 | 0 | 5399,5 |
| (1S,3R,4S)-3,4-Dihydroxycyclohexane-1-carboxylate | 1214 | $C_7H_{12}O_4$ | YES | | | 0 | 5822 | 0 | 550,75 |
| 7,8-Dihydrofolate | 1215 | $C_{19}H_{21}N_7O_6$ | YES | | | 628 | 4699 | 0 | 3809 |
| trans-2,3-Dihydroxycinnamate | 1216 | $C_9H_8O_4$ | YES | | | 0 | 4392 | 0 | 108,75 |
| 1,4-Dihydroxy-2-naphthoate | 1217 | $C_{11}H_8O_4$ | YES | | | 0 | 0 | 0 | 2199 |

| | | | | | | | | | |
|---|---|---|---|---|---|---|---|---|---|
| 2-Amino-4-hydroxy-6-(D-erythro-1,2,3-trihydroxypropyl)-7,8-dihydropteridine | 1218 | C9H13N5O4 | YES | | | 0 | 2767 | 0 | 119,75 |
| (S)-Dihydroorotate | 1219 | C5H6N2O4 | YES | | | 0 | 0 | 0 | 173 |
| Dihydroneopterin phosphate | 1220 | C9H14N5O7P | YES | | | 0 | 3961 | 0 | 4866,75 |
| 3-(2,3-Dihydroxyphenyl)propanoate; 2,3-Dihydroxyphenylpropanoate | 1221 | C9H10O4 | YES | | | 6 | 0 | 0 | 326,5 |
| Dihydropteroate | 1222 | C14H14N6O3 | YES | | | 0 | 9 | 8273 | 398,5 |
| (S)-4,5-dihydroxypentan-2,3-dione | 1223 | C5H8O4 | YES | | | 225 | 3428 | 115 | 6917,25 |
| 3-Dehydrosphinganine | 1224 | C18H37NO2 | YES | | | 0 | 3607 | 0 | 3495,75 |
| 3',5-Dihydroxy-3,4',7-trimethoxyflavone | 1225 | C18H16O7 | YES | | | 0 | 0 | 0 | 5772,75 |
| (5R)-3,4-Dihydroxy-5-(hydroxymethyl)furan-2(5H)-one | 1226 | C5H6O5 | YES | | | 130 | 2673 | 0 | 6048,75 |
| 2,4-Dibromophenol | 1227 | C6H4Br2O | YES | | | 0 | 2111 | 4220 | 665,5 |
| 2,6-Dibromophenol | 1228 | C6H4Br2O | YES | | | 1686 | 412 | 671 | 732 |
| Deoxyguanosine | 1229 | C10H13N5O4 | YES | | | 654 | 3678 | 0 | 2267 |
| Dibenzofuran | 1230 | C12H8O | YES | | | 0 | 0 | 0 | 541 |
| Dibenzo-p-dioxin | 1231 | C12H8O2 | YES | | | 0 | 0 | 882 | 348,5 |
| dTMP | 1232 | C10H15N2O8P | YES | | | 580 | 3287 | 3494 | 5440,5 |
| 4-Chlorophenylacetate | 1233 | C8H7ClO2 | YES | | | 0 | 8517 | 0 | 54 |
| D-Iditol | 1234 | C6H14O6 | YES | | | 0 | 9555 | 0 | 18813,5 |
| Digallate | 1235 | C14H10O9 | YES | | | 0 | 1214 | 37 | 557,75 |
| Dihydrocoumarin | 1236 | C9H8O2 | YES | | | 0 | 2737 | 0 | 2128,75 |

| | | | | | | | | | |
|---|---|---|---|---|---|---|---|---|---|
| 9-Hydroxynonanoate | 1237 | C9H18O3 | NO | | 24668727 | 0 | 259 | 0 | 329 |
| 10-Hydroxydecanoate | 1238 | C10H20O3 | YES | | | 0 | 0 | 0 | 403,25 |
| 2,4-Dinitrotoluene | 1239 | C7H6N2O4 | YES | | | 45 | 1032 | 0 | 275 |
| Deoxyinosine | 1240 | C10H12N4O4 | YES | | | 504 | 5066 | 62 | 1147,75 |
| Dioxybenzone | 1241 | C14H12O4 | YES | | | 0 | 0 | 0 | 121,5 |
| 2,3-Bis(3-Hydroxytetradecanoyl)-D-glucosaminyl-1,6-beta-D-2,3-bis(3-hydroxytetradecanoyl)-beta-D-glucosaminyl 1-phosphate | 1242 | C68H129N2O20P | YES | | | 4 | 2881 | 2150 | 7366 |
| 2,3-Diketo-5-methylthiopentyl-1-phosphate | 1243 | C6H11O6PS | YES | | | 0 | 2053 | 0 | 860,5 |
| 4-Carboxymethylenebut-2-en-4-olide | 1244 | C6H4O4 | YES | | | 63 | 0 | 0 | 1230,75 |
| cis-2-Methyl-5-isopropylhexa-2,5-dienoic acid | 1245 | C10H16O2 | YES | | | 134 | 0 | 0 | 1207 |
| cis-2-Methyl-5-isopropylhexa-2,5-dienoyl-CoA | 1246 | C31H50N7O17P3S | YES | | | 91 | 15065 | 0 | 346,5 |
| D-Mannuronate | 1247 | C6H10O7 | YES | | | 0 | 0 | 40 | 172 |
| D-Lyxose | 1248 | C5H10O5 | YES | | | 48 | 67 | 0 | 6421,75 |
| Dihydrolipoamide | 1249 | C8H17NOS2 | YES | | | 4118 | 9841 | 0 | 8300,5 |
| D-Mannonate | 1250 | C6H12O7 | YES | | | 0 | 6768 | 3833 | 422,25 |
| Dimethoxybenzidine o-dianisidine | 1251 | C14H16N2O2 | NO* | | | 0 | 0 | 0 | 811,75 |
| 5,6-Dimethylbenzimidazole | 1252 | C9H10N2 | YES | | | 0 | 0 | 0 | 203,25 |
| Dimethylglycine | 1253 | C4H9NO2 | YES | | | 0 | 4800 | 0 | 300,75 |
| Nalpha,Nalpha- | 1254 | C8H13N3O2 | YES | | | 0 | 0 | 0 | 1470,25 |

| | | | | | | | | | |
|---|---|---|---|---|---|---|---|---|---|
| Dimethyl-L-histidine | | | | | | | | | |
| DNA substrate lambda DNA + Sau3A | 1255 | # | NO | | | 0 | 0 | 0 | 114,75 |
| DNA resolvase substrate 5-GACGCTGCCGAATTCTGGCTTGCTAGGACATCTTTGCCCACGTTGACCC-3 | 1256 | # | NO | | | 0 | 1229 | 0 | 3090,5 |
| 4alpha-Methylzymosterol | 1257 | C29H46O | YES | | | 0 | 926 | 0 | 519 |
| 6,7-Dimethyl-8-(1-D-ribityl)lumazine | 1258 | C13H18N4O6 | YES | | | 734 | 0 | 0 | 1067,75 |
| Dimethylallyl diphosphate | 1259 | C5H12O7P2 | YES | | | 8466 | 0 | 0 | 337 |
| DNA substrate lambda DNA + HindIII | 1260 | # | NO | | | 197 | 2068 | 0 | 5696,75 |
| DNA substrate TAAGCTCCGGATTGTCCGGGAGGTAAAGCCCTGAT | 1261 | # | NO | | | 0 | 3307 | 38 | 325,25 |
| DNA substrate CACAGGAAGCTCTACAGGTACTCCG | 1262 | # | NO | | | 37 | 564 | 62 | 465 |
| DNA substrate TGGTCATCAGGGCTTTACCTCCCGGACAATCCGGAGCTTACGGAGTACCTGTAGAGCTTCCTGTGCAAGC | 1263 | # | NO | | | 0 | 1863 | 0 | 703,5 |
| DNA substrate SspI X174 DNA | 1264 | # | NO | | | 401 | 0 | 7202 | 5615,25 |
| DNA Helicase substrate dsDNA: 5- | 1265 | # | NO | | | 126 | 7147 | 1580 | 7228,75 |

| | | | | | | | | | |
|---|---|---|---|---|---|---|---|---|---|
| GCACTGGCCGTCG TTTTACC-3 | | | | | | | | | |
| DNA Gyrase substrate relaxed pBR322 | 1266 | # | NO | | | 0 | 0 | 5889 | 546,75 |
| DNA Topoisomerase substrate 40-base single-stranded oligodeoxyribonucleotides | 1267 | # | NO | | | 0 | 1710 | 5891 | 5798,25 |
| Deamino-NAD+ | 1268 | C21H27N6O15P2 | YES | | | 0 | 334 | 0 | 3068,5 |
| n-Dodecyl -D-maltoside | 1269 | C24H46O11 | NO | Lauryl-beta-D-maltoside; n-Dodecyl beta-D-maltoside | 24893931 | 77 | 25929 | 6732 | 173 |
| Docosan-1-ol | 1270 | C22H46O | NO | 1-Docosanol; Behenyl alcohol; Docosanol | 207364 | 0 | 2065 | 0 | 258 |
| Dodecane | 1271 | C12H26 | YES | | | 43 | 0 | 0 | 4666,5 |
| Dodecanoyldihydoxyacetonephosphate | 1272 | C3H6O6PR | YES* | O-Alkylglycerone phosphate | | 221 | 0 | 29 | 375,75 |
| Dolichol | 1273 | C20H36O(C5H8)5 | YES | | | 0 | 2408 | 0 | 2483,75 |
| Dolichyl D-mannosyl phosphate | 1274 | C26H47O9P(C5H8)5 | YES | | | 0 | 274 | 30 | 51 |
| Dolichyl phosphate | 1275 | C20H37O4P(C5H8)5 | YES | | | 0 | 2302 | 0 | 4903,25 |
| Dehydrodolichol diphosphate | 1276 | C20H36O7P2(C5H8)5 | YES | | | 0 | 4996 | 151 | 388 |
| O-Decanoyl-L-carnitine | 1277 | C17H33NO4 | YES | | | 111 | 0 | 0 | 7194,5 |
| O-Propanoylcarnitine | 1278 | C10H19NO4 | YES | | | 0 | 3706 | 0 | 1098,25 |
| Dephospho-CoA | 1279 | C21H35N7O13P2S | YES | | | 1882 | 0 | 0 | 7683,25 |
| Dethiobiotin | 1280 | C10H18N2O3 | YES | | | 3556 | 2 | 2433 | 1299,75 |
| 2-[3-Carboxy-3- | 1281 | C11H19N4O4 | YES | | | 0 | 0 | 0 | 3682 |

| | | | | | | | | | |
|---|---|---|---|---|---|---|---|---|---|
| (methylammonio)propyl]-L-histidine | | | | | | | | | |
| Diethyl (2R,3R)-2-methyl-3-hydroxysuccinate | 1282 | C9H16O5 | YES | | | 42 | 1081 | 0 | 5301,25 |
| dTDP | 1283 | C10H16N2O11P2 | YES | | | 1355 | 2899 | 3587 | 2879,75 |
| D-Ribitol 5-phosphate | 1284 | C5H13O8P | YES | | | 0 | 2819 | 0 | 1165 |
| D-Ribonate | 1285 | C5H10O6 | YES | | | 90 | 0 | 0 | 1983,5 |
| D-Ribulose | 1286 | C5H10O5 | YES | | | 99 | 3434 | 1610 | 8307,75 |
| Diethyl (2S,3R)-2-methyl-3-hydroxysuccinate | 1287 | C9H16O5 | YES | | | 0 | 0 | 2 | 100 |
| Phenanthrene-4,5-dicarboxylate | 1288 | C16H8O4 | NO | see http://umbbd.ahc.umn.edu | | 0 | 5248 | 22 | 642 |
| Deoxyribose | 1289 | C5H10O4 | YES | | | 0 | 0 | 0 | 328 |
| 2-Acetamido-2,6-dideoxy-D-glucose | 1290 | C8H15NO5 | YES | | | 0 | 0 | 2828 | 2636,5 |
| dTDP-4-Amino-4,6-dideoxy-D-glucose | 1291 | C16H27N3O14P2 | YES | | | 0 | 3168 | 0 | 156,5 |
| dTDP-4-Dehydro-6-deoxy-D-glucose | 1292 | C16H24N2O15P2 | YES | | | 62 | 483 | 0 | 123,5 |
| dTDP-4-Dehydro-6-deoxy-L-mannose | 1293 | C16H24N2O15P2 | YES | | | 19 | 2790 | 0 | 217,75 |
| dTDP-4-oxo-6-deoxy-alpha-D-glucose | 1294 | C16H24N2O15P2 | YES | | | 0 | 0 | 66 | 152,25 |
| dTDP-6-deoxy-L-mannose | 1295 | C16H26N2O15P2 | YES | | | 0 | 0 | 0 | 4335,25 |
| dTDP-6-deoxy-L-talose | 1296 | C16H26N2O15P2 | YES | | | 0 | 4662 | 0 | 252,75 |
| dTDP-alpha-D-desosamine | 1297 | C18H31N3O13P2 | YES | | | 20 | 4396 | 0 | 196,25 |
| dTDP-glucose | 1298 | C16H26N2O16P2 | YES | | | 3068 | 0 | 0 | 487 |
| dTDP-D-galactose | 1299 | C16H26N2O1 | YES | | | 0 | 0 | 0 | 227 |

| | | | | | | | | | |
|---|---|---|---|---|---|---|---|---|---|
| | | 6P2 | | | | | | | |
| dTDP-D-fucose | 1300 | C16H26N2O15P2 | YES | | | 0 | 0 | 0 | 782 |
| dTDP-L-rhamnose | 1301 | C16H26N2O15P2 | YES | | | 0 | 4968 | 30 | 1666,5 |
| Dibenzothiophene | 1302 | C12H8S | NO | | 151751 | 0 | 2880 | 7181 | 150,5 |
| 4,5-Dihydroxy-4,5-dihydropyrene | 1303 | C16H12O2 | NO | see http://umbbd.ahc.umn.edu | | 22 | 0 | 0 | 464,5 |
| D-Xylulose | 1304 | C5H10O5 | YES | | | 0 | 2364 | 0 | 3441,75 |
| dTDP-D-galacturonate | 1305 | C16H24N2O17P2 | YES | | | 0 | 7949 | 0 | 4133,5 |
| 1-Deoxy-D-xylulose | 1306 | C5H10O4 | YES | | | 0 | 364 | 1661 | 61,25 |
| Thiamin triphosphate | 1307 | C12H20N4O10P3S | YES | | | 0 | 2789 | 35 | 5022,5 |
| Tropinone | 1308 | C8H13NO | YES | | | 0 | 1791 | 0 | 106 |
| 1,2-Dioctanoyl-sn-glycerol | 1309 | C5H6O5(C8H16O2)2 | YES* | 1,2-Diacyl-sn-glycerol | | 102 | 5286 | 0 | 5731,25 |
| Quinazoline | 1310 | C8H6N2 | NO | Phenmiazine; 1,3-Benzodiazine | 152401 | 0 | 3090 | 0 | 65,75 |
| 1,2-Dihexadecanoyl-sn-glycerol | 1311 | C5H6O5(C16H32O2)2 | YES* | 1,2-Diacyl-sn-glycerol | | 11 | 6391 | 90 | 4439 |
| 1-Deoxy-D-xylulose 5-phosphate | 1312 | C5H11O7P | YES | | | 0 | 173 | 0 | 1435,5 |
| D-Xylonate | 1313 | C5H10O6 | YES | | | 0 | 194 | 2881 | 6464,75 |
| 4,5-Dihydroxypyrene | 1314 | C16H10O2 | NO | see http://umbbd.ahc.umn.edu | | 0 | 0 | 0 | 2271,25 |
| Ethyl 3-oxohexanoate | 1315 | C8H14O3 | YES | | | 0 | 0 | 0 | 5310 |
| 3,4-Dihydroxyphenanthrene | 1316 | C14H10O2 | NO | see http://umbbd.ahc.umn.edu | | 0 | 0 | 11392 | 273,5 |
| 3,6-Dichloro-cis-1,2-dihydroxycyclohexa-3,5-diene | 1317 | C6H6Cl2O2 | YES | | | 184 | 0 | 0 | 300,75 |
| L-Erythro-4-Hydroxyglutamate | 1318 | C5H9NO5 | YES | | | 358 | 37 | 0 | 288,5 |

| | | | | | | | | | |
|---|---|---|---|---|---|---|---|---|---|
| 1,2-Epoxypropane | 1319 | C3H6O | YES | | | 0 | 0 | 0 | 752 |
| Docosapentaenoic acid methyl ester | 1320 | C23H36O2 | NO* | | | 39 | 0 | 0 | 1117,5 |
| Docosapentaenoic acid | 1321 | C22H34O2 | YES | | | 91 | 53 | 0 | 6506 |
| D-erythro-1-(Imidazol-4-yl)glycerol 3-phosphate | 1322 | C6H11N2O6P | YES | | | 0 | 0 | 0 | 3561,5 |
| 2-Indanone oxime | 1323 | C9H9NO | YES | | | 38 | 51 | 0 | 911 |
| (+)-Epicatechin | 1324 | C15H14O6 | YES | | | 0 | 0 | 0 | 2442,25 |
| 2,3-Dihydroxy-4'-chlorobiphenyl | 1325 | C12H9ClO2 | YES | | | 139 | 0 | 0 | 162,5 |
| Epimelibiose | 1326 | C12H22O11 | YES | | | 0 | 0 | 0 | 433,75 |
| Epichlorohydrin | 1327 | C3H5ClO | YES | | | 0 | 0 | 21 | 200 |
| 1,2-Didecanoyl-sn-glycerol | 1328 | C5H6O5(C12H24O2)2 | YES* | 1,2-Diacyl-sn-glycerol | | 54 | 7551 | 0 | 319,75 |
| Episterol | 1329 | C28H46O | YES | | | 0 | 2139 | 0 | 4953,25 |
| Ethyl (R)-3-Hydroxyhexanoate | 1330 | C8H16O3 | YES | | | 0 | 0 | 2874 | 1746 |
| trans-2-Methyl-5-isopropylhexa-2,5-dienoic acid | 1331 | C10H16O2 | YES | | | 0 | 7756 | 0 | 181,25 |
| trans-2-Chlorodienelactone | 1332 | C6H3ClO4 | YES | | | 0 | 2278 | 0 | 410,5 |
| 1-Formyl-2-indanone | 1333 | C10H8O2 | YES | | | 0 | 1945 | 0 | 5496,75 |
| Erythrose | 1334 | C4H8O4 | YES | | | 0 | 1022 | 0 | 2563,75 |
| (R)-2-Hydroxystearate | 1335 | C18H36O3 | YES | | | 0 | 3606 | 0 | 252,75 |
| D-Erythrulose | 1336 | C4H8O4 | YES | | | 2186 | 0 | 0 | 197 |
| Erythritol | 1337 | C4H10O4 | YES | | | 0 | 0 | 0 | 306,25 |
| Ethyl (S)-3-Hydroxyhexanoate | 1338 | C8H16O3 | YES | | | 0 | 0 | 0 | 57 |
| N-Isopropylammelide | 1339 | C6H10N4O2 | YES | | | 12 | 0 | 0 | 410,5 |
| Ethyl benzoate | 1340 | C9H10O2 | NO | Ethyl ester benzoic acid; Benzoic ether | 150204 | 0 | 0 | 0 | 314,25 |

| | | | | | | | | | |
|---|---|---|---|---|---|---|---|---|---|
| Ethyl butyrate | 1341 | C6H12O2 | NO | Ethyl butanoate; Ethyl ester butyric acid | 150853 | 0 | 0 | 0 | 333,75 |
| Ethyl cinnamate | 1342 | C11H12O2 | NO | Ethyl (E)-3-phenylprop-2-enoate | 150731 | 0 | 0 | 4291 | 307,5 |
| Ethylguaiacol | 1343 | C9H12O2 | NO | 4-Ethyl-2-methoxyphenol; p-Ethylguaiacol; 4-Ethylguaiacol; Homocresol | 203890 | 0 | 0 | 10843 | 266,5 |
| Ethanolamine | 1344 | C2H7NO | YES | | | 412 | 0 | 0 | 148,5 |
| Ethylhexyl palmitate | 1345 | C24H48O2 | NO | 2-Ethylhexyl hexadecanoate; Octyl palmitate | 205020 | 0 | 0 | 0 | 908,5 |
| Ethyl heptanoate | 1346 | C9H18O2 | NO | Ethyl heptylate | 150891 | 3 | 0 | 0 | 110 |
| Ethylphenyl sulfide | 1347 | C8H12OS | NO* | | | 49 | 9530 | 10 | 310,5 |
| Ethylbenzene | 1348 | C8H10 | YES | | | 68 | 0 | 5356 | 419,25 |
| Ethyl lactate | 1349 | C5H10O3 | NO | Ethyl 2-hydroxypropanoate; L-Lactic acid ethyl ester | 150406 | 0 | 0 | 0 | 115,75 |
| Ethynylbenzene | 1350 | C8H6 | NO | Phenylethyne; Ethynyl-benzene | 154130 | 0 | 4035 | 0 | 2324,25 |
| o-Hydroxybenzoic acid | 1351 | C7H6O3 | YES | | | 0 | 0 | 0 | 2759,5 |
| Ethyl paraben | 1352 | C9H10O3 | NO | Ethyl 4-hydroxybenzoate | 151584 | 0 | 23310 | 0 | 610,75 |
| Ethyl salicylate | 1353 | C9H10O3 | NO | Ethyl 2-hydroxybenzoate | 151511 | 0 | 7548 | 0 | 132,75 |
| D-Fructose 1-phosphate | 1354 | C6H13O9P | YES | | | 0 | 4141 | 0 | 311 |
| Formaldehyde | 1355 | CH2O2 | YES | | | 1692 | 4293 | 0 | 284,75 |
| D-Fructose 2,6-bisphosphate | 1356 | C6H14O12P2 | YES | | | 0 | 3951 | 0 | 1548 |
| FAD | 1357 | C27H33N9O15P2 | YES | | | 0 | 1076 | 0 | 175,5 |
| FADH2 | 1358 | C27H35N9O15P2 | YES | | | 0 | 5039 | 0 | 494 |
| 1-Hexanoyl-sn-glycerol 3-phosphate | 1359 | C4H8O7P(C6H12O2) | YES* | 1-Acyl-sn-glycerol 3-phosphate | | 231 | 9303 | 0 | 5625,5 |

| | | | | | | | | | |
|---|---|---|---|---|---|---|---|---|---|
| N-Formylanthranilate | 1360 | C8H7NO3 | YES | | | 0 | 0 | 2 | 9009 |
| Farnesol | 1361 | C15H26O | YES | | | 0 | 189 | 0 | 544,5 |
| FAXX | 1362 | # | NO* | O-(5-O-(Feruloyl)-alpha-arabinofuranosyl)-(1-3)-O-beta-xylopyranosyl-(1-4)-xylopyranose; (E)-O-5-O-(3-(4-Hydroxy-3-methoxyphenyl)-1-oxo-2-propenyl)-alpha-L-arabinofuranosyl-(1-3)-O-beta-D-xylopranosyl-(1-4)-D-xylose | | 0 | 3351 | 0 | 282,75 |
| L-Fuculose 1-phosphate | 1363 | C6H13O8P | YES | | | 0 | 0 | 0 | 132,25 |
| L-Fuculose | 1364 | C6H12O5 | YES | | | 0 | 0 | 0 | 1842 |
| 1-Hexadecanol | 1365 | C16H34O | YES | | | 32 | 7763 | 0 | 142 |
| Feruloyl-CoA | 1366 | C31H44N7O19P3S | YES | | | 0 | 0 | 0 | 210 |
| Ferulate | 1367 | C10H10O4 | YES | | | 0 | 3526 | 0 | 30,75 |
| Methyl ferulate | 1368 | C11H12O4 | NO | Methyl (E)-3-(4-hydroxy-3-methoxyphenyl)prop-2-enoate; Methyl ferulate; Ferulic acid methyl ester; Ferulic acid methylester | 160046 | 0 | 1048 | 5075 | 158 |
| 5-Hydroxyferulate | 1369 | C10H10O5 | YES | | | 0 | 4244 | 0 | 213,75 |
| Diphenylenemethane | 1370 | C13H10 | YES | | | 0 | 0 | 4304 | 1125 |
| Flavone | 1371 | C15H10O2 | YES | | | 0 | 1111 | 0 | 162,75 |
| Fluorobenzene | 1372 | C6H5F | YES | | | 964 | 0 | 14 | 5575,75 |
| Formiminoglycine | 1373 | C3H6N2O2 | YES | | | 0 | 3466 | 0 | 287 |
| N2-Formyl-N1-(5-phospho-D-ribosyl)glycinamide | 1374 | C8H15N2O9P | YES | | | 278 | 4951 | 0 | 4143,75 |
| FMN | 1375 | C17H21N4O9P | YES | | | 0 | 5847 | 63 | 337 |
| Flavonol | 1376 | C15H10O3 | YES | | | 160 | 2432 | 0 | 386,25 |

| | | | | | | | | | |
|---|---|---|---|---|---|---|---|---|---|
| Formate | 1377 | CH2O2 | YES | | | 0 | 0 | 5557 | 5500,75 |
| 2-Hydroxy-6-oxo-6-(4'-chlorophenyl)-hexa-2,4-dienoate | 1378 | C12H9ClO4 | YES | | | 0 | 0 | 26 | 124 |
| 2-Dehydro-3-deoxy-D-fuconate | 1379 | C6H10O5 | YES | | | 123 | 13871 | 219 | 632 |
| N-Formimidoyl-L-glutamate | 1380 | C6H10N2O4 | YES | | | 0 | 5174 | 0 | 4893,5 |
| (R)-2,3-Dihydroxy-3-methylpentanoate | 1381 | C6H12O4 | YES | | | 2 | 0 | 0 | 2536 |
| 5-Formamido-1-(5-phospho-D-ribosyl)imidazole-4-carboxamide | 1382 | C10H15N4O9P | YES | | | 183 | 2533 | 81 | 4352,25 |
| 4'-O-beta-D-Glucosyl-cis-p-coumarate | 1383 | C15H18O8 | YES | | | 0 | 3804 | 0 | 213 |
| Farnesyl diphosphate | 1384 | C15H28O7P2 | YES | | | 0 | 0 | 0 | 154,25 |
| D-Fructose | 1385 | C6H12O6 | YES | | | 2 | 3934 | 11522 | 1059,75 |
| Fructosamine | 1386 | C6H13NO5 | NO* | | | 3 | 4502 | 0 | 379 |
| D-Fructuronate | 1387 | C6H10O7 | YES | | | 108 | 3636 | 10349 | 274 |
| Farnesal | 1388 | C15H24O | YES | | | 0 | 7 | 0 | 252,25 |
| L-Fucose 1-phosphate | 1389 | C6H13O8P | YES | | | 0 | 52 | 0 | 4112 |
| Fumarylacetoacetate | 1390 | C8H8O6 | YES | | | 612 | 0 | 0 | 4679,5 |
| D-Fuconate | 1391 | C6H12O6 | YES | | | 0 | 2152 | 0 | 6510,5 |
| Fumarate | 1392 | C4H4O4 | YES | | | 1701 | 3428 | 0 | 4999 |
| L-Fucose | 1393 | C6H12O5 | YES | | | 0 | 63 | 0 | 3808 |
| 4-Formylsalicylic acid | 1394 | C8H6O4 | NO | see http://umbbd.ahc.umn.edu | | 0 | 3078 | 0 | 5230,25 |
| D-Glucose 1-phosphate | 1395 | C6H13O9P | YES | | | 3104 | 0 | 0 | 152,25 |
| Glutathionyl-OH-1,2-dihydronaphthalene | 1396 | C20H24N2O7S | NO* | | | 0 | 2912 | 0 | 142,75 |
| Glutathionyl-1,2-dihydronaphthalene | 1397 | C20H24NO5S | NO* | | | 0 | 2751 | 0 | 271 |

| | | | | | | | | | |
|---|---|---|---|---|---|---|---|---|---|
| D-Glucono-1,5-lactate | 1398 | C6H10O6 | NO* | | | 0 | 0 | 0 | 4164,5 |
| Furazan-3,4-diol | 1399 | C2H2N2O3 | NO* | | | 0 | 2838 | 0 | 160,25 |
| Glycerol-3-phosphate | 1400 | C3H9O6P | YES | | | 0 | 3514 | 0 | 4121,5 |
| Glyceraldehyde 3-phosphate | 1401 | C3H7O6P | YES | | | 191 | 11 | 0 | 337,75 |
| sn-Glycero-3-phosphocholine | 1402 | C8H21NO6P | YES | | | 0 | 0 | 0 | 3670,5 |
| Glycerophosphoglycerol | 1403 | C6H15O8P | YES | | | 0 | 0 | 0 | 158,5 |
| sn-Glycero-3-phosphoethanolamine | 1404 | C5H14NO6P | YES | | | 2584 | 2263 | 0 | 262,5 |
| sn-Glycero-3-phospho-1-inositol | 1405 | C9H19O11P | YES | | | 0 | 0 | 0 | 6051,75 |
| beta-D-Glucose 6-phosphate | 1406 | C6H13O9P | YES | | | 120 | 0 | 61 | 4910,5 |
| Hexadecanoate | 1407 | C16H32O2 | YES | | | 0 | 3130 | 0 | 0 |
| 4-Aminobutyraldehyde | 1408 | C4H9NO | YES | | | 12 | 0 | 0 | 3430,25 |
| gamma-Amino-gamma-cyanobutanoate | 1409 | C5H8N2O2 | YES | | | 0 | 828 | 0 | 1632 |
| D-Galactose | 1410 | C6H12O6 | YES | | | 0 | 2775 | 2198 | 211,25 |
| alpha-D-Galactose 1-phosphate | 1411 | C6H13O9P | YES | | | 0 | 2012 | 2698 | 20 |
| D-Galactosamine | 1412 | C6H13NO5 | YES | | | 14 | 5843 | 0 | 5869 |
| Galactomannan | 1413 | C18H32O16 | YES | | | 0 | 3763 | 9080 | 3894,25 |
| D-Galactosaminoglycan | 1414 | # | YES | | | 0 | 5723 | 0 | 78 |
| Galactinol | 1415 | C12H22O11 | YES | | | 0 | 1285 | 0 | 3548,5 |
| D-Galactarate | 1416 | C6H10O8 | YES | | | 0 | 0 | 0 | 3469 |
| D-Galactonate | 1417 | C6H12O7 | YES | | | 0 | 0 | 4363 | 290 |
| 3-O-Methylgallate | 1418 | C8H8O5 | YES | | | 124 | 0 | 0 | 293,25 |
| Gallate | 1419 | C7H6O5 | YES | | | 0 | 5523 | 0 | 209 |

| | | | | | | | | | |
|---|---|---|---|---|---|---|---|---|---|
| n-Propyl gallate | 1420 | C10H12O5 | YES | | | 0 | 2758 | 0 | 268,5 |
| Galactitol | 1421 | C6H14O6 | YES | | | 0 | 0 | 7578 | 545 |
| Galactitol 1-phosphate | 1422 | C6H15O9P | YES | | | 0 | 4932 | 31557 | 4336,75 |
| D-Glucosamine 6-phosphate | 1423 | C6H14NO8P | YES | | | 1184 | 24 | 0 | 6017,75 |
| D-Glucosamine 1-phosphate | 1424 | C6H14NO8P | YES | | | 3396 | 0 | 0 | 7628,5 |
| D-Galacturonate | 1425 | C6H10O7 | YES | | | 0 | 0 | 0 | 255,75 |
| D-Glucosamine | 1426 | C6H13NO5 | YES | | | 0 | 0 | 53 | 6 |
| Octanoyl-CoA | 1427 | C29H50N7O17P3S | YES | | | 0 | 0 | 3775 | 2892 |
| gamma-Butyrolactone | 1428 | C4H6O2 | NO | 4-Butyrolactone; Oxolan-2-one | 150354 | 0 | 6217 | 7730 | 4276,75 |
| 4-Guanidinobutanoate | 1429 | C5H11N3O2 | YES | | | 0 | 2845 | 8672 | 3581,25 |
| Butyro-betaine | 1430 | C8H18NO | YES | | | 123 | 187 | 0 | 0 |
| 4-Guanidinobutanamide | 1431 | C5H12N4O | YES | | | 5634 | 5686 | 0 | 64,25 |
| Glycolaldehyde | 1432 | C2H4O2 | YES | | | 0 | 2754 | 0 | 931,75 |
| Cyclohexa-3,5-diene-1,2-dicarboxylate | 1433 | C8H8O4 | YES | | | 0 | 808 | 0 | 3021 |
| Glycolyl-D-mannosamine | 1434 | C8H15NO7 | YES | | | 0 | 0 | 0 | 731,75 |
| Glycolyl-D-mannosaminolactone | 1435 | C8H13NO7 | YES | | | 0 | 1779 | 0 | 735,75 |
| Octanoic acid | 1436 | C8H16O2 | YES | | | 0 | 0 | 0 | 319,5 |
| GDP-6-deoxy-D-mannose | 1437 | C16H25N5O15P2 | YES | | | 0 | 0 | 0 | 267,75 |
| GDP-6-deoxy-D-talose | 1438 | C16H25N5O15P2 | YES | | | 0 | 0 | 56 | 286,75 |
| GDP-4-dehydro-6-deoxy-D-mannose | 1439 | C16H23N5O15P2 | YES | | | 8 | 0 | 0 | 48,25 |
| GDP-D-mannose | 1440 | C16H25N5O16P2 | YES | | | 725 | 0 | 0 | 3598 |

| | | | | | | | | | |
|---|---|---|---|---|---|---|---|---|---|
| GDP-L-fucose | 1441 | C16H25N5O15P2 | YES | | | 0 | 0 | 52 | 329 |
| 6-Deoxy-D-glucose | 1442 | C6H12O5 | YES | | | 0 | 3817 | 0 | 239,25 |
| Gentiobiose | 1443 | C12H22O11 | YES | | | 0 | 0 | 0 | 1091,75 |
| Gentisate | 1444 | C7H6O4 | YES | | | 290 | 0 | 0 | 1239,75 |
| Geranyl acetate | 1445 | C12H20O2 | YES | | | 52 | 0 | 0 | 3386,75 |
| Geranic acid | 1446 | C10H16O2 | YES | | | 0 | 4113 | 0 | 65 |
| Geranial | 1447 | C10H16O | YES | | | 0 | 4652 | 0 | 100 |
| Geraniol | 1448 | C10H18O | YES | | | 240 | 0 | 0 | 5731,25 |
| trans-Geranyl-CoA | 1449 | C31H50N7O17P3S | YES | | | 0 | 490 | 0 | 189 |
| cis-Geranyl-CoA | 1450 | C31H50N7O17P3S | YES | | | 0 | 2318 | 0 | 470,75 |
| Geranylate | 1451 | C10H16O2 | NO | see http://umbbd.ahc.umn.edu | | 0 | 0 | 524 | 4831,25 |
| 3-beta-D-Galactosyl-sn-glycerol | 1452 | C9H18O8 | YES | | | 0 | 4475 | 0 | 3277,5 |
| Geranylgeranyl diphosphate | 1453 | C20H36O7P2 | YES | | | 17 | 0 | 0 | 4144,25 |
| beta-D-Glucose | 1454 | C6H12O6 | YES | | | 0 | 0 | 0 | 351,5 |
| gamma-Hexachlorocyclohexane | 1455 | C6H6Cl6 | YES | | | 0 | 0 | 0 | 4076,5 |
| 1-alpha-D-Galactosyl-myo-inositol | 1456 | C12H22O11 | YES | | | 0 | 0 | 0 | 169,75 |
| Nitrobenzene | 1457 | C6H5NO2 | YES | | | 0 | 0 | 0 | 406,75 |
| D-Gluconate | 1458 | C6H12O7 | YES | | | 3473 | 3333 | 0 | 129,25 |
| Glutarate | 1459 | C5H8O4 | YES | | | 0 | 0 | 0 | 2388,5 |
| D-Glucuronate | 1460 | C6H10O7 | YES | | | 0 | 0 | 1 | 3740,75 |
| D-Glutamine | 1461 | C5H10N2O3 | YES | | | 2 | 2186 | 0 | 291,75 |
| L-Glutamine | 1462 | C5H10N2O3 | YES | | | 4360 | 5578 | 0 | 3396 |
| L-Glutamate 1-semialdehyde | 1463 | C5H9NO3 | YES | | | 0 | 0 | 0 | 432,75 |
| L-Glutamate 5- | 1464 | C5H9NO3 | YES | | | 1562 | 52 | 0 | 6906,25 |

| | | | | | | | | | |
|---|---|---|---|---|---|---|---|---|---|
| semialdehyde | | | | | | | | | |
| L-Glutamate 5-phosphate | 1465 | C5H10NO7P | YES | | | 0 | 4669 | 145 | 5579 |
| D-Glucurono-6,2-lactone | 1466 | C6H8O6 | YES | | | 0 | 1370 | 0 | 249,75 |
| gamma-L-Glutamyl-D-alanine | 1467 | C8H14N2O5 | YES | | | 0 | 0 | 0 | 4653,5 |
| beta-D-Glucan | 1468 | C12H22O11(C6H10O5)10 | YES | | | 90 | 0 | 0 | 2541,5 |
| alpha-D-Glucose | 1469 | C6H12O6 | YES | | | 0 | 0 | 0 | 385 |
| gamma-L-Glutamyl-L-cysteine | 1470 | C8H14N2O5S | YES | | | 0 | 0 | 0 | 5895,25 |
| D-Glutamate | 1471 | C5H9NO4 | YES | | | 0 | 3478 | 0 | 659,75 |
| 3-Hydroxy-5-methylhex-4-enoyl-CoA | 1472 | C28H46N7O18P3S | YES | | | 2907 | 0 | 0 | 2452,75 |
| Glutaryl-CoA | 1473 | C26H42N7O19P3S | YES | | | 0 | 0 | 0 | 7443 |
| D-Glucurono-3,6-lactone | 1474 | C6H8O6 | YES | | | 0 | 31 | 0 | 4306,75 |
| Glucomannan | 1475 | C24H42O21 | YES | | | 39 | 0 | 0 | 177,25 |
| 2-Hydroxycinnamate | 1476 | C9H8O3 | YES | | | 0 | 0 | 2313 | 7399 |
| L-Glutamate | 1477 | C5H9NO4 | YES | | | 2895 | 0 | 11270 | 369 |
| Glyoxylate | 1478 | C2H2O3 | YES | | | 504 | 3047 | 10228 | 310 |
| alpha-D-Glutamyl phosphate | 1479 | C5H10NO7P | YES | | | 316 | 7287 | 0 | 0 |
| 2,3-Bisphospho-D-glycerate | 1480 | C3H8O10P2 | YES | | | 0 | 2229 | 5070 | 445 |
| Glycine | 1481 | C2H5NO2 | YES | | | 1264 | 2582 | 0 | 195,75 |
| 1-Hydroxy-2-naphthaldehyde | 1482 | C11H8O2 | YES | | | 161 | 0 | 0 | 2839,25 |
| D-Glyceraldehyde | 1483 | C3H6O3 | YES | | | 0 | 2650 | 0 | 4326 |
| D-Glycerate 3-phosphate | 1484 | C3H7O7P | YES | | | 2591 | 0 | 2180 | 457 |
| L-Glutamate | 1485 | C6H11NO4 | YES | | | 0 | 4687 | 32 | 217 |

| | | | | | | | | | |
|---|---|---|---|---|---|---|---|---|---|
| methylester | | | | | | | | | |
| D-Glycero-D-manno-heptose 1,7-bisphosphate | 1486 | C7H16O13P2 | YES | | | 0 | 0 | 0 | 143,5 |
| D-Glycero-D-manno-heptose 1-phosphate | 1487 | C7H15O10P | YES | | | 0 | 126 | 9181 | 343,75 |
| D-Glycero-D-manno-heptose 7-phosphate | 1488 | C7H15O10P | YES | | | 0 | 2880 | 0 | 5393 |
| Glycogen | 1489 | C24H42O21 | YES | | | 24 | 0 | 0 | 77,5 |
| (R)-Glycerate | 1490 | C3H6O4 | YES | | | 0 | 3310 | 0 | 142 |
| Glycerone | 1491 | C3H6O3 | YES | | | 0 | 0 | 0 | 1025,25 |
| 1,3-Bisphospho-D-glycerate | 1492 | C3H8O10P2 | YES | | | 3111 | 0 | 0 | 1728,5 |
| Glycerol | 1493 | C3H8O3 | YES | | | 2043 | 0 | 0 | 491,25 |
| Glycolate | 1494 | C2H4O3 | YES | | | 343 | 0 | 0 | 3100,75 |
| D-Glucosaminate | 1495 | C6H13NO6 | YES | | | 0 | 5676 | 0 | 4077 |
| D-Glucosaminide | 1496 | C12H24N2O10(C6H11NO3)2 | YES | | | 0 | 0 | 0 | 2651,5 |
| Glycerone phosphate | 1497 | C3H7O6S | YES | | | 0 | 22360 | 0 | 651,667 |
| Glyoxalate | 1498 | C2H2O3 | YES | | | 711 | 4171 | 0 | 6360,25 |
| D-Galactono-1,4-lactone | 1499 | C6H10O6 | YES | | | 0 | 2123 | 0 | 446,25 |
| P1,P4-Bis(5'-guanosyl) tetraphosphate | 1500 | C20H28N10O21P4 | YES | | | 0 | 3322 | 0 | 1634 |
| Geranyl diphosphate | 1501 | C10H20O7P2 | YES | | | 0 | 4256 | 4674 | 5495 |
| Guanosine | 1502 | C10H13N5O5 | YES | | | 9 | 1397 | 0 | 248 |
| Glutathione oxidized | 1503 | C20H32N6O12S2 | YES | | | 0 | 2823 | 0 | 223,5 |
| Glutathione reduced | 1504 | C10H17N3O6S | YES | | | 0 | 3953 | 0 | 113,25 |
| 2,3,4,5-Tetrahydrodipicolinate | 1505 | C7H9NO4 | YES | | | 0 | 0 | 0 | 449,333 |

| | | | | | | | | | |
|---|---|---|---|---|---|---|---|---|---|
| Glutathionylspermidine | 1506 | C17H34N6O5S | YES | | | 0 | 0 | 0 | 75,25 |
| Tetrahydropteroyltri-L-glutamate | 1507 | C29H37N9O12 | YES | | | 0 | 0 | 0 | 692 |
| Guanidoacetate | 1508 | C3H7N3O2 | YES | | | 0 | 3746 | 0 | 211 |
| Guaiacol | 1509 | C7H8O2 | YES | | | 74 | 42 | 0 | 141,5 |
| L-Gulose | 1510 | C6H12O6 | YES | | | 0 | 2450 | 0 | 311,75 |
| Glucuronoxylan 4-O-methyl-D-glucuronate | 1511 | (C22H34O18)10 | YES | | | 0 | 0 | 0 | 128,5 |
| Glucuronoxylan D-glucuronate | 1512 | (C21H32O18)10 | YES | | | 0 | 0 | 0 | 333,75 |
| 1-Hydroxy-2-methyl-2-(E)-butenyl 4-diphosphate | 1513 | C5H12O8P2 | YES | | | 0 | 0 | 4697 | 6533 |
| (E)-4-Hydroxy-3-methylbut-2-en-1-yl diphosphate | 1514 | C5H12O8P2 | YES | | | 0 | 6743 | 0 | 357,75 |
| (S)-3-Hydroxy-3-methylglutaryl-CoA | 1515 | C27H44N7O20P3S | YES | | | 1701 | 3693 | 0 | 3408,75 |
| Galactarate | 1516 | C6H10O8 | YES | | | 79 | 0 | 8067 | 4575,5 |
| But-1-ene-1,2,4-tricarboxylate | 1517 | C7H8O6 | YES | | | 32 | 3950 | 0 | 245,75 |
| Hydroxyacetone phosphate | 1518 | C3H7O5P | YES | | | 46 | 4470 | 1727 | 4215,5 |
| cis-4-(2-hydroxynaph-3-yl)-2-oxobut-3-enoic acid | 1519 | C14H10O42 | NO | see http://umbbd.ahc.umn.edu | | 0 | 4109 | 0 | 150,75 |
| 3-Hydroxy-2-naphthoate | 1520 | C11H8O3 | YES | | | 0 | 0 | 0 | 322 |
| 2,2',3,4',5,5',6-Heptachloro-4-biphenylol | 1521 | C12H3Cl7O | YES | | | 0 | 166 | 0 | 235,25 |
| (15S)-15-Hydroxy-5,8,11-cis-13-trans-eicosatetraenoate | 1522 | C20H32O3 | YES | | | 1 | 4477 | 0 | 5193,25 |

| | | | | | | | | | |
|---|---|---|---|---|---|---|---|---|---|
| L-Homocysteine | 1523 | C4H9NO2S | YES | | | 0 | 0 | 0 | 2971,25 |
| alpha-D-Glucose 6-phosphate | 1524 | C6H13O9P | YES | | | 694 | 3236 | 5795 | 6423,75 |
| Hexadecenoic acid | 1525 | C16H30O2 | NO | Palmitoleic acid; Palmitolinoleic acid; (E)-Palmitoleic acid; (E)-hexadec-9-enoic acid | 5282745 | 0 | 0 | 0 | 5127,75 |
| trans-Hexadec-2-enoyl-CoA | 1526 | C37H64N7O17P3S | YES | | | 1886 | 4568 | 0 | 5581 |
| cis-4-[2-(3-Hydroxy)-thionaphthenyl]-2-oxo-3-butenoate | 1527 | C12H8O4S | NO | see http://umbbd.ahc.umn.edu | | 0 | 0 | 3059 | 4495 |
| RNA Helicase substrate | 1528 | # | NO* | | | 0 | 912 | 1088 | 9912 |
| Heptane | 1529 | C7H16 | NO | | 152052 | 0 | 0 | 0 | 5201,25 |
| Heptadecanoyldolichol | 1530 | C37H50O2(C5H8)10 | YES* | Acyldolichol | | 153 | 0 | 0 | 387,5 |
| Heptadecanoic acid | 1531 | C17H34O2 | NO | | 153751 | 0 | 0 | 2865 | 4830,5 |
| Heptadecane | 1532 | C17H36 | YES | | | 0 | 0 | 4839 | 3529 |
| Heptadecanoyl-CoA | 1533 | C38H68N7O17P3S | NO | | 3883216 | 0 | 1156 | 0 | 222,5 |
| all-trans-Heptaprenyl diphosphate | 1534 | C35H60O7P2 | YES | | | 20 | 1965 | 20 | 396 |
| Heptanoic acid | 1535 | C7H14O2 | NO | | 151218 | 0 | 2491 | 0 | 1193,25 |
| trans-2-Methyl-5-isopropylhexa-2,5-dienoyl-CoA | 1536 | C31H50N7O17P3S | YES | | | 0 | 3526 | 5350 | 68 |
| Heptanal | 1537 | C7H14O | YES | | | 0 | 4296 | 53 | 131 |
| Hexanoic acid | 1538 | C6H12O2 | YES | | | 0 | 0 | 0 | 3266,25 |
| Hexacosane | 1539 | C26H54 | NO | | 155729 | 0 | 4009 | 0 | 475,75 |
| Hexadecane | 1540 | C16H34 | NO | Cetane; n-Cetane | 154310 | 29 | 10679 | 0 | 389 |
| 2,6-Dimethyl-5-methylene-3-oxo-heptanoyl-CoA | 1541 | C31H50N7O18P3S | YES | | | 3256 | 2999 | 0 | 272,25 |
| Hexacosanoate | 1542 | C26H52O2 | NO | Cerotic acid; Ceratinic | 153758 | 0 | 0 | 0 | 4357 |

| | | | | | | | | | |
|---|---|---|---|---|---|---|---|---|---|
| | | | | acid; Cerinic acid | | | | | |
| Hexanal | 1543 | C6H12O | NO | Caproaldehyde; n-Caproaldehyde | 149090 | 722 | 2533 | 6129 | 409,25 |
| Hexyl cinnamaldehyde | 1544 | C15H20O | NO | (2Z)-2-(phenylmethylidene)octanal; 2-Benzylideneoctanal; 2-Hexylcinnamaldehyde; alpha-Hexylcinnamaldehyde | 150665 | 0 | 3565 | 0 | 320,25 |
| 2,2',4,4',5,5'-Hexachlorobiphenyl | 1545 | C12H4Cl6 | YES | | | 0 | 0 | 0 | 784,5 |
| 2,2',4,4',6,6'-Hexachlorobiphenyl | 1546 | C12H4Cl6 | YES | | | 0 | 0 | 12527 | 500 |
| 2,2',3,3',5,5'-Hexachlorobiphenyl | 1547 | C12H4Cl6 | YES | | | 139 | 4528 | 0 | 4221,75 |
| 2,2',3,3',6,6'-Hexachlorobiphenyl | 1548 | C12H4Cl6 | YES | | | 30 | 44487 | 0 | 5363 |
| Hexanoyl-CoA | 1549 | C27H46N7O17P3S | YES | | | 0 | 244 | 64 | 5967 |
| Hexadecanoyl-CoA | 1550 | C37H66N7O17P3S | YES | | | 0 | 0 | 20 | 357,75 |
| 1,2-Epoxyhexadecane | 1551 | C16H32O | YES | | | 113 | 6328 | 0 | 1041,75 |
| Hexadecanoylglycerone phosphate | 1552 | C4H6O7P(C16H32O2) | YES* | 1-Acyl-glycerone 3-phosphate | | 0 | 4103 | 0 | 1223,5 |
| Hexanoyldolichol | 1553 | C21H35O2(C5H8)10C6H12O2 | YES* | Acyldolichol | | 0 | 0 | 0 | 644,75 |
| L-Histidinol phosphate | 1554 | C6H12N3O4P | YES | | | 1054 | 10 | 0 | 6736,75 |
| Histamine | 1555 | C5H9N3 | YES | 2-(4-Imidazolyl)ethylamine | | 2019 | 0 | 0 | 149,25 |
| (S)-3-Hydroxyisobutyrate | 1556 | C4H8O3 | YES | | | 0 | 2087 | 0 | 952,75 |
| (S)-3- | 1557 | C25H42N7O1 | YES | | | 0 | 0 | 4724 | 5765,75 |

| | | | | | | | | | |
|---|---|---|---|---|---|---|---|---|---|
| Hydroxyisobutyryl-CoA | | 8P3S | | | | | | | |
| Homoisocitrate | 1558 | C7H10O7 | YES | | | 111 | 0 | 0 | 4174,5 |
| Histone-arginine | 1559 | C7H13N5O2(CH3)2 | YES | | | 75 | 0 | 3500 | 814 |
| L-Histidine | 1560 | C6H9N3O2 | YES | | | 31 | 3028 | 0 | 1955,5 |
| Histone L-lysine | 1561 | C7H13N3O2(CH3)2 | YES | | | 0 | 67 | 14261 | 2104,5 |
| Histone N(omega)-methyl-L-arginine | 1562 | C8H15N5O2(CH3)2 | YES | | | 18 | 0 | 0 | 406 |
| 3,3',4',5,7,8-Hexahydroxyflavone | 1563 | C15H10O8 | YES | | | 0 | 0 | 5389 | 420,75 |
| D-Hexose | 1564 | C6H12O6 | YES | | | 0 | 0 | 0 | 3136,75 |
| L-Histidinol | 1565 | C6H11N3O | YES | | | 40 | 94 | 52216 | 610,75 |
| Histone | 1566 | C6H11N3O | YES | | | 0 | 2600 | 1114 | 299,25 |
| 2-Hydroxy-6-oxonona-2,4-diene-1,9-dioate | 1567 | C9H10O6 | YES | | | 133 | 5065 | 0 | 5869,5 |
| 2-Oxohept-3-enedioate | 1568 | C7H8O5 | YES | | | 0 | 2643 | 0 | 374,5 |
| 3-Hydroxy-L-kynurenine | 1569 | C10H12N2O4 | YES | | | 0 | 4583 | 45 | 678 |
| Hydroxymethylbilane | 1570 | C40H46N4O17 | YES | | | 0 | 2082 | 0 | 431 |
| (S)-3-Hydroxy-2-methylbutyryl-CoA | 1571 | C26H44N7O18P3S | YES | | | 156 | 0 | 0 | 7011,5 |
| trans-4-[2-(3-Hydroxy)-thionaphthenyl]-2-oxo-3-butenoate | 1572 | C12H8O4S | NO | see http://umbbd.ahc.umn.edu | | 0 | 6 | 0 | 192,25 |
| L-Homoserine | 1573 | C4H9NO3 | YES | | | 632 | 0 | 1199 | 4867 |
| 2-Hydroxy-2H-benzo[h]chromene-2-carboxylate | 1574 | C14H10O4 | YES | | | 0 | 2259 | 0 | 1371,25 |
| Histone N6-methyl-L- | 1575 | C8H15N3O2( | YES | | | 0 | 0 | 0 | 2199,25 |

| | | | | | | | | | |
|---|---|---|---|---|---|---|---|---|---|
| lysine | | CH3)2 | | | | | | | |
| Homogentisate | 1576 | C8H8O4 | YES | | | 1 | 4725 | 0 | 7548 |
| 3-Hydroxypropanoate | 1577 | C3H6O3 | YES | | | 52 | 102 | 0 | 1262,75 |
| 3-Hydroxypropionyl-CoA | 1578 | C24H40N7O18P3S | YES | | | 84 | 0 | 2204 | 3118,75 |
| (S)-3-Hydroxybutanoate | 1579 | C4H8O3 | YES | | | 0 | 15543 | 0 | 3610,25 |
| trans-4-Hydroxy-L-proline | 1580 | C5H9NO3 | YES | | | 847 | 0 | 4889 | 174 |
| all-trans-Hexaprenyl diphosphate | 1581 | C30H52O7P2 | YES | | | 0 | 13 | 8 | 7212,75 |
| Hydroxypyruvate | 1582 | C3H4O4 | YES | | | 4253 | 4879 | 2358 | 5125,5 |
| cis-4-Hydroxy-L-proline | 1583 | C5H9NO3 | YES | | | 43 | 0 | 0 | 628,75 |
| 4-Hydroxy-2-oxohexanoate | 1584 | C6H10O4 | YES | | | 40 | 2855 | 0 | 2799,75 |
| Hypoxanthine | 1585 | C5H4N4O | YES | | | 158 | 0 | 2724 | 5284,5 |
| trans-Hexacos-2-enoyl-CoA | 1586 | C49H88O19N7P3S | NO* | | | 28 | 6101 | 0 | 3520 |
| Hexadecanal | 1587 | C16H32O | YES | | | 0 | 35 | 0 | 5211,5 |
| 4-Hydroxy-2-oxopentanoate | 1588 | C5H8O4 | YES | | | 0 | 0 | 0 | 342,75 |
| Hydroxyatrazine | 1589 | C8H15N5O | YES | | | 9456 | 0 | 0 | 3110,75 |
| Hydantoin-5-propionate | 1590 | C6H8N2O4 | YES | | | 0 | 0 | 2232 | 766,5 |
| 2-(Indol-3-yl)acetaldehyde | 1591 | C10H9NO | YES | | | 1335 | 1693 | 0 | 6728 |
| Indole-3-acetyl-beta-1-D-glucose | 1592 | C16H19NO7 | YES | | | 0 | 0 | 0 | 8646,75 |
| Indole-3-acetyl-L-glutamine | 1593 | C16H19NO7 | NO* | | | 0 | 5247 | 95 | 7183,5 |
| Indole-3-acetyl-myo-inositol | 1594 | C16H19NO7 | YES | | | 0 | 24 | 66 | 3126,25 |
| 2-Phenylacetamide | 1595 | C8H9NO | YES | | | 4046 | 2651 | 4251 | 7480,5 |
| Indole-3-acetamide | 1596 | C10H10N2O | YES | | | 9501 | 646 | 0 | 3439,75 |

| | | | | | | | | | |
|---|---|---|---|---|---|---|---|---|---|
| (Indol-3-yl)pyruvate | 1597 | C11H9NO3 | YES | | | 7 | 3033 | 122 | 3094,75 |
| Pentadecanal | 1598 | C15H30O | YES | | | 0 | 1404 | 8 | 6861,5 |
| Indole-3-ethanol | 1599 | C10H11NO | YES | | | 0 | 6890 | 0 | 5508,25 |
| (Indol-3-yl)acetate | 1600 | C10H9NO2 | YES | | | 0 | 2603 | 0 | 3895,25 |
| Iminoaspartate | 1601 | C4H5NO4 | YES | | | 0 | 4926 | 64 | 1331,5 |
| 2-Methylpropanoyl-CoA | 1602 | C25H42N7O17P3S | YES | | | 181 | 2088 | 746 | 5465,75 |
| Isobutyryl-CoA | 1603 | C25H42N7O17P3S | YES | 2-Methylpropanoyl-CoA | | 0 | 0 | 6 | 6570,75 |
| 6-Aminohexanoate | 1604 | C6H13NO2 | YES | | | 0 | 3467 | 0 | 4376,25 |
| Isochorismate | 1605 | C10H10O6 | YES | | | 0 | 0 | 0 | 1815,25 |
| L-Idonate | 1606 | C6H12O7 | YES | | | 18 | 0 | 0 | 4104,75 |
| Indole-3-acetaldehyde | 1607 | C10H9NO | YES | | | 0 | 0 | 743 | 950,75 |
| Isocitrate | 1608 | C6H8O7 | YES | | | 5145 | 0 | 0 | 5974,25 |
| Inosine 5'-diphosphate | 1609 | C10H14N4O11P2 | YES | IDP | | 1522 | 5014 | 973 | 5363,25 |
| Gentisate aldehyde | 1610 | C7H6O3 | YES | | | 0 | 0 | 705 | 4692 |
| Indoleglycerol phosphate | 1611 | C11H14NO6P | YES | | | 0 | 0 | 2 | 4220 |
| cis-3,4-Dihydroxyphenanthrene-4-carboxylate | 1612 | C15H12O4 | NO | see http://umbbd.ahc.umn.edu | | 0 | 10067 | 0 | 385,75 |
| L-Isoleucine | 1613 | C6H13NO2 | YES | | | 0 | 113 | 0 | 381,25 |
| 3-(Imidazol-4-yl)-2-oxopropyl phosphate | 1614 | C6H9N2O5P | YES | | | 0 | 3350 | 6295 | 5240 |
| L-Histidinal | 1615 | C6H9N3O | YES | | | 352 | 4323 | 112 | 4405,5 |
| Imidazo[1,5a]pyrimidine | 1616 | C6H5N3 | NO* | | | 0 | 3465 | 19 | 1180 |
| 4-Imidazolone-5-propanoate | 1617 | C6H8N2O3 | YES | | | 28 | 0 | 0 | 15744,5 |
| Indoxazene | 1618 | C7H5NO | NO | 1,2-Benzisoxazole | 10516490 | 0 | 467 | 0 | 569,75 |
| Indane | 1619 | C9H10 | NO | 2,3-Dihydro-1H-indene | 686131 | 0 | 159 | 0 | 438,5 |
| Indene | 1620 | C9H8 | NO | 1H-Indene; | 150267 | 58 | 2562 | 0 | 90,5 |

| | | | | | | | | | |
|---|---|---|---|---|---|---|---|---|---|
| | | | | Indonaphthene | | | | | |
| Methyl 3-hydroxybenzoate | 1621 | C13H14O3 | NO | 3-Methylpent-1-yn-3-yl 3-hydroxybenzoate | 3825669 | 25 | 6753 | 1511 | 213 |
| Indoline | 1622 | C8H9N | NO | 1-Azaindan; 2,3-Dihydro-1H-indole | 153599 | 26 | 1850 | 0 | 939,75 |
| 2,3-Benzopyrrole | 1623 | C8H7N | YES | | | 1745 | 1601 | 0 | 8003,75 |
| myo-Inositol | 1624 | C6H12O6 | YES | | | 2564 | 3061 | 0 | 3879,5 |
| Indole-3-pyruvate | 1625 | C11H9NO3 | YES | | | 0 | 18 | 0 | 3887,5 |
| Inosine | 1626 | C10H12N4O5 | YES | | | 3091 | 2811 | 0 | 2405 |
| Inosine 5'-tetraphosphate | 1627 | C10H16N4O17P4 | YES | | | 0 | 0 | 0 | 3779,5 |
| Inosine 5'-triphosphate | 1628 | C10H15N4O14P3 | YES | ITP | | 856 | 1484 | 0 | 292,25 |
| Isopentenyl diphosphate | 1629 | C5H12O7P2 | YES | | | 212 | 1709 | 0 | 5052,75 |
| 2-Isopropylmalate | 1630 | C7H12O5 | YES | | | 0 | 0 | 0 | 370,5 |
| Isothiazoline | 1631 | C3H2NOS | NO* | | | 0 | 0 | 0 | 1349,75 |
| Isobenzofuran | 1632 | C8H6O | NO | 2-Benzofuran | 17425447 | 0 | 0 | 0 | 439,75 |
| Isomaltose | 1633 | C12H22O11 | YES | | | 91 | 3264 | 2272 | 3095 |
| Isochromen-3-one | 1634 | C9H6O2 | NO | | 37499628 | 0 | 23352 | 0 | 568,75 |
| Isocoumarin | 1635 | C9H6O2 | NO | Isochromen-1-one; 1H-2-Benzopyran-1-one; 3,4-Benzo-2-pyrone | 210232 | 0 | 2331 | 0 | 261,25 |
| 2-Methylpropanoate | 1636 | C4H8O2 | YES | | | 0 | 0 | 34 | 1083,25 |
| Isoorientin | 1637 | C21H20O11 | YES | | | 0 | 0 | 0 | 3134 |
| Isopentanoyl-CoA | 1638 | C26H44N7O17P3S | NO* | | | 0 | 0 | 0 | 16135 |
| Isoprene | 1639 | C5H8 | YES | | | 0 | 0 | 0 | 308,25 |
| L-Carnitinamide | 1640 | C7H17N2O2 | YES | | | 0 | 0 | 0 | 229 |
| 2-Benzazine | 1641 | C9H7N | YES | | | 6 | 3939 | 0 | 52,75 |
| Isoscoparin | 1642 | C22H22O11 | YES | | | 0 | 0 | 0 | 277,75 |
| Imidazolone | 1643 | C3H4N2O | YES | | | 129 | 4597 | 0 | 4236,75 |
| Ethyl isovalerate | 1644 | C7H14O2 | YES | | | 51 | 0 | 0 | 515,25 |
| Isovaleryl-CoA | 1645 | C26H44N7O1 | YES | | | 0 | 6263 | 0 | 5837,25 |

| | | | | | | | | | |
|---|---|---|---|---|---|---|---|---|---|
| | | 7P3S | | | | | | | |
| Isoxazolidine | 1646 | C3H7NO | NO | 1,2-Oxazolidine | 712559 | 0 | 139 | 0 | 379 |
| Isoxazole | 1647 | C3H3NO | NO | 1,2-Oxazole | 152445 | 0 | 4943 | 0 | 6350,5 |
| 7-Hydroxycoumarin | 1648 | C9H6O3 | YES | | | 158 | 3825 | 0 | 241,25 |
| Carnitine | 1649 | C7H16NO3 | YES | | | 0 | 3137 | 0 | 1114 |
| (2S)-2-Isopropyl-3-oxosuccinate | 1650 | C7H10O5 | YES | | | 488 | 0 | 128 | 1825,75 |
| 5-Carboxymethyl-2-hydroxymuconate semialdehyde | 1651 | C8H6O6 | NO | see http://umbbd.ahc.umn.edu | | 176 | 29 | 8622 | 4628,5 |
| Hexose-1,5-lactone | 1652 | C6H10O6 | YES | | | 28 | 4675 | 1014 | 3416 |
| Kanosamine 6-Phosphate | 1653 | C6H14NO8P | YES | | | 25 | 0 | 1653 | 5640 |
| 3-Deoxy-D-manno-2-octulosonate | 1654 | C8H14O8 | YES | | | 0 | 3853 | 64 | 262,5 |
| 3-Deoxy-D-manno-octulosonate 8-phosphate | 1655 | C8H15O11P | YES | | | 0 | 1597 | 0 | 6564,5 |
| alpha-Ketoglutaric acid | 1656 | C5H6O5 | YES | | | 0 | 219 | 0 | 1717 |
| Ketoprofen methyl ester | 1657 | C17H16O3 | NO | Methyl 2-[3-(benzoyl)phenyl]propanoate | 10450428 | 138 | 71 | 2776 | 7527,25 |
| (R)-2-Methylmalate | 1658 | C5H8O5 | YES | | | 0 | 0 | 0 | 6284 |
| Ketosamine | 1659 | C13H16ClNO | NO* | | | 3 | 0 | 0 | 680 |
| Ketose | 1660 | C6H12O6 | YES | | | 0 | 0 | 0 | 1643,25 |
| 5-Carboxymethyl-2-hydroxymuconate | 1661 | C8H6O72 | NO | see http://umbbd.ahc.umn.edu | | 0 | 93 | 0 | 4849,25 |
| Jasmonate | 1662 | C12H18O3 | YES | | | 0 | 2014 | 0 | 3202,25 |
| Laminarin | 1663 | C12H22O11(C6H10O5)10 | YES | | | 0 | 3352 | 0 | 641,5 |
| L-Arabinonate | 1664 | C5H10O6 | YES | | | 0 | 0 | 0 | 4213 |
| Lactitol dihydrate | 1665 | C11H22O11 | YES | | | 0 | 10 | 7600 | 2099 |
| L-Lactaldehyde | 1666 | C3H6O2 | YES | | | 0 | 6447 | 13 | 2575 |

| | | | | | | | | | |
|---|---|---|---|---|---|---|---|---|---|
| D-Lactate | 1667 | C3H6O3 | YES | | | 140 | 0 | 0 | 4879,5 |
| L-Arabinitol | 1668 | C5H12O5 | YES | | | 0 | 42 | 9 | 173,25 |
| L-Arabitol | 1669 | C5H12O5 | YES | | | 0 | 8912 | 0 | 439,5 |
| L-Arabonate | 1670 | C5H10O6 | NO* | | | 0 | 68 | 1042 | 13,25 |
| L-Lactate | 1671 | C3H6O3 | YES | | | 1115 | 5413 | 8759 | 77,5 |
| L-Arogenate | 1672 | C10H13NO5 | YES | | | 40 | 5182 | 0 | 5115,75 |
| L-Ascorbic acid | 1673 | C6H8O6 | YES | | | 353 | 0 | 0 | 405,25 |
| L-Leucine | 1674 | C6H13NO2 | YES | | | 936 | 1923 | 0 | 4872,25 |
| Itaconyl-CoA | 1675 | C26H40N7O19P3S | YES | | | 6934 | 2591 | 0 | 2140,25 |
| Itaconate | 1676 | C5H6O4 | YES | | | 1038 | 10309 | 0 | 100,5 |
| Lactose | 1677 | C12H22O11 | YES | | | 0 | 0 | 0 | 4564,5 |
| 5-Aminoimidazole | 1678 | C3H5N3 | YES | | | 72 | 24 | 27 | 148 |
| Leucine p-nitroaniline | 1679 | C12H17N3O3 | NO* | | | 0 | 31 | 159 | 629,25 |
| L-Formylkynurenine | 1680 | C11H12N2O4 | YES | | | 0 | 3020 | 0 | 462,5 |
| L-Gulono-1,4-lactone | 1681 | C6H10O6 | YES | | | 0 | 5351 | 0 | 464,75 |
| L-Leucyl-glycyl-glycine | 1682 | C10H19N3O4 | NO | | 213084 | 0 | 7138 | 0 | 200,5 |
| L-Homocitrulline | 1683 | C7H15N3O3 | YES | | | 60 | 0 | 5192 | 674,25 |
| L-Gulonate | 1684 | C6H12O7 | YES | | | 0 | 3878 | 0 | 378,25 |
| (R)-S-Lactoylglutathione | 1685 | C13H21N3O8S | YES | | | 602 | 0 | 0 | 5487,5 |
| Licodione | 1686 | C15H12O5 | YES | | | 19 | 0 | 2502 | 2809,25 |
| L-Iditol | 1687 | C6H14O6 | YES | | | 0 | 0 | 27 | 951,25 |
| Limonoate | 1688 | C26H34O10 | YES | | | 0 | 0 | 0 | 294,75 |
| Limonene-1,2-diol | 1689 | C10H18O2 | YES | | | 153 | 4676 | 56 | 2279,5 |
| (-)-(S)-Limonene | 1690 | C10H16 | YES | | | 147 | 4269 | 0 | 2085,5 |
| Limonene-1,2-epoxide | 1691 | C10H16O | YES | | | 0 | 0 | 0 | 8258,75 |
| Lindane | 1692 | C6H6Cl6 | YES | | | 0 | 0 | 6657 | 5732 |
| Linolenate | 1693 | C18H30O2 | YES | | | 0 | 0 | 17 | 4365 |
| Linoleate | 1694 | C18H32O2 | YES | | | 0 | 0 | 5 | 4097,25 |
| Linoleyl-CoA | 1695 | C39H66N7O17P3S | NO | | 841149 | 0 | 0 | 0 | 705 |

| | | | | | | | | | |
|---|---|---|---|---|---|---|---|---|---|
| 2,3-Bis(3-Hydroxytetradecanoyl)-D-glucosaminyl-1,6-beta-D-2,3-bis(3-hydroxytetradecanoyl)-beta-D-glucosaminyl 1-phosphate | 1696 | C68H129N2O20P | YES | | | 0 | 8151 | 5671 | 6060,75 |
| 2,3-Bis(3-hydroxytetradecanoyl)-beta-D-glucosaminyl 1-phosphate | 1697 | C34H66NO12P | YES | | | 0 | 0 | 7146 | 829,75 |
| 3-Methylsalicylaldehyde | 1698 | C8H8O2 | NO | see http://umbbd.ahc.umn.edu | | 45 | 10651 | 0 | 367,75 |
| L-Kynurenine | 1699 | C10H12N2O3 | YES | | | 32 | 0 | 0 | 3863 |
| DL-Leucine, benzyl ester | 1700 | C13H19NO2 | NO | Phenylmethyl 2-amino-4-methylpentanoate | 10366555 | 0 | 3717 | 1728 | 1962,5 |
| 6-Lactoyl-5,6,7,8-tetrahydropterin | 1701 | C9H13N5O3 | YES | | | 136 | 18167 | 0 | 1568 |
| 2-Methyl-3-oxopropanoate | 1702 | C4H6O3 | YES | | | 2368 | 0 | 30 | 3662,25 |
| 2-Methylhomogentisate | 1703 | C9H10O4 | YES | | | 0 | 0 | 2861 | 373,5 |
| (+)-(R)-Limonene | 1704 | C10H16 | YES | | | 0 | 0 | 0 | 1101,25 |
| L-Palmitoylcarnitine | 1705 | C23H45NO4 | YES | | | 0 | 0 | 0 | 14294,8 |
| L-Rhamno-1,4-lactone | 1706 | C6H10O5 | YES | | | 0 | 1774 | 0 | 924 |
| L-Xylono-1,4-lactone | 1707 | C5H8O5 | YES | | | 0 | 0 | 3939 | 2676,25 |
| L-Xylo-Hex-3-ulono-1,4-lactone | 1708 | C6H8O6 | YES | | | 0 | 0 | 0 | 0 |
| L-Xylonate | 1709 | C5H10O6 | YES | | | 50 | 0 | 0 | 2696 |
| Methyl-2-bromopropionate | 1710 | C4H7BrO2 | NO | Methyl-2-bromo-propionic acid | 672736 | 0 | 0 | 0 | 409,75 |
| Methyl 2-hydroxybenzoate | 1711 | C8H8O3 | NO | Methyl salicylate | 151540 | 0 | 3900 | 7037 | 5875,75 |
| N-Methyl-(R,S)-1- | 1712 | C17H19N | YES | | | 0 | 1389 | 0 | 293,5 |

| | | | | | | | | | |
|---|---|---|---|---|---|---|---|---|---|
| benzyl-1,2,3,4-tetrahydroisoquinoline | | | | | | | | | |
| alpha-D-Mannosyl-beta-D-mannosyl-diacetylchitobiosyldiphosphodolichol | 1713 | C48H84N2O27P2(C5H8)10 | YES | | | 0 | 0 | 0 | 2522,5 |
| Methyl 2,2-dimethyl-1,3-dioxane-4-carboxylate | 1714 | C10H18O4 | NO* | | | 0 | 5038 | 0 | 6861,75 |
| L-Lysine | 1715 | C6H14N2O2 | YES | | | 582 | 3011 | 2606 | 8270,75 |
| 3-Methylsalicylate | 1716 | C8H8O3 | YES | | | 3798 | 0 | 131 | 2843,75 |
| (alpha-D-Mannosyl)4-beta-D-mannosyl-diacetylchitobiosyldiphosphodolichol | 1717 | C66H114N2O42P2(C5H8)10 | YES | | | 0 | 0 | 0 | 311,25 |
| Methyl-2-hydroxy-2-methylpropionate | 1718 | C5H10O3 | NO | Methyl-3-hydroxy-2-methylpropionic acid; Methyl 2-methyllactate; Methyl 2-hydroxyisobutyrate; Methyl alpha-hydroxyisobutyrate | 217250 | 256 | 0 | 3060 | 2309 |
| Methyl-3-bromopropionate | 1719 | C4H7BrO2 | NO | Methyl-3-bromo-propionic acid; Methyl 3-bromopropionate; Methyl 3-bromopropanoate; Methyl beta-bromopropionate | 654006 | 5174 | 0 | 0 | 2228 |
| Melibiitol | 1720 | C12H24O11 | YES | | | 0 | 3127 | 1100 | 10302,8 |
| Inosine 5'-monophosphate | 1721 | C10H13N4O8P | YES | IMP | | 1007 | 0 | 3322 | 304,75 |
| 3-Oxohexanoate | 1722 | C6H10O3 | YES | | | 2821 | 0 | 0 | 376 |
| cis-1,2-Dihydroxy-4-methylcyclohexa-3,5-diene-1-carboxylate | 1723 | C8H10O4 | YES | | | 115 | 0 | 0 | 661,5 |

| | | | | | | | | | |
|---|---|---|---|---|---|---|---|---|---|
| 4-Maleylacetoacetate | 1724 | C8H8O6 | YES | | | 3659 | 4767 | 1999 | 2729,25 |
| Methyl 4-hydroxybenzoate | 1725 | C8H8O3 | NO | Methyl paraben | 150527 | 0 | 0 | 0 | 2584,25 |
| beta-D-Mannosyldiacetylchitobiosyldiphosphodolichol | 1726 | C42H74N2O22P2(C5H8)10 | YES | | | 5 | 2761 | 25411 | 417 |
| 2-Methylacetoacetyl-CoA | 1727 | C26H42N7O18P3S | YES | | | 0 | 10501 | 0 | 8566 |
| 2-Methylprop-2-enoyl-CoA | 1728 | C25H40N7O17P3S | YES | | | 127 | 3336 | 62 | 7715 |
| 3-Methylmuconolactone | 1729 | C7H8O4 | YES | | | 0 | 0 | 0 | 6020,25 |
| Malathion | 1730 | C10H19O6PS2 | YES | | | 0 | 3188 | 0 | 847,75 |
| Malonyl-CoA | 1731 | C24H38N7O19P3S | YES | | | 0 | 4743 | 0 | 4620 |
| Maleate | 1732 | C4H4O4 | YES | | | 12 | 3453 | 0 | 2461 |
| Melilotate | 1733 | C9H10O3 | YES | | | 107 | 3571 | 0 | 6986,25 |
| Maltitol | 1734 | C12H24O11 | NO* | | | 128 | 0 | 0 | 702,5 |
| Malonate | 1735 | C3H4O4 | YES | | | 85 | 1709 | 0 | 2849,75 |
| L-Mannuronate | 1736 | C6H10O7 | NO* | | | 0 | 6286 | 0 | 9479 |
| Methylmalonate | 1737 | C4H6O4 | YES | | | 98 | 3366 | 0 | 7629 |
| alpha-Maltose | 1738 | C12H22O11 | YES | | | 46 | 3621 | 93 | 234,5 |
| Maltose 6'-phosphate | 1739 | C12H23O14P | YES | | | 0 | 0 | 1 | 418 |
| beta-Maltose | 1740 | C12H22O11 | YES | | | 0 | 0 | 0 | 371,75 |
| Isomaltotriose | 1741 | C18H32O16 | YES | | | 0 | 4184 | 0 | 2510 |
| Isomaltotetraose | 1742 | C24H42O21 | YES* | Isomaltosaccharide | 3881807 | 25 | 2706 | 0 | 3502 |
| Isomaltopentaose | 1743 | C30H52O26 | YES | Isomaltosaccharide | 3882491 | 0 | 3540 | 0 | 345,25 |
| Isomaltohexaose | 1744 | C36H62O31 | NO | Isomaltosaccharide | 766498 | 0 | 0 | 2546 | 253 |
| Methyl-3-bromo-2-methylpropionate | 1745 | C5H9BrO2 | NO | Methyl-3-bromo-2-methylpropionic acid; Methyl 3-bromo-2-methylpropanoate | 10380355 | 0 | 5809 | 3941 | 6479,5 |

| | | | | | | | | | |
|---|---|---|---|---|---|---|---|---|---|
| Methylmalonate | 1746 | C4H6O4 | YES | | | 0 | 84 | 2120 | 541,75 |
| 2-Ethyl-2-methyl-4-butyrolactone | 1747 | C7H12O2 | YES | | | 0 | 5259 | 8997 | 507 |
| Mannobiose | 1748 | C12H22O11 | YES | | | 0 | 0 | 7149 | 3160,75 |
| (R)-Mandelate | 1749 | C8H8O3 | YES | | | 0 | 0 | 0 | 1984 |
| (-)-Menthol | 1750 | C10H20O | YES | | | 0 | 5448 | 0 | 718,5 |
| D-Mannosamine | 1751 | C6H13NO5 | YES | | | 0 | 0 | 0 | 4049,25 |
| D-Mannose | 1752 | C6H12O6 | YES | | | 1 | 2494 | 0 | 923,75 |
| (S)-Mandelate | 1753 | C8H8O3 | YES | | | 0 | 4874 | 0 | 430,5 |
| Mannotriose | 1754 | C18H32O16 | NO | | 698844 | 0 | 0 | 0 | 4551,25 |
| Mannotetraose | 1755 | C24H42O21 | NO | | 3883222 | 0 | 17 | 2028 | 2689,25 |
| 2-Methylbutyryl-CoA | 1756 | C26H44N7O17P3S | YES | | | 30 | 0 | 3277 | 1406,25 |
| Malonate semialdehyde | 1757 | C3H4O3 | YES | | | 2302 | 5 | 0 | 7889,5 |
| 2-Methylbut-2-enoyl-CoA | 1758 | C26H42N7O17P3S | YES | | | 3974 | 1378 | 0 | 15626,8 |
| m-Cresol | 1759 | C7H8O | YES | | | 171 | 4120 | 0 | 171 |
| Melibiose | 1760 | C12H22O11 | YES | | | 0 | 0 | 251 | 607,5 |
| 1,2-Dihydroxy-3-methylcyclohexa-3,5-dienecarboxylate | 1761 | C8H10O4 | YES | | | 143 | 2598 | 0 | 2716,25 |
| meso-2,6-Diaminopimelate | 1762 | C7H14N2O4 | YES | | | 2590 | 7101 | 0 | 323,75 |
| D-Mannose 1-phosphate | 1763 | C6H13O9P | YES | | | 3619 | 3099 | 0 | 403,5 |
| D-Mannose 6-phosphate | 1764 | C6H13O9P | YES | | | 1783 | 3497 | 36 | 850,25 |
| (-)-Menthyl acetate | 1765 | C12H22O2 | YES | | | 1878 | 1813 | 0 | 3498 |
| Mannan | 1766 | C66H100NO49 | YES | | | 204 | 1883 | 1830 | 4914 |
| (-)-Menthone | 1767 | C10H18O | YES | | | 497 | 3684 | 3446 | 1214,25 |
| Methyl 3-hydroxybutyrate | 1768 | C5H10O3 | NO | Methyl-3-hydroxybutyrate | 158466 | 0 | 0 | 418 | 1441,25 |

| | | | | | | | | | |
|---|---|---|---|---|---|---|---|---|---|
| o-Methylbenzoate | 1769 | C8H8O2 | YES | | | 110 | 0 | 0 | 7151,75 |
| 2-Keto-3-deoxy-6-phosphogluconate | 1770 | C6H11O9P | YES | | | 1813 | 4839 | 0 | 4762,75 |
| Methyl cinnamate | 1771 | C10H10O2 | YES | | | 78 | 8849 | 0 | 520,75 |
| D-Methionine | 1772 | C5H11NO2S | YES | | | 71 | 7040 | 0 | 3326,5 |
| 2-Amino-2-methylpropanoate | 1773 | C4H9NO2 | YES | | | 94 | 3982 | 0 | 2367,75 |
| 5,10-Methenyltetrahydrofolate | 1774 | C20H22N7O6 | YES | | | 697 | 0 | 0 | 603,5 |
| Methylmalonate | 1775 | C4H6O4 | YES | | | 520 | 0 | 0 | 475,75 |
| L-Methionine | 1776 | C5H11NO2S | YES | | | 1721 | 77 | 0 | 5705 |
| 3-Methyl-cis,cis-muconate | 1777 | C7H8O4 | YES | | | 779 | 49 | 1123 | 4552,75 |
| Benzylparaben | 1778 | C14H12O3 | YES | | | 228 | 0 | 6494 | 13771 |
| Methylparathion | 1779 | C8H10NO5PS | YES | | | 367 | 0 | 0 | 14297,3 |
| m-Methylbenzoate | 1780 | C8H8O2 | YES | | | 132 | 7959 | 0 | 1799,5 |
| Xanthosine | 1781 | C10H12N4O6 | YES | | | 4201 | 0 | 0 | 7409,75 |
| Mevalonic acid | 1782 | C6H12O4 | YES | | | 40 | 2246 | 870 | 280,25 |
| Methyl jasmonate | 1783 | C13H20O3 | YES | | | 0 | 4633 | 18 | 4112,25 |
| 1D-myo-Inositol 1-phosphate | 1784 | C6H13O9P | YES | | | 6119 | 1980 | 0 | 1179 |
| 3-Methylglutaconyl-CoA | 1785 | C27H42N7O19P3S | YES | | | 0 | 0 | 1163 | 1463,75 |
| 4-Methylmuconolactone | 1786 | C7H8O4 | YES | | | 0 | 4269 | 0 | 1429,5 |
| N-Methyl-L-histidine | 1787 | C7H11N3O2 | YES | | | 0 | 0 | 1970 | 812 |
| Mevaldate | 1788 | C6H10O4 | YES | | | 80 | 42 | 0 | 9369,75 |
| 7-Methylxanthine | 1789 | C6H6N4O2 | YES | | | 0 | 4063 | 1837 | 2974,5 |
| Methylisocitrate | 1790 | C7H10O7 | YES | | | 3036 | 0 | 719 | 1218,75 |
| Limonoate D-ring-lactone | 1791 | C26H30O8 | YES | | | 0 | 0 | 58 | 311 |
| 7-Methyluric acid | 1792 | C6H6N4O3 | YES | | | 42 | 2576 | 0 | 579,5 |
| 1,7-Dimethyluric acid | 1793 | C7H8N4O3 | YES | | | 0 | 6030 | 4875 | 1273,25 |

| | | | | | | | | | |
|---|---|---|---|---|---|---|---|---|---|
| L-Malate | 1794 | C4H6O5 | YES | | | 2405 | 4073 | 0 | 7019,25 |
| 5,10-Methylenetetrahydrofolate | 1795 | C20H23N7O6 | YES | | | 901 | 0 | 2527 | 1257,75 |
| (R)-Methylmalonyl-CoA | 1796 | C25H40N7O19P3S | YES | | | 44 | 0 | 3894 | 878,25 |
| (S)-Methylmalonyl-CoA | 1797 | C25H40N7O19P3S | YES | | | 224 | 0 | 0 | 6574,25 |
| (S)-Methylmalonate semialdehyde | 1798 | C4H6O3 | YES | | | 1870 | 5748 | 0 | 3656,75 |
| L-Met-L-Met-L-Met | 1799 | C17H32N4P5S3 | NO* | | | 67 | 6864 | 4580 | 6016,75 |
| N-Acetylmethionine | 1800 | C7H13NO3S | YES | | | 36 | 0 | 0 | 263,75 |
| D-Mannitol | 1801 | C6H14O6 | YES | | | 0 | 5701 | 0 | 223,75 |
| D-Mannitol 1-phosphate | 1802 | C6H15O9P | YES | | | 138 | 0 | 0 | 3331,25 |
| Tetrahydro-1,4-oxazine | 1803 | C4H9NO | YES | | | 401 | 0 | 0 | 261,75 |
| 4-Hydroxyphenylacetate | 1804 | C8H8O3 | YES | | | 0 | 4435 | 4882 | 744,25 |
| 2D-5-O-methyl-2,3,5/4,6-pentahydroxycyclohexanone | 1805 | C7H12O6 | YES | | | 0 | 0 | 22 | 7367,75 |
| Menaquinol | 1806 | C16H18O2(C5H8)10 | YES | | | 74 | 2584 | 0 | 727,75 |
| Menaquinone | 1807 | C16H16O2(C5H8)10 | YES | | | 60 | 1588 | 0 | 6015,25 |
| 5-Methyltetrahydropteroyltri-L-glutamate | 1808 | C30H39N9O12 | YES | | | 0 | 0 | 0 | 3083,25 |
| Methylglyoxal | 1809 | C3H4O2 | YES | | | 866 | 2 | 0 | 15785,5 |
| (S)-5-Oxo-2,5-dihydrofuran-2- | 1810 | C6H6O4 | YES | | | 2628 | 5174 | 0 | 4516,25 |

| | | | | | | | | | |
|---|---|---|---|---|---|---|---|---|---|
| acetate | | | | | | | | | |
| Mucic acid | 1811 | C6H10O8 | YES | | | 14 | 3622 | 0 | 4261,75 |
| (R)-Mevalonate | 1812 | C6H12O4 | YES | | | 0 | 2954 | 0 | 194 |
| Phenanthrene-4-carboxylate | 1813 | C15H10O2 | NO | see http://umbbd.ahc.umn.edu | | 0 | 0 | 0 | 5487 |
| N1-Acetylspermidine | 1814 | C9H21N3O | YES | | | 95 | 0 | 1205 | 3559 |
| cis,cis-Muconate | 1815 | C6H6O4 | YES | | | 1801 | 4398 | 0 | 1929,25 |
| 4alpha-Methylzymosterol | 1816 | C28H46O | YES | | | 0 | 1167 | 0 | 6697 |
| 3-Hydroxyisovaleryl-CoA | 1817 | C26H44N7O18P3S | YES | | | 1874 | 857 | 0 | 1094 |
| N4-Acetylaminobutanal | 1818 | C6H11NO2 | YES | | | 1436 | 1964 | 0 | 383 |
| 2-Oxopropanoate | 1819 | C3H4O3 | YES | | | 0 | 5399 | 0 | 142,5 |
| N1-Acetylspermine | 1820 | C12H28N4O | YES | | | 0 | 4671 | 0 | 194,75 |
| 2-Nonaprenyl-4-hydroxybenzoate | 1821 | C52H78O3 | NO | | 771121 | 57 | 0 | 0 | 3490,25 |
| N-Acetyl-4-O-acetylneuraminate | 1822 | C13H21NO10 | YES | | | 76 | 0 | 0 | 3831,25 |
| N-Acetyl-5-methoxytryptamine | 1823 | C13H16N2O2 | YES | | | 53 | 0 | 9371 | 5200,25 |
| Nicotinate D-ribonucleoside | 1824 | C11H14NO6 | YES | | | 1008 | 0 | 9461 | 335,75 |
| N-Acetylarylamine | 1825 | C8H9NO | YES | | | 0 | 6117 | 25 | 7527 |
| N-Benzoyl-D-arginine-4-nitroanilide | 1826 | C19H22N6O4 | YES | | | 174 | 2557 | 0 | 357,5 |
| N-Acetyl-L-aspartate | 1827 | C6H9NO5 | YES | | | 72 | 0 | 0 | 6552,5 |
| Nicotinate | 1828 | C6H5NO2 | YES | | | 783 | 1134 | 393 | 1417 |
| N-Acetylgalactosamine | 1829 | C8H15NO6 | YES | | | 0 | 563 | 9 | 0 |
| 1,2-Anthracenediol | 1830 | C14H10O2 | YES | | | 123 | 7921 | 4404 | 2448,75 |
| Nicotinamide adenine dinucleotide | 1831 | C21H28N7O14P2 | YES | NAD+; NAD | | 1166 | 3603 | 11873 | 224,5 |
| 2-Naphthaldehyde | 1832 | C11H8O | NO | see | | 432 | 0 | 112 | 487,25 |

| | | | | | | | | | |
|---|---|---|---|---|---|---|---|---|---|
| | | | | http://umbbd.ahc.umn.edu | | | | | |
| N-Acetyl-D-galactosaminoglycan | 1833 | # | YES | | | 0 | 5115 | 0 | 2982 |
| NADH | 1834 | C21H29N7O14P2 | YES | | | 139 | 0 | 147 | 6489,5 |
| Nicotinamide adenine dinucleotide phosphate | 1835 | C21H29N7O17P3 | YES | NADP+; NADP | | 1751 | 27 | 0 | 818,75 |
| N-Acetylglycinamide | 1836 | C4H8N2O2 | NO | N-Acetyl-2-aminoacetamide; 2-Acetamidoacetamide | 218018 | 68 | 0 | 0 | 2714,75 |
| N-Acetyl-D-tryptophan | 1837 | C13H14N2O3 | YES | | | 45 | 1485 | 673 | 1617,25 |
| NADPH | 1838 | C21H30N7O17P3 | YES | | | 368 | 0 | 0 | 4520 |
| N-Acetyl-N-hydroxy-L-lysine | 1839 | C8H16N2O4 | NO* | | | 702 | 1718 | 0 | 367,25 |
| N-Ribosylnicotinamide | 1840 | C11H15N2O5 | YES | | | 1790 | 0 | 0 | 4535,5 |
| N-Acetylimidazole | 1841 | C5H6N2O | YES | | | 375 | 3677 | 72 | 42,75 |
| N6-Acetyl-L-lysine | 1842 | C8H16N2O3 | YES | | | 1424 | 6449 | 68 | 0 |
| N-Acetyl-L-phenylalanine | 1843 | C11H13NO3 | YES | | | 0 | 3100 | 0 | 6070,5 |
| N-Acetyl-L-histidine | 1844 | C8H11N3O3 | YES | | | 0 | 59 | 0 | 2042 |
| Nonadecanoic acid methyl ester | 1845 | C20H40O2 | NO | Methyl nonadecanoate | 158914 | 358 | 0 | 0 | 988 |
| Nonadecanoyldolichol | 1846 | C21H35O2(C5H8)5C19H38O2 | YES* | Acyldolichol | | 43 | 49 | 0 | 1367,75 |
| 2-Naphthalenesulfonate | 1847 | C10H8O3S | NO | see http://umbbd.ahc.umn.edu | | 124 | 2771 | 403 | 4195,5 |
| n-Butanol | 1848 | C4H10O | YES | | | 7598 | 0 | 89 | 1028,25 |
| 2-Naphthalenol | 1849 | C10H8O | NO | | 151823 | 2991 | 54 | 0 | 3684,25 |
| N-Acetylserotonin | 1850 | C12H14N2O2 | YES | | | 0 | 1767 | 0 | 119,75 |
| N-Acetyl-L-tyrosine | 1851 | C13H17NO4 | YES | | | 0 | 1690 | 0 | 0 |

| | | | | | | | | | |
|---|---|---|---|---|---|---|---|---|---|
| ethyl ester | | | | | | | | | |
| N-Benzoyl-4-hydroxyanthranilate | 1852 | C14H11NO4 | YES | | | 0 | 0 | 0 | 634,25 |
| N-Benzoyl-4-methoxyanthranilate | 1853 | C15H13NO4 | YES | | | 0 | 34188 | 0 | 86,25 |
| Ethyl benzoate | 1854 | C9H10O2 | NO | Benzoic ether; Benzoic acid, ethyl ester | 150204 | 1551 | 0 | 0 | 0 |
| N-Benzoylglycine | 1855 | C9H9NO3 | YES | | | 0 | 2669 | 7987 | 380,5 |
| cis-2-Chlorodienelactone | 1856 | C6H3ClO4 | YES | | | 768 | 3366 | 51 | 149,75 |
| N-Butyryl-L-homoserine lactone | 1857 | C8H13NO3 | YES | | | 0 | 3728 | 17641 | 65,25 |
| N-(3-Oxododecanoyl)homoserine lactone | 1858 | C16H27NO4 | YES | | | 0 | 0 | 0 | 2243,5 |
| N-(3-Oxooctanoyl)homoserine lactone | 1859 | C12H19NO4 | YES | | | 69 | 0 | 383 | 3013 |
| Naphthalene | 1860 | C10H8 | YES | | | 0 | 15 | 0 | 338,25 |
| Nicotinic acid amide | 1861 | C6H6N2O | YES | | | 102 | 146 | 0 | 4638,25 |
| N-Carbamoyl-beta-alanine | 1862 | C4H8N2O3 | YES | | | 882 | 0 | 0 | 150,5 |
| 3,6-Dichlorocatechol | 1863 | C6H4Cl2O2 | YES | | | 38 | 2120 | 0 | 1949 |
| N-Carbamoylbeta-glycine | 1864 | C3H5N2O3(CH3) | YES* | N-Carbamoyl-D-amino acid | | 0 | 0 | 3406 | 2361 |
| N-Carbamoylputrescine | 1865 | C5H13N3O | YES | | | 0 | 837 | 0 | 0 |
| 2,5-Dichloro-cis,cis-muconate | 1866 | C6H4Cl2O4 | YES | | | 320 | 0 | 0 | 517,25 |
| N-Dodecanoyl-glycine | 1867 | C3H3NO3(C12H24O2) | YES* | N-Acyl-D-amino acid | | 0 | 0 | 0 | 3845,5 |
| N-Decanoyl-glycine | 1868 | C3H3NO3(C10H20O2) | YES* | N-Acyl-D-amino acid | | 92 | 17 | 0 | 511,5 |
| Nerol | 1869 | C10H18O | YES | | | 52 | 4309 | 0 | 4132 |
| 1,6-Dihydroxy-cis-2,4- | 1870 | C7H8O4 | YES | | | 3185 | 0 | 5 | 311 |

| | | | | | | | | | |
|---|---|---|---|---|---|---|---|---|---|
| cyclohexadiene-1-carboxylic acid | | | | | | | | | |
| Neral | 1871 | C10H16O | YES | | | 0 | 0 | 0 | 8305 |
| (+)-Neomenthol | 1872 | C10H20O | YES | | | 0 | 1056 | 0 | 287,75 |
| Neooctane | 1873 | C8H18 | NO* | | | 0 | 5780 | 17 | 4493,25 |
| Neotetradecane | 1874 | C14H30 | NO* | | | 2093 | 555 | 0 | 6417,5 |
| gamma-L-Glutamylputrescine | 1875 | C9H19N3O3 | YES | | | 2727 | 0 | 0 | 4845,5 |
| 7-Methylxanthosine | 1876 | C11H15N4O6 | YES | | | 3931 | 2607 | 0 | 10994,8 |
| N-Heptadecanoyl-glycine | 1877 | C3H3NO3(C17H34O2) | YES* | N-Acyl-D-amino acid | | 11 | 6298 | 0 | 6960,75 |
| L-4-Hydroxyphenylglycine | 1878 | C8H9NO3 | YES | | | 0 | 3884 | 0 | 369,25 |
| N-Hexadecanoyl-glycine | 1879 | C3H3NO3(C16H32O2) | YES* | N-Acyl-D-amino acid | | 88 | 2166 | 0 | 1778,25 |
| (3R)-3-Isoprenyl-6-oxoheptanoyl-CoA | 1880 | C31H50N7O18P3S | NO | | | 0 | 5448 | 0 | 487,5 |
| N-Hexanoyl-glycine | 1881 | C3H3NO3(C6H12O2) | YES* | N-Acyl-D-amino acid | | 0 | 1039 | 0 | 3766,25 |
| N-Hydroxy-L-lysine | 1882 | C3H3NO3(CH3) | YES* | N-Acyl-L-amino acid | | 0 | 0 | 0 | 5312,5 |
| N-Heptanoyl-glycine | 1883 | C3H3NO3(C7H14O2) | YES* | N-Acyl-D-amino acid | | 0 | 0 | 2016 | 3576,75 |
| (3R)-3-Isopropenyl-6-oxoheptanoate | 1884 | C10H16O3 | YES | see http://umbbd.ahc.umn.edu | | 571 | 6753 | 1 | 6261,25 |
| Nicotinate D-ribonucleotide | 1885 | C11H15NO9P | YES | | | 569 | 4338 | 0 | 5982,75 |
| Nicotine | 1886 | C10H14N2 | YES | | | 133 | 0 | 0 | 558 |
| Nitroglycerin | 1887 | C3H5N3O9 | YES | | | 45 | 119 | 35 | 8015,5 |
| N-Methylanthranilate | 1888 | C8H9NO2 | YES | | | 0 | 8 | 0 | 5689,75 |
| D-Glucose | 1889 | C6H12O6 | YES | | | 823 | 0 | 0 | 10507,5 |
| N-Malonylanthranilate | 1890 | C10H9NO5 | YES | | | 1133 | 3402 | 0 | 3859,25 |
| N-Methylaniline | 1891 | C7H9N | YES | | | 251 | 0 | 0 | 1278,5 |
| N-Methylindole | 1892 | C11H14N2 | YES | | | 201 | 1890 | 33 | 10426,8 |

| | | | | | | | | | |
|---|---|---|---|---|---|---|---|---|---|
| N-Methylhistamine | 1893 | C6H11N3 | YES | | | 760 | 92 | 222 | 5581 |
| N-Methyl-L-glutamate | 1894 | C6H11NO4 | YES | | | 1442 | 0 | 0 | 5353,25 |
| Pentadecan-1-ol | 1895 | C15H32O | NO | 1-Pentadecanol | 198291 | 55 | 3794 | 50 | 8618,5 |
| N-Methylphenylethanolamine | 1896 | C9H13NO | YES | | | 0 | 6192 | 0 | 2670,5 |
| N-Methyltyramine | 1897 | C9H13NO | YES | | | 0 | 0 | 8168 | 288,75 |
| N,N-Dimethylaniline | 1898 | C8H11N | YES | | | 0 | 2625 | 0 | 2552,5 |
| N-Octadecanoyl-glycine | 1899 | C3H3NO3(C18H36O2) | YES* | N-Acyl-D-amino acid | | 857 | 0 | 0 | 2502 |
| N-Octanoyl-glycine | 1900 | C3H4NO3(C8H16O2) | YES* | N-Acylglycine | | 35 | 0 | 17 | 5465,75 |
| N-(3-Oxooctanoyl)homoserine lactone | 1901 | C12H19NO4 | YES | | | 0 | 2786 | 0 | 353 |
| 2,4-Diamino-6-nitrotoluene | 1902 | C7H9N3O2 | YES | | | 3364 | 0 | 0 | 4792,25 |
| Nonadecane | 1903 | C19H40 | NO | | 155723 | 0 | 0 | 0 | 11710,3 |
| Nonadecanoic acid | 1904 | C19H38O2 | NO | | 155920 | 310 | 1836 | 98 | 1106,75 |
| Nonanoyldolichol | 1905 | C21H35O2(C5H8)5C19H38O2 | YES* | Acyldolichol | | 0 | 0 | 0 | 6789,75 |
| 4-Acetamido-2-amino-6-nitrotoluene | 1906 | C9H11N3O3 | YES | | | 0 | 0 | 0 | 4641,25 |
| Methyl nonylate | 1907 | C10H20O2 | NO | Methyl nonanoate | 158910 | 0 | 0 | 0 | 3497 |
| Nonanal | 1908 | C9H18O | NO | Pelargonaldehyde; n-Nonaldehyde; Nonaldehyde | 173340 | 111 | 4189 | 0 | 823,5 |
| Nonane | 1909 | C9H20 | NO | | 151270 | 3047 | 0 | 0 | 1189,75 |
| 2-Hydroxy-8-methylchromene-2-carboxylate | 1910 | C11H10O4 | NO | see http://umbbd.ahc.umn.edu | | 0 | 0 | 606 | 1856,75 |
| Nonanoate | 1911 | C9H18O2 | YES | | | 0 | 49 | 5 | 521,75 |
| Nonylphenol | 1912 | C15H24O | YES | | | 1025 | 0 | 0 | 753,75 |
| Nicotinamide | 1913 | C11H15N2O8 | YES | | | 821 | 14 | 0 | 2740,25 |

| | | | | | | | | | |
|---|---|---|---|---|---|---|---|---|---|
| mononucleotide | | P | | | | | | | |
| Oleoylglycerone phosphate | 1914 | C21H39O7P | YES | | | 0 | 217 | 0 | 4798,25 |
| Oleic acid methyl ester | 1915 | C19H36O2 | YES | | | 0 | 0 | 0 | 688,25 |
| all-trans-Nonaprenyl diphosphate | 1916 | C45H76O7P2 | YES | | | 0 | 0 | 0 | 2536,25 |
| Phenanthrene-3,4-oxide | 1917 | C14H10O | NO | see http://umbbd.ahc.umn.edu | | 70 | 18 | 0 | 5547,25 |
| 3-Amino-2-oxopropyl phosphate | 1918 | C3H8NO5P | YES | | | 2030 | 4045 | 1692 | 1063,25 |
| N-Succinyl-Ala-Ala-Ala p-nitroanilide | 1919 | C19H25N5O8 | NO* | | | 405 | 0 | 5301 | 1482,25 |
| N-Succinyl-Ala-Ala-Phe-7-amido-4-methyl coumarin | 1920 | C32H39N5O8 | NO* | | | 218 | 8042 | 0 | 2691,5 |
| N-Succinyl-Ala-Ala-Pro-Phe p-nitroanilide | 1921 | C30H36N6O9 | NO* | | | 1064 | 4 | 0 | 997 |
| N-Heptanoylhomoserine lactone | 1922 | C11H19NO3 | YES | | | 295 | 2253 | 2754 | 1187,25 |
| Methyloxaloacetate | 1923 | C5H6O5 | YES | | | 0 | 0 | 0 | 5307 |
| O-Acetylcholine | 1924 | C7H16NO2 | YES | | | 4409 | 0 | 0 | 11198 |
| Oxaloacetate | 1925 | C4H4O5 | YES | | | 210 | 3270 | 0 | 5063,25 |
| O-Acetyl-L-homoserine | 1926 | C6H11NO4 | YES | | | 1581 | 0 | 0 | 353,5 |
| 2-Oxohex-trans-4-enoate | 1927 | C6H8O3 | YES | | | 14 | 0 | 0 | 255,5 |
| 1-Octanal | 1928 | C8H16O | YES | | | 0 | 0 | 1825 | 4525,75 |
| Octadecanoic acid | 1929 | C18H36O2 | NO | Stearic acid | 148811 | 0 | 7251 | 107 | 1010 |
| Octadecane | 1930 | C18H38 | NO | | 154940 | 64 | 2828 | 812 | 11456,3 |
| Octadecenoic acid | 1931 | C18H34O2 | NO | Oleic acid; (Z)-Octadec-9-enoic acid | 151343 | 135 | 5 | 0 | 7746,25 |
| 9-Octadecynoic acid | 1932 | C18H32O2 | NO | Stearolic acid; 9-Stearolic acid; Octadec-9-ynoic | 24712250 | 0 | 0 | 0 | 9221 |

| | | | | | | | | | |
|---|---|---|---|---|---|---|---|---|---|
| | | | | acid | | | | | |
| 2-Butenoic acid | 1933 | C4H6O2 | NO | Crotonic acid; alpha-Butenoic acid; (E)-but-2-enoic acid | 162417 | 0 | 0 | 0 | 5521,25 |
| o-Cresol | 1934 | C7H8O | YES | | | 314 | 127 | 0 | 263 |
| Octane | 1935 | C8H18 | YES | | | 2278 | 0 | 0 | 3306,75 |
| Octane-1,8-diol | 1936 | C8H18O2 | YES | | | 2655 | 0 | 358 | 7020 |
| Stearoyl-CoA | 1937 | C39H70N7O17P3S | NO | Octadecanoyl-CoA | 24701369 | 249 | 13 | 8 | 615,5 |
| Ethyl octanoate | 1938 | C10H20O2 | YES | | | 700 | 0 | 287 | 248,75 |
| N1-(5-Phospho-D-ribosyl)glycinamide | 1939 | C7H15N2O8P | YES | | | 4591 | 0 | 930 | 1883,25 |
| L-Octanoylcarnitine | 1940 | C15H29NO4 | YES | | | 0 | 0 | 4290 | 4968,5 |
| GDP | 1941 | C10H15N5O11P2 | YES | | | 1290 | 37 | 117 | 10263,3 |
| Octadecanoyldolichol | 1942 | C21H35O2(C5H8)5C18H36O2 | YES* | Acyldolichol | | 1437 | 320 | 0 | 1505,75 |
| dAMP | 1943 | C10H14N5O6P | YES | | | 574 | 3014 | 0 | 8276 |
| Octanoyldolichol | 1944 | C21H35O2(C5H8)5C8H16O2 | YES* | Acyldolichol | | 113 | 4206 | 0 | 3815,25 |
| all-trans-Octaprenyl diphosphate | 1945 | C40H68O7P2 | YES | | | 220 | 5860 | 0 | 5861,25 |
| cis-1,2-Dihydroxy-1,2-dihydro-7-hydroxymethylnaphthalene | 1946 | C11H12O3 | YES | see http://umbbd.ahc.umn.edu | | 0 | 7386 | 0 | 7991 |
| 2-Ethylphenol | 1947 | C8H10O | YES | | | 0 | 0 | 0 | 6656,5 |
| 1-Octene | 1948 | C8H16 | NO | Caprylene; 1-Octene | 151254 | 4621 | 0 | 0 | 5200,5 |
| 1,2-Dihydroxy-7-hydroxymethylnaphthalene | 1949 | C11H10O3 | YES | see http://umbbd.ahc.umn.edu | | 0 | 39 | 4563 | 7427,5 |
| 2-Oxo-3-hydroxy-4- | 1950 | C4H7O8P | YES | | | 10072 | 0 | 0 | 4059,75 |

| | | | | | | | | | |
|---|---|---|---|---|---|---|---|---|---|
| phosphobutanoate | | | | | | | | | |
| Oleyldihydroxyacetonephosphate | 1951 | C4H6O7P(C18H36O2) | YES* | Dihydroxyacetone phosphate acyl ester | | 0 | 4412 | 0 | 4465,75 |
| 3-(5-oxo-4,5-dihydro-3H-imidazol-4-yl)propanoate | 1952 | C7H8N4O4 | NO* | | | 53 | 1410 | 0 | 998 |
| O-Demethylpuromycin | 1953 | C21H27N7O5 | YES | | | 3251 | 2525 | 0 | 3275,5 |
| O-Feruloylquinate | 1954 | C17H20O9 | YES | | | 0 | 0 | 0 | 19,75 |
| Octyl salicylate | 1955 | C15H22O3 | NO | n-Octyl salicylate; Salicylic acid, octyl ester; Octyl 2-hydroxybenzoate | 204128 | 0 | 0 | 100 | 1173 |
| 4-Hydroxylamino-2,6-dinitrotoluene | 1956 | C7H7N3O5 | YES | | | 0 | 2482 | 0 | 9937,75 |
| Oleyl alcohol | 1957 | C18H36O | NO | cis-9-Octadecen-1-ol; (Z)-octadec-9-en-1-ol | 24898063 | 0 | 0 | 0 | 5199 |
| Oleic acid | 1958 | C18H34O2 | YES | | | 208 | 81 | 0 | 7593,75 |
| 3-Methyl-2-oxobutanoate | 1959 | C5H8O3 | YES | | | 5 | 1697 | 0 | 966,75 |
| 2-Hydroxylamino-4,6-dinitrotoluene | 1960 | C7H7N3O5 | YES | | | 0 | 1420 | 0 | 8531,75 |
| 2-Oxopent-4-enoate | 1961 | C5H6O3 | YES | | | 0 | 0 | 0 | 3764,25 |
| N-Propionylglycine | 1962 | C3H3NO3(C3H6O2) | YES* | N-Acyl-D-amino acid | | 188 | 0 | 0 | 1343,75 |
| alpha-N-Phenylacetyl-L-glutamine | 1963 | C13H16N2O4 | YES | | | 138 | 0 | 6805 | 17963,5 |
| Pantetheine 4'-phosphate | 1964 | C11H23N2O7PS | YES | | | 23 | 4969 | 0 | 1759 |
| L-Ornithine | 1965 | C5H12N2O2 | YES | | | 4101 | 0 | 0 | 5418,25 |
| (9Z,12Z)-Octadecadienoyl-CoA | 1966 | C39H66N7O17P3S | YES | | | 0 | 0 | 0 | 4187,5 |
| Orotidine 5'-phosphate | 1967 | C10H13N2O11P | YES | | | 304 | 0 | 0 | 6549,5 |
| O- | 1968 | C17H20O10 | YES | | | 0 | 0 | 0 | 13734,8 |

| | | | | | | | | | |
|---|---|---|---|---|---|---|---|---|---|
| Sinapoylglucarolactone | | | | | | | | | |
| Oxalureate | 1969 | C3H4N2O4 | YES | | | 0 | 0 | 0 | 5407,75 |
| Oxaloglutarate | 1970 | C7H8O7 | YES | | | 0 | 0 | 0 | 4962 |
| Oxaloglycolate | 1971 | C4H4O6 | YES | | | 7342 | 0 | 0 | 339 |
| Oxalosuccinate | 1972 | C6H6O7 | YES | | | 8698 | 0 | 0 | 5146,25 |
| Oxalic acid | 1973 | C2H2O4 | YES | | | 142 | 0 | 0 | 121,5 |
| 4-Oxobutanoate | 1974 | C4H6O3 | YES | | | 3729 | 0 | 0 | 7063,5 |
| Oxamate | 1975 | C2H3NO3 | YES | | | 77 | 0 | 0 | 1338,25 |
| Oxazole | 1976 | C3H3NO | NO* | | | 0 | 0 | 0 | 414,25 |
| Doxepin | 1977 | C19H21NO | YES | | | 84 | 4062 | 4417 | 8689 |
| Oxirane | 1978 | C2H2OR2 | YES | | | 0 | 0 | 0 | 964,5 |
| 3-Oxopropionyl-CoA | 1979 | C24H38N7O18P3S | YES | | | 0 | 0 | 592 | 4282,75 |
| Oxomalonate | 1980 | C3H2O5 | YES | | | 0 | 0 | 0 | 909 |
| 2-Oxoadipate | 1981 | C6H8O5 | YES | | | 905 | 0 | 0 | 6325,5 |
| Phenylacetaldehyde | 1982 | C8H8O | YES | | | 93 | 0 | 0 | 9349 |
| 4-Hydroxyphenylacetyl-CoA | 1983 | C29H42N7O18P3S | YES | | | 3680 | 0 | 2693 | 4715,75 |
| Phosphatidate | 1984 | C5H7O8P(C4H8O2)2 | YES | | | 0 | 0 | 1369 | 712 |
| Orotate | 1985 | C5H4N2O4 | YES | | | 1311 | 1840 | 28 | 7479,75 |
| Panose | 1986 | C18H32O16 | YES | | | 0 | 7213 | 4286 | 16797 |
| (R)-Pantolactone | 1987 | C6H10O3 | YES | | | 0 | 4630 | 28 | 10442,3 |
| (R)-Pantothenate | 1988 | C9H17NO5 | YES | | | 0 | 0 | 0 | 7139 |
| (R)-Pantoate | 1989 | C6H12O4 | YES | | | 350 | 154 | 4281 | 3467,5 |
| Adenosine 2',5'-bisphosphate | 1990 | C10H15N5O10P2 | YES | | | 0 | 2365 | 0 | 0 |
| 3'-Phosphoadenylyl sulfate | 1991 | C10H15N5O13P2S | YES | | | 358 | 101 | 0 | 896 |
| Parathion | 1992 | C10H14NO5PS | YES | | | 0 | 0 | 0 | 10799 |
| 3-sn- | 1993 | C10H18NO8P | YES | | | 85 | 12 | 0 | 2383,5 |

| | | | | | | | | | |
|---|---|---|---|---|---|---|---|---|---|
| Phosphatidylcholine | | (C4H8O2)2 | | | | | | | |
| Choline phosphate | 1994 | C5H15NO4P | YES | | | 0 | 3414 | 3809 | 2685,25 |
| p-Cumate | 1995 | C10H12O2 | YES | | | 3815 | 0 | 0 | 3572,75 |
| p-Coumaryl alcohol | 1996 | C9H10O2 | YES | | | 663 | 0 | 2477 | 10872,3 |
| Pentachlorophenol | 1997 | C6HCl5O | YES | | | 19 | 3089 | 0 | 10,25 |
| p-Cresol | 1998 | C7H8O | YES | | | 19 | 0 | 22 | 3282,5 |
| gamma-Phenyl-epsilon-caprolactam | 1999 | C12H15ON | NO* | | | 84 | 484 | 0 | 31 |
| Pelargonic acid | 2000 | C9H18O2 | YES | | | 101 | 4153 | 6772 | 3468 |
| Palmitoyldihydroxyacetonephosphate | 2001 | C4H6O7P(C16H32O2) | YES* | Dihydroxyacetone phosphate acyl ester | | 0 | 0 | 0 | 6099 |
| Phosphatidyl-N-dimethylethanolamine | 2002 | C9H16NO8P(C4H8O2)2 | YES | | | 127 | 1 | 3161 | 832 |
| Pyridoxine 5'-phosphate | 2003 | C8H12NO6P | YES | | | 0 | 0 | 0 | 8721,75 |
| Phenethylamine | 2004 | C8H11N | YES | | | 0 | 4137 | 4503 | 9955,25 |
| Pectin | 2005 | (C26H36O24)10 | YES | | | 235 | 0 | 0 | 7480,75 |
| Pentadecanoic acid | 2006 | C15H30O2 | NO | Pentadecylic acid | 157173 | 307 | 1 | 0 | 628 |
| p-Cymene | 2007 | C10H14 | YES | | | 2676 | 0 | 0 | 1658,75 |
| Pentyl butyrate | 2008 | C9H18O2 | NO | Amyl butyrate; Pentyl butanoate; n-Amyl butyrate | 154193 | 101 | 0 | 0 | 1189,75 |
| Phosphatidylethanolamine | 2009 | C7H12NO8P(C4H8O2)2 | YES | | | 834 | 0 | 0 | 5313,5 |
| Methyl pentadecanoate | 2010 | C16H32O2 | NO | | 166092 | 545 | 0 | 0 | 2772,25 |
| Benzaldehyde | 2011 | C7H6O | YES | | | 0 | 0 | 0 | 15130 |
| 2-Aminobenzoyl-CoA | 2012 | C28H41N8O17P3S | YES | | | 8 | 0 | 0 | 9275 |
| Pentyl pentanoate | 2013 | C10H20O2 | NO | Pentyl valerate; Amyl valerate; Amyl valerianate | 203837 | 223 | 0 | 0 | 1707,75 |
| Pentanoic acid | 2014 | C5H10O2 | YES | | | 1930 | 0 | 0 | 742 |
| Phosphoenolpyruvate | 2015 | C3H5O6P | YES | | | 1966 | 121 | 1003 | 6379 |

| | | | | | | | | | |
|---|---|---|---|---|---|---|---|---|---|
| Pentadecane | 2016 | C15H32 | YES | | | 151 | 35 | 0 | 2132,75 |
| 5-Methylhexa-2,4-dienoyl-CoA | 2017 | C28H44N7O17P3S | YES | see http://umbbd.ahc.umn.edu | | 1736 | 0 | 4917 | 2643,75 |
| cis-2-Oxohept-3-ene-1,7-dioate | 2018 | C7H7O52 | NO | see http://umbbd.ahc.umn.edu | | 1038 | 0 | 0 | 6573 |
| n-Pentane | 2019 | C5H12 | NO | | 151117 | 0 | 0 | 0 | 3301,75 |
| Perdeuterobenzene | 2020 | C6H6 | NO | 1,2,3,4,5,6-Hexadeuteriobenzene | 10503502 | 0 | 0 | 0 | 974 |
| Perillyl aldehyde | 2021 | C10H14O | YES | | | 20 | 0 | 0 | 963,25 |
| Pentacen | 2022 | C22H14 | NO* | | | 38 | 12 | 15822 | 847,5 |
| Perillyl alcohol | 2023 | C10H16O | YES | | | 227 | 0 | 0 | 794,75 |
| Ethanolamine phosphate | 2024 | C2H8NO4P | YES | | | 1506 | 0 | 0 | 526,5 |
| p-Formyltoluene | 2025 | C8H8O | YES | | | 0 | 0 | 14 | 573 |
| Phenol | 2026 | C6H6O | YES | | | 3706 | 0 | 0 | 1839,25 |
| Phenylboronic acid | 2027 | C6H7BO2 | YES | | | 10385 | 7297 | 0 | 8595,25 |
| Phosphatidylglycerophosphate | 2028 | C8H14O13P2(C4H8O2)2 | YES | | | 742 | 0 | 4211 | 2389,5 |
| dATP | 2029 | C10H16N5O12P3 | YES | | | 966 | 0 | 6444 | 12287,3 |
| Phenylacetyl-CoA | 2030 | C29H42N7O17P3S | YES | | | 2040 | 0 | 962 | 926 |
| 2,4,6/3,5-Pentahydroxycyclohexanone | 2031 | C6H10O6 | YES | | | 0 | 0 | 0 | 9932,25 |
| L-1-Pyrroline-3-hydroxy-5-carboxylate | 2032 | C5H7NO3 | YES | | | 145 | 82 | 2413 | 9688,5 |
| Phenylethanolamine | 2033 | C8H11NO | YES | | | 565 | 0 | 3647 | 903 |
| Phenethyl alcohol | 2034 | C8H10O | YES | | | 26 | 0 | 3326 | 1019 |
| 1-Phenylethanol | 2035 | C8H10O | YES | | | 0 | 0 | 3642 | 2250,5 |
| L-Phenylalanine | 2036 | C9H11NO2 | YES | | | 5098 | 2924 | 0 | 327,25 |
| 2-Phenylglycine | 2037 | C8H9NO2 | NO | alpha-Phenylgycine; 2-amino-2-phenylacetic acid | 160941 | 0 | 0 | 3160 | 650,25 |

| | | | | | | | | | |
|---|---|---|---|---|---|---|---|---|---|
| Quercetin | 2038 | C15H10O7 | YES | | | 0 | 0 | 13 | 8841,75 |
| Phenanthrene | 2039 | C14H10 | YES | | | 12 | 5663 | 0 | 9981 |
| Phenazine | 2040 | C12H8N2O | NO | N-Oxyphenazine; Phenazin; Phenazine 5-oxide ; 5-oxidophenazin-5-ium | 152568 | 0 | 0 | 46 | 10979 |
| Phenetole | 2041 | C8H10O | NO | Ethoxybenzene | 150756 | 0 | 3700 | 0 | 8102,25 |
| Phosphatidylglycerol | 2042 | C8H13O10P(C4H8O2)2 | YES | | | 0 | 0 | 0 | 545,5 |
| L-Phe-Gly-Gly | 2043 | C13H17N3O4 | NO | Phenylalanyl-glycyl-glycine | 730157 | 0 | 0 | 247 | 480,75 |
| Phenothiazine | 2044 | C17H20N2S | YES | | | 108 | 0 | 0 | 6670,5 |
| Phloroglucinol | 2045 | C6H6O3 | YES | | | 24 | 4005 | 0 | 449,25 |
| O-Phospho-L-homoserine | 2046 | C4H10NO6P | YES | | | 0 | 0 | 6903 | 4012,75 |
| Phosphatidylglycerol | 2047 | C8H13O10PR2 | YES | | | 0 | 2270 | 0 | 370 |
| Phosphatidylinositol | 2048 | C11H17O13P(C4H8O2)2 | YES | | | 0 | 0 | 515 | 3486 |
| 2-Hydroxyphenylacetate | 2049 | C8H8O3 | YES | | | 14 | 8 | 3054 | 8824,75 |
| 3-Hydroxyphenylacetate | 2050 | C8H8O3 | YES | | | 172 | 5439 | 2156 | 11667,5 |
| Phenylpyruvate | 2051 | C9H8O3 | YES | | | 278 | 0 | 0 | 1908,5 |
| cis-3,4-Dihydroxy-3,4-dihydrophenanthrene | 2052 | C14H12O2 | YES | | | 0 | 220 | 0 | 7238,75 |
| cis-1,2-Dihydroxy-1,2-dihydro-8-methylnaphthalene | 2053 | C11H10O2 | NO | see http://umbbd.ahc.umn.edu | | 21 | 1129 | 0 | 6546,25 |
| 1,2-Dihydroxy-8-methylnaphthale | 2054 | C11H10O2 | NO | see http://umbbd.ahc.umn.edu | | 0 | 396 | 0 | 12382,5 |
| 2,4-Dihydroxy-hept-trans-2-ene-1,7- | 2055 | C7H9O62 | NO | see http://umbbd.ahc.umn.edu | | 0 | 1284 | 2217 | 7590,75 |

| | | | | | | | | | |
|---|---|---|---|---|---|---|---|---|---|
| dioate | | | | | | | | | |
| 1-Phosphatidyl-D-myo-inositol | 2056 | C11H17O13P(C4H8O2)2 | YES | | | 0 | 1837 | 332 | 1159,5 |
| Pimelic acid | 2057 | C7H12O4 | YES | | | 0 | 864 | 0 | 921,5 |
| O-Phospho-4-hydroxy-L-threonine | 2058 | C4H10NO7P | YES | | | 402 | 4031 | 0 | 1621,5 |
| 1-Phosphatidyl-1D-myo-inositol 3-phosphate | 2059 | C11H18O16P2(C4H8O2)2 | YES | | | 0 | 101 | 0 | 1303,75 |
| 1-Phosphatidyl-1D-myo-inositol 4-phosphate | 2060 | C11H18O16P2(C4H8O2)2 | YES | | | 0 | 3797 | 0 | 2523 |
| Piperideine | 2061 | C5H9N | YES | | | 22 | 477 | 2972 | 6903,75 |
| Piperazine | 2062 | C4H10N2 | YES | | | 134 | 53 | 2900 | 8486,5 |
| Piperidine | 2063 | C5H11N | YES | | | 0 | 0 | 1803 | 6212 |
| 2,6-Diamino-4-nitrotoluene | 2064 | C7H9N3O2 | YES | | | 0 | 2905 | 0 | 4490,75 |
| (R)-5-Phosphomevalonate | 2065 | C6H13O7P | YES | | | 0 | 2530 | 0 | 9392,5 |
| 4-Amino-2-hydroxylamino-6-nitrotoluene | 2066 | C7H9N3O3 | YES | | | 310 | 3147 | 0 | 1289,5 |
| Pimeloyl-CoA | 2067 | C28H46N7O19P3S | YES | | | 495 | 0 | 12 | 7821,75 |
| 2,4-Dihydroxylamino-6-nitrotoluene | 2068 | C7H9N3O4 | YES | | | 0 | 5004 | 0 | 7315,75 |
| 2-Amino-4,6-dinitrotoluene | 2069 | C7H7N3O4 | YES | | | 35 | 3194 | 0 | 6780,5 |
| p-Nitrophenyl alpha-D-arabinofuranoside | 2070 | C11H13NO7 | NO | 4-Nitrophenyl-alpha-L-arabinofuranoside | 841702 | 30 | 3566 | 0 | 1754,25 |
| 2-Phenyl-1,3-propanediol monocarbamate | 2071 | C10H13NO3 | YES | | | 319 | 10 | 162 | 988,75 |
| p-Nitrophenyl galactoside | 2072 | C12H15NO8 | NO | 4-Nitrophenylgalactoside | 207076 | 39 | 4495 | 0 | 5968,5 |

| | | | | | | | | | |
|---|---|---|---|---|---|---|---|---|---|
| (3S)-3-Hydroxyadipyl-CoA | 2073 | C27H44N7O20P3S | YES | | | 3351 | 4358 | 677 | 7450,75 |
| beta-D-Glucosyl-2-coumarinate | 2074 | C15H18O8 | YES | | | 4223 | 0 | 279 | 5606,75 |
| p-Nitrophenyl cellobioside | 2075 | C18H25NO13 | NO | p-Nitrophenyl beta-D-cellobioside | 841700 | 0 | 0 | 0 | 9442,75 |
| p-Nitrophenyl glucopyranoside | 2076 | C12H15NO8 | NO | p-Nitrophenyl-beta-glucoside | 669863 | 237 | 7999 | 0 | 858,75 |
| 4-Fluorocatechol | 2077 | C6H5FO2 | YES | | | 1625 | 0 | 209 | 13584,5 |
| Propenoyl-CoA | 2078 | C24H38N7O17P3S | YES | | | 392 | 140 | 6 | 8179,75 |
| p-Nitrophenyl myristate | 2079 | C20H31NO4 | NO | (4-Nitrophenyl) tetradecanoate | 661444 | 0 | 4 | 806 | 2017,25 |
| 5-Hydroxyconiferyl alcohol | 2080 | C10H12O4 | YES | | | 867 | 0 | 87 | 17468,8 |
| p-Nitrophenyl phosphate | 2081 | C6H6NO6P | NO | Nitrophenylphosphate | 152714 | 0 | 4033 | 0 | 11311,5 |
| p-Nitrophenyl alpha-L-rhamnoside | 2082 | C12H15NO7 | NO* | | | 0 | 0 | 0 | 5832 |
| p-Nitrophenyl alpha-L-rhamnopyranoside | 2083 | C12H15NO7 | NO* | | | 0 | 0 | 0 | 7680 |
| Propionate | 2084 | C3H6O2 | YES | | | 7 | 99 | 0 | 8589 |
| Propanoyl phosphate | 2085 | C3H7O5P | YES | | | 1859 | 1598 | 0 | 1243,75 |
| Phosphoramidate | 2086 | PNH4O3 | YES | | | 0 | 0 | 0 | 547,25 |
| 4-Hydroxystyrene | 2087 | C8H8O | YES | | | 0 | 399 | 75 | 5538,25 |
| 3,5,7,3',4'-Pentahydroxyflavone | 2088 | C15H10O7 | YES | | | 289 | 0 | 3765 | 9709 |
| Propanoyl-CoA | 2089 | C24H40N7O17P3S | YES | | | 824 | 16 | 0 | 597,75 |
| Prephenate | 2090 | C10H10O6 | YES | | | 1314 | 3346 | 0 | 695 |
| Guanosine 3'-diphosphate 5'-diphosphate | 2091 | C10H17N5O17P4 | YES | | | 0 | 0 | 0 | 589 |
| Phosphatidylinositol-4,5-bisphosphate | 2092 | C11H19O19P3(C4H8O2)2 | YES | | | 0 | 0 | 35512 | 5794,75 |

| | | | | | | | | | |
|---|---|---|---|---|---|---|---|---|---|
| (R)-Diphosphomevalonate | 2093 | C6H14O10P2 | YES | | | 0 | 0 | 0 | 137,5 |
| Pseudouridine | 2094 | C9H12N2O6 | YES | | | 0 | 0 | 0 | 4858,5 |
| 2-Hydroxy-4-hydroxymethylbenzalpyruvate | 2095 | C11H10O5 | YES | | | 0 | 0 | 9 | 648,25 |
| all-trans-pentaprenyl diphosphate | 2096 | C25H44O7P2 | YES | | | 0 | 635 | 0 | 1796,5 |
| 5'-Phosphoribosyl-N-formylglycinamidine | 2097 | C8H16N3O8P | YES | | | 0 | 1694 | 0 | 523,25 |
| 5-Phospho-beta-D-ribosylamine | 2098 | C5H12NO7P | YES | | | 341 | 0 | 4390 | 2208 |
| N-(5-Phospho-D-ribosyl)anthranilate | 2099 | C12H16NO9P | YES | | | 1969 | 3004 | 0 | 3322,5 |
| 1-(5-Phosphoribosyl)-AMP | 2100 | C15H23N5O14P2 | YES | | | 3058 | 1569 | 6328 | 1663,5 |
| 1-(5-Phosphoribosyl)-ATP | 2101 | C15H25N5O20P4 | YES | | | 833 | 2755 | 45 | 2904 |
| 1-(5'-Phosphoribosyl)-5-formamido-4-imidazolecarboxamide | 2102 | C10H15N4O9P | YES | | | 56 | 2971 | 0 | 832,5 |
| 1-(5-Phosphoribosyl)-5-[(5-phosphoribosylamino)methylideneamino]imidazole-4-carboxamide | 2103 | C15H25N5O15P2 | YES | | | 0 | 176 | 0 | 1996,75 |
| 5-(5-Phospho-D-ribosylaminoformimino)-1-(5-phosphoribosyl)-imidazole-4-carboxamide | 2104 | C15H25N5O15P2 | YES | | | 19 | 9 | 1319 | 2119,5 |

| | | | | | | | | | |
|---|---|---|---|---|---|---|---|---|---|
| 1-(5-Phosphoribosyl)-5-[(5-phosphoribosylamino)methylideneamino]imidazole-4-carboxamide | 2105 | C15H25N5O15P2 | YES | | | 0 | 1918 | 0 | 2844 |
| Quinate | 2106 | C7H12O6 | YES | | | 154 | 0 | 1458 | 2881,75 |
| L-Proline | 2107 | C5H9NO2 | YES | | | 1465 | 0 | 0 | 2570,75 |
| 1,4-Dipropoxybenzene | 2108 | C12H18O2 | NO | p-di-n-Propoxybenzene; 1,4-dipropoxy-benzene | 10521336 | 31 | 114 | 676 | 4782,75 |
| Propionyldolichol | 2109 | C21H35O2(C5H8)5C3H6O2 | YES* | Acyldolichol | | 0 | 0 | 0 | 8468,25 |
| 3-Phenyl-2-propen-1-ol | 2110 | C9H10O | YES | Cinnamyl alcohol | | 97 | 0 | 0 | 936,75 |
| L-Prolyl-AMP | 2111 | C13H20N5O7P | NO* | | | 0 | 115 | 0 | 1844,5 |
| Propanoic acid | 2112 | C3H6O2 | NO | | 149548 | 0 | 0 | 0 | 1048 |
| Propyl paraben | 2113 | C10H12O3 | NO | Propyl 4-hydroxybenzoate | 150215 | 0 | 98 | 3747 | 8666 |
| Pentanoyl-CoA | 2114 | C26H44N7O17P3S | YES | | | 0 | 987 | 0 | 1081 |
| Propenoyl-CoA | 2115 | C24H38N7O17P3S | YES | | | 0 | 306 | 2652 | 7262 |
| Protein substrate denatured 5 kDa | 2116 | # | NO | | | 0 | 5 | 2793 | 3142,75 |
| Protein substrate denatured 10 kDa | 2117 | # | NO | | | 0 | 4277 | 1492 | 6351,75 |
| Protein substrate denatured 15 kDa | 2118 | # | NO | | | 0 | 8270 | 0 | 3834,25 |
| Protein substrate denatured 20 kDa | 2119 | # | NO | | | 0 | 0 | 0 | 11549 |
| Protein substrate denatured 30 kDa | 2120 | # | NO | | | 0 | 0 | 0 | 5639 |
| Protein substrate denatured 40 kDa | 2121 | # | NO | | | 71 | 0 | 0 | 9362,5 |
| Protein substrate | 2122 | # | NO | | | 12 | 0 | 0 | 325,5 |

| | | | | | | | | | |
|---|---|---|---|---|---|---|---|---|---|
| denatured 60 kDa | | | | | | | | | |
| trans-O-Hydroxybenzylidenepyruvate | 2123 | C10H8O4 | YES | | | 0 | 0 | 0 | 1862 |
| 5-Phospho-alpha-D-ribose 1-diphosphate | 2124 | C5H13O14P3 | YES | | | 128 | 1202 | 0 | 1830,25 |
| Pseudocumene | 2125 | C9H12 | YES | | | 0 | 24 | 0 | 1378,75 |
| Pseudouridine 5'-phosphate | 2126 | C9H13N2O9P | YES | | | 0 | 2604 | 0 | 4379 |
| Phosphatidylserine | 2127 | C8H12NO10P (C4H8O2) | YES | | | 0 | 0 | 4245 | 4277,5 |
| O-Phospho-L-serine | 2128 | C3H8NO6P | YES | | | 91 | 0 | 1109 | 2391,25 |
| Heptylparaben | 2129 | C14H20O3 | YES | | | 54 | 2489 | 98 | 7297,5 |
| 1-Phosphatidyl-1D-myo-inositol 3,4-bisphosphate | 2130 | C11H19O19P 3(C3H6O2)2 | YES | | | 30 | 0 | 0 | 1348,75 |
| 1-Phosphatidyl-D-myo-inositol 4,5-bisphosphate | 2131 | C11H19O19P 3R2 | YES | | | 0 | 0 | 0 | 2629 |
| Phosphatidyl-N-methylethanolamine | 2132 | C8H14NO8P( C3H6O2)2 | YES | | | 0 | 1403 | 0 | 920,25 |
| 1-Phosphatidyl-D-myo-inositol | 2133 | C11H17O13P( C3H6O2)2 | YES | | | 0 | 0 | 0 | 229,5 |
| 1-(5-Phospho-D-ribosyl)-5-amino-4-imidazolecarboxylate | 2134 | C9H14N3O9P | YES | | | 1392 | 0 | 0 | 2702 |
| Pteridine | 2135 | C6H4N4 | YES | | | 0 | 0 | 0 | 615 |
| Putrescine | 2136 | C4H12N2 | YES | | | 0 | 27 | 0 | 3720 |
| Pullulan | 2137 | (C36H60O30) 10 | YES | | | 2296 | 0 | 0 | 731 |
| Purine | 2138 | C5H4N4 | YES | | | 0 | 940 | 1 | 2787,75 |
| Puromycin | 2139 | C22H29N7O5 | YES | | | 18 | 123 | 2298 | 8117 |
| Quercitrin | 2140 | C21H20O11 | YES | | | 111 | 0 | 0 | 1476,25 |
| Pyrano[3,4-b]pyrrole | 2141 | C7H7NO | NO* | | | 0 | 0 | 0 | 8031,75 |
| Pyridoxal | 2142 | C8H9NO3 | YES | | | 2005 | 439 | 2 | 9124,25 |

| | | | | | | | | | |
|---|---|---|---|---|---|---|---|---|---|
| Pyrimidine | 2143 | C4H4N2 | YES | | | 0 | 407 | 0 | 8752,25 |
| Pyridoxine | 2144 | C8H11NO3 | YES | | | 147 | 4026 | 1147 | 7325,75 |
| (R)-Phenyllactate | 2145 | C9H10O3 | YES | | | 112 | 0 | 4537 | 2153,25 |
| Pyruvate | 2146 | C3H4O3 | YES | | | 2294 | 1838 | 0 | 991 |
| Pyrazine | 2147 | C4H4N2 | NO | p-Diazine | 152453 | 8 | 1424 | 193 | 7908 |
| Pyrazolidine | 2148 | C3H8N2 | NO | | 656048 | 0 | 163 | 2095 | 5267,75 |
| Pyrazole | 2149 | C3H4N2 | YES | | | 191 | 328 | 1173 | 8779,75 |
| Pyrazolo[1,5-a]pyrimidine | 2150 | C5H3N4 | NO* | | | 0 | 6545 | 0 | 5502 |
| Pyrene | 2151 | C16H10 | YES | | | 0 | 0 | 0 | 846,25 |
| Pyridazine | 2152 | C4H4N2 | NO | Orthodiazine; o-Diazine | 152451 | 0 | 0 | 0 | 4834,25 |
| Pyridine | 2153 | C5H5N | YES | | | 0 | 0 | 0 | 5148 |
| Pyridoxal 5'-phosphate | 2154 | C8H10NO6P | YES | | | 1792 | 297 | 3781 | 14353,5 |
| Pyrogallol | 2155 | C6H6O3 | YES | | | 220 | 1201 | 3947 | 7389,25 |
| Pyrrole | 2156 | C4H5N | NO | see http://umbbd.ahc.umn.edu | | 69 | 1750 | 601 | 902 |
| Pyrrolidine | 2157 | C4H9N | NO | Azacyclopentane | 173311 | 0 | 8630 | 293 | 3317,25 |
| Ubiquinone | 2158 | C14H18O4(C5H8)5 | YES | | | 0 | 0 | 0 | 6570,75 |
| Ubiquinol | 2159 | C14H20O4(C5H8)5 | YES | | | 109 | 0 | 0 | 1879,5 |
| Pyridine-2,3-dicarboxylate | 2160 | C7H5NO4 | YES | | | 0 | 0 | 5936 | 457,25 |
| Pyridoxamine 5'-phosphate | 2161 | C8H13N2O5P | YES | | | 1362 | 0 | 0 | 6953,5 |
| dUTP | 2162 | C9H15N2O14P3 | YES | | | 2384 | 0 | 2502 | 1545,25 |
| alpha-D-Ribose 5-phosphate | 2163 | C5H11O8P | YES | | | 2452 | 5256 | 0 | 5060 |
| N-(5'-Phospho-D-1'-ribulosylformimino)-5-amino-1-(5''-phospho-D-ribosyl)-4-imidazolecarboxamid | 2164 | C15H25N5O15P2 | YES | | | 2912 | 0 | 0 | 927,25 |

| | | | | | | | | | |
|---|---|---|---|---|---|---|---|---|---|
| e | | | | | | | | | |
| D-Ribose 1,5-bisphosphate | 2165 | C5H12O11P2 | YES | | | 5812 | 600 | 0 | 2542 |
| alpha-D-Ribose 1-phosphate | 2166 | C5H11O8P | YES | | | 2702 | 1099 | 1265 | 1220 |
| Quinone | 2167 | C6H4O2 | YES | | | 0 | 2211 | 0 | 7933,25 |
| Quinoxaline | 2168 | C8H6N2 | NO | Benzoparadiazine | 50043899 | 0 | 1736 | 1541 | 6022,75 |
| Quinuclidine | 2169 | C7H13N | NO | 1-Azabicyclo[2.2.2]octane; 1,4-Ethanopiperidine; 1,4-Ethylenepiperidine | 150604 | 0 | 163 | 0 | 4531,5 |
| Quinolinate | 2170 | C7H5NO4 | YES | | | 1799 | 1353 | 0 | 2424,5 |
| L-Rhamnono-1,4-lactone | 2171 | C6H10O5 | YES | | | 23 | 1917 | 0 | 530,5 |
| 2,5-Dihydroxybenzoate | 2172 | C7H6O4 | YES | | | 0 | 130 | 0 | 1106,25 |
| Quinoline-3,4-diol | 2173 | C9H7NO2 | YES | | | 0 | 0 | 0 | 2065 |
| 2-Phenylbutanoic acid | 2174 | C10H12O2 | NO | 2-Phenylbutyric acid | 150030 | 8519 | 0 | 0 | 4066,5 |
| (R)-4-Hydroxymandelate | 2175 | C8H8O4 | YES | | | 0 | 2730 | 0 | 5548,5 |
| Pyridoxamine | 2176 | C8H12N2O2 | YES | | | 818 | 0 | 0 | 1295 |
| alpha-Ribazole 5'-phosphate | 2177 | C14H19N2O7P | YES | | | 4 | 1235 | 0 | 6092 |
| N1-(alpha-D-Ribosyl)-5,6-dimethylbenzimidazole | 2178 | C14H18N2O4 | YES | | | 3858 | 0 | 0 | 726,5 |
| Resorufin acetate | 2179 | C14H9NO4 | NO | (7-Oxophenoxazin-3-yl) acetate | 3881542 | 0 | 5259 | 0 | 8338,5 |
| 4-(1-D-Ribitylamino)-5-amino-2,6-dihydroxypyrimidine | 2180 | C9H16N4O6 | YES | | | 0 | 3925 | 30 | 849,25 |
| Raffinose | 2181 | C18H32O16 | YES | | | 0 | 3465 | 172 | 2565,5 |

| | | | | | | | | | |
|---|---|---|---|---|---|---|---|---|---|
| L-Ribulose | 2182 | C5H10O5 | YES | | | 0 | 6744 | 31189 | 732,75 |
| Resorcinol | 2183 | C6H6O2 | YES | | | 0 | 6710 | 0 | 8955 |
| Quinaldate | 2184 | C10H7NO2 | YES | | | 21 | 136 | 6096 | 7000,25 |
| Folate | 2185 | C19H19N7O6 | YES | | | 2129 | 1784 | 868 | 12191,5 |
| Dihydroresveratrol | 2186 | C14H14O3 | YES | | | 106 | 0 | 0 | 11124,3 |
| Retinol propionate | 2187 | C23H34O2 | NO | | 671406 | 40 | 0 | 0 | 451,25 |
| Retinoate | 2188 | C20H28O2 | YES | | | 0 | 2715 | 0 | 1189 |
| Retinol acetate | 2189 | C22H32O2 | NO | | 173452 | 0 | 0 | 0 | 6601,25 |
| Retinol butyrate | 2190 | C24H38O2 | NO* | | | 26 | 8469 | 0 | 5817 |
| Retinal | 2191 | C20H28O | YES | | | 86 | 0 | 0 | 15181,5 |
| Retinol | 2192 | C20H30O | YES | | | 58 | 0 | 0 | 1942,25 |
| Retinol hexanoate | 2193 | C26H42O2 | NO* | | | 0 | 0 | 0 | 3524,75 |
| Retinol octanoate | 2194 | C28H46O2 | NO* | | | 105 | 3974 | 0 | 2545,5 |
| Resorufin-beta-D-galactopyranoside | 2195 | C18H17NO8 | NO | Resorufin galactopyranoside | 702035 | 97 | 0 | 3251 | 8797,75 |
| Resorufin-beta-D-glucopyranoside | 2196 | C18H17NO8 | NO | Resorufin beta-D-glucopyranoside | 24899374 | 0 | 0 | 443 | 6222 |
| L-Rhamnonate | 2197 | C6H12O6 | YES | | | 0 | 298 | 36 | 1239 |
| gamma-Glutamyl-gamma-aminobutyraldehyde | 2198 | C9H16N2O4 | YES | | | 3 | 4351 | 0 | 6476,5 |
| (R)-Hydroxybutanoyl-CoA | 2199 | C25H42N7O18P3S | YES | | | 10 | 0 | 0 | 2909 |
| (R)-2-Hydroxystearate | 2200 | C18H36O3 | YES | | | 0 | 0 | 0 | 202,5 |
| (S)-Ribosyl-L-homocysteine | 2201 | C9H17NO6S | YES | | | 119 | 0 | 0 | 1937 |
| D-Ribose | 2202 | C5H10O5 | YES | | | 573 | 3130 | 4515 | 496 |
| (R)-3-Hydroxybutanoate | 2203 | C4H8O3 | YES | | | 98 | 0 | 2440 | 313,25 |
| Riboflavin | 2204 | C17H20N4O6 | YES | | | 0 | 5733 | 12636 | 1626,5 |
| Ribitol | 2205 | C5H12O5 | YES | | | 0 | 2229 | 0 | 2018,75 |
| L-Rhamnulose | 2206 | C6H12O5 | YES | | | 5 | 17 | 0 | 5087 |
| L-Rhamnulose 1- | 2207 | C6H13O8P | YES | | | 0 | 0 | 0 | 3252 |

| | | | | | | | | | |
|---|---|---|---|---|---|---|---|---|---|
| phosphate | | | | | | | | | |
| L-Rhamnofuranose | 2208 | C6H12O5 | YES | | | 1380 | 1633 | 0 | 1788 |
| L-Rhamnose | 2209 | C6H12O5 | YES | | | 0 | 3838 | 0 | 8815,25 |
| Resorufin butyrate | 2210 | C16H13NO4 | NO | (7-Oxophenoxazin-3-yl) butanoate | 24888123 | 1172 | 0 | 0 | 740 |
| R-S-Alanylglycine | 2211 | C5H9N2O3SR | YES | | | 67 | 2170 | 0 | 0 |
| (1R,2S)-Naphthalene 1,2-oxide | 2212 | C10H8O | YES | | | 10 | 0 | 0 | 285,25 |
| D-Ribulose 5-phosphate | 2213 | C5H11O8P | YES | | | 1792 | 101 | 4248 | 617,75 |
| 2-Hydroxy-3-methyl benzal pyruvate | 2214 | C11H10O4 | NO | see http://umbbd.ahc.umn.edu | | 0 | 0 | 4433 | 510 |
| Sedoheptulose 1,7-bisphosphate | 2215 | C7H16O13P2 | YES | | | 92 | 6425 | 6727 | 6709,25 |
| L-Ribulose 5-phosphate | 2216 | C5H11O8P | YES | | | 0 | 0 | 557 | 838 |
| cis-1,2-Dihydroxy-1,2-dihydro-7-methylnaphthalene | 2217 | C11H12O2 | YES | see http://umbbd.ahc.umn.edu | | 0 | 0 | 116 | 2037,75 |
| (S)-2,3-Epoxysqualene | 2218 | C30H50O | YES | | | 0 | 0 | 0 | 2282,25 |
| (S)-(+)-2-Phenyl-butyrate | 2219 | C10H12O2 | NO | 2-Phenylbutyric acid; 2-Phenylbutanoic acid | 150030 | 195 | 0 | 0 | 3142,5 |
| (S)-3-Hydroxyhexanoyl-CoA | 2220 | C27H46N7O18P3S | YES | | | 99 | 1959 | 3066 | 1103,25 |
| (S)-3-Hydroxyoctanoyl-CoA | 2221 | C29H50N7O18P3S | YES | | | 0 | 5981 | 0 | 487 |
| 1-(5'-Phosphoribosyl)-5-amino-4-(N-succinocarboxamide)-imidazole | 2222 | C13H19N4O12P | YES | | | 546 | 6032 | 0 | 1887,25 |
| 2-Carboxybenzaldehyde | 2223 | C8H6O3 | YES | | | 339 | 0 | 0 | 927,25 |

| | | | | | | | | | |
|---|---|---|---|---|---|---|---|---|---|
| (+)-cis-Sabinol | 2224 | C10H16O | YES | | | 0 | 861 | 4648 | 645,5 |
| (+)-Sabinone | 2225 | C10H14O | YES | | | 0 | 4195 | 0 | 4090,25 |
| Sedoheptulose 7-phosphate | 2226 | C7H15O10P | YES | | | 255 | 1743 | 11 | 523,5 |
| 3-Chloro-2-hydroxymuconic semialdehyde | 2227 | C6H5ClO4 | YES | | | 61 | 1129 | 0 | 1160,75 |
| L-Saccharopine | 2228 | C11H20N2O6 | YES | | | 0 | 6037 | 0 | 2632,5 |
| (S)-4-Hydroxymandelate | 2229 | C8H8O4 | YES | | | 42 | 2881 | 0 | 902,667 |
| Salicin 6-phosphate | 2230 | C13H19O10P | YES | | | 0 | 0 | 0 | 1377 |
| Salicylic acid | 2231 | C7H6O3 | YES | | | 0 | 0 | 3985 | 8760,5 |
| Salicylaldehyde | 2232 | C7H6O2 | YES | | | 0 | 0 | 0 | 6039,5 |
| Salicin | 2233 | C13H18O7 | YES | | | 140 | 2380 | 0 | 2156,75 |
| S-Adenosyl-(5')-3-methylthiopropylamine | 2234 | C14H23N6O3S | YES | | | 83 | 2716 | 0 | 5928,75 |
| N-Succinyl-2-amino-6-oxopimelate | 2235 | C11H15NO8 | YES | | | 2462 | 45160 | 0 | 1398 |
| Sarcosine | 2236 | C3H7NO2 | YES | | | 0 | 7969 | 0 | 4844,25 |
| D-Sorbitol 6-phosphate | 2237 | C6H15O9P | YES | | | 0 | 0 | 0 | 6825,75 |
| D-Sorbitol | 2238 | C6H14O6 | YES | | | 2453 | 0 | 0 | 6234,75 |
| L-Sorbitol | 2239 | C6H14O6 | YES | | | 112 | 2651 | 0 | 1280,75 |
| sec-Butylbenzene | 2240 | C10H14 | YES | | | 603 | 3951 | 29 | 2776,25 |
| O-Succinylbenzoyl-CoA | 2241 | C32H44N7O20P3S | YES | | | 4 | 0 | 3988 | 2979,75 |
| Scytalone | 2242 | C10H10O4 | YES | | | 1641 | 0 | 0 | 1681,75 |
| N-Succinyl-L-2,6-diaminopimelate | 2243 | C11H18N2O7 | YES | | | 3567 | 3172 | 5049 | 1365,25 |
| 2-Hydroxy-4-hydroxymethylbenzalpyruvate | 2244 | C11H10O5 | YES | see http://umbbd.ahc.umn.edu | | 262 | 0 | 2770 | 3270,25 |
| Sedoheptulose | 2245 | C7H14O7 | YES | | | 25 | 0 | 16 | 1429,75 |

| | | | | | | | | | |
|---|---|---|---|---|---|---|---|---|---|
| 2-Amino-5-oxocyclohex-1-enecarbonyl-CoA | 2246 | C28H43N8O18P3S | YES | see http://umbbd.ahc.umn.edu | | 89 | 62400 | 0 | 505,75 |
| 1,4-Cyclohexanedione | 2247 | C6H8O2 | YES | see http://umbbd.ahc.umn.edu | | 0 | 0 | 0 | 2067,25 |
| L-Seryl-AMP | 2248 | C13H19N6O9P | NO | Seryl-adenylate; L-Seryl-adenosine monophosphate | 740207 | 0 | 0 | 0 | 8209 |
| D-Serine | 2249 | C3H7NO3 | YES | | | 244 | 6466 | 0 | 7811,75 |
| L-Serine | 2250 | C3H7NO3 | YES | | | 0 | 0 | 6705 | 17230 |
| S-Formylglutathione | 2251 | C11H17N3O7S | YES | | | 0 | 290 | 147 | 527,75 |
| (S)-Hydroxybutanoyl-CoA | 2252 | C25H42N7O18P3S | YES | | | 0 | 0 | 491 | 280 |
| S-Succinyldihydrolipoamide | 2253 | C12H21NO4S2 | YES | | | 894 | 0 | 0 | 2316,75 |
| 4-Hydroxymethylsalicylaldehyde | 2254 | C8H8O3 | YES | see http://umbbd.ahc.umn.edu | | 83 | 7 | 24 | 1588,25 |
| Sphinganine | 2255 | C18H39NO2 | YES | | | 0 | 1340 | 0 | 10149,8 |
| Sphingenine | 2256 | C18H37NO2 | YES | | | 0 | 0 | 0 | 2163,25 |
| S-(Hydroxymethyl)glutathione | 2257 | C11H19N3O7S | YES | | | 6406 | 0 | 0 | 156,25 |
| (S)-2-Hydroxyoctadecanoate | 2258 | C2H3O3R | YES* | (S)-2-Hydroxymonocarboxylic acid | | 54 | 3222 | 0 | 664,25 |
| Styrene cis-glycol | 2259 | C8H10O2 | YES | | | 159 | 2222 | 5 | 3084,25 |
| Sinapate | 2260 | C11H12O5 | YES | 3,5-Dimethoxy-4-hydroxycinnamic acid | | 0 | 0 | 0 | 2175,25 |
| Sinapoyl-CoA | 2261 | C32H46N7O20P3S | YES | | | 104 | 0 | 495 | 18474,8 |
| Sinapyl alcohol | 2262 | C11H14O4 | YES | | | 210 | 3974 | 0 | 1575 |
| Sinapoyl-(S)-malate | 2263 | C15H16O9 | YES | | | 55 | 0 | 17910 | 14565,8 |

| | | | | | | | | | |
|---|---|---|---|---|---|---|---|---|---|
| Sinapaldehyde | 2264 | C11H12O4 | YES | | | 47 | 56 | 0 | 882 |
| Sinapoyltartronate | 2265 | C14H14O9 | YES | | | 0 | 0 | 874 | 8000,5 |
| Shikimate | 2266 | C7H10O5 | YES | | | 1299 | 123 | 0 | 7042 |
| N-Succinyl-LL-2,6-diaminoheptanedioate | 2267 | C11H18N2O7 | YES | | | 164 | 0 | 0 | 7581,5 |
| N-Succinyl-2-L-amino-6-oxoheptanedioate | 2268 | C11H15NO8 | YES | | | 0 | 3495 | 0 | 912,25 |
| (S)-2-Methyl-3-oxopropanoyl-CoA | 2269 | C25H40N7O19P3S | YES | | | 427 | 0 | 0 | 4445,75 |
| Shikimate 3-phosphate | 2270 | C7H11O8P | YES | | | 471 | 0 | 0 | 1452,5 |
| Shikimate 5-phosphate | 2271 | C7H11O8P | YES | | | 0 | 0 | 1 | 6536,5 |
| (S)-beta-Methylindolepyruvate | 2272 | C12H11NO3 | YES | | | 9 | 0 | 1 | 10689,8 |
| (S)-Methylthioglycolate | 2273 | C3H6O2S | YES | | | 0 | 0 | 0 | 2881,5 |
| 2-Hydroxychromene-2-carboxylate | 2274 | C10H8O4 | YES | | | 0 | 0 | 0 | 5287,5 |
| L-Sorbose | 2275 | C6H12O6 | YES | | | 2214 | 301 | 0 | 7553,25 |
| Sorbose 1-phosphate | 2276 | C6H13O9P | YES | | | 2917 | 0 | 0 | 5192,5 |
| Citronellate | 2277 | C10H18O2 | YES | | | 286 | 0 | 0 | 2477,5 |
| Sphinganine 1-phosphate | 2278 | C18H40NO5P | YES | | | 0 | 1848 | 0 | 8795,75 |
| Spermidine | 2279 | C7H19N3 | YES | | | 208 | 49 | 0 | 2035,75 |
| Spermine | 2280 | C10H26N4 | YES | | | 0 | 3670 | 0 | 945,25 |
| Squalene | 2281 | C30H50 | YES | | | 0 | 0 | 181 | 8833,5 |
| Sphinganine | 2282 | C18H39NO2 | YES | | | 0 | 0 | 0 | 7471 |
| (1S,2R)-Naphthalene 1,2-oxide | 2283 | C10H8O | YES | | | 367 | 3664 | 0 | 5995 |
| (S)-Squalene-2,3-epoxide | 2284 | C30H50O | YES | | | 0 | 3981 | 5721 | 2927,25 |
| Stachyose | 2285 | C24H42O21 | YES | | | 120 | 0 | 17953 | 2254,75 |

| | | | | | | | | | |
|---|---|---|---|---|---|---|---|---|---|
| Starch | 2286 | (C12H20O10)20 | YES | | | 2302 | 0 | 4849 | 7234,75 |
| Stearyl heptanoate | 2287 | C25H50O2 | NO | Octadecyl heptanoate | 682722 | 0 | 0 | 0 | 5436,75 |
| 2,5-Dichloroaniline | 2288 | C6H5Cl2N | YES | | | 58 | 3475 | 3851 | 5532,75 |
| (1R,4R)-Dihydrocarvone | 2289 | C10H16O | YES | see http://umbbd.ahc.umn.edu | | 43 | 36 | 0 | 7017,25 |
| (1S,4S)-Dihydrocarvone | 2290 | C10H16O | YES | | | 0 | 0 | 0 | 9923,75 |
| Succinate semialdehyde | 2291 | C4H6O3 | YES | | | 145 | 0 | 0 | 8291,75 |
| N2-Succinyl-L-arginine | 2292 | C10H18N4O5 | YES | | | 10 | 7 | 0 | 1914 |
| (1S,4R)-1-Hydroxy-2-oxolimonene | 2293 | C10H16O2 | YES | see http://umbbd.ahc.umn.edu | | 0 | 0 | 0 | 523 |
| Sucrose 6-phosphate | 2294 | C12H23O14P | YES | | | 0 | 6544 | 0 | 1536,25 |
| O-Succinylbenzoate-CoA | 2295 | C32H46N7O21P3S | NO* | | | 0 | 35 | 3012 | 702,25 |
| 2-Succinylbenzoate | 2296 | C11H10O5 | YES | | | 0 | 0 | 0 | 2334,75 |
| m-Tolualdehyde | 2297 | C8H8O | YES | | | 18 | 2794 | 0 | 1840 |
| o-Tolualdehyde | 2298 | C8H8O | YES | | | 70 | 0 | 1991 | 330,5 |
| Hydroxycinnamoyl-CoA | 2299 | C30H42N7O18P3S | YES | | | 0 | 0 | 0 | 2597 |
| 1,3,4,6-Tetrachloro-1,4-cyclohexadiene | 2300 | C6H4Cl4 | YES | | | 90 | 2776 | 3637 | 1612,75 |
| O-Succinyl-L-homoserine | 2301 | C8H13NO6 | YES | | | 0 | 0 | 3035 | 6948,75 |
| N2-Succinyl-L-ornithine | 2302 | C9H16N2O5 | YES | | | 0 | 2 | 0 | 462,75 |
| Sucrose | 2303 | C12H22O11 | YES | | | 0 | 2 | 0 | 1830,5 |
| Succinic semialdehyde | 2304 | C4H6O3 | YES | | | 0 | 0 | 904 | 437,5 |
| Syringaldehyde | 2305 | C9H10O4 | NO | 4-Hydroxy-3,5-dimethoxybenzaldehyde | 151814 | 0 | 0 | 0 | 761,5 |
| trans-2,3-epoxysuccinate | 2306 | C4H4O5 | YES | | | 0 | 698 | 0 | 149 |

| | | | | | | | | | |
|---|---|---|---|---|---|---|---|---|---|
| trans-Cinnamate | 2307 | C9H8O2 | YES | | | 0 | 3593 | 0 | 3868,75 |
| cis,cis-2-Hydroxy-6-oxohept-2,4-dienoate | 2308 | C7H8O4 | NO | see http://umbbd.ahc.umn.edu | | 0 | 5458 | 22379 | 3716 |
| D-Tagatose | 2309 | C6H12O6 | YES | | | 132 | 138 | 0 | 2227,25 |
| D-Tagatose 6-phosphate | 2310 | C6H13O9P | YES | | | 0 | 0 | 0 | 1660 |
| D-Tagatose 1,6-bisphosphate | 2311 | C6H14O12P2 | YES | | | 36 | 0 | 0 | 1376 |
| D-Tagaturonate | 2312 | C6H10O7 | YES | | | 0 | 0 | 0 | 1240,25 |
| D-Talose | 2313 | C6H12O6 | YES | | | 10 | 0 | 0 | 2697 |
| D-Tartrate | 2314 | C4H6O6 | YES | | | 0 | 0 | 0 | 3067,5 |
| L-Tartaric acid | 2315 | C4H6O6 | YES | | | 84 | 0 | 0 | 3555,25 |
| 2,3,4,5-Tetrachlorophenol | 2316 | C6H2Cl4O | NO | | 174748 | 0 | 0 | 0 | 3802 |
| trans-1,2-Dihydrobenzene-1,2-diol | 2317 | C6H8O2 | YES | | | 0 | 0 | 0 | 202,25 |
| GMP | 2318 | C10H14N5O8P | YES | | | 860 | 1763 | 0 | 6366,25 |
| N-Succinyl-L-glutamate | 2319 | C9H13NO7 | YES | | | 7635 | 3197 | 0 | 291 |
| N-Succinyl-L-glutamate 5-semialdehyde | 2320 | C9H13NO6 | YES | | | 1033 | 5093 | 0 | 4900 |
| Taurine | 2321 | C2H7NO3S | YES | | | 2858 | 3575 | 3770 | 2388,75 |
| 2',6,3',4'-Tetrachlorobiphenyl | 2322 | C12H6Cl4 | NO | | 180008 | 0 | 0 | 3594 | 1905 |
| 2,4,4',6-Tetrachlorobiphenyl | 2323 | C12H6Cl4 | YES | | | 20 | 0 | 0 | 4590,5 |
| 2,2',5,5'-Tetrachlorobiphenyl | 2324 | C12H6Cl4 | YES | | | 0 | 0 | 0 | 6842,5 |
| 2,3,4,4'-Tetrachlorobiphenyl | 2325 | C12H6Cl4 | YES | | | 0 | 84 | 0 | 9597,5 |
| 2,3,4,5-Tetrachlorobiphenyl | 2326 | C12H6Cl4 | YES | | | 0 | 5516 | 0 | 1829 |

| | | | | | | | | | |
|---|---|---|---|---|---|---|---|---|---|
| 2,3,4,4'-Tetrachlorobiphenyl | 2327 | C12H6Cl4 | YES | | | 0 | 4609 | 0 | 5642,75 |
| Succinate | 2328 | C4H6O4 | YES | | | 1274 | 1667 | 2066 | 7174,25 |
| Tetrahydrothiophene | 2329 | C4H8S | NO* | | | 0 | 4799 | 5272 | 1912,5 |
| D-Threose | 2330 | C4H8O4 | YES | | | 0 | 6060 | 0 | 486,75 |
| (2E)-Tetradecenoyl-CoA | 2331 | C35H60N7O17P3S | YES | | | 93 | 0 | 0 | 1101 |
| Toluene-cis-dihydrodiol | 2332 | C7H10O2 | YES | | | 0 | 4322 | 6908 | 259 |
| 2-Hydroxy-7-hydroxymethylchromene-2-carboxylate | 2333 | C11H10O5 | NO | see http://umbbd.ahc.umn.edu | | 0 | 3198 | 0 | 4768,5 |
| Tetracosane | 2334 | C24H50 | NO | | 155921 | 41 | 0 | 0 | 0 |
| N-Tetradecanoyl-glycine | 2335 | C36H31O3N | NO* | | | 71 | 0 | 4750 | 772,5 |
| Tetradecanoyldolichol | 2336 | C21H35O2(C5H8)5C14H28O2 | YES* | Acyldolichol | | 35 | 0 | 0 | 11882 |
| Succinyl-CoA | 2337 | C25H40N7O19P3S | YES | | | 889 | 1937 | 0 | 6779,5 |
| Tetrahydrofuran | 2338 | C4H8O | NO | Furanidine; Oxolane | 151143 | 202 | 0 | 2669 | 5541,25 |
| trans-Tetradec-2-enoyl-CoA | 2339 | C35H60N7O17P3S | YES | | | 520 | 0 | 3479 | 6353,25 |
| Tetradecanoyl-CoA | 2340 | C35H62N7O17P3S | YES | | | 1794 | 0 | 4696 | 0 |
| 3',4',5,6-Tetrahydroxy-3,7-dimethoxyflavone | 2341 | C17H14O8 | YES | | | 299 | 0 | 0 | 11653 |
| Deoxycytidine | 2342 | C9H13N3O4 | YES | | | 3790 | 3517 | 0 | 2104,5 |
| GTP | 2343 | C10H16N5O14P3 | YES | | | 13316 | 7192 | 0 | 15810,5 |
| 5,6,7,8-Tetrahydrofolate | 2344 | C19H23N7O6 | YES | | | 885 | 4 | 54 | 11685,8 |
| THF-L-glutamate | 2345 | C24H30N8O9 | YES | THF-L-glutamate | | 0 | 0 | 0 | 7087,25 |
| Thymidine | 2346 | C10H14N2O5 | YES | | | 909 | 2124 | 4938 | 8256 |

| | | | | | | | | | |
|---|---|---|---|---|---|---|---|---|---|
| 1,3-Thiazole | 2347 | C3H3NS | NO | Thiazole | 152448 | 153 | 0 | 62 | 7697,5 |
| Thiomorpholine | 2348 | C4H9NS | NO | Tetrahydro-2H-1,4-thiazine | 24851748 | 73 | 4483 | 0 | 6119,5 |
| Thiophene | 2349 | C4H4S | NO | Thiofuran | 151146 | 224 | 5088 | 40 | 8310,75 |
| Thiamin monophosphate | 2350 | C12H18N4O4PS | YES | | | 11510 | 142 | 4264 | 8923 |
| Thiamin | 2351 | C12H17N4OS | YES | | | 248 | 0 | 0 | 7935,25 |
| Thiamine diphosphate | 2352 | C12H19N4O7P2S | YES | | | 818 | 17 | 3266 | 351,75 |
| 3-Ethylphenol | 2353 | C8H10O | YES | | | 0 | 33 | 0 | 282 |
| dUDP | 2354 | C9H14N2O11P2 | YES | | | 2362 | 3590 | 0 | 303,25 |
| 1,3,6,8-Tetrahydroxynaphthalene | 2355 | C10H8O4 | YES | | | 0 | 3206 | 48 | 7723,25 |
| Guanine | 2356 | C5H5N5O | YES | | | 115 | 5197 | 0 | 4068,25 |
| L-Threonine | 2357 | C4H9NO3 | YES | | | 11147 | 3518 | 0 | 4165 |
| L-Threonate | 2358 | C4H8O5 | YES | | | 1419 | 0 | 0 | 11791,3 |
| alpha-Tocopherol | 2359 | C29H50O2 | YES | | | 33 | 0 | 0 | 1274,75 |
| 5,7,3',4'-Tetrahydroxyflavone | 2360 | C15H10O6 | YES | | | 175 | 3956 | 0 | 1475,5 |
| Thiepin | 2361 | C6H12S | NO | | | 0 | 73 | 0 | 8648,5 |
| 3-Hydroxy-3-isohexeneylglutaryl-CoA | 2362 | C32H52N7O20P3S | NO | see http://umbbd.ahc.umn.edu | | 0 | 0 | 0 | 0 |
| 2-Chlorohexane | 2363 | C6H13Cl | NO | | 155845 | 0 | 0 | 0 | 0 |
| N,N,N-Trimethyl-L-histidine | 2364 | C9H15N3O2 | NO* | | | 185 | 0 | 0 | 3559,25 |
| 2,4,6-Trinitrotoluene | 2365 | C7H5N3O6 | YES | | | 4726 | 0 | 0 | 0 |
| Thymine | 2366 | C5H6N2O2 | YES | | | 1671 | 2147 | 5587 | 7902 |
| gamma-Tocopherol | 2367 | C28H48O2 | YES | | | 0 | 3713 | 0 | 3758 |
| Toluene-4-sulfonate | 2368 | C7H8O3S | YES | | | 0 | 0 | 0 | 4945 |
| Toluene | 2369 | C7H8 | YES | | | 0 | 135 | 0 | 672 |
| 4-Ethylphenol | 2370 | C8H10O | YES | | | 0 | 0 | 0 | 7728 |

| | | | | | | | | | |
|---|---|---|---|---|---|---|---|---|---|
| D-myo-Inositol 1,4,5-trisphosphate | 2371 | C6H15O15P3 | YES | | | 0 | 3805 | 17 | 7440 |
| 1-Bromohexane | 2372 | C6H13Br | NO | n-Hexyl bromide; Hexyl bromide; Bromohexane | 151227 | 0 | 0 | 0 | 479 |
| 1-Chlorohexane | 2373 | C6H13Cl | NO | | 154295 | 86 | 0 | 0 | 106,75 |
| 7-Methyl-3-oxo-6-octenoyl-CoA | 2374 | C30H48N7O18P3S | NO | see http://umbbd.ahc.umn.edu | | 2024 | 2365 | 0 | 700,75 |
| 1,2-Dibromohexane | 2375 | C6H12Br2 | NO* | | | 0 | 826 | 0 | 889,75 |
| Oxidized thioredoxin | 2376 | C6H7NO2S2(Protein)2 | YES | | | 12 | 4832 | 0 | 0 |
| Reduced thioredoxin | 2377 | C6H9NO2S2(Protein(2) | YES | | | 0 | 0 | 0 | 5180,75 |
| Trehalose | 2378 | C12H22O11 | YES | | | 0 | 0 | 0 | 3766,75 |
| Trehalose 6-phosphate | 2379 | C12H23O14P | YES | | | 168 | 0 | 0 | 3184 |
| Benzoylformate | 2380 | C8H6O3 | YES | | | 0 | 5190 | 0 | 5178,25 |
| Triacetin | 2381 | C6H5O6(C2H4O2)3 | YES* | Triglyceride | | 98 | 0 | 6317 | 2786,25 |
| Glyceryl tributyrate | 2382 | C15H26O6 | YES | | | 0 | 0 | 0 | 105,75 |
| Isohexenyl-glutaconyl-CoA | 2383 | C32H50N7O19P3S | YES | | | 0 | 65 | 41 | 801,75 |
| Citronellyl-CoA | 2384 | C31H52N7O17P3S | NO | see http://umbbd.ahc.umn.edu | | 965 | 0 | 0 | 0 |
| Glyceryl tridecanoate | 2385 | C6H5O6(C10H20O2)3 | YES* | Triglyceride | | 2319 | 0 | 0 | 493 |
| Glyceryl tridodecanoate | 2386 | C6H5O6(C12H24O2)3 | YES* | Triglyceride | | 12 | 89 | 10470 | 138 |
| 3-Fluoro-cis,cis-muconate | 2387 | C6H5FO4 | YES | | | 3251 | 6459 | 0 | 5056,25 |
| 3-Fluorocatechol | 2388 | C6H5FO2 | NO | see http://umbbd.ahc.umn.edu | | 4600 | 0 | 2189 | 2337,75 |
| 4,5,6-Trinitrotriazine | 2389 | C3N6O6 | NO | | 33741649 | 0 | 0 | 0 | 5908,25 |
| Glyceryl trihexanoate | 2390 | C6H5O6(C6H12O2)3 | YES* | Triglyceride | | 239 | 0 | 0 | 45 |
| Glyceryl trioctanoate | 2391 | C6H5O6(C8H | YES* | Triglyceride | | 0 | 3682 | 0 | 924,75 |

| | | | | | | | | | |
|---|---|---|---|---|---|---|---|---|---|
| | | 16O2)3 | | | | | | | |
| Glycerol trioleate | 2392 | C6H5O6(C18 H36O2)3 | YES* | Triglyceride | | 0 | 1195 | 0 | 4136,5 |
| 1,4-Lactone | 2393 | C4H6O2 | YES | | | 0 | 99 | 0 | 4032,5 |
| Glyceryl trihexadecanoate | 2394 | C6H5O6(C16 H32O2)3 | YES* | Triglyceride | | 0 | 3143 | 3266 | 508 |
| Glyceryl tripropionate | 2395 | C6H5O6(C3H 6O2)3 | YES* | Triglyceride | | 811 | 0 | 0 | 47,25 |
| Tropate | 2396 | C9H10O3 | YES | | | 0 | 152 | 0 | 259,75 |
| Glyceryl tritetradecanoate | 2397 | C6H5O6(C14 H28O2)3 | YES* | Triglyceride | | 53 | 3727 | 0 | 4867,5 |
| Tropine | 2398 | C8H15NO | YES | | | 0 | 26 | 0 | 2472 |
| dUMP | 2399 | C9H13N2O8P | YES | | | 819 | 2024 | 1164 | 5882,25 |
| L-Tryptophan | 2400 | C11H12N2O2 | YES | | | 0 | 0 | 1683 | 346,25 |
| D-Tryptophan | 2401 | C11H12N2O2 | YES | | | 0 | 0 | 1887 | 96,5 |
| Tryptamine | 2402 | C10H12N2 | YES | | | 0 | 1560 | 212 | 5744 |
| 1-Methyl-1-phenylethylene | 2403 | C9H10 | YES | | | 0 | 0 | 0 | 8238,25 |
| Tetracosanoic acid | 2404 | C24H48O2 | YES | | | 0 | 0 | 0 | 0 |
| Tetracosanoyl-CoA | 2405 | C45H82N7O1 7P3S | YES | | | 6 | 0 | 0 | 331,25 |
| Tylactone | 2406 | C23H38O5 | YES | | | 0 | 0 | 0 | 1362,25 |
| Tetradecane | 2407 | C14H30 | NO | | 155710 | 0 | 0 | 0 | 2653,5 |
| Methyl tetradecenoate | 2408 | C15H28O2 | NO | Methyl (Z)-tetradec-9-enoate; Methyl cis-9-tetradecenoate | 720777 | 15 | 0 | 25 | 2405,5 |
| (9Z)-Tetradecenoic acid | 2409 | C14H26O2 | YES | | | 0 | 0 | 0 | 2847,5 |
| Tetradecanoate | 2410 | C14H28O2 | YES | | | 258 | 0 | 0 | 6511 |
| UDP-2,3-bis(3-hydroxytetradecanoyl)glucosamine | 2411 | C43H77N3O2 0P2 | YES | | | 0 | 84 | 4 | 1290 |
| L-Tyrosine | 2412 | C9H11NO3 | YES | | | 8086 | 0 | 0 | 8294 |
| Tyramine | 2413 | C8H11NO | YES | | | 941 | 4625 | 4591 | 10882,3 |

| | | | | | | | | | |
|---|---|---|---|---|---|---|---|---|---|
| UDP-3-O-(3-hydroxytetradecanoyl)-N-acetylglucosamine | 2414 | C31H53N3O19P2 | YES | | | 0 | 2130 | 0 | 8421,5 |
| UDP-3-O-(3-hydroxytetradecanoyl)-D-glucosamine | 2415 | C29H55N3O20P2 | NO* | | | 218 | 0 | 0 | 574,25 |
| UDP-N-acetyl-2-amino-2-deoxy-D-glucuronate | 2416 | C17H25N3O18P2 | YES | | | 76 | 31 | 0 | 8205,5 |
| UDP-N-Acetyl-3-O-(1-carboxyvinyl)-D-glucosamine | 2417 | C20H29N3O19P2 | YES | | | 252 | 0 | 0 | 2750,25 |
| Undecaprenyl-diphospho-N-acetylmuramoyl-(N-acetylglucosamine)-L-alanyl-D-glutamyl-meso-2,6-diaminopimeloyl-D-alanyl-D-alanine | 2418 | C95H156N8O28P2 | YES | | | 0 | 718 | 0 | 457 |
| UDP-N-Acetyl-D-glucosamine | 2419 | C17H27N3O17P2 | YES | | | 659 | 85 | 0 | 1551 |
| UDP-N-Acetyl-D-mannosamine | 2420 | C17H27N3O17P2 | YES | | | 546 | 0 | 28 | 9067 |
| UDP-N-Acetyl-D-mannosaminouronate | 2421 | C17H25N3O18P2 | YES | | | 32 | 4492 | 0 | 10098 |
| Undecaprenyl-diphospho-N-acetylmuramoyl-L-alanyl-D-glutamyl-meso-2,6-diaminopimeloyl-D-alanyl-D-alanine | 2422 | C87H143N7O23P2 | YES | | | 0 | 0 | 0 | 7143,75 |
| UDP-N- | 2423 | C23H36N4O2 | YES | | | 79 | 0 | 0 | 1574,5 |

| | | | | | | | | | |
|---|---|---|---|---|---|---|---|---|---|
| Acetylmuramoyl-L-alanine | | 0P2 | | | | | | | |
| UDP-N-Acetylmuramoyl-L-alanyl-D-glutamate | 2424 | C28H43N5O23P2 | YES | | | 0 | 7142 | 0 | 1419,5 |
| UDP-N-Acetylmuramate | 2425 | C20H31N3O19P2 | YES | | | 0 | 0 | 0 | 8782,5 |
| Uridine 5'-diphosphate | 2426 | C9H14N2O12P2 | YES | | | 1293 | 200 | 7423 | 444,25 |
| Undecaprenyl phosphate | 2427 | C55H91O4P | YES | | | 75 | 197 | 26 | 9151 |
| Undecaprenyl diphosphate | 2428 | C55H92O7P2 | YES | | | 54 | 0 | 0 | 8448,25 |
| UDP-D-glucose | 2429 | C15H24N2O17P2 | YES | | | 2877 | 0 | 0 | 4396,5 |
| Methylitaconate | 2430 | C6H8O4 | YES | | | 148 | 0 | 0 | 1091 |
| UDP-D-galactose | 2431 | C15H24N2O17P2 | YES | | | 207 | 0 | 0 | 8673,5 |
| UDP-D-Galacto-1,4-furanose | 2432 | C15H24N2O17P2 | YES | | | 48 | 2474 | 1431 | 11092,8 |
| UDP-D-xylose | 2433 | C14H22N2O16P2 | YES | | | 0 | 0 | 25 | 10754,5 |
| UDP-D-glucuronate | 2434 | C15H22N2O18P2 | YES | | | 1138 | 48 | 0 | 6684 |
| UDP-3-Ketoglucose | 2435 | C15H22N2O17P2 | YES | | | 75 | 178 | 105 | 8487 |
| UDP-L-Rhamnose | 2436 | C15H24N2O16P2 | YES | | | 1007 | 0 | 0 | 270 |
| UDP-N-Acetyl-D-galactosamine | 2437 | C17H27N3O17P2 | YES | | | 398 | 193 | 25 | 2086,5 |
| UDP-N-acetylmuramoyl-L-alanyl-D-gamma-glutamyl-meso-2,6-diaminopimelate | 2438 | C35H55N7O26P2 | YES | | | 384 | 0 | 0 | 8299 |

| | | | | | | | | | |
|---|---|---|---|---|---|---|---|---|---|
| UDP-N-acetylmuramoyl-L-alanyl-D-glutamyl-meso-2,6-diaminopimeloyl-D-alanyl-D-alanine | 2439 | C41H65N9O28P2 | YES | | | 1548 | 0 | 0 | 2888,75 |
| UMP | 2440 | C9H13N2O9P | YES | Uridine monophosphate | | 1116 | 5 | 0 | 8099,75 |
| 2,3-Didehydro-pimeloyl-CoA | 2441 | C28H44N7O19P3S | YES | | | 408 | 2510 | 0 | 1582 |
| 3-Oxopimeloyl-CoA | 2442 | C28H44N7O20P3S | YES | | | 117 | 552 | 0 | 4302,75 |
| Undecanoic acid | 2443 | C11H22O2 | NO | Undecylic acid | 151309 | 0 | 240 | 0 | 908,5 |
| 3-Hydroxy-5-oxohexanoyl-CoA | 2444 | C27H44N7O19P3S | YES | | | 2456 | 0 | 0 | 5580,5 |
| 4-Hydroxy-2-oxovalerate | 2445 | C5H8O4 | YES | | | 106 | 5336 | 0 | 12810,8 |
| Cyclohexaamylose | 2446 | C36H60O30 | NO | alpha-Cyclodextrin | 167587 | 0 | 63 | 0 | 9298,5 |
| Uracil | 2447 | C4H4N2O2 | YES | | | 2213 | 0 | 40 | 0 |
| 5-Ureido-4-imidazole carboxylate | 2448 | C5H6N4O3 | YES | | | 606 | 0 | 0 | 5930,75 |
| 3',5'-Cyclic IMP | 2449 | C10H11N4O7P | YES | | | 0 | 0 | 5346 | 147,333 |
| Urea-1-carboxylate | 2450 | C2H4N2O3 | YES | | | 0 | 3938 | 0 | 2121,75 |
| Butanoyl phosphate | 2451 | C4H9O5P | YES | | | 25 | 0 | 0 | 674,75 |
| Urocanate | 2452 | C6H6N2O2 | YES | | | 141 | 22 | 0 | 408,75 |
| 2-Oxovalerate | 2453 | C5H8O3 | YES | UTP | | 62 | 0 | 0 | 0 |
| Cyclopentanol | 2454 | C5H10O | YES | | | 270 | 0 | 7240 | 0 |
| p-Nitrophenol octanoate | 2455 | C14H21O4N | NO* | | | 120 | 6649 | 0 | 6839,25 |
| Vanillin | 2456 | C8H8O3 | YES | | | 3 | 0 | 0 | 6034 |
| L-Valine | 2457 | C5H11NO2 | YES | | | 332 | 121 | 0 | 0 |
| Vanillate | 2458 | C8H8O4 | YES | | | 190 | 29 | 34943 | 4286,25 |
| Neuraminic acid | 2459 | C9H17NO8 | YES | | | 76 | 72 | 0 | 0 |
| delta-Valerolactone | 2460 | C5H8O2 | YES | | | 1151 | 0 | 3522 | 1853 |

| | | | | | | | | | |
|---|---|---|---|---|---|---|---|---|---|
| gamma-Undecalactone | 2461 | C11H20O2 | YES | | | 36 | 0 | 0 | 0 |
| 5-Methyl-3-oxo-4-hexenoyl-CoA | 2462 | C28H44N7O18P3S | YES | | | 3822 | 2942 | 0 | 5755,25 |
| Vermelone | 2463 | C10H10O3 | YES | | | 0 | 546 | 0 | 621 |
| 1,3,7-Trimethylxanthine | 2464 | C8H10N4O2 | YES | | | 0 | 78 | 0 | 0 |
| Methyl salicylate | 2465 | C8H8O3 | YES | | | 9 | 166 | 0 | 0 |
| Xanthosine 5'-phosphate | 2466 | C10H13N4O9P | YES | | | 0 | 0 | 0 | 0 |
| o-Xylene | 2467 | C8H10 | YES | | | 0 | 3863 | 0 | 0 |
| Xanthene | 2468 | C13H10O | YES | | | 85 | 4859 | 0 | 108 |
| o-Xylene | 2469 | C8H10 | YES | | | 0 | 187 | 0 | 433,5 |
| L-Xylulose 1-phosphate | 2470 | C5H11O8P | YES | | | 0 | 139 | 2391 | 0 |
| 1,3-beta-D-Xylan | 2471 | (C5H8O5)20 | YES | | | 50 | 141 | 0 | 6087,5 |
| L-Xylulose 5-phosphate | 2472 | C5H11O8P | YES | | | 0 | 0 | 0 | 792 |
| D-Xylulose 5-phosphate | 2473 | C5H11O8P | YES | | | 1609 | 0 | 28 | 1327,5 |
| D-Xylose | 2474 | C5H10O5 | YES | | | 0 | 0 | 0 | 295 |
| Chloroxylenol | 2475 | C8H9ClO | YES | | | 0 | 0 | 0 | 3311 |
| m-Xylene | 2476 | C8H10 | YES | | | 0 | 0 | 0 | 4587,5 |
| L-Xylose | 2477 | C5H10O5 | YES | | | 0 | 0 | 0 | 0 |
| Xyloglucan | 2478 | (C5H10O5)20 | YES | | | 2243 | 1078 | 0 | 0 |
| p-Xylene | 2479 | C8H10 | YES | | | 296 | 216 | 0 | 959,75 |
| Xylitol | 2480 | C5H12O5 | YES | | | 333 | 0 | 0 | 3824 |
| Xylitol 5-phosphate | 2481 | C5H13O8P | YES | | | 92 | 0 | 0 | 0 |
| L-Xylulose | 2482 | C5H10O5 | YES | | | 755 | 0 | 0 | 0 |
| Zymosterol | 2483 | C27H44O | YES | | | 0 | 0 | 1417 | 138,25 |
| | | | | | | | | | |

Compound in row 243 (2,2-Dichloropropanoic acid) is the control for Pseudomonas putida Experiments
Compound in row 1746 (Methyl-3-bromo-2-methylpropionate) is the control for Metagenomic Experiments
YES* represent molecules which do not match exactly to KEGG but they are named generally in KEGG
NO* represent molecules not found in KEGG and PubChem Databases.
*B= block, C=columna, R=row

table 4. Detailed information of hypothetical proteins identified in the present study through the analysis of KT2440 single cells and VUL, KOL and L'A communities. REBr sequence is also shown.

| Gene name | Gene length | Acc. Number | Peptides Q-TOF | Primers for amplification from library | PCR amplification primers | DNA sequence | Enzyme name | Enzyme class |
|---|---|---|---|---|---|---|---|---|
| PP_1394 | 1638 | NP_743553 | - | - | PP_1394 F, GACGACGACAAGATGGCAACCTGCGGCGAAGTACTG; PP_1394 R, GAGGAGAAGCCCGGTCAACCCAACACCGCGGCCTG | ATGGCAACCTGCGGCGAAGTACTGGTCAAACTCCTTGAAGGCTATGGCGTCGATCATGTCTTCGGCATCCCCGGTGTGCATACCGTGGAGCTCTATCGTGGCCTGGCGGGCTCTTCCATTCGCCACATCACCCCGCGTCATGAGCAAGGTGCCGGGTTCATGGCTGACGGCTATGCGCGCACCCGCGGCAAACCCGGGGTATGCTTCATCATCACTGGCCCGGGCATGACCAATATCACCACTGCCATGGGCCAGGCCTATGCCGACTCGATCCCGATGCTGGTGATTTCCAGCGTGCAGTCCCGTGACCAACTGGGCGGTGGCCGTGGCAAGTTGCACGAGCTGCCCAACCAAGCCGCGCTGGTATCGGGGGTGGCGGCGTTTTCCCACACCCTGATGAGTGCAGCCGACTTGCCGCAGGTGCTGGCCCGGGCATTTGCCGTGTTCGACAGTGCCCGGCCACGCCCGGTGCATATCGAAATCCCGCTCGATGTGCTGGTTGAACCGGCCGACTTCCTGCTGCCGGGCCGCCCTGTACGTGGCAGTCGGGCTGGGGCTGCACCGCAGGCCGTGGCGCAGATGGCTGAGCGACTGGCCAGTGCACGTCGGCCGCTGATTCTGGCCGGTGGCGGGGCGTTGGCTGCGGGCGCTGCGCTGGCACGCCTGGCCGAACACCTTCAGGCCCCGGTGGCGCTGACCATCAATGCCAAAGGCTTGCTGCCAGCCAGCCACCCATTGCAGATCGGCTCGACCCAGTCGTTACCCGCCACACGGGCGCTGGTGGCCGAGGCCGACGTGGTGCTTCGGTACCGAACTGGCTGAAACCGATTATGACGTGACCTTCAAGGGAGGCTTCGAGATCCCGGGCAGGCTGCTGCGTATTGACATCGACCCGGACCAGACCGTGCGCAATTATC | PP_1394 | 2-keto-4-pentenoate hydratase |

| | | | | | | | | |
|---|---|---|---|---|---|---|---|---|
| | | | | | | TGCCGGAGCTGGCGCTGGTAGCCGATGCCGAGCTGGCAGCCGAAGCGCTGCTCGGTGCCGTGCAAGCCCAGCCGCAGCCAGTGCACGAAAGCACTTGGGGCGTGGCCCGCGTGGCCGACCTGCGCAAGGTCCTGGCAGCAGACTGGGACCAGCCAACCCTGAGCCAGACACGTTTGCTGAGCGCCATACTGGAGCGCCTGCCTGATGCGATTCTGGTGGGCGACTCGACCCAACCTGTGTACACCGGCAACCTGACACTCGACATGCAGCAGCCACGCCGCTGGTTCAACGCCTCGACCGGTTACGGCACCTTGGGTTACGCGTTGCCAGCAGCCATGGGCGCCTGGTTGGGCAGTGCCGAGCAAGCCGTCGAACGCGCTCCGGCGGTGTGCCTGATTGGTGATGGTGGTTTGCAGTTCACCCTGCCGGAACTGGCCAGTGCAGTGGAGGCGCAGGTACCGCTGATCGTACTGCTGTGGAATAACCAGGGGTACGAGGAAATCAAGAAATACATGGTCAATCGGGCGATCGAGCCAGTCGGGGTGGATATCCATACCCCGGATTTCATCGGCGTGGCGCGGGCGTTGGGCGCGGCAGCAGAGAACGTGGCCGATATCGCCCAATTGCAGGCCGCGCTCGGGCAAGCGGTGGAGCGCAAGGGGCCGACCTTGATTCAGGTGGACCAGAATCAGTGGCAGGCCGCGGTGTTGGGTTGA | | |
| PP_1752 | 1494 | NP_743908 | - | - | PP_1752 F, GACGACGACAAGATGTTCGAATCTGCCGAAATCGGCCAC; PP_1752 R, GAGGAGAAGCCCGGCTATTTCTTGTGCTTGGCAAACGCCG | ATGTTCGAATCTGCCGAAATCGGCCACAGCATCGACAAGGAGGCTTACGACGCCGAGGTACCCGCTTTACGCGAGGCCCTGCTCGAAGCCCAGTACGAACTCAAGCAGCAGGCGCGTTTTCCGGTGATCGTGCTGATCAACGGCATTGAAGGCGCCGGCAAGGGTGAGACGGTAAAACTGCTCAACGAGTGGATGGACCCGCGCATGATCGATGTGCTCACCTTCGACCAGCAGACCGACGAAGAGCTGGCCCGCCCGCCCGCCTGGCGCTATTGGCGGGCCTTGCCGCCCAAGGGGCGAATGGGCGTTTTCTTTGGCAACTGGTACAGCCAGATGCTGCAGGGGCGGGTGCACGGGGTGTTCAAGGATGCCGTGCTCGATCAGGCCATTACCGGTGCCGAACGCCTGGAGGAGATGCTGTGCGA | PP_1752 | 3-carboxy-*cis,cis*-muconate cycloisomerase |

| | | | | | | | | |
|---|---|---|---|---|---|---|---|---|
| | | | | | | TGAAGGTGCGCTGATCATCAAGTTCTGGTTCCAC<br>CTGTCCAAGAAGCAGATGAAGGCACGGCTGAAA<br>TCGCTCAAGGACGACCCGCTGCACAGCTGGAAG<br>ATCAGCCCGCTGGATTGGCAGCAGTCGCAAACC<br>TACGACCGTTTCGTGCGCTTTGGCGAGCGCGTG<br>CTGCGCCGCACCAGCCGTGACTATGCGCCATGG<br>CACATCATCGAAGGGGTAGACCCGAATTACCGC<br>AGCCTGGCGGTGGGGCGCATTCTGCTGGAAAG<br>CCTGCAAGCCGCGCTGGCCCACAATCCCAAGGG<br>CAAGCACCAGGGCAACGTGGCTCCATTGGGCCG<br>CAGCATCGACGACCGCAGCCTGCTTGGCGCCCT<br>GGACATGACCTTGCGCCTGGACAAGGCCGACTA<br>TCAGGAACAGTTGATCACCGAACAGGCGCGTCT<br>GGCCGGCCTGCTGCGTGACAAGCGCATGCGCC<br>GGCACGCCCTGGTGGCGGTGTTCGAAGGCAAC<br>GACGCCGCCGGCAAGGGGGGCGCCATCCGCCG<br>CGTGGCGGCGGCGCTGGACCCGCGTCAGTACC<br>GTATCGTACCGATTGCCGCGCCGACCGAAGAAG<br>AGCGCGCCCAGCCGTACCTGTGGCGGTTCTGG<br>CGGCACATCCCGGCACGCGGCAAGTTCACCATC<br>TTCGACCGTTCCTGGTATGGCCGGGTGCTGGTG<br>GAGCGGGTGGAAGGCTTCTGCAGCCCCGCTGA<br>CTGGATGCGTGCCTATAGCGAGATCAACGACTTT<br>GAAGAGCAGTTGGTGGATGCCGGCGTGGTGGT<br>GGTCAAGTTCTGGCTGGCGATCGACCAGCAGAC<br>CCAACTGGAGCGCTTCGAAGAGCGTGAGCAGAT<br>TCCGTTCAAACGCTACAAGATCACCGAGGATGA<br>CTGGCGCAACCGCGACAAGTGGGACGAATACTC<br>CCAGGCGGTGGGCGACATGGTCGACCGCACCA<br>GCAGCGAGATTGCCCCTTGGACGCTGGTGGAG<br>GCCAATGACAAGCGCTGGGCGCGGGTGAAGGT<br>GGTGCGCACAATCAACCAGGCGCTTGAGGCGG<br>CGTTTGCCAAGCACAAGAAATAG | | |
| PP_2 949 | 867 | NP_7 4509 | - | - | PP_2949 F, GACGACGACAAGATGGAAA | ATGGAAAAATCAGCAACCGGGTGGATCAACGGT<br>TTCATAGGCGTCGCCATTTTTGCGGGTTCGTTGC | PP_294 9 | 3- hydroxyanth |

| | | | | | | | | |
|---|---|---|---|---|---|---|---|---|
| | | `3 | | | AATCAGCAACCGGGTGGATC; PP_2949 R; GAGGAGAAGCCCGGTTAGGCATATTTCCTGGCCCCGTAAAC | CGGCAACCCGAGTGGCAGTGGCCGACTTCGAACCGACGTTCCTCACCTGTGCCCGGGCAACAATTGCCGCCATGCTGGGCGCACTTTTTCTGATCGTGCTACGCCAGCCTCGACCCAAACGGCGGGATTTGTCGCCTTTGGCTGTAACTGCGCTCGGCGTTGTTATCGGTTTCCCGCTACTGACAGCATTTGCCCTTCAGCACATAAGCTCTGCTCACTCCATTGTTTTTGTCGGGCTCCTGCCATTGTGTACCGCAGGATTTGCGGTTCTGCGGGGCGGTGAACGACCTCGGCCATTGTTCTGGCTGTTCTCGTTGACAGGTGCCGGGTTGGTCGCTGGCTATGCGTTGATGAATGGAGGCGAGGCGTCGGCGGTGGGCGATCTGCTGATGTTGGCTGCGGTTGTGGTCTGTGGGTTGGGCTATGCCGAAGGAGCGCGCCTCTCGCGGACATTGGGTGGTTGGCAGGTGATCAGCTGGGCGTTGCTGGTAGCGTTGCCGTTCATGCTGCTGCTGACGGTGGTCAATCTTCCAGCCCCTGATGACTTTGCCAGGGTAAGTGCCCCTGCGTGGTTCAGCTTTGGCTACGTTTCACTGTTCAGCATGCTGATCGGGTTTGTGTTCTGGTACCGAGGGCTCGTCCAGGGGGGGATTGCGGCAGTGGGCCAATTGCAACTCTTTCAGCCGTTCATGGGGCTTGGGCTGGCAGCGTTGCTTCTGCACGAGCATGTCAGCTGGACGATGCTGCTCGTGACGCTGGGTGCTGTCATCTGTGTTTACGGGGCCAGGAAATATGCCTAA | | ranilate 3,4-dioxygenase |
| PP_1642 | 672 | NP_743799 | - | - | PP_1642 F, GACGACGACAAGATGCGTGATTACCAGTGGTTGCATGAG; PP_1642 R, GAGGAGAAGCCCGGTCAATGGTTGACGGTGTGCGCC | ATGCGTGATTACCAGTGGTTGCATGAGTATTGCCTGAACCGCTTTGGTTCGGCCCAGGCGCTGGAGGCTTTCCTGCCGCAGCCGCGCACGCCGGCGCAACTGCGCGACATCAGTGACGACCGCTACCTGTCGACATTGGCCCTGCGCGTGTTCCGCGCGGGGCTCAAGCACAGCCTGGTGGATGCCAAGTGGCCGGCGTTCGAGCAGGTGTTCTTTGGCTTCGACCCGGAGAAAGTGGTGCTGATGGGCGCCGAGCATCTGGAGCGGTTGATGCAGGATGAGCGCATTATCCGCCACCTGGGCAAGCTCAAGAGCGTGCCACGCAATGC | PP_1642 | isopentenyl-diphosphate delta-isomerase |

| | | | | | | | | |
|---|---|---|---|---|---|---|---|---|
| | | | | | | GCAAATGGTGCTGGATATCGCCAAGGCGCATGGCAGTTTTGGTGCATTCATCGCCGATTGGCCAGTGACCGACATTGTCGGCTTGTGGAAGTACCTGGCCAAGCACGGCAACCAGTTGGGCGGTTGTCGGCGCCACGGTTCTTGCGCATGGTCGGCAAGGACACGTTCATCCCGACCGATGACATGGCAGCGGCGTTGATTGCGCAGAAGGTGATCGACAAGCAGCCAACCAGCCAGCGCGACCTGGCTCTGGTGCAGCAGGCGTTCAACCAGTGGCATGCAGAGAGCGGGCGGCCGCTGTGCCAGTTGTCGGTGATGCTGGCGCACACCGTCAACCATTGA | | |
| _ | 405 | _ | TTEHGTGA & PPCRFDVM | Fw1: ACIACIGARGARGGIACIGGIGC; Rev1 CATNACNTCNAANCKRCANGGNGG | Kulguev-1 F, ATGTGGATACCTCGCAACAAGGCTAAGTCG; Kulguev-1 R, TTAAAACGCATTACTAATCCAATCGAAGATAACTGG | ATGTGGATACCTCGCAACAAGGCTAAGTCGAGTGCCAGTAAAGGCAAAACCGGTGATAGCAGTCATCGTGCCGCAGGCGAACGCCGAGAATTAGACGCCGAAGAGTGGCTGATACAACGTGGCTTTGTACCCATAACCCGCAATTACCGCACGCGCGGCGGCGAAATCGATCTGATTATGCGCGACGCCGATACCCTTGTGTTTGTAGAAGTACGTTATCGTAAAACCACGGAGCACGGCACGGGGGCAGAAACCATTACCTATCACAAACAGCAGCGACTACGTCGTGCTGCCCTACACTACCTGCAAAAGCATTTTGGTAGCCGCGAACCGCCTTGTCGATTTGATGTTATGTCAGGTACTGGCGACCCAGTTATCTTCGATTGGATTAGTAATGCGTTTTAA | KOL1 | alkane hydroxylase |
| _ | 888 | _ | PYAGLAYAG & WVGDLQAGIA | Fwd1: CCNTANGCNGGNYTNGCNTAYGCNGG; Fwd2: AANGANGANGANMGNCCNTAYGCNGG and Rev1: GCNATNCCNGCYTGNARYTCNCCNACCC | Kulguev-7 F, ATGTGGCCAGCTATCGCTTCGGCC; Kulguev-7 R, TCAAAACGCCCGAGCCACCACCAG | ATTGTAATACGACTCACTATAGGGCGAATTGGGCCCGACGTCGCATGCTCCCGGCCGCCATGGCGGCCGCGGGAATTCGATTATGTGGCCAGCTATCGCTTCGGCCATCAGATTTACACCCCCGATGACATCGAAATAGCGTCATTGATAGAAGACGATCGCCCTTATGCGGGCCTAGCCTATGCTGGCCTGTCGATCTTCCAATCCCGGGACCAGGGGCAATGGCGCGATAGTCGTGCCTGGCACATGGATCTGGGCCTAGTCGGCCCGGGTGCCGGAGGCCAGCGCTTCCAGAGTGCTGTCCATGGTGCTACCGGCAGTGATGAGCCCAAAGGTTGGGACAATCAGCTTGAGAATGAACCC | KOL7 | S-ribosylhomocysteine lyase |

| | | | | | | | | |
|---|---|---|---|---|---|---|---|---|
| | | | | | | TTCTTCAACGTGGCCTACGGGCAGCGTTGGTGGCGGCAGTCCCGCCTGGGATCTTTGGAGCTTGAATACGGGCCTGCAATGGGCGCGGCGGCTGGCAATCTTTACACCTATGCTTCCAGCGGCCTTGGCTTGCGCTTTGGCAAAGGGCTGGAACACAGCTTGGGGTTACCGTCTATCAACCCGGGCTATGGTAGCGGCGCCTACTTCGAGCCGGGGCAATCGTTCGCCTGGTTCGGTTACGTCAATGTTGATGGCCGTTACATGGCTCATAACATGCTGCTGGACGGAAACACCTTCAGCAACAGCCATTCCGTAGACCGGGAGCAATGGGTCGGCGATCTGCAGGCGGGTATCGCCCTGACCTGGGAGCGCTGGCAAGTGAGCTTTGCCAGTGTGTGGCGCACCCGGGAGTTCAAGGGCCAGACTGAACCCGATCAATTTGGGTCGCTGGTGGAATCACTAGTGAATTCGCGGCCGCCTGCAgGTCGACCATATGGGAGAGCTCCCAACGCGTTGGATGCATGA | | |
| – | 519 | – | GVPKYVEV QVM & ASKAVF | Fw1: GGNGTNCCNANTANGTNGARGTNCARGTNATG; Rev1: RAANACNGCNTTNSWNGC | Kulguev-2 F, ATGCATCAGTTGGCAGTCACCGGC; Kulguev-2 R, TTAATTGACCAGAAAGCGCCTGCTGCC | ATGCATCAGTTGGCAGTCACCGGCAAGCAGGCAGCTGATGTGGCTGTGCTGCTGGGCGGTCAACAGCTTGAGGTGCACCGGATAGAGCGGGATGACGTCTTGATCCAGCACCTGATCGAGCTGGAACGTCAGTTCTGGCATTACGTTGAAACCGACACACCGCCACCAGCCGACGGCTCTGATTCCGCTGACATGGCACTGCGTCTGCTCTACCCGGAAGACACAGGTATGGTTATTGACCTCTCTCAGGATCAGTCCCTGAACGAGTCCTATACCGAGCTCAAGCAGGTACGGCAGTCACTCTCTGATCTGAGCACCCGAGAATCTGTTCTCAAGCAGCGTCTTCAAGAGTCCATGGGGTCAGCCAGTAAAGCCGTGTTTGCCAATGGCTCTATCACTTGGAAGAAAGCCAAGGATGGCATCGCCATGGATATGGAGGCCCTGTTCAAGGCGCACCCTGACCTGAAAACCCAATACCAGATCAGCAAGCCTGGCAGCAGGCGCTTTCTGGTCAATTAA | KOL2 | haloalkane dehalogenase |
| – | 474 | – | VEELHQSG & EAARSM & PDVNRA | Fw1: GTNGANGANNTNCANCARWS | Volcano-9 F, ATGTTGCGCTTGGTGCAGGGG; Volcano-9 R, | ATGTTGCGCTTGGTGCAGGGGGGATTCAGTAATATGGCAGCACCGATTCAGAAAGTCATCCGACAGGAACCCGTCAAAAATCCGCTTGAGTCTGGGAGC | VUL9 | phosphatidyl inositol-bisphosphat |

| | | | | | | | | |
|---|---|---|---|---|---|---|---|---|
| | | | & QFAIGF | NGG; Fw2: GAIGCIGCIMGIWGIATG; Rev1: CNCKNTTNACRTCNGG; Rev2: RAANCCNATNGCRAAYTG | CTAGATAGTTTTCCCGAGACC | CCGTCGGCTTCGGAGGAACTTCAACTATTGGTTGAAGAACTGCATCAGAGCGGGGTTCTCGAGGCTGCACGGTCGATGCTTGGAGCAAAGGATTCCATCGCCAAAATCCTGGTCGAGCAACTGCTGAGAAAGGATGTACTGACGCTTATCAACAATTTGATGGCGGCAGGCACCGTTCTGACAAAGCTCGATCCAGCGCAGCTCGAGCGCTTGACCGAAGGACTGAGTGCCGGGGTAACAGAGGCACATCAGACAATCGAAGCGAATCAGTCAATCAGTATCATGGGACTTTTGAAGACATTGCAAGACCCCGATGTAAACCGGGCACTCCAGTTTGCCATCGGGTTCCTCAGGGGTCTCGGGAAAACTATCTGA | | ase |
| – | 486 | – | LLMAEMTEEKL & KEIALGLNLSLSQV & PEKVPEIMAKYK | pET41L'A62Fwd: GACGACGACAAGATGATGTTATTGATGGCAGAGATGACAG; pET41L'A62Rev: GAGGAGAAGCCCGGTTATTTATACTTTGCCATAATTTCAGGAAC | pET41L'A62Fwd, GACGACGACAAGATGATGTTATTGATGGCAGAGATGACAG; pET41L'A62Rev, GAGGAGAAGCCCGGTTATTTATACTTTGCCATAATTTCAGGAAC | TTATTGATGGCAGAGATGACAGAAGAAAAATTAGCTGAGTATTTAGAACCTTTATCAGAAATTTTATCACGCTATGAAAAAAAAGAGAGATATTTAATTCCGGTTTTACAGGAAGCTCAGGAGGAATATGGTTATTTACCGGAAGAAGTAATGAAAGAAATAGCATTGGGCTTAAATCTTTCTTTAAGTCAGGTATATGGGGTTGTAACATTTTACAGTCAGTTTCATCAGGAGCCAAGAGGTAATAATATTATTCGGGTTTGTCTGGGAACAGCCTGTCATGTTAGAGGTGGAGATGGAATCTTAAATGCTATTAAAGATGAACTGGGAATTGATGCAGGAGAAACAACTGATGATTTAGAATTTACACTTGAATCTGTGGCCTGTATTGGTGCCTGTGGTCTGGCTCCAGTTATAATGGTCAATGATGATACCCACGGCCGTTTAACTCCGGAAAAAGTTCCTGAAATTATGGCAAAGTATAAA | L'A62 | Hydrogenase |
| (De)haloge nase REBr | | | | | | ATGAATCTCGTTAAAAGCCTGATAATACTGCTTGTGATCGCAGTGATCGGAATATTTGCTAGCGTCCACTTTGCTCCGTTCGAAACAGCTTCTTGTCTTGTCGATATTAAACGCAATATCGCCGGCCTAGAGCGCAAAAGCATTTCCCTCGCCGATGGTAACCAATATGTTTATCTTGAAGGCGGCAAGGGTGAAACCTTGGTATTACTTCACGGTTTTGGTGCCGATAAAGATAA | REBr | (De)halogenase |

| |
|---|
| |
| |
| CTTTACCGAAGTTAGCCCTTACTTAACGGGCGAC<br>TTCCATGTTATTGCTCCCGACCACATTGGCTTTG<br>GTGAATCCTCTAAGCCAACAGGCGCAGATTATAG<br>CCCTATCGCTCAAGCGCAGCGTTTGCATGAATTG<br>GTTGCGCGCCTCGGTTTAGAGCGCTTCCACTTA<br>GGTGGAAGCTCAATGGGTGGCCACATAGCGATG<br>ACATACGCCACGCTATACCCGCATGAAGTTAAGA<br>GCTTATGGTTACTCGACCCAGGTGGTGTTTGGTC<br>CGCTCCGGAAGCCGAAATGCGCACTATCATTCG<br>TAAAACTGGGGTGAATCCGTTAACAGCTAAAACC<br>CCAGAAGAGTTCCGTAAAGTATTCGATATCGTGA<br>TGAGCAAGCCCCCTTTCATCCCAGGCTTTGTACT<br>CGATGAAATGGCGAAAAAGCGTATCGCTAACTTC<br>GATTTAGAACAGAATATTTTTGCCCAACTATCTGC<br>AGACAATGTCGAAGAACGCGTCCGCGGCCTAAC<br>CACCCCTACCTTGCTAGTATGGGGCGCTGAAGA<br>TCGAGTACTCAACCCAGAAGCAGCACCCATTCT<br>GGAAGGACTTTTGACCAATGTTAAAACGATTATC<br>ATGCCAGGTATTGGTCACCTGCCTATGCTAGAAG<br>CGCCAAAGCAAACAGCAACCGACTTAAAAGCATT<br>TATTGCTGATTTGCCTGAATAA |
| |
| |
| |
| |
| |

## Claims

1. A probe compound for detecting specific enzyme-substrate interactions comprising a transition metal complex and a reactive component of general formula (X):

$$His\text{-}L_{His\text{-}TC}\text{-}TC\text{-}L_{TC\text{-}IC}\text{-}IC\text{-}L_{IC\text{-}His}\text{-}His \quad \text{formula (X)}$$

wherein His represents a histidine residue, TC represents a test component, IC represents an indicator component, and each of $L_{His\text{-}TC}$, $L_{TC\text{-}IC}$ and $L_{IC\text{-}His}$ independently represents optional linker components,
wherein the reactive component is linked to the transition metal complex by the two histidine residues, wherein the test component comprises a substrate, a metabolite, a pseudo-substrate or an inhibitor of an enzyme, and wherein the indicator component comprises a dye.

2. The probe compound according to claim 1, wherein the transition metal complex comprises a cobalt or copper atom.

3. The probe compound according to claim 1 or 2, wherein the transition metal complex further comprises a multidentate ligand, preferably comprising nitrotriacetic acid.

4. The probe compound according to one of the preceding claims, wherein the transition metal complex comprises a nitrotriacetic acid cobalt(II) moiety.

5. The probe compound according to one of the preceding claims, wherein the multidentate ligand of the transition metal complex further comprises an anchoring component.

6. The probe compound according to one of the preceding claims, wherein the transition metal complex is a cobalt(II) complex of $N_{\alpha}$,$N_{\alpha}$-bis-(carboxymethyl)-L-lysine.

7. The probe compound according to one of the preceding claims, wherein the indicator component comprises a fluorescence dye.

8. The probe compound according to one of the preceding claims, wherein the test component comprises a substrate selected from the group listed in Table 1.

9. The probe compound according to one of the preceding claims, wherein the indicator component comprises a fluorescene dye, preferably a Cy3 dye, and wherein the linker component $L_{TC\text{-}IC}$ comprises a quaternary ammine function.

10. A method of preparing a probe compound according to any one of claims 1 to 9, which comprises the steps of:

Preparing a transition metal complex by reacting a salt of a transition metal with a ligand molecule;
Preparing a reactive component by linking a test component to a histidine residue, optionally using a linker component, linking a dye component to a histidine component, optionally using a linker component, and linking the test component to the dye component, optionally using a linker component; and
Linking the reactive component to the transition metal complex by the two histidine residues.

11. An array for detecting enzymes comprising a plurality of different probe compounds according to any one of claims 1 to 9.

12. A method for producing an array according to claim 11, comprising the production of a plurality of different probe compounds according to the method of claim 10, wherein an anchoring component is linked to the ligand of the transition metal complex of the probe compound, and the arrangement of the different probe compounds in an array.

13. An isolation means comprising a nanoparticle and a probe compound according to any one of claims 1 to 9.

14. A method for producing an isolation means according to claim 13, comprising the production of a probe compound according to the method of claim 10, wherein an anchoring component is linked to the ligand of the transition metal complex of the probe compound, and the probe compound is attached to a nanoparticle.

15. A method for detecting enzymes, using the probe compound according to any one of claims 1 to 9, or the array according to claim 11, wherein the probe compound or the array is brought into contact with a sample of an analyte solution comprising enzymes.

**16.** A method for isolating enzymes, using the isolation means according to claim 13.

**Patentansprüche**

**1.** Sondenverbindung zum Nachweisen von spezifischen Enzym-Substrat-Wechselwirkungen, umfassend einen Übergangsmetallkomplex und eine reaktive Komponente mit der allgemeinen Formel (X):

$$His\text{-}L_{His\text{-}TC}\text{-}TC\text{-}L_{TC\text{-}IC}\text{-}IC\text{-}L_{IC\text{-}His}\text{-}His \qquad \text{Formel (X)}$$

wobei His einen Histidinrest darstellt, TC eine Testkomponente darstellt, IC eine Indikatorkomponente darstellt, und jede von $L_{His\text{-}TC}$, $L_{TC\text{-}IC}$ und $L_{IC\text{-}His}$ unabhängig voneinander optionale Verbinderkomponenten darstellt, wobei die reaktive Komponente mit dem Übergangsmetallkomplex durch die zwei Histidinreste verbunden ist, wobei die Testkomponente ein Substrat, einen Metaboliten, ein Pseudosubstrat oder einen Inhibitor eines Enzyms umfasst, und wobei die Indikatorkomponente einen Farbstoff umfasst.

**2.** Sondenverbindung nach Anspruch 1, wobei der Übergangsmetallkomplex ein Kobalt- oder Kupferatom umfasst.

**3.** Sondenverbindung nach Anspruch 1 oder 2, wobei der Übergangsmetallkomplex weiter einen vielzähnigen Liganden umfasst, vorzugsweise umfassend Nitrotriessigsäure.

**4.** Sondenverbindung nach einem der vorhergehenden Ansprüche, wobei der Übergangsmetallkomplex eine Nitrotriessigsäure-Kobalt(II)-Einheit umfasst.

**5.** Sondenverbindung nach einem der vorhergehenden Ansprüche, wobei der vielzähnige Ligand des Übergangsmetallkomplexes weiter eine verankernde Komponente umfasst.

**6.** Sondenverbindung nach einem der vorhergehenden Ansprüche, wobei der Übergangsmetallkomplex ein Kobalt (II)-Komplex von $N_\alpha$,$N_\alpha$-Bis-(carboxymethyl)-L-lysin ist.

**7.** Sondenverbindung nach einem der vorhergehenden Ansprüche, wobei die Indikatorkomponente einen Fluoreszenzfarbstoff umfasst.

**8.** Sondenverbindung nach einem der vorhergehenden Ansprüche, wobei die Testkomponente ein Substrat umfasst, das aus der Gruppe ausgewählt wird, die in Tabelle 1 aufgelistet ist.

**9.** Sondenverbindung nach einem der vorhergehenden Ansprüche, wobei die Indikatorkomponente einen Fluoreszenzfarbstoff umfasst, vorzugsweise einen Cy3-Farbstoff, und wobei die Verbinderkomponente $L_{TC\text{-}IC}$ eine quartäre Aminfunktion umfasst.

**10.** Verfahren zum Herstellen einer Sondenverbindung nach einem der Ansprüche 1 bis 9, das die folgenden Schritte umfasst:

Herstellen eines Übergangsmetallkomplexes, indem ein Salz eines Übergangsmetalles mit einem Ligandmolekül zur Reaktion gebracht wird;
Herstellen einer reaktiven Komponente, indem eine Testkomponente mit einem Histidinrest verbunden wird, wobei optional eine Verbinderkomponente verwendet wird, eine Farbstoffkomponente mit einer Histidinkomponente verbunden wird, wobei optional eine Verbinderkomponente verwendet wird, und die Testkomponente mit der Farbstoffkomponente verbunden wird, wobei optional eine Verbinderkomponente verwendet wird; und Verbinden der reaktiven Komponente mit dem Übergangsmetallkomplex durch die zwei Histidinreste.

**11.** Anordnung (Array) zum Nachweisen von Enzymen, umfassend eine Vielzahl von unterschiedlichen Sondenverbindungen nach einem beliebigen der Ansprüche 1 bis 9.

**12.** Verfahren zum Herstellen einer Anordnung nach Anspruch 11, umfassend die Herstellung einer Vielzahl von unterschiedlichen Sondenverbindungen nach dem Verfahren gemäß Anspruch 10, wobei eine verankernde Komponente mit dem Liganden des Übergangsmetallkomplexes der Sondenverbindung verbunden wird, und die Zusammenstellung der verschiedenen Sondenverbindungen in einer Anordnung (Array).

**13.** Mittel zum Isolieren, umfassend einen Nanopertikel und eine Sondenverbindung nach einem beliebigen der Ansprüche 1 bis 9.

**14.** Verfahren zum Herstellen eines Mittels zum Isolieren nach Anspruch 13, umfassend die Herstellung einer Sondenverbindung nach dem Verfahren gemäß Anspruch 10, wobei eine verankernde Komponente mit dem Liganden des Übergangsmetallkomplexes der Sondenverbindung verbunden wird, und die Sondenverbindung an einem Nonapartikel angebracht wird.

**15.** Verfahren zum Nachweisen von Enzymen, in dem die Sondenverbindung nach einem beliebigen der Ansprüche 1 bis 9 oder die Anordnung nach Anspruch 11 verwendet wird, wobei die Sondenverbindung oder die Anordnung in Kontakt mit einer Probe einer Analytlösung gebracht wird, die Enzyme umfasst.

**16.** Verfahren zum Isolieren von Enzymen, in dem das Mittel zum Isolieren nach Anspruch 13 verwendet wird.

## Revendications

**1.** Composé sonde pour détecter des interactions enzyme-substrat spécifiques comprenant un complexe de métal de transition, et un composant réactif de formule générale (X) :

$$\text{His-}L_{\text{His-TC}}\text{-TC-}L_{\text{TC-IC}}\text{-IC-}L_{\text{IC-His}}\text{-His} \qquad \text{formule (X)}$$

dans laquelle His représente un résidu d'histidine, TC représente un composant test, IC représente un composant indicateur, et chacun de $L_{His-TC}$, $L_{TC-IC}$, et $L_{IC-His}$ représente indépendamment des composants de liaison facultatifs, dans lequel le composant réactif est lié au complexe de métal de transition par les deux résidus d'histidine, dans lequel le composant test comprend un substrat, un métabolite, un pseudo-substrat ou un inhibiteur d'enzyme, et dans lequel le composant indicateur comprend un colorant.

**2.** Composé sonde selon la revendication 1, dans lequel le complexe de métal de transition comprend un atome de cobalt ou de cuivre.

**3.** Composé sonde selon l'une quelconque des revendications 1 ou 2, dans lequel le complexe de métal de transition comprend en outre un ligand polydentate, comprenant de préférence de l'acide nitrotriacétique.

**4.** Composé sonde selon l'une quelconque des revendications précédentes, dans lequel le complexe de métal de transition comprend un groupement d'acide nitrotriacétique de cobalt (II).

**5.** Composé sonde selon l'une quelconque des revendications précédentes, dans lequel le ligand polydentate du complexe de métal de transition comprend en outre un élément d'ancrage.

**6.** Composé sonde selon l'une quelconque des revendications précédentes, dans lequel le complexe de métal de transition est un complexe de cobalt (II) de $N_\alpha$,$N_\alpha$-bis-(carboxyméthyl)-L-lysine.

**7.** Composé sonde selon l'une quelconque des revendications précédentes, dans lequel le composant indicateur comprend un colorant fluorescent.

**8.** Composé sonde selon l'une quelconque des revendications précédentes, dans lequel le composant test comprend un substrat choisi dans le groupe indiqué dans le tableau 1.

**9.** Composé sonde selon l'une quelconque des revendications précédentes, dans lequel le composant indicateur comprend un colorant fluorescent, préférablement un colorant Cy3, et dans lequel le composant de liaison $L_{TC-IC}$ comprend une fonctionnalité d'amine quaternaire.

**10.** Procédé de préparation d'un composé sonde selon l'une quelconque des revendications 1 à 9, qui comprend les étapes consistant à:

Préparer un complexe de métal de transition par réaction d'un sel d'un métal de transition avec une molécule ligand,

Préparer un composant réactif en liant un composant test avec un résidu d'histidine, éventuellement en utilisant un composant de liaison, en liant un composant colorant à un composant d'histidine, éventuellement en utilisant un composant colorant, et en liant le composant test au composant colorant, éventuellement en utilisant un composant de liaison ; et
Lier le composant réactif au complexe de métal de transition via les deux résidus d'histidine.

**11.** Arrangement (Array) pour détecter des enzymes comprenant une pluralité de différents composés sonde selon l'une quelconque des revendications 1 à 9.

**12.** Procédé de fabrication d'un arrangement selon la revendication 11, comprenant la production d'une pluralité de différents composés sonde selon le procédé de la revendication 10, dans lequel un élément d'ancrage est lié au ligand du complexe de métal de transition du composé sonde, et l'arrangement des différents composés sonde dans un arrangement.

**13.** Moyen d'isolement comprenant une nanoparticule et un composé sonde selon l'une quelconque des revendications 1 à 9.

**14.** Procédé de production d'un moyen d'isolement selon la revendication 13, comprenant la production d'un composé sonde selon le procédé de la revendication 10, dans lequel le composant d'ancrage est lié au ligand du complexe de métal de transition du composé sonde, et le composé sonde est attaché à une nanoparticule.

**15.** Procédé de détection d'enzymes, qui utilise le composé sonde selon l'une quelconque des revendications 1 à 9, ou l'arrangement selon la revendication 11, dans lequel le composé sonde ou l'arrangement est amené en contact avec un échantillon d'une solution à analyser qui comprend des enzymes.

**16.** Procédé pour isoler des enzymes, qui utilise le moyen d'isolement selon la revendication 13.

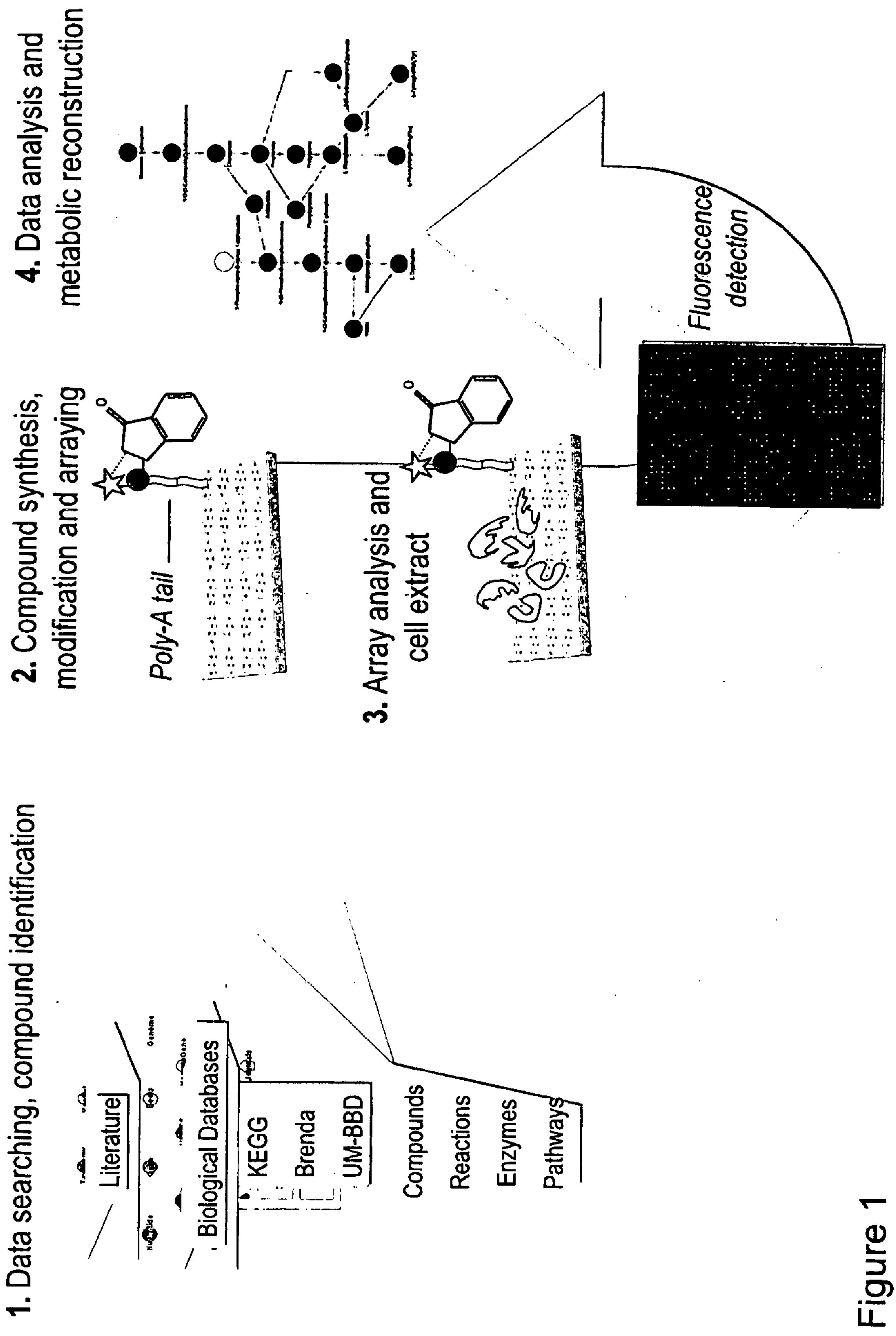
1. Data searching, compound identification
Literature
Biological Databases
KEGG
Brenda
UM-BBD
Compounds
Reactions
Enzymes
Pathways
2. Compound synthesis, modification and arraying
Poly-A tail
3. Array analysis and cell extract
Fluorescence detection
4. Data analysis and metabolic reconstruction

Figure 1

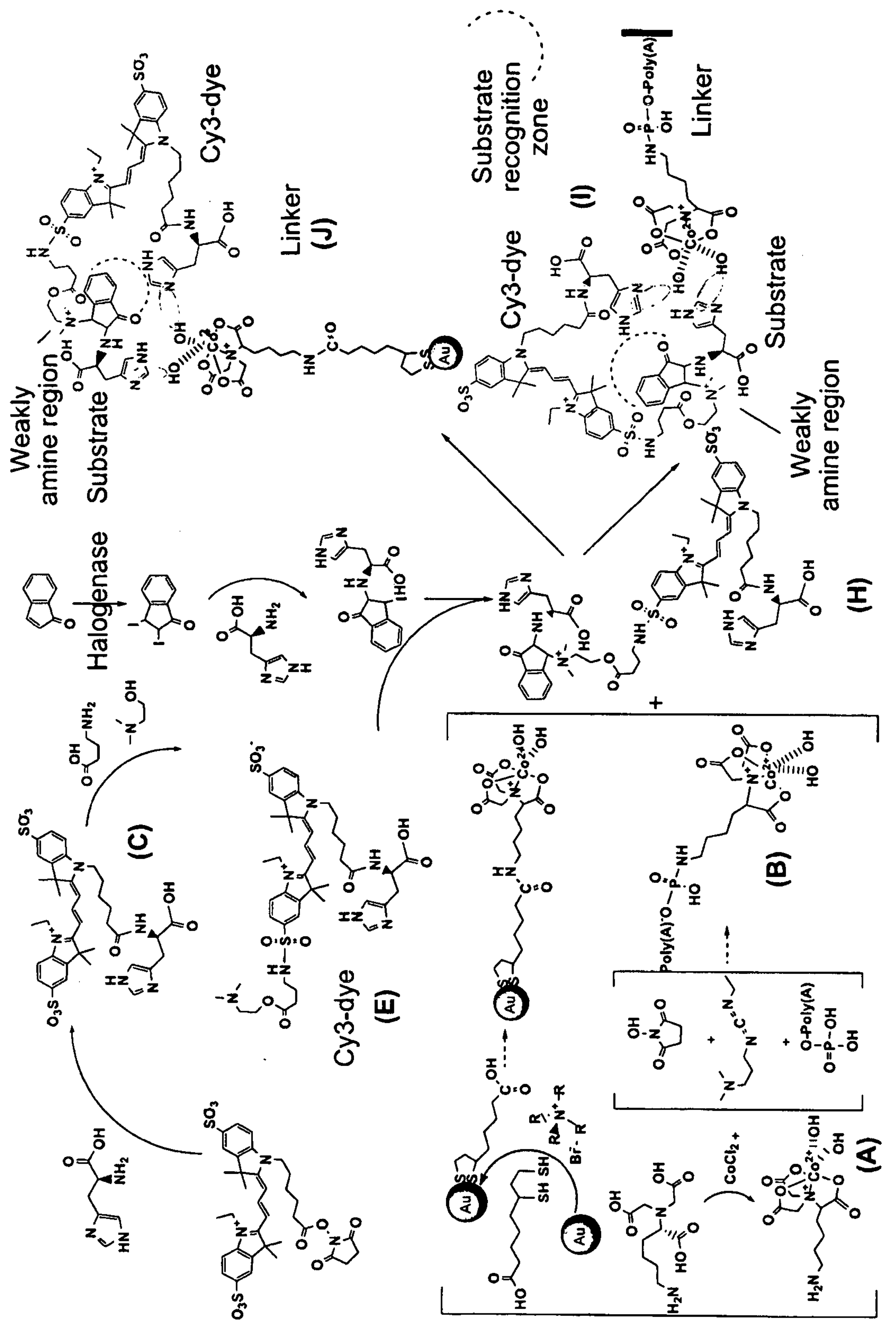
Weakly amine region
Substrate
Linker (J)
Cy3-dye
Substrate recognition zone
(I)
Linker
Cy3-dye
Substrate
Weakly amine region
Halogenase
(C)
(H)
Cy3-dye (E)
(B)
CoCl2 +
(A)

Figure 2

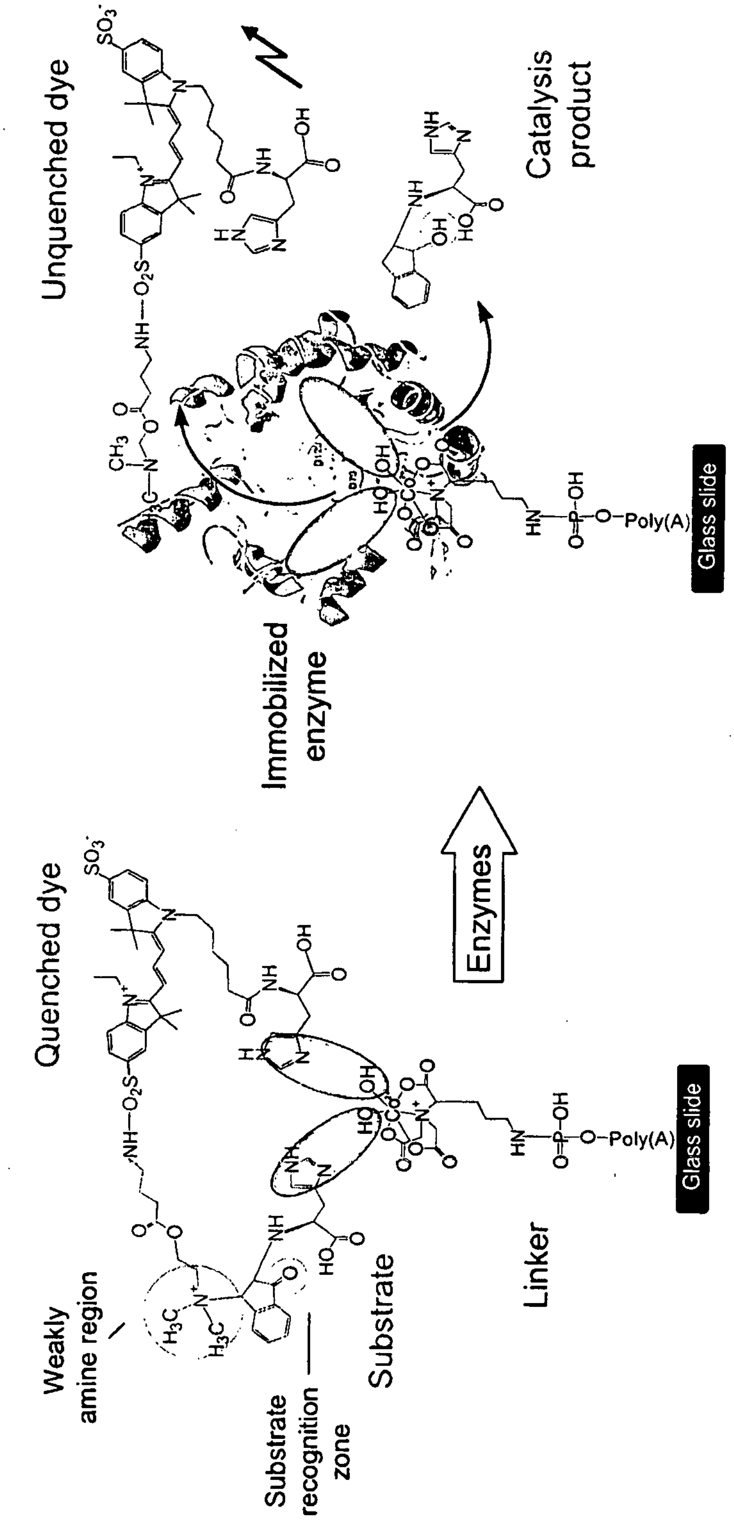
Quenched dye
Weakly amine region
Substrate recognition zone
Substrate
Linker
Glass slide
Enzymes
Immobilized enzyme
Glass slide
Unquenched dye
Catalysis product

Figure 3

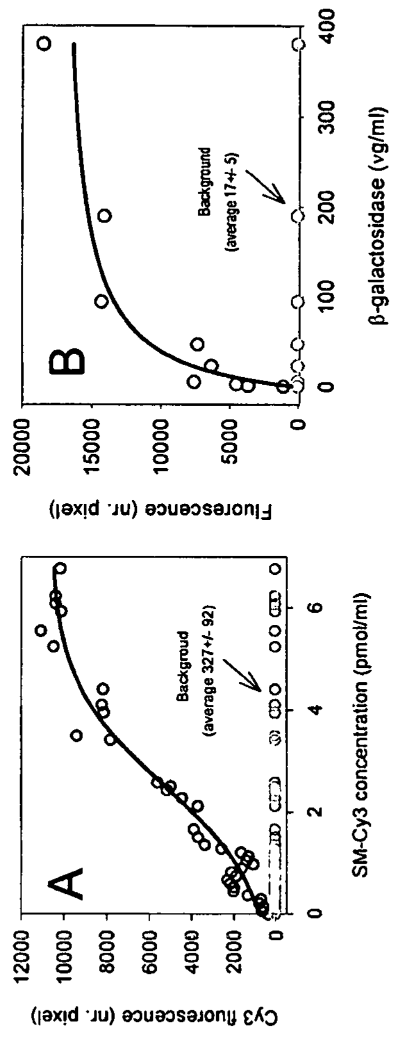
A
Cy3 fluorescence (nr. pixel)
12000
10000
8000
6000
4000
2000
0
0
2
4
6
SM-Cy3 concentration (pmol/ml)
Backgroud
(average 327+/- 92)
B
Fluorescence (nr. pixel)
20000
15000
10000
5000
0
0
100
200
300
400
β-galactosidase (vg/ml)
Background
(average 17+/- 5)

Figure 4

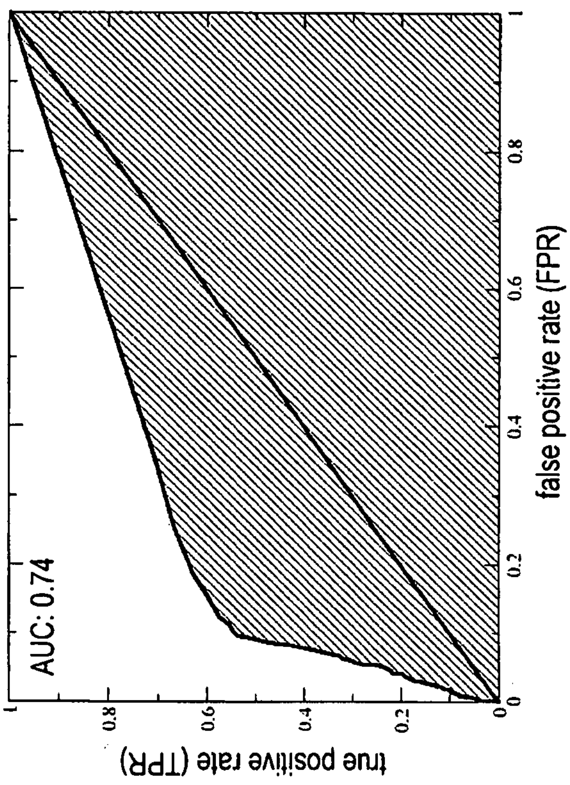
AUC: 0.74
true positive rate (TPR)
false positive rate (FPR)
0
0.2
0.4
0.6
0.8
1

Figure 5

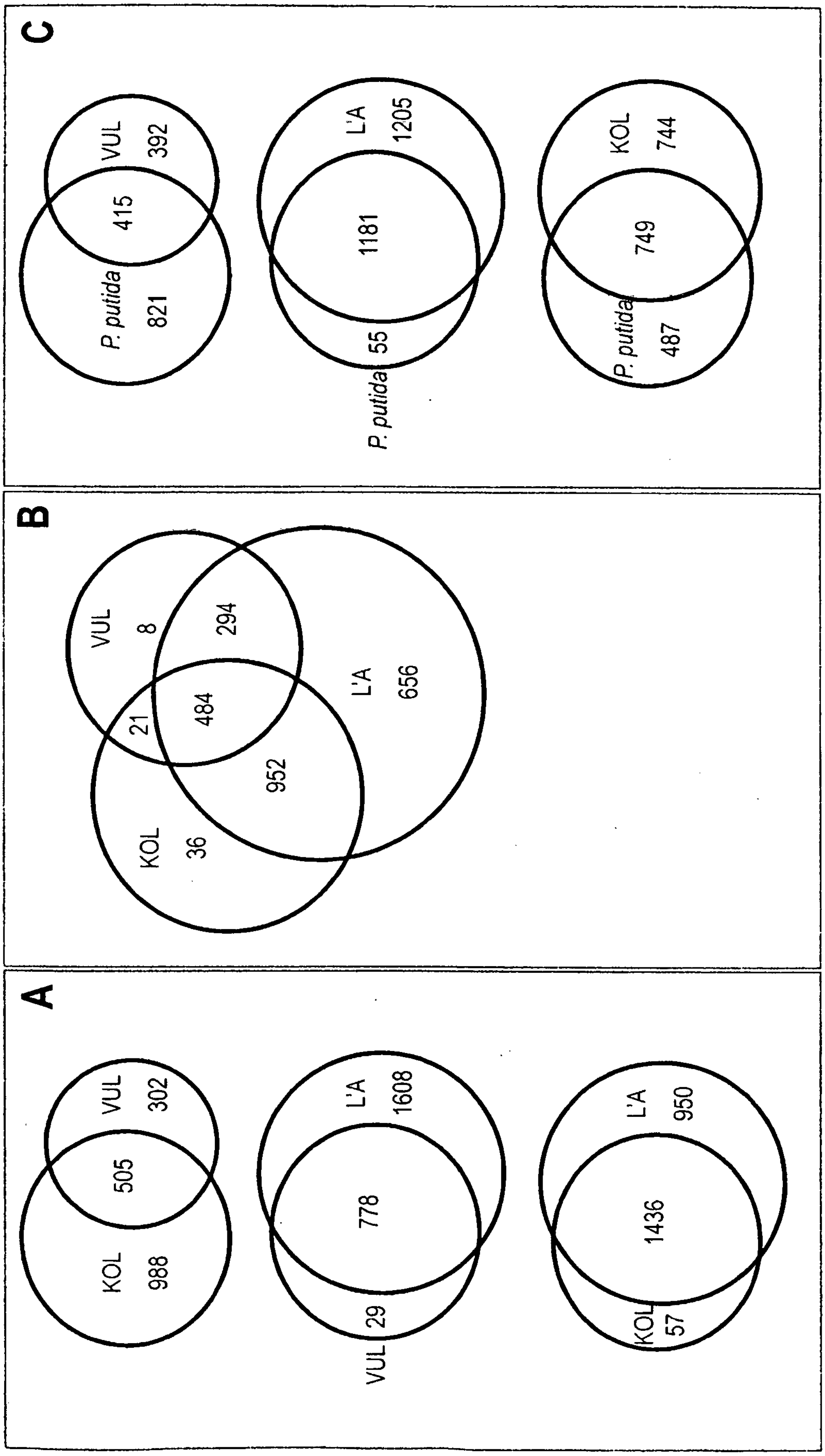
A
KOL
988
505
VUL
302
VUL
29
778
L'A
1608
KOL
57
1436
L'A
950
B
KOL
36
21
484
952
VUL
8
294
L'A
656
C
P. putida
821
415
VUL
392
P. putida
55
1181
L'A
1205
P. putida
487
749
KOL
744

Figure 6

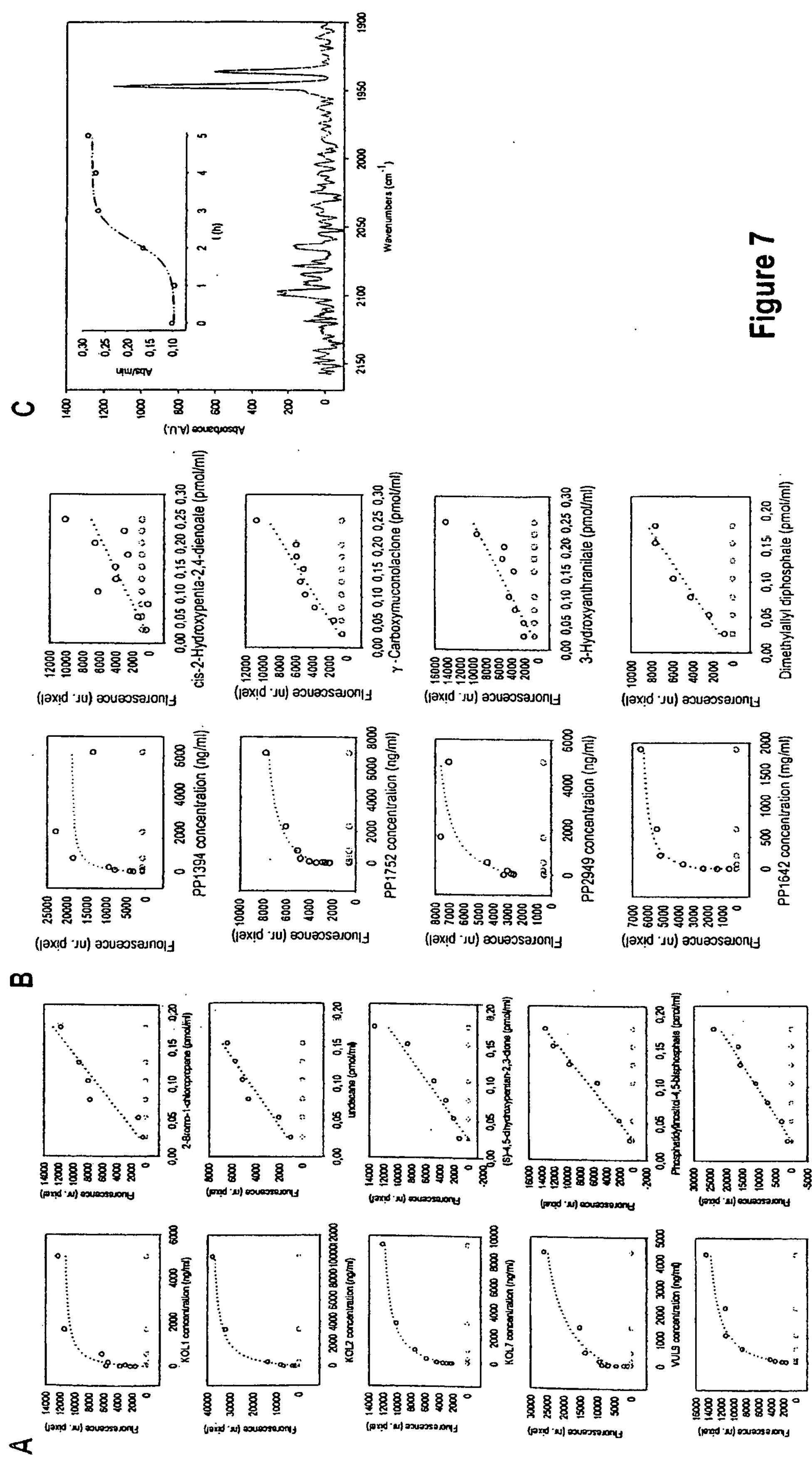
A
B
C
KOL1 concentration (ng/ml)
KOL2 concentration (ng/ml)
KOL7 concentration (ng/ml)
VUL9 concentration (ng/ml)
2-Bromo-1-chloropropane (pmol/ml)
undecane (pmol/ml)
methyl viologen (pmol/ml)
Fluorescence (nr. pixel)
PP1394 concentration (ng/ml)
PP1752 concentration (ng/ml)
PP2949 concentration (ng/ml)
PP1642 concentration (mg/ml)
cis-2-Hydroxypenta-2,4-dienoate (pmol/ml)
γ-Carboxymuconolactone (pmol/ml)
3-Hydroxyanthranilate (pmol/ml)
Dimethylallyl diphosphate (pmol/ml)
Absorbance (A.U.)
Abs/min
t (h)
Wavenumbers (cm-1)

Figure 7

Schematic representation of the synthetic method 1

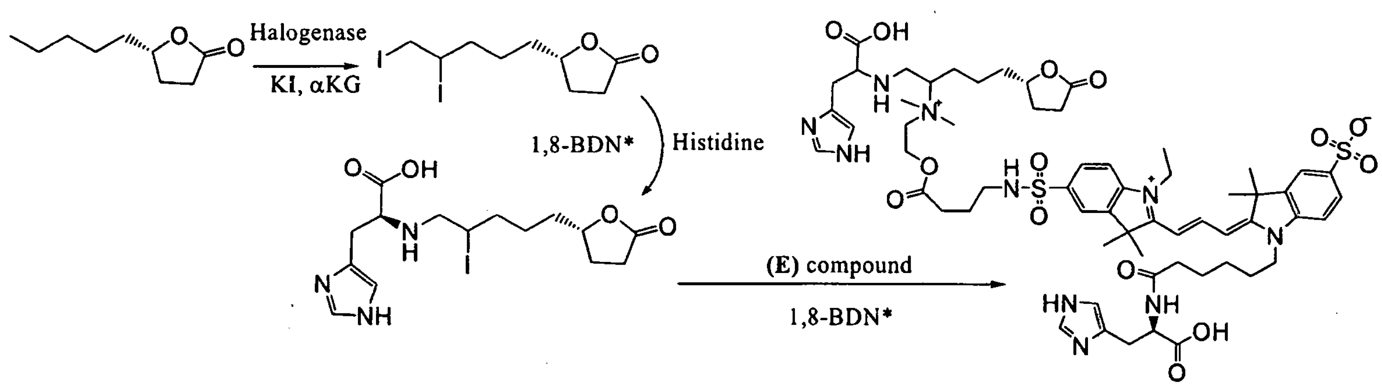


Schematic representation of the synthetic method 2

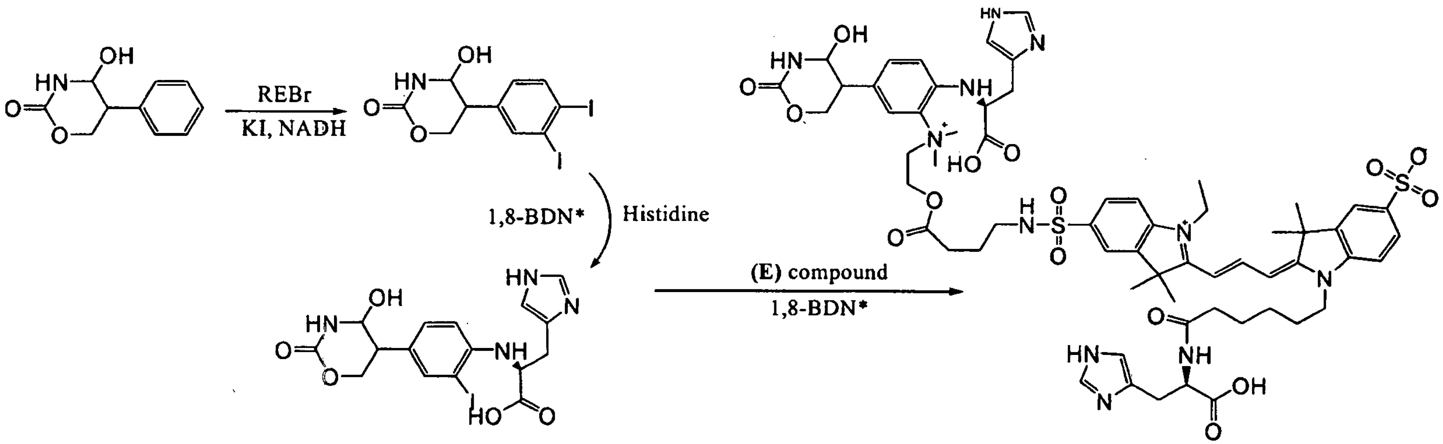


*Figure 8*

Schematic representation of the synthetic method 3

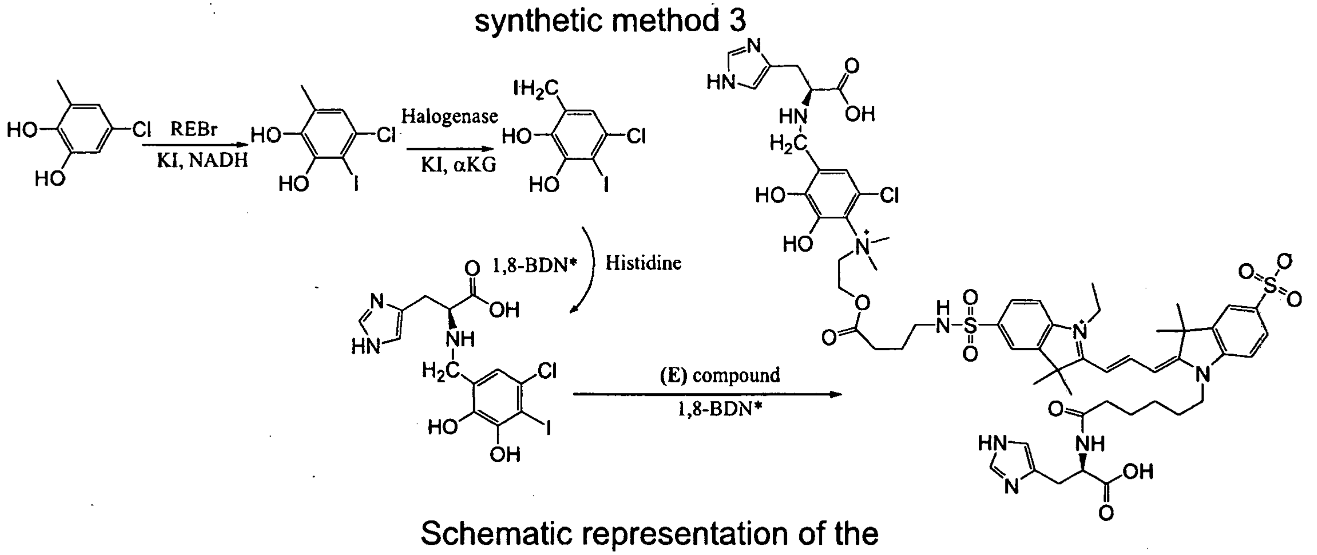


Schematic representation of the synthetic method 4

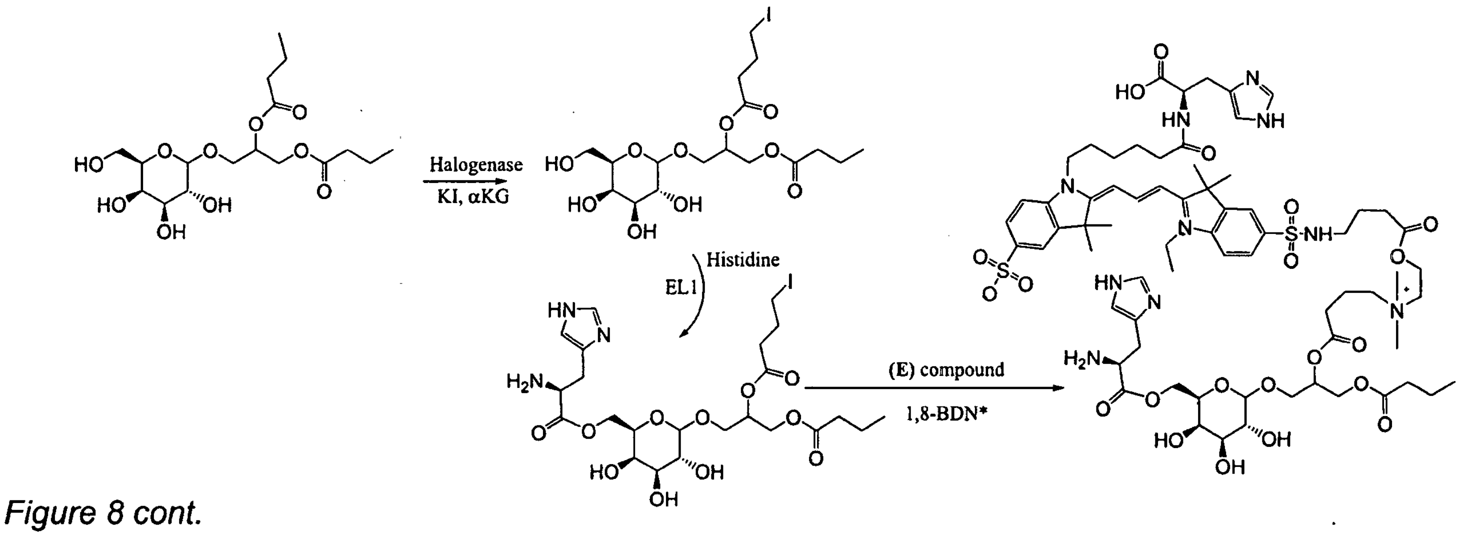


*Figure 8 cont.*

Schematic representation of the synthetic method 5

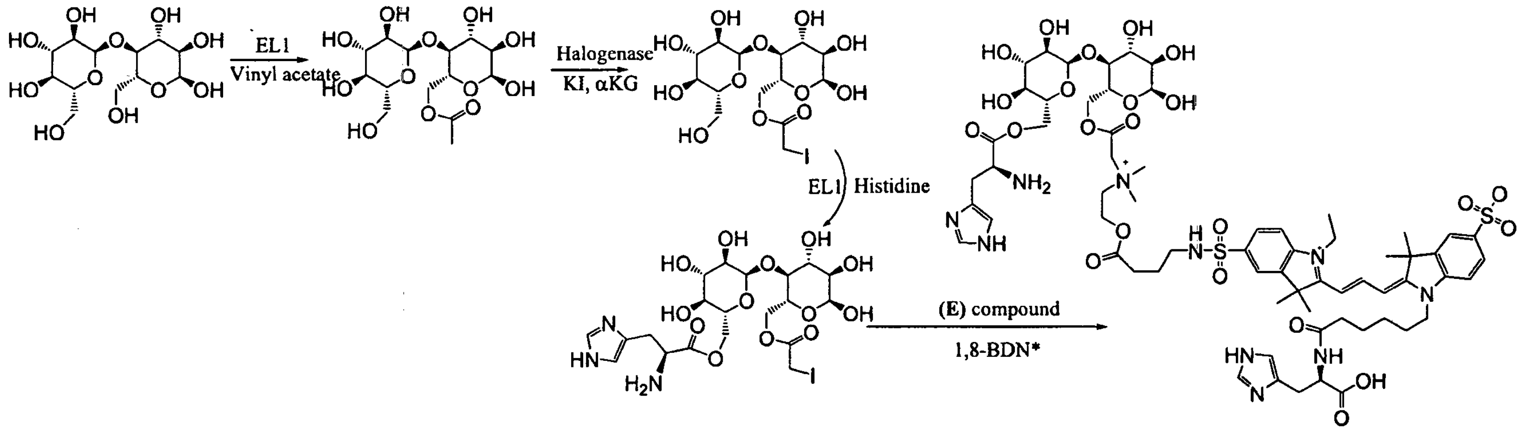


Schematic representation of the synthetic method 6

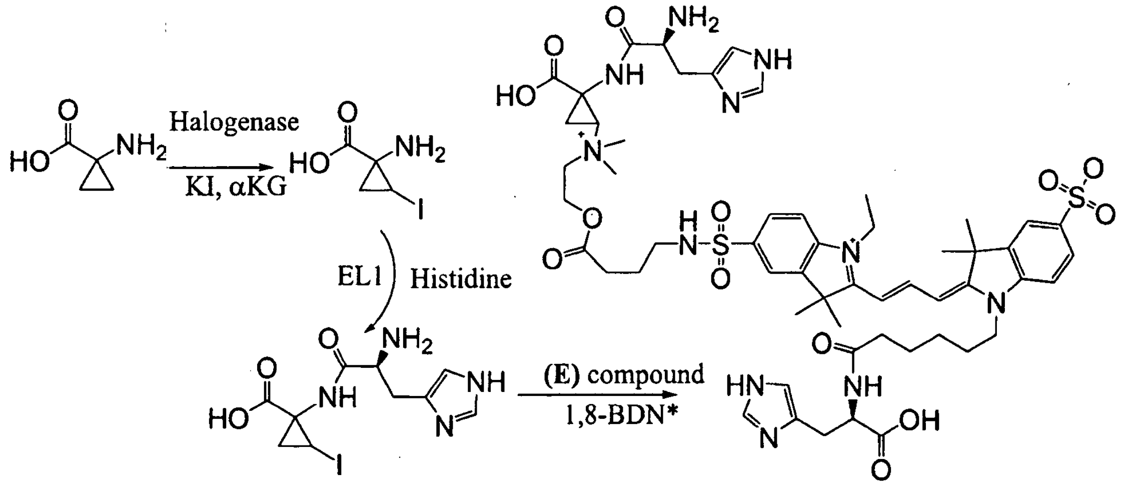


*Figure 8 cont.*

## Schematic representation of the synthetic method 7

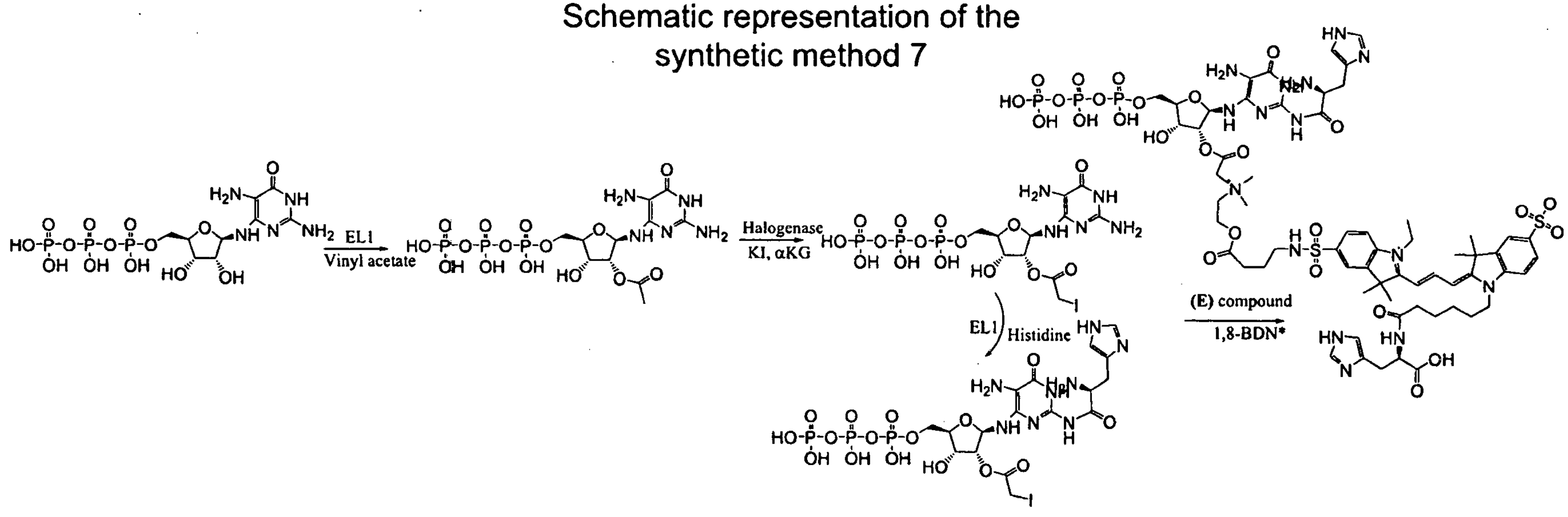


## Schematic representation of the synthetic method 8

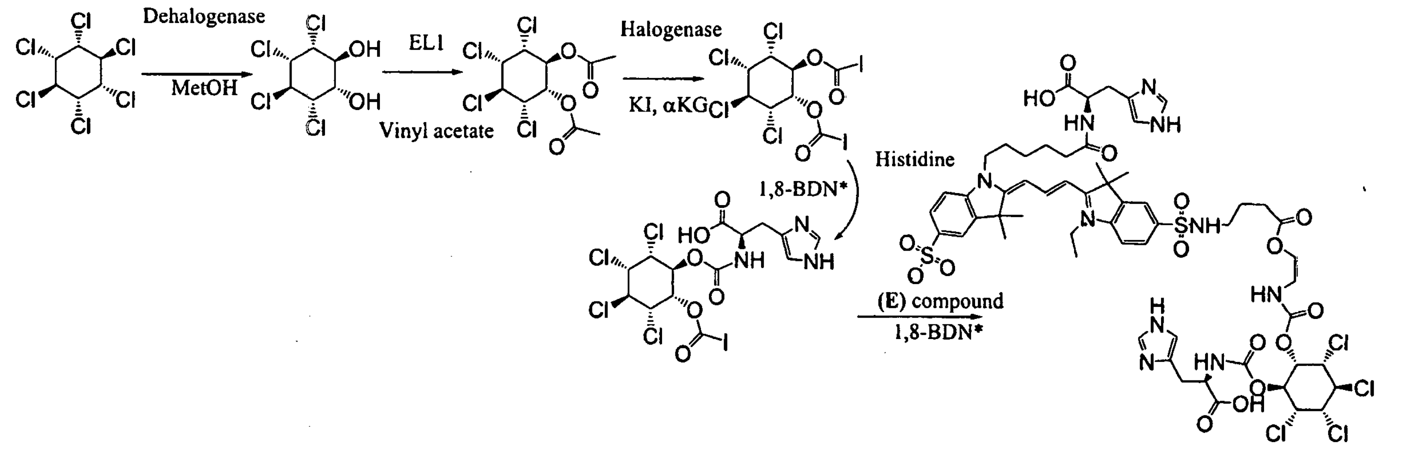


*Figure 8 cont.*

Schematic representation of the synthetic method 9

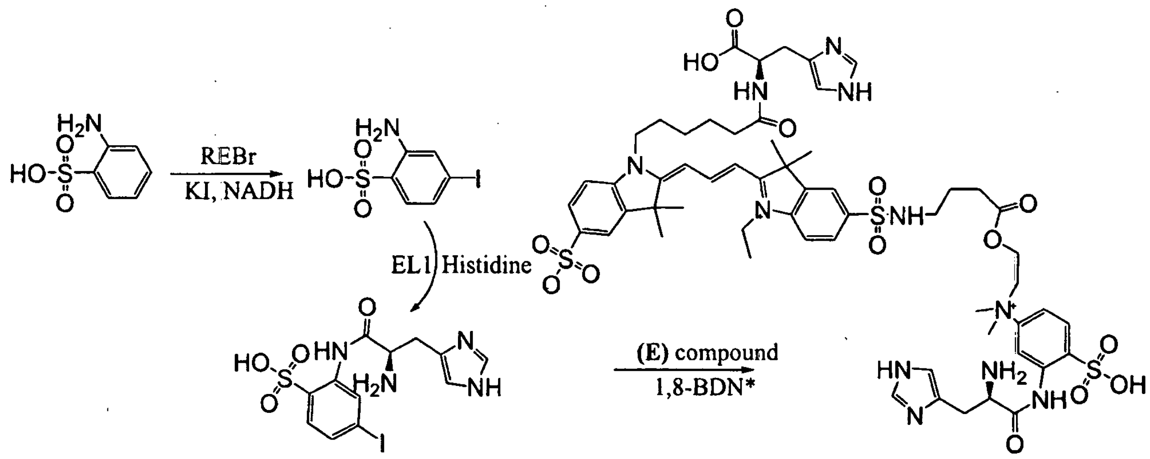


Schematic representation of the synthetic method 10

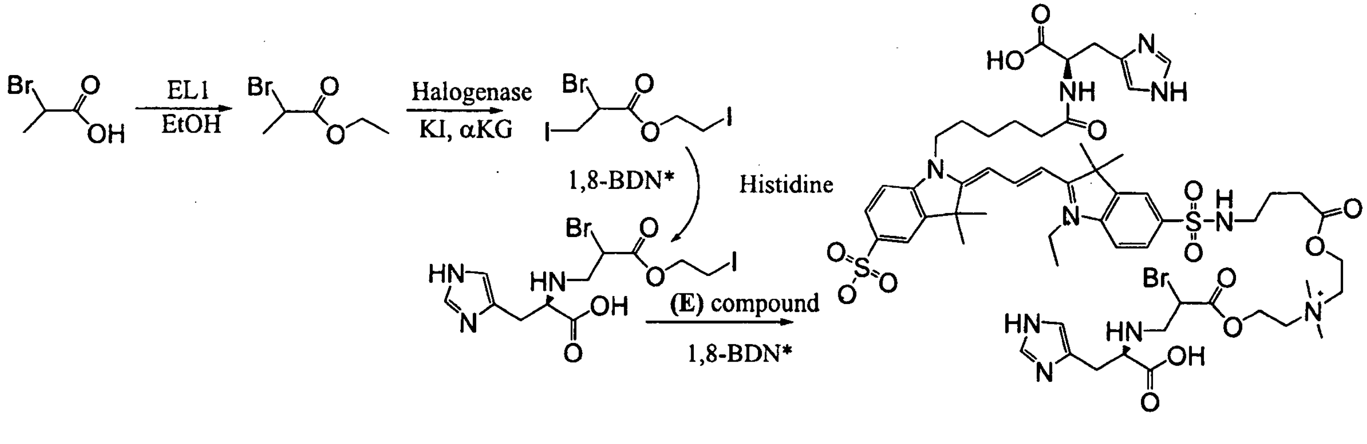


*Figure 8 cont.*

Schematic representation of the synthetic method 11

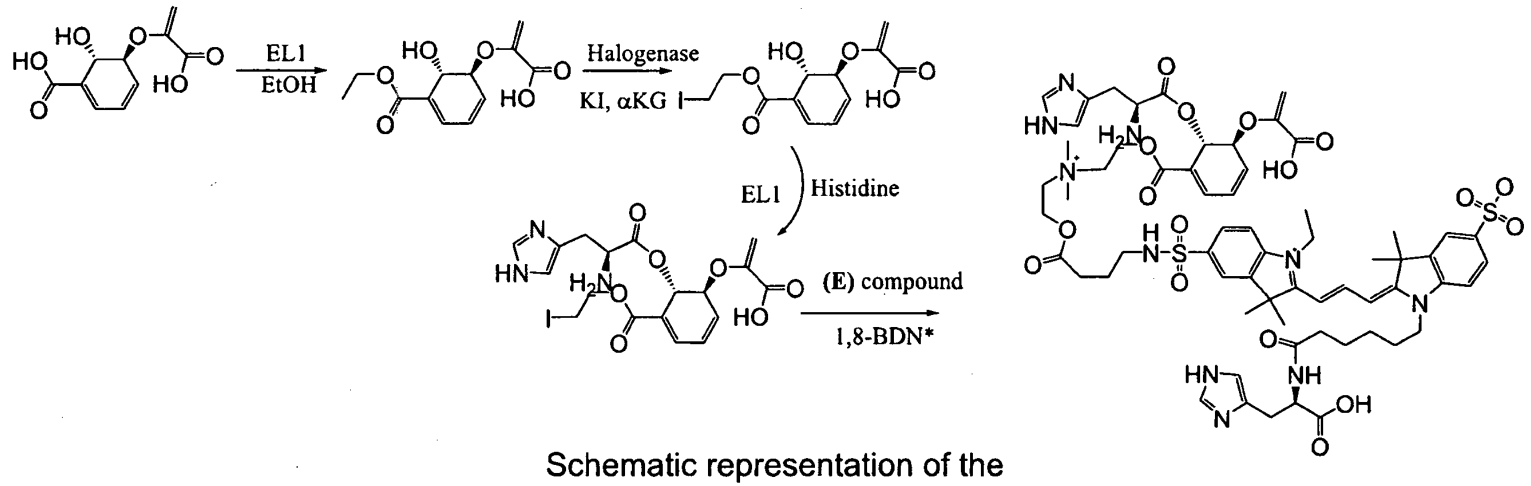


Schematic representation of the synthetic method 12

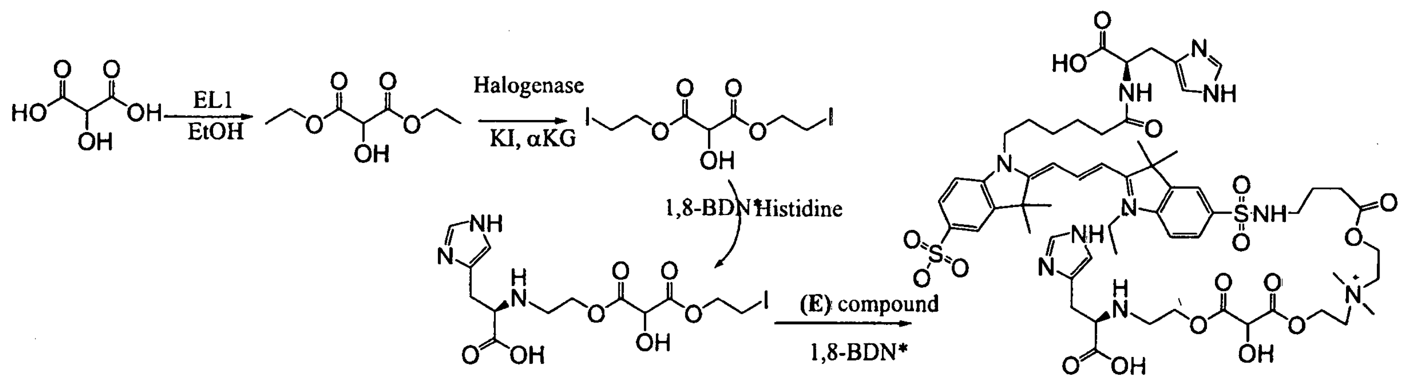


*Figure 8 cont.*

Schematic representation of the synthetic method 13

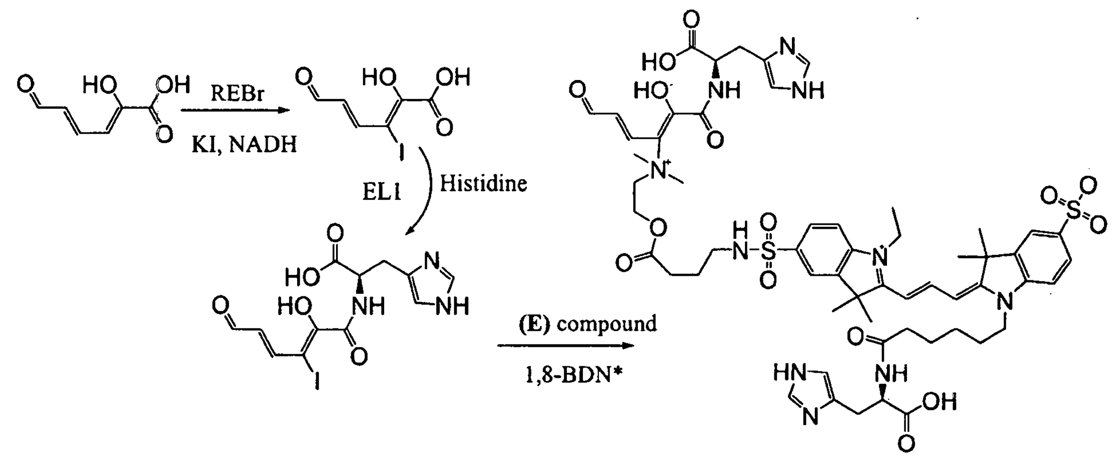


Schematic representation of the synthetic method 14

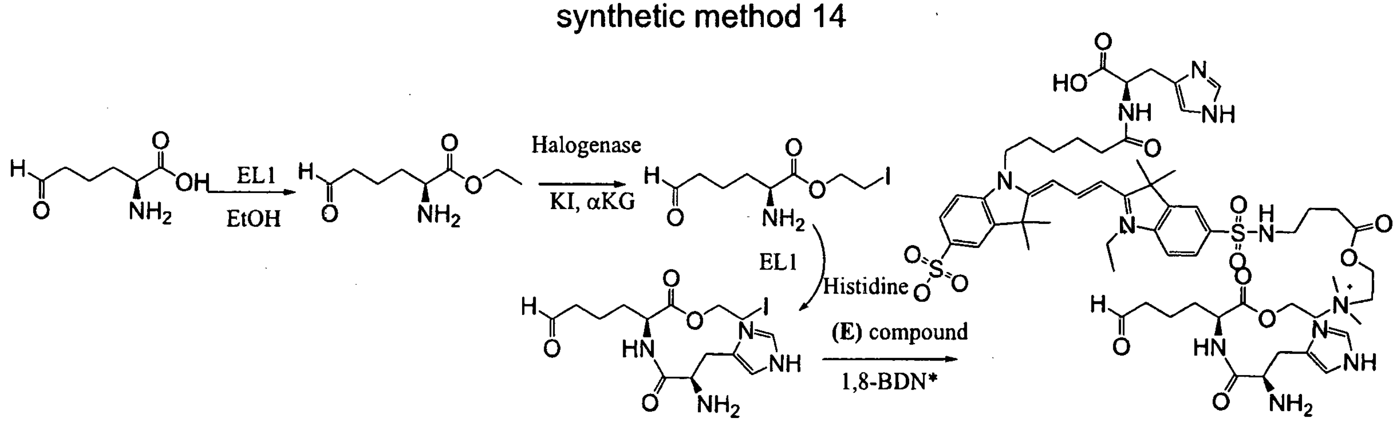


*Figure 8 cont.*

Schematic representation of the synthetic method 15

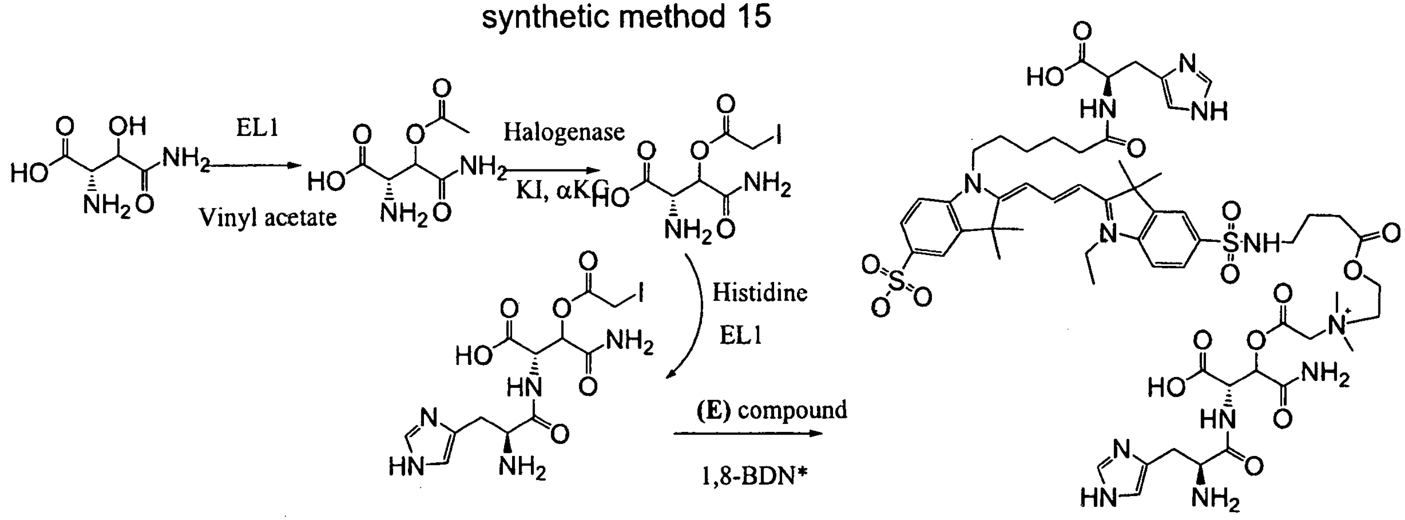


Schematic representation of the synthetic method 16

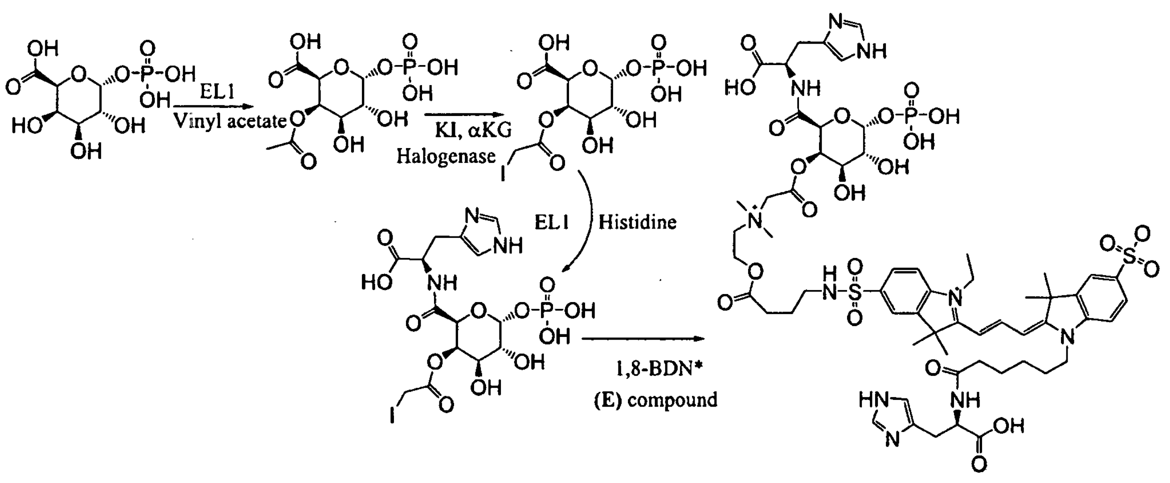


*Figure 8 cont.*

Schematic representation of the synthetic method 17

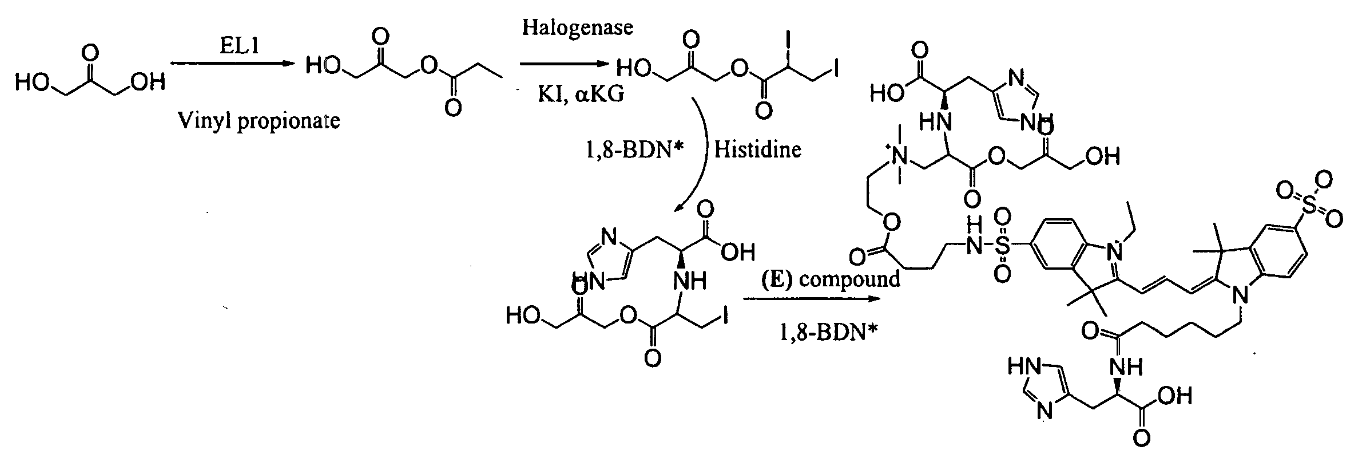


Schematic representation of the synthetic method 18

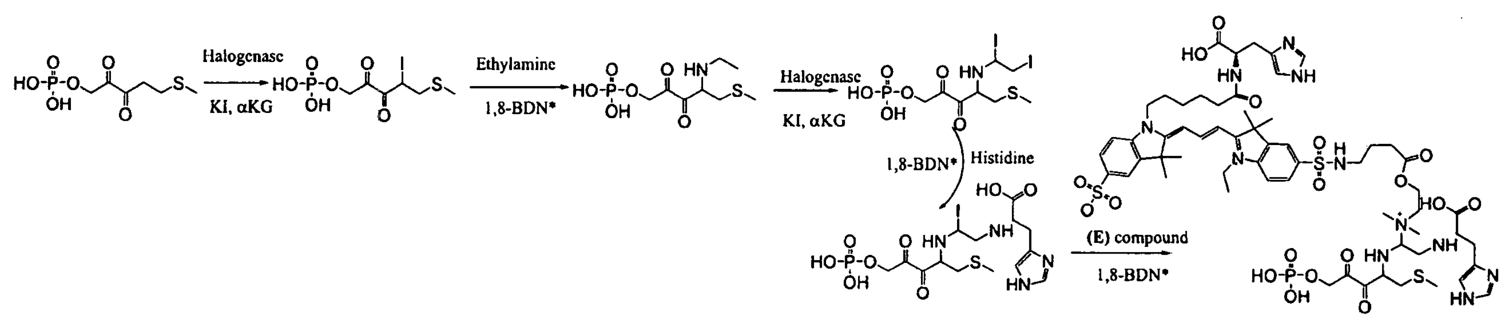


*Figure 8 cont.*

Schematic representation of the synthetic method 19

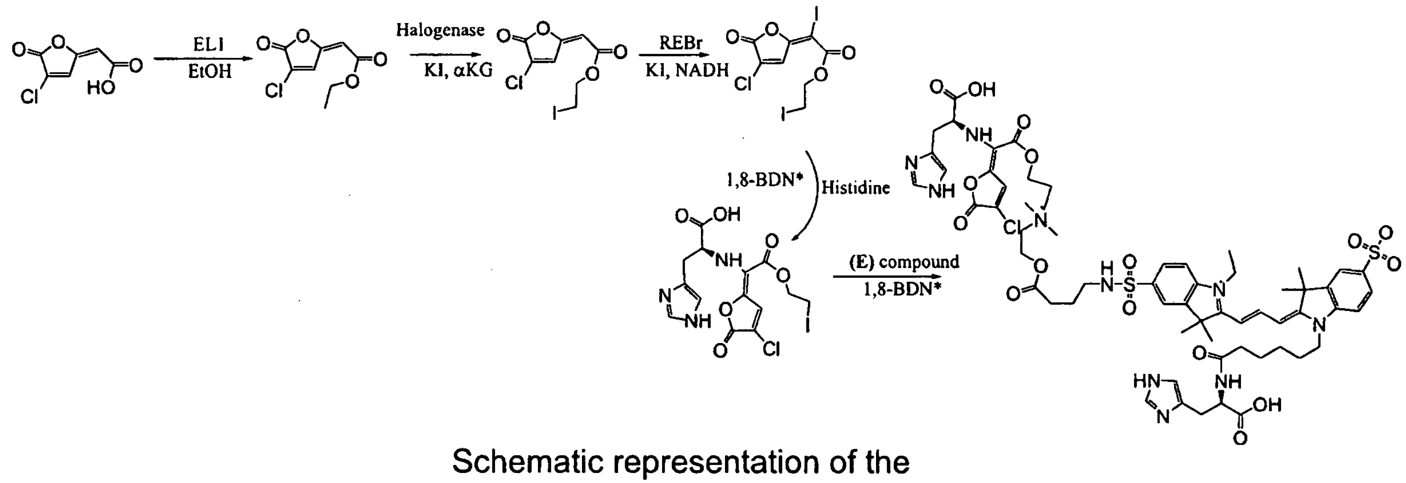


Schematic representation of the synthetic method 20

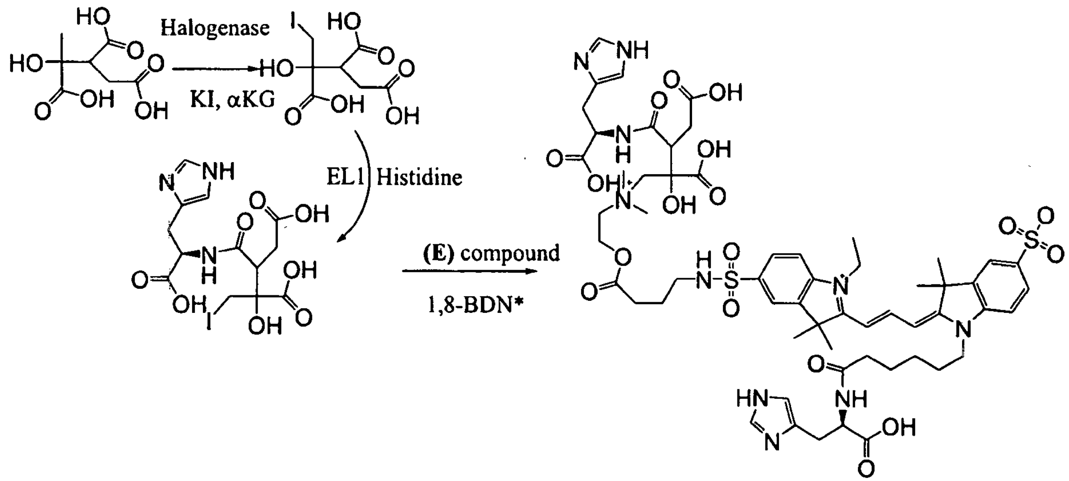


*Figure 8 cont.*

Schematic representation of the synthetic method 21

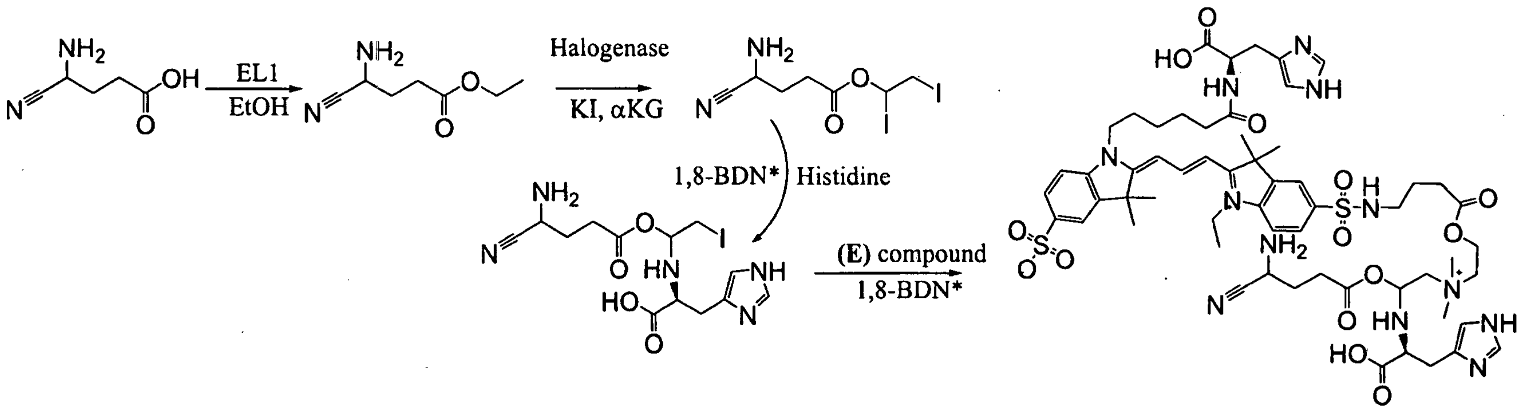


Schematic representation of the synthetic method 22

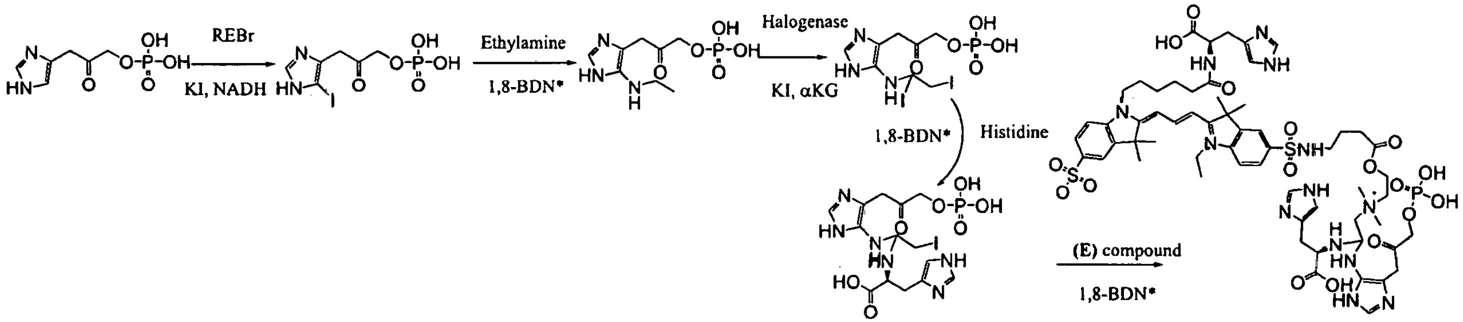


*Figure 8 cont.*

Schematic representation of the synthetic method 23

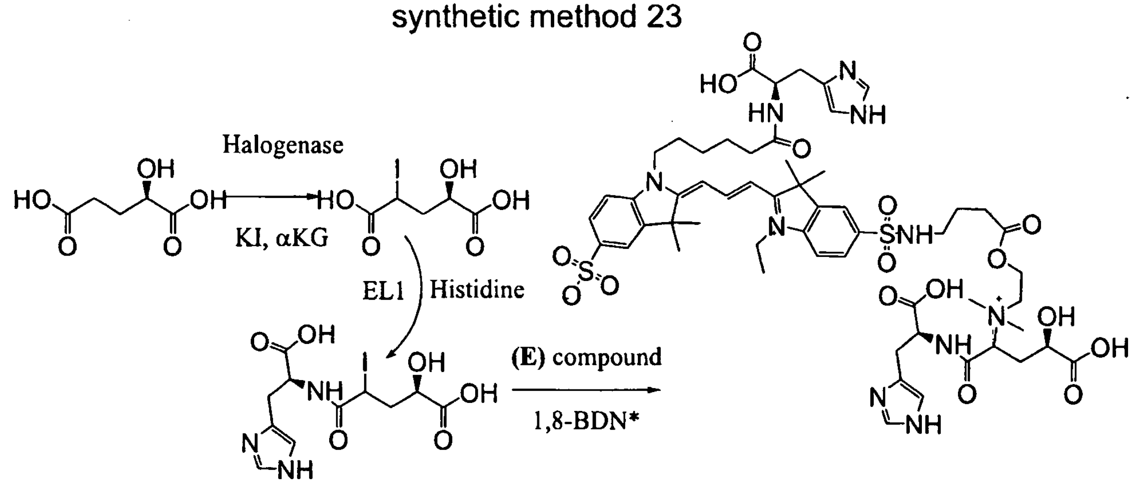


Schematic representation of the synthetic method 24

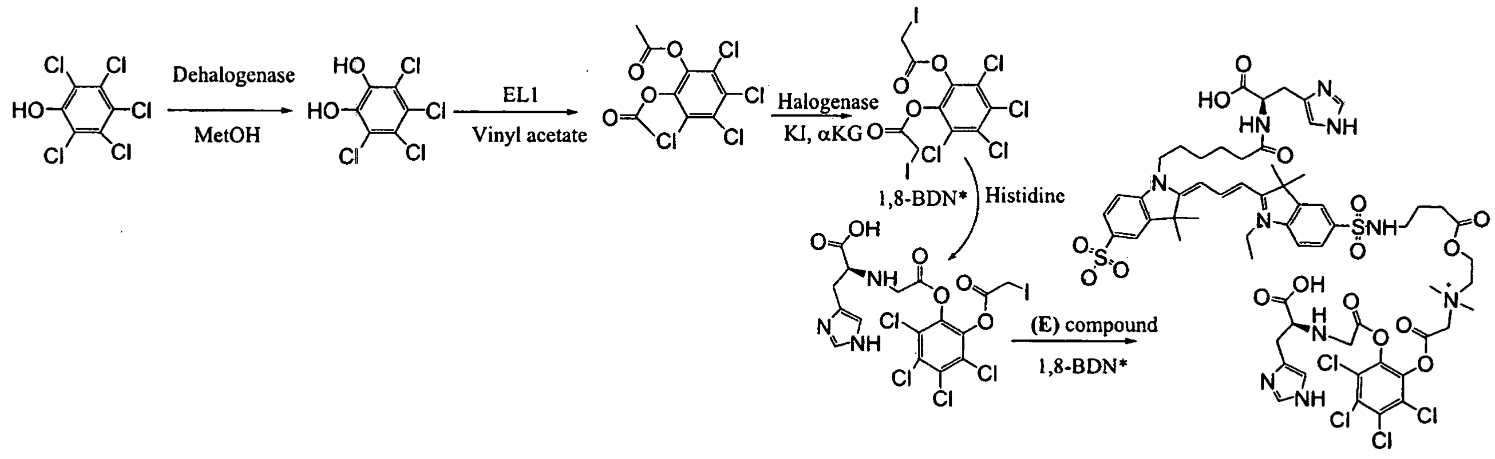


*Figure 8 cont.*

Schematic representation of the synthetic method 25

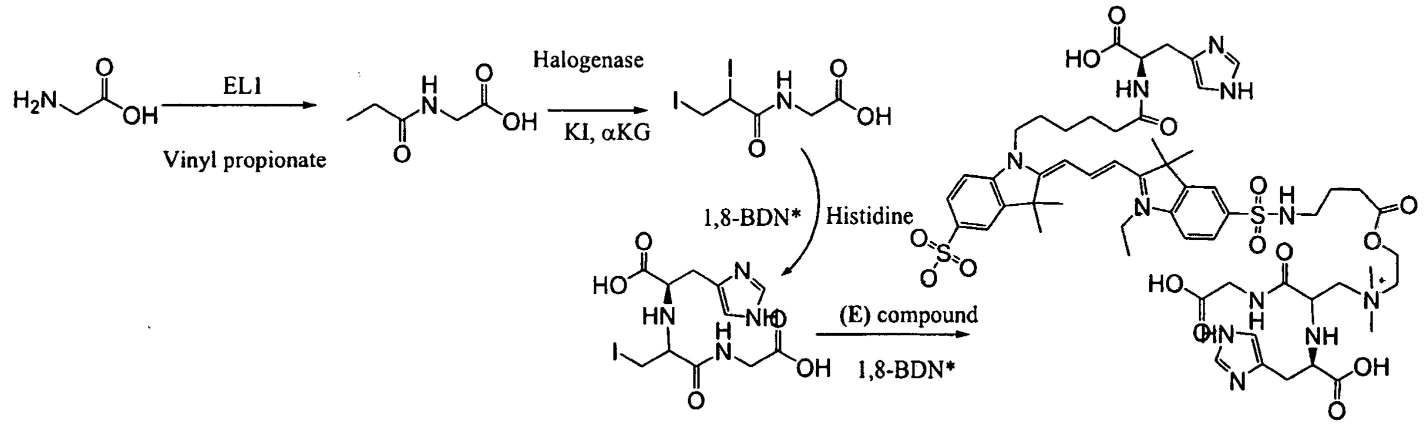


Schematic representation of the synthetic method 26

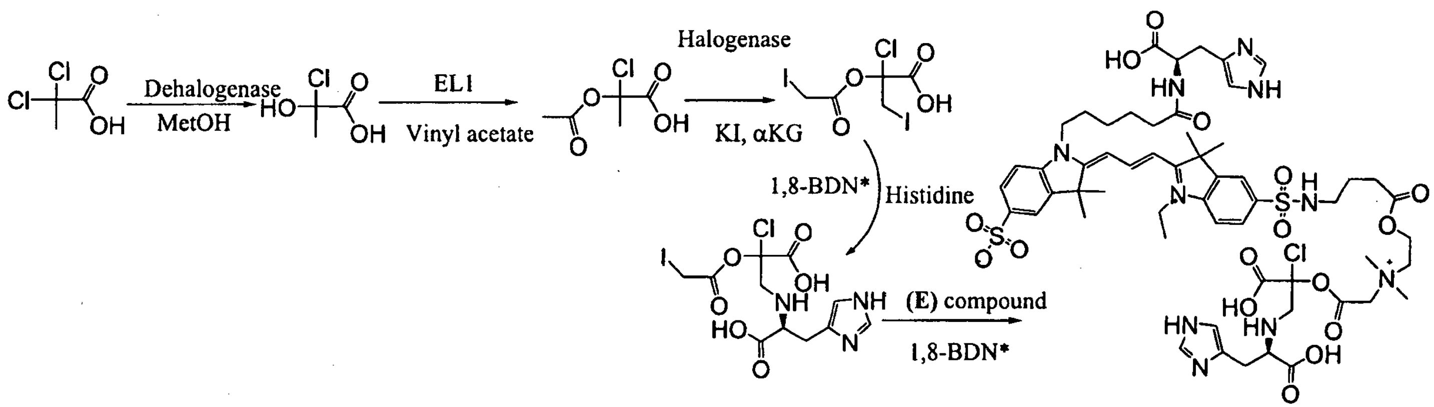


*Figure 8 cont.*

**Example method 1:**
Molecule 1 according to Table 1

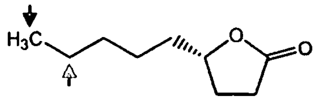

**Example method 2:**
Molecule 7 according to Table 1

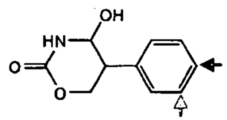

**Example method 3:**
Molecule 8 according to Table 1

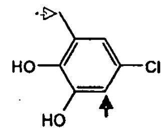

**Example method 4:**
Molecule 36 according to Table 1

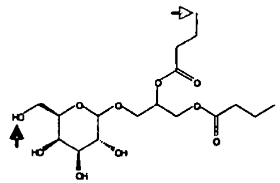

**Example method 5:**
Molecule 132 according to Table 1

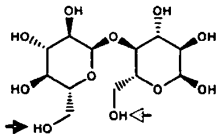

**Example method 6:**
Molecule 154 according to Table 1

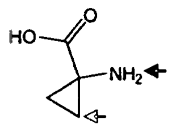

**Example method 7:**
Molecule 196 according to Table 1

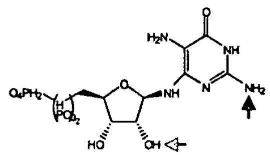

**Example method 8:**
Molecule 1692 according to Table 1

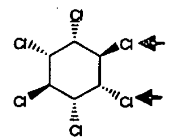

**Example method 9:**
Molecule 306 according to Table 1

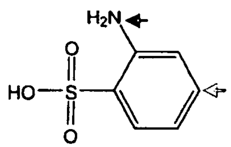

**Example method 10:**
Molecule 327 according to Table 1

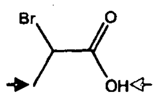

**Example method 11:**

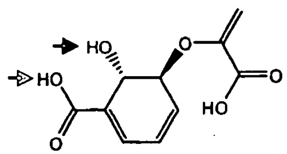

**Example method 12:**
Molecule 397 according to Table 1

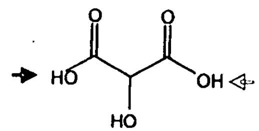

**Example method 13:**
Molecule 398 according to Table 1

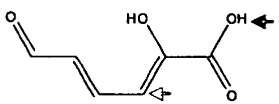

**Example method 14:**
Molecule 860 according to Table 1

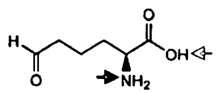

**Example method 15:**
Molecule 543 according to Table 1

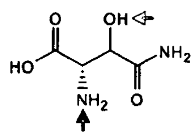

**Example method 16:**
Molecule 1179 according to Table 1

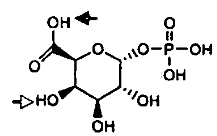

*Figure 9*

**Example method 17:**
Molecule 1212 according to Table 1

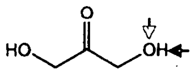

**Example method 18:**
Molecule 417 according to Table 1

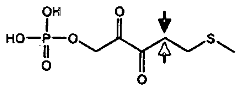

**Example method 19:**
Molecule 1332 according to Table 1

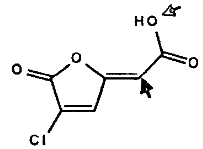

**Example method 20:**
Molecule 1790 according to Table 1

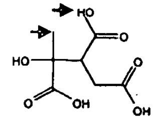

**Example method 21:**
Molecule 1409 according to Table 1

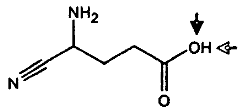

**Example method 22:**
Molecule 1614 according to Table 1

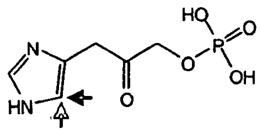

**Example method 23:**
Molecule 394 according to Table 1

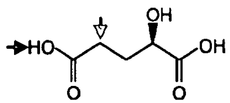

**Example method 24:**
Molecule 1997 according to Table 1

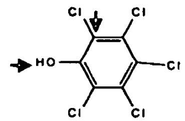

**Example method 25:**
Molecule 1481 according to Table 1

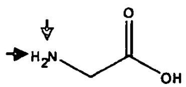

**Example method 26:**
Molecule 242 according to Table 1

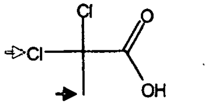

**Example method 27:**
DNA oligonucleotide

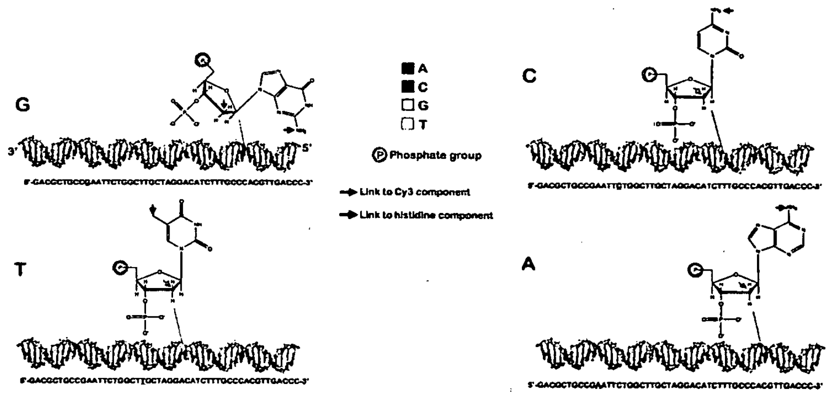


*Figure 9 cont.*

## Example method 28:
DNA oligonucleotide

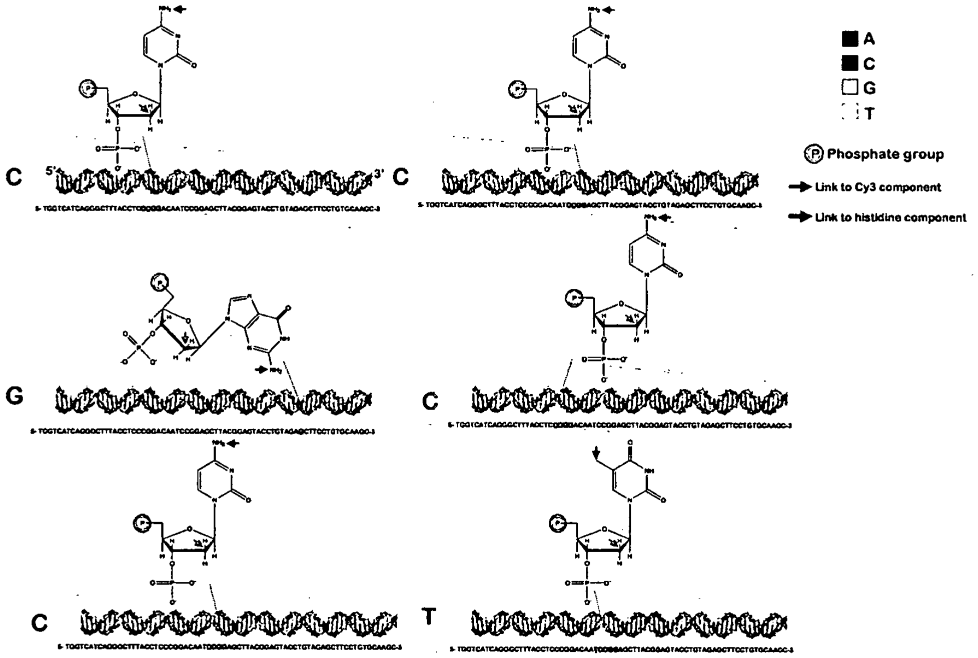


## Example method 29:
DNA molecule

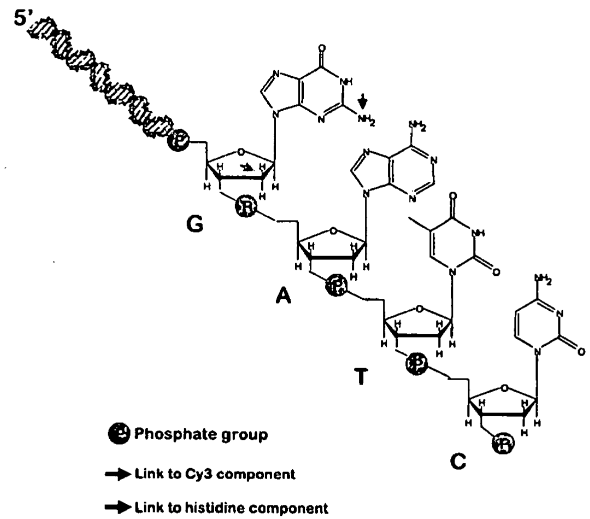


## Example method 30:
Protein substrate

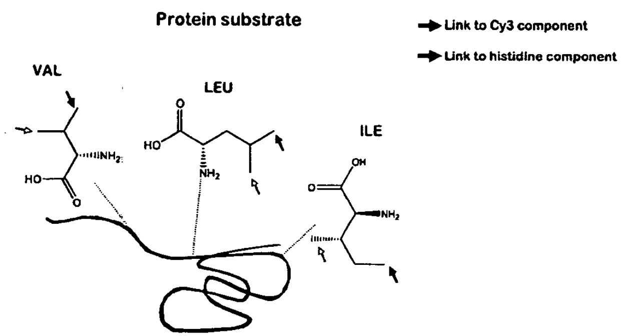


*Figure 9 cont.*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2004093478 B
- EP 1669760 A
- EP 09003977 W


### Non-patent literature cited in the description


- **ABAD et al.** *J. Am. Chem. Soc.,* 2005, vol. 127, 5689
- **U.K. LAEMMLI.** *Nature,* 1970, vol. 227, 680
- **F. STEPHEN et al.** *Nucleic Acids Res,* 1997, vol. 25, 3389
- **FERRER et al.** *Tetrahedron,* 2000, vol. 56, 4053-4061
- **PLOU et al.** *Journal of Biotechnology,* 2002, vol. 96, 55-66
- **CALLAHAN et al.** *Bioorganic and Medical Chemistry Letters,* 2006, vol. 16, 3802
- *Bull. Chem. Soc. Jpn.,* 1981, vol. 54, 1879
- **MÉDEBIELLE.** *Tetrahedron Letters,* 1996, vol. 37, 5119-5122
- **J. M. ABAD et al.** *J Am Chem Soc,* 2005, vol. 127, 5689
- **D. REYES-DUARTE et al.** *Angew Chem Int Ed Engl,* 2005, vol. 44, 7553
- **C.R. CANTOR ; I. TINOCO.** *J. Mol. Biol.,* 1965, vol. 13, 65
- **J. SOKOLOWSKA-GAJDA ; H.S. FREEMAN.** *Dyes and Pigm,* 1990, vol. 14, 35
- **Z. WAI et al.** *The proceeding of the 3rd International Conference on Functional Molecules,* 2005, 167
- **C. TSOPELAS et al.** *J Nucl Med,* 2002, vol. 43, 1377
- *Angewandte Chemie International Edition,* 2005, vol. 44, 7553-7557
- **F. MAZZETTI ; R. M. LEMMON.** *J Org Chem,* 1957, vol. 22, 228
- **FERRER et al.** *Mol. Microbiol.,* 2005, vol. 53, 167-182
- **ABAD et al.** *J Am Chem Soc,* 2005, vol. 127, 5689
- **M. FERRER et al.** *Nature,* 2007, vol. 445, 91
- **T. FAWCETT.** *Pattern Recogn Lett,* 2006, vol. 27, 861
- **DE LACEY et al.** *J Biol Anorg Chem,* 2004, vol. 9, 636
- **J.C. FONTECILLA-CAMPS et al.** *Chem Rev,* 2007, vol. 107, 4273
- **A. DE LACEY et al.** *Biochem Biophys Acta,* 1985, vol. 832, 69
- **V. M. FERNANDEZ et al.** *Biochim Biophys Acta,* 1985, vol. 832, 69
- **P.M. VIGNAIS ; B. BILLOUD.** *Chem Rev,* 2007, vol. 107, 4206

SEQUENCE LISTING

<110> Helmholtz-zentrum für Infektionsforschung GmbH

<120> PROBE COMPOUND FOR DETECTING AND ISOLATING ENNZYMES AND MEANS AND METHODS USING THE SAME

<130> 18977-PCT

<140> new PCT application based on EP 09 003 977.7<141> 2010-03-19

<160> 59

<170> PatentIn version 3.3

<210> 1
<211> 20
<212> DNA
<213> artificial

<220>
<223> PCR amplification primer

<400> 1
agagtttgat cmtggctcag 20

<210> 2
<211> 19
<212> DNA
<213> artificial

<220>
<223> PCR amplification primer

<400> 2
tccgtgccag cagccgccg 19

<210> 3
<211> 20
<212> DNA
<213> artificial

<220>
<223> PCR amplification primer

<400> 3
cggytacctt gttacgactt 20

<210> 4
<211> 22
<212> DNA
<213> artificial

<220>
<223> PCR amplification primer

<400> 4
gacgttgtaa aacgacggcc ag 22

<210> 5
<211> 21
<212> DNA
<213> artificial

<220>
<223> PCR amplification primer

<400> 5
gaggaaacag ctatgaccat g 21

<210> 6
<211> 11
<212> PRT
<213> artificial

<220>
<223> target (no.914) peptide available from sigma-Aldrich-Fluka

<400> 6

```
Arg Pro Lys Pro Gln Gln Phe Phe Gly Leu Met
1               5                   10
```

<210> 7
<211> 49
<212> DNA
<213> artificial

<220>
<223> target (no. 1256) DNA resolvase substrate available from Sigma Genosys

<400> 7
gacgctgccg aattctggct tgctaggaca tctttgccca cgttgaccc 49

<210> 8
<211> 35
<212> DNA
<213> artificial

<220>
<223> target (no. 1261) DNA substrate available from Sigma Genosys

<400> 8
taagctccgg attgtccggg aggtaaagcc ctgat 35

<210> 9
<211> 25
<212> DNA
<213> artificial

<220>
<223> target (no. 1262) DNA substrate available from sigma Genosys

<400> 9

cacaggaagc tctacaggta ctccg 25

<210> 10
<211> 70
<212> DNA
<213> artificial

<220>
<223> target (no. 1263) DNA substrate available from sigma Genosys

<400> 10

tggtcatcag ggctttacct cccggacaat ccggagctta cggagtacct gtagagcttc 60
ctgtgcaagc 70

<210> 11
<211> 20
<212> DNA
<213> artificial

<220>
<223> target (no. 1265) DNA Helicase substrate dsDNA available from sigma Genosys

<400> 11
gcactggccg tcgttttacc 20

<210> 12
<211> 4
<212> PRT
<213> artificial

<220>
<223> target (no. 1921) peptide substrate available from Sigma-Aldrich-Fluka

<220>
<221> misc_feature Ala
<222> (1)..(1)
<223> protected by succinyl group

<220>
<221> misc_feature phe
<222> (4)..(4)
<223> protected by 4-nitroanilide group

<400> 12

Ala Ala Pro Phe
1

<210> 13
<211> 405
<212> DNA
<213> unknown

<220>
<223> isolated from unknown organism from coastal seawater of Kolguev Island, Barents Sea, Russia (KOL)

<400> 13

atgtggatac ctcgcaacaa ggctaagtcg agtgccagta aaggcaaaac cggtgatagc 60
agtcatcgtg ccgcaggcga acgccgagaa ttagacgccg aagagtggct gatacaacgt 120
ggctttgtac ccataacccg caattaccgc acgcgcggcg gcgaaatcga tctgattatg 180
cgcgacgccg atacccttgt gtttgtagaa gtacgttatc gtaaaaccac ggagcacggc 240
acgggggcag aaaccattac ctatcacaaa cagcagcgac tacgtcgtgc tgccctacac 300
tacctgcaaa agcattttgg tagccgcgaa ccgccttgtc gatttgatgt tatgtcaggt 360
actggcgacc cagttatctt cgattggatt agtaatgcgt tttaa 405

<210> 14
<211> 134
<212> PRT
<213> unknown

<220>
<223> isolated from unknown organism from coastal seawater of Kolguev Island, Barents Sea, Russia (KOL)

<400> 14

Met Trp Ile Pro Arg Asn Lys Ala Lys Ser Ser Ala Ser Lys Gly Lys
1 5 10 15

Thr Gly Asp Ser Ser His Arg Ala Ala Gly Glu Arg Arg Glu Leu Asp
20 25 30

Ala Glu Glu Trp Leu Ile Gln Arg Gly Phe Val Pro Ile Thr Arg Asn
35 40 45

Tyr Arg Thr Arg Gly Gly Glu Ile Asp Leu Ile Met Arg Asp Ala Asp
50 55 60

Thr Leu Val Phe Val Glu Val Arg Tyr Arg Lys Thr Thr Glu His Gly
65 70 75 80

Thr Gly Ala Glu Thr Ile Thr Tyr His Lys Gln Gln Arg Leu Arg Arg
85 90 95

Ala Ala Leu His Tyr Leu Gln Lys His Phe Gly Ser Arg Glu Pro Pro
100 105 110

Cys Arg Phe Asp Val Met Ser Gly Thr Gly Asp Pro Val Ile Phe Asp
115 120 125

Trp Ile Ser Asn Ala Phe
130

<210> 15
<211> 8
<212> PRT

<213> unknown

<220>
<223> isolated from unknown organism from coastal seawater of Kolguev Island, Barents Sea, Russia (KOL)

<400> 15

```
Thr Thr Glu His Gly Thr Gly Ala
1               5
```

<210> 16
<211> 8
<212> PRT
<213> unknown

<220>
<223> isolated from unknown organism from coastal seawater of Kolguev Island, Barents Sea, Russia (KOL)

<400> 16

```
Pro Pro Cys Arg Phe Asp Val Met
1               5
```

<210> 17
<211> 23
<212> DNA
<213> artificial

<220>
<223> PCR amplification primer

<220>
<221> misc_feature
<222> (3)..(3)
<223> n is inosine and is represented by 'i' in Table 4

<220>
<221> misc_feature
<222> (6)..(6)
<223> n is inosine and is represented by 'i' in Table 4

<220>
<221> misc_feature
<222> (15)..(15)
<223> n is inosine and is represented by 'i' in Table 4

<220>
<221> misc_feature
<222> (18)..(18)
<223> n is inosine and is represented by 'i' in Table 4

<220>
<221> misc_feature
<222> (21)..(21)
<223> n is inosine and is represented by 'i' in Table 4

<400> 17
acnacngarg arggnacngg ngc 23

<210> 18
<211> 24
<212> DNA
<213> artificial

<220>
<223> PCR amplification primer

<220>
<221> misc_feature
<222> (4)..(4)
<223> n is a, c, g, or t

<220>
<221> misc_feature
<222> (7)..(7)
<223> n is a, c, g, or t

<220>
<221> misc_feature
<222> (10)..(10)
<223> n is a, c, g, or t

<220>
<221> misc_feature
<222> (13)..(13)
<223> n is a, c, g, or t

<220>
<221> misc_feature
<222> (19)..(19)
<223> n is a, c, g, or t

<220>
<221> misc_feature
<222> (22)..(22)
<223> n is a, c, g, or t

<400> 18
catnacntcn aanckrcang gngg 24

<210> 19
<211> 30
<212> DNA
<213> artificial

<220>
<223> PCR amplification primer

<400> 19
atgtggatac ctcgcaacaa ggctaagtcg 30

<210> 20
<211> 36
<212> DNA
<213> artificial

<220>

<223> PCR amplification primer

<400> 20
ttaaaacgca ttactaatcc aatcgaagat aactgg 36

<210> 21
<211> 519
<212> DNA
<213> unknown

<220>
<223> isolated from unknown organism from coastal seawater of Kolguev Island, Barents Sea, Russia (KOL)

<400> 21

atgcatcagt tggcagtcac cggcaagcag gcagctgatg tggctgtgct gctgggcggt 60
caacagcttg aggtgcaccg gatagagcgg gatgacgtct tgatccagca cctgatcgag 120
ctggaacgtc agttctggca ttacgttgaa accgacacac cgccaccagc cgacggctct 180
gattccgctg acatggcact gcgtctgctc tacccggaag acacaggtat ggttattgac 240
ctctctcagg atcagtccct gaacgagtcc tataccgagc tcaagcaggt acggcagtca 300
ctctctgatc tgagcacccg agaatctgtt ctcaagcagc gtcttcaaga gtccatgggg 360
tcagccagta aagccgtgtt tgccaatggc tctatcactt ggaagaaagc caaggatggc 420
atcgccatgg atatggaggc cctgttcaag gcgcaccctg acctgaaaac ccaataccag 480
atcagcaagc ctggcagcag gcgctttctg gtcaattaa 519

<210> 22
<211> 172
<212> PRT
<213> unknown

<220>
<223> isolated from unknown organism from coastal seawater of Kolguev Island, Barents Sea, Russia (KOL)

<400> 22

Met His Gln Leu Ala Val Thr Gly Lys Gln Ala Ala Asp Val Ala Val
1               5                   10                  15

Leu Leu Gly Gly Gln Gln Leu Glu Val His Arg Ile Glu Arg Asp Asp
            20                  25                  30

Val Leu Ile Gln His Leu Ile Glu Leu Glu Arg Gln Phe Trp His Tyr
        35                  40                  45

Val Glu Thr Asp Thr Pro Pro Pro Ala Asp Gly Ser Asp Ser Ala Asp
    50                  55                  60

Met Ala Leu Arg Leu Leu Tyr Pro Glu Asp Thr Gly Met Val Ile Asp
65                  70                  75                  80

Leu Ser Gln Asp Gln Ser Leu Asn Glu Ser Tyr Thr Glu Leu Lys Gln
                85                  90                  95

Val Arg Gln Ser Leu Ser Asp Leu Ser Thr Arg Glu Ser Val Leu Lys
            100                 105                 110

Gln Arg Leu Gln Glu Ser Met Gly Ser Ala Ser Lys Ala Val Phe Ala
        115                 120                 125

Asn Gly Ser Ile Thr Trp Lys Lys Ala Lys Asp Gly Ile Ala Met Asp
    130                 135                 140

Met Glu Ala Leu Phe Lys Ala His Pro Asp Leu Lys Thr Gln Tyr Gln
145                 150                 155                 160

Ile Ser Lys Pro Gly Ser Arg Arg Phe Leu Val Asn
                165                 170

<210> 23
<211> 11
<212> PRT
<213> unknown

<220>
<223> isolated from unknown organism from coastal seawater of Kolguev Island, Barents Sea, Russia (KOL)

<400> 23

Gly Val Pro Lys Tyr Val Glu Val Gln Val Met
1               5                   10

<210> 24
<211> 6
<212> PRT
<213> unknown

<220>
<223> isolated from unknown organism from coastal seawater of Kolguev Island, Barents Sea, Russia (KOL)

<400> 24

Ala Ser Lys Ala Val Phe
1 5

<210> 25
<211> 33
<212> DNA
<213> artificial

<220>
<223> PCR amplification primer

<220>
<221> misc_feature
<222> (3)..(3)
<223> n is a, c, g, or t

<220>
<221> misc_feature
<222> (6)..(6)
<223> n is a, c, g, or t

<220>
<221> misc_feature
<222> (9)..(9)
<223> n is a, c, g, or t

<220>
<221> misc_feature
<222> (12)..(12)
<223> n is a, c, g, or t

<220>
<221> misc_feature
<222> (15)..(15)
<223> n is a, c, g, or t

<220>
<221> misc_feature
<222> (18)..(18)
<223> n is a, c, g, or t

<220>
<221> misc_feature
<222> (24)..(24)
<223> n is a, c, g, or t

<220>
<221> misc_feature
<222> (30)..(30)
<223> n is a, c, g, or t

<400> 25
ggngtnccna antangtnga rgtncargtn atg 33

<210> 26
<211> 18

<212> DNA
<213> artificial

<220>
<223> PCR amplification primer

<220>
<221> misc_feature
<222> (4)..(4)
<223> n is a, c, g, or t

<220>
<221> misc_feature
<222> (7)..(7)
<223> n is a, c, g, or t

<220>
<221> misc_feature
<222> (10)..(10)
<223> n is a, c, g, or t

<220>
<221> misc_feature
<222> (13)..(13)
<223> n is a, c, g, or t

<220>
<221> misc_feature
<222> (16)..(16)
<223> n is a, c, g, or t

<400> 26
raanacngcn ttnswngc 18

<210> 27
<211> 24
<212> DNA
<213> artificial

<220>
<223> PCR amplification primer

<400> 27
atgcatcagt tggcagtcac cggc 24

<210> 28
<211> 27
<212> DNA
<213> artificial

<220>
<223> PCR amplification primer

<400> 28
ttaattgacc agaaagcgcc tgctgcc 27

<210> 29
<211> 888

<212> DNA
<213> unknown

<220>
<223> isolated from unknown organism from coastal seawater of Kolguev Island, Barents Sea, Russia (KOL)

<400> 29

```
attgtaatac gactcactat agggcgaatt gggcccgacg tcgcatgctc ccggccgcca     60
tggcggccgc gggaattcga ttatgtggcc agctatcgct tcggccatca gatttacacc    120
cccgatgaca tcgaaatagc gtcattgata gaagacgatc gcccttatgc gggcctagcc    180
tatgctggcc tgtcgatctt ccaatcccgg gaccaggggc aatggcgcga tagtcgtgcc    240
tggcacatgg atctgggcct agtcggcccg ggtgccggag gccagcgctt ccagagtgct    300
gtccatggtg ctaccggcag tgatgagccc aaaggttggg acaatcagct tgagaatgaa    360
cccttcttca acgtggccta cgggcagcgt tggtggcggc agtcccgcct gggatctttg    420
gagcttgaat acgggcctgc aatgggcgcg gcggctggca atctttacac ctatgcttcc    480
agcggccttg gcttgcgctt tggcaaaggg ctggaacaca gcttggggtt accgtctatc    540
aacccgggct atggtagcgg cgcctacttc gagccggggc aatcgttcgc ctggttcggt    600
tacgtcaatg ttgatggccg ttacatggct cataacatgc tgctggacgg aaacaccttc    660
agcaacagcc attccgtaga ccgggagcaa tgggtcggcg atctgcaggc gggtatcgcc    720
ctgacctggg agcgctggca agtgagcttt gccagtgtgt ggcgcacccg ggagttcaag    780
ggccagactg aacccgatca atttgggtcg ctggtggaat cactagtgaa ttcgcggccg    840
cctgcaggtc gaccatatgg gagagctccc aacgcgttgg atgcatga                 888
```

<210> 30
<211> 295
<212> PRT
<213> unknown

<220>
<223> isolated from unknown organism from coastal seawater of Kolguev Island, Barents Sea, Russia (KOL)

<400> 30

```
Ile Val Ile Arg Leu Thr Ile Gly Arg Ile Gly Pro Asp Val Ala Cys
1               5                   10                  15

Ser Arg Pro Pro Trp Arg Pro Arg Glu Phe Asp Tyr Val Ala Ser Tyr
            20                  25                  30

Arg Phe Gly His Gln Ile Tyr Thr Pro Asp Asp Ile Glu Ile Ala Ser
        35                  40                  45
```

Leu Ile Glu Asp Asp Arg Pro Tyr Ala Gly Leu Ala Tyr Ala Gly Leu
50 55 60

Ser Ile Phe Gln Ser Arg Asp Gln Gly Gln Trp Arg Asp Ser Arg Ala
65 70 75 80

Trp His Met Asp Leu Gly Leu Val Gly Pro Gly Ala Gly Gly Gln Arg
85 90 95

Phe Gln Ser Ala Val His Gly Ala Thr Gly Ser Asp Glu Pro Lys Gly
100 105 110

Trp Asp Asn Gln Leu Glu Asn Glu Pro Phe Phe Asn Val Ala Tyr Gly
115 120 125

Gln Arg Trp Trp Arg Gln Ser Arg Leu Gly Ser Leu Glu Leu Glu Tyr
130 135 140

Gly Pro Ala Met Gly Ala Ala Ala Gly Asn Leu Tyr Thr Tyr Ala Ser
145 150 155 160

Ser Gly Leu Gly Leu Arg Phe Gly Lys Gly Leu Glu His Ser Leu Gly
165 170 175

Leu Pro Ser Ile Asn Pro Gly Tyr Gly Ser Gly Ala Tyr Phe Glu Pro
180 185 190

Gly Gln Ser Phe Ala Trp Phe Gly Tyr Val Asn Val Asp Gly Arg Tyr
195 200 205

Met Ala His Asn Met Leu Leu Asp Gly Asn Thr Phe Ser Asn Ser His
210 215 220

Ser Val Asp Arg Glu Gln Trp Val Gly Asp Leu Gln Ala Gly Ile Ala
225 230 235 240

Leu Thr Trp Glu Arg Trp Gln Val Ser Phe Ala Ser Val Trp Arg Thr
245 250 255

Arg Glu Phe Lys Gly Gln Thr Glu Pro Asp Gln Phe Gly Ser Leu Val
260 265 270

Glu Ser Leu Val Asn Ser Arg Pro Pro Ala Gly Arg Pro Tyr Gly Arg
275 280 285

Ala Pro Asn Ala Leu Asp Ala
290 295

<210> 31
<211> 9
<212> PRT
<213> unknown

<220>
<223> isolated from unknown organism from coastal seawater of Kolguev Island, Barents Sea, Russia (KOL)

<400> 31

Pro Tyr Ala Gly Leu Ala Tyr Ala Gly
1               5

<210> 32
<211> 10
<212> PRT
<213> unknown

<220>
<223> isolated from unknown organism from coastal seawater of Kolguev Island, Barents Sea, Russia (KOL)

<400> 32

Trp Val Gly Asp Leu Gln Ala Gly Ile Ala
1               5                   10

<210> 33
<211> 26
<212> DNA
<213> artificial

<220>
<223> PCR amplification primer

<220>
<221> misc_feature
<222> (3)..(3)
<223> n is a, c, g, or t

<220>
<221> misc_feature
<222> (6)..(6)
<223> n is a, c, g, or t

<220>
<221> misc_feature
<222> (9)..(9)
<223> n is a, c, g, or t

<220>
<221> misc_feature
<222> (12)..(12)
<223> n is a, c, g, or t

<220>
<221> misc_feature
<222> (15)..(15)
<223> n is a, c, g, or t

<220>
<221> misc_feature
<222> (18)..(18)

<223> n is a, c, g, or t

<220>
<221> misc_feature
<222> (24)..(24)
<223> n is a, c, g, or t

<400> 33
ccntangcng gnytngcnta ygcngg 26

<210> 34
<211> 26
<212> PRT
<213> artificial

<220>
<223> PCR amplification primer

<400> 34

```
Ala Ala Asn Gly Ala Asn Gly Ala Asn Gly Ala Asn Met Gly Asn Cys
1               5                   10                  15

Cys Asn Thr Ala Tyr Gly Cys Asn Gly Gly
            20                  25
```

<210> 35
<211> 28
<212> DNA
<213> artificial

<220>
<223> PCR amplification primer

<220>
<221> misc_feature
<222> (3)..(3)
<223> n is a, c, g, or t

<220>
<221> misc_feature
<222> (6)..(6)
<223> n is a, c, g, or t

<220>
<221> misc_feature
<222> (9)..(9)
<223> n is a, c, g, or t

<220>
<221> misc_feature
<222> (15)..(15)
<223> n is a, c, g, or t

<220>
<221> misc_feature
<222> (21)..(21)

<223> n is a, c, g, or t

<220>
<221> misc_feature
<222> (24)..(24)
<223> n is a, c, g, or t

<400> 35
gcnatnccng cytgnarytc nccnaccc 28

<210> 36
<211> 24
<212> DNA
<213> artificial

<220>
<223> PCR amplification primer

<400> 36
atgtggccag ctatcgcttc ggcc 24

<210> 37
<211> 24
<212> DNA
<213> artificial

<220>
<223> PCR amplification primer

<400> 37
tcaaaacgcc cgagccacca ccag 24

<210> 38
<211> 474
<212> DNA
<213> unknown

<220>
<223> isolated from unknown organism from acidic of Porto Levante, vulcanic Island, Italy (VUL)

<400> 38

atgttgcgct tggtgcaggg gggattcagt aatatggcag caccgattca gaaagtcatc 60
cgacaggaac ccgtcaaaaa tccgcttgag tctgggagcc cgtcggcttc ggaggaactt 120
caactattgg ttgaagaact gcatcagagc ggggttctcg aggctgcacg gtcgatgctt 180
ggagcaaagg attccatcgc caaaatcctg gtcgagcaac tgctgagaaa ggatgtactg 240
acgcttatca acaatttgat ggcggcaggc accgttctga caaagctcga tccagcgcag 300
ctcgagcgct tgaccgaagg actgagtgcc ggggtaacag aggcacatca gacaatcgaa 360
gcgaatcagt caatcagtat catgggactt ttgaagacat tgcaagaccc cgatgtaaac 420
cgggcactcc agtttgccat cgggttcctc aggggtctcg ggaaaactat ctga 474

<210> 39
<211> 157

<212> PRT
<213> unknown

<220>
<223> isolated from unknown organism from acidic of Porto Levante, vulcanic Island, Italy (VUL)

<400> 39

```
Met Leu Arg Leu Val Gln Gly Gly Phe Ser Asn Met Ala Ala Pro Ile
1               5                   10                  15

Gln Lys Val Ile Arg Gln Glu Pro Val Lys Asn Pro Leu Glu Ser Gly
            20                  25                  30

Ser Pro Ser Ala Ser Glu Glu Leu Gln Leu Leu Val Glu Glu Leu His
        35                  40                  45

Gln Ser Gly Val Leu Glu Ala Ala Arg Ser Met Leu Gly Ala Lys Asp
    50                  55                  60

Ser Ile Ala Lys Ile Leu Val Glu Gln Leu Leu Arg Lys Asp Val Leu
65                  70                  75                  80

Thr Leu Ile Asn Asn Leu Met Ala Ala Gly Thr Val Leu Thr Lys Leu
                85                  90                  95

Asp Pro Ala Gln Leu Glu Arg Leu Thr Glu Gly Leu Ser Ala Gly Val
            100                 105                 110

Thr Glu Ala His Gln Thr Ile Glu Ala Asn Gln Ser Ile Ser Ile Met
        115                 120                 125

Gly Leu Leu Lys Thr Leu Gln Asp Pro Asp Val Asn Arg Ala Leu Gln
    130                 135                 140

Phe Ala Ile Gly Phe Leu Arg Gly Leu Gly Lys Thr Ile
145                 150                 155
```

<210> 40
<211> 8
<212> PRT
<213> unknown

<220>
<223> isolated from unknown organism from acidic of Porto Levante, vulcanic Island, Italy (VUL)

<400> 40

```
Val Glu Glu Leu His Gln Ser Gly
1               5
```

<210> 41
<211> 6
<212> PRT

<213> unknown

<220>
<223> isolated from unknown organism from acidic of Porto Levante, vulcanic Island, Italy (VUL)

<400> 41

```
Glu Ala Ala Arg Ser Met
1               5
```

<210> 42
<211> 6
<212> PRT
<213> unknown

<220>
<223> isolated from unknown organism from acidic of Porto Levante, Vulcanic Island, Italy (VUL)

<400> 42

```
Pro Asp Val Asn Arg Ala
1               5
```

<210> 43
<211> 6
<212> PRT
<213> unknown

<220>
<223> isolated from unknown organism from acidic of Porto Levante, vulcanic Island, Italy (VUL)

<400> 43

```
Gln Phe Ala Ile Gly Phe
1               5
```

<210> 44
<211> 23
<212> DNA
<213> artificial

<220>
<223> PCR amplification primer

<220>
<221> misc_feature
<222> (3)..(3)
<223> n is a, c, g, or t

<220>
<221> misc_feature
<222> (6)..(6)
<223> n is a, c, g, or t

<220>
<221> misc_feature
<222> (9)..(10)

<223> n is a, c, g, or t

<220>
<221> misc_feature
<222> (12)..(12)
<223> n is a, c, g, or t

<220>
<221> misc_feature
<222> (15)..(15)
<223> n is a, c, g, or t

<220>
<221> misc_feature
<222> (21)..(21)
<223> n is a, c, g, or t

<400> 44
gtngangann tncancarws ngg 23

<210> 45
<211> 18
<212> DNA
<213> artificial

<220>
<223> PCR amplification primer

<220>
<221> misc_feature
<222> (3)..(3)
<223> n is inosine and is represented by ’i’ in Table 4

<220>
<221> misc_feature
<222> (6)..(6)
<223> n is inosine and is represented by ’i’ in Table 4

<220>
<221> misc_feature
<222> (9)..(9)
<223> n is inosine and is represented by ’i’ in Table 4

<220>
<221> misc_feature
<222> (12)..(12)
<223> n is inosine and is represented by ’i’ in Table 4

<220>
<221> misc_feature
<222> (15)..(15)
<223> n is inosine and is represented by ’i’ in Table 4

<400> 45
gangcngcnm gnwgnatg 18

<210> 46
<211> 16

<212> DNA
<213> artificial

<220>
<223> PCR amplification primer

<220>
<221> misc_feature
<222> (2)..(2)
<223> n is a, c, g, or t

<220>
<221> misc_feature
<222> (5)..(5)
<223> n is a, c, g, or t

<220>
<221> misc_feature
<222> (8)..(8)
<223> n is a, c, g, or t

<220>
<221> misc_feature
<222> (14)..(14)
<223> n is a, c, g, or t

<400> 46
cncknttnac rtcngg 16

<210> 47
<211> 18
<212> DNA
<213> artificial

<220>
<223> PCR amplification primer

<220>
<221> misc_feature
<222> (4)..(4)
<223> n is a, c, g, or t

<220>
<221> misc_feature
<222> (7)..(7)
<223> n is a, c, g, or t

<220>
<221> misc_feature
<222> (10)..(10)
<223> n is a, c, g, or t

<400> 47
raanccnatn gcraaytg 18

<210> 48
<211> 21
<212> DNA

<213> artificial

<220>
<223> PCR amplification primer

<400> 48
atgttgcgct tggtgcaggg g 21

<210> 49
<211> 21
<212> DNA
<213> artificial

<220>
<223> PCR amplification primer

<400> 49
ctagatagtt ttcccgagac c 21

<210> 50
<211> 486
<212> DNA
<213> unknown

<220>
<223> isolated from unknown organism from brine-seawater interface above hypersaline anoxic basin L'Atalante, Eastern Mediterranean Sea (L'A)

<400> 50

ttattgatgg cagagatgac agaagaaaaa ttagctgagt atttagaacc tttatcagaa 60

attttatcac gctatgaaaa aaaagagaga tatttaattc cggttttaca ggaagctcag 120

gaggaatatg gttatttacc ggaagaagta atgaaagaaa tagcattggg cttaaatctt 180

tctttaagtc aggtatatgg ggttgtaaca ttttacagtc agtttcatca ggagccaaga 240

ggtaataata ttattcgggt ttgtctggga acagcctgtc atgttagagg tggagatgga 300

atcttaaatg ctattaaaga tgaactggga attgatgcag gagaaacaac tgatgattta 360

gaatttacac ttgaatctgt ggcctgtatt ggtgcctgtg gtctggctcc agttataatg 420

gtcaatgatg atacccacgg ccgtttaact ccggaaaaag ttcctgaaat tatggcaaag 480

tataaa 486

<210> 51
<211> 162
<212> PRT
<213> unknown

<220>
<223> isolated from unknown organism from brine-seawater interface above hypersaline anoxic basin L'Atalante, Eastern Mediterranean Sea (L'A)

<400> 51

Leu Leu Met Ala Glu Met Thr Glu Glu Lys Leu Ala Glu Tyr Leu Glu
1 5 10 15

Pro Leu Ser Glu Ile Leu Ser Arg Tyr Glu Lys Lys Glu Arg Tyr Leu
20 25 30

Ile Pro Val Leu Gln Glu Ala Gln Glu Glu Tyr Gly Tyr Leu Pro Glu
35 40 45

Glu Val Met Lys Glu Ile Ala Leu Gly Leu Asn Leu Ser Leu Ser Gln
50 55 60

Val Tyr Gly Val Val Thr Phe Tyr Ser Gln Phe His Gln Glu Pro Arg
65 70 75 80

Gly Asn Asn Ile Ile Arg Val Cys Leu Gly Thr Ala Cys His Val Arg
85 90 95

Gly Gly Asp Gly Ile Leu Asn Ala Ile Lys Asp Glu Leu Gly Ile Asp
100 105 110

Ala Gly Glu Thr Thr Asp Asp Leu Glu Phe Thr Leu Glu Ser Val Ala
115 120 125

Cys Ile Gly Ala Cys Gly Leu Ala Pro Val Ile Met Val Asn Asp Asp
130 135 140

Thr His Gly Arg Leu Thr Pro Glu Lys Val Pro Glu Ile Met Ala Lys
145 150 155 160

Tyr Lys

<210> 52
<211> 11
<212> PRT
<213> unknown

<220>
<223> isolated from unknown organism from brine-seawater interface above hypersaline anoxic basin L'Atalante, Eastern Mediterranean Sea (L'A)

<400> 52

Leu Leu Met Ala Glu Met Thr Glu Glu Lys Leu
1 5 10

<210> 53
<211> 14
<212> PRT
<213> unknown

<220>
<223> isolated from unknown organism from brine-seawater interface above hypersaline anoxic basin L'Atalante,

Eastern Mediterranean Sea (L'A)

<400> 53

```
Lys Glu Ile Ala Leu Gly Leu Asn Leu Ser Leu Ser Gln Val
1               5                   10
```

<210> 54
<211> 12
<212> PRT
<213> unknown

<220>
<223> isolated from unknown organism from brine-seawater interface above hypersaline anoxic basin L'Atalante, Eastern Mediterranean Sea (L'A)

<400> 54

```
Pro Glu Lys Val Pro Glu Ile Met Ala Lys Tyr Lys
1               5                   10
```

<210> 55
<211> 40
<212> DNA
<213> artificial

<220>
<223> PCR amplification primer

<400> 55
gacgacgaca agatgatgtt attgatggca gagatgacag 40

<210> 56
<211> 44
<212> DNA
<213> artificial

<220>
<223> PCR amplification primer

<400> 56
gaggagaagc ccggttattt atactttgcc ataatttcag gaac 44

<210> 57
<211> 5210
<212> DNA
<213> unknown

<220>
<223> multifunctunal alpha/beta-hydrolase mined from a metagenome library of a microbial community in seawater contaminated with petroleum hydrocarbons

<220>
<221> misc_feature
<222> (3298)..(3298)
<223> n is a, c, g, or t

<400> 57

```
cgctcccgca cgaatatgaa cgctgtgaaa ttccggtgta tatgcgccaa caaggaccaa     60
atcatgccag tcaagccaaa ctatggccta ataaggtctg ggtttacgag taccgcctaa    120
gtactattgg ccatcgtcga atcctaacag gtgactacac atctgtttat ccacccatcg    180
ataacaataa ggtttactga tgaatctcgt taaaagcctg ataatactgc ttgtgatcgc    240
agtgatcgga atatttgcta gcgtccactt tgctccgttc gaaacagctt cttgtcttgt    300
cgatattaaa cgcaatatcg ccggcctaga gcgcaaaagc atttccctcg ccgatggtaa    360
ccaatatgtt tatcttgaag gcggcaaggg tgaaaccttg gtattacttc acggttttgg    420
tgccgataaa gataacttta ccgaagttag cccttactta acgggcgact tccatgttat    480
tgctcccgac cacattggct ttggtgaatc ctctaagcca acaggcgcag attatagccc    540
tatcgctcaa gcgcagcgtt tgcatgaatt ggttgcgcgc ctcggtttag agcgcttcca    600
cttaggtgga agctcaatgg gtggccacat agcgatgaca tacgccacgc tatacccgca    660
tgaagttaag agcttatggt tactcgaccc aggtggtgtt tggtccgctc cggaagccga    720
aatgcgcact atcattcgta aaactggggt gaatccgtta acagctaaaa ccccagaaga    780
gttccgtaaa gtattcgata tcgtgatgag caagcccccct ttcatcccag gctttgtact    840
cgatgaaatg gcgaaaaagc gtatcgctaa cttcgattta gaacagaata tttttgccca    900
actatctgca gacaatgtcg aagaacgcgt ccgcggccta accaccccta ccttgctagt    960
atggggcgct gaagatcgag tactcaaccc agaagcagca cccattctgg aaggactttt   1020
gaccaatgtt aaaacgatta tcatgccagg tattggtcac ctgcctatgc tagaagcgcc   1080
aaagcaaaca gcaaccgact taaaagcatt tattgctgat ttgcctgaat aaaactaata   1140
ggcgttgaat ttaacagcgc ccagaaaaaa gcggccctct gcaaataaac aggggccgct   1200
tttttatac ttaaaaggtc ggattaatta gcttcaatgc tcacatctaa agacatttca   1260
```

ccaccgttct catccgtacc ggacgccgaa aaagccagtt gatcatcact cgttgcggta 1320
atttcaaacg cataagtcgc cgtgctctga caggcatcat cactctcatc acacgtacct 1380
tcgccataaa tatcactttg accggaaata atatcctgcg agaaggtgtt gtcgtaaacc 1440
aaagttacgt tcaatccgat tgtgctcgca tcgccagaaa acgctgcctt ctcccccgcc 1500
agcgtcatga tttcagcgta attgacagcg gatatagagg tgttgctatt caccacaccg 1560
gcaccaactc ccttcgaagc accaacacac gctaacgaat cacttaattc tgcaaaggtg 1620
gcactggcgg aacccacagt aaaatcgaca cctatcgcgt attcactagc ggcatcggtg 1680
aacgaagtgg catcgctaac cttcacaccc accatgcgcg tattttgcgc aatgctgtac 1740
ccgtcccccg tcgcatcagt ctgcaagctg ccagacaagc tgcggttgtt gggttactat 1800
cagactccaa ttgtaagagt cctcaaccga gccacttaat gtgccatcag caagcgttaa 1860
cgtgatatcg gtaaaatcta attcgtgagc aatattacat gcccaaaagc ggtactcacc 1920
ttcggtttct aatgcgctca gtaataccag ttggcgacct tgatagtagt tggtggtatc 1980
actgctatcg gtcacttcat aatcaaactt cgaactggta tttatcagcc ataagccttc 2040
aggggtatct gaacttaatg ccgcatccgc tgctggaaga tctgccccca attcagctaa 2100
ggtttgttgc gcaagttgcg tggtagggtc aacgtcgatc acggtagcga cattggtact 2160
gctactgtta ccactgcagg ccgataccag tgcagcaacg accaagcaat aggccgactt 2220
tttgaattgg gagtgcatca tgatttcctt ttgatattct tatttaatcc tagaagggga 2280
tcatagcaga cgcaattgca gacctaaggg caattcgggc ccattaccaa tacgattatc 2340
cccacttacc ttggcctcct agcgttcacg ccgttataat cgcccgcaga aattcatcct 2400
cctatcctca cgggactatc tcatgacctc taaaaccgtc gtttccgaac gcctgtatga 2460
tggctacgcc caatccttca ctgtgagcga actgctgttt gaagtgaaaa cggaacacca 2520
acatctggaa atcttcgaaa cgccgtttct aggccgcgtt atgctgctgg atggagtggt 2580
acaaaccacc gagaaagacg aatttattta ccacgaaagc atggtccatg tgcctttgtt 2640
tgcccaccca gcccctaacg tgtgctaatt attggtggcg gcgacggcgg catcttgcgt 2700
gaagtgttgc gccacaaaaa cgtagaacac gtaacccaag ttgaaatcga cggcagcgtc 2760
atcgacatgt gcaaagaata cttcccacgt cattctaatg gtgccttcga tgacccgcgc 2820
gccactatcg tgatcgccga tggcaaagaa ttcgtcgcca actgccaaga caaatacgac 2880
gtcatcatct ccgactccac cgaccctatc ggcccaggcg aagtgctgtt tacctccgat 2940
ttctatgccg acgaaaaaac ctgcctgaac gaaggcggca tcatggtggc acagaacggc 3000
gtgccgttta tgcaaggcca agaaatcacc aataccttcc agcgcctaag caaactgtac 3060
gcggacaaca gcttctacgt tgcccccgtg ccaacctatg caggtggttt tatgacctta 3120
gcctgggcaa ccgacgacgc ctcattgcgc aagcaaagcg ttgaacagat tcaagcgcgt 3180
tacgacgccg cagggtttag cacacgttat tacaacccag agattcatgt tgcggcattt 3240
gcattgccga attatgtgaa agctttgatg gtgtgatttt atgcttgaag tcaaatgnct 3300

tcgaccaatc ttgtaggagg gagtcagcga acgtttttg ttcgtactcc cgattactgc 3360
agttttcgtc actgctcgct gtcgctccct gagtgactcc tacagaaaga tccacccttg 3420
gttttaagtc cgtgttgcga aaagaactcg cggcgtcagc cgcaaaaccc accaatagct 3480
acaaaatccc accgtcactg ttataatctc gcgtctttaa attttcgaac gttgcataac 3540
gcacgcaaac aggaatcctc gctgtgagcc agagtcgcca taacgtcaaa acattccaag 3600
gcttaatcgc tgccttgcag gaatactggt ccgaacaggg ctgtgtaatc aaccaaccac 3660
tcgatatgga agtcggtgcc ggtactttcc ataccgcgac gtttttgcgt gctattggcc 3720
cagaaaactg gagtgctgct tatgttcaac caagccgccg tcctactgat ggccgctatg 3780
gtgaaaaccc gaaccgcttg caacattact accaatttca ggtagtgatg aagccaaacc 3840
cagtggatat tcaagaaaag taccttgagt cgctgcgcgt gatgggcgtt gatcctttgg 3900
ttcacgatat tcgtttcgtt gaagacaact gggaatcacc aacgctaggc gcttggggtt 3960
tgggctggga agtttggctt aacggtatgg aagtgactca gttcacttac ttccagcaag 4020
ttggcggttt ggaatgtttc cccgtaaccg gcgaaatcac ttacggtctt gagcgtatcg 4080
ccatgtacct gcaagaagtg gattctgtct acgacttagt ttggacttac ggcccagacg 4140
gcaaagctgt gacctacggc gatgtgttcc atcaaaacga agtagaacag tcggcctata 4200
acttcgaaca cgccgatgtc gatttcttat tcaaagcctt cgaccaatac gagaaagact 4260
gcaaacgtct gatcgaagtt ggcttgccgc tacccgctta cgagcaggta ttgaaaggct 4320
cgcatacctt taacttactc gatgctcgcg gcgcgatttc tgtgactgag cgtcagggct 4380
atatcttgcg tgtacgtacc ttagcgcgct cggttgctga agcttacttc aacagccgtg 4440
ccgaaaaagg cttcccgctg gcgaccgaag caaaccgcgc cgaagtatta gcgaagtacg 4500
aagcagccaa ggcgaaaaaa gccgataaag acgctgccca gcaggagacc aaataatgag 4560
cactcgtgat ttcttagtag agctaggcac cgaagagctg ccaccgaaag cgcttaaaaa 4620
tctgtctaac gcgtttgccc agggcattga acaaggtttg aaagacgccg gtttaaccat 4680
gggtgcgatt gaacaattcg ccgcgccacg tcgtttagcc gtgcgcattt cggagttacc 4740
agagcagcaa gccgatcaag aagaagtgct atacggcccg ccagccaaca tcgcctttga 4800
cgccgatggt aagccaacca aagccgcctt aggctttgcg gcccgcgccg gtgccgatgc 4860
gtctgaatta aaaacagcgc cagattctga caaaaagaat gccggtaagc taatgctcga 4920
acgtacgatc aaaggcaaaa ataccactga gctattggcc gctattgtgc aaaacagctt 4980
agataagttg ccgattccta agcgtatgcg ctggggttcg tcacgtattg aattcgtgcg 5040
ccccgtacag tggttagtca tgctgtttgg taacgacgtg gtcgatgccg aagcgcttgg 5100
cttaaaagcc ggcaacacca gccgtggtca ccgcttccat gcgccgggcg agatccaaag 5160
aattcaaaaa gcttctcgag agtacttcta gagcggccgc gggcccatcg 5210

<210> 58
<211> 933
<212> DNA
<213> unknown

<220>
<223> multifunctunal alpha/beta-hydrolase mined from a metagenome library of a microbial community in seawater contaminated with petroleum hydrocarbons

<400> 58

```
atgaatctcg ttaaaagcct gataatactg cttgtgatcg cagtgatcgg aatatttgct     60
agcgtccact ttgctccgtt cgaaacagct tcttgtcttg tcgatattaa acgcaatatc    120
gccggcctag agcgcaaaag catttccctc gccgatggta accaatatgt ttatcttgaa    180
ggcggcaagg gtgaaacctt ggtattactt cacggttttg gtgccgataa agataacttt    240
accgaagtta gcccttactt aacgggcgac ttccatgtta ttgctcccga ccacattggc    300
tttggtgaat cctctaagcc aacaggcgca gattatagcc ctatcgctca agcgcagcgt    360
ttgcatgaat tggttgcgcg cctcggttta gagcgcttcc acttaggtgg aagctcaatg    420
ggtggccaca tagcgatgac atacgccacg ctatacccgc atgaagttaa gagcttatgg    480
ttactcgacc caggtggtgt ttggtccgct ccggaagccg aaatgcgcac tatcattcgt    540
aaaactgggg tgaatccgtt aacagctaaa accccagaag agttccgtaa agtattcgat    600
atcgtgatga gcaagccccc tttcatccca ggctttgtac tcgatgaaat ggcgaaaaag    660
cgtatcgcta acttcgattt agaacagaat atttttgccc aactatctgc agacaatgtc    720
gaagaacgcg tccgcggcct aaccacccct accttgctag tatggggcgc tgaagatcga    780
gtactcaacc cagaagcagc acccattctg gaaggacttt tgaccaatgt taaaacgatt    840
atcatgccag gtattggtca cctgcctatg ctagaagcgc caaagcaaac agcaaccgac    900
ttaaaagcat ttattgctga tttgcctgaa taa                                 933
```

<210> 59
<211> 310
<212> PRT
<213> unknown

<220>
<223> multifunctunal alpha/beta-hydrolase mined from a metagenome library of a microbial community in seawater contaminated with petroleum hydrocarbons

<400> 59

```
Met Asn Leu Val Lys Ser Leu Ile Ile Leu Leu Val Ile Ala Val Ile
1               5                   10                  15

Gly Ile Phe Ala Ser Val His Phe Ala Pro Phe Glu Thr Ala Ser Cys
            20                  25                  30

Leu Val Asp Ile Lys Arg Asn Ile Ala Gly Leu Glu Arg Lys Ser Ile
        35                  40                  45

Ser Leu Ala Asp Gly Asn Gln Tyr Val Tyr Leu Glu Gly Gly Lys Gly
    50                  55                  60
```

```
Glu Thr Leu Val Leu Leu His Gly Phe Gly Ala Asp Lys Asp Asn Phe
65                  70                  75                  80

Thr Glu Val Ser Pro Tyr Leu Thr Gly Asp Phe His Val Ile Ala Pro
                85                  90                  95

Asp His Ile Gly Phe Gly Glu Ser Ser Lys Pro Thr Gly Ala Asp Tyr
            100                 105                 110

Ser Pro Ile Ala Gln Ala Gln Arg Leu His Glu Leu Val Ala Arg Leu
        115                 120                 125

Gly Leu Glu Arg Phe His Leu Gly Gly Ser Ser Met Gly Gly His Ile
    130                 135                 140

Ala Met Thr Tyr Ala Thr Leu Tyr Pro His Glu Val Lys Ser Leu Trp
145                 150                 155                 160

Leu Leu Asp Pro Gly Gly Val Trp Ser Ala Pro Glu Ala Glu Met Arg
                165                 170                 175

Thr Ile Ile Arg Lys Thr Gly Val Asn Pro Leu Thr Ala Lys Thr Pro
            180                 185                 190

Glu Glu Phe Arg Lys Val Phe Asp Ile Val Met Ser Lys Pro Pro Phe
        195                 200                 205

Ile Pro Gly Phe Val Leu Asp Glu Met Ala Lys Lys Arg Ile Ala Asn
    210                 215                 220

Phe Asp Leu Glu Gln Asn Ile Phe Ala Gln Leu Ser Ala Asp Asn Val
225                 230                 235                 240

Glu Glu Arg Val Arg Gly Leu Thr Thr Pro Thr Leu Leu Val Trp Gly
                245                 250                 255

Ala Glu Asp Arg Val Leu Asn Pro Glu Ala Ala Pro Ile Leu Glu Gly
            260                 265                 270

Leu Leu Thr Asn Val Lys Thr Ile Ile Met Pro Gly Ile Gly His Leu
        275                 280                 285

Pro Met Leu Glu Ala Pro Lys Gln Thr Ala Thr Asp Leu Lys Ala Phe
    290                 295                 300

Ile Ala Asp Leu Pro Glu
305                 310
```